# EUROPEAN PATENT SPECIFICATION

(11) **EP 1 911 461 B1**
(45) Date of publication and mention of the grant of the patent: **07.12.2011**
(21) Application number: 07023116.2
(22) Date of filing: 18.10.2001
(51) Int. Cl.: A61K 39/00

(54) **HLA class I and II binding peptides and their uses**
HLA-Klasse-I- und -Klasse-II-bindende Peptide und ihre Verwendungen
Peptides de liaisons HLA de classe I et II et leurs utilisations

(30) Priority: 19.10.2000 US 242350 P; 20.04.2001 US 285624 P
(43) Date of publication of application: 16.04.2008
(62) Divisional of application: 01274676.4
(73) Proprietor: Epimmune Inc., San Diego, CA 92121 (US)
(72) Inventor: Sette, Alessandro, La Jolla, CA 92037 (US); Sidney, John, San Diego, CA 92130 (US); Southwood, Scott, Santee, CA 92071 (US)
(74) Representative: Inspicos A/S

(56) References cited:
- WO-A-99/27954
- RESSING M E ET AL: "HUMAN CTL EPITOPES ENCODED BY HUMAN PAPILLOMAVIRUS TYPE 16 E6 AND E7 IDENTIFIED THROUGH IN VIVO AND IN VITRO IMMUNOGENICITY STUDIES OF HLA-A*0201-BINDING PEPTIDES" JOURNAL OF IMMUNOLOGY, THE WILLIAMS AND WILKINS CO. BALTIMORE, US, vol. 154, 1 June 1995 (1995-06-01), pages 5934-5943, XP002915422 ISSN: 0022-1767
- DATABASE PROTEIN [Online] 15 February 2000 (2000-02-15), "E6 protein [Human papillomavirus type 16]" XP002470722 retrieved from NCBI Database accession no. CAB45116
- HADIDA F ET AL: "CARBOXYL-TERMINAL AND CENTRAL REGIONS OF HUMAN IMMUNODEFICIENCY VIRUS-1 NEF RECOGNIZED BY CYTOTOXIC T LYMPHOCYTES FROM LYMPHOID ORGANS AN IN-VITRO LIMITING DILUTION ANALYSIS" JOURNAL OF CLINICAL INVESTIGATION, NEW YORK, NY, US, vol. 89, no. 1, 1992, pages 53-60, XP002406423 ISSN: 0021-9738
- DATABASE PROTEIN [Online] 27 December 2007 (2007-12-27), "E6 [Human papillomavirus type 45]" XP002470723 retrieved from NCBI Database accession no. ABP99888
- DATABASE PROTEIN [Online] 21 May 2007 (2007-05-21), "E6 protein [Human papillomavirus type 31]." XP002470724 retrieved from NCBI Database accession no. ABQ44344
- DATABASE PROTEIN [Online] 21 May 2007 (2007-05-21), "E6 protein [Human papillomavirus type 52]." XP002470725 retrieved from NCBI Database accession no. ABQ44341
- DATABASE PROTEIN [Online] 21 May 2007 (2007-05-21), "E6 protein [Human papillomavirus type 58]." XP002470726 retrieved from NCBI Database accession no. ABQ44337
- DATABASE PROTEIN [Online] 21 May 2007 (2007-05-21), "E6 protein [Human papillomavirus type 33]." XP002470727 retrieved from NCBI Database accession no. ABQ44339

## Description

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either class I or class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β₂ microglobulin. The peptide-MHC class I complex is then routed to the cell surface for expression and potential recognition by specific CTLs.

A complex of an HLA molecule and a peptidic antigen acts as the ligand recognized by HLA-restricted T cells (Buus, S. et:al., Cell 47:1071, 1986; Babbitt, B. P.. et al., Nature 317:359, 1985; Townsend, A. and Bodmer, H., Annu. Rev. Immunol. 7:601, 1989; Germain, R. N., Annu. Rev. Immunol. 11:403, 1993). Through the study of single amino acid substituted antigen analogs and the sequencing of endogenously bound, naturally processed peptides, critical residues that correspond to motifs required for specific binding to HLA antigen molecules have been identified *(see* also, *e.g*., Southwood, et al., J. Immunol. 160:3363, 1998; Rammensee, et al., Immunogenetics 41:178, 1995; Rammensee et al., SYFPEITHI, access via web at: http://134.2.96.221/scripts.hlaserver.dll/home.htm; Sette, A. and Sidney, J. Curr. Opin. Immunol. 10:478, 1998; Engelhard, V. H., Curr. Opin. Immunol. 6:13, 1994; Sette, A. and Grey, H. M., Curr. Opin. Immuno/. 4:79,1992; Sinigaglia, F. and Hammer, J. Curr. Biol. 6:52,1994; Ruppert et al., Cell 74:929-937, 1993; Kondo et al., J. Immunol. 155:4307-4312, 1995; Sidney et al., J. Immunol. 157:3480-3490, 1996; Sidney et al., Human Immunol. 45:79-93,1996; Sette, A. and Sidney, J. Immunogenetics 50:201-212, 1999).

Furthermore, x-ray crystallographic analysis of HLA-peptide complexes has revealed pockets within the peptide binding cleft of HLA molecules which accommodate, in an allele-specific mode, specific residues of peptide ligands; these residues in turn determine the HLA binding capacity of the peptides in which they are present. (*See, e.g.,* Madden, D.R. Annu. Rev. Immunol. 13:587, 1995; Smith, et al., Immunity 4:203, 1996; Fremont et al., Immunity 8:305, 1998; Stern et al., Structure 2:245, 1994; Jones, E.Y. Curr. Opin. Immunol. 9:75, 1997; Brown, J. H. et al., Nature 364:33, 1993; Guo, H. C. et al., Proc. Natl. Acad Sci.-USA 90:8053, 1993; Guo, H. C. et al., Nature 360:364,1992; Silver, M. L. et al., Nature 360:367,1992; Matsumura, M. et al., Science 257:927, 1992; Madden et al., Cell 70:1035, 1992; Fremont, D. H. et al., Science 257:919, 1992; Saper, M. A. , Bjorkman, P. J. and Wiley, D. C., J. Mol. Biol. 219:277, 1991.).

Peptides of the present invention may also comprise epitopes that bind to HLA class II DR molecules. A greater degree of heterogeneity in both size and binding frame position of the motif, relative to the N and C termini of the peptide, exists for class II peptide ligands. This increased heterogeneity of HLA class II peptide ligands is due to the structure of the binding groove of the HLA class II molecule which, unlike its class I counterpart, is open at both ends. Crystallographic analysis of HLA class IIDRB*0101-peptide complexes showed that the major energy of binding is contributed by peptide residues complexed with complementary pockets on the DRB*0101 molecules. An important anchor residue engages the deepest hydrophobic pocket (*see, e.g.,* Madden, D.R. Ann. Rev. Immunol. 13:587, 1995) and is referred to as position 1. (P1). P1 may represent the N-terminal residue of a class II binding peptide epitope, but more typically is flanked towards the N-terminus by one or more residues. Other studies have also pointed to an important role for the peptide residue in the 6^{th} position towards the C-terminus, relative to P1, for binding to various DR molecules.

In the past few years evidence has accumulated to demonstrate that a large fraction of HLA class I and class II molecules can be classified into a relatively few supertypes, each characterized by largely overlapping peptide binding repertoires, and consensus structures of the main peptide binding pockets. Thus, peptides of the present invention are identified by any one of several HLA-specific amino acid motifs, or if the presence of the motif corresponds to the ability to bind several allele-specific HLA molecules, a supermotif. The HLA molecules that bind to peptides that possess a particular amino acid-supermotif are collectively referred to as an HLA "supertype)."

Accordingly, the definition of class I and class II allele-specific HLA binding motifs, or class I or class II supermotifs allows identification of regions within a protein that have the potential of binding particular HLA molecules.

Despite the developments in the art, the prior art has yet to provide a useful human epitope-based vaccine or therapeutic agent based on this work. The present invention provides these and other advantages.

### SUMMARY OF THE INVENTION

The present invention provides peptide analogs derived from the HPV E6 protein, characterized by the properties and amino acid sequences set forth in claim 1. For HLA class I epitopes, which bind to the appropriate HLA Class I allele, peptides typically comprise epitopes from 8-11 amino acids in length, often 9 to 10 residues in length, that comprise conserved residues at certain positions such as positions 2 and the C-terminal position. Moreover, peptides preferably do not comprise negative binding residues as defined herein at other positions such as, in an HLA-A2.1 motif-bearing epitope, positions 1; 3,6 and/or 7 in the case of peptides 9 amino acids in length and positions 1, 3, 4, 5, 7, 8 and/or 9 in the case of peptides 10 amino acids in length. For HLA class II epitopes peptides typically comprise a motif of 6 to about 25 amino acids for a class II HLA motif, typically, 9 to 13 amino acids in length, which is recognized by a particular HLA molecule.

Epitopes on a number of immunogenic target proteins, *i.e*., target antigens, have been identified. Examples of antigens include tumor-associated antigens such as tyrosinase related proteins 1 and 2 (TRP1 and TRP), which are frequently associated with melanoma; MART1, p53, carcinoembryonic antigen (CEA), Her2/neu, and MAGE, including MAGE1, MAGE2, and MAGE3, which are expressed on a broad range of tumors; prostate cancer-associated antigens such as prostate specific antigen (PSA), human kallikrein (huK2), prostate specific membrane antigen (PSM), and prostatic acid phosphatase (PAP); antigens from viruses such as hepatitis B *(e.g.,* HBV core and surface antigens (HBVc, HBVs)) hepatitis C (HCV), Epstein-Barr virus, human immunodeficiency type-1 virus (HIV1), Kaposi's sarcoma herpes virus (KSHV), human papilloma virus (HPV), influenza virus, and Lassa virus antigens, *Mycobacterium tuberculosis* (MT) antigens, trypanosome; *e.g., Trypansoma cruzi* (T. cruzi), antigens such as surface antigen (TSA), and malaria antigens.

### DEFINITIONS

The term "peptide" is used interchangeably with "oligopeptide" in the present specification to designate a series of residues, typically L-amino acids, connected one to the other, typically by peptide bonds between the α-amino and carboxyl groups of adjacent amino acids. The preferred CTL-inducing peptides of the invention are 13 residues or less in length and usually consist of between about 8 and about 11 residues, preferably 9 or 10 residues.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) receptors. In an immune system setting, *in vivo* or *in vitro,* an epitope is the collective features of a molecule, such as primary, secondary and tertiary peptide structure, and charge, that together form a site recognized by an immunoglobulin, T cell receptor or HLA molecule. Throughout this disclosure epitope and peptide are often used interchangeably.

It is to be appreciated that protein or peptide molecules that comprise an epitope of the invention as well as additional amino acid(s) are still within the bounds of the invention. In certain embodiments, there is a limitation on the length of a peptide of the invention. The embodiment that is length-limited occurs when the protein/peptide comprising an epitope of the invention comprises a region (i.e., a contiguous series of amino acids) having 100% identity with a native sequence. In order to avoid the definition of epitope from reading, *e.g*., on whole natural molecules, there is a limitation on the length of any region that has 100% identity with a native peptide sequence. Thus, for a peptide comprising an epitope of the invention and a region with 100% identity with a native peptide sequence, the region with 100% identity to a native sequence generally has a length of: less than or equal to 600 amino acids, often less than or equal to 500 amino acids, often less than or equal to 400 amino acids, often less than or equal to 250 amino acids, often less than or equal to 100 amino acids, , often less than or equal to 85 amino acids, often less than or equal to 75 amino acids, often less than or equal to 65 amino acids, and often less than or equal to 50 amino acids. In certain embodiments, an "epitope" of the invention is comprised by a peptide having a region with less than 51 amino acids that has 100% identity to a native peptide sequence, in any increment down to 5 amino acids.

Accordingly, peptide or protein sequences longer than 600 amino acids are within the scope of the invention, so long as they do not comprise any contiguous sequence of more than 600 amino acids that have 100% identity with a native peptide sequence. For any peptide that has five contiguous residues or less that correspond to a native sequence, there is no limitation on the maximal length of that peptide in order to fall within the scope of the invention. It is presently preferred that a CTL epitope be less than 600 residues long in any increment down to eight amino acid residues.

An "immunogenic peptide" or peptide epitope" is a peptide that comprises an allele-specific motif or supermotif such that the peptide will bind an HLA molecule and induce a CTL. Thus, immunogenic peptides of the invention are capable of binding to an appropriate HLA molecule and thereafter inducing a cytotoxic T cell response to.the antigen from which the immunogenic peptide is derived.

The term "derived" when used to discuss an epitope is a synonym for "prepared." A derived epitope can be isolated from a natural source, or it can be synthesized in accordance with standard protocols in the art. Synthetic epitopes can comprise artificial amino acids "amino acid mimetics," such as D isomers of natural occurring L amino acids or non-natural amino acids such as cyclohexylalanine. A derived/prepared epitope can be an analog of a native epitope.

Immunogenic peptides are conveniently identified using the algorithms of the invention. The algorithms are mathematical procedures that produce a score which enables the selection of immunogenic peptides. Typically one uses the algorithmic score with a "binding threshold" to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

A binding affinity threshold associated with immunogenicity has been determined for HLA Class I. A threshold binding affinity of about 500 nM or less (preferably 50 nM or less) typically determines the capacity of a peptide epitope to elicit a CTL response. A binding affinity threshold of about 1000 nM of less, preferably 100 nM or less, typically determines the capacity of a peptide epitope to elicit an HTL response. As used herein, "high affinity" with respect to HLA class I molecules is defined as binding with an IC₅₀, or K_{D} value, of 50 nM or less; "intermediate affinity" is binding with an IC₅₀ or K_{D} value of between about 50 and about 500 nM. "High affinity" with respect to binding to HLA class II molecules is defined as binding with an IC₅₀ or K_{D} value of 100 nM or less; "intermediate affinity" is binding with an IC₅₀ or K_{D} value of between about 100 and about 1000 nM.

"IC₅₀" is the concentration of peptide in a binding assay at which 50% inhibition of binding of a reference peptide is observed. Given the conditions in which the assays are run (*i*.*e*., limiting HLA proteins and labeled peptide concentrations), these values approximate K_{D} values. Assays for determining binding are described in detail, *e.g*., in PCT publications WO 94/20127 and WO 94/03205. It should be noted that IC₅₀ values can change, often dramatically, if the assay conditions are varied, and depending one the particular reagents used (*e.g.,* HLA preparation, etc.). For example, excessive concentrations of HLA molecules will increase the apparent measured IC₅₀ of a given ligand.

Alternatively, binding is expressed relative to a reference peptide. Although as a particular assay becomes more, or less, sensitive, the Ic₅₀'s of the peptides tested may change somewhat, the binding relative to the reference peptide will not significantly change. For example, in an assay run under conditions such that the IC₅₀ of the reference peptide increases 10-fold, the IC₅₀ values of the test peptides will also shift approximately 10-fold. Therefore, to avoid ambiguities, the assessment of whether a peptide is a good, intermediate, weak, or negative binder is generally based on its IC₅₀, relative to the IC₅₀ of a standard peptide.

Binding may also be determined using other assay systems including those using: live cells (*e.g.,* Ceppellini et al., Nature 339:392 (1989); Christnick et al., Nature 352:67 (1991); Busch et al., Int. Immunol. 2:443 (1990); Hill et al., J. Immunol. 147:189 (1991); del Guercio et al., J. Immunol. 154:685 (1995)), cell free systems using detergent lysates (*e.g.,* Cerundolo et al., J. Immunol. 21:2069 (1991)), immobilized purified MHC (*e.g*., Hill et al., J. Immunol. 152, 2890 (1994); Marshall et al., J. Immunol. 152:4946 (1994)), ELISA systems (*e.g*., Reay et al., EMBO J. 11:2829 (1992)), surface plasmon resonance *(e.g.,* Khilko et al., J. Biol. Chem. 268:15425 (1993)); high flux soluble phase assays (Hammer et al., J. Exp. Med. 180:2353 (1994)), and measurement of class I MHC stabilization or assembly *(e.g.,* Ljunggren et al., Nature 346:476 (1990); Schumacher et al., Cell 62:563 (1990); Townsend et al., Cell 62:285 (1990); Parker et al., J. Immunol. 149:1896 (1992)).

A "conserved residue" is an amino acid which occurs in a significantly higher frequency than would be expected by random distribution at a particular position in a peptide. Typically a conserved residue is one where the MHC structure may provide a contact point with the immunogenic peptide. At least one to three or more, preferably two, conserved residues within a peptide of defined length defines a motif for an immunogenic peptide. These residues are typically in close contact with the peptide binding groove, with their side chains buried in specific pockets of the groove itself.
Typically, an immunogenic peptide will comprise up to three conserved residues, more usually two conserved residues.

As used herein, "negative binding residues" are amino acids which if present at certain positions (for example, positions 1,3 and/or 7 of a 9-mer) will result in a peptide being a nonbinder or poor binder and in turn fail to be immunogenic i.e. induce a CTL response.

The term "motif" refers to the pattern of residues in a peptide of defined length, usually a peptide of from about 8 to about 13 amino acids, often 8 to 11 amino acids, for a class I HLA motif and from about 6 to about 25 amino acids for a class II HLA motif, which is recognized by a particular HLA molecule. Peptide motifs are typically different for each protein encoded by each human HLA allele and differ in the pattern of the primary and secondary anchor residues.

The binding motif for an allele can be defined with increasing degrees of precision. In one case, all of the conserved residues are present in the correct positions in a peptide and there are no negative residues in positions 1,3 and/or 7.

A "supermotif" is a peptide binding specificity shared by HLA molecules encoded by two or more HLA alleles. Preferably, a supermotif-bearing peptide is recognized with high or intermediate affinity (as defined herein) by two or more HLA molecules.

An "HLA supertype or family", as used herein, describes sets of HLA molecules grouped on the basis of shared peptide-binding specificities. HLA class I molecules that share somewhat similar binding affinity for peptides bearing certain amino acid motifs are grouped into HLA supertypes. The terms HLA superfamily, HLA supertype family, HLA family, and HLA xx-like molecules (where xx denotes a particular HLA type), are synonyms.

"Heteroclitic analogs" are defined herein as a peptide with increased potency for a specific T cell, as measured by increased responses to a given dose, or by a requirement of lesser amounts to achieve the same response as a homologous native class I peptide. Advantages of heteroclitic analogs include that the antigens can be more potent, or more economical (since a lower amount is required to achieve the same effect as a homologous class I peptide). In addition, heteroclitic analogs are also useful to overcome antigen-specific T cell unresponsiveness (T cell tolerance).

The phrases "isolated" or "biologically pure" refer to material which is substantially or essentially free from components which normally accompany it as found in its native state. Thus, the peptides of this invention do not contain materials normally associated with their *in situ* environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

"Link" or "join" refers to any method known in the art for functionally connecting peptides, including, without limitation, recombinant fusion, covalent bonding, disulfide bonding, ionic bonding, hydrogen bonding, and electrostatic bonding.

"Pharmaceutically acceptable" refers to a generally non-toxic, inert, and/or physiologically compatible composition.

A "pharmaceutical excipient" comprises a material such as an adjuvant, a carrier, pH-adjusting and buffering agents, tonicity adjusting agents, wetting agents, preservatives, and the like.

"Synthetic peptide" refers to a peptide that is not naturally occurring, but is man-made using such methods as chemical synthesis or recombinant DNA technology.

A "non-native" sequence or "construct" refers to a sequence that is not found in nature, *i.e.,* is "non-naturally occurring". Such sequences include, *e.g*., peptides that are lipidated or otherwise modified, and polyepitopic compositions that contain epitopes that are not contiguous in a native protein sequence.

As used herein, a "vaccine" is a composition that contains one or more peptides of the invention. There are numerous embodiments of vaccines in accordance with the invention, such as by a cocktail of one or more peptides; one or more epitopes of the invention comprised by a polyepitopic peptide; or nucleic acids that encode such peptides or polypeptides, e.g., a minigene that encodes a polyepitopic peptide. The "one or more peptides" can include any whole unit integer from 1-150, e.g., at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 , 45, 50, 55, 60, 65, 70, 75, 80, 85, 90, 95, 100, 105, 110, 115, 120, 125, 130, 135, 140, 145, or 150 or more peptides of the invention. The peptides or polypeptides can optionally be modified, such as by lipidation, addition of targeting or other sequences. HLA class I-binding peptides of the invention can be admixed with, or linked to, HLA class II-binding peptides, to facilitate activation of both cytotoxic T lymphocytes and helper T lymphocytes. Vaccines can also comprise peptide-pulsed antigen presenting cells, *e.g*., dendritic cells.

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino-and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

### DESCRIPTION OF THE PREFERRED EMBODIMENTS

The present invention relates to the determination of allele-specific peptide motifs for human class I and class II allele subtypes. These motifs are then used to define T cell epitopes from any desired antigen, particularly those associated with human viral diseases, cancers or autoiummune diseases, for which the amino acid sequence of the potential antigen or autoantigen targets is known. The application of supermotifs and motifs and binding analysis to the identification of epitopes is described in WO01/21189 and co-pending U.S. applications numbers 09/239,043, filed 1/27/99; 09/350,401, filed 7/8/99; 09/412,863 filed 10/5/99; 09/390,061 filed 9/3/99; 09/458,302 filed 1210/99; 09, 458,297 filed 12 10/99; 09/458,298 filed 12/10/99, 09/633,364 filed 8/7/00; 09/458,299 filed 12 10/99; and 09/641,528 filed 8/15/00.

Epitopes on a number of immunogenic target proteins can be identified . Examples of suitable antigens include tumor-associated antigens such as TRP1, p53, CEA, Her2/neu, and MAGE, including MAGE1, MAGE2, and MAGE3; prostate cancer-associated antigens such as prostate specific antigen (PSA), human kallikrein (huK2), prostate specific membrane antigen (PSM), and prostatic acid phosphatase (PAP); antigens from viruses such as hepatitis B (*e.g*., HBV core and surface antigens (HBVc, HBVs)) hepatitis C, Epstein-Barr virus, human immunodeficiency type-1 virus (HIV1), Kaposi's sarcoma herpes (KSHV), human papilloma virus (HPV), influenza virus, and Lassa virus antigens, *Mycobacterium tuberculosis* (MT) antigens, trypanosome, *e.g., Trypansoma cruzi* (T. cruzi), antigens such as surface antigen (TSA), and malaria antigens. The peptides are thus useful in pharmaceutical compositions for both *in vivo* and *ex vivo* therapeutic and diagnostic applications.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary *in vitro* or *in vivo* CTL responses that can give rise to CTL populations capable of reacting with virally infected target cells or tumor cells as potential therapeutic agents.

The HLA class I antigens are encoded by the HLA-A, B, and C loci. HLA-A and B antigens are expressed at the cell surface at approximately equal densities, whereas the expression of HLA-C is significantly lower (perhaps as much as 10-fold lower): Each of these loci have a number of alleles. The peptide binding motifs are relatively specific for each allelic subtype.

For epitope-based vaccines, the peptides preferably comprise a supermotif and/or motif recognized by an HLA I or HLA II molecule having a wide distribution in the human population.

### Identification of Epitopes

The large degree of HLA polymorphism is an important factor to be taken into account with the epitope-based approach to vaccine development. To address this factor, epitope selection encompassing identification of peptides capable of binding at high or intermediate affinity to multiple HLA molecules is preferably utilized, most preferably these epitopes bind at high or intermediate affinity to two or more allele-specific HLA molecules.

Immunogenic peptides of interest for vaccine compositions preferably include those that have an IC₅₀ or binding affinity value for a class I HLA molecule(s) of 500 nM or better (*i.e.,* the value is ≤ 500 nM) or, for class II HLA molecules, 1000 nM or better (*i.e*., the value is ≤ 1000 nM). For example, peptide binding is assessed by testing the capacity of a candidate peptide to bind to a purified HLA molecule *in vitro.* Peptides exhibiting high or intermediate affinity are then considered for further analysis. Selected peptides are generally tested on other members of the supertype family. In preferred embodiments, peptides that exhibit cross-reactive binding are then used in cellular screening analyses or vaccines.

### Peptide Epitope Binding Motifs and Supermotifs

Through the study of single amino acid substituted antigen analogs and the sequencing of endogenously bound, naturally processed peptides, critical residues required for allele-specific binding to HLA molecules have been identified. The presence of these residues correlates with binding affinity for HLA molecules. The identification of motifs and/or supermotifs that correlate with high and intermediate affinity binding is an important issue with respect to the identification of immunogenic peptide epitopes for the inclusion in a vaccine. Kast et al. (J. Immunol. 152:3904-3912, 1994) have shown that motif-bearing peptides account for 90% of the epitopes that bind to allele-specific HLA class I molecules. In this study all possible peptides of 9 amino acids in length and overlapping by eight amino acids (240 peptides), which cover the entire sequence of the E6 and E7 proteins of human papillomavirus type 16, were evaluated for binding to five allele-specific HLA molecules that are expressed at high frequency among different ethnic groups. This unbiased set of peptides allowed an evaluation of the predictive value of HLA class I motifs. From the set of 240 peptides, 22 peptides were identified that bound to an allele-specific HLA molecule with high or intermediate affinity. Of these 22 peptides, 20 (*i.e*. 91%) were motif-bearing. Thus, this study demonstrates the value of motifs for the identification of peptide epitopes for inclusion in a vaccine: application of motif-based identification techniques will identify about 90% of the potential epitopes in a target antigen protein sequence.

A relationship between binding affinity for HLA class I molecules and immunogenicity of discrete peptide epitopes on bound antigens was determined by the present inventors. As disclosed in greater detail herein, higher HLA binding affinity is correlated with greater immunogenicity.

Greater immunogenicity can be manifested in several different ways. Immunogenicity corresponds to whether an immune response is elicited at all, and to the vigor of any particular response, as well as to the extent of a population in which a response is elicited. For example, a peptide might elicit an immune response in a diverse array of the population, yet in no instance produce a vigorous response. In accordance with these principles, close to 90% of high binding peptides have been found to elicit a response and thus be "immunogenic," as contrasted with about 50% of the peptides that bind with intermediate affinity. (*See, e.g.,* Schaeffer et al. PNAS (1988)) Moreover, not only did peptides with higher binding affinity have an enhanced probability of generating an immune response, the generated response tended to be more vigorous than the response seen with weaker binding peptides. As a result, less peptide is required to elicit a similar biological effect if a high affinity binding peptide is used rather than a lower affinity one. Thus, in preferred embodiments of the invention, high affinity binding epitopes are used.

The correlation between binding affinity and immunogenicity was analyzed by the present inventors by two different experimental approaches (*see, e.g.,* Sette, et al., J. Immunol. 153:5586-5592 (1994)). In the first approach, the immunogenicity of potential epitopes ranging in HLA binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL from acute hepatitis patients. Pursuant to these approaches, it was determined that an affinity threshold value of approximately 500 nM (preferably 50 nM or less) determines the capacity of a peptide epitope to elicit a CTL response. These data are true for class I binding affinity measurements for naturally processed peptides and for synthesized T cell epitopes.

An affinity threshold associated with immunogenicity in the context of HLA class II DR molecules has also been delineated (*see, e.g.*, Southwood et al. J. Immunology 160:3363-3373,1998, and WO99/61916). In order to define a biologically significant threshold of DR binding affinity, a database of the binding affinities of 32 DR-restricted epitopes for their restricting element (*i.e*., the HLA molecule that binds the motif) was compiled. In approximately half of the cases (15 of 32 epitopes), DR restriction was associated with high binding affinities, *i.e.* binding affinity values of 100 nM or less. In the other half of the cases (16 of 32), DR restriction was associated with intermediate affinity (binding affinity values in the 100-1000 nM range). In only one of 32 cases was DR restriction associated with an IC₅₀ of 1000 nM or greater. Thus, 1000 nM can be defined as an affinity threshold associated with immunogenicity in the context of DR molecules.

Definition of motifs that are predictive of binding to specific class I and class II alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs and/or supermotifs.

### HLA Class I Motifs Indicative of CTL Inducing Peptide Epitopes:

The primary anchor residues of the HLA class I peptide epitope supermotifs and motifs are delineated below. In some cases, peptide epitopes may be listed in both a motif and a supermotif Table. The relationship of a particular motif and respective supermotif is indicated in the description of the individual motifs.

The HLA-A1 supermotif is characterized by the presence in peptide ligands of a small (T or S) or hydrophobic (L, I, V, or M) primary anchor residue in position 2, and an aromatic (Y, F, or W) primary anchor residue at the C-terminal position of the epitope. The corresponding family of HLA molecules that bind to the A1 supermotif (*i.e*., the HLA-A1 supertype) is comprised of at least A*0101, A*2601, A*2602, A*2501; and A*3201 (*see, e.g*., DiBrino, M. et al., J. Immunol. 151:5930, 1993; DiBrino, M. et al., J. Immunol. 152:620,1994; Kondo, A. et al., Immunogenetics 45:249,1997). Other allele-specific HLA molecules predicted to be members of the A1 superfamily are shown in Table 1. Peptides binding to each of the individual HLA proteins can be modulated by substitutions at primary and/or secondary anchor positions, preferably choosing respective residues specified for the supermotif.

Primary anchor specificities for allele-specific HLA-A2.1 molecules (*see, e.g*., Falk et al., Nature 351:290-296, 1991; Hunt et al., Science 255:1261-1263, 1992; Parker et al., J. Immunol. 149:3580-3587, 1992; Ruppert et al., Cell 74:929-937, 1993) and cross-reactive binding among HLA-A2 and -A28 molecules have been described. (*See, e.g.,* Fruci et al., Human Immunol. 38:187-192, 1993; Tanigaki et al.; Human Immunol. 39:155-162, 1994; Del Guercio et al., J. Immunol. 154:685-693, 1995; Kast et al., J. Immunol. 152:3904-3912, 1994 for reviews of relevant data.) These primary anchor residues define the HLA-A2 supermotif; which presence in peptide ligands corresponds to the ability to bind several different HLA-A2 and -A28 molecules. The HLA-A2 supermotif comprises peptide ligands with L, I, V, M, A, T, or Q as a primary anchor residue at position 2 and L, I, V, M, A, or T as a primary anchor residue at the C-terminal position of the epitope.

The corresponding family of HLA molecules (*i.e.,* the HLA-A2 supertype that binds these peptides) is comprised of at least: A*0201, A*0202, A*0203, A*0204, A*0205, A*0206, A*0207, A*0209, A*0214, A*6802, and A*6901. Other allele-specific HLA molecules predicted to be members of the A2 superfamily are shown in Table 1. As explained in detail below, binding to each of the individual allele-specific HLA molecules can be modulated by substitutions at the primary anchor and/or secondary anchor positions, preferably choosing respective residues specified for the supermotif.

The HLA-A3 supermotif is characterized by the presence in peptide ligands of A, L, I, V, M, S, or, T as a primary anchor at position 2, and a positively charged residue, R or K, at the C-terminal position of the epitope, *e.g*., in position 9 of 9-mers (*see, e.g.,* Sidney et al., Hum. Immunol. 45:79, 1996). Exemplary members of the corresponding family of HLA molecules (the HLA-A3 supertype) that bind the A3 supermotif include at least A*0301, A*1101, A*3101, A*3301, and A*6801. Other allele-specific HLA molecules predicted to be members of the A3 supertype are shown in Table 1. As explained in detail below, peptide binding to each of the individual allele-specific HLA proteins can be modulated by substitutions of amino acids at the primary and/or secondary anchor positions of the peptide, preferably choosing respective residues specified for the supermotif.

The HLA-A24 supermotif is characterized by the presence in peptide ligands of an aromatic (F, W, or Y) or hydrophobic aliphatic (L, I, V, M, or T) residue as a primary anchor in position 2, and Y, F, W, L, I, or M as primary anchor at the C-terminal position of the epitope (*see, e.g.,* Sette and Sidney, Immunogenetics, in press, 1999). The corresponding family of HLA molecules that bind to the A24 supermotif (*i.e*., the A24 supertype) includes at least A*2402, A*3001, and A*2301. Other allele-specific HLA molecules predicted to be members of the A24 supertype are shown in Table 1. Peptide binding to each of the allele-specific HLA molecules can be modulated by substitutions at primary and/or secondary anchor positions, preferably choosing respective residues specified for the supermotif.

The HLA-B7 supermotif is characterized by peptides bearing proline in position 2 as a primary anchor, and a hydrophobic or aliphatic amino acid (L, I, V, M, A, F, W, or Y) as the primary anchor at the C-terminal position of the epitope. The corresponding family of HLA molecules that bind the B7 supermotif (*i.e*., the HLA-B7 supertype) is comprised of at least twenty six HLA-B proteins including: B*0702, B*0703, B*0704, B*0705, B*1508, B*3501, B*3502, B*3503, B*3504, B*3505, B*3506, B*3507, B*3508, B*5101, B*5102, B*5103, B*5104, B*5105, B*5301, B*5401, B*5501, B*5502, B*5601, B*5602, B*6701, and B*7801 (*see*, *e.g*., Sidney, et al., J. Immunol. 154:247, 1995; Barber, et al., Curr. Biol. 5:179, 1995; Hill, et al., Nature 360:434, 1992; Rammensee, et al., Immunogenetics 41:178, 1995 for reviews of relevant data). Other allele-specific HLA molecules predicted to be members of the B7 supertype are shown in Table 1. As explained in detail below, peptide binding to each of the individual allele-specific HLA proteins can be modulated by substitutions at the primary and/or secondary anchor positions of the peptide, preferably choosing respective residues specified for the supermotif.

The HLA-B27 supermotif is characterized by the presence in peptide ligands of a positively charged (R, H, or K) residue as a primary anchor at position 2, and a hydrophobic (F, Y, L, W, M, I, A, or V) residue as a primary anchor at the C-terminal position of the epitope (*see, e.g.,* Sidney and Sette, Immunogenetics, in press, 1999). Exemplary members of the corresponding family of HLA molecules that bind to the B27 supermotif (*i.e*., the B27 supertype) include at least B*1401, B*1402, B*1509, B*2702, B*2703, B*2704, B*2705, B*2706, B*3801, B*3901, B*3902, and B*7301. Other allele-specific HLA molecules predicted to be members of the B27 supertype are shown in Table 1. Peptide binding to each of the allele-specific HLA molecules can be modulated by substitutions at primary and/or secondary anchor positions, preferably choosing respective residues specified for the supermotif.

The HLA-B44 supermotif is characterized by the presence in peptide ligands of negatively charged (D or E) residues as a primary anchor in position 2, and hydrophobic residues (F, W, Y, L, I, M, V, or A) as a primary anchor at the C-terminal position of the epitope (*see*, *e.g*., Sidney et al., Immunol. Today 17:261, 1996). Exemplary members of the corresponding family of HLA molecules that bind to the B44 supermotif (*i.e*., the B44 supertype) include at least: B*1801, B*1802, B*3701, B*4001, B*4002, B*4006, B*4402, B*4403, and B*4006. Peptide binding to each of the allele-specific HLA molecules can be modulated by substitutions at primary and/or secondary anchor positions; preferably choosing respective residues specified for the supermotif.

The HLA-B58 supermotif is characterized by the presence in peptide ligands of a small aliphatic residue (A, S, or T) as a primary anchor residue at position 2, and an aromatic or hydrophobic residue (P, W, Y, L, I, V, M, or A) as a primary anchor residue at the C-terminal position of the epitope (*see, e.g.,* Sidney and Sette, Immunogenetics, in press, 1999 for reviews of relevant data). Exemplary members of the corresponding family of HLA molecules that bind to the B58 supermotif (*i.e*., the B58 supertype) include at least: B*1516, B*1517, B*5701, B*5702, and B*5801. Other allele-specific HLA molecules predicted to be members of the B58 supertype are shown in Table 1. Peptide binding to each of the allele-specific HLA molecules can be modulated by substitutions at primary and/or secondary anchor positions, preferably choosing respective residues specified for the supermotif.

The HLA-B62 supermotif is characterized by the presence in peptide ligands of the polar aliphatic residue Q or a hydrophobic aliphatic residue (L, V, M, I, or P) as a primary anchor in position 2, and a hydrophobic residue (F, W, Y, M, I, V, L, or A) as a primary anchor at the C-terminal position of the epitope (*see, e.g.,* Sidney and Sette, Immunogenetics, in press, 1999). Exemplary members of the corresponding family of HLA molecules that bind to the B62 supermotif (*i.e*., the B62 supertype) include at least: B*1501, B*1502, B*1513, and B5201. Other allele-specific HLA molecules predicted to be members of the B62 supertype are shown in Table 1. Peptide binding to each of the allele-specific HLA molecules can be modulated by substitutions at primary and/or secondary anchor positions, preferably choosing respective residues specified for the supermotif.

**Table 1**

| **HLA supermotif** | **Allelle-specific HLA-supertype members** | |
|---|---|---|
| | Verified^{a} | Predicted^{b} |
| A1 | A*0101, A*2501, A*2601, A*2602, | A*0102, A*2604, A*3601, A*4301, |
| | A*3201 | A*8001 |
| A2 | A*0201, A*0202, A*0203, A*0204, | A*0208, A*0210, A*0211, A*0212, |
| | A*0205, A*0206, A*0207, A*0209, | A*0213 |
| | A*0214, A*6802, A*6901 | |
| A3 | A*0301, A*1101, A*3101,A*3301, | A*0302, A*1102, A*2603, A*3302, |
| | A*6801 | A*3303, A*3401, A*3402, A*6601, |
| | | A*6602, A*7401 |
| A24 | A*2301, A*2402, A*3001 | A*2403, A*2404, A*3002, A*3003 |
| B7 | B*0702, B*0703, B*0704, B*0705, | B*1511, B*4201, B*5901 |
| | B*1508, B*3501, B*3502, B*3503, | |
| | B*3503, B*3504, B*3505, B*3506, | |
| | B*3507, B*3508, B*5101, B*5102, | |
| | B*5103, B*5104, B*5105, B*5301, | |
| | B*5401, B*5501, B*5502, B*5601, | |
| | B*5602, B*6701, B*7801 | |
| B27 | B*1401, B*1402, B*1509, B*2702, | B*2701, B*2707, B*2708, B*3802, |
| | B*2703, B*2704, B*2705, B*2706, | B*3903, B*3904, B*3905, H*4801, |
| | B*3801, B*3901, B*3902, B*7301 | B*4802, B*1510, B*1518, B*1503 |
| B44 | B*1801, B*1802, B*3701, B*4402, | B*4101, B*4501, B*4701, B*4901, |
| | B*4403, B*4404, B*4001, B*4002, | B*5001 |
| | B*4006 | |
| B58 | B*5701, B*5702, B*5801, B*5802, | |
| | B*1516, B*1517 | |
| B62 | B*1501, B*1502, B*1513, B*5201 | B*1301, B*1302, B*1504, B*1505, |
| | | B*1506, B*1507, B*1515, B*1520, |
| | | B*1521, B*1512, B*1514, B*1510 |

| | | |
|---|---|---|
| a. Verified alleles include alleles whose specificity has been determined by pool sequencing analysis, peptide binding assays, or by analysis of the sequences of CTL epitopes. b. Predicted alleles are alleles whose specificity is predicted on the basis of B and F pocket structure to overlap with the supertype specificity. | | |

The HLA-A1 motif is characterized by the presence in peptide ligands of T, S, or M as a primary anchor residue at position 2 and the presence of Y as a primary anchor residue at the C-terminal position of the epitope. An alternative allele-specific A1 motif is characterized by a primary anchor residue at position 3 rather than position 2. This motif is characterized by the presence of D, E, A, or S as a primary anchor residue in position 3, and a Y as a primary anchor residue at the C-terminal position of the epitope (*see*, *e.g*., DiBrino et al., J. Immunol., 152:620, 1994; Kondo et al., Immunogenetics 45:249, 1997; and Kubo et al., J. Immunol. 152:3913, 1994 for reviews of relevant data). An HLA-A1 extended motif includes a D residue in position 3 and A, I, L, or F at the C-terminus. Peptide binding to HLA A1 can be modulated by substitutions at primary and/or secondary anchor positions, preferably choosing respective residues specified for the motif. Residues T, S, or M at position 2 and Y at the C-terminal position are a subset of the A1 supermotif primary anchors.

An HLA-A2*0201 motif was characterized by the presence in peptide ligands of L or M as a primary anchor residue in position 2, and L or V as a primary anchor residue at the C-terminal position of a 9-residue peptide (*see, e.g.,* Falk et al., Nature 351:290-296, 1991) and was further found to comprise an I at position 2 and I or A at the C-terminal position of a nine amino acid peptide (*see, e.g.,* Hunt et al., Science 255:1261-1263, March 6, 1992; Parker et al., J. Immunol. 149:3580-3587, 1992). The A*0201 allele-specific motif has also been defined to additionally comprise V, A, T, or Q as a primary anchor residue at position 2, and M or T as a primary anchor residue at the C-terminal position of the epitope (*see*, *e.g*., Kast et al., J. Immunol. 152:3904-3912, 1994). Thus, the HLA-A*0201 motif comprises peptide ligands with L, I, V, M, A, T, or Q as primary anchor residues at position 2 and L, I, V, M, A, or T as a primary anchor residue at the C-terminal position of the epitope. The preferred and tolerated residues that characterize the primary anchor positions of the HLA-A*0201 motif are identical to the residues describing the A2 supermotif. (For reviews of relevant data, *see*, *e.g*., Del Guercio et al., J. Immunol. 154:685-693,1995; Ruppert et al., Cell 74:929-937,1993; Sidney et al., Immunol. Today 17:261-266, 1996; Sette and Sidney, Curr. Opin. in Immunol. 10:478-482, 1998). Secondary anchor residues that characterize the A*0201 motif have additionally been defined (*see, e.g.,* Ruppert et al., Cell 74:929-937, 1993). Peptide binding to HLA-A*0201 molecules can be modulated by substitutions at primary and/or secondary anchor positions, preferably choosing respective residues specified for the motif.

The HLA-A3 motif is characterized by the presence in peptide ligands of L, M, V, I, S, A, T, F, C, G, or D as a primary anchor residue at position 2, and the presence of K, Y, R, H, F, or A as a primary anchor residue at the C-terminal position of the epitope (*see, e.g.,* DiBrino et al., Proc. Natl. Acad. Sci USA 90:1508, 1993; and Kubo et al., J. Immunol. 152:3913-3924, 1994). Peptide binding to HLA-A3 can be modulated by substitutions at primary and/or secondary anchor positions, preferably choosing respective residues specified for the motif.

The HLA-A11 motif is characterized by the presence in peptide ligands of V, T, M, L, I, S, A, G, N, C, D, or F. as a primary anchor residue in position 2, and K, R, Y, or H as a primary anchor residue at the C-terminal position of the epitope (*see*, *e.g*., Zhang et al., Proc. Natl. Acad. Sci USA 90:2217-2221, 1993; and Kubo et al., J. Immunol. 152:3913-3924,1994). Peptide binding to HLA-A11 can be modulated by substitutions at primary and/or secondary anchor positions, preferably choosing respective residues specified for the motif.

The HLA-A24 motif is characterized by the presence in peptide ligands of Y, F, W, or M as a primary anchor residue in position 2, and F, L, I, or W as a primary anchor residue at the C-terminal position of the epitope (*see*, *e.g*., Kondo el al., J. Immunol. 155:4307-4312, 1995; and Kubo et al., J. Immunol. 152:3913-3924, 1994). Peptide binding to HLA-A24 molecules can be modulated by substitutions at primary and/or secondary anchor positions; preferably choosing respective residues specified for the motif.

### Motifs Indicative of Class II HTL Inducing Peptide Epitopes

The primary anchor residues of the HLA class II supermotifs and motifs are delineated below.

### HLA DR-1-4-7 supermotif

Motifs have also been identified for peptides that bind to three common HLA class II allele-specific HLA molecules: HLA DRB1*0401, DRB1*0101, and DRB1*0701 (*see, e.g.,* the review by Southwood et al. J. Immunology 160:3363-3373,1998). Collectively, the common residues from these motifs delineate the HLA DR-1-4-7 supermotif. Peptides that bind to these DR molecules carry a supermotif characterized by a large aromatic or hydrophobic residue (Y, F, W, L, I, V, or M) as a primary anchor residue in position 1, and a small, non-charged residue (S, T, C, A, P, V, I, L, or M) as a primary anchor residue in position 6 of a 9-mer core region. Allele-specific secondary effects and secondary anchors for each of these HLA types have also been identified (Southwood *et al*., *supra*). These are set forth in Table III. Peptide binding to HLA-DRB1*0401, DRB1*0101, and/or DRB1*0701 can be modulated by substitutions at primary and/or secondary anchor positions, preferably choosing respective residues specified for the supermotif.

Two alternative motifs (*i.e*., submotifs) characterize peptide epitopes that bind to HLA-DR3 molecules (*see*, *e*.*g*., Geluk et al., J. immunol. 152:5742,1994). In the first motif (submotif DR3A) a large, hydrophobic residue (L, I, V, M, F, or Y) is present in anchor position 1 of a 9-mer core, and D is present as an anchor at position 4, towards the carboxyl terminus of the epitope. As in other class II motifs, core position 1 may or may not occupy the peptide N-terminal position.

The alternative DR3 submotif provides for lack of the large, hydrophobic residue at anchor position 1, and/or lack of the negatively charged or amide-like anchor residue at position 4, by the presence of a positive charge at position 6 towards the carboxyl terminus of the epitope. Thus, for the alternative allele-specific DR3 motif (submotif DR3B): L, I, V, M, F, Y, A, or Y is present at anchor position 1; D, N, Q, E, S, or T is present at anchor position 4; and K, R, or H is present at anchor position 6. Peptide binding to HLA-DR3 can be modulated by substitutions at primary and/or secondary anchor positions, preferably choosing respective residues specified for the motif.

### Evaluation of motif-bearing peptide epitopes

Upon identification of motif-bearing sequences, peptides corresponding to the sequences are then synthesized and, typically, evaluated for binding to the corresponding HLA allele. The capacity to bind MHC Class molecules is measured in a variety of different ways. One means is a Class I molecule binding assay as described in the related applications, noted above. Other alternatives describe in the literature include inhibition of antigen presentation (Sette, et al., J. Immunol. 141:3893 (1991), *in vitro* assembly assays (Townsend, et al., Cell 62:285 (1990), and FACS based assays using mutated ells, such as RMA-S (Melief, et al., Eur. J. Immunol. 21:2963 (1991)).

Peptides that test positive in the binding assay are assayed for the ability of the peptides to induce specific CTL (or HTL, for class II motif-bearing peptides) responses *in vitro*. For instance, antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 (1988)).

Alternatively, mutant mammalian cell lines that are deficient in their ability to load class I molecules, with internally processed peptides, such as the mouse cell lines RMA-S (Kärre, et al., Nature, 319:675 (1986); Ljunggren, et al., Eur. J. Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce *in vitro* primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (*see* Schneider, J. Embryol. Exp. Morphol. 27:353-365 (1927)).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations *in vitro* for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and HLA restriction of the CTL is determined by testing against different peptide target cells expressing appropriate or inappropriate human HLA class I. The peptides that test positive in the HLA binding assays and give rise to specific CTL responses are referred to herein as immunogenic peptide.

After determining their binding affinity, additional confirmatory work can be performed to select, amongst these vaccine candidates, epitopes with preferred characteristics in terms of population coverage, antigenicity, and immunogenicity.

Thus, various strategies can be utilized to evaluate immunogenicity, including:
1) Evaluation of primary T cell cultures from normal individuals (*see, e.g.,* Wentworth, P. A. et al., Mol. Immunol. 32:603,1995; Celis, E. et al., Proc. Natl. Acad. Sci. USA 91:2105, 1994; Tsai, V. et al., J. Immunol. 158:1796,1997; Kawashima, I. et al., Human Immunol. 59:1, 1998); This.procedure involves the stimulation of peripheral blood lymphocytes (PBL) from normal subjects with a test peptide in the presence of antigen presenting cells *in vitro* over a period of several weeks. T cells specific for the peptide become activated during this time and are detected using, *e.g*., a lymphokine-release or a ⁵¹Cr cytotoxicity assay involving peptide sensitized target cells.
2) Immunization of HLA transgenic mice (*see, e.g.,* Wentworth, P. A. et al., J. Immunol. 26:97, 1996; Wentworth, P. A. et al., Int. Immunol. 8:651, 1996; Alexander, J. et al., J. Immunol. 159:4753, 1997); In this method, peptides in incomplete Freund's adjuvant are administered subcutaneously to HLA transgenic mice. Several weeks following immunization, splenocytes are removed and cultured *in vitro* in the presence of test peptide for approximately one week. Peptide-specific T cells are detected using, *e.g*., a ⁵¹Cr-release assay involving peptide sensitized target cells and target cells expressing endogenously generated antigen.
3) Demonstration of recall T cell responses from patients who have been effectively vaccinated or who have a tumor, (*see, e.g.,* Rehermann, B. et al., J. Exp. Med. 181:1047,1995; Doolan, D. L. et al., Immunity 7:97, 1997; Bertoni, R. et al., J. Clin. Invest. 100:503, 1997; Threlkeld, S. C. et al., J. Immunol. 159:1648, 1997; Diepolder, H. M. et al., J. Virol. 71:6011, 1997; Tsang et al., J. Natl. Cancer Inst. 87:982-990,1995; Disis et al., J. Immunol. 156:3151-3158,1996). In applying this strategy, recall responses are detected by culturing PBL from patients with cancer who have generated an immune response "naturally", or from patients who were vaccinated with tumor antigen vaccines. PBL from subjects are cultured *in vitro* for 1-2 weeks in the presence of test peptide plus antigen presenting cells (APC) to allow activation of "memory" T cells, as compared to "naive" T cells. At the end of the culture period, T cell activity is detected using assays for T cell activity including ⁵¹Cr release involving peptide-sensitized targets, T cell proliferation, or lymphokine release.

### Preparation of peptides

Peptides that comprise epitopes of the invention can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Often, the peptide will be as small as possible while still maintaining substantially all of the biological activity of the large peptide. In some embodiments, it may be desirable to optimize peptides of the invention to a length of 8, 9, 10, or 11 amino acid residues, commensurate in size with endogenously processed viral peptides or tumor cell peptides that are bound to MHC class I molecules on the cell surface.

As the coding sequence for peptides of the length contemplated herein can be synthesized by chemical techniques, for example, the phosphotriester method of Matteucci et al., J. Am. Chem. Soc. 103:3185 (1981),

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982), which is incorporated herein by reference. For example, a coding sequence encoding a peptide of the invention can be provided with appropriate linkers and ligated into expression vectors commonly available in the art, and the vectors used to transform suitable hosts to produce the desired fusion protein. A number of such vectors and suitable host systems are now available. Expression constructs, i.e., minigenes are described in greater detail in the sections below.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell.. For instance, the peptides may be subject to various changes, such as substitutions, either conservative or non-conservative, where such changes might provide for certain advantages in their use, such as improved MHC binding. By conservative substitutions is meant replacing an amino acid-residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany & Merrifield, The Peptides, Gross & Meienhofer, eds. (N.Y., Academic Press), pp. 1-284 (1979); and Stewart & Young, Solid Phase Peptide Synthesis, (Rockford, Ill., Pierce), 2d Ed. (1984), incorporated by reference herein.

The peptides can also be modified by extending or decreasing the compound's amino acid sequence, e.g., by the addition or deletion of amino acids. The peptides or analogs can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well as many derivatives of L-α-amino acids.

Typically, a series of peptides with single amino acid substitutions are employed to determine the effect of electrostatic charge, hydrophobicity, etc. on binding. For instance, a series of positively charged (*e.g*., Lys or Arg) or negatively charged (*e.g*., Glu) amino acid substitutions are made along the length of the peptide revealing different patterns of sensitivity towards various MHC molecules and T cell receptors. In addition, multiple substitutions using small; relatively neutral-moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (*e.g*., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place.

Substantial changes in function (*e.g*., affinity for MHC molecules or T cell receptors) are made by selecting substitutions that are less conservative than those in Table 3, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, *e.g*. leucyl, isoleucyl, phenylalanyl, valyl or alanyl; (b) a residue having an electropositive side chain, *e.g*., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, *e.g*., glycine.

The peptides may also comprise isosteres of two or more residues in the immunogenic peptide. An isostere as defined here is a sequence of two or more residues that can be substituted for a second sequence because the steric confirmation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, Peptides and Proteins, Vol. VII (Weinstein ed., 1983).

Modifications of peptides with various amino acid mimetics or unnatural amino acids are particularly useful in increasing the stability of the peptide *in vivo*.
Stability can be assayed in a number of ways. For instance, peptidases and various biological media, such as human plasma and serum, have been used to test stability. *See, e.g*., Verhoef et al., Eur. J. Drug Metab Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

Another embodiment for generating effective peptide analogs involves the substitution of residues that have an adverse impact on peptide stability or solubility in, *e.g*., a liquid environment. This substitution may occur at any position of the peptide epitope. For example, a cysteine (C) can be substituted out in favor of α-amino butyric acid. Due to its chemical nature, cysteine has the propensity to form disulfide bridges and sufficiently alter the peptide structurally so as to reduce binding capacity. Substituting α-amino butyric acid for C not only alleviates this problem, but actually improves binding and crossbinding capability in certain instances (*see*, *e.g*., the review by Sette et al., In: Persistent Viral Infections, Eds. R. Ahmed and I. Chen, John Wiley & Sons, England, 1999). Substitution of cysteine with α-amino butyric acid may occur at any residue of a peptide epitope, *i.e*. at either anchor or non-anchor positions.

### Modification of binding activity

The binding activity, particularly modification of binding affinity or cross-reactivity among HLA supertype family members, of peptides can also be altered using analoging, which is described in co-pending U.S. application number 09/226,775 filed 1/6/99. In brief, the analoging strategy utilizes the motifs or supermotifs that correlate with binding to certain HLA molecules. Analog peptides can be created by substituting amino acid residues at primary anchor, secondary anchor, or at primary and secondary anchor positions. Generally, analogs are made for peptides that already bear a motif or supermotif. For a number of the motifs or supermotifs , residues are defined which are deleterious to binding to allele-specific HLA molecules or members of HLA supertypes that bind the respective motif or supermotif (*see*, e.g., Rupert et al. Cell 74:929, 1993; Sidney, J. et al., Hu. Immunol. 45:79, 1996; and Sidney *et al.;* Sidney, et al., J. Immunol. 154:247,1995). Accordingly, removal of such residues that are detrimental to binding can be performed . For example, in the case of the A3 supertype, when all peptides that have such deleterious residues are removed from the population of peptides used in the analysis, the incidence of cross-reactivity increased from 22% to 37% (*see, e.g.,* Sidney, J. et al., Hu. Immunol. 45:79, 1996).

Thus, one strategy to improve the cross-reactivity of peptides within a given supermotif is simply to delete one or more of the deleterious residues present within a peptide and substitute a small "neutral" residue such as Ala (that may not influence T cell recognition of the peptide). An enhanced likelihood of cross-reactivity is expected if, together with elimination of detrimental residues within a peptide, "preferred" residues associated with high affinity binding to an allele-specific HLA molecule or to multiple HLA molecules within a superfamily are inserted.

To ensure that an analog peptide, when used as a vaccine, actually elicits a CTL response to the native epitope *in vivo,* the analog peptide may be used to induce T cells *in vitro* from individuals of the appropriate HLA allele. Thereafter, the immunized cells' capacity to lyse wild type peptide sensitized target cells is evaluated. Alternatively, evaluation of the cells' activity can be evaluated by monitoring IFN release. Each of these cell monitoring strategies evaluate the recognition of the APC by the CTL. It will be desirable to use as antigen presenting cells, typically cells that have been either infected, or transfected with the appropriate genes to establish whether endogenously produced antigen is also recognized by the T cells induced by the analog peptide. It is to be noted that peptide/protein-pulsed dendritic cells can be used to present whole protein antigens for both HLA class I and class II.

A analogs of weak binding peptides can be created, to thereby ensure adequate numbers of cellular binders. Class I binding peptides exhibiting binding affinities of 500-5000 nM, and carrying an acceptable but suboptimal primary anchor residue at one or both positions can be "fixed" by substituting preferred anchor residues in accordance with the respective supertype. The analog peptides can then be tested for binding and/or cross-binding capacity.

Analogs of peptides that are already cross-reactive binders and are vaccine candidates, but which bind weakly to one or more alleles of a supertype can also be created. If the cross-reactive binder carries a suboptimal residue (less preferred or deleterious) at a primary or secondary anchor position, the peptide can be analoged by substituting out a deleterious residue and replacing it with a preferred or less preferred one, or by substituting out a less preferred residue and replacing it with a preferred one. The analog peptide can then be tested for cross-binding capacity.

### Heteroclitic analogs

Heteroclitic analog peptides are particularly useful to induce an immune response against antigens to which a patient's immune system has become tolerant. Tolerance refers to a specific immunologic nonresponsiveness induced by prior exposure to an antigen. Thus, tolerance can be overcome in the patient by identifying a particular class I peptide epitope to which a patient is tolerant, modifying the peptide epitope sequence according to the methods described herein, and inducing an immune response that cross-reacts against the tolerized epitope (antigen). Overcoming tolerance is particularly desirable, for example, when a patient's immune system is tolerant of a viral or tumor-associated antigen, the latter antigens being often over-expressed self-proteins as a consequence of cell transformation. Heteroclitic analoging is described in co-pending US provisional application number 60/166,529 filed 11/18/99 and US provisional application for "Heteroclitic Analogs And Related Methods", Tangri *et al*., inventors, Attorney Docket number 018623-015810US, filed 10/6/00.

### Combinations of CTL and HTL epitopes

The peptides or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, *e.g*., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helper peptides include tetanus toxoid 830-843, influenza 307-319, malaria circumsporozoite 382-398 and 378-389.

### Combination with agents to prime the immune response

In some embodiments it may be desirable to include in the pharmaceutical compositions of the invention at least one component which assists in priming CTL.

Lipids have been identified as agents capable of assisting the priming CTL *in vivo* against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL, response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH2 acylation, e.g., by alkanoyl (C1-C20) or thioglycolyl acetylation, terminal-carboxyl amidation, e.g., ammonia, methylamine, etc. In some instances these modifications may provide sites for linking to a support or other molecule.

### Vaccine compositions

The peptides of the present invention and pharmaceutical and vaccine compositions thereof are useful for administration to mammals, particularly humans, to treat and/or prevent viral infection and cancer. Examples of diseases which can be treated or prevented using the immunogenic peptides of the invention include prostate cancer, hepatitis B, hepatitis C, HPV infection, AIDS, renal carcinoma, cervical carcinoma, lymphoma, CMV, malaria, and condlyloma acuminatum.

Vaccines that contain an immunogenically effective amount of one or more peptides as described herein are a further embodiment of the invention. Once appropriately immunogenic epitopes have been defined, they can be delivered by various means, herein referred to as "vaccine" compositions. Such vaccine compositions can include, for example, lipopeptides (*e.g*., Vitiello, A. et al., J. Clin. Invest. 95:341, 1995), peptide compositions encapsulated in poly(DL-lactide-co-glycolide) ("PLG") microspheres (*see*, *e.g*., Eldridge, et al., Molec. Immunol. 28:287-294, 1991. Alonso et al., Vaccine 12:299-306, 1994; Jones et al., Vaccine 13:675-681, 1995), peptide compositions contained in immune stimulating complexes (ISCOMS) (*see, e.g.,* Takahashi et al., Nature 344:873-875, 1990; Hu et al., Clin Exp Immunol. 113:235-243, 1998), multiple antigen peptide systems (MAPs) (*see e.g.,* Tam, J. P., Proc. Natl. Acad. Sci. U.S.A. 85:5409-5413, 1988; Tam, J.P., J. Immunol. Methods 196:17-32, 1996), viral delivery vectors (Perkus, M. E. et al., In: Concepts in vaccine development, Kaufmann, S. H. E., ed., p. 379, 1996; Chakrabarti, S. et al., Nature 320:535, 1986; Hu, S. L. et al., Nature 320:537, 1986; Kieny, M.-P. et al., AIDS BiolTechnology 4:790,1986; Top, F. H. et al., J. Infect. Dis. 124:148, 1971; Chanda, P.K. et al., Virology 175:535, 1990), particles of viral or synthetic origin (*e.g.,* Kofler, N. et al., J. Immunol. Methods. 192:25, 1996; Eldridge, J. H. et al., Sem. Hematol. 30:16, 1993; Falo, L. D., Jr. et al., Nature Med. 7:649,1995), adjuvants (Warren, H. S., Vogel, F. R., and Chedid, L. A. Annu. Rev. Immunol. 4:369, 1986; Gupta, R. K. et al., Vaccine 11:293, 1993), liposomes (Reddy, R. et al., J. Immunol. 148:1585, 1992; Rock, K. L., Immunol. Today 17:131, 1996), or, naked or particle absorbed cDNA (Ulmer, J. B. et al., Science 259:1745, 1993; Robinson, H. L., Hunt, L. A., and Webster, R. G., Vaccine 11:957, 1993; Shiver, J. W. et al., In: Concepts in vaccine development, Kaufmann, S. H. E., ed., p. 423, 1996; Cease, K. B., and Berzofsky, J. A, Annu. Rev. Immunol. 12:923, 1994 and Eldridge, J. H. et al., Sem. Hematol. 30:16, 1993). Toxin-targeted delivery technologies, also known as receptor mediated targeting, such as those of Avant Immunotherapeutics, Inc. (Needham, Massachusetts) may also be used.

Vaccine compositions of the invention include nucleic acid-mediated modalities. DNA or RNA encoding one or more of the peptides of the invention can also be administered to a patient. This approach is described, for instance, in Wolff et. al., Science 247:1465 (1990) as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") or pressure-mediated delivery (*see, e.g.,* U.S. Patent No. 5,922,687).

For therapeutic or prophylactic immunization purposes, the peptides of the invention can be expressed by viral or bacterial vectors. Examples of expression vectors include attenuated viral hosts, such as vaccinia or fowlpox. This approach involves the use of vaccinia virus, for example, as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL and/or HTL response. Vaccinia vectors and methods useful in immunization protocols are described in, *e.g.,* U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the peptides of the invention, *e.g*. adeno and adeno-associated virus vectors, retroviral vectors, *Salmonella typhi* vectors, detoxified anthrax toxin vectors, and the like, will be apparent to those skilled in the art from the description herein.

Furthermore, vaccines in accordance with the invention can encompass one or more of the peptides of the invention. Accordingly, a peptide can be present in a vaccine individually. Alternatively, the peptide can be individually linked to its own carrier, alternatively, the peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism or tumor-related peptide targeted for an immune response. The composition may be a naturally occurring region of an antigen or may be prepared, *e.g*., recombinantly or by chemical synthesis.

Carriers that can be used with vaccines of the invention are well known in the art, and include, *e.g*., thymglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (i.e., acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, CTL responses can be primed by conjugating peptides of the invention to lipids, such as tripalmitoyl-S-glycerylcysteinlyseryl- serine (P₃CSS).

Upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs specific for the desired antigen. Consequently, the host becomes at least partially immune to later infection, or at least partially resistant to developing an ongoing chronic infection, or derives at least some therapeutic benefit when the antigen was tumor-associated.

In certain embodiments, components that induce T cell responses are combined with component that induce antibody responses to the target antigen of interest, combine class I peptide vaccines of the invention with vaccines which induce or facilitate neutralizing antibody responses to the target antigen of interest, particularly to viral envelope antigens. A preferred embodiment of such a composition comprises class I and class II epitopes in accordance with the invention. An alternative embodiment of such a composition comprises a class I epitope in accordance with the invention, along with a pan DR binding molecules, *e.g*., PADRE™ (Epimmune, San Diego, CA) (described, for example, in U.S. Patent Number 5,736,142).

### Minigenes

A preferred means of administering nucleic acids encoding the peptides of the invention uses minigene constructs encoding multiple epitopes of the invention. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. The ends of the oligonucleotides are joined using T4 DNA ligase. This synthetic minigene, encoding the CTL epitope polypeptide, can then be cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E*. *coli* origin of replication; and an *E. coli* selectable marker (*e.g*. ampicillin or kanamycin resistance). Numerous promoters can be used for this purpose, *e.g*., the human cytomegalovirus (hCMV) promoter. See, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used Examples of proteins or polypeptides that could beneficially enhance the immune response if co-expressed include cytokines (*e.g*., IL2, IL12, GM-CSF), cytokine-inducing molecules (*e.g*. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (*e.g*. TGF-β) may be beneficial in certain diseases.

Once an expression vector is selected, the minigene is cloned into the polylinker region downstream of the promoter. This plasmid is transformed into an appropriate E. coli strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in E. coli, followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by ⁵¹Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (*e.g*. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

### Ex vivo administration of epitopes

An embodiment of a vaccine composition in accordance with the invention comprises *ex vivo* administration of a cocktail of epitope-bearing peptides to PBMC, or isolated DC therefrom, from the patient's blood. After pulsing the DC with peptides and prior to reinfusion into patients, the DC are washed to remove unbound peptides. In this embodiment, a vaccine comprises peptide-pulsed DCs which present the pulsed peptide epitopes in HLA molecules on their surfaces.

Dendritic cells can also be transfected, *e.g*., with a minigene comprising nucleic acid sequences encoding the epitopes in accordance with the invention, in order to elicit immune responses. Vaccine compositions can be created *in vitro,* following dendritic cell mobilization and harvesting, whereby loading of dendritic cells occurs *in vitro.*

Antigenic peptides are used to elicit a CTL response ex *vivo,* as well. The resulting CTL cells, can be used to treat chronic infections, or tumors in patients that do not respond to other conventional forms of therapy, or will not respond to a therapeutic vaccine peptide or nucleic acid in accordance with the invention. *Ex vivo* CTL or HTL responses to a particular antigen (infectious or tumor-associated antigen) are induced by incubating in tissue culture the patient's, or genetically compatible, CTL or HTL precursor cells together with a source of antigen-presenting cells (APC), such as dendritic cells, and the appropriate immunogenic peptide. After an appropriate incubation time (typically about 7-28 days), in which the precursor cells are activated and expanded into effector cells, the cells are infused back into the patient, where they will destroy their specific targets cell (an infected cell or a tumor cell). Transfected dendritic cells may also be used as antigen presenting cells.

### Administration of vaccine compositions

For pharmaceutical compositions, the immunogenic peptides of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus and to cure or at least partially arrest symptoms and/or complications. An amount adequate to accomplish this is defined as "therapeutically effective dose." Amounts effective for this use will depend on, *e.g*., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 50,000 µg of peptide for a 70 kg patient, followed by boosting dosages of from about 1.0 µg to about 10,000 µg of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 50,000 µg, preferably about 5 µg to 10,000 µg for a 70 kg patient per dose. Immunizing doses followed by boosting doses at established intervals, *e.g*., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, *e.g*., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, *e.g*., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides of the invention may also be administered via liposomes, which target the peptides to a particular cells tissue, such as lymphoid tissue. Liposomes are also useful in increasing the half-life of the peptides. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, *e.g.*, a receptor prevalent among lymphoid cells, such as monoclonal antibodies which bind to the CD45 antigen, or with other therapeutic or immunogenic compositions. Thus, liposomes filled with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellent. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

In some instances it may be desirable to combine the peptide vaccines of the invention with vaccines which induce neutralizing antibody responses to the virus of interest, particularly to viral envelope antigens.

Antigenic peptides may be used to elicit CTL *ex vivo,* as well. The resulting CTL, can be used to treat chronic infections (viral or bacterial) or tumors in patients that do not respond to other conventional forms of therapy, or will not respond to a peptide vaccine approach of therapy. *Ex vivo* CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell). In order to optimize the *in vitro* conditions for the generation of specific cytotoxic T cells, the culture of stimulator cells is maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that will allow about 200, and preferably 200 or more, human Class I MHC molecules loaded with peptide to be expressed on the surface of each stimulator cell. Often, the stimulator cells are incubated with >20 µg/ml peptide.

Resting or precursor CD8+ cells are then incubated in culture with the appropriate stimulator cells for a time period sufficient to activate the CD8+ cells. Preferably, the CD8+ cells are activated in an antigen-specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the within-described treatment modality is used. Preferably, however, the lymphocyte:stimulator cell ratio is in the range of about 30:1 to 300:1. The effector/stimulator culture may be maintained for as long a time as is necessary to stimulate a therapeutically useable or effective number of CD8+ cells.

The induction of CTL *in vitro* requires the specific recognition of peptides that are bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses. Peptide loading of empty major histocompatability complex molecules on cells enables the induction of primary cytotoxic T lymphocyte responses.

Since mutant cell lines do not exist for every human MHC allele, it is advantageous to use a technique to remove endogenous MHC-associated peptides from the surface of APC, followed by loading the resulting empty MHC molecules with the immunogenic peptides of interest. The use of non-transformed (non-tumorigenio), non-infected cells, and preferably, autologous cells of patients as APC is desirable for the design of CTL induction protocols directed towards development of *ex vivo* CTL therapies. This application discloses methods for stripping the endogenous MHC-associated peptides from the surface of APC followed by the loading of desired peptides.

A stable MHC class I molecule is a trimeric complex formed of the following elements: 1) a peptide usually of 8-10 residues, 2) a transmembrane heavy polymorphic protein chain which bears the peptide-binding site, and 3) a non-covalently associated non-polymorphic light chain, β2 microglobulin. Removing the bound peptides and/or dissociating the β2 microglobulin from the complex renders the MHC class I molecules nonfunctional and unstable, resulting in rapid degradation. All MHC class I molecules isolated from peripheral blood monocytic cells (PBMC) have endogenous peptides bound to them. Therefore, the first step is to remove all endogenous peptides bound to MHC class I molecules on the APC without causing their degradation before exogenous peptides can be added to them.

Two possible ways to free up MHC class I molecules of bound peptides include lowering the culture temperature from 37°C to 26°C overnight to destablize β2microglobulin and stripping the endogenous peptides from the cell using a mild acid treatment. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells hot actively synthesizing MHC molecules (*e.g*., resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction; since it is important to remove the endogenous peptides while preserving APC viability and an optimal metabolic state which is critical for antigen presentation. Mild acid solutions of pH 3 such as glycine or citrate-phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen-specific CTL. The resulting APC are efficient in inducing peptide-specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibody-tagged molecules may then be extracted from the stimulator-effector cell admixture via appropriate means, e.g., via well-known immunoprecipitation or immunoassay methods.

Effective, cytotoxic amounts of the activated CD8+ cells can vary between *in vitro* and *in vivo* uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Preferably, as discussed above, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells is not achieved, there is no inherent danger known to be associated with the administration of a small number of stimulator cells, whereas administration, of mammalian tumor-promoting cells may be extremely hazardous.

Methods of re-introducing cellular components are known in the art and included procedures such as those exemplified in U.S. Patent No: 4,844;893 to Hosisk, et al. and U.S. Patent No. 4,690,915 to Rosenberg. For example, administration of activated CD8+ cells via intravenous infusion is appropriate.

### Use of Peptide Epitopes as Diagnostic Agents for Evaluating Immune Responses

HLA class I and class II binding peptides can be used as reagents to evaluate an immune response. The evaluated immune response can be induced by any immunogen. For example, the immunogen may result in the production of antigen-specific CTLs or HTLs that recognize the peptide epitope(s) employed as the reagent. Thus, a peptide of the invention may or may not be used as the immunogen. Assay systems that can be used for such analyses include tetramer-based protocols, staining for intracellular lymphokines, interferon release assays, or ELISPOT assays.

For example, following exposure to a putative immunogen, a peptide of the invention can be used in a tetramer staining assay to assess peripheral blood mononuclear cells for the presence of any antigen-specific CTLs. The HLA-tetrameric complex is used to directly visualize antigen-specific CTLs and thereby determine the frequency of such antigen-specific CTLs in a sample of peripheral blood mononuclear cells (*see, e.g.,* Ogg et al., Science 279:2103-2106, 1998; and Altman et al., Science 174:94-96,1996).

A tetramer reagent comprising a peptide of the invention is generated as follows: A peptide that binds to an HLA molecule is refolded in the presence of the corresponding HLA heavy chain and β₂-microglobulin to generate a trimolecular complex. The complex is biotinylated at the carboxyl terminal end of the HLA heavy chain, at a site that was previously engineered into the protein. Tetramer formation is then induced by adding streptavidin. When fluorescently labeled streptavidin is used, the tetrameric complex is used to stain antigen-specific cells. The labeled cells are then readily identified, *e.g.*, by flow cytometry. Such procedures are used for diagnostic or prognostic purposes; the cells identified by the procedure can be used for therapeutic purposes.

Peptides of the invention are also used as reagents to evaluate immune recall responses. (*see, e.g.*, Bertoni et al.; J. Clin. Invest. 100:503-513,1997 and Penna et al., J. Exp. Med. 174:1565-1570,1991.) For example, a PBMC sample from an individual expressing a disease-associated antigen (e.g. a tumor-associated antigen such as CEA, p53, MAGE2/3,HER2neu, or an organism associated with neoplasia such as HPV or HSV) can be analyzed for the presence of antigen-specific CTLs or HTLs using specific peptides. A blood sample containing mononuclear cells may be evaluated by cultivating the PBMCs and stimulating the cells with a peptide of the invention. After an appropriate cultivation period, the expanded cell population may be analyzed, for example, for CTL or for HTL activity.

Thus, the peptides can be used to evaluate the efficacy of a vaccine. PBMCs obtained from a patient vaccinated with an immunogen may be analyzed by methods such as those described herein. The patient is HLA typed, and peptide epitopes that are bound by the HLA molecule(s) present in that patient are selected for analysis. The immunogenicity of the vaccine is indicated by the presence of CTLs and/or HTLs directed to epitopes present in the vaccine. o

The peptides of the invention may also be used to make antibodies, using techniques well known in the art (see, *e.g.* CURRENT PROTOCOLS IN IMMUNOLOGY, Wiley/Greene, NY; and Antibodies A Laboratory Manual Harlow, Harlow and Lane, Cold Spring Harbor Laboratory Press,1989). Such antibodies are useful as reagents to determine the presence of disease-associated antigens or may be used therapetucially. Antibodies in this category include those that recognize a peptide when bound by an HLA molecule, *i.e.*, antibodies that bind to a peptide-MHC complex.

Epitopes in accordance with the present invention were successfully used to induce an immune response. Immune responses with these epitopes have been induced by administering the epitopes in various forms. The epitopes have been administered as peptides, as nucleic acids, and as viral vectors comprising nucleic acids that encode the epitope(s) of the invention. Upon administration of peptide-based epitope forms, immune responses have been induced by direct loading of an epitope onto an empty HLA molecule that is expressed on a cell, and via internalization of the epitope and processing via the HLA class I pathway; in either event, the HLA molecule expressing the epitope was then able to interact with and induce a CTL response. Peptides can be delivered directly or using such agents as liposomes. They can additionally be delivered using ballistic delivery, in which the peptides are typically in a crystalline form. When DNA is used to induce an immune response, it is administered either as naked DNA, generally in a dose range of approximately 1-5 mg, or via the ballistic "gene gun" delivery, typically in a dose range of approximately 10-100 µg. The DNA can be delivered in a variety of conformations, e.g., linear, circular etc. Various viral vectors have also successfully been used that comprise nucleic acids which encode epitopes in accordance with the invention.

Accordingly compositions in accordance with the invention exist in several forms. Embodiments of each of these composition forms in accordance with the invention have been successfully used to induce an immune response.

One composition in accordance with the invention comprises a plurality of peptides. This plurality or cocktail of peptides is generally admixed with one or more pharmaceutically acceptable excipients. The peptide cocktail can comprise multiple copies of the same peptide or can comprise a mixture of peptides. The peptides can be analogs of naturally occurring epitopes. The peptides can comprise artificial amino acids and/or chemical modifications such as addition of a surface active molecule, e.g., lipidation; acetylation, glycosylation, biotinylation, phosphorylation etc. In a preferred embodiment the peptide cocktail comprises a plurality of different CTL epitopes and at least one HTL epitope. The HTL epitope can be naturally or non-naturally (*e.g.*, PADRE®, Epimmune Inc., San Diego, CA). The number of distinct epitopes in an embodiment of the invention is generally a whole unit integer from one through one hundred fifty (*e.g.,* 1, 2, 3, 4, 5, 6, 7, 8, 9, 10, 11, 12, 13, 14, 15, 16, 17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or, 100).

An additional embodiment of a composition in accordance with the invention comprises a polypeptide multi-epitope construct, i.e., a polyepitopic peptide. Polyepitopic peptides in accordance with the invention are prepared by use of technologies well-known in the art. By use of these known technologies, epitopes in accordance with the invention are connected one to another. The polyepitopic peptides can be linear or non-linear, *e.g*., multivalent. These polyepitopic constructs can comprise artificial amino acids, spacing or spacer amino acids, flanking amino acids, or chemical modifications between adjacent epitope units. The polyepitopic construct can be a heteropolymer or a homopolymer. The polyepitopic constructs generally comprise epitopes in a quantity of any whole unit integer between 2-150 (*e.g*., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 ,35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or, 100). One or more of the epitopes in the construct can be modified, e.g., by addition of a surface active material, e.g. a lipid, or chemically modified, e.g., acetylation, etc. Moreover, bonds in the multiepitopic construct can be other than peptide bonds, *e.g*., covalent bonds, ester or ether bonds, disulfide bonds, hydrogen bonds, ionic bonds etc.

Alternatively, a composition in accordance with the invention comprises construct which comprises a series, sequence, stretch, etc., of amino acids that have homology to ( i.e., corresponds to or is contiguous with) to a native sequence. This stretch of amino acids comprises at least one subsequence of amino acids that, if cleaved or isolated from the longer series of amino acids, functions as an HLA class I epitope in accordance with the invention. In this embodiment, the peptide sequence is modified, so as to become a construct as defined herein, by use of any number of techniques known or to be provided in the art The polyepitopic constructs can contain homology to a native sequence in any whole unit integer increment from 70-100%, *e.g.*, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or, 100 percent.

A further embodiment of a composition in accordance with the invention is an antigen presenting cell that comprises one or more epitopes in accordance with the invention. The antigen presenting cell can be a "professional" antigen presenting cell, such as a dendritic cell. The antigen presenting cell can comprise the epitope of the invention by any means known or to be determined in the art. Such means include pulsing of dendritic cells with one or more individual epitopes or with one or more peptides that comprise multiple epitopes, by nucleic acid administration such as ballistic nucleic acid delivery or by other techniques in the art for administration of nucleic acids, including vector-based, e.g: viral vector, delivery of nucleic acids.

Further embodiments of compositions in accordance with the invention comprise nucleic acids that encode one or more peptides of the invention, or nucleic acids which encode a polyepitopic peptide in accordance with the invention. As appreciated by one of ordinary skill in the art, various nucleic acids compositions will encode the same peptide due to the redundancy of the genetic code. Each of these nucleic acid compositions falls within the scope of the present invention. This embodiment of the invention comprises DNA or RNA, and in certain embodiments a combination of DNA and RNA. It is to be appreciated that any composition comprising nucleic acids that will encode a peptide in accordance with the invention or any other peptide based composition in accordance with the invention, falls within the scope of this invention.

It is to be appreciated that peptide-based forms of the invention (as well as the nucleic acids that encode them) comprise analogs of epitopes generated using priniciples already known, or to be known, in the art. Principles related to analoging are now known in the art, and are disclosed herein; moreover, analoging principles are disclosed in co-pending application serial number U.S.S.N. 09/226,775 filed 6 January 1999. Generally the compositions of the invention are isolated or purified.

Although the foregoing invention has been described in some detail by way of illustration and example for purposes of clarity of understanding, it will be readily ... apparent to one of ordinary skill in the art in light of the teachings of this invention that certain changes and modifications may be made thereto without departing from the spirit or scope of the appended claims.

### EXAMPLES

The following examples are provided by way of illustration only and not by way of limitation. Those of skill in the art will readily recognize a variety of noncritical parameters that could be changed or modified to yield essentially similar results.

### Example 1

HLA class I supermotif and motif analysis of antigens of interest was performed as described herein and in the related applications, noted above. Peptides comprising the appropriate HLA I motif or supermotif were then synthesized and assayed for binding activity. A detailed description of the protocol utilized to measure the binding of peptides to Class I and Class II MHC has been published (Sette et al., Mol. Immunol 31:813, 1994; Sidney et al., in Current Protocols in Immunology, Margulies, Ed., John Wiley & Sons, New York, Section 18.3, 1998).

Since under these conditions [label]<[HLA] and IC₅₀≥[HLA], the measured IC₅₀ values are reasonable approximations of the true K_{D} values. To allow comparison of the data obtained in different experiments, a relative binding figure is calculated for each peptide by dividing the IC₅₀ of a positive control for inhibition by the IC₅₀ for each tested peptide (typically unlabeled versions of the radiolabeled probe peptide). For inter-experiment comparisons, relative binding values are compiled. These values can subsequently be converted back into IC₅₀ nM values by dividing the IC₅₀ nM of the positive controls for inhibition by the relative binding of the peptide of interest. This method of data compilation has proven to be the most accurate and consistent for comparing peptides that have been tested on different days, or with different lots of purified MHC.

HLA class I supermotif and motif-bearing peptides from HIV regulatory proteins, *e.g.,* nef, rev, vif, tat, and vpr, are shown in Tables 2-11. In these tables, "% conserv" refers to percent conservance, which is the degree to which the sequences are conserved in the strains evaluated to identify the sequences. The "A" designation indicates that the peptide is an analog of the native sequence. In the motif column, the designation "i" refers to individual motif and "s" refers to supermotif.

HLA class I supermotif and motif-bearing peptides from other antigens, *e.g*., cancer antigens such as CEA, p53, Her2/neu, MART1, MAGE2, MAGE3, tyrosinase, flu, gp100, HBV, HCV, HIV, HPV (including the strain designation), Epstein Barr Virus (EBV), prostate-cancer-associated antigens, gliadin, *Mycobacterium leprae, Mycobacterium tuberculosis, T. cruzi, Candida* antigens, and malaria (*Plasmodium falciparum*) antigens are shown in Tables 12-24.

Tables 12 and 13 show peptides bearing an HLA-A1 supermotif and/or motif.

Tables 14-17 shows peptides bearing an HLA-A2 supermotif.

Tables 18 and 19 show peptides bearing an HLA-A3 supermotif and/or motif.

Tables 20 and 21 show peptides bearing an HLA-A24 supermotif and/or motif.

Tables 22 and 23 show peptides bearing an HLA-B7 supermotiff.

Table 24 shows peptides bearing an HLA-B44 supermotif.

Peptide binding data for the designated HLA molecules are provided as IC₅₀ values unless otherwise indicated. The "A" designation indicates that the peptide is an analog of the native sequence.

### Examples 2

Using the HLA class II supermotif and motifs identified in related applications and as described above, sequences from various pathogens and tumor-related proteins were analyzed for the presence of these motifs. Screening and binding assays was carried out as described in the related applications designated herein.

HLA class II DR supermotif and DR3 motif-bearing peptides from HIV regulatory proteins, *e.g*., nef, rev, vif, tat, and vpr, are shown in Tables 25 and 31. The term "% conserv" refers to percent conservance, which is the degree to which the sequences are conserved in the strains evaluated to identify the sequences. In Table 31, in the "sequence" column, the core sequence of the motif-bearing peptide is in lower case.

Tables 26-31 shows HLA class II DR supermotif and DR 3 motif bearing peptides and the antigens from which they are derived. The peptide reference number, sequence, antigen protein/position of the sequence in the antigen, and binding data are shown in the tables. Table 27a shows binding data for DRB1*0101, *0301, *0401, *0404, and *0405. Table 27b shows binding data for DRB1*0701, *0802, *0901, *1101, *1201, *1302, *1501, DRB3*0101, DRB4*0101, DRB5*0101, and DQB1*0301. Table 28a and 29a provide the peptide reference number sequence and protein antigen/position of sequence in antigen for the peptides. Binding data are provided in Tables 28b and 29B.

Peptide binding data for the designated HLA molecules are provided as IC₅₀ values unless otherwise indicated. The "A" designation indicates that the peptide is an analog of the native sequence.

**TABLE 2**

| Motif | Sequence | Source | Analog | % Consv. |
|---|---|---|---|---|
| A01i | ARELHPEY | HIV.nef.322 | | 38 |
| A01i | ATELHPEY | HIV.nef.322 | A | 38 |
| A01i | ARDLHPEY | HIV.nef.322 | A | 38 |
| A01i | ARELHPEYY | HIV.nef.322 | | 33 |
| A01i | ATELHPEYY | HIV.nef.322 | A | 33 |
| A01i | ARDLHPEYY | HIV.nef.322 | A | 33 |
| A01s | DILDLWVY | HIV.nef.185 | | 31 |
| A01s | DTLDLWVY | HIV.nef.185 | A | 31 |
| A01s | DLWVYHTQGY | HIV.nef.188 | | 33 |
| A01s | DTWVYHTQGY | HIV.nef.188 | A | 33 |
| A01s | DLWVYHTQGYF | HIV.nef.188 | | 33 |
| A01s | DTWVYHTQGYF | HIV.nef.188 | A | 33 |
| A01s | DLWVYHTQGYY | HIV.nef.188 | A | 33 |
| A01s | EILDLWVY | HIV.nef.185 | | 52 |
| A01s | ETLDLWVY | HIV.nef.185 | A | 52 |
| A01s | PLTFGWCF | HIV.nef.219 | | 67 |
| A01s | PTTFGWCF | HIV.nef.219 | A | 67 |
| A01s | PLTFGWCY | HIV.nef.219 | A | 67 |
| A01s | QVPLRPMTY | HIV.nef.100 | | 72 |
| A01s | QTPLRPMTY | HIV.nef.100 | A | 72 |
| A01i | RQEILDLWVY | HIV.nef.182 | | 50 |
| A01i | RTEILDLWVY | HIV.nef.182 | A | 50 |
| A01i | RQDILDLWVY | HIV.nef.182 | A | 50 |
| A01i | RQDILDLWVY | HIV.nef.182 | | 31 |
| A01i | RTDILDLWVY | HIV.nef.182 | A | 31 |
| A01s | WSTCSSIVGW | HIV.nef.5 | | 31 |
| A01s | WTKSSIVGW | HIV.nef.5 | A | 31 |
| A01s | WSKSSIVGY | HIV.nef.5 | A | 31 |
| A01s | WVYHTQGY | HIV.nef.191 | | 33 |
| A01s | WTYHTQGY | HIV.nef.191 | A | 33 |
| A01s | WVYHTQGYF | HIV.nef.191 | | 33 |
| A01s | WTYHTQGYF | HIV.nef.191 | A | 33 |
| A01s | WVYHTQGYY | HIV.nef.191 | A | 33 |
| A01i | YTPGPGIRY | HIV.nef.207 | | 27 |
| A01i | YTDGPGIRY | HIV.nef.207 | A | 27 |
| A01s | IIKILYQSNPY | HIV.rev.20 | | 28 |
| A01s | ITKILYQSNPY | HIV.rev.20 | A | 28 |
| A01s | ILYQSNPY | HIV.rev.23 | | 42 |
| A01s | ITYQSNPY | HIV:rev.23 | A | 42 |
| A01s | KILYQSNPY | HIV.rev.22 | | 41 |
| A01s | KTLYQSNPY | HIV.rev.22 | A | 41 |
| A01s | PVDPNLEPW | HIV.tat.3 | | 31 |
| A01s | PTDPNLEPW | HIV.tat.3 | A | 31 |
| A01s | PVDPNLEPY | HIV.tat.3 | A | 31 |

**TABLE 3**

| Motif | Sequence | Source | Analog | % Consv. |
|---|---|---|---|---|
| A02i/s | AAEGVGAV | HIV.nef.42 | | 28 |
| A02i/s | ALEGVGAV | HIV.nef.42 | A | 28 |
| A02i/s | AITSSNTA | HIV.nef.63 | | 42 |
| A02i/s | ALTSSNTA | HIV.nef.63 | A | 42 |
| A02i/s | AITSSNTV | HIV.nef.63 | A | 42 |
| A02i/s | AQEEEEVGFPV | HIV.nef.83 | | 27 |
| A02i/s | ALEEEEVGFPV | HIV.nef.83 | A | 27 |
| A02i/s | EAQEEEEV | HIV.nef.82 | | 25 |
| A02i/s | ELQEEEEV | HN.nef.82 | A | 25 |
| A02i/s | EVGFPVRPQV | HIV.nef.91 | | 63 |
| A02i/s | ELGFPVRPQV | HIV.nef.91 | A | 63 |
| A02i/s | EILDLWVYHT | HIV.nef.185 | | 34 |
| A02i/s | ELLDLWVYHT | HIV.nef.185 | A | 34 |
| A02i/s | EILDLWVYHV | HIV.nef.185 | A | 34 |
| A02i/s | FLKEKGGL | HIV.nef.117 | | 88 |
| A02i/s | FLKEKGGV | HIV.nef.117 | A | 88 |
| A02i/s | FLKEKGGLEGL | HIV.nef.117 | | 45 |
| A02i/s | FLKEKGGLEGV | HN.nef.117 | A | 45 |
| A02i/s | FLKEKGGLDGL | HIV.nef.117 | | 41 |
| A02i/s | FLKEKGGLDGV | HIV.nef.117 | A | 41 |
| A02i/s | GVGAVSRDL | HIV.nef.45 | | 27 |
| A02i/s | GLGAVSRDL | HIV.nef.45 | A | 27 |
| A02i/s | GVGAVSRDV | HIV.nef.45 | A | 27 |
| A02i/s | GAITSSNT | HIV.nef.62 | | 50 |
| A02i/s | GLITSSNT | HIV.nef.62 | A | 50 |
| A02i/s | GAITSSNV | HIV.nef.62 | A | 50 |
| A02i/s | GAITSSNTA | HIV.nef.62 | | 42 |
| A02i/s | GLITSSNTA | HIV.nef.62 | A | 42 |
| A02i/s | GAITSSNTV | HIV.nef.62 | A | 42 |
| A02i/s | GLIYSKKRQEI | HIV.nef.173 | | 28 |
| A02i/s | GLIYSKKRQEV | HIV.nef.173 | A | 28 |
| A02i/s | ILDLWVYHT | HIV.nef.186 | | 53 |
| A02i/s | ILDLWVYHV | HIV.nef.186 | A | 53 |
| A02i/s | ILDLWVYNT | HIV.nef.186 | | 30 |
| A02i/s | ILDLWVYNV | HIV.nef.186 | A | 30 |
| A02i/s | LIYSKKRQEI | HIV.nef.174 | | 28 |
| A02i/s | LLYSKKRQEI | HIV.nef.174 | A | 28 |
| A02i/s | LIYSKKRQEV | HIV.nef.174 | A | 28 |
| A02i/s | LIYSKKRQEIL | HIV.nef.174 | | 28 |
| A02i/s | LLYSKKRQEIL | HIV.nef.174 | A | 28 |
| A02i/s | LIYSKKRQEIV | HIV.nef.174 | A | 28 |
| A02i/s | LTFGWCFKL | HIV.nef.221 | | 61 |
| A02i/s | LLFGWCFKL | HIV.nef.221 | A | 61 |
| A02i/s | LTFGWCFKV | HIV.nef.221 | A | 61 |
| A02i/s | LTFGWCFKLV | HIV.nef.221 | | .55 |
| A02i/s | LLFGWCFKLV | HIV.nef.221 | A | 55 |
| A02i/s | NADCAWLEA | HIV.nef.73 | | 27 |
| A02i/s | NLDCAWLEA | HIV.nef.73 | A | 27 |
| A02i/s | NADCAWLEV | HIV.nef.73 | A | 27 |
| A02i/s | PAAEGVGA | HIV.nef.41 | | 33 |
| A02i/s | PLAEGVGA | HIV.nef.41 | A | 33 |
| A02i/s | PAAEGVGV | HIV.nef.41 | A | 33 |
| A02i/s | PVRPQVPL | HIV.nef.95 | | 75 |
| A02i/s | PLRPQVPL | HIV.nef.95 | A | 75 |
| A02i/s | PVRPQVPV | HIV.nef.95 | A | 75 |
| A02i/s | PVRPQVPLRPM | HIV.nef.95 | | 73 |
| A02i/s | PLRPQVPLRPM | HIV.nef.95 | A | 73 |
| A02i/s | PVRPQVPLRPV | HIV.nef.95 | A | 73 |
| A02i/s | PQVPLRPM | HIV.nef.99 | | 88 |
| A02i/s | PLVPLRPM | HIV.nef.99 | A | 88 |
| A02i/s | PQVPLRPV | HIV.nef.99 | A | 88 |
| A02i/s | PQVPLRPMT | HIV.nef.99 | | 88 |
| A02i/s | PLVPLRPMT | HIV.nef.99 | A | 88 |
| A02i/s | PQVPLRPMV | HIV.nef.99 | A | 88 |
| A02i/s | PLRPMTYKGA | HIV.nef.102 | | 39 |
| A02i/s | PLRPMTYKGV | HIV.nef.102 | A | 39 |
| A02i/s | PLRPMTYKA | HIV.nef.102 | | 33 |
| A02i/s | PLRPMTYKV | HIV.nef.102 | A | 33 |
| A02i/s | PLRPMTYKAA | HIV.nef.102 | | 31 |
| A02i/s | PLRPMTYKAV | HIV.nef.102 | A | 31 |
| A02i/s | PLTFGWCFKL | HIV.nef.219 | | 61 |
| A02i/s | PLTFGWCFKV | HIV.nef.219 | A | 61 |
| A02i/s | PLTFGWCFKLV | HIV.nef.219 | | 55 |
| A02i/s | QAEPAAAGV | HIV.nef.34 | | 33 |
| A02i/s | QLEPAAAGV | HIV.nef.34 | A | 33 |
| A02i/s | QAEPAAAGVGA | HIV.nef.34 | | 33 |
| A02i/s | QLEPAAAGVGA | HIV.nef.34 | A | 33 |
| A02i/s | QAEPAAAGVGV | HIV.nef.34 | A | 33 |
| A02i/s | QVPLRPMT | HIV.nef.100 | | 89 |
| A02i/s | QLPLRPMT | HIV.nef.100 | A | 89 |
| A02i/s | QVPLRPMV | HIV.nef.100 | A | 89 |
| A02i/s | QVPLRPMTYKA | HIV.nef.100 | | 31 |
| A02i/s | QLPLRPMTYKA | HIV.nef.100 | A | 31 |
| A02i/s | QVPLRPMTYKV | HIV.nef.100 | A | 31 |
| A02i/s | RQEILDLWV | HIV.nef.182 | | 55 |
| A02i/s | RLEILDLWV | HIV.nef.182 | A | 55 |
| A02i/s | RQDILDLWV | HIV.nef.182 | | 31 |
| A02i/s | RLDILDLWV | HIV.nef.182 | A | 31 |
| A02i/s | WQNYTPGPGT | HIV.nef.204 | | 33 |
| A02i/s | WLNYTPGPGT | HIV.nef.204 | A | 33 |
| A02i/s | WQNYTPGPGV | HIV.nef.204 | A | 33 |
| A02i/s | WQNYTPGPGI | HIV.nef.204 | | 29 |
| A02i/s | WLNYTPGPGI | HIV.nef.204 | A | 29 |
| A02i/s | WQNYTPGPGV | HIV.nef.204 | A | 29 |
| A02i/s | YTPGPGIRYPL | HIV.nef.207 | | 25 |
| A02i/s | YLPGPGIRYPL | HIV.nef.207 | A | 25 |
| A02i/s | YTPGPGIRYPV | HIV.nef.207 | A | 25 |
| A02i/s | GTSGTQGV | HIV.rev.94 | | 33 |
| A02i/s | GLSGTQGV | HIV.rev.94 | A | 33 |
| A02i/s | LQLPPLERL | HIV.rev.77 | | 56 |
| A02i/s | LLLPPLERL | HIV.rev.77 | A | 56 |
| A02i/s | LQLPPLERV | HIV.rev.77 | A | 56 |
| A02i/s | LQLPPLERLT | HIV.rev.77 | | 27 |
| A02i/s | LLLPPLERLT | HIV.rev.77 | A | 27 |
| A02i/s | LQLPPLERLV | HIV.rev.77 | A | 27 |
| A02i/s | LQLPPLERLTL | HIV.rev.77 | | 27 |
| A02i/s | LLLPPLERLTL | HIV.rev.77 | A | 27 |
| A02i/s | LQLPPLERLTV | HIV.rev.77 | A | 27 |
| A02i/s | PAEPVPLQL | HIV.rev.71 | | 33 |
| A02i/s | PLEPVPLQL | HIV.rev.71 | A | 33 |
| A02i/s | PAEPVPLQV | HIV.rev.71 | A | 33 |
| A02i/s | PVPLQLPPL | HIV.rev.74 | | 55 |
| A02i/s | PLPLQLPPL | HIV.rev.74 | A | 55 |
| A02i/s | PVPLQLPPV | HIV.rev.74 | A | 55 |
| A02i/s | PLQLPPLERL | HIV.rev.76 | | 53 |
| A02i/s | PLQLPPLERV | HIV.rev.76 | A | 53 |
| A02i/s | QLPPLERLT | HIV.rev.78 | | 28 |
| A02i/s | QLPPLERLV | HIV.rev.78 | A | 28 |

**TABLE 4**

| Motif | Sequence | Source | Analog | % Consv. |
|---|---|---|---|---|
| A03i | ADCAWLEA | HIV.nef.74 | | 27 |
| A03i | AVCAWLEA | HIV.nef.74 | A | 27 |
| A03i | ADCAWLEK | HIV.nef.74 | A | 27 |
| A03i | AFDLSFFLK | HIV.nef.111 | | 28 |
| A03i | AVDLSFFLK | HIV.nef.111 | A | 28 |
| A03i | AFDLSFFLKEK | HIV.nef.111 | | 27 |
| A03i | AVDLSFFLKEK | HIV.nef.111 | A | 27 |
| A03i | DLSHFLKEK | HIV.nef.113 | | 42 |
| A03i | DVSHFLKEK | HIV.nef.113 | A | 42 |
| A03i | DLSFFLKEK | HIV.nef.113 | | 34 |
| A03i | DVSFFLKEK | HIV.nef.113 | A | 34 |
| A03i | EAQEEEEVGF | HIV.nef.82 | | 25 |
| A03i | EVQEEEEVGF | HIV.nef.82 | A | 25 |
| A03i | EAQEEEEVGK | HIV.nef.82 | A | 25 |
| A03i | EILDLWVYH | HIV.nef.185 | | 34 |
| A03i | EVLDLWVYH | HIV.nef.185 | A | 34 |
| A03i | EILDLWVYK | HIV.nef.185 | A | 34 |
| A03s | ELHPEYYK | HIV.nef.324 | | 34 |
| A03s | EVHPEYYK | HIV.nef.324 | A | 34 |
| A03i | FDLSFFLK | HIV.nef.112 | | 28 |
| A03i | FVLSFFLK | HIV.nef.112 | A | 28 |
| A03i | FDLSFFLKEK | HIV.nef.112 | | 27 |
| A03i | FVLSFFLKEK | HIV.nef.112 | A | 27 |
| A03i | FFPDWQNY | HIV.nef.199 | | 27 |
| A03i | FVPDWQNY | HIV.nef.199 | A | 27 |
| A03i | FFPDWQNK | HIV.nef.199 | A | 27 |
| A03i | GFPVRPQVPLR | HIV.nef.93 | | 75 |
| A03i | GVPVRPQVPLR | HIV.nef.93 | A | 75 |
| A03i | GFPVRPQVPLK | HIV.nef.93 | A | 75 |
| A03i | GGLEGLIY | HIV.nef.124 | | 30 |
| A03i | GVLEGLIY | HIV.nef.124 | A | 30 |
| A03i | GGLEGLIK | HIV.nef.124 | A | 30 |
| A03i | GGLDGLIYSK | HIV.nef.124 | | 25 |
| A03i | GVLDGLIYSK | HIV.nef.124 | A | 25 |
| A03i | GLDGLIYSK | HIV.nef.125 | | 25 |
| A03i | GVDGLIYSK | HIV.nef.125 | A | 25 |
| A03i | GLIYSKKR | HIV.nef.173 | | 36 |
| A03i | GVIYSKKR | HIV.nef.173 | A | 36 |
| A03i | GLIYSKKK | HIV.nef.173 | A | 36 |
| A03i | GFFPDWQNY | HIV.nef.198 | | 27 |
| A03i | GVFPDWQNY | HIV.nef.198 | A | 27 |
| A03i | GFFPDWQNK | HIV.nef.198 | A | 27 |
| A03i | HGAITSSNTA | HIV.nef.61 | | 42 |
| A03i | HVAITSSNTA | HIV.nef.61 | A | 42 |
| A03i | HGATTSSNTK | HIV.nef.61 | A | 42 |
| A03i | ILDLWVYH | HIV.nef.186 | | 53 |
| A03i | IVDLWVYH | HIV.nef.186 | A | 53 |
| A03i | ILDLWVYK | HIV.nef.186 | A | 53 |
| A03i | KGGLEGLIY | HIV.nef.122 | | 30 |
| A03i | KVGLEGLIY | HIV.nef.122 | A | 30 |
| A03i | KGGLEGLIK | HIV.nef.122 | A | 30 |
| A03i | LSHFLKEK | HIV.nef.114 | | 42 |
| A03i | LVHFLKEK | HIV.nef.114 | A | 42 |
| A03i | LSFFLKEK | HIV.nef.114 | | 34 |
| A03i | LVFFLKEK | HIV.nef.114 | A | 34 |
| A03i | LDGLIYSK | HIV.nef.171 | | 25 |
| A03i | LVGLIYSK | HIV.nef.171 | A | 25 |
| A03i | LDLWVYHTQGY | HIV.nef.187 | | 33 |
| A03i | LVLWVYHTQGY | HIV.nef.187 | A | 33 |
| A03i | LDLWVYHTQGK | HIV.nef.187 | A | 33 |
| A03i | LTFGWCFK | HIV.nef.221 | | 61 |
| A03i | LLHPICQH | HIV.nef.257 | | 28 |
| A03i | LVHPICQH | HIV.nef.257 | A | 28 |
| A03i | LLHPICQK | HIV.nef.257 | A | 28 |
| A03i | LLHPMSQH | HIV.nef.257 | | 27 |
| A03i | LVHPMSQH | HIV.nef.257 | A | 27 |
| A03i | ILHPMSQK | HIV.nef.257 | A | 27 |
| A03i | PVRPQVPLR | HIV.nef.95 | | 75 |
| A03i | PVRPQVPLK | HIV.nef.95 | A | 75 |
| A03i | PLRPMTYK | HN.nef.102 | | 77 |
| A03i | PVRPMTYK | HIV.nef.102 | A | 77 |
| A03i | PLTFGWCFK | HIV.nef.219 | | 61 |
| A03i | PVTFGWCFK | HIV.nef.219 | A | 61 |
| A03i | QVPLRPMTYK | HIV.nef.100 | | 72 |
| A03i | QDLDLWVY | HIV.nef.184 | | 31 |
| A03i | QVTLDLWVY | HIV.nef.184 | A | 31 |
| A03i | QDILDLWVK | HIV.nef.184 | A | 31 |
| A03i | QGFFPDWQNY | HIV.nef.196 | | 27 |
| A03i | QVFFPDWQNY | HIV.nef.196 | A | 27 |
| A03i | QGFFPDWQNK | HIV.nef.196 | A | 27 |
| A03i | RDLEKHGA | HIV.nef.51 | | 25 |
| A03i | RVLEKHGA | HIV.nef.51 | A | 25 |
| A03i | RDLEKHGK | HIV.nef.51 | A | 25 |
| A03i | RFPLTFGWCF | HIV.nef.216 | | 27 |
| A03i | RVPLTFGWCF | HIV.nef.216 | A | 27 |
| A03i | RFPLTFGWCK | HIV.nef.16 | A | 27 |
| A03i | RFPLTFGWCFK | HIV.nef.216 | | 27 |
| A03i | RVPLTFGWCFK | HIV.nef.216 | A | 27 |
| A03i | YTPGPGTR | HIV.nef.207 | | 33 |
| A03i | YTPGPGTK | HIV.nef.207 | A | 33 |
| A03i | YTPGPGIR | HIV.nef.207 | | 31 |
| A03i | YTPGPGIK | HIV.nef.207 | A | 31 |
| A03i | EGTRQARR | HIV.rev.35 | | 42 |
| A03i | EVTRQARR | HIV.rev.35 | A | 42 |
| A03i | EGTRQARK | HIV.rev.35 | A | 42 |
| A03i | EGTRQARRNR | HIV.rev.35 | | 42 |
| A03i | EVTRQARRNR | HIV.rev.35 | A | 42 |
| A03i | EGTRQARRNK | HIV.rev.35 | A | 42 |
| A03i | EGTRQARRNRR | HIV.rev.35 | | 42 |
| A03i | EVTRQARRNRR | HIV.rev.35 | A | 42 |
| A03i | EGTRQARRNRK | HIV.rev.35 | A | 42 |
| A03i | GTRQARRNR | HIV.rev.36 | | 53 |
| A03i | GTRQARRNK | HIV.rev.36 | A | 53 |
| A03i | GTRQARRNRR | HIV.rev.36 | | 53 |
| A03i | GTRQARRNRK | HIV.rev.36 | A | 53 |
| A03i | GTRQARKNRRR | HIV.rev.36 | | 53 |
| A03i | GTRQARRNRRK | HIV.rev.36 | A | 53 |
| A03i | GTRQTRKNR | HIV.rev.37 | | 50 |
| A03i | GTRQTRKNK | HIV.rev.37 | A | 50 |
| A03i | GTRQTRKNRR | HIV.rev.37 | | 50 |
| A03i | GTRQTRKNRK | HIV.rev.37 | A | 50 |
| A03i | GTRQTRKNRRR | HIV.rev.37 | | 50 |
| A03i | GTRQTRKNRRK | HIV.rev.37 | A | 50 |
| A03i | PVPLQLPPLER | HIV.rev.74 | | 53 |
| A03i | PVPLQLPPLEK | HIV.rev.74 | A | 53 |
| A03i | PLQLPPLER | HIV.rev.76 | | 55 |
| A03i | PVQLPPLER | HIV.rev.76 | A | 55 |
| A03i | PLQLPPLEK | HIV.rev.76 | A | 55 |
| A03i | QARRNRRR | HIV.rev.40 | | 59 |
| A03i | QVRRNRRR | HIV.rev.40 | A | 59 |
| A03i | QARRNRRK | HIV.rev.40 | A | 59 |
| A03i | QARRNRRRR | HIV.rev.40 | | 59 |
| A03i | QVRRNRRRR | HIV.rev.40 | A | 59 |
| A03i | QARRNRRRK | HIV.rev.40 | A | 59 |
| A03i | QARRNRRRRWR | HIV.rev.40 | | 58 |
| A03i | QVRRNRRRRWR | HIV.rev.40 | A | 58 |
| A03i | QARRNRRRRWK | HIV.rev.40 | A. | 58 |
| A03i | QARKNRRR | HIV.rev.40 | | 27 |
| A03i | QVRRNRRR | HIV.rev.40 | A | 27 |
| A03i | QARIQVRRR | HIV.rev.40 | A | 27 |
| A03i | QARKNRRRR | HIV.rev.40 | | 25 |
| A03i | QVRKNRRRR | HIV.rev.40 | A | 25 |
| A03i | QARKNRRRK | HIV.rev.40 | A | 25 |
| A03i | QARKNRRRRWR | HIV.rev.40 | | 25 |
| A03i | QVRKNRRRRWR | HIV.rev.40 | A | 25 |
| A03i | QARKNRKRRWK | HIV.rev.40 | A | 25 |
| A03i | TTRQAKRNRRR | HIV.rev.37 | | 50 |
| A03i | TTRQARRNRRK | HIV.rev.37 | A | 50 |
| A03i | ACNNCYCK | HIV.tat.24 | | 27 |
| A03i | AVNNCYCK | HIV.tat.24 | A | 27 |
| A03i | AGPGGYPRR | HIV.tat.102 | | 50 |
| A03i | AVPGGYPRR | HIV.tat.102 | A | 50 |
| A03i | AGPGGYPRK | HIV.tat.102 | A | 50 |
| A03i | AGPGGYPRRK | HIV.tat.102 | | 50 |
| A03i | AVPGGYPRRK | HIV.tat.102 | A | 50 |
| A03i | ETGPSGQPCH | HIV.tat.101 | | 50 |
| A03i | ETGPSGQPCK | HIV.tat.101 | A | 50 |
| A03i | GGYPRRKGSCH | HIV.tat.105 | | 50 |
| A03i | GVYPRRRGSCH | HIV.tat.105 | A | 50 |
| A03i | GGYPRRKGSCK | HIV.tat.105 | A | 50 |
| A03i | ISYGRKKRRQR | HIV.tat.48 | | 61 |
| A03i | IVYGRKKRRQR | HIV.tat.48 | A | 61 |
| A03i | ISYGRKKRRQK | HIV.tat.48 | A | 61 |
| A03i | KAGPGGYPRR | HIV.tat.101 | | 50 |
| A03i | KVGPGGYPRR | HIV.tat.101 | A | 50 |
| A03i | KAGPGGYPRK | HIV.tat.101 | A | 50 |
| A03i | KAGPGGYPRRK | HIV.tat.101 | | 50 |
| A03i | KVGPGGYPRRK | HIV.tat.101 | A | 50 |
| A03i | LGISYGRKKRR | HIV.tat.46 | | 70 |
| A03i | LVISYGRKKRR | HIV.tat.46 | A | 70 |
| A03i | LGISYGRKKRK | HIV.tat.46 | A | 70 |
| A03i | PGSQPKTA | HIV.tat.17 | | 41 |
| A03i | PVSQPKTA | HIV.tat.17 | A | 41 |
| A03i | PGSQPKTK | HIV.tat.17 | A | 41 |
| A03i | PTGPKESK | HIV.tat.88 | | 31 |
| A03i | PTGPKBSKK | HIV.tat.88 | | 28 |
| A03i | PGGYPRRK | HIV.tat.104 | | 50 |
| A03i | PVGYPRRK | HIV.tat.104 | A | 50 |
| A03s | PTGPKESKK | HIV.tat.88 | | 28 |
| A03i | RGDPTGPK | HIV.tat.84 | | 25 |
| A03i | RVDPTGPK | HIV.tat.84 | A | 25 |
| A03i | TACNNCYCK | HIV.tat.23 | | 27 |
| A03i | TVCNNCYCK | HIV.tat.23 | A | 27 |
| A03i | TGPKESKK | HIV.tat.89 | | 30 |
| A03i | TVPKESKK | HIV.tat.89 | A | 30 |
| A03i | TGPSGQPCH | HIV.tat.102 | | 50 |
| A03i | TVPSGQPCH | HIV.tat.102 | A | 50 |
| A03i | TGPSGQPCK | HIV.tat.102 | A | 50 |
| A03i | VDPNLEPWNH | HIV.tat.4 | | 25 |
| A03i | VVPNLEPWNH | HIV.tat.4 | A | 25 |
| A03i | VDPNLEPWNK | HIV.tat.4 | A | 25 |
| A03i | YGRKKRRQR | HIV.tat.50 | | 64 |
| A03i | YVRKKRRQR | HIV.tat.50 | A | 64 |
| A03i | YGRKKRRQK | HIV.tat.50 | A | 64 |
| A03i | YGRKKRRQRR | HIV.tat.50 | | 59 |
| A03i | YVRKIGRRQRR | HIV.tat.50 | A | 59 |
| A03i | YGRKKRRQRK | HIV.tat.50 | A | 59 |
| A03i | YGRKKRRQRRR | HIV.tat.50 | | 34 |
| A03i | YVRICKRRQRRR | HIV.tat.50 | A | 34 |
| A03i | YGRKKRRQRRK | HIV.tat.50 | A | 34 |

**TABLE 5**

| Motif | Sequence | Source | Analog | % Consv. |
|---|---|---|---|---|
| A11i | QNYTPGPGTR | HIV.nef.205 | | 31 |
| A11i | QVYTPGPGTR | HIV.nef.205 | A | 31 |
| A11i | QNYTPGPGTK | HIV.nef.205 | A | 31 |
| A11i | QNYTPGPGIR | HIV.nef.205 | | 28 |
| A11i | QVYTPGPGIR | HIV.nef.205 | A | 28 |
| A11i | QNYTPGPGIK | HIV.nef.205 | A | 28 |
| A11i | KNRRRRWR | HIV.rev.43 | | 33 |
| A11i | KVRRRRWR | HIV.rev.43 | A | 33 |
| A11i | KNRRRRWK | HIV.rev.43 | A | 33 |
| A11i | KNRRRRWRAR | HIV.rev.43 | | 30 |
| A11i | KVRRRRWRAR | HIV.rev.43 | A | 30 |
| A11i | KIVRRRRWRAK | HIV.rev.43 | A | 30 |
| A11i | RNRRRRWR | HIV.rev.43 | | 63 |
| A11i | RVRRRRWR | HIV.rev.43 | A | 63 |
| A11i | RNRRRRWK | HIV.rev.43 | A | 63 |
| A11i | RNRRRRWRAR | HIV.rev.43 | | 36 |
| A11i | RVRRRRWRAR | HIV.rev.43 | A | 36 |
| A11i | RNRRRRWRAK | HIV.rev.43 | A | 36 |
| A11i | PNLEPWNH | HIV.tat.9 | | 27 |
| A11i | PVLEPWNH | HIV.tat.9 | A | 27 |
| A11i | PNLFPWNK | HIV.tat.9 | A | 27 |

**TABLE 6**

| Motif | Sequence | Source | Analog | % Consv. |
|---|---|---|---|---|
| A24i | AFDLSFFL | HIV.nef.111 | | 28 |
| A24i | AYDLSFFL | HIV.nef.111 | A | 28 |
| A24i | AFDLSFFF | HIV.nef.111 | A | 28 |
| A24i | DWQNYTPGPGI | HIV.nef.203 | | 28 |
| A24i | DYQNYTPGPGI | HIV.nef.203 | A | 28 |
| A24i | DWQNYTPGPGF | HIV.nef.203 | A | 28 |
| A24i | FFLREKGGL | HIV.nef.116 | | 41 |
| A24i | FYLKEKGGL | HIV.nef.116 | A | 41 |
| A24i | FFLKEKGGF | HIV.nef.116 | A | 41 |
| A24i | GFPVRPQVPL | HIV.nef.93 | | 75 |
| A24i | GYPVRPQVPL | HIV.nef.93 | A | 75 |
| A24i | GFPYRPQVPF | HIV.nef.93 | A | 75 |
| A24s | GVGAVSQDL | HIV.nef.45 | | 33 |
| A24s | GYGAVSQDL | HIV.nef.45 | A | 33 |
| A24s | GVGAVSQDF | HIV.nef.45 | A | 33 |
| A24i | HFLKEKGGL | HIV.nef.116 | | 45 |
| A24i | HYLKEKGGL | HIV.nef.116 | A | 45 |
| A24i | HFLKEKGGF | HIV.nef.116 | A | 45 |
| A24i | IYSKKRQEI | HIV.nef.175 | | 29 |
| A24i | IYSKKRQEF | HIV.nef.175 | A | 29 |
| A24i | IYSKKRQEIL | HIV.nef.175 | | 29 |
| A24i | IYSKKRQEIF | HIV.nef.175 | A | 29 |
| A24i | KWSKSSIVGW | HIV.nef.4 | | 31 |
| A24i | KYSKSSIVGW | HIV.nef.4 | A | 31 |
| A24i | KWSKSSIVGF | HIV.nef.4 | A | 31 |
| A24i | LWVYHTQGYF | HIV.nef.190 | | 33 |
| A24i | LYVYHTQGYF | HIV.nef.190 | A | 33 |
| A24s | LWVYHTQGY | HIV.nef.190 | | 33 |
| A24s | LYVYHTQGY | HIV.nef.190 | A | 33 |
| A24s | LWVYHTQGF | HIV.nef.190 | A | 33 |
| A24i | NYTPGPGI | HIV.nef.206 | | 31 |
| A24i | NYTPGPGF | HIV.nef.206 | A | 31 |
| A24s | NYTPGPGIRY | HIV.nef.206 | | 27 |
| A24s | NYTTGPGIRF | HIV.nef.206 | A | 27 |
| A24i | RYPLTFGW | HIV.nef.216 | | 43 |
| A24i | RYPLTFGF | HIV.nef.216 | A | 43 |
| A24i | RYPLTFGWCF | HIV.nef.216 | | 33 |
| A24i | RFPLTFGW | HIV.nef.216 | | 32 |
| A24i | RYPLTFGW | HIV.nef.216 | A | 32 |
| A24i | RFPLTFGF | HIV.nef.216 | A | 32 |
| A24i | SFFLKEKGGL | HIV.nef.115 | | 34 |
| A24i | SYFLKEKGGL | HIV.nef.115 | A | 34 |
| A24i | SFFLKEKGGP | HIV.nef.115 | A | 34 |
| A24i | TFGWCFKL | HIV.nef.222 | | 63 |
| A24i | TYGWCFKL | HIV.nef.222 | A | 63 |
| A24i | TFGWCFKF | HIV.nef.222 | A | 63 |
| A24i | VYHTQGYF | HIV.nef.192 | | 33 |
| A24i | VYHTQGYFPDW | HIV.nef.192 | | 33 |
| A24i | VYHTQGYFPDF | HIV.nef.192 | A | 33 |
| A24s | QLPPLERL | HIV.rev.78 | | 58 |
| A24s | QYPPLERL | HIV.rev.78 | A | 58 |
| A24s | QLPPLERF | HIV:rev.78 | A | 58 |
| A24s | QLPPLERLTL | HIV.rev.78 | | 28 |
| A24s | QYPPLERLTL | HIV.rev.78 | A | 28 |
| A24s | QLPPLERLTF | HIV.rev.78 | A | 28 |
| A24i | RWRARQRQI | HIV.rev.48 | | 55 |
| A24i | RYRARQRQI | HIV.rev.48 | A | 55 |
| A24i | RWRARQRQF | HIV.rev.48 | A | 55 |

**TABLE 7**

| Motif | Sequence | Source | Analog | % Consv. |
|---|---|---|---|---|
| B07s | APTAAKGV | HIV.nef.34 | | 33 |
| B07s | APTAAKGI | HIV.nef.34 | A | 33 |
| B07s | APTAAKGVGA | HIV.nef.34 | | 33 |
| B07s | APTAAKGVGI | HIV.nef.34 | A | 33 |
| B07s | APTAAKGVGAV | HIV.nef.34 | | 33 |
| B07s | APTAAKGVGAI | HIV.nef.34 | A | 33 |
| B07s | FPVRPQVPL | HIV.nef.94 | | 75 |
| B07s | FPVRPQVPI | HIV.nef.94 | A | 75 |
| B07s | FPLTFGWCF | HIV.nef.217 | | 27 |
| B07s | FPLTFGWCI | HIV.nef:217 | A | 27 |
| B07s | FPLTFGWCFKL | HIV.nef.217 | | 27 |
| B07s | FPLTFGWCFKI | HIV.nef.217 | A | 27 |
| B07s | GPGIRYPL | HIV.nef.210 | | 27 |
| B07s | GPGIRYPI | HIV.nef.210 | A | 27 |
| B07s | RPQVPLRPM | HIV.nef.98 | | 73 |
| B07s | RPQVPLRPI | HIV.nef.98 | A | 73 |
| B07s | RPQVPLRPMTY | HIV.nef.98 | | 56 |
| B07s | RPQVPLRPMTI | HIV.nef.98 | A | 56 |
| B07s | RPMTYKAA | HIV.nef.104 | | 36 |
| B07s | RFMTYKAI | HIV.nef.104 | A | 36 |
| B07s | TPGPGIRY | HIV.nef.208 | | 27 |
| B07s | TPGPGIRI | HIV.nef.208 | A | 27 |
| B07s | VPLRPMTY | HIV.nef.101 | | 73 |
| B07s | VPLRPMTI | HIV.nef.101 | A | 73 |
| B07s | VPLRPMTYKGA | HIV.nef.101 | | 37 |
| B07s | VPLRPMTYKGI | HIV.nef.101 | A | 37 |
| B07s | VPLRPMTYKA | HIV.nef.101 | | 32 |
| B07s | VPLRPMTYKI | HIV.nef.101 | A | 32 |
| B07s | VPLRPMTYKAA | HIV.nef.101 | | 30 |
| B07s | VPLRPMTYKAI | HIV.nef.101 | A | 30 |
| B07s | YPLTFGWCF | HIV.nef.217 | | 38 |
| B07s | YPLTFGWCI | HIV.nef.217 | A | 38 |
| B07s | LPPLERLTL | HIV.rev.79 | | 30 |
| B07s | LPPLERLTI | HIV.rev.79 | A | 30 |
| B07s | PPLERLTL | HIV.rev.80 | | 30 |
| B07s | PPLERLTI | HIV.rev.80 | A | 30 |
| B07s | RPAEPVPL | HIV.rev.70 | | 31 |
| B07s | RPAEPVPI | HIV.rev.70 | A | 31 |
| B07s | RPAEPVPLQL | HIV.rev.70 | | ' 31 |
| B07s | RPAEPVPLQI | HIV.rev.70 | A | 31 |
| B07s | VPLQLPPL | HIV.rev.75 | | 56 |
| B07s | VPLQLPPI | HIV.rev.75 | A | 56 |
| B07s | HPGSQPKTA | HIV.tat.16 | | 41 |
| B07s | HPGSQPKTI | HIV.tat.16 | A | 41 |

**TABLE 8**

| Motif | Sequence | Source | Analog | % Consv. |
|---|---|---|---|---|
| B27s | EKGGLEGL | HIV.nef.121 | | 53 |
| B27s | EKGGLEGLI | HIV.nef.121 | | 42 |
| B27s | EKGGLDGL | HIV.nef.121 | | 41 |
| B27s | EKGGLDGLI | HIV.nef.121 | | 36 |
| B27s | EKGGLEGLIY | HIV.nef.121 | | 30 |
| B27s | GKWSKSSI | HIV.nef.3 | | 28 |
| B27s | GKWSKSSIVGW | HIV.nef.3 | | 28 |
| B27s | KKRQEILDL | HIV.nef.179 | | 39 |
| B27s | KKRQEILDLW | HIV.nef.179 | | 39 |
| B27s | KRQEILDL | HIV.nef.181 | | 50 |
| B27s | KRQEILDLW | HIV.nef.181 | | 50 |
| B27s | KRQEILDLWVY | HIV.nef.181 | | 45 |
| B27s | KRQDILDL | HIV.nef.181 | | 28 |
| B27s | KRQDILDLW | HIV.nef.181 | | 28 |
| B27s | KRQDILDLWVY | HIV.nef.181 | | 25 |
| B27s | LKEKGGLDGL | HIV.nef.118 | | 42 |
| B27s | LKEKGGLDGLI | HIV.nef.118 | | 37 |
| B27s | SKSSIVGW | HIV.nef.6 | | 31 |
| B27s | SKKRQEIL | HIV.nef.177 | | 39 |
| B27s | SKKRQEILDL | HIV.nef.177 | | 39 |
| B27s | SKKRQEILDLW | HIV.nef.177 | | 39 |
| B27s | VRPQVPLRPM | HIV.nef.97 | | 73 |
| B27s | ARKNRRRRW | HIV.nef.41 | | 28 |
| B27s | GRSGDSDEEL | HIV.rev.3 | | 27 |
| B27s | GRSGDSDEELL | HIV.rev.3 | | 25 |
| B27s | GRPAEPVPL | HIV.rev.69 | | 31 |
| B27s | GRPAEPVPLQL | HIV.rev.69 | | 31 |
| B27s | IKILYQSNPY | HIV.rev.21 | | 39 |
| B27s | RKNRRRRW | HIV.rev.42 | | 33 |
| B27s | RRWRARQRQI | HIV.rev.47 | | 53 |
| B27s | VRIIKILY | HIV.rev.18 | | 28 |
| B27s | TKGLGISY | HIV.tat.43 | | 30 |

**TABLE 9**

| Motif | Sequence | Source | Analog | % Consv. |
|---|---|---|---|---|
| B44s | AEPAAEGV | HIV.nef.34 | | 33 |
| B44s | ABPAAEGVGA | HIV.nef.34 | | 33 |
| B44s | AEPAAEGVGAV | HIV.nef.34 | | 33 |
| B44s | DEEVGFPV | HIV.nef.89 | | 50 |
| B44s | EEEEVGFPV | HIV.nef.86 | | 27 |
| B44s | EEEVGFPV | HIV.nef.87 | | 33 |
| B44s | EEVGFPVRPQV | HIV.nef.88 | | 31 |
| B44s | EEVGFPVRPQI | HIV.nef.89 | | 50 |
| B44s | EEVGFPVRPQV | HIV.nef.90 | | 60 |
| B44s | KEKGGLDGL | HIV.nef.120 | | 41 |
| B44s | KEKGGLDGLI | HIV.nef.120 | | 37 |
| B44s | KEKGGLEGLIY | HIV.nef.120 | | 27 |
| B44s | QBEEEVGF | HIV.nef.84 | | 27 |
| B44s | QEEEEVGFPV | HIV.nef.84 | | 27 |
| B44s | QEILDLWV | HIV.nef.184 | | 55 |
| B44s | QEILDLWVY | HIV.nef.184 | | 50 |
| B44s | QDILDLWV | HIV.nef.184 | | 31 |
| B44s | QEILDLWV | HIV.nef.184 | A | 31 |
| B44s | TEPAAVGV | HIV.nef.33 | | 33 |
| B44s | TEPAAVGVGA | HIV.nef.33 | | 33 |
| B44s | TEPAAVGVGAV | HIV.nef.33 | | 33 |
| B44s | AEPVPLQL | HIV.rev.72 | | 52 |
| B44s | EDCGTSGTQGV | HIV.nef.91 | | 30 |
| B44s | EECGTSGTQGV | HIV.rev.91 | A | 30 |
| B44s | GDSDEELL | HIV.rev.6 | | 25 |
| B44s | GESDEELL | HIV.rev.6 | A | 25 |
| B44s | VDPNLEPW | HIV.tat.4 | | 34 |
| B44s | VEPNLEPW | HIV.tat.4 | A | 34 |

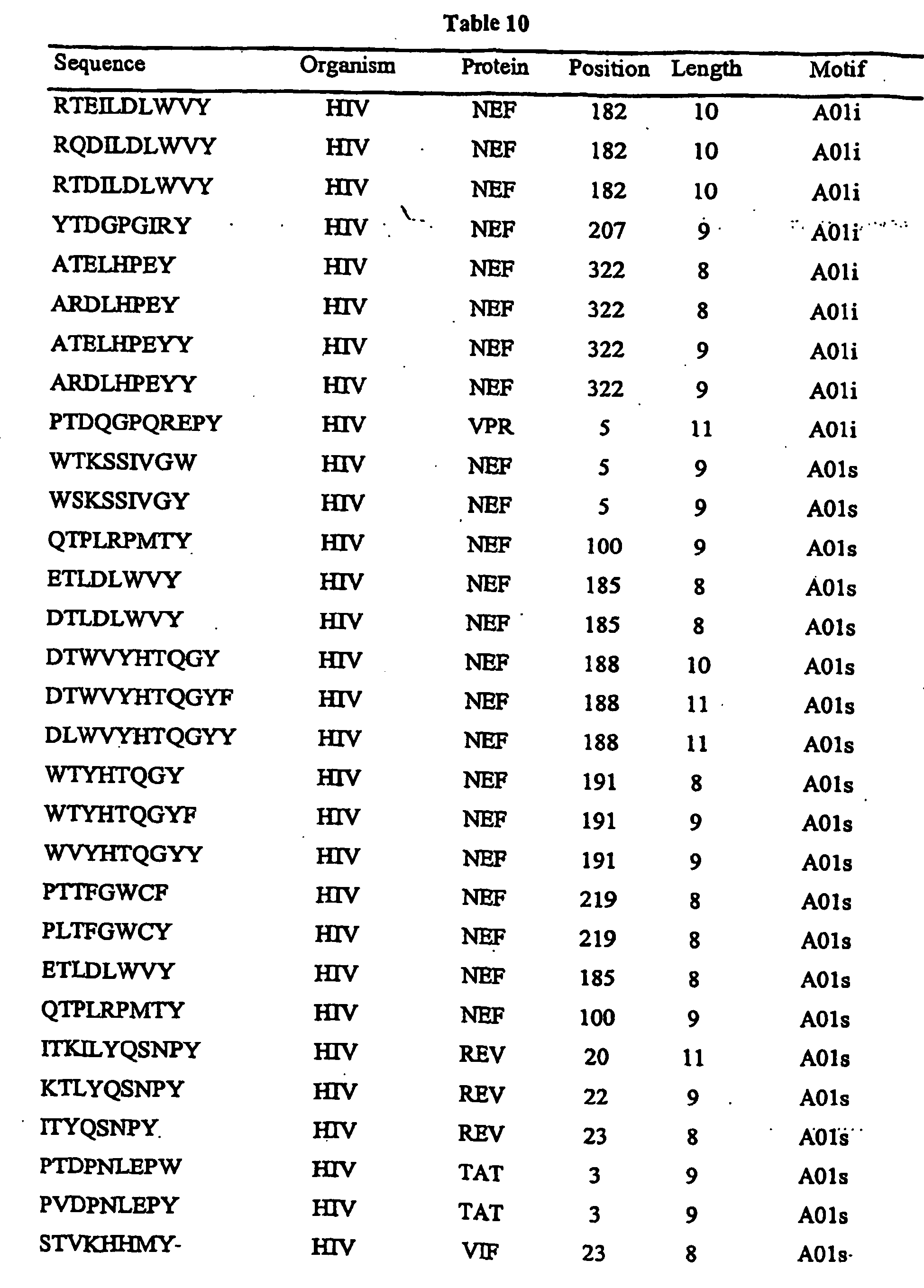

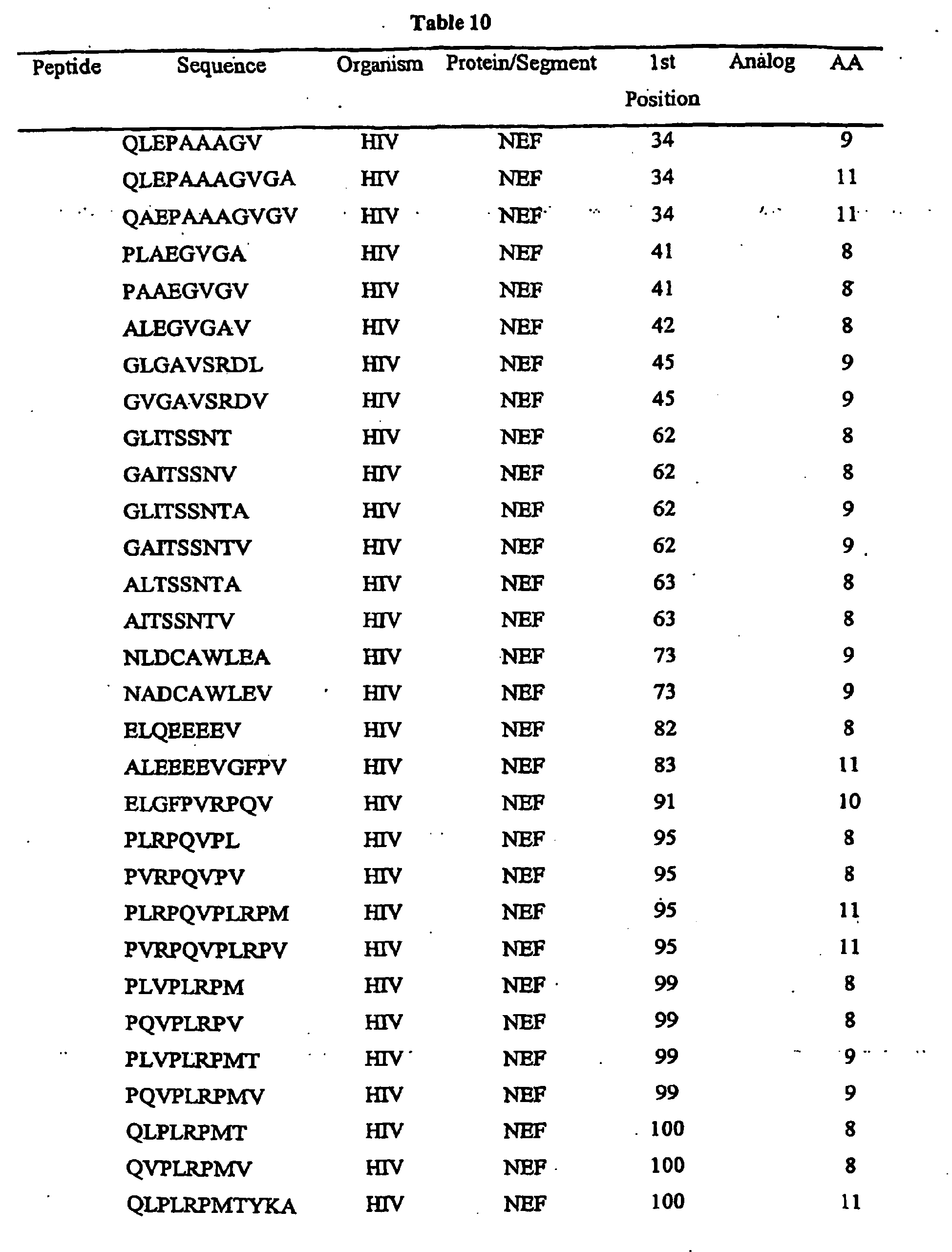

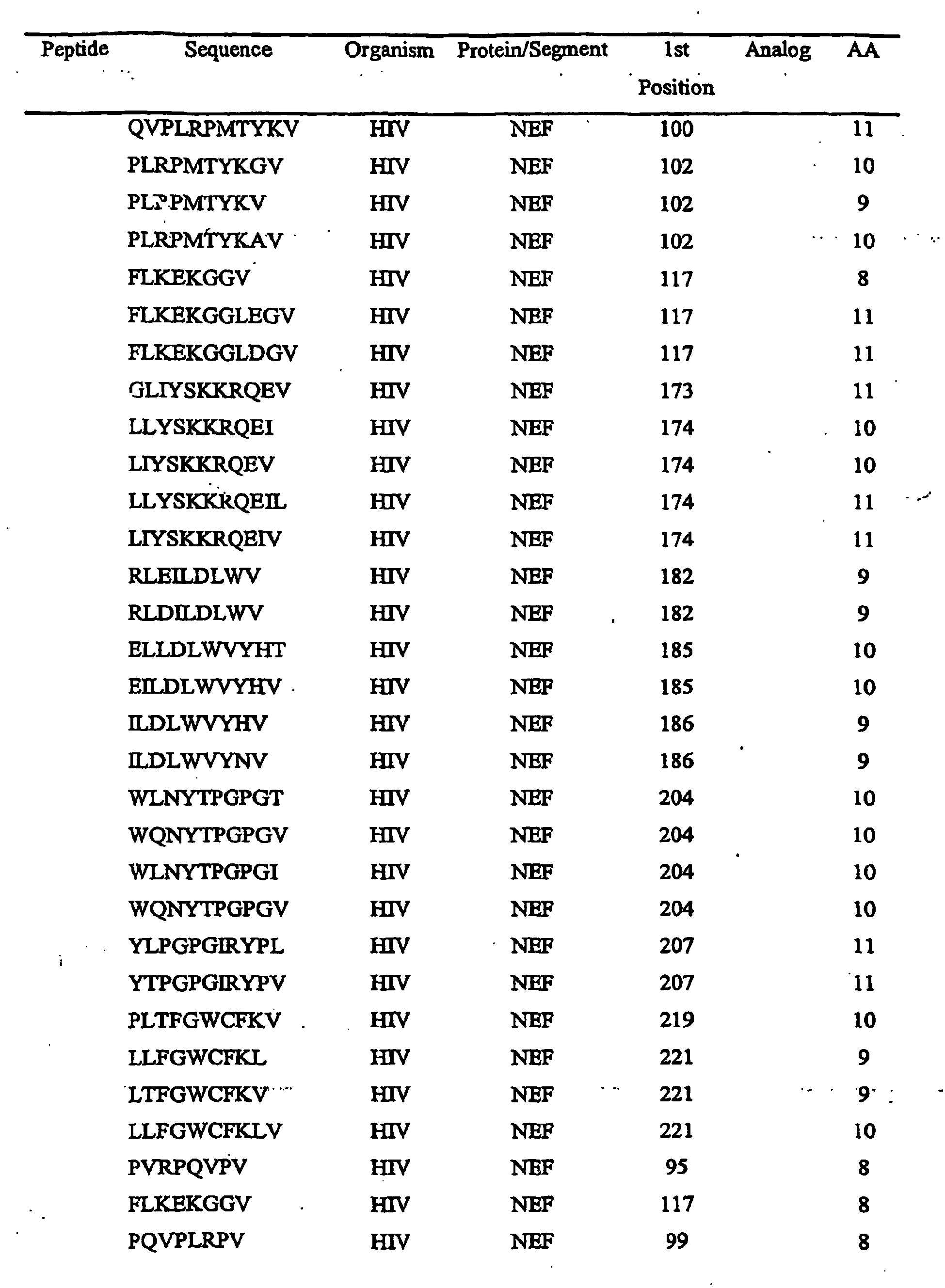

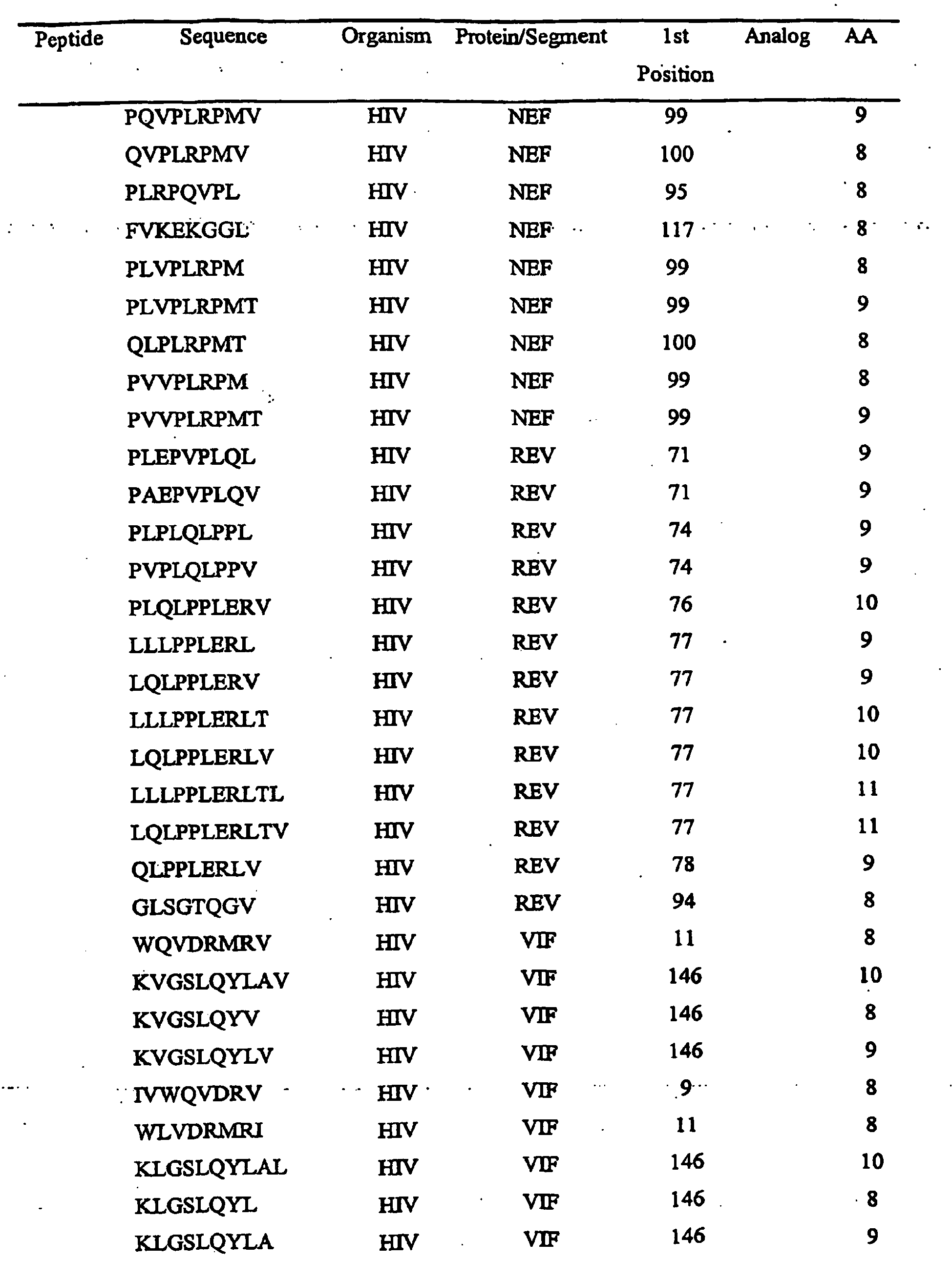

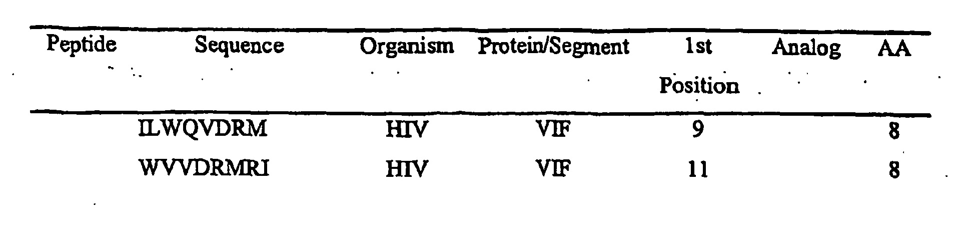

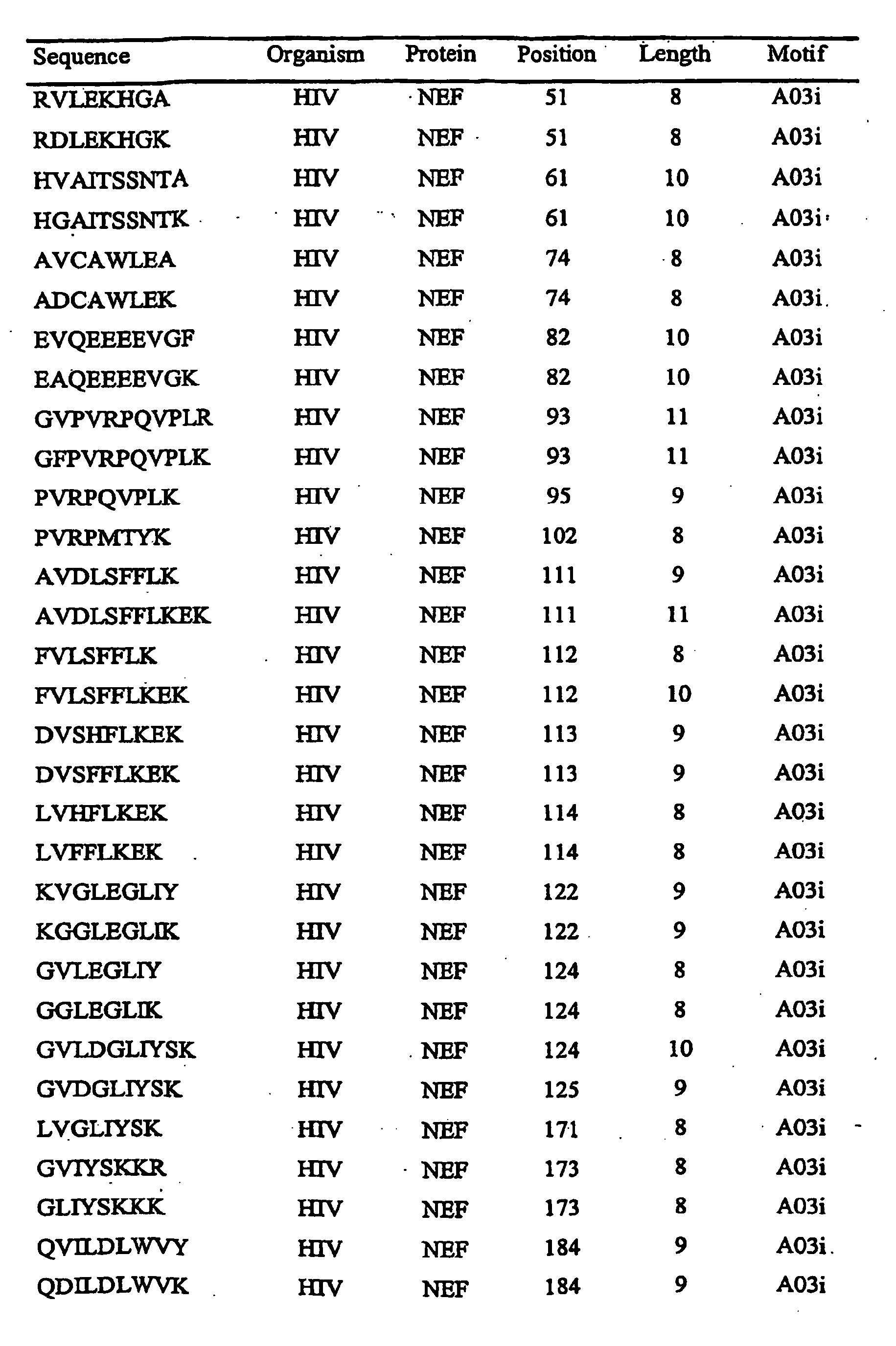

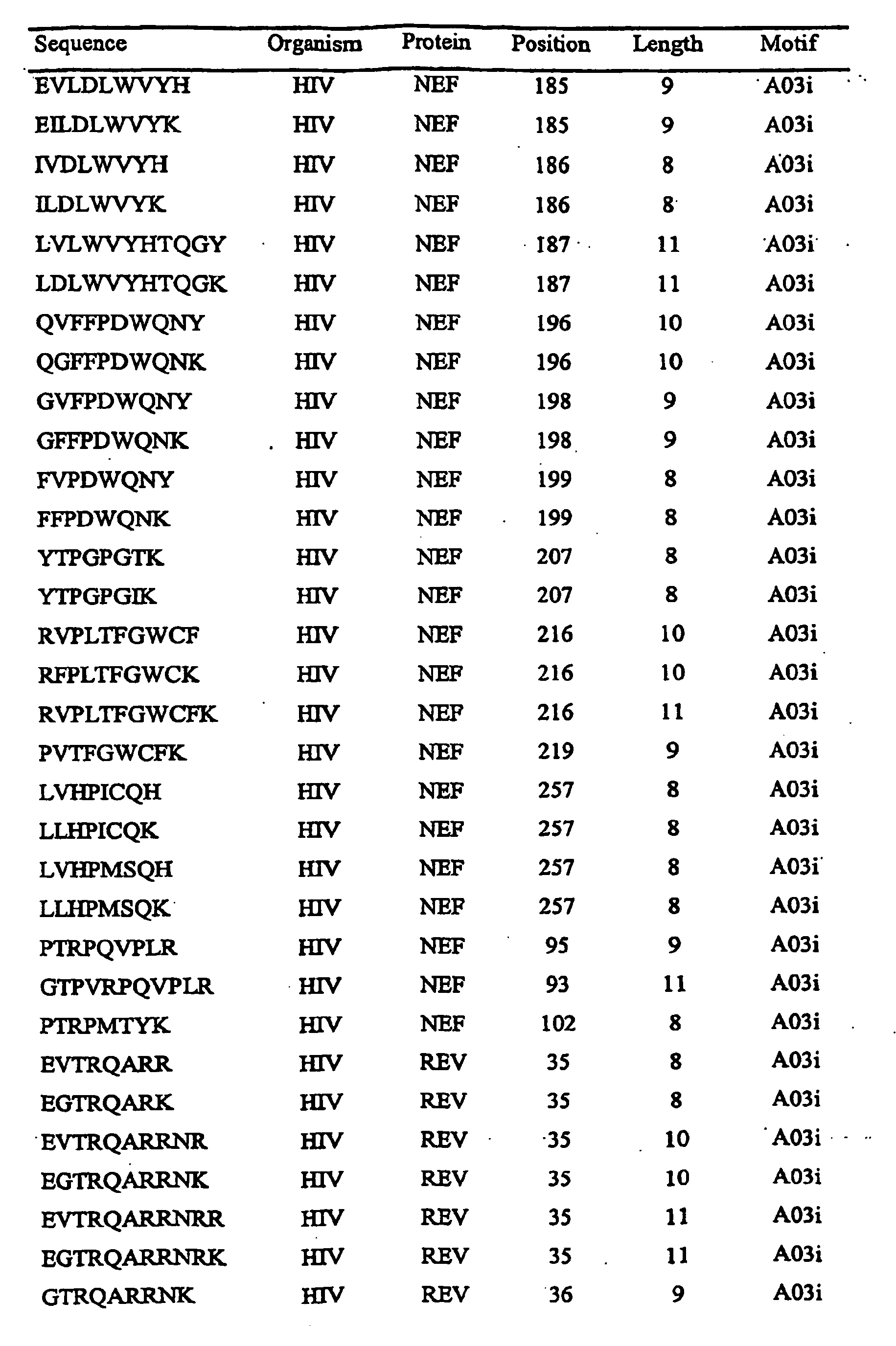

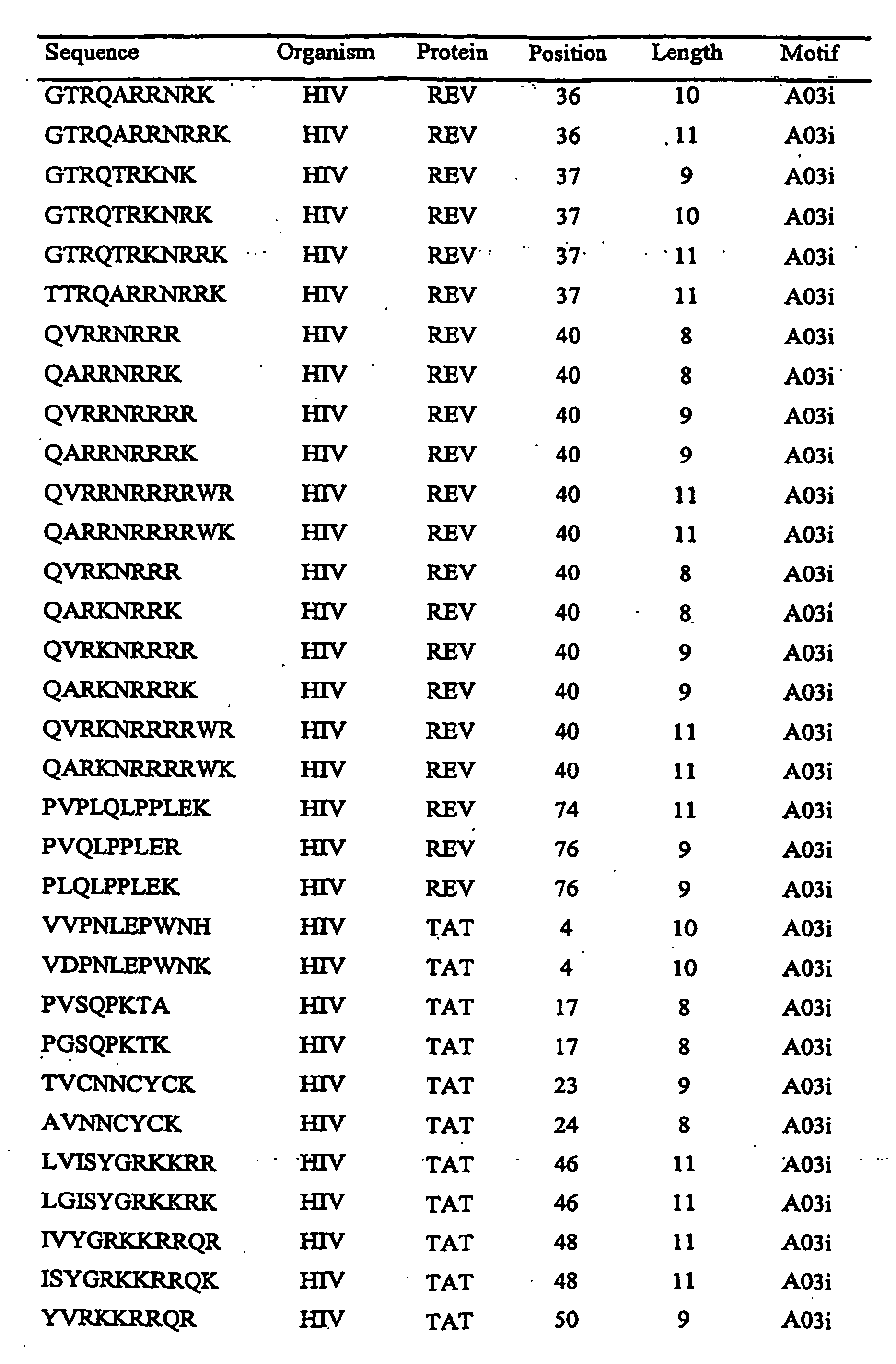

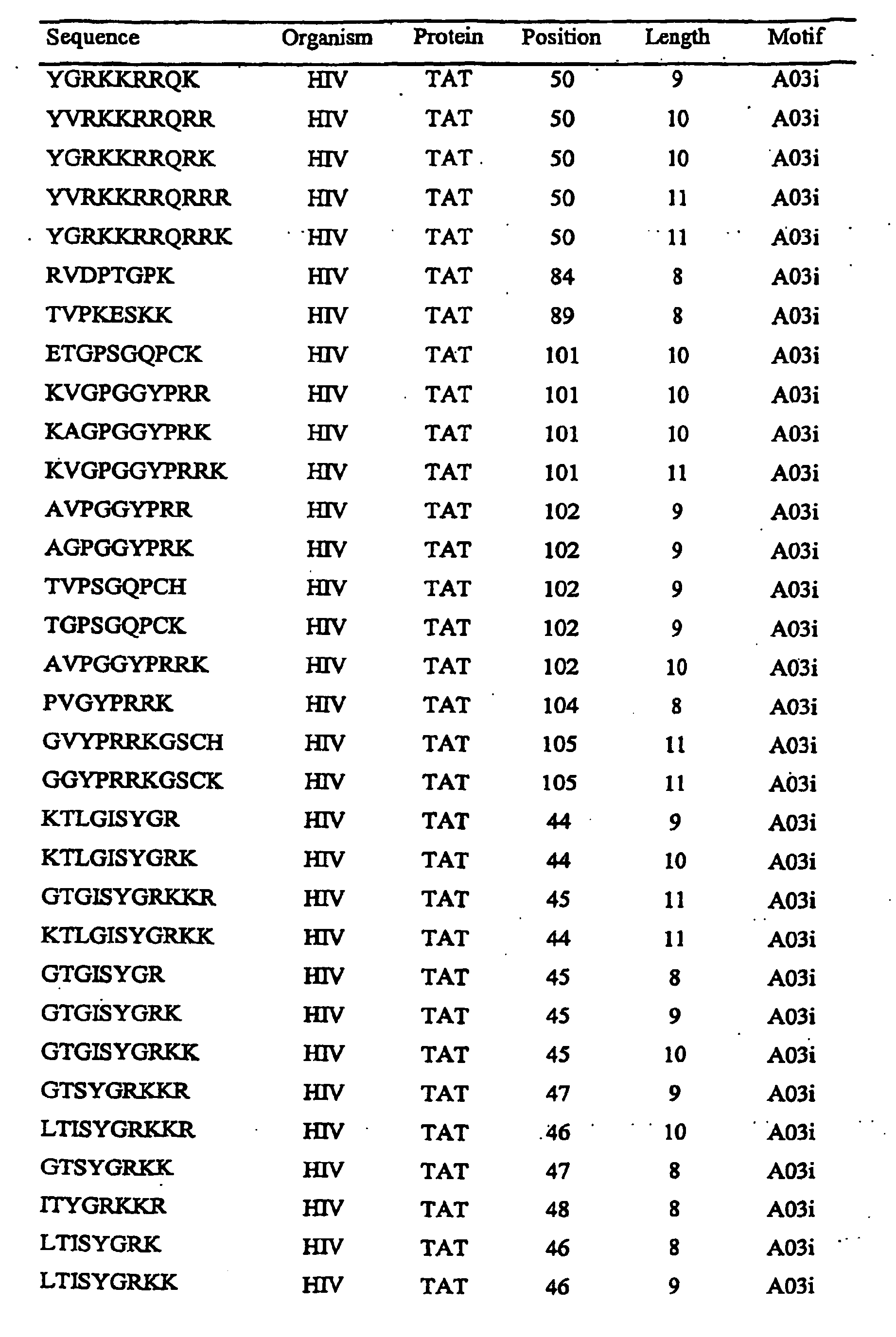

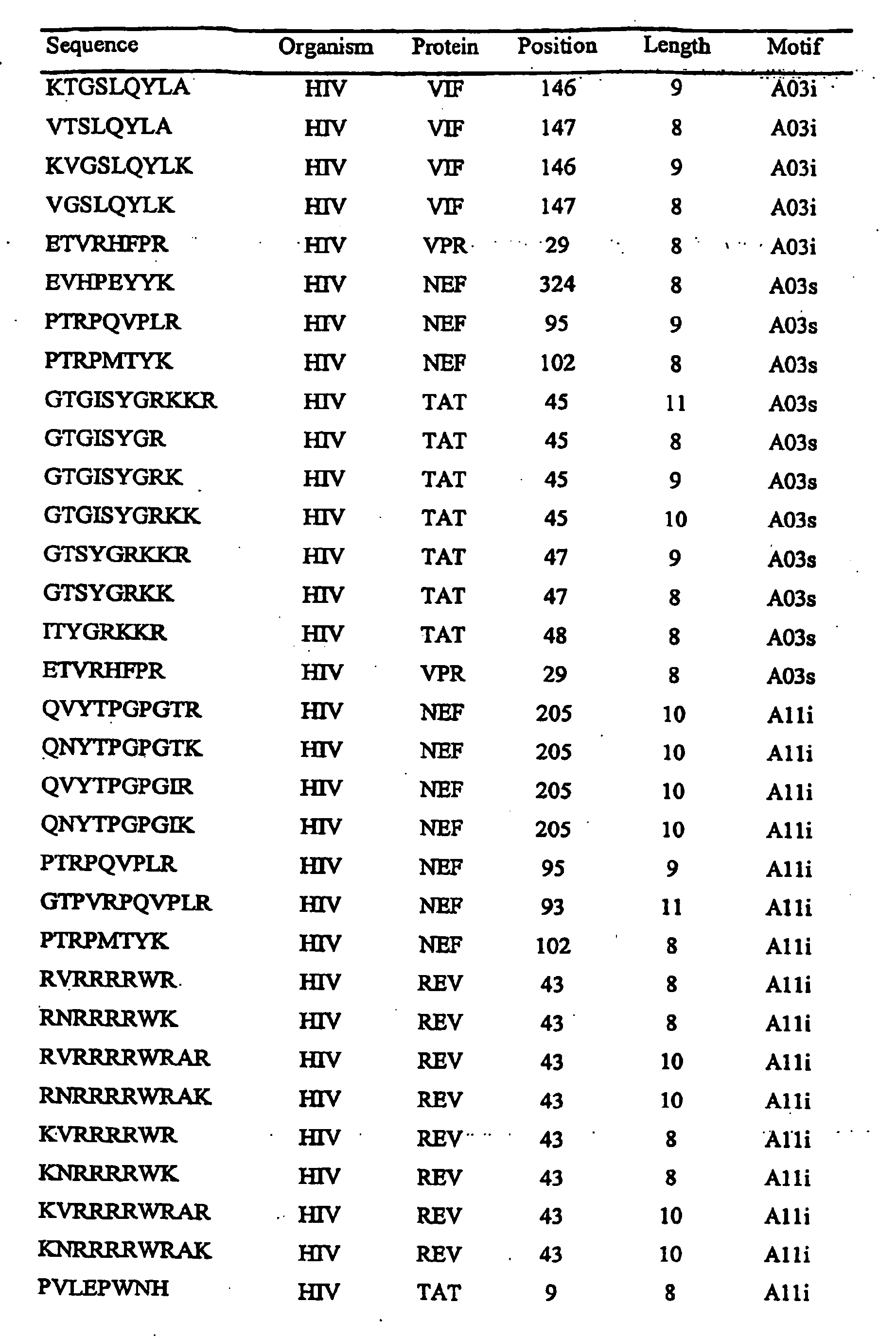

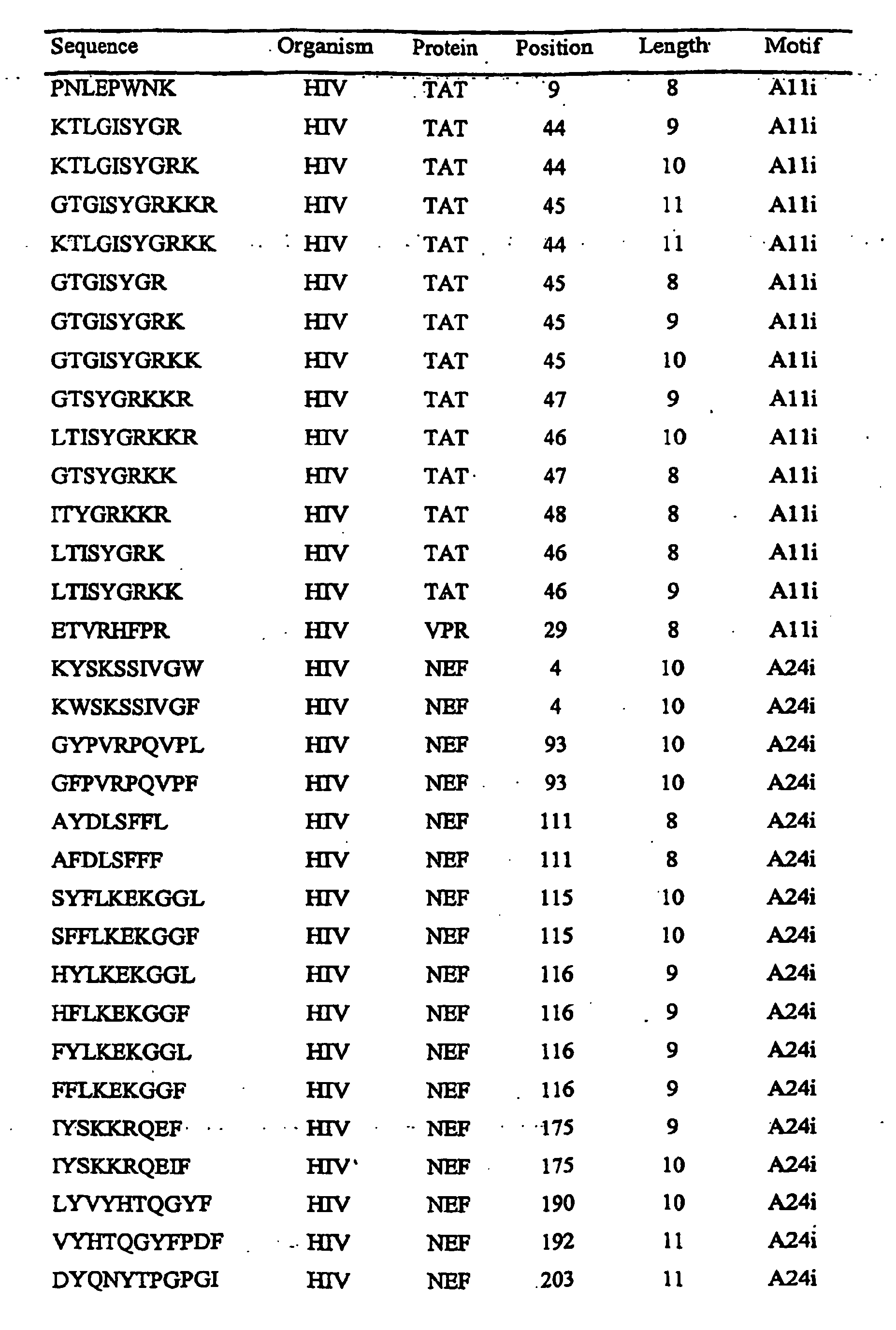

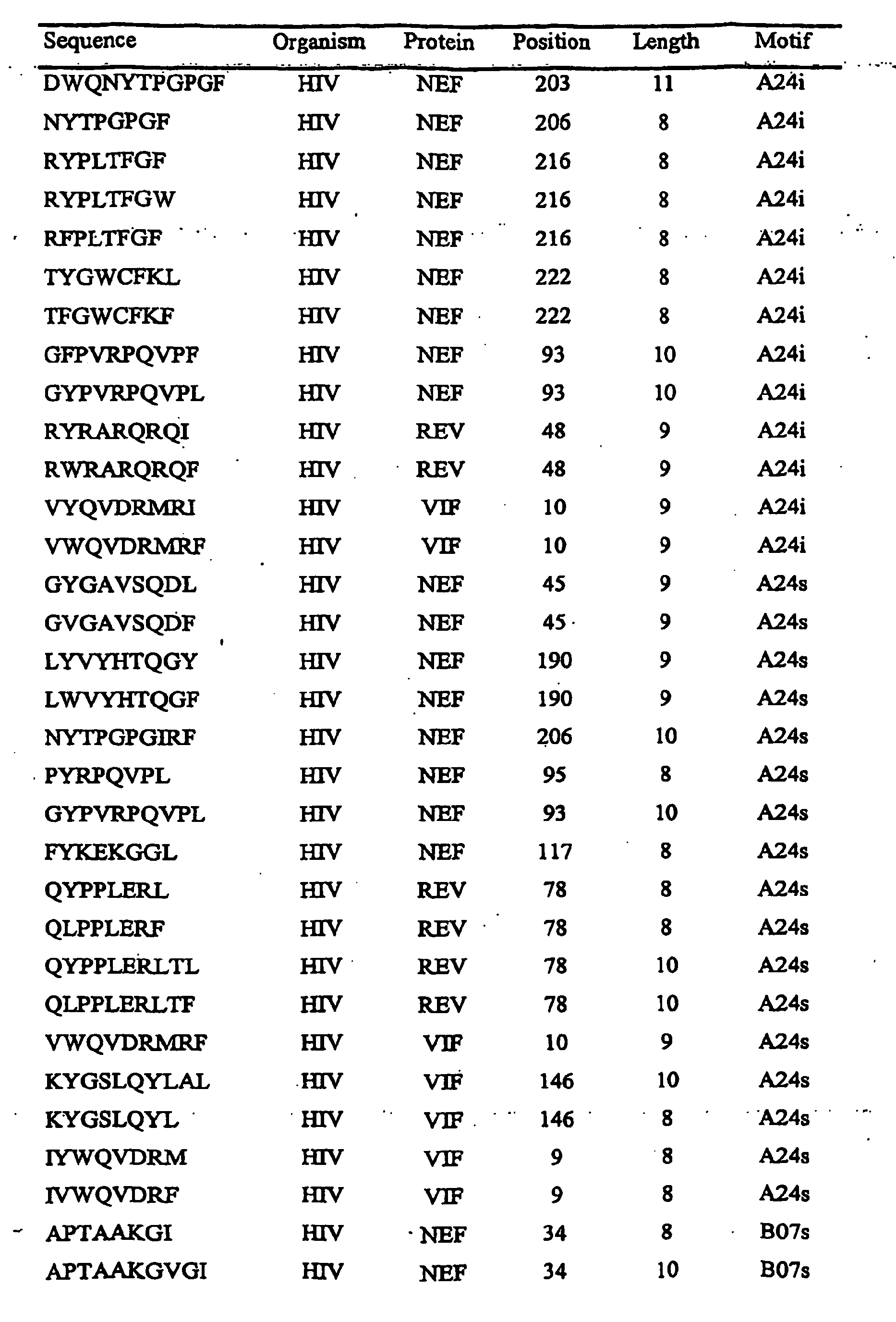

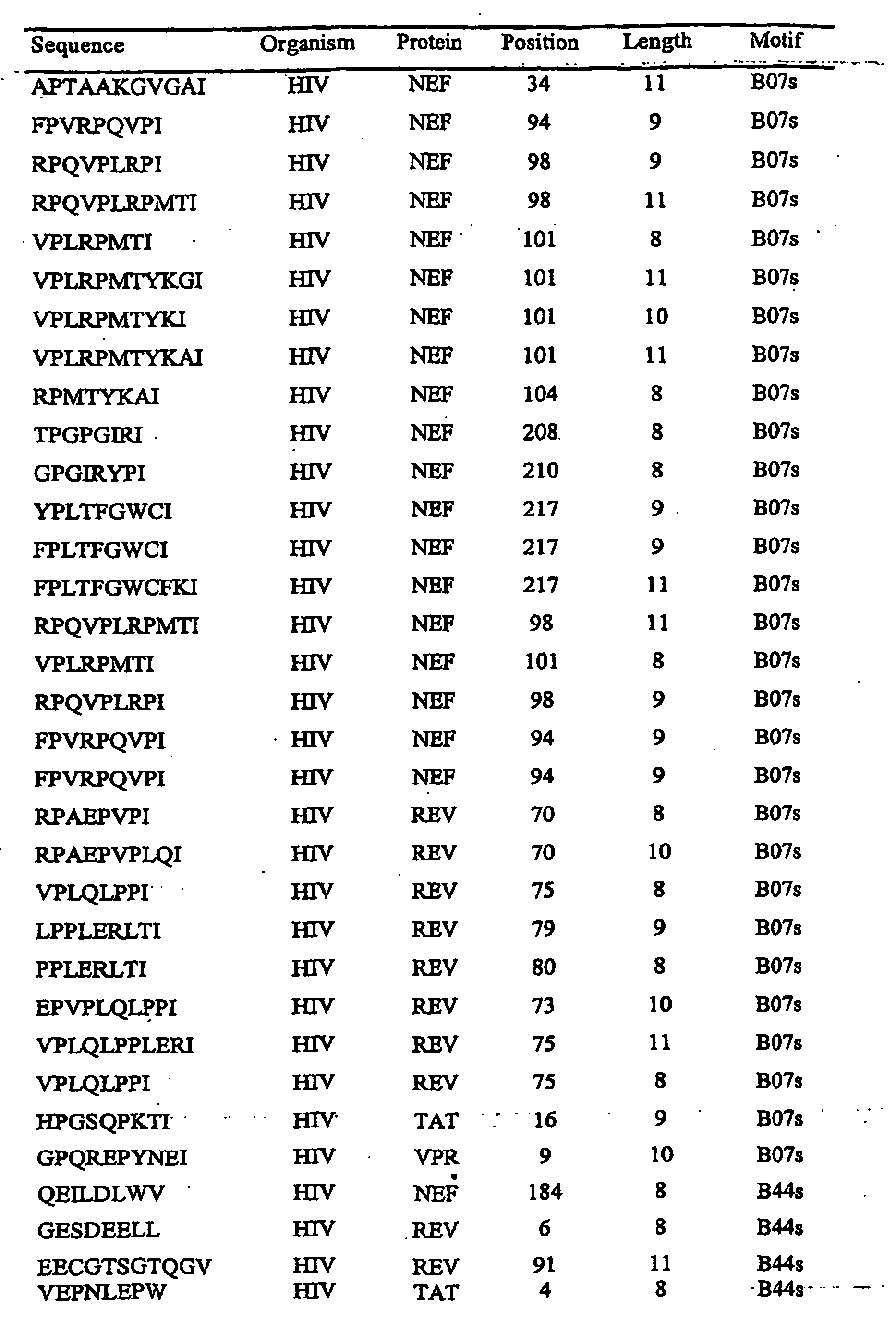

**Table 11**

| Motif | Parent source | Length | Sequence |
|---|---|---|---|
| A24i | Nef4 | 10 | KYSKSSIVGW |
| A24i | Nef 4 | 10 | KWSKSSIVGF |
| A01s | Nef 5 | 9 | WTKSSIVGW |
| A01s | Nef 5 | 9 | WSKSSIVGY |
| A02i/s | Nef 34 | 9 | QLEPAAAGV |
| A02i/s | Nef 34 | 11 | QLEPAAAGVGA |
| A02i/s | Nef 34 | 11 | QAEPAAAGVGV |
| B07s | Nef 34 | 8 | APTAAKGI |
| B07s | Nef 34 | 10 | APTAAKGVGI |
| B07s | Nef 34 | 11 | APTAAKGVGAI |
| A02i/s | Nef 41 | 8 | PLAEGVGA |
| A02i/s | Nef 41 | 8 | PAAEGVGV |
| A02i/s | Nef 42 | 8 | ALEGVGAV |
| A02i/s | Nef 45 | 9 | GLGAVSRDL |
| A02i/s | Nef 45 | 9 | GVGAVSRDV |
| A24s | Nef 45 | 9 | GYGAVSQDL |
| A24s | Nef 45 | 9 | GVGAVSQDF |
| A03i | Nef 51 | 8 | RVLEKHGA |
| A03i | Nef 51 | 8 | RDLEKHGK |
| A03i | Nef 61 | 10 | HVAITSSNTA |
| A03i | Nef 61 | 10 | HGAITSSNTK |
| A02i/s | Nef 62 | 8 | GLITSSNT |
| A02i/s | Nef 62 | 8 | GAITSSNV |
| A02i/s | Nef 62 | 9 | GLITSSNTA |
| A02i/s | Nef 62 | 9 | GAITSSNTV |
| A02i/s | Nef 63 | 8 | ALTSSNTA |
| A02i/s | Nef 63 | 8 | AITSSNTV |
| A02i/s | Nef 73 | 9 | NLDCAWLEA |
| A02ils | Nef 73 | 9 | NADCAWLEV |
| A03i | Nef 74 | 8 | AVCAWLEA |
| A03i | Ref 74 | 8 | ADCAWLEK |
| A02i/s | Nef 82 | 8 | ELQEEEEV |
| A03i | Nef 82 | 10 | EVQEEEEVGF |
| A03i | Nef 82 | 10 | EAQEEEEVGK |
| A02i/s | Nef 83 | 11 | ALEEEEVGFPV |
| A02i/s | Nef 91 | 10 | ELGFPVRPQV |
| A03i | Nef 93 | 11 | GVPVRPQVPLR |
| A03i | Nef 93 | 11 | GFPVRPQVPLK |
| A24i | Nef 93 | 10 | GYPVRPQVPL |
| A24i | Nef 93 | 10 | GFPVRPQVPF |
| B07s | Nef 94 | 9 | FPVRPQVPI |
| A02i/s | Nef 95 | 8 | PLRPQVPL |
| A02i/s | Nef 95 | 8 | PVRPQVPV |
| A02i/s | Nef 95 | 11 | PLRPQVPLRPM |
| A02i/s | Nef 95 | 11 | PVRPQVPLRPV |
| A03i | Nef 95 | 9 | PVRPQVPLK |
| B07s | Nef 98 | 9 | RPQVPLRPI |
| B07s | Nef 98 | 11 | RPQVPLRPMTI |
| A02i/s | Nef 99 | 8 | PLVPLRPM |
| A02i/s | Nef 99 | 8 | PQVPLRPV |
| A02i/s | Nef 99 | 9 | PLVPLRPMT |
| A02i/s | Nef 99 | 9 | PQVPLRPMV |
| A01s | Nef 100 | 9 | QTPLRPMTY |
| A02i/s | Nef 100 | 8 | QLPLRPMT |
| A02i/s | Nef 100 | 8 | QVPLRPMV |
| A02i/s | Nef 100 | 11 | QLPLRPMTYKA |
| A02i/s | Nef 100 | 11 | QVPLRPMTYKV |
| B07s | Nef 101 | 8 | VPLRPMTI |
| B07s | Nef 101 | 11 | VPLRPMTYKGI |
| B07s | Nef 101 | 10 | VPLRPMTYKI |
| B07s | Nef 101 | 11 | VPLRPMTYKAI |
| A02i/s | Nef 102 | 10 | PLRPMTYKGV |
| A02i/s | Nef 102 | 9 | PLRPMTYKV |
| A02i/s | Nef 102 | 10 | PLRPMTYKAV |
| A03i | Nef 102 | 8 | PVRPMTYK |
| B07s | Nef 104 | 8 | RPMTYKAI |
| A03i | Nef 111 | 9 | AVDLSFFLK |
| A03i | Nef 111 | 11 | AVDLSFFLKEK |
| A24i | Nef 111 | 8 | AYDLSFFL |
| A24i | Nef 111 | 8 | AFDLSFFF |
| A03i | Nef 112 | 8 | FVLSFFLK |
| A03i | Nef 112 | 10 | FVLSFFLKEK |
| A03i | Nef 113 | 9 | DVSHFLKBK |
| A03i | Nef 113 | 9 | DVSFFLKEK |
| A03i | Nef 114 | 8 | LVHFLKEK |
| A03i | Nef 114 | 8 | LVFFLKEK |
| A24i | Nef 115 | 10 | SYFLKEKGGL |
| A24i | Nef 115 | 10 | SFFLKEKGGF |
| A24i | Nef 116 | 9 | HYLKEKGGL |
| A24i | Nef 116 | 9 | HFLKEKLGGF |
| A24i | Nef 116 | 9 | FYLKEKGGL |
| A24i | Nef 116 | 9 | FFLKEKGGF |
| A02i/s | Nef 117 | 8 | FLKEKGGV |
| A02i/s | Nef 117 | 11 | FLKEKGGLEGV |
| A02i/s | Nef 117 | 11 | FLKEKGGLDGV |
| A03i | Nef 122 | 9 | KVGLEGLIY |
| A03i | Nef 122 | 9 | KGGLEGLIK |
| A03i | Nef 124 | 8 | GVLEGLIY |
| A03i | Nef 124 | 8 | GGLEGLIK |
| A03i | Nef 124 | 10 | GVLDGLIYSK |
| A03i | Nef 125 | 9 | GVDGLIYSK |
| A03i | Nef 171 | 8 | LVGLIYSK |
| A02i/s | Nef 173. | 11 | GLIYSKKRQEV |
| A03i | Nef 173 | 8 | GVIYSKKR |
| A03i | Nef 173 | 8 | GLIYSKKK |
| A02i/s | Nef 174 | 10 | LLYSICKRQEI |
| A02i/s | Nef 174 | 10 | LIYSKKRQEV |
| A02i/s | Nef 174 | 11 | LLYSKKRQEIL |
| A02i/s | Nef 174 | 11 | LIYSKKRQEIV |
| A24i | Nef 175 | 9 | IYSKKRQEF |
| A24i | Nef 175 | 10 | IYSKKRQEIF |
| A01i | Nef 182 | 10 | RTEILDLWVY |
| A01i | Nef 182 | 10 | RQDILDLWVY |
| A01i | Nef 182 | 10 | RTDILDLWVY |
| A02i/s | Nef 182 | 9 | RLEILDLWV |
| A02i/s | Nef 182 | 9 | RLDILDLWV |
| A03i | Nef 184 | 9 | QVILDLWVY |
| A03i | Nef 184 | 9 | QDILDLWVK |
| B44s | Nef 184 | 8 | QEILDLWV |
| A01s | Nef 185 | 8 | ETLDLWVY |
| A01s | Nef 185 | 8 | DTLDLWVY |
| A02i/s | Nef 185 | 10 | ELLDLWVYHT |
| A02i/s | Nef 185 | 10 | EILDLWVYHV |
| A03i | Nef 185 | 9 | EVLDLWVYH |
| A03i | Nef 185 | 9 | EILDLWVYK |
| A02i/s | Nef 186 | 9 | ILDLVWYHV |
| A02i/s | Nef 186 | 9 | ILDLWVYNV |
| A03i | Nef 186 | 8 | IVDLWVYH |
| A03i | Nef 186 | 8 | ILDLWVYK |
| A03i | Nef 187 | 11 | LVLWVYHTQGY |
| A03i | Nef 187 | 11 | LDLWVYHTQGK |
| A01s | Nef 188 | 10 | DTWVYHTQGY |
| A01s | Nef 188 | 11 | DTWVYHTQGYF |
| A01s | Nef 188 | 11 | DLWVYHTQGYY |
| A24i | Nef 190 | 10 | LYVYHTQGYF |
| A24s | Nef 190 | 9 | LYVYHTQGY |
| A24s | Nef 190 | 9 | LWVYHTQGF |
| A01s | Nef 191 | 8 | WTYHTQGY |
| A01s | Nef 191 | 9 | WTYHTQGYF |
| A01s | Nef 191 | 9 | WVYHTQGYY |
| A24i | Nef 192 | 11 | VYHTQGYFPDF |
| A03i | Nef 196 | 10 | QVFFPDWQNY |
| A03i | Nef 196 | 10 | QGFFPDWQNK |
| A03i | Nef 198 | 9 | GVFPDWQNY |
| A03i | Nef 198 | 9 | GFFPDWQNK |
| A03i | Nef 199 | 8 | FVPDWQNY |
| A03i | Nef 199 | 8 | FFPDWQNK |
| A24i | Nef 203 | 11 | DYQNYTPGPGI |
| A24i | Nef 203 | 11 | DWQNYTPGPGF |
| A02i/s | Nef 204 | 10 | WLNYTPGPGT |
| A02i/s | Nef 204 | 10 | WQNYTPGPGV |
| A02i/s | Nef 204 | 10 | WLNYTPGPGI |
| A02i/s | Nef 204 | 10 | WQNYTPGPGV |
| A11i | Nef 205 | 10 | QVYTPGPGTR |
| A11i | Nef 205 | 10 | QNYTPGPGTK |
| A11i | Nef 205 | 10 | QVYTPGPGIR |
| A11i | Nef 205 | 10 | QNYTPGPGIK |
| A24i | Nef 206 | 8 | NYTPGPGF |
| A24s | Nef 206 | 10 | NYTPGPGIRF |
| A01i | Nef 207 | 9 | YTDGPGIRY |
| A02i/s | Nef 207 | 11 | YLPGPGIRYPL |
| A02i/s | Nef 207 | 11 | YTPGPGIRYPV |
| A03i | Nef 207 | 8 | YTPGPGTK |
| A03i | Nef 207 | 8 | YTPGPGIK |
| B07s | Nef 208 | 8 | TPGPGIRI |
| B07s | Nef 210 | 8 | GPGIRYPI |
| A03i | Nef 216 | 10 | RVPLTFGWCF |
| A03i | Nef 216 | 10 | RFPLTFGWCK |
| A03i | Nef 216 | 11 | RVPLTFGWCFK |
| A24i | Nef 216 | 8 | RYPLTFGF |
| A24i | Nef 216 | 8 | RYPLTFGW |
| A24i | Nef 216 | 8 | RFPLTFGF |
| B07s | Nef 217 | 9 | YPLTFGWCI |
| B07s | Nef 217 | 9 | FPLTFGWCI |
| B07s | Nef 217 | 11 | FPLTFGWCFKI |
| A01s | Nef 219 | 8 | PTTFGWCF |
| A01s | Nef 219 | 8 | PLTFGWCY |
| A02i/s | Nef 219 | 10 | PLTFGWCFKV |
| A03i | Nef 219 | 9 | PVTFGWCFK |
| A02i/s | Nef 221 | 9 | LLFGWCFKL |
| A02i/s | Nef 221 | 9 | LTFGWCFKV |
| A02i/s | Nef 221 | 10 | LLFGWCFKLV |
| A24i | Nef 222 | 8 | TYGWCFKL |
| A24i | Nef 222 | 8 | TFGWCFKF |
| A03i | Nef 257 | 8 | LVHPICQH |
| A03i | Nef 257 | 8 | LLHPICQK |
| A03i | Nef 257 | 8 | LVHPMSQH |
| A03i | Nef 257 | 8 | LLHPMSQK |
| A01i | Nef 322 | 8 | ATELHPEY |
| A01i | Nef 322 | 8 | ARDLHPEY |
| A01i | Nef 322 | 9 | ATELHPEYY |
| A01i | Nef 322 | 9 | ARDLHPEYY |
| A03s | Nef 324 | 8 | EVHPEYYK |
| A01s | Nef 185 | 8 | ETLDLWVY |
| B07s | Nef 98 | 11 | RPQVPLRPMTI |
| A01s | Nef 100 | 9 | QTPLRPMTY |
| B07s | Nef 101 | 8 | VPLRPMTI |
| B07s | Nef 98 | 9 | RPQVPLRPI |
| A02i/s | Nef 95 | 8 | PVRPQVPV |
| A24s | Nef 95 | 8 | PYRPQVPL |
| B07s | Nef 94 | 9 | FPVRPQVPI |
| B07s | Nef 94 | 9 | FPVRPQVPI |
| A03i | Nef 95 | 9 | PTRPQVPLR |
| A03s | Nef 95 | 9 | PTRPQVPLR |
| A11i | Nef 95 | 9 | PTRPQVPLR |
| A24i | Nef 93 | 10 | GFPVRPQVPF |
| A24s | Nef 93 | 10 | GYPVRPQVPL |
| A03i | Nef 93 | 11 | GTPVRPQVPLR |
| A11i | Nef 93 | 11 | GTPVRPQVPLR |
| A03i | Nef 102 | 8 | PTRPMTYK |
| A03s | Nef 102 | 8 | PTRPMTYK |
| A11i | Nef 102 | 8 | PTRPMTYK |
| A02i/s | Nef 117 | 8 | FLKEKGGV |
| A24s | Nef 117 | 8 | FYKBKGGL |
| A02i/s | Nef 99 | 8 | PQVPLRPV |
| A02i/s | Nef 99 | 9 | PQVPLRPMV |
| A02i/s | Nef 100 | 8 | QVPLRPMV |
| A02i/s | Nef 95 | 8 | PLRPQVPL |
| A24i | Nef 93 | 10 | GYPVRPQVPL |
| A02i/s | Nef 117 | 8 | FVKEKGGL |
| A02i/s | Nef 99 | 8 | PLVPLRPM |
| A02i/s | Nef 99 | 9 | PLVPLRPMT |
| A02i/s | Nef 100 | 8 | QLPLRPMT |
| A02i/s | Nef 99 | 8 | PVVPLRPM |
| A02i/s | Nef 99 | 9 | PVVLRPMT |
| B44s | Rev 6 | 8 | GESDEELL |
| A01 s | Rev 20 | 11 | ITKILYQSNPY |
| A01s | Rev 22 | 9 | KTLYQSNPY |
| A01s | Rev 23 | 8 | ITYQSNPY |
| A03i | Rev 35 | 8 | EVTRQARR |
| A03i | Rev 35 | 8 | EGTRQARK |
| A03i | Rev 35 | 10 | EVTRQARRNR |
| A03i | Rev 35 | 10 | EGTRQARRNK |
| A03i | Rev 35 | 11 | EVTRQARRNRR |
| A03i | Rev 35 | 11 | EGTRQARRNRK |
| A03i | Rev 36 | 9 | GTRQARRNK |
| A03i | Rev 36 | 10 | GTRQARRNRK |
| A03i | Rev 36 | 11 | GTRQARRNRRK |
| A03i | Rev 37 | 9 | GTRQTRKNK |
| A03i | Rev 37 | 10 | GTRQTRKNRK |
| A03i | Rev 37 | 11 | GTRQTRKNRRK |
| A03i | Rev 37 | 11 | TTRQARRNRRK |
| A03i | Rev 40 | 8 | QVRRNRRR |
| A03i | Rev 40 | 8 | QARRNRRK |
| A03i | Rev 40 | 9 | QVRRNRRRR |
| A03i | Rev 40 | 9 | QARRNRRRK |
| A03i | Rev 40 | 11 | QVRRNRRRRWR |
| A03i | Rev 40 | 11 | QARRNRRRRWK |
| A03i | Rev 40 | 8 | QVRKNRRR |
| A03i | Rev 40 | 8 | QARKNRRK |
| A03i | Rev 40 | 9 | QVRKNRRRR |
| A03i | Rev 40 | 9 | QARKNRRRK |
| A03i | Rev 40 | 11 | QVRKNRRRRWR |
| A03i | Rev 40 | 11 | QARKNRRRRWK |
| A11i | Rev 43 | 8 | RVRRRRWR |
| A11i | Rev 43 | 8 | RNRRRRWK |
| A11i | Rev 43 | 10 | RVRRRRWRAR |
| A11i | Rev 43 | 10 | RNRRRRWRAK |
| A11i | Rev 43 | 8 | KVRRRRWR |
| A11i | Rev 43 | 8 | KNRRRRWK |
| A11i | Rev 43 | 10 | KVRRRRWRAR |
| A11i | Rev 43 | 10 | KNRRRRWRAK |
| A24i | Rev 48 | 9 | RYRARQRQI |
| A24i | Rev 48 | 9 | RWRARQRQF |
| B07s | Rev 70 | 8 | RPAEPVPI |
| B07s | Rev 70 | 10 | RPAEPVPLQI |
| A02i/s | Rev 71 | 9 | PLEPVPLQL |
| A02i/s | Rev 71 | 9 | PAEPVPLQV |
| A02i/s | Rev 74 | 9 | PLPLQLPPL |
| A02i/s | Rev 74 | 9 | PVPLQLPPV |
| A03i | Rev 74 | 11 | PVPLQLPPLEK |
| B07s | Rev 75 | 8 | VPLQLPPI |
| A02i/s | Rev 76 | 10 | PLQLPPLERV |
| A03i | Rev 76 | 9 | PVQLPPLER |
| A03i | Rev 76 | 9 | PLQLPPLEK |
| A02i/s | Rev 77 | 9 | LLLPPLERL |
| A02i/s | Rev 77 | 9 | LQLPPLERV |
| A02i/s | Rev 77 | 10 | LLLPPLERLT |
| A02i/s | Rev 77 | 10 | LQLPPLERLV |
| A02i/s | Rev 77 | 11 | LLLPPLERLTL |
| A02i/s | Rev 77 | 11 | LQLPPLERLTV |
| A02i/s | Rev 78 | 9 | QLPPLERLV |
| A24s | Rev 78 | 8 | QYPPLERL |
| A24s | Rev 78 | 8 | QLPPLERF |
| A24s | Rev 78 | 10 | QYPPLERLTL |
| A24s | Rev 78 | 10 | QLPPLERLTF |
| B07s | Rev 79 | 9 | LPPLERLTI |
| B07s | Rev 80 | 8 | PPLERLTI |
| B44s | Rev 91 | 11 | EECGTSGTQGV |
| A02i/s | Rev 94 | 8 | GLSGTQGV |
| B07s | Rev 73 | 10 | EPVPLQLPPI |
| B07s | Rev 75 | 11 | VPLQLPPLERI |
| B07s | Rev 75 | 8 | VPLQLPPI |
| A01s | Tat 3 | 9 | PTDPNLEPW |
| A01s | Tat 3 | 9 | PVDPNLEPY |
| A03i | Tat 4 | 10 | VVPNLEPWNH |
| A03i | Tat 4 | 10 | VDPNLEPWNK |
| B44s | Tat 4 | 8 | VEPNLEPW |
| A11i | Tat 9 | 8 | PVLEPWNH |
| A11i | Tat 9 | 8 | PNLEPWNK |
| B07s | Tat 16 | 9 | HPGSQPKTI |
| A03i | Tat 17 | 8 | PVSQPKTA |
| A03i | Tat 17 | 8 | PGSQPKTK |
| A03i | Tat 23 | 9 | TVCNNCYCK |
| A03i | Tat 24 | 8 | AVNNCYCK |
| A03i | Tat 46 | 11 | LVISYGRKKRR |
| A03i | Tat 46 | 11 | LGISYGRKKRK |
| A03i | Tat 48 | 11 | IVYGRKKRRQR |
| A03i | Tat 48 | 11 | ISYGRKKRRQK |
| A03i | Tat 50 | 9 | YVRKKRRQR |
| A03i | Tat 50 | 9 | YGRKKRRQK |
| A03i | Tat 50 | 10 | YVRKKRRQRR |
| A03i | Tat 50 | 10 | YGRKKRRQRK |
| A03i | Tat 50 | 11 | YVRKKRRQRRR |
| A03i | Tat 50 | 11 | YGRKKRRQRRK |
| A03i | Tat 84 | 8 | RVDPTGPK |
| A03i | Tat 89 | 8 | TVPKESKK |
| A03i | Tat 101 | 10 | ETGPSGQPCK |
| A03i | Tat 101 | 10 | KVGPGGYPRR |
| A03i | Tat 101 | 10 | KAGPGGYPRK |
| A03i | Tat 101 | 11 | KVGPGGYPRRK |
| A03i | Tat 102 | 9 | AVPGGYPRR |
| A03i | Tat 102 | 9 | AGPGGYPRK |
| A03i | Tat 102 | 9 | TVPSGQPCH |
| A03i | Tat 102 | 9 | TGPSGQPCK |
| A03i | Tat 102 | 10 | AVPGGYPRRK |
| A03i | Tat 104 | 8 | PVGYPRRK |
| A03i | Tat 105 | 11 | GVYPRRKGSCH |
| A03i | Tat 105 | 11 | GGYPRRKGSCK |
| A03i | Tat 44 | 9 | KTLGISYGR |
| A11i | Tat 44 | 9 | KTLGISYGR |
| A03i | Tat 44 | 10 | KTLGISYGRK |
| A11 i | Tat 44 | 10 | KTLGISYGRK |
| A03i | Tat 45 | 11 | GTGISYGRKKR |
| A03s | Tat 45 | 11 | GTGISYGRKKR |
| A11i | Tat 45 | 11 | GTGISYGRKKR |
| A03i | Tat 44 | 11 | KTLGISYGRKK |
| A11i | Tat 44 | 11 | KTLGISYGRKK |
| A03i | Tat 45 | 8 | GTGISYGR |
| A03s | Tat 45 | 8 | GTGISYGR |
| A11i | Tat 45 | 8 | GTGISYGR |
| A03i | Tat 45 | 9 | GTGISYGRK |
| A03s | Tat 45 | 9 | GTGISYGRK |
| A11i | Tat 45 | 9 | GTGISYGRK |
| A03i | Tat 45 | 10 | GTGISYGRKK |
| A03s | Tat 45 | 10 | GTGISYGRKK |
| A11i | Tat 45 | 10 | GTGISYGRKK |
| A03i | Tat 47 | 9 | GTSYGRKKR |
| A03s | Tat 47 | 9 | GTSYGRKKR |
| A11i | Tat 47 | 9 | GTSYGRKKR |
| A03i | Tat 46 | 10 | LTISYGRKKR |
| A11i | Tat 46 | 10 | LTISYGRKKR |
| A03i | Tat 47 | 8 | GTSYGRKK |
| A03s | Tat 47 | 8 | GTSYGRKK |
| A11i | Tat 47 | 8 | GTSYGRKK |
| A03i | Tat 48 | 8 | ITYGRKKR |
| A03s | Tat 48 | 8 | ITYGRKKR |
| A11i | Tat 48 | 8 | ITYGRKKR |
| A03i | Tat 46 | 8 | LTISYGRK |
| A11i | Tat 46 | 8 | LTISYGRK |
| A03i | Tat 46 | 9 | LTISYGRKK |
| A11i | Tat 46 | 9 | LTISYGRKK |
| A01s | Vif 23 | 8 | STVKHHMY |
| A02i/s | Vif 11 | 8 | WQVDRMRV |
| A24i | Vif 10 | 9 | VYQVDRMRI |
| A24s | Vif 10 | 9 | VWQVDRMRF |
| A02i/s | Vif 146 | 10 | KVGSLQYLAV |
| A24s | Vif 146 | 10 | KYGSLQYLAL |
| A02i/s | Vif 146 | 8 | KVGSLQYV |
| A24s | Vif 146 | 8 | KYGSLQYL |
| A02i/s | Vif 146 | 9 | KVGSLQYLV |
| A03i | Vif 146 | 9 | KTGSLQYLA |
| A03i | Vif 147 | 8 | VTSLQYLA |
| A02i/s | Vif 9 | 8 | IVWQVDRV |
| A24s | Vif 9 | 8 | IYWQVDRM |
| A02i/s | Vif 11 | 8 | WLVDRMRI |
| A24i | Vif 10 | 9 | VWQVDRMRF |
| A02i/s | Vif 146 | 10 | KLGSLQYLAL |
| A02i/s | Vif 146 | 8 | KLGSLQYL |
| A02i/s | Vif 146 | 9 | KLGSLQYLA |
| A03i | Vif 146 | 9 | KVGSLQYLK |
| A03i | Vif 147 | 8 | VGSLQYLK |
| A02i/s | Vif 9 | 8 | ILWQVDRM |
| A24s | Vif 9 | 8 | IVWQVDRF |
| A02i/s | Vif 11 | 8 | WVVDRMRI |
| B07s | Vpr 9 | 10 | GPQREPYNEI |
| A01i | Vpr 5 | 11 | PTDQGPQREPY |
| A03i | Vpr 29 | 8 | ETVRHFPR |
| A03s | Vpr 29 | 8 | ETVRHFPR |
| A11i | Vpr 29 | 8 | ETVRHFPR |

**Table 12**

| | | | | HLA- A1 supertype binding affinity (IC50 nM) | | |
|---|---|---|---|---|---|---|
| Peptide | Sequence | Source | Analog | A*0101 | A*2902 | A*3002 |
| 57.0007 | AADNPPAQY | .CEA.261 | A | 9.2 | | |
| 57.0026 | GGDVENPEY | .Her2/neu.1188 | A | 399 | | |
| 57.0021 | VTAGVGSPY | .Her2/neu.773 | A | 269 | | |
| 1074.17 | AC-YTAVVPLVY | Jchain. | A | 37 | | |
| 1074.20 | AYTAVVPLVY | .Jchain. | A | 455 | | |
| 1074.19 | RYTAVVPLVY | .Jchain. | A | >296.17 | | |
| 1489.15 | LVTCLGLSY | .MAGE2.178 | | 4132 | 18 | 4101 |
| 1489.16 | LVQENYLEY | .MAGE2.250 | | 48 | 13 | 72 |
| 57.0029 | STFSTTINY | .MAGE2.69 | A | 114 | | |
| 1461.06 | MEVDPIGHLY | .MAGE3.167 | | 371 | 333 | 87 |
| 1489.18 | FVQENYLEY | .MAGE3.250 | | 26 | 3.2 | 99 |
| 26.0224 | SAEQSPPPY | .MART1.108 | | 269 | | |
| 57.0128 | VGDDCTTIHY | .p53.225 | A | 465 | | |
| 1044.12 | SEVDPIGHLY | .PAP.135 | A | 395 | | |
| 26.0231 | SMHNALHIY | .Tyrosinase.361 | | 345 | | |
| 26.0484 | ISSKDLGYDY | .Tyrosinase.440 | | 185 | | |
| 954.06 | AADAAAAKAY | Artificial sequence | PolyA | 107 | | |
| 21.0009 | AADAAAAKY | Artificial sequence | PolyA | 20 | | |
| 954.07 | AAEAAAAKY | Artificial sequence | PolyA | 149 | | |
| 954.04 | AAEKAAAAAY | Artificial sequence | PolyA | 236 | | |
| 954.10 | ALAKAAAAAY | Artificial sequence | PolyA | 456 | | |
| 954.20 | ALEKAAAAAY | Artificial sequence | PolyA | 173 | | |
| 954.19 | ALEKAAAAY | Artificial sequence | PolyA | 151 | | |
| 26.0033 | GMQNIYIKY | Artificial sequence | | 294 | | |
| 26.0034 | ITYFKKIYY | Artificial sequence | | 285 | | |
| 962.13 | YTAQAAAKF | Artificial sequence | Poly | 127 | | |
| 1489.10 | LHGPTPLLY | HCV.II.1623 | | >34879.01 | 180 | 1075 |
| 1489.11 | AQPGYPWPLY | HCV.II.77 | | >31382.68 | 133 | 3.5 |
| 1489.06 | AATLGFGAY | HCV.IV.1264 | | 44768 | 2240 | 491 |
| 1489.12 | TCGFADLMGY | HCV.IV.127 | | 2321 | 350 | 356 |
| 1489.05 | CSFSIFLLA | HCV.IV.172 | | 28906 | 153 | 11100 |
| 78.0359 | RFHNIRGRW | HPV.16.E6.131 | | 53191 | 18 | 58 |
| 78.0001 | IHDIILECVY | HPV.16.E6.30 | | 316 | 8873 | 8939 |
| 78.0002 | YSKISEYRHY | HPV.16.E6.77 | | 151 | 6448 | 205 |
| 78.0177 | EYRHYCYSLY | HPV.16.E6.82 | | 2564 | 2449 | 120 |
| 78.0006 | LQDIEITCVY | HPV.18.E6.25 | | 1517 | 4044 | 148 |
| 78.0005 | RFEDPTRRPY | HPV.18.E63 | | 19231 | 17839 | 145 |
| 78.0361 | LFVVYRDSI | HPV.18.E6.52 | | 20921 | 464 | 100 |
| 78.0363 | RFHNIGGRW | HPV.31.E6.124 | | >35714.29 | 1595 | 9.5 |
| 78.0009 | YSKVSEFRWY | HPV.31.E6.70 | | 539 | 4514 | 185 |
| 78.0366 | RFHNISGRW | HPV.33.E6.124 | | 48544 | 174 | 37 |
| 78.0364 | VYDFAFADL | HPV.33.E6.42 | | 19763 | 163 | 579 |
| 78.0365 | RFLSKISEY | HPV.33.E6.68 | | >35714.29 | 34623 | 23 |
| 78.0243 | ISEYRHYNY | HPV.33.E6.73 | | 25 | 1329 | 32 |
| 78.0180 | EYRHYNYSVY | HPV.33.E6.75 | | 31447 | 16016 | 253 |
| 78.0348 | LYPEPTDLY | HPV.33.E7.15 | | 31 | 36 | 71 |
| 78.0244 | LKEYVLDLY | HPV.33.E7.8 | | 3270 | 127123 | 16 |
| 78.0246 | ATLERTEVY | HPV.45.E6.37 | | 35 | 11383 | 175 |
| 78.0368 | AFKDLCIVY | HPV.45.E6.48 | | >35714.29 | 32 | 0.49 |
| 78.0349 | AYAACHKCI | HPV.45.E6.61 | | 32895 | 3300 | 139 |
| 78.0182 | FYSRIRELRY | HPV.45.E6.71 | | 10142 | 20 | 150 |
| 78.0247 | YSRIRELRY | HPV.45.E6.72 | | 41 | 1991 | 22 |
| 78.0369 | VYGETLEKI | HPV.45.E6.85 | | >35714.29 | 12156 | 13 |
| 78.0017 | ELDPVDLLCY | HPV.45.E7.20 | | 26 | 4291 | 12746 |
| 78.0371 | VYKFLFML | HPV.52.E6.42 | | >35714.29 | 1707 | 27 |
| 78.0372 | LFTDLRIVY | HPV.52.E6.46 | | >35714.29 | 25003 | 22 |
| 78.0019 | ISKISEYRHY | HPV.52.E6.70 | | 14327 | 55190 | 186 |
| 78.0248 | ISEYRHYQY | HPV.52.E6.73 | | 283 | >84107.89 | 3765 |
| 78.0183 | EYRHYQYSLY | HPV.52.E6.75 | | 2268 | 2372 | 130 |
| 78.0249 | QAEQATSNY | HPV.52.E7.46 | | 74 | 4203 | 109 |
| 78.0022 | QAEQATSNYY | HPV.52.E7.46 | | 101 | 39409 | 1436 |
| 78.0023 | ATSNYYIVTY | HPV.52.E7.50 | | 2209 | 2117 | 118 |
| 78.0250 | TSNYYIVTY | HPV.52.E7.51 | | >74955.86 | 3313 | 76 |
| 78.0376 | NFACTELKL | HPV.56.E6.47 | | >35971.22 | 202 | 1.0 |
| 1488.11 | AVCRVCLLFY | HPV.56.E6.64 | | 105 | | |
| 78.0378 | LFYSKVRKY | HPV.56.E6.71 | | 1262 | 12 | 89 |
| 78.0176 | FYSKVRKYRY | HPV.56.E6.72 | | >32894.74 | 146 | 326 |
| 1497.15 | KYRYYDYSVY | HPV.56.E6.78 | | | 17797 | 2.3 |
| 78.0379 | DYSVYGATL | HPV.56.E6.83 | | >35971.22 | 1460 | 19 |
| 78.0252 | LCDLLIRCY | HPV.56.E6.99 | | 656 | 557 | 26 |
| 78.0384 | RFHNISGRW | HPV.58.E6.124 | | >35971.22 | 201 | 35 |
| 78.0253 | KTLQRSEVY | HPV.58.E6.35 | | 308 | 6276 | 566 |
| 78.0381 | VYDFVFADL | HPV.58.E6.42 | | >35971.22 | 27168 | 27 |
| 78.0027 | LSKISEYRHY | HPV.58.E6.70 | | 17182 | 101409 | 159 |
| 78.0254 | ISEYRHYNY | HPV.58.E6.73 | | 46 | 853 | 81 |
| 78.0354 | EYRHYNYSL | HPV.58.E6.75 | | 54348 | 268 | 1473 |
| 78.0185 | EYRHYNYSLY | HPV.58.E6.75 | | 2405 | 1774 | 67 |
| 26.0271 | RMAWHAAGTY | MT.104 | | 423 | | |
| 26.0075 | VTPEQRPLY | MT.1752 | | 331 | | |
| 26.0283 | QMGLGW1GSSY | MT.294 | | 340 | | |
| 26.0037 | RTGLYGAQY | MT.344 | | 116 | | |
| 1489.22 | ALFQEYQCY | Pf.CSP.18 | | >38534.16 | 149 | 1032 |
| 1489.55 | YYGKQENWY | Pf.CSP.55 | | | 373 | 3155 |
| 1489.61 | FYFILVNLI | Pf.LSA1.9 | | | 362 | 8591 |
| 1489.27 | FVVPGAATPY | Pf.SSP2.520 | | 31338 | 317 | 4621 |

**TABLE 13**

| Peptide | Sequence | AA | Source | Analog | A*0101 (IC50 nM) |
|---|---|---|---|---|---|
| 57.0007 | AADNPPAQY | 9 | CEA.261 | A | 46 |
| 57.0013 | AADNPPAQY | 9 | CEA.439 | A | 46 |
| 57.0106 | HSDSNPSPQY | 10 | CEA.616 | A | 46 |
| 57.0105 | HTASNPSPQY | 10 | CEA.616 | A | 134 |
| 57.0014 | ITDKNSGLY | 9 | CEA.467 | A | 12 |
| 57.0008 | ITDNNSGSY | 9 | CEA.289 | A | 110 |
| 57.0103 | PTDSPLNTSY | 10 | CEA.240 | A | 292 |
| 57.0011 | PTDSPSYTY | 9 | CEA.418 | A | 38 |
| 57.0104 | PTDSPSYTYY | 10 | CEA.418 | A | 2.0 |
| 57.0004 | QQDTPGPAY | 9 | CEA.87 | A | 57 |
| 57.0012 | TIDPSYTYY | 9 | CEA.419 | A | 3.5 |
| 57.0010 | VTDNDVGPY | 9 | CEA.383 | A | 4.5 |
| 26.0155 | WSQKRSVPY | 9 | gp100.143 | | 242 |
| 57.0018 | ATCVTACPY | 9 | Her2/neu.293 | A | 57 |
| 57.0024 | ATPLDSTFY | 9 | Her2/neu.997 | A | 40 |
| 57.0113 | CTQIAKGMSY | 10 | Her2/neu.826 | A | 20 |
| 57.0019 | ETDEEITGY | 9 | Her2/neu.401 | A | 18 |
| 57.0111 | ETMPNPEGRY | 10 | Her2/neu.280 | A | 4.1 |
| 57.0114 | FTDQSDVWSY | 10 | Her2/neu.899 | A | 0.64 |
| 57.0027 | FTPAFDNLY | 9 | Her2/neu.1213 | A | 9.1 |
| 57.0117 | FTPAFDNLYY | 10 | Her2/neu.1213 | A | 0.82 |
| 57.0017 | GTDLFEDNY | 9 | Her2/neu.104 | A | 0.91 |
| 57.0107 | GTDMKLRLPY | 10 | Her2/neu.28 | A | 50 |
| 57.0118 | GTDTAENPEY | 10 | Her2/neu.1239 | A | 27 |
| 57.0016 | HTDMLRHLY | 9 | Her2/neu.42 | A | 2.0 |
| 57.0023 | LTDIDETEY | 9 | Her2/neu.869 | A | 6.5 |
| 57.0025 | LTDSPQPEY | 9 | Her2/neu.1131 | A | 36 |
| 57.0116 | MTDLVDAEEY | 10 | Her2/neu.1014 | A | 2.5 |
| 57.0115 | PADPLDSTFY | 10 | Her2/neu.996 | A | 19 |
| 57.0129 | PTDCCHEQCAY | 11 | Her2/neu.232 | A | 18 |
| 57.0109 | PTDCCHEQCY | 10 | Her2/neu.232 | A | 47 |
| 57.0028 | SPDFDNLYY | 9 | Her2/neu.1214 | A | 73 |
| 57.0112 | TLDEITGYLY | 10 | Her2/neu.402 | A | 3.6 |
| 57.0022 | VMDGVGSPY | 9 | Her2/neu.773 | A | 41 |
| 57.0120 | ASDFSTTINY | 10 | MAGE2.68 | A | 24 |
| 57.0123 | ASDLPTTMNY | 10 | MAGE3.68. | A | 2.6 |
| 57.0119 | ATSFSTTINY | 10 | MAGE2.68 | A | 476 |
| 57.0122 | ATSLPTTMNY | 10 | MAGE3.68 | A | 214 |
| 57.0032 | GTVVGNWQY | 9 | MAGE3.137 | A | 37 |
| 57.0034 | ITGGPHISY | 9 | MAGE3.293 | A | 36 |
| 57.0124 | LTDHFVQENY | 10 | MAGE3.246 | A | 2.4 |
| 57.0121 | LTQDLVQENY | 10 | MAGE2.246 | A | 64 |
| 57.0030 | MTDLVQENY | 9 | MAGE2.247 | A | 0.79 |
| 57.0031 | STLPTTMNY | 9 | MAGE3.69 | A | 58 |
| 57.0003 | VTDLGLSY | 8 | MAGE2.179 | A | 2.7 |
| 26.0468 | LSAEQSPPPY | 10 | MART1.107 | | 35 |
| 57.0035 | GTDCTTIHY | 9 | p53.226 | A | 0.85 |
| 57.0126 | GTDKSVTCTY | 10 | p53.117 | A | 43 |
| 57.0125 | PTQKTYQGSY | 10 | p53.98 | A | 37 |
| 57.0127 | RVDGNLRVEY | 10 | p53.196 | A | 47 |
| 1381.01 | PSDKHIKEY | 9 | Pf.CSP.310 | | 27 |
| 26.0480 | DSDPDSFQDY | 10 | Tyrosinase.454 | | 5.5 |
| 1074.17 | YTAVVPLVY | 10 | Jchain. | A | 186 |
| F011.03 | ATDFKFAMY | 9 | Naturally processed | | 0.75 |
| F011.06 | DSDGSFFLY | 9 | Naturally processed | | 0.65 |
| F020.01 | DSGDSFFLY | 9 | Naturally processed | | 34 |
| F011.07 | GTDENRLLY | 9 | Naturally processed | | 1.3 |
| F011.01 | IADMGHLKY | 9 | Naturally processed | | 3.2 |
| F023.04 | YLDDPDLKY | 9 | Naturally processed | | 3.2 |
| F011.02 | YTDYGGLIFNSY | 12 | Naturally processed | | 2.0 |
| F029.03 | YTNPQFNVY | 9 | Naturally processed | | 25 |
| F011.04 | YTSDYFISY | 9 | Naturally processed | | 4.8 |
| F023.02 | YTSDYFYSY | 9 | Naturally processed | | 0.25 |
| 21.0009 | AADAAAAKY | 9 | Artificial sequence | Poly A | 101 |
| 954.05 | AADAAAAKY | 9 | Artificial sequence | Poly A | 64 |
| 962.10 | AADAKAAAY | 9 | Artificial sequence | Poly A | 95 |
| 954.18 | ALDKAAAAAY | 10 | Artificial sequence | Poly A | 31 |
| 954.17 | ALDKAAAAY | 9 | Artificial sequence | Poly A | 39 |
| 962.09 | ATDAKAAAY | 9 | Artificial sequence | Poly A | 6.9 |
| 982.18 | ATDKAAAAA | 9 | Artificial sequence | Poly | 73 |
| 962.08 | ATDPKAAAY | 9 | Artificial sequence | Poly A | 6.5 |
| 954.22 | ATEKAAAAAY | 10 | Artificial sequence | Poly A | 9.6 |
| 954.21 | ATEKAAAAY | 9 | Artificial sequence | Poly A | 6.2 |
| 962.14 | YTAQAAAKY | 9 | Artificial sequence | Poly | 4.2 |
| 1074.02 | YTAVVPLVF | 9 | Artificial sequence | A | 21 |
| 962.11 | YTDPKLINY | 9 | Artificial sequence | Poly | 8.2 |
| 962.12 | YTDQAVIKY | 9 | Artificial sequence | Poly | 2.8 |

**Table 14**

| | | | | HLA- A2 supertype binding affinity (IC50 nM) | | | | | |
|---|---|---|---|---|---|---|---|---|---|
| Peptide | Sequence | Source | Analog | A*0201 | A*0202 | A*0203 | A*0205 | A*0206 | A*6802 |
| 70.0094 | FLLAQFTSAI | HBV.Pol.503 | | 65 | 1.9 | 4.8 | | 148 | 533 |
| 70.0088 | FLPSDYFPSV | HBV.Core.18 | A | 8.5 | 3.3 | 3.2 | | 2.2 | 276 |
| 1369.03 | FVLSLGIHV | HBV.pol.562 | A | 45 | 400 | 2837 | 1414 | 133 | 62 |
| 1369.01 | GVLGWSPQV | HBV.env.62 | A | 22 | 157 | 393 | 37 | 28 | 9357 |
| 1369.02 | HVYSHPIIV | HBV.pol.1076 | A | 151 | 1921 | 14 | 11026 | 1200 | 122 |
| 1369.26 | IVRGTSFVYV | HBV.pol.773 | A | 36765 | 5298 | 68 | 1828 | 5386 | 1222 |
| 1369.13 | PVLPIFFCV | HBV.env.377 | A | 8.7 | 3140 | 13643 | 43000 | 22 | 1814 |
| 924.201 | QFLPSDFFPSV | HBV.core.27 | A | 394 | | | | | |
| 1369.14 | VVQAGFFLV | HBV.env.177 | A | 438 | 78 | 2484 | 785 | 81 | 624 |
| 1369.15 | YVDDVVLGV | HBV.pol.538 | A | 18 | 14 | 70 | 91 | 16 | 360 |
| 1489.30 | FLLALLSCLT | HCV.II.177 | | 90 | 21 | 65 | | 488 | 4235 |
| 1472.05 | RVLEDGVNYA | HCV.Core.156 | | 3785 | 24 | 3292 | | 39 | 190167 |
| 1327.12 | TLWARMILM | HCV.II.2871 | | 11 | 36 | 118 | | 204 | 519 |
| 1472.06 | VLEDGVNYA | HCV.Core.157 | | 2141 | 53 | 64 | | 10898 | 194602 |
| 1489.29 | YQATVCARA | HCV.IV.1594 | | 405 | 13 | 23 | | 118 | 4976 |
| 1482.09 | ALETSVHEI | HPV58.E6.18 | | 1514 | 248 | 317 | | 12546 | >447213.6 |
| 1491.78 | ALETSVHEV | HPV.58.E6.18 | A | 265 | 114 | 148 | | 2272 | 68376 |
| 1481.49 | ALETTIHNI | HPV33.E6.18 | | 2122 | 189 | 894 | | 2265 | 97129 |
| 1491.36 | ALETTIHNV | BPV.33.E6.18 | A | 286 | 34 | 272 | | 2070 | 42534 |
| 1482.04 | ALTVTCPLCA | HPV56.E7.93 | | 8382 | 1310 | 335 | | 157625 | >165379.65 |
| 1481.60 | AQPATADYYI | HPV33.E7.45 | | 17763 | 224 | 21164 | | 5272 | 318546 |
| 1482.20 | AQPATANYYI | HPV58.E7.46 | | 9604 | 141 | 3584 | | 2190 | 15996 |
| 1090.75 | CVYCKQQLL | HPV.16.E6.37 | | 38396 | 425 | 9535 | | 43026 | 9097 |
| 1481.10 | DLLMGTLGIV | HPV16.E7.81 | | 857 | 70 | 71 | | 607 | 12006 |
| 1481.42 | ELLMGSFGIV | HPV31.E7.81 | | 996 | 202 | 26 | | 273 | 10648 |
| 1481.13 | ELTEVFEFA | HPV18.E6.40 | | 8079 | 47 | 6241 | | 396 | 51 |
| 1481.76 | ELYNLLERCL | HPV45.E6.97 | | 11777 | 1351 | 425 | | 15935 | 2191 |
| 1481.86 | EVLEIPLIDL | HPV56.E6.20 | | 144749 | 2625 | 18829 | | 6013 | 484 |
| 1481.51 | EVYDFAFADL | HPV33.E6.41 | | 36182 | 16188 | 2710 | | 80 | 25 |
| 1482.11 | EVYDFVFADL | HPV58.E6.41 | | 49309 | 12065 | 3138 | | 60 | 9.8 |
| 1481.89 | FACTELKLV | HPV56.E6.48 | | 4173 | 3200 | 38 | | 186 | 62013 |
| 1481.52 | FAFADLTW | HPV33.E6.45 | | 42 | 16 | 5.3 | | 22 | 277 |
| 1481.70 | FAFKDLCIV | HPV45.E6.147 | | 142 | 1817 | 12 | | .62 | 2618 |
| 1481.14 | FAFKDLPVV | HPV18.E6.47 | | 20 | 191 | 5.8 | | 12 | 35 |
| 1481.32 | FAFTDLTIV | HPV31.E6.45 | | 137 | 75 | 10 | | 24 | 179 |
| 1491.61 | FLCTELKLV | HPV56.E6.48 | A | 247 | 88 | 143 | | 379 | 50150 |
| 1491.79 | FLFADLRIV | HPVS8.E6.45 | A | 32 | 9.4 | 5.7 | | 85 | 4744 |
| 1491.38 | FLPADLTVV | HPV.33.E6.45 | A | 12 | 0.3 | 2.5 | | 23 | 327 |
| 1491.48 | FLFKDLCIV | HPV.45.E6.47 | A | 12 | 1.3 | 5.5 | | 9.3 | 2446 |
| 1491.14 | FLFKDLFVV | HPV.18.E6.47 | A | 6.0 | 0.9 | 3.5 | | 8.4 | 274 |
| 1491.07 | FLFRDLCIV | HPV.16.E6.52 | A | 16 | 3.2 | 8.2 | | 50 | 582 |
| 1483.01 | FLFTDLRIV | HPV.52.E6.45 | | 18 | 1.7 | 3.3 | | 34 | 3803 |
| 1491.29 | FLFIDLTIV | HPV.31.E6.45 | A | 17 | 1.3 | 35 | | 20 | 1904 |
| 1491.18 | FLQLFLNTL | HPV.18.E7.86 | A | 17 | 1.4 | 225 | | 54 | 12994 |
| 1481.24 | FQQLFLNTL | HPV18.E7.86 | | 572 | 18 | 11 | | 20 | 280745 |
| 1491.19 | FQQLFLNTV | HPV.18.E7.86 | A | 104 | 18 | 2.7 | | 21 | 23131 |
| 1482.12 | FVFADLRIV | BPV58.E6.45 | | 98 | 98 | 4.0 | | 85 | 334 |
| 1482.03 | GALTVTCPL | HPV56.E7.92 | | 1271 | 1969 | 7183 | | 140 | 67111 |
| 1491.72 | GALTVTCPV | HPV.56.E7.92 | A | 281 | 3341 | 3059 | | 105 | 8374 |
| 1481.53 | GICKLCLRFL | HPV33.E6.61 | | 733 | 199 | 310 | | 2089 | 11432 |
| 1491.40 | GICICLCLRFV | HPV.33.E6.61 | A | 132 | 32 | 28 | | 404 | 19028 |
| 1491.39 | GLCKLCLRFL | HPV.33.E6.61 | A | 127 | 2.5 | 18 | | 4135 | 15406 |
| 1491.08 | GLLGIVCPI | HPV.161E7.85 | A | 13 | 3.0 | 14 | | 61 | 4758 |
| 1491.71 | GLLTVTCPL | HPVSb.E7.92 | A | 45 | 18 | 256 | | 83 | 68853 |
| 1481.19 | GLYNLLIRCL | HPV18.E6.97 | | 1757 | 18 | 6.9 | | .2336 | 9405 |
| 1491.22 | GLYNLLURCV | HPV.18.E6.97 | A | 167 | 13 | 5.3 | | 1221 | 3203 |
| 1491.09 | GTLGNCPV | HPV.16B7.85 | A | 20 | 49 | 68 | | 33 | 32 |
| 1481.85 | IRBEVLEIPL | HPV56.E6.17 | | 1032 | 3.3 | 586 | | 2063 | 486 |
| 1491.59 | HLSEVLEIPV | HPV.56.E6.17 | A | 508 | 15 | 65 | | 507 | 176 |
| 1491.49 | ELYRDCLAYA | HPV.45.E6.54 | A | 108 | 1.5 | 3.3 | | 118 | 3627 |
| 1483.07 | IMCLRFLSKI | HPV.52.E6.64 | | 1857 | 483 | 239 | | 4398 | 17904 |
| 1491.89 | IMCLRFLSKV | HPV.52.E6.64 | A | 2713 | 574 | 81 | | 2357 | 5259 |
| 1481.63 | IQQLLMGTV | HPV33.E7.79 | | 6592 | 17489 | 36 | | 805 | >379509.93 |
| 1481.75 | ITNTELYNL | BPV45.E6.93 | | 9682 | 52 | 3456 | | 2002 | 6153 |
| 1481.71 | IVYRDCIAYA | HBV45.E6.54 | | 1162 | 16 | 3.9 | | 63 | 1096 |
| 1491.50 | IVYRDCIAYV | HPV.45.E6.54 | A | 697 | 16 | 4.3 | | 68 | 74 |
| 1481.74 | KITNTELYNL | HPV45.E6.92 | | 3256 | 18 | 3310 | | 293 | 378556 |
| 1491.56 | KITNTELYNV | HPV.45.E6.92 | A | 199 | 109 | 19 | | 114 | 80759 |
| 1481.54 | KLCLRFLSKI | HPV33.E6.64 | | 1588 | 8581 | 335 | | 1006 | 17208 |
| 1491.41 | KLCLRFLSKV | HPV.33.E6.64 | A | 1759 | 594 | 83 | | 5124 | 8915 |
| 1481.26 | KLHELSSAL | HPV31.E6.11 | | 178 | 1.1 | 3.8 | | 992 | >223606.8 |
| 1491.24 | KLHELSSAV | HPV.31.Eb.11 | A | 144 | 25 | 3.7 | | 1168 | 47805 |
| 1491.62 | KLHTCYLIHV | HPV.56.E7.54 | A | 235 | 71 | 94 | | 115 | 6991 |
| 1491.12 | KLPDLCTEV | HPV. 18/45.E6.13 | A | 65 | 2.7 | 11 | | 101 | 40670 |
| 1491.05 | KLPQLCTEV | HPV.16.E6.18 | A | 93 | 17 | 37 | | 92 | 72458 |
| 1481.34 | KLTNKGICDL | HPV31.E6.90 | | 205 | 440 | 585 | | 484 | >96308.68 |
| 1491.30 | KLTNKGICDV | HPV.31.E6.90 | A | 344 | 25 | 54 | | 190 | >41894 |
| 1491.55 | KLTNTELYNL | HPV.45.E6.92 | A | 1412 | 8.0 | 34 | | 11272 | >41369.22 |
| 1491.20 | KLTNTGLYNV | HPV.18.E6.92 | A | 106 | 2.9 | 4.7 | | 83 | 688 |
| 1491.13 | KLVLELTEV | HPV.18.E6.36 | A | 88 | 16 | 24 | | 226 | >52914.06 |
| 1481.97 | KQHTCYLIHV | HPV56.E7.54 | | 1137 | 1688 | 39 | | 79 | 36431 |
| 1481.12 | KTVLELTEV | HPV18.E6.36 | | 496 | 3715 | 41 | | 96 | 7576 |
| 1481.88 | LIDLRLSCV | HPV56.E6.26 | | 496 | 96 | 44 | | 165 | 4413 |
| 1491.60 | LLDLRLSCV | HPV.56.E6.26 | A | 256 | 1445 | 708 | | 139 | 1905 |
| 1491.45 | LLDVSIACV | HPV.45.E6.25 | A | 61 | 48 | 387 | | 41 | 3379 |
| 1482.02 | LLMGALTVT | HPV56.E7.89 | | 140 | 52 | 33 | | 164 | 139561 |
| 1491.70 | LLMGALTVV | HPV.56.E7.89 | A | 40 | 6.9 | 18 | | 103 | 1537 |
| 1481.44 | LLMGSFGN | HPV3LE7.82 | | 138 | 42 | 3.0 | | 20 | 64853 |
| 1482.25 | LLMGTCTIV | HPV58.E7.83 | | 76 | 3.3 | 6.0 | | 138 | 29355 |
| 1481.65 | LLMGTVNIV | HPV33.E7.82 | | 56 | 2.4 | 4.2 | | 128 | 21582 |
| 1491.53 | LLQLFLSTL | HPV.45.E7.87 | A | 98 | 1.5 | 3.4 | | 181 | 4252 |
| 1491.63 | LLYRDDFPYA | HPV.56.E6.55 | A | 162 | 3.6 | 145 | | 25 | 4031 |
| 1481.67 | LQDVSIACV | HPV45.E6.25 | | 192 | 3106 | 6013 | | 108 | 199469 |
| 1481.82 | LQQLFLSTL | HPV45.E7.87 | | 4398 | 146 | 81 | | 185 | 115466 |
| 1491.54 | I:QQLFLSTV | HPV.45.E7.87 | A | 180 | 39 | 5.4 | | 67 | 24253 |
| 1481.17 | LTNTGLYNL | HPV18.E6.93 | | 13609 | 20 | 4987 | | 1835 | 1580 |
| 1481.90 | LVYRDDFPYA | HPV56.E6.55 | | 914 | 178 | 628 | | 12 | 305 |
| 1491.64 | LVYRDDFPYV | HPV.56.E6.55 | A | 331 | 2487 | 693 | | 53 | 161 |
| 1481.21 | MLCMCCKCEA | HPV18.E7.61 | | 1728 | 178 | 346 | | 750 | 3782 |
| 1491.15 | MLCMCCKCEV | HPV.18.E7.61 | A | 383 | 123 | 235 | | 928 | 1374 |
| 1483.03 | MLLGTLQW | HPV.52.E7.84 | | 36 | 14 | 9.9 | | 52 | 2775 |
| 1481.87 | PLIDLRLSCV | HPV56.E6.25 | | 569 | 39 | 6.0 | | 146 | 1965 |
| 1482.08 | QALETSVHEI | HPV58.E6.17 | | 1144 | 123 | 346 | | 9600 | 163332 |
| 1491.77 | QALETSVHEV | HPV.58.E6.17 | A | 1785 | 298 | 925 | | 7345 | 6502 |
| 1481.48 | QALETTIHNI | HPV33.E6.17 | | 4288 | 181 | 965 | | 3309 | 247293 |
| 1491.35 | QALETTIHNV | HPV.33.E6.17 | A | 1217 | 155 | 475 | | 1509 | 19421 |
| 1481.83 | QLFLSTLSFV | HPV45.E7.89 | | 106 | 3.8 | 11 | | 75 | 362 |
| 1491.76 | QLLETSVHEI | HPV.58.E6.17 | A | 2427 | 189 | 923 | | 11863 | 39909 |
| 1491.34 | QLLETTIFEVI | HPV.33.E6.17 | A | 1702 | 168 | 474 | | 1128 | 44525 |
| 1491.86 | QLLLGTLQVV | HPV.52.E7.83 | A | 85 | 26 | 6.3 | | 137 | 7677 |
| 1482.01 | QLLMGALTV | HPV56.E7.88 | | 201 | 990 | 149 | | 1360 | 68105 |
| 1481.99 | QLLMGALTVT | HPV56.B7.88 | | 1235 | 238 | 147 | | 1163 | 97963 |
| 1491.69 | QLLMGALTVV | HPV.56.E7.88 | A | 162 | 12 | 4.7 | | 208 | 1358 |
| 148224 | QLLMGTCTIV | HPV58.E7.82. | | 304 | 20 | 41 | | 835 | 119377 |
| 1481.64 | QLLMGTVNIV | HPV33.E7.81 | | 502 | 25 | 158 | | 3476 | 99642 |
| 1491.91 | QLMLLGTLQV | HPV.52.E7.82 | A | 77 | 95 | 68 | | 1298 | 9110 |
| 1483.04 | QMLLGTLQV | HPV.52.E7.83 | | 374 | 186 | 251 | | 1523 | 32794 |
| 1483.05 | QMLLGTLQVV | HPV.52.E7.83 | | 77 | 12 | 6.0 | | 120 | 2977 |
| 1483.09 | QQMLLGTLQV | HPV.52.E7.82 | | 675 | 331 | 344 | | 71 | 2012 |
| 1481.04 | QQYNKPLCDL | HPV16.E6.97 | | 25370 | 12085 | 52 | | 2166 | 121435 |
| 1481.23 | RAFQQLFLNT | HPV18.E7.84 | | 170400 | 0 | 4270 | | 114 | 142375 |
| 1481.06 | RLCVQSTHV | HPV16.E7.66 | | 1883 | 250 | 128 | | 725 | 25116 |
| 1481.41 | PLCVQSTQV | HPV31.E7.66 | | 1416 | 1941 | 152 | | 2084 | 78507 |
| 1491.93 | RLLHELCEV | HPV.52.E6.10 | A | 25 | 18 | 61 | | 93 | >63492.06 |
| 1491.65 | RLVQQLLMGA | HPV.56.E7.84 | A | 873 | 1626 | 9.4 | | 875 | >37331.55 |
| 1481.07 | RTLEDLLMGT | HPV16.E7.77 | | 17556 | 701 | 7397 | | 102 | >60329.7 |
| 1481.46 | RTLF1DLCQA | HPV33.E6.10 | | 8121 | 34 | 678 | | 96 | 61604 |
| 1482.06 . | RTLHDLCQA | HPV58.E6.10 | | 5274 | 142 | 473 | | 73 | >447213.6 |
| 1483.11 | RTLHELCEV | HPV.52.E6.10 | | 82 | 46 | 341 | | 7.0 | 5670 |
| 1481.98 | RVVQQLLMGA | HPV56.E7.84 | | 1587 | 7065 | 372 | | 102 | 13817 |
| 1491.66 | RVVQQLLMGV | HPV.56.E7.84 | A | 254 | 2412 | 8.8 | | 69 | 2336 |
| 1481.27 | SALEIPYDEL | HPV31.E6.17 | | 11239 | 2263 | 111943 | | 306 | 135671 |
| 1481.68 | SIACVYCKA | HPV45.E6.29 | | 8938 | 31 | 599 | | 8665 | 2724 |
| 1491.87 | SLHEIRLQCV | HPV.52.E6.22 | A | 1003 | 12 | 10 | | 7923 | 11701 |
| 1481.66 | SLQDVSIACV | HPV45.E6.24 | | 67 | 22 | 27 | | 251. | 82033 |
| 1491.67 | SLYGATLESI | HPV.56.E6.85 | A | 451 | 23 | 23 | | 401 | 2114 |
| 1491.16 | SLYGDTLEKL | HPV.18.E6.84 | A | 139 | 2.5 | 11 | | 275 | 7640 |
| 1482.15 | SLYGDTLEQT | HPV58.E6.82 | | 408 | 9.7 | 65 | | 1005 | 261482 |
| 1491.82 | SLYGDTLEQV | HPV.58.E6.82 | A | 251 | 9.2 | 27 | | 306 | 257 |
| 1491.51 | SLYGETLEKI | HPV.43.E6.84 | A | 301 | 4.0 | 25 | | 358 | 3491 |
| 1491.42 | SLYGNTLEQT | HPV.33.E6.82 | A | 216 | 2.5 | 9.5 | | 513 | 4712 |
| 1482.10 | SVHEIELKCV | HPV58.E6.22 | | 12550 | 1214 | 1636 | | 871 | 351 |
| 1483.06 | SVHEIRLQCV | HPV.52.E6.22 | | 1262 | 130 | 262 | | 369 | 38 |
| 1481.93 | SVYGATLESI | HPV56.E6.85 | | 813 | 25 | 27 | | 110 | 470 |
| 1491.68 | SVYGATLESV | HPV.56.E6.85 | A | 248 | 12 | 19 | | 131 | 35 |
| 1481.16 | SVYGDTLEKL | HPV18.E6.84 | | 2238 | 19 | 82 | | 130 | 909 |
| 1491.17 | SVYGDTLEKV | HPV.18.E6.84 | A | 198 | 9.6 | 5.6 | | 130 | 29 |
| 1481.73 | SVYGETLEKI | HPV45.E6.84 | | 995 | 14 | 23 | | 241 | 1529 |
| 1491.52 | SVYGETLEKV | HPV.45.E6.84 | A | 320 | 7.8 | 23 | | 171 | 30 |
| 1481.55 | SVYGNTLEQT | HPV33.E6.82 | | 1056 | 75 | 32 | | 560 | 3065 |
| 1491.43 | SVYGNTLEQV | HPV.33.E6.82 | A | 157 | 3.5 | 12 | | 70 | 10 |
| 1481.33 | SVYGTTLEKL | HPV31.E6.82 | | 11249 | 40 | 932 | | 240 | 1095 |
| 1481.50 | TIHNIELQCV | HPV33.E6.22 | | 3411 | 231 | 273 | | 301 | 2630 |
| 1481.62 | TIQQLLMGTV | HPV33.E7.78 | | 6902 | 5860 | 26 | | 7943 | 3844 |
| 1481.09 | TLEDLLMGT | HPV16.E7.78 | | 13241 | 317 | 1162 | | 2126 | >67134.51 |
| 1481.08 | TLEDLLMGTL | HPV16.E7.78 | | 12724 | 474 | 3738 | | 81842 | >60329.7 |
| 1491.10 | TLGIVCPV | HPV.16.E7.86 | A | 9.0 | 3.2 | 7.2 | | 24 | 165 |
| 1491.06 | TLHDIILECV | HPV.16.E6.29 | A | 3.6 | 0.5 | 1.9 | | 92 | 2947 |
| 1481.47 | TLHDLCQAL | HPV33.E6.11 | | 1404 | 2.7 | 40 | | 2182 | 70390 |
| 1482.07 | TLHDLCQAL | HPV58.E6.11 | | 1043 | 8.3 | 46 | | 11185 | 130225 |
| 1491.73 | TLHDLCQAV | HPVS8.E6.11 | A | 331 | 13 | 15 | | 10585 | 2288 |
| 1491.01 | TLHEYMLDV | HPV.16.E7.7 | A | 74 | 4.4 | 9.8 | | 78 | 2424 |
| 1491.37 | TLHNIELQCV | HPV.33.E6.22 | A | 206 | 3.4 | 7.5 | | 584 | 2475 |
| 1481.57 | TLKEYVLDL | HPV33.E7.7 | | 8004 | 70 | 12 | | 117530 | 615105 |
| 1491.11 | TLQDIVLHV | HPV.18.E7.7 | A | 20 | 23 | 7.0 | | 684 | 1683 |
| 1481.95 | TLQDVVLEL | HPV56.E7.7 | | 24 | 7.8 | 23 | | 649 | 13838 |
| 1491.58 | TLQDVVLEV | HPV.56.E7.7 | A | 52 | 73 | 25 | | 90 | 2275 |
| 1481.36 | TLQDYVLDL | HPV31.E7.7 | | 209 | 32 | 90 | | 2294 | 91800 |
| 1491.23 | TLQDYVLDV | HPV.31.E7.7 | A | 28 | 30 | 6.1 | | 711 | 6181 |
| 1481.77 | TLQEIVLHL | HPV45.E7.7 | | 124 | 20 | 155 | | 2692 | 254071 |
| 1491.44 | TLQEIVLHV | HPV.45.E7.7 | A | 19 | 30 | 5.1 | | 309 | 2457 |
| 1481.81 | TLQQLFLSTL | HPV45.E7.86 | | 10329 | 145 | 173 | | 1917 | 295602 |
| 1482.23 | TLQQLLMGT | HPV58.E7.79 | | 5407 | 4161 | 343 | | 19328 | 355458 |
| 1483.08 | TLQQMLLGT | HPV.52.E7.80 | | 3994 | 1876 | 47 | | 940 | 66940 |
| 1483.15 | TLQQMLLGTL | HPV.52.E7.80 | | 11590 | 1207 | 82 | | 94848 | 63961 |
| 1491.90 | TLQQMLLGV | HPV.52.E7.80 | A | 42 | 17 | 4.6 | | 28 | 454 |
| 1483.20 | TLQVVCPGCA | HPV.52.E7.88 | | 173805 | 8146 | 446 | | 226554 | 48401 |
| 1482.17 | TLREYILDL | HPV58.E7.7 | | 13653 | 1372 | 6.2 | | 148231 | >389869.03 |
| 1483.19 | TLRLCIHSTA | HPV.52.E7.66 | | 69139 | 5407 | 329 | | 59923 | 9270 |
| 1481.40 | TLRLCVQST | HPV31.E7.64 | | 6394 | 22268 | 136 | | 28285 | 4163 |
| 1481.25 | TLSFVCPWCA | HPV18.E7.93 | | 1611 | 221 | 521 | | 27321 | 13228 |
| 1481.84 | TLSFVCPWCA | HPV45.E7.94 | | 1499 | 292 | 763 | | 3657 | 7714 |
| 1491.57 | TLSFVCPWCV | HPV.45.E7.94 | A | 786 | 91 | 370 | | 4357 | 348 |
| 1491.21 | TLSFVCPWCV | HPV.18.E7.93 | | 368 | 118 | 255 | | 7573 | 2299 |
| 1483.10 | VLEESVHEI | HPV.S2.E6.18 | | 426 | 22 | 354 | | 831 | >56545.93 |
| 1491.92 | VLEESVHEV | HPV.52.E6.18 | A | 236 | 30 | 163 | | 1283 | 198762 |
| 1481.15 | VVYRDSIPHA | HPV18.E6.54 | | 20174 | 261 | 393 | | 702 | 15021 |
| 1481.91 | YAVCRVCLL | HPV56.E6.63 | | 9827 | 27573 | 937 | | 327 | 6152 |
| 1482.18 | YILDLHPEPT | HPV58.E7.11 | | 136 | 639 | 28 | | 1236 | 219394 |
| 1491.75 | YILDLHPEPV | BPV.58.E7.11 | A | 91 | 69 | 5.8 | | 64 | 335 |
| 1483.02. | YILDLQPET | HPV.52.E7.1 | | 449 | 2778 | 467 | | 186 | 67516 |
| 1483.16 | YILDLQPETT | HPV.52.E7.11 | | 428 | 1684 | 336 | | 7730 | 66404 |
| 1491.94 | YILDLQPETV | HPV.52.E7.11 | A | 35 | 11 | 10 | | 131 | 264 |
| 1491.84 | YILDLQPEV | EPV.52.E7.11 | A | 145 | 166 | 245 | | 78 | 6387 |
| 1491.80 | YLCG7TVRL | HPV.58.E7.60 | A | 246 | 12 | 43 | | 1369 | 2553 |
| 1491.46 | YLFAFKDLCI | HPV.45.E6.45 | A | 24 | 2.2 | 8.1 | | 81 | 4067 |
| 1491.74 | YLLDLHPEPT | HPV.58.E7.11 | A | 82 | 15 | 29 | | 1456 | 100474 |
| 1491.25 | YLLDLQPEA | HPV.31.E7.11 | A | 20 | 2.2 | 6.6 | | 136 | 46519 |
| 1491.27 | YLLDLQPEAT | HPV.31.E7.11 | A | 24 | 16 | 6.4 | | 975 | >57908.91 |
| 1491.02 | YLLDLQPET | HPV.16.E7.11 | A | 16 | 57 | 78 | | 159 | >47202.54 |
| 1491.03 | YLLDLQPETT | HPV.16.E7.11 | A | 93 | 18 | 16 | | 565 | 39475 |
| 1491.04 | YMLDLQPETV | HPV.16.E7.11 | A | 19 | 1.9 | 4.5 | | 86 | 5446 |
| 1202.13 | YN1LDLQPEV | HPV.16.E7.11 | A | 19 | 1.6 | 14 | | 25 | 18840 |
| 1481.69 | YQFAFKDLCI | HPV45.E6.45 | | 87 | 95 | 23 | | 441 | 68211 |
| 1491.47 | YQFAFKDLCV | HPV.45.E6.45 | A | 15 | 1.3 | 4.2 | | 10.0 | 3698 |
| 1483.18 | YQYSLYOKTL | HPV.52.E6.79 | | 91919 | 2095 | 297 | | 4709 | 74927 |
| 1482.21 | YTCGITVRL | HPV58.E7.60 | | 841 | 32 | 950 | | 530 | 376 |
| 1491.81 | YTCGTRVRV | HPV.58.E7.60 | A | 2301 | 845 | 353 | | 1061 | 42 |
| 1481.37 | YVLDLQPEA | HPV31.E7.11 | | 822 | 385 | 3342 | | 92 | 7409 |
| 1491.28 | YVLDLQPEAV | HPV.31.E7.11 | A | 28 | 26 | 6.4 | | 115 | 222 |
| 1491.26 | YVLDLQPBV | HPV.31.E7.11 | A | 27 | 169 | 430 | | 76 | 849 |
| 1481.58 | YVLDLYPEPT | HPV33.E7.11 | | 118 | 2490 | 324 | | 332 | 44993 |
| 1491.33 | YYLDLYPEPV | HPV.33.E7.11 | A | 25 | 12 | 3.4 | | 29 | 29 |
| 1419.61 | ALFPPEGVSV | PAP. | | 15 | 1.1 | 18 | | 119 | 4444 |
| F063.58 | ALGIGILTV | MART127 | A | 11 | | | | | |
| 1389.14 | ALGTTCYV | PSA.143 | A | 93 | 6.7 | 12 | | 288 | 29169 |
| F188.048 | ALLTILGGGL | | | 8.7 | | | | | |
| F188.039 | ALNIWDRFDV | | | 386 | | | | | |
| F188.010 | ALVCNTLWGV | | | 18 | | | | | |
| F188.033 | ALWECGCATL | | | 14 | | | | | |
| F063.59 | AMGIGILTV | MART1.27 | A | 15 | | | | | |
| 63.0109 | DLMLLRLSEPV | Kallikrein2.120 | A | 70 | 66 | 31 | | 119 | 2759 |
| 1485.06 | ELCCEHLWQI | Gliadin.155 | A | 1440 | 25427 | 5727 | | 133 | 32 |
| 1485.07 | EICCQHLWEI | Gliadin.155 | A | 1161 | 1257 | 1639 | | 147 | 37 |
| 1485.04 | ELIPCMDVV | Gliadin.123 | A | 5190 | 1491 | 101 | | 1361 | 75 |
| 1485.05 | ELIPCMDVVL | Gliadin.123 | A | 4678 | 1917 | 15851 | | 2081 | 329 |
| 1485.08 | ELQPFLQPQL | Gliadin.57 | A | 14568 | 1707 | 5444 | | 170276 | 477 |
| F188.052 | FILEYQDSL | | | 218 | | | | | |
| F188.077 | FIPLGLFWV | | | 131 | | | | | |
| F188.030 | FLDSGFVGL | | | 21 | | | | | |
| F188.057 | FLLFFEFLL | | | 81 | | | | | |
| F188.075 | FLLGLIFFI | | | 166 | | | | | |
| F188.011 | FLLRGSNNYV | | | 134 | | | | | |
| F188.024 | FLNTPWILGI | | | 186 | | | | | |
| F188.086 | FLPLALLWV | | | 17 | | | | | |
| F188.009 | GIGLDLPFV | | | 434 | | | | | |
| F188.001 | GLAATHMCV | | | 195 | | | | | |
| F188.089 | GLFERENCV | | | 214 | | | | | |
| F188.037 | GLFGVKGTTV | | | 221 | | | | | |
| F188.027 | GLFLGFSSL | | | 449 | | | | | |
| 1419.62 | GLHGQDLFGV | PAP. | | 12 | 2.3 | 3.1 | | 18 | 80000 |
| F188.080 | GLIFFIPLGL | | | 111 | | | | | |
| F188.045 | GLLPAVPMFI | | | 29 | | | | | |
| F188.050 | GLLVGLLPAV | | | 19 | | | | | |
| F188.051 | GLPEELPPV | | | 25 | | | | | |
| F188.091 | HLNPLSPNV | | | 113 | | | | | |
| F188.029 | ILALIQTPL | | | 16 | | | | | |
| F188.083 | ILFLPLALL | | | 94 | | | | | |
| F188.076 | ILIFNYPGV | | | 146 | | | | | |
| F188.093 | ILIYNYPGV | | | 174 | | | | | |
| F188.058 | ILQEAEQMV | | | 237 | | | | | |
| 1485.13 | ILQEQLEPCM | Gliadin.119 | A | 366 | 1501 | 5350 | | 2058 | >75515.83 |
| F188.082 | ILSDDVIKEV | | | 196 | | | | | |
| F188.098 | ILVYNYPGV | | | 461 | | | | | |
| F188.044 | ILYRSTAHL | | | 487 | | | | | |
| 1389.06 | ILYSAHDTTV | PAP.384 | A | 391 | 1.1 | 13 | | 1471 | 6218 |
| 1418.26 | ITYSAHDTTV | PAP.284 | A | 4167 | 115 | 238 | | 154 | 82 |
| 1389.07 | IVYSAHDTTV | PAP.284 | A | 7710 | 91 | 623 | | 671 | 745 |
| 1489.31 | KILSVFFLAL | Pf.EXP1.2 | | 311 | 18 | 499 | | 53 | 1940 |
| F188.100 | KLIGAMLV | | | 134 | | | | | |
| F188.094 | KLLGANILI | | | 122 | | | | | |
| FI88.085 | KLVCWNIDSV | | | 46 | | | | | |
| 1419.58 | LLALFPPEGV | PAP. | | 5.0 | 0.7 | 1.6 | | 148 | 163 |
| F188.061 | LLFFEFLLV | | | 209 | | | | | |
| F188.095 | LLIGLIIPPL | | | 34 | | | | | |
| F188.070 | LLLDVIPLSL | | | 19 | | | | | |
| F189.10 | LLMWITQCFL | NY-ESO-1.158 | | 43 | | | | | |
| F188.043 | LLPAVPMFI | | | 353 | | | | | |
| 1419.64 | LLPPYASCHV | PAP. | | 88 | 15 | 16 | | 97 | 5333 |
| F188.063 | LLQAALLQSV | | | 117 | | | | | |
| F188.047 | LLVGLLPAV | | | 47 | | | | | |
| 1419.69 | LLWQPIPVHV | PAP. | | 25 | 1.8 | 18 | | 285 | 62 |
| F189.08 | LMWITQCFL | NY-ESO-1.159 | | 78 | | | | | |
| 1419.59 | LVALFPPEGV | PAP. | | 156 | 17 | 4.8 | | 463 | 28 |
| 1389.10 | MLLRLSEPV | PSA.118 | A | 48 | 29 | 48 | | 686 | 432 |
| 7.0045 | MPSLTMACM | LCMV.NP.175 | | 323 | | | | | |
| F188.087 | NLLVFNYPCI | | | 485 | | | | | |
| 63.0128 | PLVCNGVLQGV | Kallikrein2.216 | A | 92 | 421 | 36 | | 210 | 1633 |
| 1419.17 | PLVCNGVLQGV | Kallikrein2.216 | A | 26 | 126 | 19 | | 264 | 4211 |
| 1485.09 | QLEPFLQPQL | Gliadin.57 | A | 2741 | 35 | 4200 | | 4061 | 41451 |
| F188.013 | QLFSLLKITV | | | 215 | | | | | |
| 1485.10 | QLQPFLEPQL | Gliadin.57 | A | 2417 | 103 | 2899 | | 6540 | >74461.3 |
| 1485.11 | QLQPFLQPEL | Gliadin.57 | A | 216 | 20 | 638 | | 3760 | 5514 |
| F188.105 | RLATLSFSM | | | 35 | | | | | |
| F188.101 | RLFGWGTKAV | | | 136 | | | | | |
| F188.031 | RLIEKQSQV | | | 378 | | | | | |
| F188.078 | RLLEIIWGV | | | 16 | | | | | |
| F188.007 | RLLNLSFFV | | | 25 | | | | | |
| F188.036 | RLNSLVPYI | | | 118 | | | | | |
| F188.056 | RLQEENAQL | | | 252 | | | | | |
| F188.065 | SLADIQAALV | | | 63 | | | | | |
| F188.067 | SLQDAVTNI | | | 194 | | | | | |
| 1419.52 | SLSLGFLFLV | PAP. | | 1.9 | 3.9 | 17 | | 42 | 348 |
| 1419.50 | SLSLGFLFV | PAP. | | 77 | 25 | 21 | | 93 | 26667 |
| F188.035 | SLSNGVVEL | | | 261 | | | | | |
| F188.017 | SLWGSFSDI | | | 211 | | | | | |
| F096.13 | SVYDFFVWL | | | 36 | 169 | 226 | | 10.0 | 1.2 |
| F001.02 | SXPSGGXGV | | A | 234 | | | | | |
| 1389.03 | TLMSAMTNV | PAP.112 | A | 628 | 14 | 35 | | 2159 | 482 |
| F188.020 | TLTGLSIPV | | | 23 | | | | | |
| F001.01 | TXWVDPYEV | | A | 24 | | | | | |
| 1485.01 | VLEQSTYQL | Gliadin.144 | A | 929 | 15 | 11311 | | 2183 | 24884 |
| F188.016 | VLGQFVFEV | | | 9.7 | | | | | |
| F188.102 | VLIGLIILPL | | | 140 | | | | | |
| F188.069 | VLLLDVIPL | | | 29 | | | | | |
| 1485.02 | VLQESTYQL | Gliadin.144 | A | 88 | 2.3 | 147 | | 267 | 16437 |
| 1485.03 | VLQQSTYEL | Gliadin.144 | A | 147 | 8.5 | 302 | | 356 | 12794 |
| 63.0105 | VLVHPQWVLTV | Kallikrein2.53 | A | 11 | 1.6 | 3.0 | | 13 | 4444 |
| 1419.11 | VLVHPQWVLTV | Kallikrein2.53 | A | 11 | 1.5 | 16 | | 31 | 8889 |
| 60.0180 | VLVHPQWVV | Kallikrein2.53 | A | 564 | 65 | 1982 | | 3199 | 16000 |
| F188.106 | VLWKDGVSFV | | | 117 | | | | | |
| F188.025 | VMNNVPVML | | | 255 | | | | | |
| 1418.24 | VTAKELKFV | PAP.30 | A | 7143 | 2688 | 40 | | 137 | 26667 |
| F189.09 | WITQCFLPV | NY-ESO-1.161 | | 173 | | | | | |
| F188.015 | YAFSPMFEV | | | 16 | | | | | |
| F188.107 | YLNDYVLPYA | | | 13 | | | | | |
| F188.059 | YMYGITDSL | | | 112 | | | | | |
| F188.040 | YVFDRILKV | | | 43 | | | | | |
| F188.108 | YVLPYASVSI | | | 234 | | | | | |

**TABLE 15**

| | | | | HLA- A2 supertype binding affinity (IC50 nM) | | | | | | |
|---|---|---|---|---|---|---|---|---|---|---|
| Peptide | Sequence | Source | Analog | A*0201 | A*0202 | A*0203 | A*0205 | A*0206 | A*0207 | A*6802 |
| F109.09 | FVNHRFTLV | Clone4.551 | | 373 | | | | | | |
| F108.13 | GIRFYEILA | Clone4.135 | | 34 | | | | | | |
| F108.15 | WVFPESISPV | Clone4.302 | | 120 | | | | | | |
| 36.0132 | ALKMTMASV | Flu.NSI.76 | | 230 | | | | | | |
| 36.0148 | ALLKHRFEI | Flu.RRP2.70 | | 128 | | | | | | |
| 36.0189 | ALLKHKFEII | Flu.RRP2.70 | | 226 | | | | | | |
| 36.0149 | CLLQSLQQI | Flu.RRK.584 | | 160 | | | | | | |
| 36.0138 | FLEESHPGI | Flu.RRP1.94 | | 118 | | | | | | |
| 36.0133 | FLWHVRKRV | Flu.NS1.14 | | 205 | | | | | | |
| 36.0128 | GLISLILQI | Flu.NRAM.18 | | 38 | | | | | | |
| 36.0139 | GMFNMLSTV | Flu.RRP1.410 | | 28 | | | | | | |
| 36.0129 | GMGWLTIGI | Flu.NRAM.172 | | 143 | | | | | | |
| 36.0135 | LLLEVEQEI | Flu.NS2.105 | | 239 | | | | | | |
| 36.0190 | LLMDALKLSI | Flu.RRP2.283 | | 38 | | | | | | |
| 36.0158 | LMDALKLSI | Flu.RRP2.284 | | 41 | | | | | | |
| 36.0185 | MLSTVLGVSI | Flu.RRP1.414 | | 252 | | | | | | |
| 36.0186 | NLYNIRNLHI | Flu.RRP1.597 | | 63 | | | | | | |
| 36.0144 | RLIDFLKDV | Flu.RRP1.162 | | 58 | | | | | | |
| 36.0145 | RLNKRSYLI | Flu.RRP1.211 | | 90 | | | | | | |
| 36.0167 | RMQFSSFTV | Flu.RRP3.630 | | 28 | | | | | | |
| 36.0161 | SMIEAESSV | Flu.RRP2.594 | | 185 | | | | | | |
| 36.0172 | WMMAMKYPI | Flu.RRP3.49 | | 73 | | | | | | |
| F063.12 | ALWGQYWQV | gp100.154 | A | 2.3 | | | | | | |
| F063.08 | ATWGQYWQV | gp100.154 | A | 7.7 | | | | | | |
| F06337 | FLDQVPFSV | gp100.209 | A | 2.4 | | | | | | |
| F063.43 | FLEPGPVTA | gp100.280 | A | 113 | | | | | | |
| F063.15 | FLWGQYWQV | gp100.154 | A | 2.9 | | | | | | |
| F063.21 | FTDQVPFSV | gp100.209 | A | 61 | | | | | | |
| F063.06 | FTWGQYWQV | gp100.154 | A | 1.8 | | | | | | |
| F063.20 | IIDQVPFSV | gp100.209 | A | 46 | | | | | | |
| F063.33 | ILAQVPFSV | gp100.209 | A | 11 | | | | | | |
| F063.18 | ILDQVPFSV | gp100.209 | A | 13 | | | | | | |
| F063.31 | ILFQVPFSV | gp100.209 | A | 2.1 | | | | | | |
| F063.34 | ILMQVPFSV | gp100.209 | A | 7.6 | | | | | | |
| F063.35 | ILSQVPFSV | gp100.209 | A | 20 | | | | | | |
| F063.30 | ILWQVPFSV | gp100.209 | A | 1.7 | | | | | | |
| F063.32 | ILYQVPFSV | gp100.209 | A | 6.9 | | | | | | |
| F063.19 | IMDQVPFSV | gp100.209 | A | 21 | | | | | | |
| F063.27 | ITAQVPFSV | gp100.209 | A | 134 | | | | | | |
| F063.25 | ITFQVPFSV | gp100.209 | A | 68 | | | | | | |
| F063.28 | ITMQVPFSV | gp100.209 | A | 42 | | | | | | |
| F063.24 | ITWQVPFSV | gp100.209 | A | 35 | | | | | | |
| F063.26 | ITYQVPFSV | gp100.209 | A | 33 | | | | | | |
| F063.04 | KIWGQYWQV | gp100.154 | A | 4.9 | | | | | | |
| F063.02 | KLWGQYWQV | gp100.154 | A | 2.1 | | | | | | |
| F063.03 | KMWGQYWQV | gp100.154 | A | 6.6 | | | | | | |
| F063.11 | KTFGQYWQV | gp100.154 | A | 48 | | | | | | |
| F063.10 | KTYGQYWQV | gp100.154 | A | 32 | | | | | | |
| F063.13 | LLWGQYWQV | gp100.154 | A | 1.6 | | | | | | |
| F063.09 | LTWGQYWQV | gp100.154 | A | 3.5 | | | | | | |
| F06336 | WLDQVPFSV | gp100.209 | A | 12 | | | | | | |
| F063.14 | WLWGQYWQV | gp100.154 | A | 4.6 | | | | | | |
| F063.05 | WTWGQYWQV | gp100.154 | A | 72 | | | | | | |
| F063.50 | YLAPGPVTA | gp100.280 | A | 9.4 | | | | | | |
| F063.53 | YLAPGPVTV | gp100.280 | A | 15 | | | | | | |
| F063.38 | YLDQVPFSV | gp100.209 | A | 2.6 | | | | | | |
| F063.42 | YLEPGPVTI | gp100.280 | A | 68 | | | | | | |
| F063.41 | YLEPGPVTL | gp100.280 | A | 146 | | | | | | |
| F063.40 | YLEPGPVTV | gp100.280 | A | 56 | | | | | | |
| F063.47 | YLFPGPVTA | gp100.280 | A | 3.7 | | | | | | |
| F063.55 | YLFPGPVTV | gp100.280 | A | 5.9 | | | | | | |
| F063.48 | YLMPGPVTA | gp100.280 | A | 4.4 | | | | | | |
| F063.51 | YLMPGPVTV | gp100.280 | A | 12 | | | | | | |
| F063.49 | YLSPGPVTA | gp100.280 | A | 48 | | | | | | |
| F063.52 | YLSPGPVTV | gp100.280 | A | 24 | | | | | | |
| F063.16 | YLWGQYWQV | gp100.154 | A | 1.7 | | | | | | |
| F063.46 | YLWPGPVTA | gp100.280 | A | 3.4 | | | | | | |
| F063.56 | YLWPGPVTV | gp100280 | A | 7.5 | | | | | | |
| F063.45 | YLYPGPVTA | gp100.280 | A | 17 | | | | | | |
| F063.34 | YLYPGPVTV | gp100.280 | A | 9.1 | | | | | | |
| F06323 | YTDQVPFSV | gp100.209 | A | 94 | | | | | | |
| F063.07 | YTWGQYWQV | gp100.154 | A | 3.5 | | | | | | |
| 33.0038 | FFPSDYFPSV | HBV.core.18 | A | 1553 | 1496 | 739 | 2171 | 225 | | 1538 |
| 33.0037 | FIPSDYFPSV | BBV.core.18 | A | 29 | 3 | 28 | 4 | 2 | | 133 |
| 33.0027 | FLKSDYFPSV | HBV.core.18 | A | 15 | 11 | 1 | 16 | 22 | | 3636 |
| 33.0028 | FLPKDYFPSV | HBV.core.18 | A | 5.6 | 5.3 | 4.8 | 7.3 | 3.0 | | 1860 |
| 33.0030 | FLPSDKFPSV | HBV.core.18 | A | 9.4 | 6.26 | 1.62 | 11.05 | 9.58 | | 500 |
| 33.0032 | FLPSDYFKSV | HBV.core.18 | A | 20 | 21 | 2 | 58 | 13 | | 8889 |
| 33.0033 | FLPSDYFPKV | HBV.core.18 | A | 36 | 26 | 50 | 24 | 16 | | 1905 |
| 33.0044 | FLPSDYFPSI | HBV.core.18 | A | 25 | 7 | 67 | 18 | 15 | | 2051 |
| 33.0043 | FLPSDYFPSL | HHV.core.18 | A | 31 | 10 | 47 | 14 | 53 | | 8889 |
| 33.0048 | FLPSDYFPSP | HBV.core.18 | A | 220 | 10852 | 2122 | 11467 | 314 | | 80000 |
| 33.0049 | FLPSDYFPST | HBV.core.18. | A | 166 | 96 | 301 | 155 | 27 | | 80000 |
| 33.0031 | FLPSDYKPSV | HBV.core.18 | A | 555 | 1254 | 136 | 2107 | 2301 | | 80000 |
| 33.0029 | FLPSKYFPSV | HBV.core.18 | A | 8.5 | 5.74 | 0.63 | 7.23 | 5.63 | | 1038.96 |
| 33.0042 | FTPSDYFPSV | HBV.core.18 | A | 423 | 5.5 | 419 | 15 | 12 | | 73 |
| 1369.32 | FVGPLLVLQV | HBV.env.171 | A | 927 | 51 | 176 | 35 | 21 | | 241 |
| 1369.31 | FVLLLCLIFV | HBV.env.249 | A | 1992 | 306 | 9032 | 7246 | 9027 | | 11364 |
| 1369.03 | FVLSLGIHV | HBV.pol.562 | A | 45 | 400 | 2837 | 1414 | 133 | | 62 |
| 1369.08 | FVLSLGIHV | HBV.pol.573 | A | 439 | 885 | 18018 | 14098 | 487 | | 1303 |
| 33.0036 | FVPSDYFPSV | HBV.core.18 | A | 77 | 2 | 101 | 3 | 4 | | 80 |
| 55.0123 | GLSRYVPRL | HBV.pol.455 | A | 94 | | | | | | |
| 1369.01 | GVLGWSPQV | HBV.env.62 | A | 22 | 157 | 393 | 37 | 28 | | 9357 |
| 1369.09 | GVLGWSPQV | HBV.env.73 | A | 21 | 134 | 637 | 52 | 21 | | 9886 |
| 1369.28 | GVSPTVWLSV | HBV.env.359 | A | 197 | 77 | 4.8 | 81 | 55 | | 549 |
| 1369.02 | HVYSHPIIV | HBV.pol.1076 | A | 151 | 1921 | 14 | 11026 | 1200 | | 122 |
| 1369.10 | HVYSHPIIV | HBV.pol.502 | A | 255 | 3562 | 11 | 9038 | 1043 | | 204 |
| 1369.26 | IVRGTSFVYV | HBV.pol.773 | A | 36765 | 5298 | 68 | 1828 | 5386 | | 1222 |
| 1369.34 | IVRGTSFVYV | BBV.pol.773 | A | 15661 | 4367 | 88 | 2440 | 2468 | | 1085 |
| 1369.24 | IVSTLPETTV | HBV.core.168 | A | 7447 | 38 | 554 | 403 | 994 | | 4538 |
| 33.0025 | KLPSDYFPSV | HHV.core.18 | A | 24 | 6 | 14 | 12 | 2 | | 40000 |
| 1172.01 | KQYLNLYPV | HBV.pol.668 | | 85 | 22 | 35 | | 28 | | >30237.16 |
| 1369.23 | LVDYQGMLPV | HBV.env.271 | A | 994 | 1037 | 510 | 1118 | 188 | | 9863 |
| 1369.30 | LVVLQAGFFV | HBV.env.175 | A | 1999 | 1105 | 6107 | 732 | 191 | | 900 |
| 1369.12 | LVWFHISCV | HBV.core.129 | A | 177 | 272 | 97 | 2179 | 385 | | 467 |
| 1369.07 | MVWYWGPSV | HBV.env.360 | A | 169 | 532 | 903 | 416 | 45 | | 17 |
| 1360.13 | PVLPIFFCV | HBV.env.377 | A | 8.7 | 3140.36 | 13642.6 | 43000 | 21.88 | | 1813.99 |
| 1369.18 | PVLPIFFCV | HBV.env.388 | A | 16 | 3538 | 23419 | 43000 | 17 | | 1621 |
| 924.20 | QFLPSDFFPSV | HBV.core.27 | A | 394 | | | | | | |
| 924.17 | RFLPSDFFPSV | HBV.core.27 | A | 481 | | | | | | |
| 1369.14 | VVQAGFFLV | HBV.env.177 | A | 438 | 78 | 2484 | 785 | 81 | | 624 |
| 1369.17 | VVQAGFFLV | HBV.env.177 | A | 1708 | 226 | 2244 | 855 | 28 | | 753 |
| 1369.19. | WVLRGTSFV | HBV.pol.770 | A | 500 | 1023 | 125 | 495 | 86 | | 351 |
| 924.19 | YFLPSDFFPSV | HBV.core.27 | A | 151 | | | | | | |
| 1369.15 | YVDDVVLGV | HBV.pol.538 | A | 18 | 14 | 70 | 91 | 16 | | 360 |
| 1369.20 | YVDDVVLGV | HBV.pol.549 | A | 16 | 2 | 108 | 112 | 16 | | 412 |
| 1369.25 | YVHTLWKAGV | HBV.pol.147 | A | 1826 | 324 | 62 | 429 | 1081 | | 169 |
| 1369.16 | YVVSFGVWV | HBV.core.147 | A | 54 | 76 | 33 | 32 | 64 | | 8 |
| 35.0083 | ALVAYQATV | HCV | | 68 | 632 | 4 | 521 | 167 | | 1778 |
| 1369.05 | AVSTGLIHV | HCV.NS1.686 | A | 2497 | 891 | 336 | 1506 | 120 | | 4855 |
| 1369.04 | IVSPGALVV | HCV.NS4.1891 | A | 19305 | 3749 | 184 | 2115 | 2990 | | 11495 |
| 35.0060 | KLVAYQATV | HCV | | 73 | 282 | 6 | 265 | 76 | | 8889 |
| 136927 | RVHGLSAFSV | HCV.NS5.2918 | A | 512 | 389 | 398 | 39 | 51 | | 684 |
| 35.0071 | YFVAYQATV | HCV | | 1071 | 15739 | 139 | 7049 | 16645 | | 186 |
| 35.0070 | YIVAYQATV | HCV | | 58 | 57 | 3 | 41 | 27 | | 31 |
| 35.0062 | YLKAYQATV | HCV | | 32 | 166 | 3 | 83 | 318 | | 2222 |
| 35.0084 | YLQAYQATV | HCV | | 36 | 140 | 4 | 52 | 36 | | 80 |
| 35.0086 | YLVAAQATV | HCV | | 40 | 77 | 4 | 61 | 70 | | 174 |
| 35.0064 | YLVAKQATV | HCV | | 183 | 516 | 5.9 | 243 | 208 | | 6154 |
| 35.0087 | YLVAYAATV | HCV | | 72 | 34 | 3 | 63 | 40 | | 276 |
| 35.0065 | YLVAYKATV | HCV | | 33 | 423 | 7 | 218 | 205 | | 808 |
| 35.0089 | YLVAYQAFV | HCV | | 9.1 | 19.08 | 2.65 | 9.58 | 22.92 | | 38.1 |
| 35.0067 | YLVAYQAKV | HCV | | 70 | 104 | 5 | 65 | 144 | | 381 |
| 35.0078 | YLVAYQATF | HCV | | 232 | 6806 | 53 | 3457 | 3322 | | 40000 |
| 35.0077 | YLYAYQATI | HCV | | 78 | 305 | 4 | 97 | 166 | | 1270 |
| 35.0076 | YLVAYQATL | HCV | | 17 | 44 | 3 | 26 | 75 | | 1538 |
| 35.0082 | YLVAYQATT | HCV | | 97 | 3774 | 20 | 1475 | 953 | | 26667 |
| 35.0066 | YLVAYQKTV | HCV | | 303 | 875 | 5.5 | 380 | 310 | | 1127 |
| 35.0088 | YLVAYQQTV | HCV | | 40 | 82 | 5 | 41 | 57 | | 727 |
| 35.0063 | YLVKYQATV | HCV | | 45 | 75 | 4 | 50 | 48 | | 276 |
| 35.0085 | YLVQYQATV | HCV | | 15 | 44 | 2 | 35 | 29 | | 250 |
| 35.0073 | YQVAYQATV | HCV | | 19 | 25 | 4 | 10 | 35 | | 3636 |
| 35.0075 | YTVAYQATV | HCV | | 29 | 56 | 16 | 31 | 28 | | 1 |
| 35.0069 | YVVAYQATV | HCV | | 60 | 49 | 8 | 27 | 39 | | 2 |
| 1390.01 | ALBRWGLLL | Her2/neu.5 | A | 245 | 0.6 | 12 | | 5983 | | 8071 |
| 1382.03 | ATBRWGLLV | Her2/neu.5 | A | 14994 | 240 | 372 | | 2221 | | 1759 |
| 1382.04 | AVBRWGLLV | Het2/neu.5 | A | 9517 | 249 | 146 | | 2166 | | 4778 |
| F106.05 | CLLTLLGSYI | HIV | | 167 | | | | | | |
| F10629 | FIISLSLLI | HIV | | 76 | | | | | | |
| F106.34 | FITVLIFKV | HIV | | 115 | | | | | | |
| F106.14 | FLSLQIMDYL | HIV | | 142 | | | | | | |
| F106.19 | IIFSIAFITV | HIV | | 34 | | | | | | |
| F106.11 | IISLSLLIGV | HIV | | 161 | | | | | | |
| 54.0063 | ILHEPVHGV | HIV.pol.476 | A | 50 | | | | | | |
| 55.0158 | ILKDPVHGV | HIV.pol.476 | A | 355 | | | | | | |
| 55.0170 | ILKEFVHGV | HN.pol.476 | A | 58 | | | | | | |
| 1304.09 | ILKEPVMGV | HIV.pol.476 | A | 218 | | | | | | |
| 55.0169 | ILKESVHGV | HIV.pol.476 | A | 150 | | | | | | |
| 55.0168 | ILKETVHGV | HIV.pol.476 | A | 295 | | | | | | |
| 55.0167 | ILKFPVHGV | HIV-pol.476 | A | 136 | | | | | | |
| 55.0166 | IKYPVHGV | HIV.pol.476 | A | 306 | | | | | | |
| 55.0155 | ILLEPVHGV | HIV.pol.476 | A | 49 | | | | | | |
| 55.0153 | ILVEPVHGV | HIV.pol.476 | A | 459 | | | | | | |
| 1304.07 | ILYEPVHGV | HIV.pol.476 | A | 34 | | | | | | |
| 1304.15 | IMHEPVHGV | HIV.pol.476 | A | 83 | | | | | | |
| 1304.10 | IMKEPVHGV | HIV.pol.476 | A | 280 | | | | | | |
| 1304.17 | IMKEPVYGV | HIV.pol.476 | A | 187 | | | | | | |
| 1304.14 | IMYEPVHGV | HIV.pol.476 | A | 32 | | | | | | |
| 1304.01 | KLTPLCVTV | HIV.env.134 | A | 20 | | | | | | |
| F106.31 | KMMIIFSIA | HIV | | 251 | | | | | | |
| 1304.02 | KMTPLCVTL | HIV.env.134 | A | 20 | | | | | | |
| 1304.03 | KMTPLCVTV | HIV.env.134 | A | 9.3 | | | | | | |
| F106.04 | LLGSYIELPA | HIV | | 499 | | | | | | |
| F106.16 | MLFI1SLSL | HIV | | 38 | | | | | | |
| 1304.04 | MLKEPVHGV | HIV.pol.476 | A | 110 | | | | | | |
| 1304.11 | MMKEPVHGV | HIV.pol.476 | A | 97 | | | | | | |
| F106.10 | QIMDYLLCL | HIV | | 3.9 | | | | | | |
| F106.03 | QLLDFCLSI | HIV | | 0.56 | | | | | | |
| 11.0050 | RIHIGPGRA | HIV.gp160.311 | | 386 | | | | | | |
| F106.33 | RLIKCMNSV | HIV | | 387 | | | | | | |
| F106.12 | SLQIMDYLL | HIV | | 67 | | | | | | |
| F106.36 | TLLGSYIEL | HIV | | 19 | | | | | | |
| F106.26 | TLQLLDFCL | HIV | | 17 | | | | | | |
| F106.20 | TMLFIISLSL | HIV | | 155 | | | | | | |
| 1304.06 | WLKEPVHGV | HIV.pol.476 | A | 350 | | | | | | |
| F106.35 | YLLCLLTLL | HIV | | 21 | | | | | | |
| F106.18 | YLLPFLSLQI | HIV | | 3.2 | | | | | | |
| 1304.12 | YMKEPVHGV | HIV.pol.476 | A | 70 | | | | | | |
| Hafler C | LLNGYPVYV | HTLV.11 | A | 23 | | | 302 | | | |
| F018.03 | LLLDVPTAAVQAIP30-P15 | | | 41 | | | | | | |
| F018.02 | LLLDVPTAAV | IP30-P16 | | 8.6 | | | | | | |
| 71.0001 | ALGTTAYV | Kallikrein2.147 | A | 479 | 27 | 62 | | 37000 | | 6970 |
| 1418.12 | ALGTTCYV | Kallikrein2.147 | A | 18 | 5 | 16 | | 1028 | | 80000 |
| 71.0002 | ALGTTPYV | Kallikrein2.147 | A | 879 | 1232 | 273 | | 37000 | | 80000 |
| 71.0003 | ALGTTSYV | Kallikrein2.147 | A | 1753 | 120 | 96 | | 37000 | | 3508 |
| 71.0004 | ALGTTTYV | Kallikrein2.147 | A | 422 | 72 | 46 | | 18500 | | 10518 |
| 71.0005 | ALGTTVYV | Kallikrein2.147 | A | 59 | 12 | 15 | | 16547 | | >80000 |
| 1418.36 | ALPLIQSRIV | Kallikrein2.17 | A | 8333 | 1870 | 233 | | 7400 | | 80000 |
| 71.0006 | ALSVGATGV | Kallikrein2.9 | A | 774 | 94 | 40 | | 3482 | | 11795 |
| 1418.15 | ALSVGCTGV | Kallikrein2.9 | A | 24 | 17 | 9 | | 264 | | 40000 |
| 71.0007 | ALSVGPTGV | Kallikrein2.9 | A | 770 | 176 | 119 | | 37000 | | >80000 |
| 71.0008 | ALSVGSTGV | Kallikrein2.9 | A | 2428 | 72 | 77 | | 12827 | | >80000 |
| 71.0009 | ALSVGTTGV | Kallikrein2.9 | A | 2250 | 47 | 142 | | 12333 | | >80000 |
| 71.0010 | ALSVGVTGV | Kallikrein2.9 | A | 26 | 14 | 8 | | 671 | | >80000 |
| 1418.34 | ATSVGCTGAV | Kallikrein2.9 | A | 7143 | 8600 | 385 | | 3364 | | 8889 |
| 1418.16 | AVPLIQSRV | Kallikrein2.17 | A | 3846 | 1536 | 127 | | 2313 | | 73 |
| 1418.33 | AVSVGCTGAV | Kallikrein2.9 | A | 2381 | 2688 | 189 | | 740 | | 20000 |
| 1418.68 | DLCARAYSEKV | Kallikrein2.182 | A | 16667 | 2263 | 1282 | | 12333 | | 211 |
| 71.0079 | DLLLLRLSEPV | Kallikrein2.120 | A | 1070 | 528 | 115 | | 870 | | 10000 |
| 63.0109 | DLMLLRLSEPV | Kallikrein2.120 | A | 70 | 66 | 31 | | 119 | | 2759 |
| 1418.70 | DTCARAYSEKV | Kallikrein2.182 | A | 50000 | 1792 | 1786 | | 7400 | | 421 |
| 1418.32 | DTVLSIALSV | Kallikrein2.3 | A | 3571 | 1000 | 192 | | 712 | | 178 |
| 1418.69 | DVCARAYSEKV | Kallikrein2.182 | A | 50000 | 1593 | 67 | | 5286 | | 308 |
| 71.0011 | FLLAAGLWT | Kallikrein2.195 | A | 17 | 368 | 112 | | 302 | | 40000 |
| 71.0012 | FLLPAGLWT | Kallikrein2.195 | A | 2.7 | 375 | 70 | | 134 | | >80000 |
| 71.0013 | FLLSAGLWT | Kallikrein2.195 | A | 41 | 374 | 239 | | 66 | | 32660 |
| 710014 | FLLTAGLWT | Kallikrein2.195 | A | 14 | 193 | 190 | | 259 | | 40000 |
| 71.0077 | FLRPRSLQAV | Kallikrein2.165 | A | 353 | 30 | 12 | | 1013 | | 80000 |
| 1419.44 | FLRPRSLQBV | Kallikrein2. | | 38 | 2 | 6 | | 1850 | | 80000 |
| 1435.02 | FLRPRSLQPV | Kallikrein2. | | 4545 | 113 | 111 | | 3700 | | 80000 |
| 1435.01 | FLRPRSLQSV | Kallikrein2. | | 1389 | 36 | 26 | | 5286 | | 80000 |
| 71.0076 | FLRPRSLQTV | Kallikrein2.165 | A | 741 | 112 | 220 | | 1379 | | 21036 |
| 71.0075 | FLRPRSLQVV | Kallikrein2.165 | A | 363 | 16 | 19 | | 254 | | 26667 |
| 71.0016 | FMLAAGLWV | Kallikrein2.195 | A | 2.2 | 34 | 2.8 | | 11 | | 40000 |
| 1418.22 | FMLCAGLWV | Kallikrein2.195 | A | 29 | 12 | 91 | | 51 | | 80000 |
| 71.0017 | FMLPAGLWV | Kallikrein2.195 | A | 7.3 | 41 | 8.9 | | 6.3 | | 27734 |
| 71.0018 | FMLSAGLWV | Kallikrein2.195 | A | 3.0 | 15 | 4.4 | | 10 | | 19230 |
| 71.0019 | FMLTAGLWV | Kallikrein2.195 | A | 5.0 | 47 | 17 | | 15 | | 26667 |
| 71.0020 | FMLVAGLWV | Kallikrein2.195 | A | 13 | 277 | 14 | | 25 | | 40000 |
| 71.0022 | FTLPAGLWT | Kallikrein2.195 | A | 1469 | 21500 | 18257 | | 149 | | 18566 |
| 1418.51 | FTRPRSLQCV | Kallikrein2.165 | A | 4167 | 1000 | 4 | | 514 | | 5333 |
| 71.0026 | FVLAAGLWT | Kallikrein2.195 | A | 1150 | 1669 | 1601 | | 166 | | 34943 |
| 71.0027 | FVLPAGLWT | Kallikrein2.195 | A | 719 | 24826 | 6276 | | 115 | | 46188 |
| 71.0028 | FVLSAGLWT | Kallikrein2.195 | A | 1578 | 13598 | 8771 | | 304 | | 56569 |
| 1418.50 | FVRPRSLQCV | Kallikrein2.165 | A | 1389 | 269 | 11 | | 474 | | 8000 |
| 1418.63 | GLPTQEPALGV | Kallikrein2.140 | A | 1136 | 796 | 91 | | 3700 | | 80000 |
| 67.0063 | GTVPLIQSRI | Kallikrein2.16 | A | >61224.10 | 6448 | 11919 | | 6678 | | 85 |
| 1419.46 | HLLSNDMBARA | Kallikrein2. | A | 3571 | 143 | 105 | | 2313 | | 80000 |
| 1418.67 | HLLSNDMCARV | Kallikrein2.177 | A | 26 | 1 | 5 | | 37 | | 860 |
| 1418.20 | HLLSNDMCV | Kallikrein2.177 | A | 119 | 102 | 278 | | 176 | | 80000 |
| 71.0031 | ILLRLSEPV | Kallikrein2.122 | A | 112 | 61 | 177 | | 244 | | 24228 |
| 71.0080 | ILLSVGATGAV | Kallikrein2.8 | A | 2129 | 1882 | 187 | | 2383 | | 33193 |
| 1418.57 | ILLSVGCTGAV | Kallikrein2.8 | A | 36 | 33 | 36 | | 308 | | 10000 |
| 71.0081 | ILLSVGPTGAV | Kallikrein2.8 | A | 971 | 205 | 85 | | 7400 | | 11795 |
| 71.0082 | ILLSVGSTGAV | Kallikrein2.8 | A | 1927 | 1749 | 214 | | 15105 | | >56568.54 |
| 71.0083 | ILLSVGTTGAV | Kallikrein2.8 | A | 2221 | 986 | 144 | | 5286 | | >56568.54 |
| 71.0084 | ILLSVGVTGAV | Kallikein2.8 | A | 229 | 136 | 22 | | 481 | | 10053 |
| 1418.59 | ITLSVGCTGAV | Kallikrein2.8 | A | 294 | 134 | 40 | | 206 | | 121 |
| 1418.58 | IVLSVGCTGAV | Kallikrein2.8 | A | 1351 | 705 | 208 | | 597 | | 2162 |
| 67.0039 | KITDVVKVV | Kallikrein2.131 | A | 476 | 586 | 84 | | 859 | | 29314 |
| 67.0036 | KLTDVVKVL | Kallikrein2.131 | A | 712 | 15 | 20 | | 2439 | | 81679 |
| 67.0037 | KVTDVVKVL | Kallikrein2.131 | A | 5158 | 686 | 259 | | 2372 | | 3835 |
| 1435.05 | KVTEFMLAAGV | Kallikrein2. | | 179 | 33 | 13 | | 52 | | 308 |
| 1418.71 | KVTEFMLCAGV | Kallikrein2.191 | A | 56 | 10 | 26 | | 28 | | 143 |
| 1418.21 | KVTEFMLCV | Kallikrein2.191 | A | 53 | 27 | 31 | | 34 | | 6667 |
| 1435.07 | KVTEFMLPAGV | Kallikrein2. | | 2381 | 935 | 137 | | 385 | | 13333 |
| 1435.06 | KVTEFMLSAGV | Kallikrein2. | | 1786 | 430 | 56 | | 394 | | 3333 |
| 1435.04 | KVTEFMLTAGV | Kallikrein2. | | 7143 | 1536 | 119 | | 2643 | | 10000 |
| 71.0059 | LLLLRLSEPA | Kalliknin2.121 | A | 1130 | 748 | 96 | | 1097 | | 11547 |
| 71.0060 | LLLLRLSEPV | Kallikrein2.121 | A | 379 | 361 | 56 | | 317 | | 26667 |
| 71.0042 | LLLRLSEPV | Kallikrein2.122 | A | 35 | 82 | 90 | | 179 | | 25746 |
| 1418.13 | LLLSIALSV | Kallikrein2.4 | A | 88 | 179 | 147 | | 185 | | 80000 |
| 1418.44 | LLRLSEPAKV | Kallikrein2.123 | A | 2174 | 518 | 23 | | 9250 | | 80000 |
| 1418.53 | LLSNDMCARV | Kallilkrein2.178 | A | 5.3 | 0.69 | 4.4 | | 10.3 | | 1702 |
| 1418.43 | LMLLRLSEPV | Kallikrein2.121 | A | 114 | 67 | 29 | | 25 | | 6154 |
| 71.0062 | LTLLRLSEPV | Kallikrein2.121 | A | 1761 | 6207 | 860 | | 424 | | 949 |
| 1418.14 | LTLSIALSV | Kallikrein2.4 | A | 641 | 287 | 313 | | 949 | | 2051 |
| 71.0063 | LVANGVLQGV | Kallikrein2.121 | A | 125 | 25 | 3 | | 26 | | 12 |
| 1419.47 | LVBNGVLQGI | Kallikrein2. | A | 1282 | 215 | 9.1 | | 43 | | 118 |
| 1418.72 | LVCNGVLQGIV | Kallikrein2.217 | A | 1923 | 1593 | 500 | | 370 | | 13333 |
| 1418.55 | LVCNGVLQGV | Kallikrein2.217 | A | 10 | 3 | 12 | | 6 | | 3 |
| 1418.60 | LVHPQWVLTAV | Kallikrein2.54 | A | 2083 | 148 | 370 | | 148 | | 2424 |
| 1418.40 | LVHPQWVLTV | Kallikrein2.54 | A | 3571 | 1049 | 67 | | 77 | | 13333 |
| 71.0066 | LVPNGVLQGV | Kallikrein2217 | A | 28 | 15 | 4 | | 7 | | 20 |
| 71.0067 | LVSNGVLQGV | Kallikrein2.217 | A | 133 | 11 | 1 | | 23 | | 9 |
| 71.0068 | LVTNGVLQGV | Kallikrein2.217 | A | 182 | 56 | 2.2 | | 10 | | 17 |
| 71.0069 | LVVNGVLQGV | Kallikrein2.217 | A | 55 | 50 | 5 | | 40 | | 19 |
| 1418.18 | MLLRLSEPV | Kallikrein2.122 | A | 79 | 31 | 40 | | 161 | | 1778 |
| 1418.42 | NMSLLKHQSV | Kallikrein2.102 | A | 8333 | 1000 | 455 | | 18500 | | 40000 |
| 67.0040 | PLLGTTCYA | Kallikrein2.146 | A | 3541 | 434 | 114 | | 6111 | | 46839 |
| 71.0085 | PLVANGVLQGV | Kallikrein2.216 | A | 45 | 683 | 75 | | 212 | | 2843 |
| 63.0128 | PLVCNGVLQGV | Kallikrein2.216 | A | 92 | 421 | 36 | | 210 | | 1633 |
| 1419.17 | PLVCNGVLQGV | Kallikrein2.216 | A | 26 | 126 | 19 | | 264 | | 4211 |
| 71.0086 | PLVPNGVLQGV | Kallikrein2.216 | A | 26 | 93 | 18 | | 40 | | 17889 |
| 71.0087 | PLVSNGVLQGV | Kallikrein2.216 | A | 57 | 227 | 76 | | 287 | | 5234 |
| 71.0088 | PLVTNGVLQGV | Kallikrein2.216 | A | 138 | 1267 | 137 | | 882 | | 11507 |
| 71.0089 | PLVVNGVLQGV | Kallikrein2.216 | A | 348 | 5852 | 774 | | 683 | | 10000 |
| 67.0042 | PTLGTTCYA | Kallikrein2.146 | A | 9690 | 1268 | 223 | | 54852 | | 9485 |
| 67.0041 | PVLGTTCYA | Kallikrein2.146 | A | 8211 | 756 | 196 | | 6836 | | 10645 |
| 1418.38 | QVAVYSHGWV | Kallikrein2.39 | A | 12500 | 331 | 714 | | 3083 | | 333 |
| 1418.17 | QVWLGRHNV | Kallikrein2.72 | A | 1351 | 6143 | 2222 | | 1480 | | 103 |
| 67.0093 | SIALSVGCTGV | Kallikrein2.7 | A | 3959 | 544 | 136 | | 2137 | | 587 |
| 67.0090 | SLALSVGCTGA | Kallikrein2.7 | A | 4789 | 125 | 214 | | 3142 | | 5095 |
| 71.0090 | SLHLLSNDMAA | Kallikrein2.175 | A | 65 | 3 | 89 | | 1165 | | 20435 |
| 1419.45 | SLHLLSNDMBA | Kallikrein2. | | 12 | 1 | 36 | | 12333 | | 20000 |
| 14.18.66 | SLHLLSNDMCV | Kallikrein2.175 | A | 8.6 | 0.78 | 10.2 | | 2312.5 | | 2162.16 |
| 71.0091 | SLHLLSNDMPA | Kallikrein2.175 | A | 1687 | 41 | 717 | | 37000 | | >80000 |
| 71.0092 | SLHLLSNDMSA | Kallikrein2.175 | A | 346 | 4.0 | 84 | | 9250 | | >80000 |
| 71.0093 | SLHLLSNDMTA | Kallikrein2.175 | A | 93 | 2 | 44 | | 26163 | | 80000 |
| 71.0094 | SLHLLSNDMVA | Kallikrein2.175 | A | 112 | 5 | 74 | | 7126 | | 40000 |
| 71.0070 | SLQAVSLHLV | Kallikrein2.170 | A | 142 | 13 | 8 | | 219 | | 1404 |
| 1418.52 | SLQCVSLHLV | Kallikrein2.170 | A | 13 | 6 | 3 | | 5 | | 205 |
| 1418.19 | SLQCVSLHV | Kallikrein2.170 | A | 56 | 165 | 48 | | 4111 | | 1600 |
| 71.0071 | SLQPVSLHLV | Kallikrein2.170 | A | 346 | 21 | 35 | | 95 | | 5521 |
| 71.0072 | SLQSVSLHLV | Kallikrein2.170 | A | 202 | 37 | 73 | | 78 | | 6963 |
| 71.0073 | SLQTVSLHLV | Kallikrein2.170 | A | 170 | 42 | 11 | | 82 | | 2464 |
| 71.0074 | SLQWSLHLV | Kallikrein2.170 | A | 138 | 56 | 11 | | 63 | | 3036 |
| 1418.35 | SVGCTGAVPV | Kallikren2.11 | A | 104 | 287 | 154 | | 552 | | 216 |
| 1418.64 | TTCYASGWGSV | Kallikrein2.150 | A | 6250 | 2150 | 769 | | 2467 | | 500 |
| 1418.56 | VLSIALSVGCV | Kallikrein2.5 | A | 1020 | 1103 | 83 | | 1947 | | 3333 |
| 63.0105 | VLVHPQWVLTV | Kallikrein2.53 | A | 11 | 2 | 3 | | 13 | | 4444 |
| 1419.11 | VLVHPQWVLTV | Kallikrein2.53 | A | 11 | 2 | 16 | | 31 | | 8889 |
| 60.0180 | VLVHPQWVV | Kallikrein2.53 | A | 564 | 65 | 1982 | | 3199 | | 16000 |
| 1418.54 | VTEFMLCAGV | Kallikrein2.192 | A | 625 | 1483 | 714 | | 137 | | 131 |
| 1369.36 | FVEEQMTWV | KSHV.105 | A | 46 | 172 | 552 | 326 | 27 | | 6425 |
| 1369.35 | LVYHIYSKV | KSHV.153 | A | 319 | 133 | 16 | 1370 | 328 | | 152 |
| 7.0025 | HLNLTMPNA | LCMV.GP.83 | | 188 | | | | | | |
| 7.0045 | MPSLTMACM | LCMV.NP.175 | | 323 | | | | | | |
| 7.0039 | SAYLVSIFL | LCMV.GP.445 | | 130 | | | | | | |
| 55.0234 | YLQLFFGIEV | MAGE2.157 | A | 133 | 68 | 511 | | 482 | | 3497 |
| 54.0084 | YLQLIFGIEV | MAGE2.157 | A | 259 | 58 | 791 | | 462 | | 19372 |
| F063.65 | AAFIGILTV | MART1.27 | A | 76 | | | | | | |
| F063.64 | AAWIGILTV | MART1.27 | A | 67 | | | | | | |
| F063.66 | AAYIGILTV | MART1.27 | A | 102 | | | | | | |
| F063.60 | AIGIGILTV | MART1.27 | A | 86 | | | | | | |
| F063.74 | ALFIGILTV | MART1.27 | A | 2.1 | | | | | | |
| F063.58 | ALGIGILTV | MART1.27 | A | 11 | | | | | | |
| F063.73 | ALWIGILTV | MART1.27 | A | 5.8 | | | | | | |
| F063.75 | ALYIGILTV | MART1.27 | A | 3.9 | | | | | | |
| F063.59 | AMGIGILTV | MART1.27 | A | 15 | | | | | | |
| F063.62 | FAGIGILTV | MART1.27 | A | 268 | | | | | | |
| F063.71 | FLGIGILTV | MART1.27 | A | 1.4 | | | | | | |
| F063.69 | KIGIGILTV | MART1.27 | A | 103 | | | | | | |
| F063.67 | KLGIGILTV | MART1.27 | A | 20 | | | | | | |
| F063.68 | KMGIGILTV | MART1.27 | A | 27 | | | | | | |
| F063.70 | WLGIGILTV | MART1.27 | A | 9.8 | | | | | | |
| F063.63 | YAGIGILTV | MART1.27 | A | 304 | | | | | | |
| F063.72 | YLGIGILTV | MART1.27 | A | 2.5 | | | | | | |
| 39.0188 | KTCPVQLWVSA | mp53.136 | A | 458 | | | | | | |
| 1413.13 | LLDRDSFEV | mp53.261 | A | 4.0 | | | | | | |
| 141320 | LLGRDSHEV | mp53.261 | A | 8.3 | | | | | | |
| 1413.18 | LLGRDSLEV | mp53.261 | A | 177 | | | | | | |
| 1413.19 | LLGRDSMEV | mp53.261 | A | 417 | | | | | | |
| 1413.17 | LLGRGSFEV | mp53.261 | A | 192 | | | | | | |
| 1413.16 | LLGRQSFEV | mp53.261 | A | 76 | | | | | | |
| 1413.14 | LLHRDSFEV | mp53.261 | A | 2.8 | | | | | | |
| 1413.12 | LLSRDSFEV | mp53.261 | A | 29 | | | | | | |
| F097.02 | SIIDPLIYA | MSH.291 | A | 455 | 5.4 | 25 | | 17 | | 2.3 |
| F097.01 | SVMDPLIYA | MSH.291 | A | 63 | 6 | 88 | | 7 | | 8 |
| F097.12 | TMLLGVFTV | MSH.244 | A | 3.1 | | | | | | |
| 31.0143 | LLANNTRVWV | MT.242 | | 239 | | | | | | |
| 54.0005 | CMPPPGTRV | p53.149 | A | 788 | 1611 | 100 | | 2303 | | 7851 |
| 1326.04 | ELAPPVAPV | p53.68 | A | 60 | | | | | | |
| 1323.28 | FLHSGTAKSVV | p53.113 | A | 1222 | 568 | 28 | | 9087 | | 11001 |
| 1413.08 | GLAPPQQLIRV | p53.187 | A | 11 | | | | | | |
| 1413.10 | GLAPPQSLIRV | p53.187 | A | 3.3 | | | | | | |
| 1413.04 | GLVPPAHLIRV | p53.187 | A | 20 | | | | | | |
| 55.0049 | SMPPPGPRV | p53.149 | A | 641 | 429 | 1144 | | 3222 | | 702 |
| 1323.23 | YLCNSSCV | p53.236 | A | 14814 | 31114 | 138 | | >21361.90 | | >35777.09 |
| 63.0040 | ALFPPEGVSV | PAP.122 | A | 11 | 1 | 3 | | 12 | | 138 |
| 1419.61 | ALFPPEGVSV | PAP. | | 15 | 1 | 18 | | 119 | | 4444 |
| 60.0203 | FLFLLFFWV | PAP.18 | A | 42 | 307 | 625 | | 308 | | 90 |
| 63.0047 | GLHGQDLFGV | PAP.196 | A | 19 | 4 | 4 | | 6 | | 976 |
| 1419.62 | GLHGQDLFGV | PAP. | | 12 | 2 | 3 | | 18 | | 80000 |
| 63.0041 | GVSIWNPILV | PAP.128 | A | 250 | 93 | 23 | | 451 | | 2286 |
| 60.0207 | GVSIWNPIV | PAP.128 | A | 455 | 269 | 909 | | 308 | | 80000 |
| 71.0051 | ILYSAHDTTV | PAP.284 | A | 991 | 9.1 | 100 | | 1871 | | 24228 |
| 1389.06 | ILYSAHDTTV | PAP.384 | A | 391 | 1.1 | 13 | | 1471 | | 6218 |
| 1418.25 | ITLWQPIPV | PAP.135 | A | 33 | 1720 | 6 | | 26 | | 32 |
| 71.0052 | ITYSAHDTTV | PAP.284 | A | 25000 | 181 | 848 | | 1818 | | 733 |
| 1418.26 | ITYSAHDTTV | PAP.284 | A | 4167 | 115 | 238 | | 154 | | 82 |
| 1389.05 | IVLWQPIPV | PAP.135 | A | 391 | 1865 | 289 | | 17607 | | 831 |
| 71.0053 | IVYSAHDTTV | PAP.284 | A | 16667 | 289 | 7001 | | 1149 | | 1552 |
| 1389.07 | IVYSAHDTTV | PAP.284 | A | 7710 | 91 | 623 | | 671 | | 745 |
| 63.0048 | KLRELSELSV | PAP.234 | A | 263 | 9.2 | 7.1 | | 49 | | 1818 |
| 63.0046 | KLSGLHGQDV | PAP.193 | A | 1064 | 69 | 385 | | 1057 | | 26667 |
| 63.0038 | LLALFPPEGV | PAP.120 | A | 31 | 0 | 5 | | 37 | | 160 |
| 1419.58 | LLALFPPEGV | PAP. | | 5.0 | 0.7 | 1.6 | | 148 | | 163 |
| 63.0031 | LLARAASLSV | PAP.7 | A | 109 | 10 | 21 | | 378 | | 727 |
| 60.0201 | LLLARAASV | PAP.6 | A | 18 | 215 | 7 | | 95 | | 20000 |
| 63.0053 | LLPPYASCHV | PAP.306 | A | 94 | 18 | 15 | | 218 | | 2963 |
| 1419.64 | LLPPYASCHV | PAP. | | 88 | 15 | 16 | | 97 | | 5333 |
| 63.0044 | LLWQPIPVHV | PAP.136 | A | 5.8 | 2.1 | 18 | | 116 | | 42 |
| 1419.69 | LLWQPIPVHV | PAP. | | 25 | 2 | 18 | | 285 | | 62 |
| 1418.23 | LTFFWLDRSV | PAP.21 | A | 116 | 11 | 10 | | 43 | | 16 |
| 63.0039 | LVALFPPEGV | PAP.120 | A | 116 | 14 | 12 | | 47 | | 3 |
| 1419.59 | LVALFPPEGV | PAP. | | 156 | 17 | 5 | | 463 | | 28 |
| 1389.01 | LVFFWLDRSV | PAP.31 | A | 1424 | 259 | 80 | | 64 | | 82 |
| 63.0042 | PLLLWQPIPV | PAP.134 | A | 238 | 47 | 19 | | 336 | | 3333 |
| 63.0045 | RLHPYKDFIV | PAP.180 | A | 862 | 24 | 1887 | | 1609 | | 80000 |
| 63.0033 | SLLAKELKFV | PAP.29 | A | 64 | 6 | 4 | | 38 | | 6667 |
| 63.0032 | SLSLGFLFLV | PAP.13 | A | 33 | 11 | 27 | | 40 | | 842 |
| 1419.52 | SLSLGFLFLV | PAP. | | 1.9 | 3.9 | 17 | | 42 | | 348 |
| 60.0202 | SLSLGFLFV | PAP.13 | A | 42 | 6 | 5 | | 28 | | 4706 |
| 1419.50 | SLSLGFLFV | PAP. | A | 77 | 25 | 21 | | 93 | | 26667 |
| 63.0037 | TLMSAMTNLV | PAP.112 | A | 63 | 4 | 12 | | 43 | | 242 |
| 1389.03 | TLMSAMTNV | PAP.112 | A | 628 | 14 | 35 | | 2159 | | 482 |
| 1419.56 | TLMSAMTNV | PAP. | | 9.6 | 2.39 | 3.57 | | 54.41 | | 61.54 |
| 60.0213 | TVSGLQMAV | PAP.292 | A | 294 | 12 | 122 | | 195 | | 5.7 |
| 63.0034 | VLAKELKFVV | PAP.30 | A | 31 | 12 | 189 | | 86 | | 2286 |
| 1418.24 | VTAKELKFV | PAP.30 | A | 7143 | 2688 | 40 | | 137 | | 26667 |
| 1419.53 | VTAKELKFV | PAP. | | 6250 | 1024 | 53 | | 137 | | 40000 |
| 1419.54 | VVAKELKFV | PAP. | | 926 | 478 | 22 | | 119 | | 40000 |
| 1369.21 | IVSVSSFLFV | Pf.CSP.7 | A | 1066 | 797 | 200 | 993 | 77 | | 437 |
| F103.01 | ALYGALLLA | PLP.80 | A | 7.8 | | | | | | |
| F103.05 | FLYGAALLA | PLP.80 | A | 3.6 | | | | | | |
| F103.06 | FLYGALALA | PLP.80 | A | 2.4 | | | | | | |
| F103.07 | FLYGALLAA | PLP.80 | A | 6.4 | | | | | | |
| F103.08 | FLYGALRLA | PLP.80 | A | 7.2 | | | | | | |
| F103.09 | FLYGALVLA | PLP.80 | A | 41 | | | | | | |
| F103.10 | FLYGALYLA | PLP.80 | A | 2.6 | | | | | | |
| F103.04 | FLYGGLLLA | PLP.80 | A | 1.1 | | | | | | |
| 1419.34 | ALGTTBYA | PSA. | | 50 | 13 | 0 | | 18500 | | 80000. |
| 1419.35 | ALGTTBYV | PSA. | | 6.7 | 3.6 | 2.8 | | 3083 | | 80000 |
| 939.09 | ALGTTCYAS | PSA. | | 417 | 741 | 667 | | 435 | | 80000 |
| 1389.14 | ALGTTCYV | PSA.143 | A | 93 | 7 | 12 | | 288 | | 29169 |
| 63.0195 | DLMLLRLSEPV | PSA.116 | A | 359 | 180 | 183 | | 1746 | | 5000 |
| 63.0186 | FLTLSVTWIGV | PSA.3 | A | 5.2 | 3.5 | 20 | | 74 | | 114 |
| 60.0216 | FLTLSVTWV | PSA.3 | A | 96 | 9 | 12 | | 58 | | 13333 |
| 1419.36 | FLTPKKLQBV | PSA. | | 71 | 3 | 4 | | 137 | | 80000 |
| 71.0047 | FLTPKKLQPV | PSA.161 | A | 5270 | 204 | 65 | | 1232 | | >80000 |
| 71.0048 | FLTPKKLQSV | PSA.161 | A | 607 | 51 | 52 | | 836 | | >80000 |
| 71.0049 | FLTPKKLQTV | PSA.161 | A | 876 | 55 | 57 | | 1318 | | >80000 |
| 71.0050 | FLTPKKLQVV | PSA.161 | A | 1548 | 33 | 48 | | 1230 | | >80000 |
| 1418.27 | FTTPKKLQCV | PSA.161 | A | 5000 | 1387 | 15 | | 308 | | 26667 |
| 1389.16 | FVTPKKLQCV | PSA.161 | A | 24628 | 2737 | 30 | | 7102 | | >23094.01 |
| 63.0203 | HLISNDVCAQV | PSA.173 | A | 2724 | 84 | 426 | | 15321 | | 308 |
| 71.0032 | KLQAVDLHV | PSA.166 | A | 504 | 471 | 818 | | 9553 | | 40000 |
| 71.0054 | KLQAVDLHVV | PSA.166 | A | 92 | 9 | 32 | | 211 | | >40000 |
| 1419.37 | KLQBVDLHV | PSA. | | 31 | 36 | 67 | | 712 | | 80000 |
| 1419.38 | KLQBVDLHVI | PSA. | | 217 | 27 | 20 | | 264 | | 80000 |
| 1419.39 | KLQBVDLHVV | PSA. | | 38 | 20 | 5 | | 86 | | 80000 |
| 63.0058 | KLQCVDLHVV | PSA.166 | A | 13 | 89 | 29 | | 528 | | 26667 |
| 71.0055 | KLQPVDLHVV | PSA.166 | A | 1023 | 58 | 30 | | 130 | | >80000 |
| 71.0056 | KLQSVDLHVV | PSA.166 | A | 526 | 27 | 23 | | 446 | | >80000 |
| 71.0057 | KLQTVDLHVV | PSA.166 | A | 233 | 47 | 34 | | 171 | | 30526 |
| 71.0036 | KLQVVDLHV | PSA.166 | A | 1135 | 350 | 554 | | 12333 | | >80000 |
| 71.0058 | KLQVVDLHVV | PSA.166 | A | 49 | 41 | 4 | | 92 | | 34943 |
| 71.0037 | KVTKFMLAV | PSA.187 | A | 144 | 136 | 11 | | 34 | | 7628 |
| 1419.40 | KVTKFMLBA | PSA. | | 1786 | 139 | 20 | | 58 | | 40000 |
| 1419.41 | KVTKFMLBV | PSA. | | 128 | 226 | 5 | | 31 | | 6667 |
| 60.0220 | KVTKFMLCV | PSA.187 | A | 70 | 456 | 80 | | 143 | | 16000 |
| 71.0038 | KVTKFMLPV | PSA.187 | A | 33 | 71 | 9 | | 15 | | 1827 |
| 71.0039 | KVTKFMLSV | PSA.187 | A | 90 | 180 | 2 | | 33 | | 11547 |
| 71.0040 | KVTKFMLTV | PSA.187 | A | 83 | 95 | 9 | | 48 | | 11547 |
| 71.0041 | KVTKFMLVV | PSA.187 | A | 223 | 371 | 11 | | 67 | | 18267 |
| 1389.12 | MLLRLSEPAEV | PSA.118 | A | 255 | 367 | 130 | | 2627 | | 4905 |
| 1389.10 | MLLRLSEPV | PSA.118 | A | 48 | 29 | 48 | | 686 | | 432 |
| 1419.43 | PLVBNGVLQGV | PSA. | | 14 | 269 | 185 | | 394 | | 6154 |
| 63.0194 | SVFHPEDTGQV | PSA.75 | A | 1725 | 50 | 57 | | 2064 | | 32 |
| 60.0217 | TLSVTWIGV | PSA.5 | A | 27 | 4 | 33 | | 777 | | 229 |
| 63.0191 | VLVHPQWVLTV | PSA.49 | A | 68 | 14 | 36 | | 360 | | 10000 |
| 60.0218 | VLVHPQWVV | PSA.49 | A | 267 | 206 | 1877 | | 4696 | | 6154 |
| 63.0185 | VVFLTLSVTWV | PSA.1 | A | 101 | 202 | 78 | | 885 | | 13333 |
| F108.33 | FVNHDFTVV | pSI2.508 | | 305 | | | | | | |
| F108.29 | IAGGVMAVV | pSI2.71 | | 204 | | | | | | |
| F108.44 | LLGLWGLTGL | pSI2.668 | | 1.4 | | | | | | |
| F108.35 | LLLLGLWGL | pSI2.666 | | 22 | | | | | | |
| F108.31 | SVYVDAKLV | pSI2.589 | | 117 | | | | | | |
| F108.30 | VLAKDGTEV | pSI2.50 | | 51 | | | | | | |
| 60.0232 | ALGLPSIPV | PSM.286 | A | 71 | 6 | 13 | | 3147 | | 5477 |
| 63.0063 | ALVLAGGFFV | PSM.25 | A | 713 | 65 | 798 | | 1404 | | 28522 |
| 1418.30 | ATFDIESKV | PSM.711 | A | 238 | 27 | 43 | | 82 | | 258 |
| 1389.34 | AVFDIESKV | PSM.711 | A | 850 | 62 | 41 | | 111 | | 207 |
| 60.0229 | ELAHYDVLV | PSM.109 | A | 4412 | 40 | 221 | | 111874 | | 86 |
| 63.0068 | FLDELKAENV | PSM.61 | A | 419 | 24 | 262 | | 4800 | | 43248 |
| 60.0239 | GLLGSTEWV | PSM.427 | A | 415 | 87 | 138 | | 393 | | >84187.21 |
| 1389.22 | GLPEGDLVYV | PSM.168 | A | 43 | 2 | 2 | | 113 | | 1000 |
| 1389.24 | GLPSIPVHPV | PSM.288 | A | 77 | 1 | 1 | | 315 | | 155 |
| 1418.29 | GTPEGDLVYV | PSM.168 | A | 313 | 134 | 53 | | 40 | | 571 |
| 1389.23 | GVPEGDLVYV | PSM.168 | A | 1401 | 258 | 836 | | 214 | | 711 |
| 63.0065 | LLGFLFGWFV | PSM.34 | A | 55 | 9 | 100 | | 2696 | | 88410 |
| 63.0079 | LLQERGVAYV | PSM.441 | A | 535 | 8 | 19 | | 616 | | 9038 |
| 71.0043 | LLYSLVHNL | PSM.469 | A | 13 | 1 | 10 | | 61 | | 376 |
| 71.0044 | LTYSLVHNL | PSM.469 | A | 20 | 2 | 14 | | 15 | | 2 |
| 63.0064 | LVLAGGFFLV | PSM.26 | A | 781 | 44 | 151 | | 182 | | 734 |
| 60.0224 | LVLAGGFFV | PSM.26 | A | 95 | 81 | 1031 | | 37 | | 592 |
| 71.0045 | LVYSLVHNL | PSM.469 | A | 272 | 7.4 | 147 | | 53 | | 18 |
| 63.0085 | MLFELANSIV | PSM.583 | A | 42 | 4 | 13 | | 440 | | 837 |
| 60.0247 | MVFELANSV | PSM.583 | A | 75 | 7 | 11 | | 50 | | 2 |
| 63.0060 | NVLHETDSAV | PSM.3 | A | 6300 | 97 | 583 | | 5087 | | 11149 |
| 60.0244 | PLFKYHLTV | PSM.568 | A | 962 | 119 | 167 | | 1779 | | 90571 |
| 1389.32 | QLMFLERAFV | PSM.667 | A | 553 | 12 | 50 | | 739 | | 2 |
| 60.0251 | QLYVAAFTV | PSM.731 | A | 2328 | 122 | 1233 | | 4039 | | 198 |
| 60.0252 | QVYVAAFTV | PSM.731 | A | 9602 | 6544 | 7795 | | 733 | | 26 |
| 63.0090 | SLFSAVKNFV | PSM.631 | A | 598 | 8 | 85 | | 2203 | | 1024 |
| 60.0242 | SLYETYELV | PSM.554 | A | 71 | 6 | 17 | | 86 | | 5178 |
| 60.0240 | TLRVDCTPV | PSM.461 | A | 5851 | 598 | 146 | | 10706 | | 47483 |
| 1389.20 | VLAGGFFLV | PSM.27 | A | 28 | 0 | 8 | | 57 | | 244 |
| 63.0086 | VLPFDCRDYV | PSM.592 | A | 806 | 76 | 392 | | 1546 | | 8033 |
| 63.0062 | WLCAGALVLV | PSM.20 | A | 4252 | 74 | 214 | | 2510 | | 39011 |
| 53.0065 | AANPHATFGV | T.cruzi.74 | | 15 | | | | | | |
| 53.0083 | AASTLLYATV | T.cruzi.FL160.377 | | 477 | | | | | | |
| 53.0034 | AIVFDHYDV | T.cruzi.FL160.108 | | 332 | | | | | | |
| 53.0038 | ALKNNGKVV | T.cruzi.FL160.230 | | 409 | | | | | | |
| 53.0004 | ALSLAAVLV | T.cruzi.7 | | 113 | | | | | | |
| 53.0062 | ALSLAAVLVV | T.cruzi.7 | | 101 | | | | | | |
| 53.0091 | AMALIGDSTV | T.cruzi.FL160.972 | | 356 | | | | | | |
| 53.0048 | FANSKFTLV | T.cruzi.FL160.529 | | 377 | | | | | | |
| 53.0087 | FANSKFTLVA | T.cruzi.FL160.529 | | 214 | | | | | | |
| 53.0011 | FLARLHAAA | T.cruzi.67 | | 48 | | | | | | |
| 53.0068 | GLMNNAFEWI | T.cruzi.188 | | 47 | | | | | | |
| 53.0088 | IILNGSLLTL | T.cruzi.FL160.581 | | 62 | | | | | | |
| 53.0045 | KLYCSYEVA | T.cruzi.FL160.401 | | 256 | | | | | | |
| 53.0061 | LLGLWGTAA | T.cruzi.FL160.993 | | 333 | | | | | | |
| 53.0090 | LLLEHGQFDL | T.cruzi.FL160.961 | | 37 | | | | | | |
| 53.0094 | LLLLGLWGTA | T.cruzi.FL160.991 | | 245 | | | | | | |
| 53.0021 | LLVGYNDSA | T.cruzi.287 | | 195 | | | | | | |
| 53.0013 | LMNNAFEWI | T.cruzi.189 | | 447 | | | | | | |
| 53.0067 | LTNLSEQMLV | T.cruzi.167 | | 175 | | | | | | |
| 53.0018 | MTYTGGVMT | T.cruzi.267 | | 356 | | | | | | |
| 53.0089 | QMDYSNGLFV | T.cruzi.FL160.610 | | 94 | | | | | | |
| 53.0042 | RLHLWLSDM | T.cruziFL160.352 | | 72 | | | | | | |
| 53.0092 | RVFGLLLLGL | T.cruzi.FL160.987 | | 62 | | | | | | |
| 53.0026 | RVFTSAVLL | T.cruzi.FL160.4 | | 11 | | | | | | |
| 53.0010 | SVFRENLFL | T.cruzi.60 | | 59 | | | | | | |
| 53.0028 | VLLLVVVMM | T.cruzi.FL160.10 | | 55 | | | | | | |
| 53.0008 | VMACLVPAA | T.cruzi.16 | | 10 | | | | | | |
| 41.0161 | AACDQRVLIV | TRP1 | | 44 | | | | | | |
| 41.0173 | AVVAALLLVA | TRP1 | | 13 | | | | | | |
| 41.0159 | DLLPSSGPGT | TRP1 | | 420 | | | | | | |
| 41.0188 | DLLPSSGPGV | TRP1 | | 94 | | | | | | |
| 41.0113 | ELPNPNHSM | TRP1 | | 44 | | | | | | |
| 41.0156 | FLMLFYQVWA | TRP1 | | 62 | | | | | | |
| 41.0179 | LIFGTASYLI | TRP1 | | 160 | | | | | | |
| 41.0064 | LLLFQQARA | TRP1 | | 211 | | | | | | |
| 41.0182 | LLTDHYQRYA | TRP1 | | 16 | | | | | | |
| 41.0176 | LLVALIFGTA | TRP1 | | 91 | | | | | | |
| 41.0069 | MAKRTTHPL | TRP1 | | 22 | | | | | | |
| 41.0185 | PLLLFQQARV | TRP1 | | 6.2 | | | | | | |
| 41.0144 | PLTNTEMFV | TRP1 | | 21 | | | | | | |
| 41.0081 | QLERDMQEM | TRP1 | | 7.5 | | | | | | |
| 41.0128 | QLERDMQEV | TRP1 | | 3.3 | | | | | | |
| 41.0089 | RLPEPQDVT | TRP1 | | 369 | | | | | | |
| 41.0165 | SLEEYDTLGT | TRP1 | | 13 | | | | | | |
| 41.0076 | SVKKTFLGV | TRP1 | | 9.2 | | | | | | |
| 41.0074 | SVYNYFVWT | TRP1 | | 3.5 | | | | | | |
| 41.0087 | TLGTLCNST | TRP1 | | 283 | | | | | | |
| 41.0095 | VLLHTFTDA | TRP1 | | 21 | | | | | | |
| F096.41 | ALVGLFVLL | TRP2.482 | | 28 | 13544 | 3208 | | 4447 | | 2731 |
| F096.14 | FVWLHYYSV | TRP2.185 | | 15 | 13544 | 974 | | 24 | | 1 |
| F096.18 | LIGNESFAL | TRP2.234 | | 400 | 1817 | 8513 | | 2855 | | 4000 |
| F096.32 | NMVPFFPPV | TRP2.431 | | 6.1 | 28 | 23 | | 7.1 | | 1.1 |
| F096.22 | SLDDYNHLV | TRP2.288 | | 26 | 1159 | 441 | | 735 | | 2582 |
| F096.48 | SLHNLVHSFL | TRP2.367 | | 277 | 139 | 8.4 | | 37000 | | 252 |
| F096.13 | SVYDFFVWL | TRP2.180 | | 36 | 169 | 226 | | 10 | | 1 |
| F096.26 | TLDSQVMSL | TRP2.360 | | 167 | 43000 | 646 | | 1217 | | >10596.26 |
| F096.50 | TLLVVMGTLV | TRP2.472 | | 361 | 10750 | 363 | | 1818 | | 596 |
| F096.51 | TLVALVGLFV | TRP2.479 | | 34 | 21500 | 35 | | 57 | | 6 |
| F096.30 | VLHSFTDAI | TRP2.394 | | 424 | 623 | 45 | | 9889 | | 1527 |
| F096.38 | VMGTLVALV | TRP2.476 | | 31 | 585 | 6 | | 404 | | 267 |
| F096.16 | VTWHRYHLL | TRP2.217 | | 164 | 1087 | 9410 | | 760 | | 38 |
| F096.37 | VVMGTLVAL | TRP2.475 | | 67 | 66 | 163 | | 51 | | 43 |
| F096.33 | YAIDLPVSV | TRP2.455 | | 16 | 46 | 14 | | 14 | | 1 |
| F096.08 | YVITTQHWL | TRP2.156 | | 105 | 41 | 417 | | 44 | | 1 |
| 980.05 | AAAKAAAAV | Artificial sequence | A | 8333 | 861 | 68 | 345 | 8273 | | 3 |
| 980.13 | ACAKAAAAV | Artificial sequence | A | 25000 | >8600 | 640 | 46237 | >1321.43 | | 314 |
| 980.10 | AGAKAAAAV | Artificial sequence | A | 25000 | 4389 | 418 | 3202 | 37000 | | 1960 |
| 953.15 | AIAKAAAAAL | Artificial sequence | A | >217.39 | | | | | | |
| 953.23 | AIAKAAAAAT | Artificial sequence | A | >217.39 | | | | | | |
| 953.11 | AIAKAAAAL | Artificial sequence | A | 50000 | 281 | 133 | | 37000 | | 121 |
| 953.19 | AIAKAAAAT | Artificial sequence | A | >238.1 | 43000 | 2113 | | >1321.43 | | 2333 |
| 953.03 | AIAKAAAAV | Artificial sequence | A | 1786 | 253 | 4.9 | 185 | 1277 | | 19 |
| 980.17 | ALAEAAAAV | Artificial sequence | A | 72 | | | | | | |
| 980.31 | ALAKAAAAA | Artificial sequence | A | 1563 | 21500 | 59 | 6604 | >1321.43 | | 6532 |
| 980.29 | ALAKAAAAI | Artificial sequence | A | 625 | 377 | 11 | 385 | 13081 | | 433 |
| 953.09 | ALAKAAAAL | Artificial sequence | A | 2778 | 80 | 4.2 | 109 | 15105 | | 320 |
| 980.30 | ALAKAAAAM | Artificial sequence | A | 4545 | 624 | 149 | 797 | 37000 | | >3468.44 |
| 953.17 | ALAKAAAAT | Artificial sequence | A | 25000 | 14333 | 175 | 9840 | >1321.43 | | 14142 |
| 980.28 | ALAKAAALV | Artificial sequence | A | 122 | | | | | | |
| 980.26 | ALAKAAAQV | Artificial sequence | A | 278 | | | | | | |
| 980.27 | ALAKAAATV | Artificial sequence | A | 294 | | | | | | |
| 980.24 | ALAKAAYAV | Artificial sequence | A | 64 | | | | | | |
| 980.22 | ALAKAIAAV | Artificial sequence | A | 93 | | | | | | |
| 980.19 | ALAKKAAAV | Artificial sequence | A | 294 | | | | | | |
| 980.21 | ALAKLAAAV | Artificial sequence | A | 114 | | | | | | |
| 980.20 | ALAKYAAAV | Artificial sequence | A | 119 | | | | | | |
| 980.18 | ALASAAAAV | Artificial sequence | A | 139 | | | | | | |
| 1266.02 | ALDKAYVLL | Artificial sequence | A | 59 | | | | | 212 | |
| 1266.01 | ALDKYTVLL | Artificial sequence | A | 80 | | | | | 648 | |
| 980.14 | ALFKAAAAV | Artificial sequence | A | 119 | | | | | | |
| 980.15 | ALSKAAAAV | Artificial sequence | A | 294 | | | | | | |
| 1037.02 | AMAKAAAAA | Artificial sequence | A | 2529 | 3011 | 5.9 | | 37000 | | 2818 |
| 953.22 | AMAKAAAAAT | Artificial sequence | A | >217.39 | | | | | | |
| 953.10 | AMAKAAAAL | Artificial sequence | A | 22 | 123 | 4 | | 18500 | | 320 |
| 1037.03 | AMAKAAAAM | Artificial sequence | A | 6455 | 1344 | 106 | | >1321.43 | | 7807 |
| 1037.13 | AMAKAAAAS | Artificial sequence | A | 25000 | >10750 | 166 | | >1321.43 | | >4588.31 |
| 953.18 | AMAKAAAAT | Artificial sequence | A | 16667 | 12413 | 84 | | >1321.43 | | 10000 |
| 953.02 | AMAKAAAAV | Artificial sequence | A | 273 | 196 | 6.7 | 69 | 1485 | | 177 |
| 953.02 | AMAKAAAAV | Artificial sequence | A | 131 | | | | | | |
| 953.02 | AMAKAAAAV | Artificial sequence | A | 167 | | | | | | |
| 980.06 | ANAKAAAAV | Artificial sequence | A | 25000 | >8600 | 4454 | 50000 | >1321.43 | | 109 |
| 980.12 | APAKAAAAV | Artificial sequence | A | 25000 | >8600 | 15430 | >24826.0t | >1321.43 | | 60 |
| 980.04 | ASAKAAAAV | Artificial sequence | A | 16667 | 1109 | 241 | 2847 | 26163 | | 14 |
| 953.16 | AVAKAAAAAL | Artificial sequence | A | >217.39 | | | | | | |
| 953.12 | AVAKAAAAL | Artificial sequence | A | >238.1 | 90 | 241 | | 8273 | | 4.1 |
| 953.20 | AVAKAAAAT | Artificial sequence | A | 50000 | 8600 | 2595 | | >1321.43 | | 157 |
| 953.04 | AVAKAAAAV | Artificial sequence | A | 2362 | 105 | 12 | 30 | 863 | | 1.2 |
| 95345 | AXAKAAAAL | Artificial sequence | A | 50000 | 467 | 3226 | | 37000 | | 11429 |
| 953.25 | AXAKAAAAL | Artificial sequence | A | >25000 | 469 | 3300 | | 37000 | | >3468.44 |
| 980.03 | FLAKAAAAV | Artificial sequence | A | 114 | | | | | | |
| 1266.07 | FLDSDYFPSI | Artificial sequence | A | 196 | | | | | 3240 | |
| 1266.06 | FLDSDYFPSL | Artificial sequence | A | 321 | | | | | 1571 | |
| 1266.05 | FLDSDYFPSV | Artificial sequence | A | 11 | | | | | 668 | |
| 1266.08 | FLDSYIAPL | Artificial sequence | A | 25 | | | | | 60 | |
| 980.02 | KLAKAAAAV | Artificial sequence | A | 238 | | | | | | |

**Table 16**

| Sequence | Protein/Segment | 1st Position | Analog | AA | A*0201 | A*0202 | A*0203 | A*0206 | A*6802 | A2 Cross-reactivity |
|---|---|---|---|---|---|---|---|---|---|---|
| LQCVSLHLL | Kallikrein2 | 171 | | 9 | 0.0018 | 0.010 | 0.0094 | 0.17 | 0.0002 | 2 |
| ALGTTCYV | Kallikrein2 | 147 | A | 8 | 0.28 | 0.93 | 0.63 | 0.0036 | 0.0001 | 3 |
| LLLSIALSV | Kallikrein2 | 4 | A | 9 | 0.057 | 0.024 | 0.068 | 0.020 | 0.0001 | 4 |
| LTLSIALSV : | Kallikrein2 | 4 | A | 9 | 0.0078 | 0.013 | 0.032 | 0.0039 | 0.0039 | 2 |
| ALSVGCTGV | Kallikrein2 | 9 | A | 9 | 0.21 | 0.26 | 1.1 | 0.014 | 0.0002 | 4 |
| AVPLIQSRV | Kallikrein2 | 17 | A | 9 | 0.0013 | 0.0028 | 0.079 | 0.0016 | 0.11 | 2 |
| QVWLGRHNV | Kallikrein2 | 72 | A | 9 | 0.0037 | 0.0007 | 0.0045 | 0.0025 | 0.078 | 1 |
| MLLRLSEPV | Kallikrein2 | 122 | A | 9 | 0.063 | 0.14 | 0.25 | 0.023 | 0.0045 | 4 |
| SLQCVSLHV | Kallikrein2 | 170 | A | 9 | 0.089 | 0.026 | 0.21 | 0.0009 | 0.0050 | 3 |
| HLLSNDMCV | Kallikrein2 | 177 | A | 9 | 0.042 | 0.042 | 0.036 | 0.021 | 0.0001 | 4 |
| KVTEFMLCV | Kallikrein2 | 191 | A | 9 | 0.095 | 0.16 | 0.32 | 0.11 | 0.0012 | 4 |
| FMLCAGLWV | Kallikrein2 | 195 | A | 9 | 0.17 | 0.37 | 0.11 | 0.073 | 0.0001 | 4 |
| LTFFWLDRSV | PAP | 21 | A | 10 | 0.043 | 0.40 | 1.0 | 0.086 | 0.49 | 5 |
| VTAKELKFV | PAP | 30 | A | 9 | 0.0007 | 0.0016 | 0.25 | 0.027 | 0.0003 | 2 |
| ITLWQPIPV | PAP | 135 | A | 9 | 0.15 | 0.0025 | 1.6 | 0.14 | 0.25 | 4 |
| ITYSAHDTTV | PAP | 284 | A | 10 | 0.0012 | 0.0375 | 0.042 | 0.024 | 0.097 | 4 |
| FTTPKKLQCV | PSA | 161 | A | 10 | 0.0010 | 0.0031 | 0.65 | 0.012 | 0.0003 | 2 |
| GTPEGDLVYV | PSM | 168 | A | 10 | 0.016 | 0.032 | 0.19 | 0.093 | 0.014 | 4 |
| ATFDIESKV | PSM | 711 | A | 9 | 0.021 | 0.16 | 0.23 | 0.045 | 0.031 | 5 |
| DTVLSIALSV | Kallikrein2 | 3 | A | 10 | 0.0014 | 0.0043 | 0.052 | 0.0052 | 0.045 | 2 |
| SVGCTGAVPV | Kallikrein2 | 11 | A | 10 | 0.048 | 0.015 | 0.065 | 0.0067 | 0.037 | 4 |
| QVAVYSHGWV | Kallikrein2 | 39 | A | 10 | 0.0004 | 0.013 | 0.014 | 0.0012 | 0.024 | 2 |
| LVHPQWVLTV | Kallikrein2 | 54 | A | 10 | 0.0014 | 0.0041 | 0.15 | 0.048 | 0.0006 | 2 |
| LMLLRLSEPV | Kallikrein2 | 121 | A | 10 | 0.044 | 0.064 | 0.35 | 0.15 | 0.0013 | 4 |
| FVRPRSLQCV | Kallikrein2 | 165 | A | 10 | 0.0036 | 0.016 | 0.92 | 0.0078 | 0.0010 | 3 |
| SLQCVSLHLV | Kallikrein2 | 170 | A | 10 | 0.39 | 0.68 | 3.6 | 0.71 | 0.039 | 5 |
| LLSNDMCARV | Kallikrein2 | 178 | A | 10 | 0.94 | 6.2 | 2.3 | 0.36 | 0.0047 | 4 |
| VTEFMLCAGV | Kallikrein2 | 192 | A | 10 | 0.0080 | 0.0029 | 0.014 | 0.027 | 0.061 | 2 |
| LVCNGVLQGV | Kallikrein2 | 217 | A | 10 | 0.48 | 1.5 | 0.84 | 0.66 | 2.3 | 5 |
| ILLSVGCTGAV | Kallikrein2 | 8 | A | 11 | 0.14 | 0.13 | 0.28 | 0.012 | 0.0008 | 4 |
| ITLSVGCTGAV | Kallikrein2 | 8 | A | 11 | 0.017 | 0.032 | 0.25 | 0.018 | 0.066 | 5 |
| LVHPQWVLTAV | Kallikrein2 | 54 | A | 11 | 0.0024 | 0.029 | 0.027 | 0.025 | 0.0033 | 3 |
| SLHLLSNDMCV | Kallikrein2 | 175 | A | 11 | 0.58 | 5.5 | 0.98 | 0.0016 | 0.0037 | 3 |
| HLLSNDMCARV | Kallikrein2 | 177 | A | 11 | 0.19 | 3.3 | 1.9 | 0.099 | 0.0093 | 4 |
| DVCARAYSEKV | Kallikrein2 | 182 | A | 11 | 0.0001 | 0.0027 | 0.15 | 0.0007 | 0.026 | 2 |
| KVTEFMLCAGV | Kallikrein2 | 191 | A | 11 | 0.089 | 0.45 | 0.39 | 0.13 | 0.056 | 5 |
| ALGTTCYA | Kallikrein2 | 147 | | 8 | 0.33 | 0.23 | 0.75 | 0.0066 | <0.0001 | 3 |
| MLLRLSEPA | Kallikrein2 | 122 | | 9 | 0.026 | 0.0058 | 0.069 | 0.076 | 0.0003 | 3 |
| ALSVGCTGAV | Kallikrein2 | 9 | | 10 | 0.095 | 0.058 | 0.57 | 0.0068 | 0.0001 | 3 |
| FLRPRSLQCV | Kallikrein2 | 165 | | 10 | 0.027 | 0.89 | 2.4 | 0.0003 | <0.0001 | 3 |
| VLVHPQWVLTA | Kallikrein2 | 53 | | 11 | 0.017 | 0.56 | 0.099 | 0.0018 | 0.0001 | 3 |
| VLVHPQWVLTV | Kallikrein2 | 53 | A | 11 | 0.45 | 2.8 | 0.64 | 0.12 | 0.0009 | 4 |
| SLHLLSNDMCA | Kallikrein2 | 175 | | 11 | 0.070 | 0.90 | 0.14 | <0.0001 | <0.0001 | 3 |
| HLLSNDMCARA | Kallikrein2 | 177 | | 11 | 0.012 | 0.011 | 0.040 | 0.0099 | 0.0001 | 4 |
| PLVCNGVLQGI | Kallikrein2 | 216 | | 11 | 0.0059 | 0.0089 | 0.023 | 0.0023 | 0.0001 | 2 |
| PLVCNGVLQGV | Kallikrein2 | 216 | A | 11 | 0.19 | 0.034 | 0.53 | 0.014 | 0.0019 | 4 |
| PLVBNGVLQGV | PSA | 212 | A | 11 | 0.36 | 0.016 | 0.054 | 0.0094 | 0.0013 | 4 |
| FLRPRSLQBV | Kallikrein2 | 165 | A | 10 | 0.13 | 2.5 | 1.6 | 0.0020 | <0.0001 | 3 |
| SLHLLSNDMBA | Kallikrein2 | 175 | A | 11 | 0.43 | 3.6 | 0.28 | 0.0003 | 0.0004 | 3 |
| LLLARAASL | PAP | 6 | | 9 | 0.024 | 0.32 | 0.35 | 0.0087 | 0.0001 | 4 |
| SLSLGFLFLL | PAP | 13 | | 10 | 0.087 | 0.66 | 0.0035 | 0.073 | 0.0093 | 3 |
| LLARAASLSL | PAP | 7 | | 10 | 0.010 | 0.82 | 0.16 | 0.0004 | 0.0014 | 2 |
| SVLAKELKFV | PAP | 29 | | 10 | 0.0023 | 0.0009 | 0.024 | 0.012 | 0.0002 | 2 |
| LAALFPPEGV | PAP | 120 | | 10 | 0.0022 | 0.023 | 0.23 | 0.0012 | 0.024 | 3 |
| TLMSAMTNLA | PAP | 112 | | 10 | 0.013 | 1.2 | 0.27 | 0.0010 | 0.0012 | 3 |
| GLHGQDLFGI | PAP | 196 | | 10 | 0.20 | 4.7 | 4.0 | 0.012 | <0.0001 | 4 |
| VVFLTLSVTWI | PSA | 1 | | 11 | 0.013 | 0.027 | 0.16 | 0.0013 | 0.0001 | 3 |
| ALGTTBYA | PSA | 143 | A | 8 | 0.10 | 0.34 | 22 | 0.0002 | <0.0001 | 3 |
| ALGTTBYV | PSA | 143 | A | 8 | 0.75 | 1.2 | 3.6 | 0.0012 | <0.0001 | 3 |
| FLTPKKLQBV | PSA | 161 | A | 10 | 0.070 | 1.7 | 2.4 | 0.027 | <0.0001 | 4 |
| KLQBVDLHV | PSA | 166 | A | 9 | 0.16 | 0.12 | 0.15 | 0.0052 | <0.0001 | 3 |
| KLQBVDLHVI | PSA | 166 | A | 10 | 0.023 | 0.16 | 0.51 | 0.014 | <0.0001 | 4 |
| KLQBVDLHVV | PSA | 166 | A | 10 | 0.13 | 0.22 | 2.0 | 0.043 | <0.0001 | 4 |
| KVTKFMLBA | PSA | 187 | A | 9 | 0.0028 | 0.031 | 0.51 | 0.064 | 0.0002 | 3 |
| KVTKFMLBV | PSA | 187 | A | 9 | 0.039 | 0.019 | 2.0 | 0.12 | 0.0012 | 4 |
| HLLSNDMBARA | Kallikrein2 | 177 | A | 11 | 0.0014 | 0.030 | 0.095 | 0.0016 | 0.0001 | 2 |
| LVBNGVLQGI | Kallikrein2 | 217 | A | 10 | 0.0039 | 0.020 | 1.1 | 0.086 | 0.068 | 4 |
| LLLARAASLSL | PAP | 6 | | 11 | 0.19 | 0.018 | 0.013 | 0.0010 | 0.0005 | 2 |
| LLARAASLSL | PAP | 7 | | 10 | 0.0035 | 0.24 | 0.13 | 0.0002 | 0.0010 | 2 |
| SLSLGFLFV | PAP | 13 | A | 9 | 0.065 | 0.17 | 0.47 | 0.040 | 0.0003 | 4 |
| SLSLGFLFLL | PAP | 13 | | 10 | 0.13 | 0.33 | 0.025 | 0.17 | 0.0009 | 4 |
| SLSLGFLFLV | PAP | 13 | A | 10 | 2.7 | 1.1 | 0.59 | 0.088 | 0.023 | 5 |
| VTAKELKFV | PAP | 30 | A | 9 | 0.0008 | 0.0042 | 0.19 | 0.027 | 0.0002 | 2 |
| VVAKELKFV | PAP | 30 | A | 9 | 0.0054 | 0.0090 | 0.46 | 0.031 | 0.0002 | 3 |
| FLNESYKHEQV | PAP | 92 | | 11 | 0.17 | 3.0 | 1.8 | 0.0097 | 0.0013 | 4 |
| TLMSAMTNV | PAP | 112 | A | 9 | 0.52 | 1.8 | 2.8 | 0.068 | 0.13 | 5 |
| LAALFPPEGV | PAP | 120 | | 10 | 0.0025 | 0.023 | 0.60 | 0.0015 | 0.035 | 3 |
| LLALFPPEGV | PAP | 120 | A | 10 | 1.0 | 5.9 | 6.2 | 0.025 | 0.049 | 5 |
| LVALFPPEGV | PAP | 120 | A | 10 | 0.032 | 0.25 | 2.1 | 0.0080 | 0.29 | 5 |
| ALFPPEGVSI | PAP | 122 | | 10 | 0.018 | 0.39 | 0.075 | 0.0014 | 0.0005 | 3 |
| ALFPPEGVSV | PAP | 122 | A | 10 | 0.33 | 4.1 | 0.55 | 0.031 | 0.0018 | 4 |
| GLHGQDLFGV | PAP | 196 | A | 10 | 0.43 | 1.9 | 3.2 | 0.21 | <0.0001 | 4 |
| LLPPYASCHL | PAP | 306 | | 10 | 0.0062 | 0.22 | 0.062 | 0.011 1 | 0.0002 | 3 |
| LLPPYASCHV | PAP | 306 | A | 10 | 0.057 | 0.29 | 0.63 | 0.038 | 0.0015 | 4 |
| SLQCVSLHLL | Kallikrein2 | 170 | | 10 | 0.019 | 0.88 | 0.14 | 0.0083 | 0.0016 | 4 |
| LLWQPIPVHT | PAP | 136 | | 10 | 0.0082 | 0.098 | 0.017 | 0.0003 | 0.0018 | 1 |
| LLWQPIPVHV | PAP | 136 | A | 10 | 0.20 | 2.4 | 0.57 | 0.013 | 0.13 | 5 |
| FLRPRSLQSV | Kallikrein2 | 165 | A | 10 | 0.0036 | 0.12 | 0.38 | 0.0007 | <0.0001 | 2 |
| FLRPRSLQPV. | Kallikrein2 | 165 | A | 10 | 0.0011 | 0.038 | 0.090 | 0.0010 | <0.0001 | 2 |
| KVTEFMLAAGV | Kallikrein2 | 191 | A | 11 | 0.028 | 0.13 | 0.76 | 0.071 | 0.026 | 5 |
| KVTEFMLSAGV | Kallikrein2 | 191 | A | 11 | 0.0028 | 0.010 | 0.18 | 0.0094 | 0.0024 | 3 |
| KVTEFMLPAGV | Kallikrein2 | 191 | A | 11 | 0.0021 | 0.0046 | 0.073 | 0.0096 | 0.0006 | 2 |
| ALGTTAYV | Kallikrein2 | 147 | A | 8 | 0.010 | 0.16 | 0.17 | 0.0002 | 0.0013 | 3 |
| ALGTTSYV | Kallikrein2 | 147 | A | 8 | 0.0029 | 0.027 | 0.10 | 0.0001 | 0.0018 | 2 |
| ALGTTTYV | Kallikrein2 | 147 | A | 8 | 0.0064 | 0.044 | 0.24 | 0.0002 | 0.0008 | 2 |
| ALGTTVYV | Kallikrein2 | 147 | A | 8 | 0.0857 | 0.40 | 0.97 | 0.0003 | 0.0001 | 3 |
| ALSVGATGV | Kallikrein2 | 9 | A | 9 | 0.0077 | 0.055 | 0.39 | 0.0012 | 0.0024 | 2 |
| ALSVGPTGV | Kallikrein2 | 9 | A | 9 | 0.0069 | 0.029 | 0.13 | 0.0001 | 0.0001 | 2 |
| ALSVGSTGV | Kallikrein2 | 9 | A | 9 | 0.0027 | 0.076 | 0.14 | 0.0003 | 0.0014 | 2 |
| FLLAAGLWT | Kallikrein2 | 195 | A | 9 | 0.30 | 0.012 | 0.092 | 0.014 | 0.0002 | 4 |
| FLLPAGLWT | Kallikrein2 | 195 | A | 9 | 2.1 | 0.024 | 0.15 | 0.030 | <0.0001 | 4 |
| FLLSAGLWT | Kallikrein2 | 195 | A | 9 | 7.96 | 0.038 | 0.047 | 0.028 | 0.0002 | 4 |
| FLLTAGLWT | Kallikrein2 | 195 | A | 9 | 0.53 | 0.017 | 0.069 | 0.022 | 0.0003 | 4 |
| ALSVGTTGV | Kallikrein2 | 9 | A | 9 | 0.0043 | 0.11 | 0.19 | 0.0002 | <0.0001 | 2 |
| ALSVGVTGV | Kallikrein2 | 9 | A | 9 | 0.15 | 0.38 | 1.4 | 0.0061 | 0.0001 | 3 |
| FMLAAGLWV | Kallikrein2 | 195 | A | 9 | 2.9 | 0.33 | 4.8 | 0.55 | 0.0002 | 4 |
| FMLPAGLWV | Kallikrein2 | 195 | A | 9 | 0.94 | 0.12 | 1.5 | 0.65 | 0.0003 | 4 |
| FMLSAGLWV | Kallikrein2 | 195 | A | 9 | 1.2 | 0.35 | 2.5 | 0.42 | 0.0004 | 4 |
| FMLTAGLWV | Kallikrein2 | 195 | A | 9 | 0.75 | 0.13 | 0.59 | 0.26 | 0.0003 | 4 |
| FMLVAGLWV | Kallikrein2 | 195 | A | 9 | 0.39 | 0.021 | 0.31 | 0.19 | 0.0002 | 4 |
| KLQAVDLHV | PSA | 166 | A | 9 | 0.012 | 0.015 | 0.022 | 0.0014 | 0.0002 | 3 |
| KVTKFMLAV | PSA | 187 | A | 9 | 0.042 | 0.034 | 1.0 | 0.12 | 0.0009 | 4 |
| KVTKFMLPV | PSA | 187 | A | 9 | 0.17 | 0.096 | 2.6 | 0.39 | 0.0048 | 4 |
| KVTKFMLSV | PSA | 187 | A | 9 | 0.047 | 0.025 | 2.7 | 0.11 | 0.0011 | 4 |
| KVTKFMLTV | PSA | 187 | A | 9 | 0.056 | 0.038 | 1.2 | 0.081 | 0.0006 | 4 |
| KVTKFMLW | PSA | 187 | A | 9 | 0.029 | 0.012 | 0.93 | 0.061 | 0.0004 | 4 |
| FLTPKKLQAV | PSA | 161 | A | 10 | 0.0009 | 0.0093 | 0.030 | 0.0028 | <0.0001 | 2 |
| FLTPKKLQPV | PSA | 161 | A | 10 | 0.0007 | 0.027 | 0.83 | 0.0068 | <0.0001 | 2 |
| FLTPKKLQSV | PSA | 161 | A | 10 | 0.011 | 0.11 | 0.14 | 0.0045 | 0.0001 | 3 |
| FLTPKKLQTV | PSA | 161 | A | 10 | 0.0062 | 0.12 | 0.19 | 0.0031 | <0.0001 | 2 |
| FLTPKKLQVV | PSA | 161 | A | 10 | 0.0071 | 0.11 | 0.21 | 0.0033 | <0.0001 | 2 |
| KLQAVDLHVV | PSA | 166 | A | 10 | 0.044 | 0.51 | 0.70 | 0.020 | 0.0002 | 4 |
| KLQPVDLHVV | PSA | 166 | A | 10 | 0.0054 | 0.11 | 0.36 | 0.030 | <0.0001 | 3 |
| KLQSVDLHVV | PSA | 166 | A | 10 | 0.017 | 0.12 | 0.43 | 0.011 | 0.0001 | 4 |
| KLQTVDLHVV | PSA | 166 | A | 10 | 0.018 | 0.11 | 0.35 | 0.022 | 0.0004 | 4 |
| KLQWDLHVV | PSA | 166 | A | 10 | 0.077 | 0.22 | 2.8 | 0.0377 | 0.0003 | 4 |
| LVANGVLQGV | Kallikrein2 | 217 | A | 10 | 0.040 | 0.19 | 4.3 | 0.15 | 0.66 | 5 |
| LVPNGVLQGV | Kallikrein2 | 217 | A | 10 | 0.25 | 0.35 | 2.8 | 0.62 | 0.41 | 5 |
| LVSNGVLQGV | Kallikrein2 | 217 | A | 10 | 0.045 | 0.40 | 8.6 | 0.13 | 1.3 | 5 |
| LVTNGVLQGV | Kallikrein2 | 217 | A | 10 | 0.028 | 0.12 | 6.6 | 0.30 | 0.89 | 5 |
| LVVNGVLQGV | Kallikrein2 | 217 | A | 10 | 0.12 | 0.087 | 2.9 | 0.096 | 0.46 | 5 |
| SLQAVSLHLV | Kallikrein2 | 170 | A | 10 | 0.019 | 0.39 | 1.6 | 0.021 | 0.0053 | 4 |
| SLQPVSLHLV | Kallikrein2 | 170 | A | 10 | 0.012 | 0.16 | 0.31 | 0.032 | 0.0013 | 4 |
| SLQSVSLHLV | Kallikrein2 | 170 | A | 10 | 0.020 | 0.12 | 0.18 | 0.035 | 0.0022 | 4 |
| SLQTVSLHLV | Kallikrein2 | 170 | A | 10 | 0.023 | 0.19 | 0.69 | 0.037 | 0.0036 | 4 |
| SLQWSLHLV | Kallikrein2 | 170 | A | 10 | 0.049 | 0.15 | 0.93 | 0.046 | 0.0033 | 4 |
| FLRPRSLQW | Kallikrein2 | 165 | A | 10 | 0.014 | 0.33 | 0.40 | 0.015 | 0.0003 | 4 |
| FLRPRSLQTV | Kallikrein2 | 165 | A | 10 | 0.0057 | 0.083 | 0.067 | 0.0039 | 0.0006 | 2 |
| FLRPRSLQAV | Kallikrein2 | 165 | A | 10 | 0.023 | 0.33 | 0.63 | 0.0038 | 0.0002 | 3 |
| ILLSVGPTGAV | Kallikrein2 | 8 | A | 11 | 0.0056 | 0.016 | 0.17 | 0.0004 | 0.0008 | 2 |
| ILLSVGVTGAV | Kallikrein2 | 8 | A | 11 | 0.023 | 0.037 | 0.33 | 0.0091 | 0.0009 | 4 |
| PLVANGVLQGV | Kallikrein2 | 216 | A | 11 | 0.11 | 0.012 | 0.13 | 0.018 | 0.0028 | 4 |
| PLVPNGVLQGV | Kallikrein2 | 216 | A | 11 | 0.19 | 0.050 | 0.55 | 0.11 | 0.0006 | 4 |
| PLVSNGVLQGV | Kallikrein2 | 216 | A | 11 | 0.088 | 0.019 | 0.13 | 0.022 | 0.0019 | 4 |
| PLVTNGVLQGV | Kallikrein2 | 216 | A | 11 | 0.038 | 0.0046 | 0.077 | 0.0090 | 0.0008 | 3 |
| SLHLLSNDMAA | Kallikrein2 | 175 | A | 11 | 0.11 | 1.8 | 0.36 | 0.0033 | 0.0004 | 3 |
| SLHLLSNDMSA | Kallikrein2 | 175 | A | 11 | 0.015 | 1.1 | 0.22 | 0.0003 | 0.0001 | 3 |
| SLHLLSNDMTA | Kallikrein2 | 175 | A | 11 | 0.058 | 2.4 | 0.33 | 0.0002 | 0.0001 | 3 |
| SLHLLSNDMVA | Kallikrein2 | 175 | A | 11 | 0.047 | 0.88 | 0.30 | 0.0006 | 0.0003 | 3 |
| ALGTTCYA | HuK2 | 147 | | 8 | 0.048 | 0.054 | 0.27 | 0.0008 | 0.012 | 3 |
| MLLRLSEPA | HuK2 | 122 | | 9 | 0.053 | 0.018 | 0.013 | 0.0081 | 0.0016 | 3 |
| SLQCVSLHL | HuK2 | 170 | | 9 | 0.01 | 0.084 | 0.024 | 0.0006 | 0.0031 | 3 |
| KVTEFMLCA | HuK2 | 191 | | 9 | 0.0035 | 0.0092 | 0.19 | 0.16 | 0.0004 | 3 |
| LLLARAASV | PAP | 6 | A | 9 | 0.28 | 0.02 | 1.5 | 0.039 | 0.0004 | 4 |
| SLSLGFLFV | PAP | 13 | A | 9 | 0.12 | 0.73 | 2.2 | 0.13 | 0.0017 | 4 |
| FLFLLFFWV | PAP | 18 | A | 9 | 0.12 | 0.014 | 0.016 | 0.012 | 0.0887 | 4 |
| GVSIWNPIV | PAP | 128 | A | 9 | 0.011 | 0.016 | 0.011 | 0.012 | 0.0001 | 3 |
| TVSGLQMAV | PAP | 292 | A | 9 | 0.017 | 0.35 | 0.082 | 0.019 | 1.4 | 5 |
| FLTLSVTWV | PSA | 3 | A | 9 | 0.095 | 0.51 | 1.2 | 0.076 | 0.0006 | 4 |
| TLSVTWIGV | PSA | 5 | A | 9 | 0.19 | 0.88 | 0.25 | 0.0052 | 0.035 | 4 |
| KVTKFMLCV | PSA | 187 | A | 9 | 0.072 | 0.0083 | 0.19 | 0.029 | 0.0005 | 3 |
| ALSVGCTGAV | HuK2 | 9 | | 10 | 0.018 | 0.26 | 0.4 | 0.0051 | 0.0012 | 3 |
| QVAVYSHGWA | HuK2 | 39 | | 10 | 0.0004 | 0.0097 | 0.02 | 0.0005 | 0.0252 | 3 |
| LMLLRLSEPA | HuK2 | 121 | | 10 | 0.025 | 0.26 | 0.15 | 0.004 | 0.0016 | 3 |
| FLRPRSLQCV | HuK2 | 165 | | 10 | 0.041 | 0.094 | 1.1 | 0.0068 | 0.0036 | 3 |
| LVCNGVLQGI | HuK2 | 217 | | 10 | 0.0068 | 0.096 | 0.11 | 0.03 | 0.016 | 4 |
| RAAPLLLARA | PAP | 2 | | 10 | 0.0026 | 0.17 | 0.2 | 0.16 | 0.0007 | 3 |
| LLARAASLSV | PAP | 7 | A | 10 | 0.046 | 0.44 | 0.47 | 0.0098 | 0.011 | 4 |
| SLSLGFLFLV | PAP | 13 | A | 10 | 0.15 | 0.38 | 0.37 | 0.093 | 0.0095 | 4 |
| SLLAKELKFV | PAP | 29 | A | 10 | 0.078 | 0.76 | 2.6 | 0.098 | 0.0012 | 4 |
| VLAKELKFVV | PAP | 30 | A | 10 | 0.16 | 0.36 | 0.053 | 0.043 | 0.0035 | 4 |
| TLMSAMTNLV | PAP | 112 | A | 10 | 0.08 | 1.1 | 0.83 | 0.086 | 0.033 | 5 |
| LLALFPPEGV | PAP | 120 | A | 10 | 0.16 | 11 | 2 | 0.1 | 0.05 | 5 |
| LVALFPPEGV | PAP | 120 | A | 10 | 0.043 | 0.31 | 0.86 | 0.078 | 2.4 | 5 |
| ALFPPEGVSV | PAP | 122 | A | 10 | 0.44 | 8 | 3.4 | 0.31 | 0.058 | 5 |
| GVSIWNPILV | PAP | 128 | A | 10 | 0.02 | 0.046 | 0.43 | 0.0082 | 0.0035 | 4 |
| PLLLWQPIPV | PAP | 134 | A | 10 | 0.021 | 0.092 | 0.52 | 0.011 | 0.0024 | 4 |
| LLWQPIPVHV | PAP | 136 | A | 10 | 0.87 | 2.1 | 0.57 | 0.032 | 0.19 | 5 |
| GLHGQDLFGV | PAP | 196 | A | 10 | 0.27 | 1.2 | 2.6 | 0.63 | 0.0082 | 4 |
| KLRELSELSV | PAP | 234 | A | 10 | 0.019 | 0.47 | 1.4 | 0.075 | 0.0044 | 4 |
| EILNHMKRAT | PAP | 266 | | 10 | 0.0019 | 0.012 | 0.064 | 0.016 | 0.0028 | 3 |
| DTTVSGLQMA | PAP | 290 | | 10 | 0.005 | 0.11 | 0.082 | 0.012 | 0.011 | 3 |
| LLPPYASCHV | PAP | 306 | A | 10 | 0.053 | 0.24 | 0.67 | 0.017 | 0.0027 | 4 |
| LTLSVTWIGA | PSA | 4 | | 10 | 0.0018 | 0.045 | 0.082 | 0.011 | 0.091 | 4 |
| KLQCVDLHVV | PSA | 166 | A | 10 | 0.4 | 0.051 | 1.1 | 0.0074 | 0.0003 | 4 |
| VLVHPQWVLTA | HuK2 | 53 | | 11 | 0.012 | 2.1 | 0.14 | 0.019 | 0.0008 | 4 |
| VLVHPQWVLTV | HuK2 | 53 | A | 11 | 0.68 | 2.9 | 3.8 | 0.34 | 0.0018 | 4 |
| LVHPQWVLTAA | HuK2 | 54 | | 11 | 0.003 | 0.015 | 0.49 | 0.03 | 0.0034 | 3 |
| DLMLLRLSEPV | HuK2 | 120 | A | 11 | 0.1 | 0.075 | 0.35 | 0.025 | 0.0029 | 4 |
| SLHLLSNDMCA | HuK2 | 175 | | 11 | 0.039 | 1.9 | 0.69 | 0.0005 | 0.0004 | 3 |
| HLLSNDMCARA | HuK2 | 177 | | 11 | 0.029 | 0.052 | 0.11 | 0.0088 | 0.0004 | 4 |
| KVTEFMLCAGL | Husk2 | 191 | | 11 | 0.001 | 0.028 | 0.028 | 0.016 | 0.0036 | 3 |
| PLVCNGVLQGV | HuK2 | 216 | A | 11 | 0.22 | 0.017 | 0.36 | 0.03 | 0.0049 | 4 |
| RAAPLLLARAA | PAP | 2 | | 11 | 0.0001 | 0.18 | 0.14 | 0.047 | 0.0037 | 3 |
| LLLARAASLSL | PAP | 6 | | 11 | 0.62 | 1.4 | 1.9 | 0.046 | 0.056 | 5 |
| AASLSLGFLFL | PAP | 11 | | 11 | 0.022 | 0.19 | 0.19 | 0.039 | 0.0006 | 4 |
| FLLFFWLDRSV | PAP | 20 | | 11 | 0.37 | 0.26 | 3.6 | 0.013 | 0.022 | 5 |
| LLFFWLDRSVL | PAP | 21 | | 11 | 0.077 | 1.5 | 3.7 | 0.0045 | 0.0018 | 3 |
| VLAKELKFVTL | PAP | 30 | | 11 | 0.72 | 0.53 | 0.47 | 0.15 | -0.0002 | 4 |
| FLNESYKHEQV | PAP | 92 | | 11 | 0.049 | 1.9 | 1.2 | 0.37 | 0.001 | 4 |
| VVFLTLSVTWV | PSA | 1 | A | 11 | 0.056 | 0.049 | 0.14 | 0.011 | 0.0006 | 4 |
| FLTLSVTWIGV | PSA | 3 | A | 11 | 0.73 | 1.4 | 0.56 | 0.057 | 0.07 | 5 |
| VLVHPQWVLTV | PSA | 49 | A | 11 | 0.1 | 0.6 | 0.44 | 0.019 | 0.0008 | 4 |
| SVFHPEDTGQV | PSA | 75 | A | 11 | 0.0038 | 0.13 | 0.19 | 0.0018 | 0.25 | 3 |
| DLMLLRLSEPV | PSA | 116 | A | 11 | 0.023 | 0.017 | 0.091 | 0.0022 | 0.0016 | 3 |
| HLISNDVCAQV | PSA | 173 | A | 11 | 0.0035 | 0.6 | 0.15 | 0.0028 | 0.026 | 3 |
| PLVCNGVLQGV | PSA | 212 | A | 11 | 0.13 | 0.022 | 0.44 | 0.035 | 0.0075 | 4 |
| RAAPLLLA | PAP | 2 | | 8 | | | | | | |
| PLLLARAA | PAP | 5 | | 8 | | | | | | |
| LLARAASL | PAP | 7 | | 8 | | | | | | |
| SLGFLFLL | PAP | 15 | | 8 | | | | | | |
| WLDRSVLA | PAP | 25 | | 8 | | | | | | |
| LAKELKFV | PAP | 31 | | 8 | | | | | | |
| ELKFVTLV | PAP | 34 | | 8 | | | | | | |
| DTFPTDPI | PAP | 51 | | 8 | | | | | | |
| GMEQHYEL | PAP | 74 | | 8 | | | | | | |
| STDVDRTL | PAP | 106 | | 8 | | | | | | |
| RTLMSAMT | PAP | 111 | | 8 | | | | | | |
| LMSAMTNL | PAP | 113 | | 8 | | | | | | |
| SAMTNLAA | PAP | 115 | | 8 | | | | | | |
| AMTNLAAL | PAP | 116 | | 8 | | | | | | |
| ALFPPEGV | PAP | 122 | | 8 | | | | | | |
| GVSIWNPI | PAP | 128 | | 8 | | | | | | |
| SIWNPILL | PAP | 130 | | 8 | | | | | | |
| PILLWQPI | PAP | 134 | | 8 | | | | | | |
| LLWQPIPV | PAP | 136 | | 8 | | | | | | |
| PLSEDQLL | PAP | 147 | | 8 | | | | | | |
| FIATLGKL | PAP | 187 | | 8 | | | | | | |
| TLGKLSGL | PAP | 190 | | 8 | | | | | | |
| FTLPSWAT | PAP | 221 | | 8 | | | | | | |
| WATEDTMT | PAP | 226 | | 8 | | | | | | |
| TMTKLREL | PAP | 231 | | 8 | | | | | | |
| KLRELSEL | PAP | 234 | | 8 | | | | | | |
| ELSELSLL | PAP | 237 | | 8 | | | | | | |
| SLLSLYGI | PAP | 242 | | 8 | | | | | | |
| RLQGGVLV | PAP | 257 | | 8 | | | | | | |
| GVLVNEIL | PAP | 261 | | 8 | | | | | | |
| ILNHMKRA | PAP | 267 | | 8 | | | | | | |
| HMKRATQI | PAP | 270 | | 8 | | | | | | |
| QIPSYKKL | PAP | 276 | | 8 | | | | | | |
| IMYSAHDT | PAP | 284 | | 8 | | | | | | |
| TTVSGLQM | PAP | 291 | | 8 | | | | | | |
| TVSGLQMA | PAP | 292 | | 8 | | | | | | |
| GLQMALDV | PAP | 295 | | 8 | | | | | | |
| ALDVYNGL | PAP | 299 | | 8 | | | | | | |
| EMYYRNET | PAP | 328 | | 8 | | | | | | |
| PLERFAEL | PAP | 352 | | 8 | | | | | | |
| FAELVGPV | PAP | 356 | | 8 | | | | | | |
| VIPQDWST | PAP | 363 | | 8 | | | | | | |
| TTNSHQGT | PAP | 374 | | 8 | | | | | | |
| LLHETDSA | PSM | 4 | | 8 | | | | | | |
| BTDSAVAT | PSM | 7 | | 8 | | | | | | |
| TARRPRWL | PSM | 14 | | 8 | | | | | | |
| WLCAGALV | PSM | 20 | | 8 | | | | | | |
| CAGALVLA | PSM | 22 | | 8 | | | | | | |
| VLAGGFFL | PSM | 27 | | 8 | | | | | | |
| LAGGFFLL | PSM | 28 | | 8 | | | | | | |
| FIKSSNEA | PSM | 42 | | 8 | | | | | | |
| MTPKHNM | PSM | 51 | | 8 | | | | | | |
| FLYNFTQI | PSM | 73 | | 8 | | | | | | |
| FTQIPHLA | PSM | 77 | | 8 | | | | | | |
| QIPHLAGT | PSM | 79 | | 8 | | | | | | |
| GTEQNFQL | PSM | 85 | | 8 | | | | | | |
| GLDSVELA | PSM | 104 | | 8 | | | | | | |
| ELAHYDVL | PSM | 109 | | 8 | | | | | | |
| LAHYDVLL | PSM | 110 | | 8 | | | | | | |
| LLSYPNKT | PSM | 116 | | 8 | | | | | | |
| DIVPPFSA | PSM | 156 | | 8 | | | | | | |
| SAFSPQGM | PSM | 162 | | 8 | | | | | | |
| GMPEGDLV | PSM | 168 | | 8 | | | | | | |
| DLVYVNYA | PSM | 173 | | 8 | | | | | | |
| RTEDFFKL | PSM | 181 | | 8 | | | | | | |
| KLERDMKI | PSM | 187 | | 8 | | | | | | |
| KINCSGKI | PSM | 193 | | 8 | | | | | | |
| VIARYGKV | PSM | 201 | | 8 | | | | | | |
| KVFRGNKV | PSM | 207 | | 8 | | | | | | |
| KVKNAQLA | PSM | 213 | | 8 | | | | | | |
| QLAGAKGV | PSM | 218 | | 8 | | | | | | |
| LAGAKGVI | PSM | 219 | | 8 | | | | | | |
| VELYSDPA | PSM | 225 | | 8 | | | | | | |
| GVQRGNL | PSM | 252 | | 8 | | | | | | |
| NILNLNGA | PSM | 257 | | 8 | | | | | | |
| PLTPGYPA | PSM | 267 | | 8 | | | | | | |
| YAYRRGIA | PSM | 277 | | 8 | | | | | | |
| GIAEAVGL | PSM | 282 | | 8 | | | | | | |
| EAVGLPSI | PSM | 285 | | 8 | | | | | | |
| SLKVPYNV | PSM | 322 | | 8 | | | | | | |
| HIHSTNEV | PSM | 345 | | 8 | | | | | | |
| STNEVTRI | PSM | 348 | | 8 | | | | | | |
| EVTRIYNV | PSM | 351 | | 8 | | | | | | |
| VTRlYNVI | PSM | 352 | | 8 | | | | | | |
| RIYNVIGT | PSM | 354 | | 8 | | | | | | |
| VIGTLRGA | PSM | 358 | | 8 | | | | | | |
| AVEPDRYV | PSM | 365 | | 8 | | | | | | |
| GIDPQSGA | PSM | 385 | | 8 | | | | | | |
| GAAVVHEI | PSM | 391 | | 8 | | | | | | |
| AAVVHEIV | PSM | 392 | | 8 | | | | | | |
| EIVRSFGT | PSM | 397 | | 8 | | | | | | |
| IVRSFGTL | PSM | 398 | | 8 | | | | | | |
| ILFASWDA | PSM | 416 | | 8 | | | | | | |
| DAEEFGLL | PSM | 422 | | 8 | | | | | | |
| LLGSTEWA | PSM | 428 | | 8 | | | | | | |
| WAEENSRL | PSM | 434 | | 8 | | | | | | |
| RLLQERGV | PSM | 440 | | 8 | | | | | | |
| LLQERGVA | PSM | 441 | | 8 | | | | | | |
| YINADSSI | PSM | 449 | | 8 | | | | | | |
| SIEGNYTL | PSM | 455 | | 8 | | | | | | |
| YTLRVDCT | PSM | 460 | | 8 | | | | | | |
| RVDCTPLM | PSM | 463 | | 8 | | | | | | |
| CTPLMYSL | PSM | 466 | | 8 | | | | | | |
| GMPRISKL | PSM | 508 | | 8 | | | | | | |
| SVYETYEL | PSM | 554 | | 8 | | | | | | |
| PMPKYHLT | PSM | 568 | | 8 | | | | | | |
| VAQVRGGM | PSM | 576 | | 8 | | | | | | |
| ELANSIVL | PSM | 586 | | 8 | | | | | | |
| AVVLRKYA | PSM | 601 | | 8 | | | | | | |
| YADKIYSI | PSM | 607 | | 8 | | | | | | |
| SMKHPQEM | PSM | 615 | | 8 | | | | | | |
| AVKNFTEI | PSM | 635 | | 8 | | | | | | |
| RMMNDQLM | PSM | 662 | | 8 | | | | | | |
| QLMFLERA | PSM | 667 | | 8 | | | | | | |
| BVKRQIYV | PSM | 727 | | 8 | | | | | | |
| QIYVAAFT | PSM | 731 | | 8 | | | | | | |
| VAAFTVQA | PSM | 734 | | 8 | | | | | | |
| AAFTVQAA | PSM | 735 | | 8 | | | | | | |
| TVQAAAET | PSM | 738 | | 8 | | | | | | |
| AAETLSEV | PSM | 742 | | 8 | | | | | | |
| LVHETDSAV | PSM | 4 | A | 9 | | | | | | |
| ETDSAVATA | PSM | 7 | | 9 | | | | | | |
| WLCAGALVV | PSM | 20 | A | 9 | | | | | | |
| LVLAGGFFV | PSM | 26 | A | 9 | | | | | | |
| FIKSSNEAT | PSM | 42 | | 9 | | | | | | |
| ITPKHNMKA | PSM | 52 | | 9 | | | | | | |
| KAFLDELKA | PSM | 59 | | 9 | | | | | | |
| GTEQNFQLA | PSM | 85 | | 9 | | | | | | |
| ELAHYDVLV | PSM | 109 | A | 9 | | | | | | |
| LVYVNYART | PSM | 174 | | 9 | | | | | | |
| GVILYSDPA | PSM | 224 | | 9 | | | | | | |
| ALGLPSIPV | PSM | 286 | A | 9 | | | | | | |
| DAQKLLEKM | PSM | 301 | | 9 | | | | | | |
| KVKMHIHST | PSM | 341 | | 9 | | | | | | |
| HIHSTNEVT | PSM | 345 | | 9 | | | | | | |
| NVIGTLRGA | PSM | 357 | | 9 | | | | | | |
| GIDPQSGAA | PSM | 385 | | 9 | | | | | | |
| TILFASWDA | PSM | 415 | | 9 | | | | | | |
| GLLGSTEWV | PSM | 427 | A | 9 | | | | | | |
| TLRVDCTPV | PSM | 461 | A | 9 | | | | | | |
| IASGRARYT | PSM | 530 | | 9 | | | | | | |
| SLYETYELV | PSM | 554 | A | 9 | | | | | | |
| LVEKFYDPM | PSM | 561 | | 9 | | | | | | |
| PLFKYHLTV | PSM | 568 | A | 9 | | | | | | |
| PVFKYHLTV | PSM | 568 | A | 9 | | | | | | |
| TVAQVRGGM | PSM | 575 | | 9 | | | | | | |
| MVFELANSV | PSM | 583 | A | 9 | | | | | | |
| YAVVLRKYA | PSM | 600 | | 9 | | | | | | |
| AVKNFTEIA | PSM | 635 | | 9 | | | | | | |
| EVKRQIYVA | PSM | 727 | | 9 | | | | | | |
| QLYVAAFTV | PSM | 731 | A | 9 | | | | | | |
| QVYVAAFTV | PSM | 731 | A | 9 | | | | | | |
| YVAAFTVQA | PSM | 733 | | 9 | | | | | | |
| VAAFTVQAA | PSM | 734 | | 9 | | | | | | |
| AAFTVQAAA | PSM | 735 | | 9 | | | | | | |
| FTVQAAAET | PSM | 737 | | 9 | | | | | | |
| AAETLSEVA | PSM | 742 | | 9 | | | | | | |
| NVLHETDSAV | PSM | 3 | A | 10 | | | | | | |
| TARRPRWLCA | PSM | 14 | | 10 | | | | | | |
| WLCAGALVLV | PSM | 20 | A | 10 | | | | | | |
| ALVLAGGFFV | PSM | 25 | A | 10 | | | | | | |
| LVLAGGFFLV | PSM | 26 | A | 10 | | | | | | |
| LLGFLFGWFV | PSM | 34 | A | 10 | | | | | | |
| ATNITPKHNM | PSM | 49 | | 10 | | | | | | |
| NITPKHNMKA | PSM | 51 | | 10 | | | | | | |
| FLDELKAENV | PSM | 61 | A | 10 | | | | | | |
| NIKKFLYNFT | PSM | 69 | | 10 | | | | | | |
| FTQIPHLAGT | PSM | 77 | | 10 | | | | | | |
| DVLLSYPNKT | PSM | 114 | | 10 | | | | | | |
| KVKNAQLAGA | PSM | 213 | | 10 | | | | | | |
| YAYRRGIAEA | PSM | 277 | | 10 | | | | | | |
| PVHPIGYYDA | PSM | 293 | | 10 | | | | | | |
| KLHIHSTNEV | PSM | 343 | A | 10 | | | | | | |
| KVHIHSTNEV | PSM | 343 | A | 10 | | | | | | |
| VTRIYNVIGT | PSM | 352 | | 10 | | | | | | |
| RTILFASWDA | PSM | 414 | | 10 | | | | | | |
| LLQERGVAYV | PSM | 441 | A | 10 | | | | | | |
| KVGSGNDFEV | PSM | 514 | A | 10 | | | | | | |
| EVFFQRLGIA | PSM | 522 | | 10 | | | | | | |
| GIASGRARYT | PSM | 529 | | 10 | | | | | | |
| RARYTKNWET | PSM | 534 | | 10 | | | | | | |
| LTVAQVRGGM | PSM | 574 | | 10 | | | | | | |
| MLFELANSIV | PSM | 583 | A | 10 | | | | | | |
| VLPFDCRDYV | PSM | 592 | A | 10 | | | | | | |
| YADKIYSISM | PSM | 607 | | 10 | | | | | | |
| SISMKHPQEM | PSM | 613 | | 10 | | | | | | |
| SVSFDSLFSA | PSM | 626 | | 10 | | | | | | |
| SLFSAVKNFV | PSM | 631 | A | 10 | | | | | | |
| SAVKNFTEIA | PSM | 634 | | 10 | | | | | | |
| FLERAFIDPV | PSM | 670 | A | 10 | | | | | | |
| YAPSSHNKYA | PSM | 692 | | 10 | | | | | | |
| EVKRQIYVAA | PSM | 727 | | 10 | | | | | | |
| YVAAFTVQAA | PSM | 733 | | 10 | | | | | | |
| VAAFTVQAAA | PSM | 734 | | 10 | | | | | | |
| AAAETLSEVA | PSM | 741 | | 10 | | | | | | |
| DVDRTLMSAMT | PAP | 108 | | 11 | | | | | | |
| RTLMSAMTNLA | PAP | 111 | | 11 | | | | | | |
| TLMSAMTNLAA | PAP | 112 | | 11 | | | | | | |
| LMSAMTNLAAL | PAP | 113 | | 11 | | | | | | |
| MAALFPPEGV | PAP | 119 | | 11 | | | | | | |
| AALFPPEGVSI | PAP | 121 | | 11 | | | | | | |
| ILLWQPIPVHT | PAP | 135 | | 11 | | | | | | |
| LLWQPIPVHTV | PAP | 136 | | 11 | | | | | | |
| HTVPLSEDQLL | PAP | 144 | | 11 | | | | | | |
| TLKSEEFQKRL | PAP | 171 | | 11 | | | | | | |
| RLHPYKDFIAT | PAP | 180 | | 11 | | | | | | |
| FIATLGKLSGL | PAP | 187 | | 11 | | | | | | |
| KVYDPLYCESV | PAP | 208 | | 11 | | | | | | |
| PLYCESVHNFT | PAP | 212 | | 11 | | | | | | |
| SVHNFTLPSWA | PAP | 217 | | 11 | | | | | | |
| FTLPSWATEDT | PAP | 221 | | 11 | | | | | | |
| TLPSWATEDTM | PAP | 222 | | 11 | | | | | | |
| TMTKLRELSEL | PAP | 231 | | 11 | | | | | | |
| KLRELSELSLL | PAP | 234 | | 11 | | | | | | |
| GIHKQKEKSRL | PAP | 248 | | 11 | | | | | | |
| RLQGGVLVNEI | PAP | 257 | | 11 | | | | | | |
| GVLVNEILNHM | PAP | 261 | | 11 | | | | | | |
| ILNHMKRATQI | PAP | 267 | | 11 | | | | | | |
| RATQIPSYKKL | PAP | 273 | | 11 | | | | | | |
| ATQIPSYKKLI | PAP | 274 | | 11 | | | | | | |
| KLIMYSAHDTT | PAP | 282 | | 11 | | | | | | |
| LIMYSAHDTTV | PAP | 283 | | 11 | | | | | | |
| DTTVSGLQMAL | PAP | 290 | | 11 | | | | | | |
| TVSGLQMALDV | PAP | 292 | | 11 | | | | | | |
| QMALDVYNGLL | PAP | 297 | | 11 | | | | | | |
| DVYNGLLPPYA | PAP | 301 | | 11 | | | | | | |
| GLLPPYASCHL | PAP | 305 | | 11 | | | | | | |
| LLPPYASCHLT | PAP | 306 | | 11 | | | | | | |
| ELYFEKGEYFV | PAP | 317 | | 11 | | | | | | |
| ETQHEPYPLML | PAP | 334 | | 11 | | | | | | |
| MLPGCSPSCPL | PAP | 343 | | 11 | | | | | | |
| VIPQDWSTECM | PAP | 363 | | 11 | | | | | | |
| NLLHETDSAVA | PSM | 3 | | 11 | | | | | | |
| LLHETDSAVAT | PSM | 4 | | 11 | | | | | | |
| AVATARRPRWL | PSM | 11 | | 11 | | | | | | |
| ATARRPRWLCA | PSM | 13 | | 11 | | | | | | |
| GALVLAGGFFL | PSM | 24 | | 11 | | | | | | |
| ALVLAGGFFLL | PSM | 25 | | 11 | | | | | | |
| LAGGFFLLGFL | PSM | 28 | | 11 | | | | | | |
| FLLGFLFGWFI | PSM | 33 | | 11 | | | | | | |
| FIKSSNEATNI | PSM | 42 | | 11 | | | | | | |
| EATNITPKHNM | PSM | 48 | | 11 | | | | | | |
| ITPKHNMKAFL | PSM | 52 | | 11 | | | | | | |
| NMKAFLDELKA | PSM | 57 | | 11 | | | | | | |
| ELKAENIKKFL | PSM | 64 | | 11 | | | | | | |
| FLYNFTQIPHL | PSM | 73 | | 11 | | | | | | |
| HLAGTEQNFQL | PSM | 82 | | 11 | | | | | | |
| LAGTEQNFQLA | PSM | 83 | | 11 | | | | | | |
| QIQSQWKEFGL | PSM | 95 | | 11 | | | | | | |
| SVELAHYDVLL | PSM | 107 | | 11 | | | | | | |
| NVSDIVPPFSA | PSM | 153 | | 11 | | | | | | |
| DMKINCSGKIV | PSM | 191 | | 11 | | | | | | |
| KINCSCKIVIA | PSM | 193 | | 11 | | | | | | |
| KVFRGNKVKNA | PSM | 207 | | 11 | | | | | | |
| NAQLAGAKGVI | PSM | 216 | | 11 | | | | | | |
| ILYSDPADYFA | PSM | 226 | | 11 | | | | | | |
| GVKSYPDGWNL | PSM | 238 | | 11 | | | | | | |
| ILNLNGAGDPL | PSM | 258 | | 11 | | | | | | |
| LTPGYPANEYA | PSM | 268 | | 11 | | | | | | |
| PANEYAYRRGI | PSM | 273 | | 11 | | | | | | |
| YAYRRGIAEAV | PSM | 277 | | 11 | | | | | | |
| GIAEAVGLPSI | PSM | 282 | | 11 | | | | | | |
| PIGYYDAQKLL | PSM | 296 | | 11 | | | | | | |
| KVPYNVGPGFT | PSM | 324 | | 11 | | | | | | |
| KMHIHSTNEVT | PSM | 343 | | 11 | | | | | | |
| HIHSTNEVTRI | PSM | 345 | | 11 | | | | | | |
| STNEVTRIYNV | PSM | 348 | | 11 | | | | | | |
| EVTRIYNVIGT | PSM | 351 | | 11 | | | | | | |
| VTRIYNVIGTL | PSM | 352 | | 11 | | | | | | |
| GAVEPDRYVIL | PSM | 364 | | 11 | | | | | | |
| VELGGHRDSWV | PSM | 372 | | 11 | | | | | | |
| GIDPQSGAAW | PSM | 385 | | 11 | | | | | | |
| VVHEIVRSFGT | PSM | 394 | | 11 | | | | | | |
| FASWDAEEFGL | PSM | 418 | | 11 | | | | | | |
| DAEEFGLLGST | PSM | 422 | | 11 | | | | | | |
| STEWAEENSRL | PSM | 431 | | 11 | | | | | | |
| RLLQERGVAYI | PSM | 440 | | 11 | | | | | | |
| GVAYINADSSI | PSM | 446 | | 11 | | | | | | |
| NADSSIEGNYT | PSM | 451 | | 11 | | | | | | |
| YTLRVDCTPLM | PSM | 460 | | 11 | | | | | | |
| RVDCTPLMYSL | PSM | 463 | | 11 | | | | | | |
| PLMYSLVHNLT | PSM | 468 | | 11 | | | | | | |
| HLTVAQVRGGM | PSM | 573 | | 11 | | | | | | |
| LTVAQVRGGMV | PSM | 574 | | 11 | | | | | | |
| QVRGGMVFELA | PSM | 578 | | 11 | | | | | | |
| GMVFELANSIV | PSM | 582 | | 11 | | | | | | |
| MVFELANSIVL | PSM | 583 | | 11 | | | | | | |
| IVLPFDCRDYA | PSM | 591 | | 11 | | | | | | |
| VLPFDCRDYAV | PSM | 592 | | 11 | | | | | | |
| AVVLRKYADKI | PSM | 601 | | 11 | | | | | | |
| SVSFDSLFSAV | PSM | 626 | | 11 | | | | | | |
| RLQDFDKSNPI | PSM | 649 | | 11 | | | | | | |
| PWUWMNDQL | PSM | 658 | | 11 | | | | | | |
| IVUMMNDQLM | PSM | 659 | | 11 | | | | | | |
| GLPDRPFYRHV | PSM | 680 | | 11 | | | | | | |
| DIESKVDPSKA | PSM | 714 | | 11 | | | | | | |
| KVDPSKAWGEV | PSM | 718 | | 11 | | | | | | |
| QIYVAAFTVQA | PSM | 731 | | 11 | | | | | | |
| YVAAFTVQAAA | PSM | 733 | | 11 | | | | | | |
| AAFTVQAAAET | PSM | 735 | | 11 | | | | | | |
| QAAAETLSEVA | PSM | 740 | | 11 | | | | | | |
| PQGFGQLT | PAP | 64 | | 8 | | | | | | |
| GQLTQLGM | PAP | 68 | | 8 | | | | | | |
| EQVYIRST | PAP | 100 | | 8 | | | | | | |
| WQPIPVHT | PAP | 138 | | 8 | | | | | | |
| FQELESET | PAP | 164 | | 8 | | | | | | |
| KQKEKSRL | PAP | 251 | | 8 | | | | | | |
| TQHEPYPL | PAP | 335 | | 8 | | | | | | |
| HQGTEDST | PAP | 378 | | 8 | | | | | | |
| SQPWQVLV | PSA | 31 | | 8 | | | | | | |
| LQCVDLHV | PSA | 167 | | 8 | | | | | | |
| AQVHPQKV | PSA | 181 | | 8 | | | | | | |
| PQKVTKFM | PSA | 185 | | 8 | | | | | | |
| SQWKEFGL | PSM | 98 | | 8 | | | | | | |
| AQKLLEKM | PSM | 302 | | 8 | | | | | | |
| TQKVKMHI | PSM | 339 | | 8 | | | | | | |
| PQSGAAVV | PSM | 388 | | 8 | | | | | | |
| AQVRGGMV | PSM | 577 | | 8 | | | | | | |
| VQAAAETL | PSM | 739 | | 8 | | | | | | |
| WQPIPVHTV | PAP | 138 | | 9 | | | | | | |
| FQELESETL | PAP | 164 | | 9 | | | | | | |
| TQIPSYKKL | PAP | 275 | | 9 | | | | | | |
| TQHEPYPLM | PAP | 335 | | 9 | | | | | | |
| PQDWSTECM | PAP | 365 | | 9 | | | | | | |
| SQPWQVLVA | PSA | 31 | | 9 | | | | | | |
| LQCVDLHVI | PSA | 167 | | 9 | | | | | | |
| AQVHPQKVT | PSA | 181 | | 9 | | | | | | |
| PQKVTKFML | PSA | 185 | | 9 | | | | | | |
| TQIPHLAGT | PSM | 78 | | 9 | | | | | | |
| PQGMPEGDL | PSM | 166 | | 9 | | | | | | |
| AQLAGAKGV | PSM | 217 | | 9 | | | | | | |
| VQRGNILNL | PSM | 253 | | 9 | | | | | | |
| LQERGVAYI | PSM | 442 | | 9 | | | | | | _ |
| PQEMKTYSV | PSM | 619 | | 9 | | | | | | |
| DQLMFLBRA | PSM | 666 | | 9 | | | | | | |
| RQIYVAAFT | PSM | 730 | | 9 | | | | | | |
| KLTDVVKVL | HuK2 | 131 | | 9 | | | | | | |
| KVTDVVKVL | HuK2 | 131 | | 9 | | | | | | |
| RTTDWKVL | HuK2 | 131 | | 9 | | | | | | |
| KITDWKW. | HuK2 | 131 | | 9 | | | | | | |
| PLLGTTCYA | HuK2 | 146 | | 9 | | | | | | |
| PVLGTTCYA | HuK2 | 146 | | 9 | | | | | | |
| PTLGTTCYA | HuK2 | 146 | | 9 | | | | | | |
| PALGTTCYV | HuK2 | 146 | | 9 | | | | | | |
| PQGFGQLTQL | PAP | 64 | | 10 | | | | | | |
| EQHYELGEYI | PAP | 76 | | 10 | | | | | | |
| EQVYIRSTDV | PAP | 100 | | 10 | | | | | | |
| LQGGVLVNEI | PAP | 258 | | 10 | | | | | | |
| TQIPSYKLI | PAP | 275 | | 10 | | | | | | |
| TQHEPYPLML | PAP | 335 | | 10 | | | | | | |
| PQDWSTECMT | PAP | 365 | | 10 | | | | | | |
| EQNFQLAKQI | PSM | 87 | | 10 | | | | | | |
| IQSQWKEFGL | PSM | 96 | | 10 | | | | | | |
| PQGMPEGDLV | PSM | 166 | | 10 | | | | | | |
| AQLAGAKGVI | PSM | 217 | | 10 | | | | | | |
| LQDFDKSNPI | PSM | 650 | | 10 | | . | | | | |
| RQIYVAAFTV | PSM | 730 | | 10 | | | | | | |
| ILLSVGCTGA | HuK2 | 8 | | 10 | | | | | | |
| IVLSVGCTGA | HuK2 | 8 | | 10 | | | | | | |
| TTLSVGGTGA | HuK2 | 8 | | 10 | | | | | | |
| IALSVGCTGV | HuK2 | 8 | | 10 | | | | | | |
| GLVPLIQSRI | HuK2 | 16 | | 10 | | | | | | |
| GVVPLIQSRI | HuK2 | 16 | | 10 | | | | | | |
| GTVPLIQSRI | HuK2 | 16 | | 10 | | | | | | |
| GAVPLIQSRV | HuK2 | 16 | | 10 | | | | | | |
| CLRAYSEKVT | HuK2 | 184 | | 10 | | | | | | |
| CVRAYSEKVT | HuK2 | 184 | | 10 | | | | | | |
| CTRAYSEKVT | HuK2 | 184 | | 10 | | | | | | |
| CARAYSEKVV | HuK2 | 184 | | 10 | | | | | | |
| TQLGMEQHYEL | PAP | 71 | | 11 | | | | | | |
| WQPIPVHTVPL | PAP | 138 | | 11 | | | | | | |
| GQDLFGIWSKV | PAP | 199 | | 11 | | | | | | |
| LQGGVLVNEIL | PAP | 258 | | 11 | | | | | | |
| TQIPSYKKLIM | PAP | 275 | | 11 | | | | | | |
| LQMALDVYNGL | PAP | 296 | | 11 | | | | | | |
| PQDWSTECMTT | PAP | 365 | | 11 | | | | | | |
| WQVLVASRGRA | PSA | 34 | | 11 | | | | | | |
| PQWVLTAAHCI | PSA | 53 | | 11 | | | | | | |
| FQVSHSFPHPL | PSA | 86 | | 11 | | | | | | |
| PQKVTKFMLCA | PSA | 185 | | 11 | | | | | | |
| SQWKEFGLDSV | PSM | 98 | | 11 | | | | | | |
| AQLAGAKGVIL | PSM | 217 | | 11 | | | | | | |
| TQKVKMHIHST | PSM | 339 | | 11 | | | | | | |
| PQSGAAVVHEI | PSM | 388 | | 11 | | | | | | |
| LQERGVAYINA | PSM | 442 | | 11 | | | | | | |
| FQRLGIASGRA | PSM | 525 | | 11 | | | | | | |
| AQVRGGMVFEL | PSM | 577 | | 11 | | | | | | |
| LQDFDKSNPN | PSM | 650 | | 11 | | | | | | |
| DQLMFLERAFI | PSM | 666 | | 11 | | | | | | |
| VQAAAETLSEV | PSM | 739 | | 11 | | | | | | |
| SLALSVGCTGA | HuK2 | 7 | | 11 | | | | | | |
| SVALSVGCTCA | HuK2 | 7 | | 11 | | | | | | |
| STALSVGCTGA | HuK2 | 7 | | 11 | | | | | | |
| SIALSVGCTGV | HuK2 | 7 | | 11 | | | | | | |

**Table 17**

| Peptide | Sequence | Organism | Protein/Segment | 1st Position | Analog | AA | A*0201 | Analog type |
|---|---|---|---|---|---|---|---|---|
| 1413.08 | GLAPPQQLIRV | Human | p53 | 187 | A | 11 | 0.45 | Heteroclitics |
| 1413.10 | GLAPPQSLIRV | Human | p53 | 187 | A | 11 | 1.5 | Heteroclitics |
| 1413.11 | LLGRDSFEV | Human | mp53 | 261 | A | 9 | 1.6 | |
| 1413.12 | LLSRDSFEV | Human | mp53 | 261 | A | 9 | 0.17 | Heteroclitics |
| 1413.13 | LLDRDSFEV | Human | mp53 | 261 | A | 9 | 1.1 | Heteroclitics |
| 1413.14 | LLHRDSFEV | Human | mp53 | 261 | A | 9 | 1.5 | Heteroclitics |
| 1413.15 | LLGRNSFEV | Human | p53 | 264 | A | 9 | 0.035 | |
| 1413.16 | LLGRQSFEV | Human | mp53 | 264 | A | 9 | 0.066 | Heteroclitics |
| 1413.17 | LLGRGSFEV | Human | mp53 | 264 | A | 9 | 0.026 | Heteroclitics |
| 1413.18 | LLGRDSLEV | Human | mp53 | 264 | A | 9 | 0.025 | Heteroclitics |
| 1413.19 | LLGRDSMEV | Human | mp53 | 264 | A | 9 | 0.012 | Heteroclitics |
| 1413.20 | LLGRDSHEV | Human | mp53 | 264 | A | 9 | 0.60 | Heteroclitics |
| 54.0078 | YLQLVFGIEV | Human | MAGE2 | 157 | A | 10 | 0.018 | |
| 54.0084 | YLQLIFGIEV | Human | MAGE2 | 157 | A | 10 | 0.013 | Heteroclitics |
| 55.0234 | YLQLFFGIEV | Human | MAGE2 | 157 | A | 10 | 0.046 | Heteroclitics |
| 54.0003 | SMPPPGTRV | Human | p53 | 149 | A | 9 | 0.079 | Heteroclitics |
| 54.0005 | CMPPPGTRV | Human | p53 | 149 | A | 9 | 0.182 | Heteroclitics |
| 55.0049 | SMPPPGPRV | Human | p53 | 149 | A | 9 | 0.011 | Heteroclitics |
| 55.0155 | ILLEPVHGV | HIV | POL | 476 | A | 9 | 0.0075 | Heteroclitics |
| 54.0063 | ILHEPVHGV | HIV | POL | 476 | A | 9 | 0.019 | Heteroclitics |
| 55.0123 | GLSRYVPRL | HIV | POL | 455 | A | 9 | 0.098 | Heteroclitics |
| 54.0042 | GLSRYVARL | HIV | POL | 455 | A | 9 | 0.057 | Heteroclitics |
| 55.0153 | ILVEPVHGV | HIV | POL | 476 | A | 9 | 0.0060 | Heteroclitics |
| 55.0158 | ILKDPVHGV | HIV | POL | 476 | A | 9 | 0.026 | Heteroclitics |
| 55.0159 | ILKQPVHGV | HIV | POL | 476 | A | 9 | 0.0063 | Heteroclitics |
| 55.0160 | ILKNPVHGV | HIV | POL | 476 | A | 9 | 0.0055 | Heteroclitics |
| 55.0161 | ILKHPVHGV | HIV | POL | 476 | A | 9 | 0.0005 | Heteroclitics |
| 55.0162 | ILKTPVHGV | HIV | POL | 476 | A | 9 | 0.0011 | Heteroclitics |
| 55.0163 | ILKRPVHGV | HIV | POL | 476 | A | 9 | 0.015 | Heteroclitics |
| 55.0164 | ILKVPVHGV | HIV | POL | 476 | A | 9 | 0.0048 | Heteroclitics |
| 55.0165 | ILKLPVHGV | HIV | POL | 476 | A | 9 | 0.010 | Heteroclitics |
| 55.0166 | ILKYPVHGV | HIV | POL | 476 | A | 9 | 0.032 | Heteroclitics |
| 55.0167 | ILKFPVHGV | HIV | POL | 476 | A | 9 | 0.077 | Heteroclitics |
| 55.0168 | ILKETVHGV | HIV | POL | 476 | A | 9 | 0.011 | Heteroclitics |
| 55.0169 | ILKESVHGV | HIV | POL | 476 | A | 9 | 0.19 | Heteroclitics |
| 55.0170 | ILKEFVHGV | HIV | POL | 476 | A | 9 | 0.024 | Heteroclitics |

**Table 18**

| | | | HLA- A3 supertype binding affinity (IC50 nM) | | | | |
|---|---|---|---|---|---|---|---|
| Peptide | Sequence | Source | A*0301 | A*1101 | A*3101 | A*3301 | A*6801 |
| 78.0064 | AACHKCIDFY | HPV45.E6.63 | 7769 | 356 | 4830 | 10647 | 4958 |
| 78.0048 | AACHKCIDFY | HPV18.E6.63 | 18824 | 306 | 20643 | >97403.22 | 31622 |
| 78.0316 | AACWRSRRR | HPV33.E6.137 | 13039 | 9433 | 407 | 913 | 983 |
| 78.00558 | AFADLTVVYR | BPV33.E6.46 | 24059 | 5093 | 140 | 249 | 39 |
| 78.0063 | AFKDLCIVYR | HPV45.E6.48 | 13166 | 3661 | 26 | 29 | 548 |
| 78.0093 | AFKDLFVVYR | HPV18.E6.48 | 46161 | 10358 | 8.3 | 14 | 365 |
| 78.0079 | AFRDLCIVYR | HPV16.E6.53 | 3106 | 4377 | 13 | 41 | 600 |
| 78.0052 | AFTDLTIVYR | HPV31.E6.46 | 26603 | 300 | 810 | 817 | 210 |
| 78.0281 | AQPATADYY | HPV33.E7.45 | 43 | 15 | 10.3 | 32 | 23 |
| 78.0294 | ATLESITKK | HPV56.E6.89 | 302 | 160 | 261 | >81119.6 | >32130.33 |
| 78.0272 | ATLQDIVLH | HPV18.E7.6 | 76 | 251 | >141552.48 | >107282.02 | 3756 |
| 78.0023 | ATSNYYTVTY | HPV52.E7.50 | 2437 | 167 | 27613 | 154330 | 24292 |
| 78.0043 | AVCDKCLKFY | HPV16.E6.68 | 561 | 88 | 1959 | 47868 | 3323 |
| 78.0068 | AVCRVCLLFY | HPV56.E6.64 | 77 | 21 | 1978 | 4520 | 1302 |
| 78.0298 | AVCWRPRRR | HPV58.E6.137 | 3.9 | 13 | 395 | 799 | 23 |
| 78.0284 | CIAYAACHK | HPV45.E6.59 | 831 | 226 | 8103 | 17514 | 1011 |
| 78.0151 | CLLFYSKVRK | HPV56.E6.69 | 342 | 249 | 3343 | 9357 | 405 |
| 78.0061 | CVYCKATLER | HPV45.E6.32 | 24489 | 28088 | 334 | 963 | 4485 |
| 78.0095 | DSIPHAACHK | HPV18.E6.58 | 25343 | 1874 | 1886 | 14 | 69 |
| 78.0049 | DSVYGDTLEK | HPV18.E6.83 | 4717 | 415 | 51213 | 116894 | 408 |
| 78.0297 | DTLEQTLKK | HPV58.E6.86 | 446 | 139 | 714 | 5826 | 2488 |
| 78.0313 | EGNPFGICK | HPV33.E6.56 | 40696 | 2662 | 602 | 585 | 28 |
| 78.0322 | ELQRREVYK | HPV52.E6.36 | 5603 | 18788 | 407 | 17506 | 25869 |
| 78.0072 | ETSVHEIELK | HPV58.E6.20 | 31644 | 5335 | 31702 | 98886 | 41 |
| 78.0335 | FADLRIVYR | HPV58.E6.47 | 5447 | 20911 | 86 | 24176 | >30740.14 |
| 78.0115 | FAFADLTVVY | HPV33.E6.45 | 18592 | 5866 | 23676 | 26768 | 402 |
| 78.0103 | FAFTDLTIVY | HPV31.E6.45 | 40343 | 21161 | 42065 | 131202 | 346 |
| 78.0111 | FCCQCKSTLR | HPV31.E7.57 | 8975 | 8510 | 2056 | 96 | 15816 |
| 78.0293 | GATLESITK | HPV56.E6.88 | 187 | 300 | 861 | 1884 | 6558 |
| 78.0069 | GATLESITKK | HPV56.E6.88 | 1860 | 283 | 8619 | 8728 | 2487 |
| 78.0046 | GIVCPICSQK | HPV16.E7.88 | 2758 | 117 | 20749 | 93473 | 2714 |
| 78.0055 | GTTLEKLTNK | HPV31.E6.85 | 2049 | 176 | 3823 | 42078 | 16401 |
| 78.0044 | GTTLEQQYNK | HPV16.E6.92 | 10850 | 69 | 34193 | 101404 | 3935 |
| 78.0106 | GVCTKCLRFY | HPV31.E6.61 | 1482 | 244 | 7528 | 8360 | 24818 |
| 78.0326 | GVDRPDGQA | HPV52.E7.39 | 214 | 1290 | 3356 | 8720 | >30740.14 |
| 78.0273 | GVNHQHLPA | HPV18.E7.43 | 816 | 26 | 862 | 1353 | 203 |
| 78.0100 | GVNHQHLPAR | HPV18.E7.43 | 3181 | 178 | 62 | 13039 | 2897 |
| 78.0134 | GVSHAQLPAR | HPV45.E7.44 | 3689 | 221 | 2788 | 46952 | 16649 |
| 78.0274 | HTMLCMCCK | HPV18.E7.59 | 636 | 274 | 6491 | 129386 | 3658 |
| 78.0265 | IILECVYCK | HPV16.E6.33 | 1644 | 235 | 5656 | 8957 | 1594 |
| 78.0165 | ILIRCIICQR | HPV58.E6.99 | 4366 | 490 | 379 | 4273 | 272 |
| 78.0117 | ILIRCIICQR | HPV33.E6.99 | 8550 | 5012 | 377 | 2480 | 537 |
| 78.0311 | IVCPNCSTR | HPV31.E7.89 | 18461 | >25210.31 | 350 | 64095 | 28178 |
| 78.0278 | IVTFCCQCK | HPV31.E754 | 4511 | 152 | 15374 | >107282.02 | >16962.83 |
| 78.0283 | IVYRDCIAY | HPV45.E6.54 | 262 | 42 | 598 | 469 | 2555 |
| 78.0286 | IVYRDNNPY | HPV52.E6.52 | 9024 | 225 | 90928 | 8249 | 295 |
| 78.0081 | KFYSKISEYR | HPV16.E6.75 | 1684 | 18047 | 48 | 79 | 264 |
| 78.0082 | KISEYRHYCY | HPV16.E6.79 | 152 | 163 | 4847 | 2348 | 2004 |
| 78.0074 | KISEYRHYNY | HPV58.E6.72 | 197 | 136 | 1759 | 40765 | 10323 |
| 78.0059 | KISEYRHYNY | HPV33.E6.72 | 42 | 112 | 1426 | 35341 | 25077 |
| 78.0331 | KQHTCYLIH | HPV56.E7.54 | 39392 | >18660.01 | 526 | 521 | 439 |
| 78.0329 | KQLCDLLIR | HPV56.E6.97 | 3513 | 9972 | 39 | 12 | 58 |
| 78.0156 | KQLHDRKRR | HPV56.E6.118 | 31894 | >4691.24 | 289 | 71431 | >13447.56 |
| 78.0083 | KQRFHNIRGR | HPV16.E6.129 | 2037 | 11596 | 178 | 4441 | 10279 |
| 78.0288 | KTLEERVKK | HPV52.E6.86 | 2694 | 296 | 13241 | 54088 | 2353 |
| 78.0296 | KVCLRLLSK | HPV58.E6.64 | 9470 | 19 | >69585.78 | 113285 | .4651 |
| 78.0275 | KVSEFRWYR | HPV31.E6.72 | 401 | 178 | 65947 | >107282.02 | 43168 |
| 78.0053 | KVSEFRWYRY | HPV31.E6.72 | 248 | 23 | 11 | 7073 | 1908 |
| 78.0155 | LCDLLIRCYR | HPV56.E6.99 | 3984 | 273 | 856 | 405 | 2126 |
| 78.0137 | LFTDLRIVYR | HPV52.E6.46 | 57146 | >4045.92 | 430 | 217 | 1890 |
| 78.0152 | LFYSKVRKYR | HPV56.E6.71 | 6622 | 6011 | 8.1 | 48 | 1892 |
| 78.0337 | LIRCIICQR | HPV58.E6.100 | 198 | 992 | 523 | 117540 | >28524.17 |
| 78.0301 | LIRCINCQK | HPV16.E6.107 | 2182 | 216 | 6859 | >74670.44 | >34318.65 |
| 78.0317 | LIRCLRCQK | HPV45.E6.102 | 5501 | 1647 | 353 | 5723 | 1321 |
| 78.0292 | LLFYSKVRK | HPV56.E6.70 | 106 | 18 | 43151 | 70793 | 33 |
| 78.0045 | LLIRCINCQK | HPV16.E6.106 | 296 | 71 | 556 | 5375 | 32 |
| 78.0108 | LLIRCITCQR | HPV31.E6.99 | 3319 | 5272 | 168 | 739 | 30 |
| 78.0066 | LLIRCLRCQK | HPV45.E6.101 | 270 | 226 | 2496 | 11367 | 44 |
| 78.0051 | LLIRCLRCQK | HPV18.E6.101 | 437 | 211 | 6612 | 28936 | 78 |
| 78.0145 | LQVVCPGCAR | HPV52.E7.89 | >55458.03 | >14665.98 | 490 | 6225 | 19498 |
| 78.0306 | LSFVCPWCA | HPV18.E7.94 | 38337 | 10864 | 4289 | 4603 | 341 |
| 78.0047 | LTEVFEFAFK | HPV18.E6.41 | 8672 | 113 | 37879 | 47752 | 27 |
| 78.0086 | MSCCRSSRTR | HPV16.E6.144 | 571 | 829 | 324 | 1142 | 26 |
| 78.0324 | NIMGRWTGR | HPV52.E6.127 | 38678 | 1940 | 41 | 448 | 26230 |
| 78.0057 | NIVTFCCQCK | HPV31.E7.53 | 3072 | 1957 | 6005 | 10314 | 199 |
| 78.0065 | NSVYGETLEK | HPV45.E6.83 | 633 | 127 | 5749 | 8928 | 289 |
| 78.0280 | NTLEQTVKK | HPV33.E6.86 | 529 | 142 | 743 | 3428 | 224 |
| 78.0330 | QQARQAKQH | HPV56.E7.48 | 7172 | 2853 | 48 | 36 | 30 |
| 78.0327 | QVVCPGCAR | HPV52.E7.90 | 3819 | 16820 | 5080 | 92 | 5341 |
| 78.0119 | RCAACWRSRR | HPV33.E6.135 | 17390 | 28729 | 486 | 2394 | 6645 |
| 78.0338 | RCAVCWRPR | HPV58.E6.135 | 54398 | 21282 | 2829 | 438 | 1440 |
| 78.0109 | RCIACWRRPR | HPV31.E6.135 | 1231 | 6604 | 64 | 438 | 5855 |
| 78.0097 | RFHNIAGHYR | HPV18.E6.126 | 2463 | 2855 | 11 | 99 | 151 |
| 78.0133 | RFHSIAGQYR | HPV45.E6.126 | 4278 | 3390 | 24 | 585 | 303 |
| 78.0140 | RFLSKISEYR | HPV52.E6.68 | 4860 | >14665.98 | 27 | 749 | 36 |
| 78.0116 | RFLSKISEYR | HPV33.E6.68 | 1640 | 18468 | 33 | 436 | 172 |
| 78.0107 | RFYSKVSEFR | HPV31.E6.68 | 1382 | 17885 | 54 | 204 | 250 |
| 78.0098 | RGQCHSCCNR | HPV18.E6.135 | 22754 | 1938 | 192 | 318 | 4791 |
| 78.0163 | RLLSKISEYR | HPV58.E6.68 | 66 | 2507 | 42 | 1574 | 240 |
| 78.0320 | RLQCVQCKK | HPV52.E6.27 | 2200 | 1289 | 386 | 336 | 993 |
| 78.0062 | RTEVYQFAFK | HPV45.E6.41 | 285 | 111 | 1691 | 9180 | 3310 |
| 78.0291 | RVCLLFYSK | HPV56.E6.67 | 347 | 166 | >77795.34 | >81119.6 | 2622 |
| 78.0302 | SCCRSSRTR | HPV16.E6.145 | 288 | 230 | 525 | 106485 | 45541 |
| 78.0087 | SCCRSSRTRR | HPV16.E6.145 | 16439 | 21422 | 401 | 223 | 3608 |
| 78.0270 | SIPHAACHK | HPV18.E6.59 | 736 | 268 | 63 | 7253 | 403 |
| 78.0142 | SLYGKTLEER | HPV52.E6.82 | 56 | 1728 | 435 | 5499 | 178 |
| 78.0271 | SVYGDTLEK | HPV18.E6.84 | 2575 | 273 | 32513 | 22456 | >17538 |
| 78.0285 | SVYGETLEK | HPV45.E6.84 | 169 | 490 | 795 | 60127 | 33480 |
| 78.0276 | SVYGTTLEK | HPV31.E6.82 | 450 | 109 | 1595 | 1969 | 439 |
| 78.0076 | TAMFQDPQER | HPV16.E6.6 | 40463 | 2877 | 11713 | 8520 | 16 |
| 78.0303 | TGLYNLLIR | HPV18.E6.96 | 2737 | 2577 | 197 | 3780 | 2592 |
| 78.0333 | TSVHEIELK | HPV58.E6.21 | 51582 | 3692 | 665 | 1305 | 312 |
| 78.0277 | TTLEKLTNK | HPV31.E6.86 | 72 | 14 | 41179 | 79901 | 17 |
| 78.0135 | TVESSAEDLR | HPV45.E7.76 | >58998.41 | 9973 | 89136 | 55083 | 205 |
| 78.0073 | VFADLRIVYR | HPV58.E6.46 | 23042 | 2089 | 31 | 52 | 197 |
| 78.0321 | VQCKKELQR | HPV52.E6.31 | 54286 | 29279 | 366 | 6049 | 21290 |
| 78.0295 | VQLDIQSTK | HPV56.E7.72 | 153 | 378 | 1066 | 40091 | 7535 |
| 78.0318 | VSHAQLPAR | HPV45.E7.45 | 19181 | 9024 | 1784 | 310 | 39 |
| 78.0282 | VSIACVYCK | HPV45.E6.28 | 19 | 3.8 | 8875 | 33911 | 15 |
| 78.0071 | WQLDIQSTK | HPV56.E7.71 | 1862 | 143 | 35232 | 83649 | 3704 |
| 78.0118 | WAGRCAACWR | FIPV33.E6.132 | 10060 | 10975 | 445 | 49 | 50 |
| 78.0075 | WTGRCAVCWR | HPV58.E6.132 | 4397 | 4806 | 358 | 107 | 59 |
| 78.0056 | WTGRCIACWR | HPV31.E6.132 | 2260 | 1035 | 179 | 17 | 31 |
| 78.0084 | WTGRCMSCCR | HPV16.E6.139 | 11458 | 7557 | 3126 | 471 | 178 |
| 78.0067 | WTGRCSECWR | HPV52.E6.132 | 20400 | 1523 | 538 | 69 | 40 |
| 78.0070 | WTGSCLGCWR | HPV56.E6.135 | 25112 | 1624 | 208 | 60 | 23 |
| 78.0300 | YAVCDKCLK | HPV16.E6.67 | 56 | 18 | 3195 | 87230 | 1948 |
| 78.0334 | YDFVFADLR | HPV58.E6.43 | 61113 | 24098 | 64 | 153 | 1332 |
| 78.0054 | YSVYGTTLEK | HPV31.E6.81 | 357 | 100 | >100076.57 | >36296.57 | 36 |

**TABLE 19**

| | | | | | HLA- A3 supertype binding affinity (IC50 nM) | | | | |
|---|---|---|---|---|---|---|---|---|---|
| Peptide | Sequence | AA | Source | Analog | A*0301 | A*1101 | A*3101 | A*3301 | A*6801 |
| 21.0073 | GIIHFSFPF | 9 | Candida.aur.168 | | 18333.3 | 293.5 | | | |
| 21.0053 | LTSQFFLPA | 9 | Candida.aur.98 | | 6821.9 | 152.2 | | | |
| 21.0201 | STMYLTHHYF | 10 | Candida.aur.301 | | >31754.26 | 36.1 | | | |
| 1371.13 | FVSNLATGK | 9 | CEA.656 | A | 3315.0 | 280.2 | >17162.33 | >48333.33 | 7.5 |
| 1371.12 | HVQVLFIAR | 9 | CEA.636 | A | 10357.1 | 11131.7 | 1627.8 | 319.7 | 479.5 |
| 1371.08 | IVPSYTYYK | 9 | CEA.420 | A | 17.9 | 63.3 | 1550.8 | 4505.5 | 21.5 |
| 1371.07 | IVPSYTYYR | 9 | CEA.420 | A | 91.4 | 14.4 | 49.6 | 118.7 | 1.9 |
| 1371.10 | RVLTLFNVTK | 10 | CEA.554 | A | 20.9 | 31.2 | 234.1 | 42522.0 | 1738.1 |
| 1371.09 | RVLTLFNVTR | 10 | CEA.554 | A | 298.3 | 75.1 | 17.7 | 7745.4 | 42.9 |
| 1371.04 | RVLTLLSVTK | 10 | CEA.376 | A | 38.6 | 50.1 | 165.5 | >48333.33 | 4466.8 |
| 1371.03 | RVLTLLSVTR | 10 | CEA.376 | A | 340.9 | 248.2 | 12.4 | 6127.4 | 519.5 |
| 1371.02 | TVSPLNTSYK | 10 | CEA.241 | A | 17.4 | 11.1 | 13258.6 | 30398.3 | 12.1 |
| 1371.01 | TVSPLNTSYR | 10 | CEA.241 | A | 465.7 | 62.4 | 194.3 | 646.5 | 12.4 |
| 1371.06 | TVSPSYTYYK | 10 | CEA.419 | A | 53.5 | 33.6 | 4172.3 | 30317.1 | 9.8 |
| 1371.05 | TVSPSYTYYR | 10 | CEA.419 | A | 2495.9 | 3103.6 | 30.9 | 270.0 | 9.0 |
| 1120.17 | TYQRTRALK | 9 | Flu.NP.147 | A | | | 32.0 | 66.5 | 16000.0 |
| 1.1076 | GILYKRETTR | 10 | HBV.pol.729 | | >17614.1 | >4615.38 | 2157.6 | 439.4 | 80000.0 |
| 1.0973 | HLQEDIINR | 9 | HBV.pol.657 | | >4032.8 | >4670.99 | 65.0 | 15.7 | 1600.0 |
| 1150.34 | LAACFARDR | 9 | HBV.pol.731 | | >27500 | 30000.0 | 4352.9 | 1435.7 | 40.0 |
| 1150.32 | LAIGHQRMR | 9 | HBV.pol.708 | | >27500 | 30000.0 | 2964.8 | 2165.8 | 66.7 |
| 1150.33 | LAIGQSGMR | 9 | HBV.pol.708 | | >27500 | 15000.0 | 16431.7 | 7259.1 | 47.1 |
| 28.0747 | UMPARFYPK | 10 | HBV.pol.109 | | 2.4 | 6.1 | | | |
| 28.0397 | LVMGHQRMR | 9 | HBV.pol.697 | | 366.7 | 498.1 | | | |
| 28.0396 | LVNHYFQTR | 9 | HBV.pol.137 | | 2053.4 | 363.7 | | | |
| 1.0966 | NVNMGLKIR | 9 | HBV.core.90 | | >4032.8 | >4670.99 | 3728.2 | 366.9 | 4705.9 |
| 28.0398 | PINRPIDWK | 9 | HBV.pol.612 | | 102.2 | | | | |
| 1.0984 | VLKLKQCFR | 9 | HBV.pol.1186 | | 15877.1 | >5720.78 | 37.6 | 668.8 | |
| 28.0835 | VSYVNTNMGLK | 11 | HBV.core.115 | | 54.0 | 45.7 | | | |
| 1150.28 | WARVHSTTR | 9 | BBV.pol.244 | | >27500 | >30000 | 10062.3 | 248.9 | 1126.8 |
| 1.0986 | YLHLYPVAR | 9 | HBV.pol.1255 | | 2948.3 | 8571.4 | 492.8 | 471.6 | |
| 1371.62 | AVPLDSTFYK | 10 | Her2/neu.997 | A | 630.7 | 33.5 | | 21481.5 | 179.0 |
| 1371.61 | AVPLDSTFYR | 10 | Her2/neu.997 | A | >38890.87 | 92.0 | 29950.1 | 2940.4 | 54.4 |
| 1371.47 | BVNBSQFLK | 9 | Her2/neu.528 | A | 35.6 | 22.1 | 70.0 | 4438.9 | 17.2 |
| 1371.46 | BVNBSQFLR | 9 | Her2/neu.528 | A | 196.3 | 80.0 | 37.7 | 57.7 | 9.8 |
| 1371.45 | HVVPWDQLFK | 10 | Her2/neu.478 | A | 184.2 | 410.1 | >46475.8 | 38359.8 | 9.5 |
| 1371.44 | HVVPWDQLFR | 10 | Her2/neu.478 | A | 7261.7 | 1434.1 | 397.5 | 205.2 | 3.9 |
| 1371.35 | IVKGGVLIQK | 10 | Her2/neu.148 | A | 26.5 | 74.7 | 470.3 | 18403.5 | 21.1 |
| 1371.34 | IVKGGVLIQR | 10 | Her2/neu.148 | A | 278.9 | 7349.1 | 70.7 | 125.1 | 32.5 |
| 1371.38 | IVWKDIFHK | 9 | Her2/neu.167 | A | 23.9 | 45.8 | 265.6 | 970.1 | 165.8 |
| 1371.39 | IVWKDIFHR | 9 | Her2/neu.167 | A | 148.4 | 282.8 | 6.1 | 18.6 | 17.0 |
| 1371.43 | IVWLGLRSLK | 10 | Her2/neu.450 | A | 4.0 | 128 | 293 | 2283 | 12 |
| 1371.42 | IVWLGLRSLR | 10 | Her2/neu.450 | A | 235.3 | 2195.7 | 11.6 | 193.6 | 7.5 |
| 1371.57 | KVTDFGLAK | 9 | Her2/neu.860 | A | 27.5 | 46.2 | 3495.9 | >17985.04 | 1193.2 |
| 1371.56 | KVTDFGLAR | 9 | Her2/neu.860 | A | 202.9 | 82.7 | 114.3 | >17985.04 | 138.0 |
| 1371.53 | LVARNVLVK | 9 | Her2/neu.846 | A | 44.3 | 217.4 | 8991.0 | >17985.04 | 169.4 |
| 1371.54 | LVARNVLVR | 9 | Her2/neu.846 | A | 281.2 | 3117.2 | 9144.6 | 20573.1 | 22.0 |
| 1371.52 | LVDHVRENK | 9 | Her2/neu.806 | A | 299.4 | 768.2 | >46475.8 | >17985.04 | 3301.1 |
| 1371.55 | LVKSPNHVR | 9 | Her2/neu.852 | A | 7898.3 | 12903.2 | 201.6 | 107.8 | 74.9 |
| 1371.60 | LVSEFSRMAK | 10 | Her2/neu.972 | A | 285.6 | 78.2 | 1710.9 | 5318.2 | 51.8 |
| 1371.59 | MVLESILRK | 9 | Her2/neu.889 | A | 62.6 | 16.4 | 20930.2 | 2848.9 | 290.1 |
| 1371.58 | MVLESILRR | 9 | Her2/neu.889 | A | 235.7 | 272.5 | 208.1 | 121.5 | 20.5 |
| 1371.41 | TVBAGGBAK | 9 | Her2/neu.218 | A | 24.2 | 29.2 | 41958.0 | 34523.8 | 7.8 |
| 1371.40 | TVBAGGBAR | 9 | Her2/neu.218 | A | 350.2 | 114.3 | 248.3 | 201.4 | 7.9 |
| 1371.36 | TVLWKDIFHK | 10 | Her2/neu.166 | A | 813.7 | 41.0 | 9970.4 | 5896.2 | 476.3 |
| 1371.37 | TVLWKDIFHR | 10 | Her2/neu.166 | A | 11439.2 | 298.9 | 593.0 | 83.8 | 44.7 |
| 1371.48 | VVFGILIKK | 9 | Herneu.669 | A | 32.9 | 19.9 | 3776.2 | 10066.0 | 28.2 |
| 1371.49 | VVRENTSPK | 9 | Her2/neu.754 | A | 98.1 | 335.9 | 753.8 | 1341.2 | 5698.0 |
| 1371.50 | VVRENTSPR | 9 | Her2/neu.754 | A | 335.2 | 5618.7 | 374.5 | 127.2 | 203.3 |
| 940.08 | AC-QVPLRPMTY) | 10 | HIV.nef.73 | A | 338.5 | 731.9 | | | |
| 940.11 | AQVPLRPMTYK | 11 | HIV.nef.73 | A | 56.6 | 82.6 | | | |
| 940.10 | RQVPLRPMTYK | 11 | HIV.nef.73 | A | 10.1 | 69.9 | | | |
| 31.0116 | KTWMDIEGR | 9 | Lassa.NP.385 | | 8500.8 | 1131.8 | 125.7 | 1902.2 | 1818.2 |
| 31.0117 | MLQKEYMER | 9 | Lassa.gp.414 | | >3990.12 | 1371.2 | 99.9 | 195.5 | 33.3 |
| 1150.47 | EMLRKDYIK | 9 | LCMV.gp.419 | | >11595.02 | 20000.0 | 1477.1 | 64.5 | >40000 |
| 1371.72 | AVIETSYVK | 9 | MAGE2.277 | A | 410.2 | 72.8 | 14064.6 | >40215.76 | 30.8 |
| 1371.73 | AVIETSYVR | 9 | MAGE2.277 | A | 31700.3 | 172.8 | 129.1 | 1177.5 | 18.8 |
| 1371.75 | IVYPPLHEK | 9 | MAGE2.299 | A | 44.6 | 108.7 | 883.6 | 3025.6 | 47.5 |
| 1371.74 | IVYPPLHER | 9 | MAGE2.299 | A | 121.3 | 391.1 | 102.1 | 37.2 | 17.7 |
| 1371.71 | SVFAHPRR | 8 | MAGE2.237 | A | 678.3 | 1573.3 | 661.1 | 630.4 | 152.3 |
| 1371.63 | SVFSITINK | 9 | MAGE2.69 | A | 20.8 | 8.5 | 3626,3 | 18804.6 | 6.0 |
| 1371.64 | SVESTTINR | 9 | MAGE2.69 | A | 60.3 | 6.8 | 46.7 | 89.1 | 6.9 |
| 1371.66 | TVINYTLWK | 9 | MAGE2.73 | A | 358.9 | 100.9 | 9126.4 | >17985.04 | 86.0 |
| 1371.65 | TVINYTLWR | 9 | MAGE2.73 | A | 300.2 | 81.0 | 713.0 | 333.6 | 14.4 |
| 1371.68 | LVHFLLLKR | 9 | MAGE2/3.116 | A | 340.4 | 368.3 | 246.3 | 92.2 | 24.3 |
| 1371.69 | YVFPVIFSK | 9 | MAGE3.138 | A | 24.8 | 3.3 | 2922.5 | 792.1 | 1.7 |
| 1371.70 | YVFPVIFSR | 9 | MAGE3.138 | A | 37.4 | 2.8 | 8.4 | 13.3 | 0.5 |
| 1371.16 | BVYSPALNK | 9 | p53.124 | A | 15.8 | 10.0 | 448.1 | 23027.5 | 801.8 |
| 1371.17 | BVYSPALNR | 9 | p53.124 | A | 27.8 | 8.5 | 34.8 | 85.8 | 11.3 |
| 1371.20 | GVRVRAMAIYK | 11 | p53.154 | A | 67.2 | 100.5 | 431.6 | >48333.33 | 31620.6 |
| 1371.21 | GVRVRAMAIYR | 11 | p53.154 | A | 2401.3 | 23166.0 | 136.6 | 5237.8 | 5305.0 |
| 1371.18 | KVFBQLAK | 8 | p53.132 | A | 652.5 | 484.7 | 6529.2 | >48333.33 | 8474.6 |
| 1371.15 | KVYQGSYGFK | 10 | p53.101 | A | 38.5 | 10.1 | 139.8 | >48333.33 | 26.7 |
| 1371.14 | KVYQGSYGFR | 10 | p53.101 | A | 37.9 | 64.2 | 74.1 | 10311.3 | 33.7 |
| 1371.31 | RVBABPGRDRK | 11 | p53.273 | A | 324.7 | 225.2 | 4670.7 | >48333.33 | 3530.5 |
| 1371.30 | RVBABPGRDRR | 11 | p53.273 | A | 3195.1 | 5070.9 | 183.9 | >48333.33 | 2463.8 |
| 1371.22 | RVRAMAIYR | 9 | p53.156 | A | 45.4 | 1691.1 | 9.1 | 149.3 | 427.1 |
| 1371.24 | SVBMGGMNK | 9 | p53.240 | A | 14.0 | 17.1 | 8933.0 | >48333.33 | 38.0 |
| 1371.26 | SVBMGGMNR | 9 | p53.240 | A | 162.4 | 96.6 | 119.3 | 885.0 | 11.6 |
| 1371.25 | SVBMGGMNRK | 10 | p53.240 | A | 102.4 | 54.5 | >17162.33 | >48333.33 | 12.9 |
| 1371.27 | SVBMGGMNRR | 10 | p53.240 | A | 1030.2 | 20.2 | 637.3 | 1515.0 | 11.0 |
| 1371.32 | SVSRHKKLMFK | 11 | p53.376 | A | 34.5 | 61.9 | 295.8 | 185555 | 2531.7 |
| 1371.33 | SVSRHKKLMFR | 11 | p53.376 | A | 211.9 | 2976.9 | 184.7 | 1571.2 | 499.8 |
| F029.09 | AAMXDTVVFK | 10 | Naturally processed | A | 258.8 | 8.3 | | | |
| F020.06 | ASFDKAKLK | 9 | Naturally processed | | 43.9 | 15.8 | | | |
| 1023.01 | EVAPPEYHR | 9 | Naturally processed | | >27500 | 60000.0 | 36000.0 | 78.4 | 36.4 |
| 1023.05 | EVILIDPFHK | 10 | Naturally processed | | >22000 | 4472.1 | >180 | 11581.5 | 26.7 |
| F023.05 | KVVNPLFEK | 9 | Naturally processed | | 162.4 | 8.4 | | | |
| F020.07 | KVVNPLPEK | 9 | Naturally processed | | 40.6 | 13.9 | | | |
| S3R | RTQNVLGEK | 9 | Naturally processed | | 152.8 | 32.5 | | | |
| F029.11 | RVEQAVESMVK | 11 | Naturally processed | | 1226.8 | 57.2 | | | |
| F020.08 | SVLNLVIVK | 9 | Naturally processed | | 129.8 | 7.2 | | | |
| 952.32 | AAAAAAAAAK | 10 | Artificial sequence | A | 501.6 | 49.7 | | | |
| 952.42 | AAYAAAAAAK | 10 | Artificial sequence | A | 26.2 | 31.3 | | | |
| 952.41 | AAYAAAAAK | 9 | Artificial sequence | A | 23.6 | 14.7 | | | |
| 952.43 | AAYAAAAAR | 9 | Artificial sequence | A | 125.5 | 124.8 | | | |
| 952.29 | AIAAAAAAAK | 10 | Artificial sequence | A | 109.6 | 47.0 | | | |
| 952.34 | AIKAAAAAR | 9 | Artificial sequence | A | 141.6 | 107.6 | | | |
| 952.30 | ALAAAAAAAK | 10 | Artificial sequence | A | 149.8 | 50.0 | | | |
| 952.39 | ALAAAAAAAR | 10 | Artificial sequence | A | 366.5 | 2667.3 | | | |
| 4.0105 | ALAAAAADK | 9 | Artificial sequence | A | 57.7 | 148.3 | | | |
| 981.31 | ALAAAAAEK | 9 | Artificial sequence | A | 189.7 | 42.9 | | | |
| 4.0106 | ALAAAAAFK | 9 | Artificial sequence | A | 27.9 | 13.4 | | | |
| 4.0111 | ALAAAAANK | 9 | Artificial sequence | A | 31.8 | 17.5 | | | |
| 4.0110 | ALAAAAAPK | 9 | Artificial sequence | A | 19.3 | 8.3 | | | |
| 981.30 | ALAAAAAQK | 9 | Artificial sequence | A | 61.1 | 37.5 | | | |
| 4.0104 | ALAAAAARK | 9 | Artificial sequence | A | 21.6 | 10.5 | | | |
| 4.0108 | ALAAAAASK | 9 | Artificial sequence | A | 38.6 | 27.4 | | | |
| 4.0109 | ALAAAAATK | 9 | Artificial sequence | A | 19.9 | 23.3 | | | |
| 981.32 | ALAAAAAVK | 9 | Artificial sequence | A | 131.0 | 79.0 | | | |
| 4.0107 | ALAAAAAYK | 9 | Artificial sequence | A | 17.0 | 10.6 | | | |
| 4.0094 | ALAAAADAK | 9 | Artificial sequence | A | 169.1 | 53.4 | | | |
| 4.0095 | ALAAAAEAK | 9 | Artificial sequence | A | 126.5 | 65.1 | | | |
| 4.0100 | ALAAAAFAK | 9 | Artificial sequence | A | 15.2 | 5.7 | | | |
| 981.27 | ALAAAALAK | 9 | Artificial sequence | A | 113.4 | 20.7 | | | |
| 981.29 | ALAAAAKAK | 9 | Artificial sequence | A | 100.0 | 133.3 | | | |
| 4.0096 | ALAAAALAK | 9 | Artificial sequence | A | 39.1 | 11.8 | | | |
| 4.0101 | ALAAAANAK | 9 | Artificial sequence | A | 51.9 | 22.9 | | | |
| 4.0103 | ALAAAAPAK | 9 | Artificial sequence | A | 11.7 | 5.5 | | | |
| 981.28 | ALAAAAQAK | 9 | Artificial sequence | A | 137.5 | 74.1 | | | |
| 4.0102 | ALAAAASAK | 9 | Artificial sequence | A | 27.6 | 37.5 | | | |
| 4.0099 | ALAAAAYAK | 9 | Artificial sequence | A | 47.9 | 10.5 | | | |
| 4.0087 | ALAAADAAK | 9 | Artificial sequence | A | 195.8 | 556.6 | | | |
| 981.24 | ALAAAIAAK | 9 | Artificial sequence | A | 100.0 | 15.8 | | | |
| 981.25 | ALAAAKAAK | 9 | Artificial sequence | A | 196.4 | 600.0 | | | |
| 4.0093 | ALAAAMAAK | 9 | Artificial sequence | A | 57.3 | 10.3 | | | |
| 4.0090 | ALAAANAAK | 9 | Artificial sequence | A | 35.6 | 19.3 | | | |
| 4.0092 | ALAAAPAAK | 9 | Artificial sequence | A | 31.2 | 29.4 | | | |
| 4.0089 | ALAAAQAAK | 9 | Artificial sequence | A | 23.8 | 13.3 | | | |
| 981.26 | ALAAASAAK | 9 | Artificial sequence | A | 78.6 | 46.2 | | | |
| 4.0091 | ALAAATAAK | 9 | Artificial sequence | A | 60.0 | 17.7 | | | |
| 4.0088 | ALAAAYAAK | 9 | Artificial sequence | A | 19.5 | 5.7 | | | |
| 4.0076 | ALAADAAAK | 9 | Artificial sequence | A | 61.2 | 113.3 | | | |
| 4.0077 | ALAAEAAAK | 9 | Artificial sequence | A | 46.9 | 132.5 | | | |
| 4.0075 | ALAAGAAAK | 9 | Artificial sequence | A | 18.8 | 36.5 | | | |
| 1161:16 | ALAAGAAAK | 9 | Artificial sequence | A | 29.1 | 32.7 | 63639.6 | >27905.26 | 2285.7 |
| 4.0085 | ALAAHAAAK | 9 | Artificial sequence | A | 33.1 | 33.3 | | | |
| 4.0078 | ALAAIAAAK | 9 | Artificial sequence | A | 20.4 | 13.7 | | | |
| 981.22 | ALAAKAAAK | 9 | Artificial sequence | A | 91.7 | 111.1 | | | |
| 4.0086 | ALAALAAAK | 9 | Artificial sequence | A | 38.0 | 61.6 | | | |
| 4.0082 | ALAANAAAK | 9 | Artificial sequence | A | 40.1 | 93.7 | | | |
| 4.0084 | ALAAPAAAK | 9 | Artificial sequence | A | 13.9 | 6.9 | | | |
| 4.0081 | ALAAQAAAK | 9 | Artificial sequence | A | 20.3 | 50.2 | | | |
| 981.23 | ALAASAAAK | 9 | Artificial sequence | A | 186.4 | 33.3 | | | |
| 4.0083 | ALAATAAAK | 9 | Artificial sequence | A | 38.4 | 6.8 | | | |
| 98121 | ALAAVAAAK | 9 | Artificial sequence | A | 91.7 | 9.2 | | | |
| 4.0079 | ALAAYAAAK | 9 | Artificial sequence | A | 9.9 | 11 | | | |
| 4.0069 | ALADAAAAK | 9 | Artificial sequence | A | 32.1 | 13.6 | | | |
| 981.17 | ALAEAAAAK | 9 | Artificial sequence | A | 423.1 | 54.6 | | | |
| 4.0070 | ALAFAAAAK | 9 | Artificial sequence | A | 11.5 | 17.7 | | | |
| 4.0068 | ALAGAAAAK | 9 | Artificial sequence | A | 12.7 | 14.6 | | | |
| 981.19 | ALAKAAAAK | 9 | Artificial sequence | A | 25.6 | 82.2 | | | |
| 981.18 | ALANAAAAK | 9 | Artificial sequence | A | 78.6 | 4.7 | | | |
| 4.0072 | ALAPAAAAK | 9 | Artificial sequence | A | 12.6 | 7.0 | | | |
| 4.0074 | ALAQAAAAK | 9 | Artificial sequence | A | 16.0 | 13.0 | | | |
| 4.0073 | ALARAAAAK | 9 | Artificial sequence | A | 7.3 | 14 | | | |
| 4.0071 | ALASAAAAK | 9 | Artificial sequence | A | 25.1 | 11.8 | | | |
| 981.20 | ALAVAAAAK | 9 | Artificial sequence | A | 40.7 | 18.2 | | | |
| 4.0064 | ALDAAAAAK | 9 | Artificial sequence | A | 121.0 | 500.0 | >73484.69 | >54804.85 | 80000.0 |
| 981.15 | ALFAAAAAK | 9 | Artificial sequence | A | 25.0 | 22.2 | 2805.0 | 96666.7 | 898.9 |
| 4.0066 | ALHAAAAAK | 9 | Artificial sequence | A | 25.6 | 28.4 | 9091.4 | >54804.85 | 20000.0 |
| 4.0067 | ALIAAAAAK | 9 | Artificial sequence | A | 36.7 | 31.0 | >25980.76 | >54804.85 | 2352.9 |
| 981.16 | ALKAAAAAK | 9 | Artificial sequence | A | 122.2 | 779.2 | 1450.5 | 72500.0 | 80000.0 |
| 4.0065 | ALMAAAAAK | 9 | Artificial sequence | A | 15.6 | 6.8 | 6266.8 | >83715.79 | 824.7 |
| 1012.11 | ALNAAAAAK | 9 | Artificial sequence | A | 60.7 | 24.0 | 4751.7 | >17985.04 | 20000.0 |
| 4.0060 | ALNAAAAAK | 9 | Artificial sequence | A | 74.0 | 20.7 | 10954.5 | >54804.85 | 80000.0 |
| 4.0063 | ALPAAAAAK | 9 | Artificial sequence | A | 69.9 | 129.6 | 90000.0 | >54804.85 | 26666.7 |
| 4.0080 | ALQAAAAAK | 9 | Artificial sequence | A | 57.0 | 65.1 | 519615 | >54804.85 | 80000.0 |
| 1012:10 | ALQAAAAAK | 9 | Artificial sequence | A | 95.7 | 68.4 | 25714.3 | >17985.04 | 80000.0 |
| 1012:08 | ALRAAAAAK | 9 | Artificial sequence | A | 55.0 | 1474.4 | 2578.0 | 96666.7 | 80000.0 |
| 4.0061 | ALSAAAAAK | 9 | Artificial sequence | A | 44.1 | 37.5 | 51961.5 | >54804.85 | 20000.0 |
| 4.0062 | ALTAAAAAK | 9 | Artificial sequence | A | 66.3 | 125.3 | >73484.69 | >54804.85 | 20000.0 |
| 981.14 | ALYAAAAAK | 9 | Artificial sequence | A | 31.4 | 21.4 | 3541.0 | 96666.7 | 2726.4 |
| 952.40 | AMAAAAAAAR | 10 | Artificial sequence | A | 482.4 | 2508.7 | | | |
| 1022.03 | AMAAAAAAH | 9 | Artificial sequence | A | 279.4 | 6396.0 | | | |
| 95236 | AMAAAAAAR | 9 | Artificial sequence | A | 73.3 | 288.7 | | | |
| 978.01 | ASYAAAAAK | 9 | Artificial sequence | A | 28.2 | 11.5 | | | |
| 955.04 | ATAAAAAAAR | 10 | Artificial sequence | A | 662.1 | 280.5 | | | |
| 1022.04 | ATAAAAAAH | 9 | Artificial sequence | A | 1457.0 | 218.2 | | | |
| 955.03 | ATAAAAAAR | 9 | Artificial sequence | A | 55.1 | 39.3 | | | |
| 952.28 | AVAAAAAAAK | 10 | Artificial sequence | A | 456.0 | 83.0 | | | |
| 952.37 | AVAAAAAAAR | 10 | Artificial sequence | A | 405.5 | 193.1 | | | |
| 952.33 | AVAAAAAAR | 9 | Artificial sequence | A | 102.5 | 72.8 | | | |
| 952.08 | AVAAAAANAK | 10 | Artificial sequence | A | 129.6 | 40.8 | | | |
| 952.05 | AVAAAAANK | 9 | Artificial sequence | A | 11.0 | 41.4 | | | |
| 952.07 | AVAAAAIAAK | 10 | Artificial sequence | A | 26.9 | 28.3 | | | |
| 952.04 | AVAAAAIAK | 9 | Artificial sequence | A | 87.6 | 21.1 | | | |
| 95206 | AVAAALAAAK | 10 | Artificial sequence | A | 191.5 | 36.5 | | | |
| 952.03 | AVAAALAAK | 9 | Artificial sequence | A | 65.8 | 31.5 | | | |
| 952.18 | AVAKAAAAAK | 10 | Artificial sequence | A | 128.3 | 52.6 | | | |
| 952.15 | AVAKAAAAK | 9 | Artificial sequence | A | 40.2 | 88.9 | | | |
| 952.02 | AVAPAAAAAK | 10 | Artificial sequence | A | 48.5 | 21.8 | | | |
| 952.01 | AVAPAAAAK | 9 | Artificial sequence | A | 51.5 | 9.6 | | | |
| 952.14 | AVAPAAAANK | 10 | Artificial sequence | A | 44.4 | 36.4 | | | |
| 952.13 | AVAPAAAIAK | 10 | Artificial sequence | A | 51.6 | 11.4 | | | |
| 952.11 | AVAPAAANAK | 10 | Artificial sequence | A | 107.7 | 57.2 | | | |
| 952.10 | AVAPAAIAAK | 10 | Artificial sequence | A | 13.1 | 20.1 | | | |
| 952.12 | AVAPAALAAK | 10 | Artificial sequence | A | 825 | 10.8 | | | |
| 952.09 | AVAPALAAAK | 10 | Artificial sequence | A | 53.3 | 15.4 | | | |
| 952.20 | AVYAAAAAAK | 10 | Artificial sequence | A | 55.2 | 38.7 | | | |
| 952.21 | AVYAAAAAAR | 10 | Artificial sequence | A | 143.5 | 76.5 | | | |
| 952.19 | AVYAAAAAK | 9 | Artificial sequence | A | 42.0 | 36.3 | | | |
| 952.22 | AVYAAAAAR | 9 | Artificial sequence | A | 12.7 | 40.4 | | | |
| 981.03 | FLAAAAAAK | 9 | Artificial sequence | A | 35.5 | 79.0 | 180000.0 | 72500.0 | 17.8 |
| 1012.01 | ILAAAAAAK | 9 | Artificial sequence | A | 33.1 | 46.3 | | | |
| 1012.02 | MLAAAAAAK | 9 | Artificial sequence | A | 13.9 | 15.0 | | | |
| 1012.07 | NLAAAAAAK | 9 | Artificial sequence | A | 138.3 | 178.4 | | | |
| 1012.04 | PLAAAAAAK | 9 | Artificial sequence | A | 294.0 | 8017.8 | | | |
| 1012.06 | QLAAAAAAK | 9 | Artificial sequence | A | 205.4 | 107.4 | | | |
| 1012.05 | SLAAAAAAK | 9 | Artificial sequence | A | 25.2 | 6.0 | | | |
| 1012.03 | TLAAAAAAK | 9 | Artificial sequence | A | 20.2 | 80.9 | | | |

**Table 20**

| | | | HLA- A24 supertype binding affinity (IC50 nM) | | | |
|---|---|---|---|---|---|---|
| Peptide | Sequence | Source | A*2402 | A*2301 | A*2902 | A*3002 |
| 1489.06 | AATLGFGAY | HCV..IV.1264 | | | 2240 | 491 |
| 1489.11 | AQPGYPWPLY | HCV..II.77 | | | 133 | 3.5 |
| 1489.05 | CSFSIFLLA | HCV..IV.172 | | | 153 | 11100 |
| 1489.1 | LHGPTPLLY | HCV..II 1623 | | | 180 | 1075 |
| 1489.12 | TCGFADLMGY | HCV..IV.127 | | | 350 | 356 |
| F185.03 | RYLRDQQLL | WV..gp41583 | 30 | | | |
| F185.05 | RYPLTFGWCF | HN..nef.138 | 17 | | | |
| 78.0346 | AFADLTVVY | HPV.33.E6.46 | 425 | 71 | 19408 | 58420 |
| 78.0368 | AFKDLCIVY | HPV.45.E6.48 | 44911 | 14034 | 32 | 0.49 |
| 78.0360 | AFKDLFVVY | HPV.18.E6.48 | 419 | 123 | 37648 | 24961 |
| 78.0246 | ATLERTEVY | HPV.45.E6.37 | >141441 | >24519.62 | 11383 | 175 |
| 78.0023 | ATSNYYIVTY | HPV52.E7.50 | >65777.37 | >22775.44 | 2117 | 118 |
| 78.0349 | AYAACHKCI | HPV.45.E6.61 | 73 | 60 | 3300 | 139 |
| 78.0358 | CYSLYGTIL | HPV.16.E6.87 | 88 | 133 | 13882 | >21070.15 |
| 78.0379 | DYSVYGATL | HPV.56.E6.83 | 33359 | 2057 | 1460 | 19 |
| 78.0177 | EYRHYCYSLY | HPV.16.E6.82 | 117278 | >18738.91 | 2449 | 120 |
| 78.0354 | EYRHYNYSL | HPV.58.E6.75 | 41671 | 23490 | 268 | 1473 |
| 78.0185 | EYRHYNYSLY | HPV58.E6.75 | 8139 | 10625 | 1774 | 67 |
| 78.0180 | EYRHYNYSVY | HPV.33.E6.75 | 45280 | 22917 | 16016 | 253 |
| 78.0350 | EYRHYQYSL | HPV.52.E6.75 | 72 | 100 | 58321 | 1098 |
| 78.0183 | EYRHYQYSLY | HPV.52.E6.75 | 8378 | 1576 | 2372 | 130 |
| 78.0176 | FYSKVRKYRY | HPV.56.E6.72 | 2309 | 983 | 146 | 326 |
| 78.0172 | FYSKVSEFRW | HPV.31.E6.69 | 8.6 | 8.8 | 1777 | 15220 |
| 78.0182 | FYSRIRELRY | HPV.45.E6.71 | 356 | 105 | 20 | 150 |
| 78.0243 | ISEYRHYNY | HPV.33.E6.73 | 125794 | >20304.18 | 1329 | . 32 |
| 78.0254 | ISEYRHYNY | HPV.58.E6.73 | >99979.17 | >24899.09 | 853 | 81 |
| 1497.13 | KFLFTDLRI | HPV.52.E6.44 | 2151 | 18 | 2319 | 1595 |
| 78.0357 | KFYSKISEY | HPV.16.E6.75 | 97 | 95 | 40636 | 14219 |
| 1497.15 | KYRYYDYSVY | HPV.56.E6.78 | 169334 | 86607 | 17797 | 2.3 |
| 78.0252 | LCDLLIRCY | HPV.56.E6.99 | >113982.57 | >25061.21 | 557 | 26 |
| 78.0372 | LFTDLRIVY | HPV.52.E6.46 | 377 | 156 | 25003 | 22 |
| 78.0361 | LFVVYRDSI | HPV.18.E6.52 | 60761 | 20899 | 464 | 100 |
| 78.0378 | LFYSKVRKY | HPV.56.E6.71 | 43591 | 12916 | 12 | 89 |
| 78.0244 | LKEYVLDLY | HPV.33.E7.8 | >116712.75 | >24519.62 | 127123 | 16 |
| 78.0006 | LQDIEITCVY | HPV.18.E6.25 | 90823 | 27246 | 4044 | 148 |
| 78.0027 | LSKISEYRHY | HPV.58.E6.70 | >65777.37 | >22775.44 | 101409 | 159 |
| 78.0019 | LSKISEYRHY | HPV.52.E6.70 | >68154.86 | >22775.44 | 55190 | 186 |
| 78.0348 | LYPEPTDLY | HPV.33.E7.15 | 58956 | 21752 | 36 | 71 |
| 78.0376 | NFACTELKL | HPV.56.E6.47 | 48436 | 18695 | 202 | 1.02 |
| 78.0347 | NYSVYGNTL | HPV.33.E6.80 | 715 | 365 | >31079.62 | 36210 |
| 78.0356 | PYAVCDKCL | HPV.16.E6.66 | 97 | 20 | 44214 | 5713 |
| 78.0377 | PYAVCRVCL | HPV.56.E6.62 | 51 | 129 | 40187 | 2092 |
| 78.0175 | PYAVCRVCLL | HPV.56.E6.62 | 133 | 79 | 11832 | 42021 |
| 78.0249 | QAEQATSNY | HPV.52.E7.46 | 250000 | >20304.18 | 4203 | 109 |
| 1497.12 | QFAFKDLCI | HPV.45.E6.46 | 20636 | 370 | >30457.25 | >27675.94 |
| 78.0005 | RFEDPTRRPY | HPV.18.E6.3 | >66361.54 | >22463.51 | 17839 | 145 |
| 78.0363 | RFHNIGGRW | HPV.31.E6.124 | 71804 | 27530 | 1595 | 9.5 |
| 78.037.5 | RFHNIMGRW | HPV.52.E6.124 | 469 | 352 | >24155.43 | >18916.25 |
| 78.0359 | RFHNIRGRW | HPV.16.E6.131 | 53237 | 11416 | 18 | 58 |
| 78.0366 | RFHNISGRW | HPV.33.E6.124 | >76072.88 | 22871 | 174 | 37 |
| 78.0384 | RFHNISGRW | HPV.58.E6.124 | 209657 | 28791 | 201 | 35 |
| 78.0370 | RFHSIAGQY | HPV.45.E6.126 | 52 | 250 | 32588 | 34549 |
| 78.0373 | RFLSKISEY | HPV.52.E6.68 | 65 | 93 | 32015 | 14086 |
| 78.0365 | RFLSKISEY | HPV.33.E6.68 | 472 | 121 | 34623 | 23 |
| 1497.04 | TFCCKCDSTL | HPV.16.E7.56 | 3627 | 427 | >28937.69 | 22604 |
| 78.0250 | TSNYYIVTY | HPV.16.E7.56 | >113982.57 | >25061.21 | 3313 | 76 |
| 78.0174 | TYCHSCDSTL | HPV.52.E7.58 | 452 | 122 | 29791 | 30747 |
| 78.0353 | VFADLRIVY | HPV.58.E6.46 | 499 | 1336 | >35952.44 | >19018.22 |
| 78.0171 | VFEFAFKDLF | HPV.18.F6.44 | 69 | 8.9 | 1090 | 14434 |
| 78.0340 | VYCKTVLEL | HPV.18.E6.33 | 456 | 348 | >31079.62 | 15605 |
| 78.0364 | VYDFAFADL | HPV.33.E6.42 | >163846.38 | >20985.44 | 163 | 579 |
| 78.0381 | VYDFVFADL | HPV.58.E6.42 | 385 | 93 | 27168 | 27 |
| 78.0369 | VYGETLEKI | HPV.45.E6.85 | 430 | 149 | 12156 | 13 |
| 78.0371 | VYKFLFTDL | HPV.52.E6.42 | 35396 | 1536 | 1707 | 27 |
| 78.0351 | VYNFACTEL | HPV.56.E6.45 | 366 | 114 | >29714.57 | 12350 |
| 78.0002 | YSKISEYRHY | HPV.16.E6.77 | >66361.54 | >22463.51 | 6448 | 205 |
| 78.0009 | YSKVSEFRWY | HPV.31.E6.70 | 73696 | >22249.75 | 4514 | 185 |
| 78.0247 | YSRIRELRY | HPV.45.E6.72 | 10906 | 7337 | 1991 | 22 |
| 1489.22 | ALFQEYQCY | Pf..CSP.18 | | | 149 | 1032 |
| 1489.27 | FVVPGAATPY | Pf..SSP2.520 | | | 317 | 4621 |
| 1489.61 | FYFILVNLLI | Pf..LSA1.9 | 20 | 3.7 | 362 | 8591 |
| 1489.55 | YYGKQENWY | Pf..CSP.55 | 2883 | 19195 | 373 | 3155 |
| F185.04 | AFLPWHRLF | Tyrosinase. | 20 | | | |
| 1489.18 | FVQENYLEY | MAGE3.250 | | | 3.2 | 99 |
| 1489.16 | LVQENYLEY | MAGE2.250 | | | 13 | 72 |
| 1489.15 | LVTCLGLSY | MAGE2.178 | | | 18 | 4101 |
| 1461.06 | MEVDPIGHLY | MAGE3.167 | | | 333 | 87 |
| F185.06 | SYLDSGIHF | ..B-catenin.29 | 50 | | | |

**TABLE 21**

| Peptide | Sequence | AA | Source | Analog | A*2402 (IC50 nM) |
|---|---|---|---|---|---|
| 57.0073 | FYNPPTTAKF | 10 | CEA.27 | A | 183 |
| 57.0037 | IYPNASLLF | 9 | CEA.101 | A | 2.6 |
| 57.0039 | LYGPDAPTF | 9 | CEA.234 | A | 65 |
| 57.0042 | LYWVNGQSF | 9 | CEA.533 | A | 16 |
| 57.0038 | LYWVNNQSF | 9 | CEA.177 | A | 64 |
| 57.0078 | QYSWLIDGNF | 10 | CEA.446 | A | 63 |
| 57.0044 | QYSWRINGF | 9 | CEA.624 | A | 108 |
| 57.0036 | RYCIPWQRF | 9 | CEA.10 | A | 191 |
| 57.0072 | RYCIPWQRLF | 10 | CEA.10 | A | 59 |
| 57.0079 | SYLSGANLNF | 10 | CEA.604 | A | 11 |
| 57.0045 | TYACFVSNF | 9 | CEA.652 | A | 9.9 |
| 57.0075 | TYQQSTQELF | 10 | CEA.276 | A | 317 |
| 57.0041 | TYYRPGVNF | 9 | CEA.425 | A | 54 |
| 57.0076 | VYAEPPKPFF | 10 | CEA.318 | A | 27 |
| 57.0074 | VYPELPKPSF | 10 | CEA.140 | A | 114 |
| 57.0077 | YYRPGVNLSF | 10 | CEA.426 | A | 12 |
| 1081.05 | SYVPSAEQI | 9 | Pf.CSP.280 | | 69 |
| 1120.10 | TYQRTRALF | 9 | Flu.NP.147 | A | 1.5 |
| 1120.12 | TYQRTRALI | 9 | Flu.NP.147 | A | 33 |
| 1120.11 | TYQRTRALL | 9 | Flu.NP.147 | A | 227 |
| 57.0049 | AYPDSLPDF | 9 | Hcr24/neu.414 | A | 43 |
| 57.0050 | AYSLTLQGF | 9 | Her2/neu.440 | A | 56 |
| 57.0048 | CYGLGMEHF | 9 | Her2/neu.342 | A | 165 |
| 57.0051 | EYVNARHCF | 9 | Her2/neu.553 | A | 150 |
| 57.0082 | GYSYLEDVRF | 10 | Her2/neu.832 | A | 235 |
| 57.0053 | KYMALESIP | 9 | Her2/neu.887 | A | 20 |
| 57.0080 | LYISAWPDSF | 10 | Her2/neu.410 | A | 10 |
| 57.0052 | PYVSRLLGF | 9 | Her2/neu.780 | A | 9.0 |
| 57.0059 | RYARDPQRF | 9 | Her2/neu.978 | A | 120 |
| 57.0046 | RYGLLLALF | 9 | Her2/neu.8 | A | 1.5 |
| 57.0058 | RYRELVSEF | 9 | Her2/neu.968 | A | 37 |
| 57.0054 | RYTHQSDVF | 9 | Her2/neu.898 | A | 60 |
| 57.0056 | SYGVTVWEF | 9 | Her2/neu.907 | A | 28 |
| 57.0047 | TYLPTNASF | 9 | Her2/neu.63 | A | 46 |
| 57.0057 | VYMIMVKCF | 9 | Her2/neu.951 | A | 20 |
| 57.0055 | VYSYGVTVF | 9 | Her2/neu.905 | A | 18 |
| F171.13 | NYKRCFPVI | 9 | Mage4.143 | | 6.1 |
| 57.0063 | EYLQLVFGF | 9 | MAGE2.156 | A | 6.5 |
| 57.0087 | EYLWGPRALF | 10 | MAGE2.270 | A | 10 |
| 57.0062 | IYSKASEYF | 9 | MAGE2.150 | A | 15 |
| 57.0061 | KYVELVHFF | 9 | MAGE2.112 | A | 8.0 |
| 57.0085 | LYILVTCLGF | 10 | MAGE2.175 | A | 20 |
| 57.0060 | MYPDLESEF | 9 | MAGE2.97 | A | 51 |
| 57.0084 | SYSTTINYTF | 10 | MAGE2.70 | A | 17 |
| 57.0088 | SYVKVLHHTF | 10 | MAGE2.282 | A | 60 |
| 57.0064 | VYPKTGLLF | 9 | MAGE2.195 | A | 6.0 |
| 57.0086 | VYPKTGLLlF | 10 | MAGE2.195 | A | 2.9 |
| 57.0068 | IYPKAGLLF | 9 | MAGE3.195 | A | 9.0 |
| 57.0093 | IYPKAGLLIF | 10 | MAGE3.195 | A | 1.4 |
| 57.0067 | IYSKASSSF | 9 | MAGE3.150 | A | 415 |
| 57.0092 | LYIFATCLGF | 10 | MAGE3.175 | A | 11 |
| 57.0066 | NYQYFFPVF | 9 | MAGE3.142 | A | 3.8 |
| 57.0090 | NYQYFFPVIF | 10 | MAGE3.142 | A | 23 |
| 57.0095 | SYPPLHEWVF | 10 | MAGE3.300 | A | 5.8 |
| 57.0065 | TYPDLESEF | 9 | MAGE3.97 | A | 220 |
| 57.0071 | SYGFRLGFF | 9 | p53.106 | A | 124 |
| 57.0096 | TYQGSYGFRF | 10 | p53.102 | A | 33 |
| 57.0070 | TYSDLWKLF | 9 | p53.18 | A | 5.7 |
| 972.05 | SYFPETTHI | 9 | | | 458 |
| 996.01 | AWAKAAAAF | 9 | Artificial sequence | Poly | 373 |
| F030.01 | AYAKAAAAAF | 10 | Artificial sequence | Poly | 38 |
| 1021.01 | AYAKAAAAW | 9 | Artificial sequence | Poly A | 140 |
| 1079.01 | KYNPMKTHI | 9 | Artificial sequence | Consensus | 56 |
| 1086.16 | KYPDFVDAL | 9 | Artificial sequence | Consensus | 62 |
| F020.09 | AYVHHMHF | 9 | Naturally processed | | 23 |
| F020.12 | KYPENFFLL | 9 | Naturally processed | | 5.5 |
| F020.15 | YYEEQHPEL | 9 | Naturally processed | | 115 |

**Table 22**

| | | | HLA-B7 supertype binding affinity (IC50 mM) | | | | |
|---|---|---|---|---|---|---|---|
| Peptide | Sequence | Source | B*0702 | B*3501 | B*5101 | B*5301 | B*5401 |
| 1259.01 | GPRLGVRAT | HCV.Core.41 | 128 | >39374.41 | >23865.72 | >48927.08 | 64167 |
| 1468.03 | IPLVGAPL | HCV.Core.137 | 25 | 295 | 134 | 7568 | 598 |
| 1468.01 | LPALSTGLI | HCV.E2.681 | 427 | 25792 | 2580 | 1036 | 5778 |
| 1468.02 | LPCSFTTLPA | HCV.NS1/E2.680 | 981 | 3145 | 1854 | 43849 | 2.2 |
| 78.0396 | CPEEKQRHL | HPV16.E6.118 | 10.3 | >44991.38 | 91667 | >79732.67 | >60017.72 |
| 78.0399 | CPEEKQRHL | HPV31.E6.111 | 3.0 | >44991.38 | 1982 | >79732.67 | 15717 |
| 78.0395 | DPQERPRKL | HPV16.E6.11 | 92 | >44278.57 | 4010 | >79732.67 | >60017.72 |
| 78.0397 | DPTRRPYKL | HPV18.E6.6 | 111 | >44991.38 | >35950.78 | >79732.67 | >69121.86 |
| 78.0191 | EPQRHTMLCM | HPV18.E7.55 | 337 | 5482 | 1824 | 3258 | >53217.09 |
| 78.0195 | FPYAVCRVCL | HPV56.E6.61 | 148 | 2253 | 73 | 3580 | 193 |
| 78.0197 | GPRETLQEIV | HPV45.E7.3 | 348 | >34241.87 | >29418.5 | >81260.68 | 41613 |
| 78.0028 | HPEPTDLFCY | HPV58.E7.16 | >131287.73 | 3186 | 18391 | 346 | >62894.33 |
| 78.0190 | IPHAACHKCI | HPV18.E6.60 | 246 | >34241.87 | 2070 | 3661 | 3002 |
| 78.0398 | KPLNPAEKL | HPV18.E6.110 | 20 | >44278.57 | 20215 | >79732.67 | >60017.72 |
| 70392 | NPQERPRSL | HPV56.E6.7 | 121 | 33015 | 246 | 34824 | 65361 |
| 78.0394 | NPTLREYIL | HPV58.E7.5 | 21 | >44991.38 | 21839 | >79732.67 | >58997.31 |
| 78.0189 | NPYAVCDKCL | HPV16.E6.65 | 6755 | >34241.87 | 218 | 115379 | 97359 |
| 78.0194 | NPYGVCIMCL | HPV52.E6.58 | 382 | 38796 | 172 | 11237 | 30085 |
| 78.0402 | RPDGQAQPA | HPV33.E7.40 | 13912 | 11171 | 383 | 5497 | >60017.72 |
| 1495.01 | RPRKLPQLCT | BPV16R.E6.15 | 38 | >48807.02 | >20249.86 | >73228.35 | 79388 |
| 78.0393 | RPRRRQTQV | HPV58.E6.141 | 275 | >44278.57 | 14963 | >79732.67 | >58997.31 |
| 78.0192 | TPHGVCTKCL | HPV31.E6.58 | 38 | >34241.87 | 14667 | 44608 | 7724 |
| 1495.10 | TPTLQDYVL | HPV31.E7.5 | 3175 | 442 | 42577 | 34294 | 41338 |
| 78.0199 | VPCCECKFVV | HPV56.E7.63 | 464 | 21110 | 174 | 4882 | 20 |
| 78.0013 | YPEPTDLYCY | HPV33.E7.16 | >131287.73 | 3133 | 16160 | 74 | >62894.33 |
| 1143.05 | LGFVFTLTV | | 55000 | >72000 | 27500 | 24013 | 464 |
| 1125.02 | LPFDFTPGF | | 27500 | 13 | | | 185 |
| 1143.04 | TAVPWNASW | | >1018.52 | 5445 | 19445 | 15 | 20000 |

**TABLE 23**

| | | | | | HLA- B7 supertype binding affinity (IC50 nM) | | | | |
|---|---|---|---|---|---|---|---|---|---|
| Peptide | Sequence | AA | Source | Analog | B*0702 | B*3501 | B*5101 | B*5301 | B*5401 |
| 1196.02 | QPRAPIRPIPT | 11 | EBNA.881 | | 109.6 | | | | 14433.8 |
| 1196.03 | RPPIFIRRL | 9 | EBNA.379 | | 21.2 | | 55000.0 | | |
| 36.0023 | APIMFSNKM | 9 | Flu.RRP1.340 | | 91.0 | | | | |
| 36.0010 | APSPYNSRF | 9 | Flu.NRAM.151 | | 19.9 | | | | |
| 36.0072 | CPVGEAPSPY | 10 | Flu.NRAM.146 | | 70.3 | | | | |
| 36.0051 | DPDEGTAGV | 9 | Flu.RRP3.678 | | 119.6 | | | | |
| 36.0122 | DPGNAEFEDL | 10 | Flu.VNUC.247 | | 222.0 | | | | |
| 36.0119 | DPLAIAANII | 10 | Flu.VMT2.24 | | 86.4 | | | | |
| 36.0078 | DPNTVSSFQV | 10 | Flu.NS1.2 | | 10.5 | | | | |
| 36.0034 | DPSHEGEGI | 9 | Flu.RRP2.294 | | 10.9 | | | | |
| 36.0114 | EPFQSLVPKA | 10 | Flu.RRP3.578 | | 27.1 | | | | |
| 36.0094 | EPNGYIEGKL | 10 | Flu.RRP2.237 | | 293.7 | | | | |
| 36.0044 | FPNEVGARI | 9 | Flu.RRP3.168 | | 44.2 | | | | |
| 36.0107 | FPNEVGARIL | 10 | Flu.RRP3.168 | | 365.2 | | | | |
| 36.0019 | FPYTGDPPY | 9 | Flu.RRP1.22 | | 11.3 | | | | |
| 36.0029 | GPATAQMAL | 9 | Flu.RRP1.540 | | 45.3 | | | | |
| 36.0024 | IPAEMLASI | 9 | Flu.RRP1.368 | | 310.7 | | | | |
| 36.0088 | IPEVCLKWEL | 10 | Flu.RRP1.606 | | 122.7 | | | | |
| 36.0080 | IPKQKVAGPL | 10 | Flu.NS1.106 | | 42.3 | | | | |
| 36.0006 | IPSIQSRGL | 9 | Flu.HEMA.338 | | 350.7 | | | | |
| 36.0067 | KPGDTIIFEA | 10 | Flu.HEMA.252 | | 35.0 | | | | |
| 36.0059 | LPFDRTTVM | 9 | Flu.VNUC.418 | | 49.0 | | | | |
| 36.0028 | LPSFGVSGI | 9 | Flu.RRP1509 | | 36.8 | | | | |
| 36.0015 | LPSLPGHTA | 9 | Flu.NS1.163 | | 41.3 | | | | |
| 36.0089 | MPAHGPAKNM | 10 | Flu.RRP1.646 | | 266.7 | | | | |
| 36.0058 | NPAHKSQLV | 9 | Flu.VNUC.321 | | 2.4 | | | | |
| 36.0062 | NPECDPLLPV | 10 | Flu.HEMA.81 | | 213.3 | | | | |
| 36.0021 | NPRMFLAMI | 9 | Flu.RRP1.314 | | 25.1 | | | | |
| 36.0022 | QPEWFRNVL | 9 | Flu.RRP1.329 | | 12.9 | | | | |
| 36.0013 | RPCFWVELI | 9 | Flu.NRAM.404 | | 67.3 | | | | |
| 36.0025 | RPLLIEGTA | 9 | Flu.RRP1.393 | | 140.0 | | | | |
| 36.0060 | SPIVPSFDM | 9 | Flu.VNUC.473 | | 50.0 | | | | |
| 36.0103 | SPKGVEESSI | 10 | Flu.RRP2.624 | | 219.0 | | | | |
| 36.0037 | SPQLEGFSA | 9 | Flu.RRP2.652 | | 63.1 | | | | |
| 36.0068 | TPLGAINSSL | 10 | Flu.HEMA.298 | | 376.8 | | | | |
| 36.0079 | VPASRYLTDM | 10 | Flu.NS1.84 | | 42.4 | | | | |
| 36.0075 | YPDTGKVMCV | 10 | Flu.NRAM.267 | | 422.7 | | | | |
| 36.0039 | YPITADKRI | 9 | Flu.RRP3.55 | | 40.2 | | | | |
| 36.0041 | YPKIYKTYF | 9 | Flu.RRP3.111 | | 75.0 | | | | |
| 36.0005 | YPRLKNSYV | 9 | Flu.HEMA.175 | | 181.5. | | | | |
| 1372.03 | IPIPSSWAFI | 10 | HBV.env.324 | A | 2147.3 | 767.3 | 10.7 | 87.9 | 15802 |
| 1372.01 | LPSDFFPSI | 9 | HBV.core.19 | A | 7935.3 | 396.6 | 2.6 | 5.5 | 35.1 |
| 39.0045 | APTLWARI | 8 | HCV.2869 | A | 452.5 | >12000 | 1142.4 | >17897.86 | 13661.2 |
| 39.0086 | FPYLVAYQI | 9 | HCV.NS3.1588 | A | 3990.3 | 101.5 | 7.5 | 5.8 | 1.1 |
| 39,0044 | IPLVGAPI | 8 | HCV.137 | A | 387.4 | >12000 | 1.6 | 7282.7 | 209.3 |
| 39.0156 | IPQAVVDMVI | 10 | HCV.E1.340 | A | 7439.2 | 1358.1 | 68.6 | 135.9 | 314.3 |
| 39.0159 | LPAILSPGAI | 10 | HCV.NS4.1888 | A | 12114.5 | >12000 | 270.5 | >17897.86 | 167.6 |
| 39.0155 | LPGCSFSIFI | 10 | HCV.core.168 | A | 11224.5 | 19354.8 | 144.8 | 313.3 | 954.2 |
| 39.0043 | LPRROPRI | 8 | HCV.37 | A | 49.5 | >12000 | 276.7 | >17897.86 | 3928.6 |
| 39.0158 | YPCTVNFTII | 10 | HCV.NS1/E2.623 | A | 7087.6 | 1781.8 | 44.3 | 146.2 | 49.0 |
| 48.0019 | FPGCLREI | 8 | Her2/neu.133 | A | 225.6 | >12727.92 | 3736.2 | >19827.67 | 2062.5 |
| 48.0020 | FPGGLREL | 8 | Her2/neu.133 | A | 25.7 | 14814.8 | >12963.62 | >19827.67 | 10050.3 |
| 48.0144 | FPGGLRELQL | 10 | Her2/neu.133 | A | 132.1 | >4696.94 | >14699.37 | 2785.2 | 1625.3 |
| 48.0027 | FPKANKEI | 8 | Het2/neu.760 | A | 64.5 | >12727.92 | 4913.5 | >19827.67 | 3079.8 |
| 45.0128 | LPAARPAGATI | 11 | Her2/neu.1157 | A | 7.0 | >13606.72 | 27.2 | 5834.3 | 5952.4 |
| 45.0030 | LPSETDGI | 8 | Hef2/neu.1120 | A | 530.5 | >20784.61 | 496.4 | >13152.19 | 29850.8 |
| 34.0264 | SPGGLRFLQI | 10 | Her2/neu.133 | A | 90.4 | >16099.69 | 7812.5 | >10960.16 | 3587.6 |
| 45.0124 | SPLDSTPYRSI | 11 | Her²/neu.998 | A | 242.8 | >18000 | 2121.6 | >93000 | 5527.9 |
| 1146.02 | FAVRPQVPL | 9 | HIV.nef.84 | A | 1845.7 | 171.4 | 526.3 | 6576.1 | 4724.6 |
| 1146:07 | FLVRPQVPL | 9 | HIV.nef.84 | A | 10041.6 | >0 | 55000.0 | 93000.0 | 100000.0 |
| 1181.14 | FPVRPQVPC | 9 | HIV.nef.84 | A | 7145.6 | 1623.0 | 55000.0 | 12427.7 | 71.4 |
| 1181.12 | FPVRPQVPG | 9 | HIV.nef.84 | A | 969.3 | 78.6 | 55000.0 | 6711.7 | 1.9 |
| 1181.11 | FPVRPQVPK | 9 | HIV.nef.84 | A | 7489.7 | 7589.5 | >152.78 | 93000.0 | 5058.0 |
| 1181.09 | FPVRPQVPN | 9 | HIV.nef.84 | A | 55000.0 | 3087.0. | 55000.0 | 20795.4 | 6.3 |
| 1181.08 | FPVRPQVPQ | 9 | HIV.nef.84 | A | 7367.7 | 353.0 | >152.78 | 5858.5 | 2078.9 |
| 1181:15 | FPVRPQVPT | 9 | HIV.nef.84 | A | 830.2 | 307.0 | 18333.3 | 65760.9 | 1.9 |
| 1181:07 | FPVRPQVPY | 9 | HIV.nef.84 | A | 1833.3 | 65.5 | >152.78 | 140.9 | 1717.0 |
| 1146.06 | FXVRPQVPL | 9 | HIV.nef.84 | A | 2690.1 | 1697.2 | 278.5 | 93000.0 | 5863.7 |
| 1372.05 | IPIHYCAPI | 9 | HIV.env.293 | A | 338.8 | 1705.6 | 38.1 | 190.6 | 162.8 |
| 1292.08 | IPYNPQSQGI | 10 | HIV.POL.883 | A | 1354.8 | >34046.7 | 159.6 | 56750.6 | 782.1 |
| 1292:04 | LPPLERLTI | 9 | HIV.REV.77 | A | 2022.3 | >32199.38 | 16.8 | 4230.7 | 20576.1 |
| 39.0047 | SPIETVPI | 8 | HIV.pol.174 | A | 30386.7 | >12000 | 175.7 | >17897.86 | >16666.67 |
| 1292.11 | TPPLVKLWI | 9 | HIV.POL.601 | A | >19445.44 | 189473.7 | 10.4 | >35150.7 | >18898.22 |
| 39.0087 | YPLASLRSI | 9 | HIV.gag.507 | A | 185.9 | 11303.0 | 13.2 | 1139.9 | 49.2 |
| 31.0124 | IPYCNYSKY | 9 | Lassa.gp.361 | | >9166.67 | 189.1 | >8109.31 | 311.1 | 25000.0 |
| 31.0188 | IPYCNYSKYW | 10 | Lassa.gp.361 | | >15877.13 | 2571.4 | >7627.13 | 262.0 | 50000.0 |
| 34.0186 | EPHISYPPI | 9 | MAGE2.296 | A | 253.0 | > 16099.69 | 84.6 | 1135.9 | > 1639.34 |
| 45.0135 | GPRALIETSYI | 11 | MAGE2.274 | A | 22.2 | >13606.72 | 852.7 | >24855.3 | >7715.17 |
| 45.0134 | HPRKLLMQDLI | 11 | MAGE2.241 | A | 66.1 | >41569.22 | 2211.6 | 40611.4 | 6747.6 |
| 1205,09 | VPFSHLYIL | 9 | MAGE2.170 | A | 43.8 | 939 | 31.3 | 2649.6 | 1672.5 |
| 1205.11 | VPISHLYIA | 9 | MAGE2.170 | A | 1184.5 | 2205.2 | 518.5 | 93000.0 | 1.1 |
| 45.0139 | GPRALVETSYI | 11 | MAGE3.274 | A | 11.8 | >13606.72 | 2632.1 | >24855.3 | >7715.17 |
| 34.0188 | YPPLHEWVI | 9 | MAGE3.301 | A | 124.0 | | | | |
| 45.0140 | APRMPEAAPPI | 11 | p53.63 | A | 2.3 | >13606.72 | 1917.1 | >24855.3 | >7715.17 |
| 48.0055 | FPALNKMF | 8 | p53.127 | A | 228.0 | 3168.8 | 17027.9 | 6254.2 | 3888.0 |
| 48.0234 | FPALNKMFCQL | 11 | p53.127 | A | 107.5 | 2714.2 | 5371.1 | 8024.2 | 524.7 |
| 48.0123 | FPGTRVRAI | 9 | p53.152 | A | 5.2 | >15350.45 | 694.2 | 24603.2 | 1723.0 |
| 48.0196 | FPPGSTKRAL | 10 | p53.299 | A | 7.4 | >8485.28 | 6395.4 | >10960.16 | >10000 |
| 48.0127 | FPQPKKKPI | 9 | p53.315 | A | 9.0 | >15350.45 | >15254.26 | >11195.88 | 16313.2 |
| 48.0128 | FPQPKKKPL | 9 | p53.315 | A | 25 | >15350.45 | >15254.26 | >11195.88 | >26726.12 |
| 34.0191 | PPGSTKRAI | 9 | p53.300 | A | 118.5 | >16099.69 | >9722.72 | >10960.16 | >6401.84 |
| 45.0141 | VPSQKTYQGSI | 11 | p53.97 | A | 246.6 | >13606.72 | 25229.4 | >24855.3 | >7715.17 |
| 1101.02 | IPSLALMLI | 9 | Pf.SHEBA.7 | | 190.7 | 2964.1 | | | 4714.1 |
| 39.0048 | LPYGRTNI | 8 | Pf.SSP2.126 | A | 6857.9 | >12000 | 318.5 | 45812.8 | 2308.9 |
| 39.0049 | TPFAGEPI | 8 | Pf.SSP2.524 | A | 5009.1 | >12000 | 359.7 | 28792.6 | 633.8 |
| 1372.04 | TPYAGEPAPI | 10 | Pf.SSP2.539 | A | 227.7 | 2457.2 | 14.5 | 162.2 | 170.3 |
| F001.11 | APRTLVLYL | 9 | Naturally processed | | 5.0 | | | | |
| F001.10 | APRTYVLLL | 9 | Naturally processed | | 3.9 | | | | |
| F020.14 | YAFNMKATV | 9 | Naturally processed | | 55000.0 | 72000.0 | | | 12.7 |
| F029.03 | YTNPQFNVY | 9 | Naturally processed | | >5500 | 262.1 | | | >16666.67 |
| 980.12 | APAKAAAAV | 9 | Artificial sequence | A | 8.9 | >7200 | | | |
| 953.12 | AVAKAAAAL | 9 | Artificial sequence | A | 32.6 | >12000 | | | |
| 1086.03 | FATPNFYTL | 9 | Artificial sequence | A | >13750 | 200.8 | | | 35355.3 |
| 1021.04 | FPFKYAAAY | 9 | Artificial sequence | A | 19445.4 | 22.6 | | | 8.6 |
| 114623 | FPVTFFFAF | 9 | Artificial sequence | A | 1664.4 | 2.7 | 2.8 | 2.1 | 206.7 |
| 1146.20 | FPVTMFFAA | 9 | Artificial sequence | A | >1018.52 | 450.0 | 1255.9 | 7508.7 | 14.2 |
| 1146.13 | FPVTMFFAF | 9 | Artificial sequence | A | 1467.8 | 2.1 | 2.9 | 1.7 | 182.3 |
| 1146.17 | FPVTMFFAI | 9 | Artificial sequence | A | 2961.1 | 20.7 | 2.4 | 4.6 | 1.2 |
| 1146.18 | FPVTMFPAV | 9 | Artificial sequence | A | 1897.7 | 5.8 | 2.1 | 1145 | 0.2 |
| 1146.14 | FPVTMFFAW | 9 | Artificial sequence | A | 18333.3 | 5.0 | 16.8 | 7.5 | 2265 |
| 1146.15 | FPVTMFFAY | 9 | Artificial sequence | A | 55000.0 | 3.3 | 19.3 | 2.9 | 148.4 |
| 1125.03 | LPFDYTPGF | 9 | Artificial sequence | A | 55000.0 | 19.8 | | | 158.0 |
| 1125.06 | LPGPYFLQF | 9 | Artificial sequence | A | 1672.8 | 265.1 | | | 100000.0 |

**Table 24**

| | | | HLA- B44 supertype binding affinity (IC50 nM) | | | | | |
|---|---|---|---|---|---|---|---|---|
| Peptide | Sequence | Source | B*1801 | B*4001 | B*4002 | B*4402 | B*4403 | B*4501 |
| 69.0007 | DBEEAIVAY | CMV.E1.379 | 5.9 | >10490.63 | >15694.12 | 6402 | 1175 | 12051 |
| 69.0066 | DEEEAIVAYT | CMV.E1.379 | 77 | >910.43 | 4304 | 1909 | 276 | 91 |
| 69.0086 | DEEEAIVAYTL | CMV.E1.379 | 34 | 95 | 103 | 2747 | 879 | 5140 |
| 69.0006 | EEAIVAYTL | CMV.E1.381 | 75 | 13 | 24 | 96 | 35 | .213 |
| 69.0008 | EEEAIVAYT | CMV.E1.380 | 746 | >10490.63 | 10292 | 1875 | 82 | 94 |
| 69.0065 | EEEAIVAYTL | CMV.E1.380 | 75 | 20 | 84 | 532 | 216 | 164 |
| 69.0005 | SDEEEAIVA | CMV.E1.378 | 152 | 8176 | 21319 | 2988 | 10596 | 666 |
| 69.0064 | SDEEEAIVAY | CMV.E1.378 | 38 | 3931 | >2387.61 | 558 | 395 | 4721 |
| 69.0085 | SDEEEAIVAYT | CMV.B1.378 | 175 | 2462 | 874 | 973 | 616 | 150 |
| 69.0094 | SDEEEAIVAYTL | CMV.E1.378 | 193 | 23 | 91 | 862 | 726 | 5939 |
| 69.0009 | DEVEFLGHY | EBV.BMLF.376 | 0.8 | >10490.63 | 19784 | 3158 | 96 | 3193 |
| 69.0077 | EENLLDFVRF | EBV.EBNA6.281 | 53 | 12425 | 5294 | 59 | 100 | 18779 |
| 69.0081 | EENLLDFVRM | EBV.EBNA6.281 | 824 | 5150 | 3191 | 369 | 845 | 8808 |
| 69.0034 | IEDPPFNSL | EBV.Imp2.200 | 13889 | 428 | 21900 | >9235.11 | >7424.95 | >10971.25 |
| 69.0052 | KBHVIQNAF | EBV.EBA6.335 | 123 | 2175 | 87 | 6670 | 1406 | 12324 |
| 69.0039 | SENDRLRLL | EBV.BZLF1.209 | 752 | 432 | 77 | 8303 | 455 | 5540 |
| 69.0055 | VEITPYKPT | EBV.EBNA4.657 | 6579 | 2668 | 130 | 9667 | 5432 | 1442 |
| 69.0054 | FEDLRVLSF | Flu.NP.338 | 54 | 1179 | 166 | 730 | 991 | > 13709.73 |
| 69.0053 | GEISPLPSL | Flu.NS1.158 | 5682 | 108 | 124 | 9347 | 4158 | 10242 |
| 1479.45 | AEDLNLGNLNV | HBV.POL38 | >20833.33 | 3002 | 522 | 5210 | 2292 | 239 |
| 1479.04 | AELLAACF | HBV.POL.717 | 1.9 | 390 | 55 | 7.1 | 22 | 277 |
| 1479.17 | AELLAACFA | HBV.POL.717 | 4348 | 311 | 21 | 253 | 47 | 8.4 |
| 1479.27 | DEAGPLEEEL | HBV.POL.16 | 2242 | 3117 | 16606 | >36027 | 14799 | 219 |
| 1479.35 | EDLNLGNLNV | HBV.POL.39 | 9259 | >19.75 | >27399.83 | >12713.88 | >36394.05 | 47430 |
| 1479.07 | EEELPRLA | HBV.POL.22 | >31250 | >54.39 | >978.7 | 11201 | 30535 | 517 |
| 1479.16 | EELGEEIRL | HBV.X.121 | 41667 | 275 | 295 | 2158 | 251 | 230 |
| 1479.18 | IDWKVCQRI | HBV.POL.617 | >34482.76 | 41395 | 303 | >11630.67 | >31732.28 | 448 |
| 1479.10 | LDSWWTSL | HBV.ENV.195 | >31250 | 78143 | 422 | >6146.26 | 37542 | 38333 |
| 1479.36 | LDSWWTSLNF | HBV.ENV.195 | 3559 | 381 | 534 | 3652 | 10321 | 14054 |
| 1479.41 | LDSWWTSLNFL | HBV.ENV.195 | 28571 | 4040 | 242 | 4148 | 5013 | 1567 |
| 1479.05 | LDTASALY | HBV.NUC.31 | 198 | >43041.42 | 25994 | 922 | 7684 | >39859.26 |
| 1479.11 | LDVSAAFY | HBV.POL.417 | 387 | >46169.74 | 44544 | 1534 | 11913 | 50317 |
| 1479.21 | LDYQGMLPV | HBV.ENV.261 | 24390 | >51.1 | 3.2 | >29516.17 | >30259.67 | >32837.14 |
| 1479.12 | LEEFLPRL | HBV.POL.21 | 174 | 27477 | 223 | >6146.26 | 45455 | 3287 |
| 1479.22 | LEEELPRLA | HBV.POL.21 | 18519 | >46371.3 | 2681 | >29516.17 | 41432 | 196 |
| 1479.19 | RDLLDTASA | HBV.NUC.28 | 38462 | 40661 | 206 | >11630.67 | 5077 | 382 |
| 1479.31 | RDLLDTASAL | HBV.NUC.28 | >30303.03 | 1526 | 424 | >17359.64 | >28814.23 | >35815.39 |
| 1479.40 | RDLLDTASALY | HBV.NUC.28 | >27027.03 | 54528 | 1891 | 405 | 204 | 32522 |
| 1479.25 | RDVLCLRPV | HBV.X.13 | >34482.76 | 538 | 36 | >39949.81 | 624 | 52946 |
| 1479.01 | RETVLEYL | HBV.NUC.141 | 21739 | 303 | 30 | 18054 | 9268 | 15626 |
| 1479.15 | RETVLEYLV | HBV.NUC.141 | 20833 | 219 | 59 | 25309 | 3910 | 230 |
| 1479.44 | TDNSVVLSRKY | HBV.POL.736 | >20833.33 | 269 | 12127 | 3452 | 2605 | 4269 |
| 1479.47 | VELLSFLPSDF | HBV.NUC.42 | 134 | 2956 | 283 | 227 | 333 | 8264 |
| 1479.03 | WEELGEEI | HBV.X.120 | 20000 | 305 | 370 | 9950 | 19970 | 30734 |
| 1479.29 | WEELGEEIRL | HBV.X.120 | >30303.03 | 27 | 226 | 2202 | 412 | 33947 |
| 64.0143 | ADLEVVTSTW | HCV.Entire.1656 | 863 | >10429.64 | 9853 | 724 | 221 | >12141.28 |
| 64.0060 | ADLMGYIPL | HCV.Entire.131 | >2150.54 | 778 | 10 | >15730.34 | >6343.71 | >9931.94 |
| 64.0022 | AEAALENL | HCV.Entire.749 | >2132.2 | 620 | 109 | 20896 | 390 | 529 |
| 64.0061 | AEAALENLV | HCV.Entire.749 | >2150.54 | 130 | 63 | 23179 | 250 | 40 |
| 64.0029 | AETAGARL | HCV.Entire.1341 | >2132.2 | 109 | 132 | >12635.38 | 3704 | 6484 |
| 64.0069 | AETAGARLV | HCV.Entire.1341 | >2188.18 | 7782 | 809 | >15625 | 5629 | 116 |
| 64.0145 | AETAGARLW | HCV.Entire.1341 | >2024.29 | 4338 | 244 | >11345.22 | 9910 | 110 |
| 64.0177 | AETAGARLVVL | HCV.Entire.1341 | >2066.12 | 59 | 53 | 14433 | 2732 | 577 |
| 64.0030 | CDELAAKL | HCV.Entire.1405 | 219 | 7178 | 5587 | 1132 | 10185 | >10913.04 |
| 64.0136 | CECYDAGCAW | HCV.Entire.1523 | 802 | >10429.64 | 9798 | 948 | 317 | 16082 |
| 64.0168 | CECYDAGCAWY | HCV.Entire.1523 | 93 | 12623 | 20016 | 479 | 275 | 8594 |
| 64.0070 | CEKMALYDV | HCV.Entire.2623 | 2421 | 13063 | 644 | 8537 | 9291 | 109 |
| 64.0146 | CEKMALYDVV | HCV.Entire.2623 | 5587 | 9400 | 111 | 9211 | 10913 | 92 |
| 64.0063 | EDLVNLLPA | HCV.Entire.1882 | >2188.18 | >12687.98 | 1318 | >15625 | >6329.11 | 500 |
| 64.0031 | GDLCGSVF | HCV.Entire.278 | 1592 | >11264.46 | 81 | 9818 | 9338 | >10913.04 |
| 64.0187 | GEGAVQWMNRL | HCV.Entire.1914 | >2066.12 | 402 | 422 | >14613.78 | 15850 | >12443.44 |
| 64.0076 | GEIPFYGKA | HCV.Entire.1376 | >2188.18 | 1196 | 68 | 21807 | 6440 | 46 |
| 64.0151 | GEIPFYGKAI | HCV.Entire.1376 | >2227.17 | 78 | 51 | 7761 | 91 | 6057 |
| 69.0038 | GQIVGGVYL | HCV.cAg.27 | 14286 | 15 | 37 | 681 | 1257 | 9032 |
| 64.0181 | IEANLLWRQEM | HCV.Entire.2236 | 213 | 76 | 69 | 1246 | 5647 | 15152 |
| 1472.01 | LEDRDRSEL | HCV.NS1/E2.6S4 | >8621.97 | 129 | 184 | >34204.99 | 46064 | >40390.98 |
| 64.0034 | LEVVTSTW | HCV.Entire.1658 | 94 | 7549 | 913 | 742 | 2927 | >10913.04 |
| 64.0074 | LEVVTSTWV | HCV.Entire.1658 | 2188 | 634 | 144 | 11345 | 10496 | 7890 |
| 64.0147 | LEVVTSTWVL | HCV.Entire.1658 | 1006 | 8.8 | 8.7 | 3338 | 2304 | 12472 |
| 64.0183 | LEVVTSTWVLV | HCV.Entire.1658 | 1101 | 128 | 99 | 4037 | 1634 | 874 |
| 64.0024 | SELSPLLL | HCV.Entire.665 | 2336 | 519 | 110 | 11094 | 553 | 7011 |
| 64.0077 | TEAMTRYSA | HCV.Entire.2793 | 1160 | 9053 | 145 | 1686 | 5832 | 60 |
| 64.0078 | VDFSLDPTF | HCV.Entire.1467 | 2304 | >11338.36 | 145 | 16241 | 9565 | >1176622 |
| 64.0188 | VDFSLDPTFTI | HCV.Entire.1467 | >2066.12 | >10037.87 | 68 | 27778 | 17350 | >12443.44 |
| 64.0040 | VDYPYRLW | HCV.Entire.614 | >2136.75 | 34027 | 15625 | >11146.5 | 417 | >10913.04 |
| 64.0018 | AEHLKTAV | HIV.POL.921 | 1289 | 6271 | 23 | 1019 | 1395 | 1267 |
| 64.0126 | AEHLKTAVQM | HIV.POL921 | >2096.44 | 96 | 78 | 7384 | 1008 | 943 |
| 64.0165 | AEHLKTAVQMA | HIV.POL.921 | >1945.53 | 8411 | 982 | >8951.41 | 7514 | 122 |
| 64.0162 | AELELAENREI | HIV.POL.488 | >1945.53 | 706 | 561 | 7675 | 90 | 98 |
| 69.0078 | AENLWVTVYY | HIV.gp120.1 | 79 | 277 | 89 | 43 | 13 | 275 |
| 64.0048 | AETFYVDGA | HIV.POL.629 | >2136.75 | 11491 | 1131 | >11146.5 | 5601 | 43 |
| 69.0062 | AETFYVDGA | HIV.RT.592 | >9090.91 | 3350 | 1406 | 2587 | 3493 | 114 |
| 64.0016 | AETGQETA | HIV.POL.843 | >2257.34 | 666 | 546 | >11608.62 | 231 | 1064 |
| 64.0124 | AETGQETAYF | HIV.POL.843 | >45045 | 6489 | 1287 | 7559 | 387 | 8634 |
| 64.0045 | EEKAFSPEV | HIV.GAG.181 | 2083 | 14512 | 112 | 6869 | 7597 | 57 |
| 64.0113 | EEKAFSPEVI | HIV.GAG.181 | >1992.03 | 14811 | 4130 | 8537 | 7524 | 253 |
| 64.0115 | EEMMTACQGV | HIV.GAG.368 | >1992.03 | 9474 | 6155 | 5613 | 2066 | 125 |
| 64.0053 | GDAYFSVPL | HIV.POL.301 | 2899 | 1430 | 7.5 | 16949 | >6343.71 | >15983.85 |
| 64.0014 | GEAMHGQV | HIV.POL.799 | >2331 | 836 | 200 | >11844.33 | >9259.26 | 8943 |
| 64.0011 | IEAEVIPA | HIV.POL.836 | 41 | 3166 | 36 | 654 | 8209 | 389 |
| 64.0001 | IEAQQHLL | HIV.ENV.645 | 58 | 31 | 28 | 6034 | 1252 | >12770 |
| 64.0010 | IETVPVKL | HIV.POL.191 | >2013.01 | 34 | 79 | 1275 | 3205 | >12770 |
| 64.0003 | KDCTERQA | HIV.GAG.457 | >2012.07 | >1762.29 | 553 | >10401.19 | 41 | 42373 |
| 64.0133 | KEPPFLWMGY | HIV.POL.412 | >2024.29 | >9386.68 | 883 | 3045 | 10.0 | 14286 |
| 64.0119 | LELAENREIL | HIV.POL.490 | >2096.44 | 9.3 | 233 | >12567.32 | 11555 | 17915 |
| 64.0017 | NEQVDKLV | HIV.POL.737 | 233 | >3032.82 | 1297 | >11608.62 | >6699.15 | 15318 |
| 64.0009 | NETPGIRY | HIV.POL326 | 19 | >1762.29 | 31250 | 12080 | 1911 | >12770 |
| 64.0117 | NETPGIRYQY | HIV.POL.326 | 58 | >12044.75 | >11524.1 | 57 | 50 | 11853 |
| 64.0012 | QDFWEVQL | HIV.POL.274 | >2012.07 | 1080 | 165 | 1534 | >9369.68 | >28690.23 |
| 69.0010 | RDYVDRFYK | HIV.Gag.292 | 6250 | >10972.49 | >2119.25 | 339 | >7635.83 | >21942.68 |
| 64.0046 | REPRGSDIA | HIV.GAG.250 | >2136.75 | >12747.77 | 863 | >11146.5 | >6970.85 | 402 |
| 64.0002 | SELYKYKV | HIV.ENV.559 | >2012.07 | 284 | 135 | 4192 | 4808 | 8845 |
| 64.0044 | SELYKYKVV | HIV.ENV.559 | 3436 | 12904 | 97 | 10903 | 4898 | 988 |
| 64.0128 | TDSQYALGII | HIV.POL.689 | 308 | 6306 | 442 | 7830 | 11506 | 1218 |
| 64.0013 | TEEKIKAL | HIV.POL.214 | 1339 | 356 | 769 | 723 | >11506.28 | >12770 |
| 64.0005 | TERQANFL | HIV.GAG.460 | 56 | 118 | 14 | 3319 | 266 | 10922 |
| 64.0052 | WEFVNTPPL | HIV.POL.606 | 1.9 | 1.4 | 5.0 | 55 | 305 | 5848 |
| 64.0121 | WEFVNTPPLV | HIV.POL.606 | 272 | 60 | 34 | 8304 | 9683 | 7237 |
| 64.0163 | WEVQLGIPHPA | HIV.POL.277 | 14 | 697 | 73 | 5872 | 5972 | 532 |
| 64.0019 | YELHPDKW | HIV.POL421 | 418 | 512 | 504 | 1547 | 159 | >12767.93 |
| 64.0129 | YELHPDKWTV | HIV.POL.421 | 891 | 123 | 9.4 | 4411 | 11022 | 7453 |
| 78.0225 | AEDLRTLQQL | HPV45.E7.81 | >24738.53 | 95 | 107 | 3905 | 297 | 175 |
| 78.0215 | AEPDTSNYNI | HPV31.E7.45 | >24738.53 | 1619 | 2024 | 1243 | 343 | 1398 |
| 78.0417 | AEPQRHTML | HPV18.E7.54 | 5.1 | 1781 | 22 | 51 | 20 | 21310 |
| 78.0440 | AEQATSNYY | HPV52.E7.47 | 12070 | 326 | 1486 | 3006 | 177 | 1608 |
| 78.0228 | AEQATSIVYYI | HPV52.E7.47 | 10638 | 291 | 51 | 92 | 104 | 15.3 |
| 78.0210 | AERPRKLHEL | HPV31.E6.6 | 14213 | 23648 | 273 | 14997 | 21440 | 2945 |
| 78.0418 | CEARIELVV | HPV18.E7.68 | 0.1 | 132 | 1.4 | 14 | 16 | 2162 |
| 78.0446 | CECKFVVQL | HPV56.E7.66 | 660 | 6966 | 401 | 303 | 21 | 34 |
| 78.0420 | DELRLNCVY | HPV31.E6.24 | >29424.49 | >26772.48 | 29013 | 9304 | 25837 | 47 |
| 78.0217 | EEKPRTLHDL | HPV33.E6.6 | >24738.53 | 19236 | 20016 | 11593 | 24273 | 470 |
| 78.0432 | EENDEADGV | HPV45.E7.37 | 5530 | 1429 | 422 | 13466 | 17582 | 4525 |
| 78.0416 | EENDEIDGV | HPV18.E7.36 | 1848 | 172 | 674 | 33228 | 17141 | 42422 |
| 78.0434 | EESVHEIRL | HPV52.E6.20 | 134 | 8354 | 49 | 1828 | 18444 | 44717 |
| 78.0239 | FEDPTRRPY | HPV18.E6.4 | 13 | 592 | 40 | 147 | 205 | 450 |
| 78.0206 | FEFAFKDLFV | HPV18.E6.45 | 2.0 | 100 | 2.0 | 956 | 174 | 3006 |
| 78.0419 | HELSSALEI | HPV31.E6.13 | 16737 | 187 | 256 | 2022 | 20691 | 25882 |
| 78.0232 | KEDLRVVQQL | HPV56.E7.80 | >7860.62 | 502 | 44 | 12913 | 2608 | 3447 |
| 78.0435 | KELQRRBVY | HPV52.E6.35 | 19090 | 50 | 44 | 17557 | >32308.25 | 29973 |
| 78.0442 | KELTRAEVY | HPV56.E6.38 | 7699 | 2122 | 275 | 25736 | 15174 | >36998.68 |
| 78.0202 | LECVYCKQQL | HPV16.E6.35 | 6984 | 175 | 135 | 11658 | 1472 | 41639 |
| 78.0436 | LEERVKKPL | HPV52.E6.88 | 24398 | 3.0 | 5.4 | 18172 | 1347 | 4031 |
| 78.0226 | LEESVHEIRL | HPV52.E6.19 | 12371 | 123 | 1679 | 16032 | 28988 | 18357 |
| 78.0230 | LEIPLIDLRL | HPV56.E6.22 | 4485 | 42 | 7.3 | 8457 | 206 | 30242 |
| 78.0211 | LEIPYDELRL | HPV31.E6.19 | 11987 | 1495 | 303 | 27643 | 2325 | 31587 |
| 78.0421 | LEKLTNKGI | HPV31.E6.88 | 37139 | 5442 | 291 | >20466.26 | >33599.1 | 20543 |
| 78.0208 | LEKLTNTGLY | HPV18.E6.90 | 861 | 162 | 6702 | 622 | 2639 | 31186 |
| 78.0444 | LELTPQTEI | HPV56.E7.13 | 3998 | 6461 | 20 | 2540 | 2716 | 243 |
| 78.0447 | LEQTLKKCL | HPV58.E6.88 | 53 | 1411 | 16 | 2674 | 200 | 313 |
| 78.0424 | LEQTVKKPL | HPV33.E6.88 | 171 | 1110 | 164 | 4453 | 249 | 236 |
| 78.0428 | LERTEVYQF | HPV45.E6.39 | 3675 | 15300 | 7845 | 1772 | 222 | >35147.94 |
| 78.0443 | LESITKKQL | HPV56.E6.91 | 74 | 122 | 6.1 | 242 | 55 | 176 |
| 78.0234 | LETSVHEIEL | HPV58.E6.19 | 627 | 25 | 38 | 11547 | 2247 | 6385 |
| 78.0218 | LETTIHNVIEL | HPV33.E6.19 | 4384 | 129 | 166 | 19310 | 16586 | 5337 |
| 78.0425 | NEILIRCII | HPV33.E6.97 | 1830 | 154 | 34 | 2071 | 209 | 3646 |
| 78.0448 | NEILIRCII | HPV58.E6.97 | 88 | 34247 | 22716 | 465 | 1332 | >37221.52 |
| 78.0414 | NEKRRFHNI | HPV18.E6.122 | 212 | 150 | 31 | 2387 | 2336 | 2044 |
| 78.0431 | NELDPVDLL | HPV45.E7.19 | 25 | 384 | 41 | 2094 | 233 | 451 |
| 78.0423 | PEATDLHCY | HPV31.E7.17 | >29424.49 | 237 | 2334 | 21016 | 25172 | 24308 |
| 78.0450 | PEPTDLFCY | HPV58.E7.17 | 5453 | 259 | 201 | 1723 | 3110 | 37844 |
| 78.0426 | QEKKRHVDL | HPV33.E6.113 | 2.4 | 8491 | 37113 | 337 | 170 | 14157 |
| 78.0449 | QEKKRHVDL | HPV58.E6.113 | >8621.97 | 1051 | 1869 | 2361 | 474 | >37221.52 |
| 78.0216 | QELLMGSFGI | HPV31.E7.80 | 1699 | 285 | 180 | 204 | 153 | 259 |
| 78.0200 | QERPRKLPQL | HPV16.E6.13 | >23870.5 | 15331 | 304 | 39234 | 28466 | 2241 |
| 78.0229 | QERPRSLHHL | HPV56.E6.9 | >7860.62 | 5752 | 494 | 6089 | 13937 | 2590 |
| 78.0207 | RELRHYSDSV | HPV18.E6.76 | 7363 | 1196 | 29 | 68524 | 20650 | 2086 |
| 78.0221 | RELRYYSNSV | HPV45.E6.76 | >8264.75 | 895 | 19 | 16950 | 17558 | 410 |
| 78.0430 | RETLQEIVL | HPV45.E7.5 | 2611 | 2025 | 55 | 388 | 255 | 32285 |
| 78.0213 | SEFRWYRYSV | HPV31.E6.74 | 9.4 | 274 | 2.8 | 197 | 21 | 35 |
| 78.0437 | SEITIRCII | HPV52.E6.97 | 3183 | 456 | 409 | 6602 | 1721 | 3763 |
| 78.0441 | SEVLEIPLI | HPV56.E6.19 | 446 | 28851 | 6049 | 238 | 262 | 32935 |
| 78.0203 | SEYRHYCYSL | HPV16.E6.81 | 133 | 16 | 13 | 1498 | 175 | 202 |
| 78.0235 | SEYRHYNYSL | HPV58.E6.74 | 64 | 9.4 | 3.6 | 1124 | 185 | 181 |
| 78.0219 | SEYRHYNYSV | HPV33.E6.74 | 132 | 1312 | 17 | 15814 | 2842 | 30 |
| 78.0227 | SEYRHYQYSL | HPV52.E6.74 | 97 | 15 | 14 | 228 | 289 | 290 |
| 78.0205 | TELNTSLQDI | HPV18.E6.19 | 12897 | 180 | 1691 | 5408 | 1129 | 11464 |
| 78.0212 | TETEVLDFAF | HPV31.E6.38 | 25 | 2114 | 244 | 192 | 41 | 41087 |
| 70.0055 | AEDLYGRLEI | Pf.LSA1.1649 | >1532.81 | 1095 | 940 | 447 | 366 | 913 |
| 64.0089 | DEFKPIVQY | Pf.LSA1.1783 | 1.2 | >11338.36 | >14084.51 | 9.2 | 324 | >13736.27 |
| 70.0030 | DEIKYREEV | Pf.SSP2.34 | 250 | >2015.33 | 7984 | 11200 | >7003.42 | 3856 |
| 70.0057 | DELSEDITKY | Pf.LSA1.1896 | 131 | >1553.5 | >1992.04 | 5254 | 5756 | >9479.14 |
| 70.0044 | DENANANNAV | Pf.CSPZ.333 | 120 | >1811.7 | 2007 | >8127.24 | >7997.92 | 1106 |
| 70.0031 | DEVDLYLLM | Pf.SSP2.45 | 5.6 | 1555 | 603 | 787 | 68 | >10605.18 |
| 70.0064 | EENIGIYKEL | Pf.LSA1.1797 | >1728.24 | 1704 | 1776 | 31 | 460 | 367 |
| 70.0080 | EEVCNDEVDL | Pf.SSP2.40 | 2788 | 434 | 1870 | 5723 | 8026 | 10252 |
| 70.0081 | GEPAPFDETL | Pf.SSP2.531 | >10869.57 | 7.6 | 13 | 10558 | 6848 | >8615.59 |
| 70.0050 | GEPLIDVHDL | Pf.PfEXP-1.44 | | 93 | | | | |
| 70.0069 | KEGKLIEHII | Pf.ISA1.119 | 2086 | 631 | 124 | 360 | 3794 | 11531 |
| 70.0070 | KEKEKFIKSL | Pf.I.SA1.1872 | 3732 | 1039 | 190 | 7933 | >6331.06 | >8900.42 |
| 64.0101 | KEKFIKSLF | Pf.LSA1.1874 | >1968.5 | 7156 | 65 | 23810 | 814 | 11702 |
| 70.0071 | LDEFKPIVQY | Pf.L.SA1.1782 | 2019 | 96 | 111 | 7022 | >6331.06 | >8747.17 |
| 70.0072 | LEEKAAKETL | Pf.LSA1.149 | 156 | >1491.97 | >3937.13 | >10838.73 | 6799 | >8747.17 |
| 64.0103 | LETVNISDV | Pf.LSA1.1744 | >1968.5 | 7497 | 122 | >16055.05 | >6892.23 | 689 |
| 70.0034 | NDEVDLYLL | Pf.SSP2.44 | 181 | 27419 | 5292 | 10590 | 3782 | >10343.38 |
| 70.0082 | NDEVDLYLLM | Pf.SSP2.44 | 7.3 | 1141 | 153 | 1616 | 296 | 16119 |
| 70.0048 | NDIEKKICKM | Pf.CSPZ.401 | 1220 | 1947 | 937 | 4833 | 11254 | 429 |
| 70.0046 | NELNYDNAGI | Pf.CSPZ.36 | 1486 | 2194 | 13820 | 80 | 58 | 513 |
| 64.0106 | QDEENIGIY | Pf.LSA1.1795 | 23 | >12506.23 | >11940.49 | 2088 | 10358 | 9016 |
| 70.0083 | QDSLKESRKL | Pf.SSP2.169 | >1357.89 | 12 | 5.0 | 330 | 386 | 105 |
| 70.0014 | QERLANEKL | Pf.LSA1.1527 | 814 | 764 | 368 | 2882 | 766 | 7733 |
| 70.0015 | QERRAKEKL | Pf.LSA1.1578 | >1901.31 | 1729 | 24 | 4159 | 4151 | 11761 |
| 70.0039 | RENANQLW | Pf.SSP2.150 | 244 | 92 | 12 | 564 | 388 | 42 |
| 70.0019 | SDLEQDRLA | Pf.LSA1.1387 | 235 | >2000.2 | 4867 | >20582.18 | 4864 | >10316.53 |
| 70.0024 | SEELSEEKI | Pf.LSA1.1827 | 165 | 89 | 1486 | 545 | 126 | 4205 |
| 70.0041 | SEKEVPSDV | Pf.SSP2.371 | 153 | >2015.33 | 122 | 8130 | >7003.42 | 777 |
| 70.0025 | SENERGYYI | Pf.LSA1.1665 | 97 | 1983 | 133 | 114 | 74 | 378 |
| 64.0112 | SESNGEPNA | Pf.PfEXP-1.127 | >2247.19 | >12506.23 | 8207 | >8805.03 | >9632.22 | 121 |
| 70.0043 | WDEWSPCSV | Pf.SSP2.247 | 155 | >2015.67 | 608 | 15002 | 6735 | >10827.8 |
| 70.0026 | YEDEISAEY | Pf.LSA1.1760 | 11 | 2082 | 7805 | 3574 | 459 | >10402.05 |
| 70.0027 | YEKTKDNNF | Pf.LSA1.1841 | 2318 | >4032.11 | 152 | 958 | 11674 | 6691 |
| 64.0086 | YENDIEKKI | Pf.CSPZ.399 | 2278 | 933 | 133 | 10938 | 574 | 9481 |
| 69.0037 | SESPIWVL | Rat.45 | 144 | 16 | 72 | 505 | 619 | 881 |
| 69.0063 | AEEAAGIGI | | >9090.91 | 23 | 565 | 492 | 140 | 67 |
| 69.0084 | AEEAAGIGIL | | 12195 | 17 | 36 | 3085 | 926 | 73 |
| 69.0093 | AEEAAGIGILT | | >5780.35 | 418 | 524 | 4732 | 686 | 175 |
| 69.0025 | AEIGEVIVL | | 9615 | 5.6 | 3.6 | 92 | 28 | 91 |
| 69.0070 | AEIGEVIVLW | | 1026 | 38 | 18 | 369 | 17 | 97 |
| 69.0090 | AEIGEVIVLWL | | 10530 | 13 | 39 | 3303 | 7.5 | 336 |
| 69.0027 | AEIPGEIAL | | 9524 | 3.3 | 5.7 | 1114 | 150 | 1086 |
| 69.0075 | AEVDKVTGRF | | 10638 | 1891 | 821 | 416 | 4152 | 2005 |
| 69.0051 | DEAPAVISY | | 0.9 | 2977 | 17056 | 508 | 70 | 11189 |
| 69.0049 | DEFIGVAIY | | 0.7 | 14509 | 1100 | 2677 | 14 | 16200 |
| 69.0050 | DENPLVKQY | | 9.2 | 989 | 9799 | 312 | 124 | >12676.78 |
| 69.0045 | DEVGIVTKY | | 4.6 | 2597 | >15074.52 | 651 | 45 | >12676.78 |
| 69.0076 | DEVPDLERKY | | 101 | 4688 | 186 | 69 | 286 | >14076.63 |
| 69.0041 | EEFQFIKKA | | 100 | 12484 | 37 | 129 | 60 | 73 |
| 69.0022 | EEFYVDLER | | 12658 | 5173 | 14825 | 10360 | 367 | 6137 |
| 69.0058 | EEKLIVVAF | | 1.0 | 863 | 184 | 68 | 52 | 806 |
| 69.0059 | EEKLIVVLF | | 4.8 | 1430 | 208 | 44 | 17 | 709 |
| 69.0029 | FEIPLLDVA | | 115 | 704 | 7.8 | 3058 | 11766 | 61 |
| 69.0048 | FEPTVVKKY | | 1294 | >1905.51 | 882 | 678 | 63 | >12676.78 |
| 69.0040 | GEFGGFGSV | | 19608 | 1438 | 241 | 4580 | 1222 | 7707 |
| 69.0020 | GEFIPGNDL | | 9524 | 20 | 146 | 10874 | 2299 | 14142 |
| 69.0018 | GEFPGKIFL | | 8264 | 30 | 107 | 11014 | 457 | >14176.58 |
| 69.0069 | GEFPGKIFLY | | 85 | 15 | 14 | 44 | 6.4 | 6986 |
| 69.0089 | GEFPGKIFLYA | | 762 | 184 | 16 | 3793 | 14 | 1926 |
| 69.0017 | GEFPNKNIL | | 20833 | 14 | 25 | 5117 | 872 | 31325 |
| 69.0068 | GEFPNKNILY | | 428 | 280 | 149 | 458 | 70 | >12241.78 |
| 69.0088 | GEFPNKNILYA | | 7463 | 457 | 48 | 14551 | 971 | 2442 |
| 69.0004 | GEFSITYK | | 10870 | 646 | 176 | 4102 | 782 | 11469 |
| 69.0002 | GEFVDLYV | | 8696 | 362 | 1130 | 8873 | 14192 | 45707 |
| 69.0042 | GEHGLIIRV | | >11363.64 | 39 | 3.2 | 3215 | 174 | 375 |
| 69.0028 | GEILDVFDA | | 15625 | 419 | 15 | 12689 | 3939 | 56 |
| 69.0026 | GEVIVLWLW | | 1230 | 652 | 9.0 | 178 | 26 | 5584 |
| 69.0073 | HEATLRCWAL | | 106 | 16 | 12 | 1291 | 5512 | 8909 |
| 69.0092 | IEVDPDTKEML | | >9009.01 | 409 | 4054 | >8202.43 | 9586 | >21808.92 |
| 69.0031 | KESTLHLVL | | 12658 | 136 | 119 | 2251 | 1895 | 16607 |
| 69.0091 | KEVGVDVALYA | | 12658 | 97 | 56 | >8681.62 | 419 | 716 |
| 69.0057 | MEVDPIGHL | | 434 | 24 | 23 | 850 | 188 | 1105 |
| 69.0079 | MEVDPIGHLY | | 6.4 | 599 | 555 | 6.0 | 23 | 256 |
| 69.0033 | REIAQDFKT | | >12658.23 | 67 | 119 | 7115 | 1408 | 1562 |
| 69.0072 | REIAQDFKTD | | 13158 | 45 | 1497 | 8272 | 11532 | 2012 |
| 69.0003 | REIIINAV | | 189 | 346 | 14 | 8136 | 7702 | 451 |
| 69.0043 | REMIPFADI | | 8333 | 4.9 | 8.5 | 176 | 129 | 8.7 |
| 69.0015 | REPVTKAEM | | >11363.64 | 167 | 138 | 8436 | 6021 | 24162 |
| 69.0044 | SEIDTVAKY | | 74 | 861 | 5709 | 367 | 338 | 1948 |
| 69.0056 | SEIWRDIDF | | 47 | 30 | 44 | 205 | 10.1 | 197 |
| 69.0016 | TEFPKERHL | | 8475 | 1248 | 100 | 10793 | 599 | 22219 |
| 69.0019 | WEFLQPILL | | 7.5 | 0.8 | 1.0 | 176 | 31 | 5200 |
| 69.0036 | YEIHDGMNL | | 12821 | 9.3 | 14 | 6897 | 1711 | 31404 |

**TABLE 25**

| Sequence | Source | Motif | % Conservation |
|---|---|---|---|
| FPDWQNYTPGPGIRY | HIV.nef.200 | DRsuper | 23 |
| FPDWQNYTPGPGTRF | HIV.nef.200 | DRsuper | 20 |
| GFPVRPQVPLRPMTY | HIV.nef.93 | DRsuper | 56 |
| GGKWSKSSNGWPAI | HIV.nef.2 | DRsuper | 8 |
| GPGIRYPLTFGWCFK | HIV.nef.210 | DRsuper | 9 |
| HGAITSSNTAATNAD | HIV.nef.61 | DRsuper | 16 |
| ILDLWVYHTQGFFPD | HN.nef.186 | DRsuper | 20 |
| ILDLWVYHTQGYFPD | HIV.nef.186 | DRsuper | 33 |
| NNCLLHPMSQHGMDD | HIV.nef.254 | DRsuper | 9 |
| NNSLLHPICQHGMED | HIV.nef.254 | DRsuper | 6 |
| RFPLTFGWCFKLVPV | HIV.nef.216 | DRsuper | 19 |
| RPQVPLRPMTYKGAF | HIV.nef.98 | DRsuper | 11 |
| RQDILDLWVYHTQGY | HIV.nef.182 | DRsuper | 14 |
| RQDILDLWVYNTQGY | HIV.nef.182 | DRsuper | 8 |
| RQEILDLWVYHTQGF | HIV.nef.182 | DRsuper | 16 |
| RQEILDLWVYHTQGY | HIV.nef.182 | DRsuper | 19 |
| RYPLTFGWCFKLVPV | HIV.nef.216 | DRsuper | 24 |
| SRDLEKHGAITSSNT | HIV.nef:50 | DRsuper | 20 |
| TFGWCFKLVPVDPRE | HIV.nef.222 | DRsuper | 11 |
| AEPVPLQLPPLERLT | HIV.rev.72 | DRsuper | 16 |
| IKFLYQSNPPPSPEG | HIV.rev.21 | DRsuper | 6 |
| LKAVRIIKILYQSNP | HIV.rev.13 | DRsuper | 9 |
| PVPLQLPPLERLTLD | HIV.rev.74 | DRsuper | 20 |
| LEPWKHPGSQPKTAC | HIV.tat.11 | DRsuper | 17 |
| LEPWNHPGSQPKTAC | HIV.tat.11 | DRsuper | 17 |
| LEPWNHPGSQPRTPC | HIV.tat.11 | DRsuper | 5 |
| LEPWNHPGSQPTTAC | HIV.tat.11 | DRsuper | 11 |
| NNCYCKKCCFHCQVC | HIV.tat.26 | DRsuper | 6 |
| QLCFLKKGLGISYGR | HIV.tat.38 | DRsupet | 5 |
| QVCFITKGLGISYGR | HlV.tat.38 | DRsuper | 11 |
| QVCFLNKGLGISYGR | HIV.tat.38 | DRsuper | 6 |
| TNCYCKKCCYHCQVC | HIV.tat.26 | DRsuper | 3 |
| LDGLIYSKKRQEILD | HIV.nef.171 | DR3 | 17 |
| LSFFLKEKGGLDGLI | HIV.nef.114 | DR3 | 22 |
| LSHFLKBKGGLBGLI | HIV.nef.114 | DR3 | 23 |
| QDAVSQDLDKCGAAA | HIV.nef.51 | DR3 | 2 |
| TQGFFPDWQNYTPGP | HIV.nef.195 | DR3 | 27 |
| TQGYFPDWQNYTPGP | HIV.nef.195 | DR3 | 52 |
| TRQARKNRRRRWRAR | HIV.rev.38 | DR3 | 20 |
| TRQARRNRRRRWRAR | HIV.rev.38 | DR3 | 28 |
| TRQARRNRRRRWRER | HIV.rev.38 | DR3 | 19 |
| KEKVERETETDPAVQ | HIV.tat.95 | DR3 | 2 |

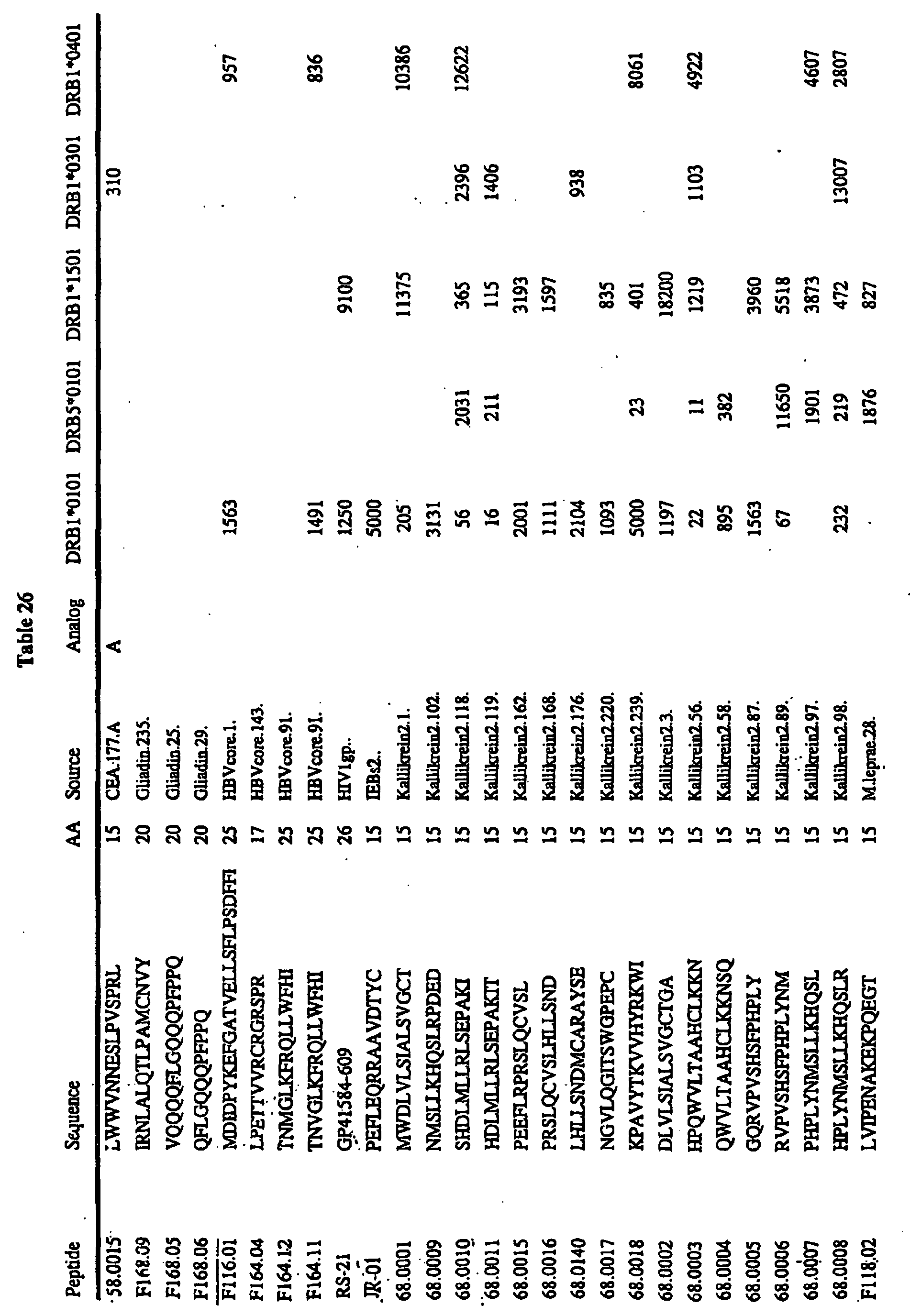

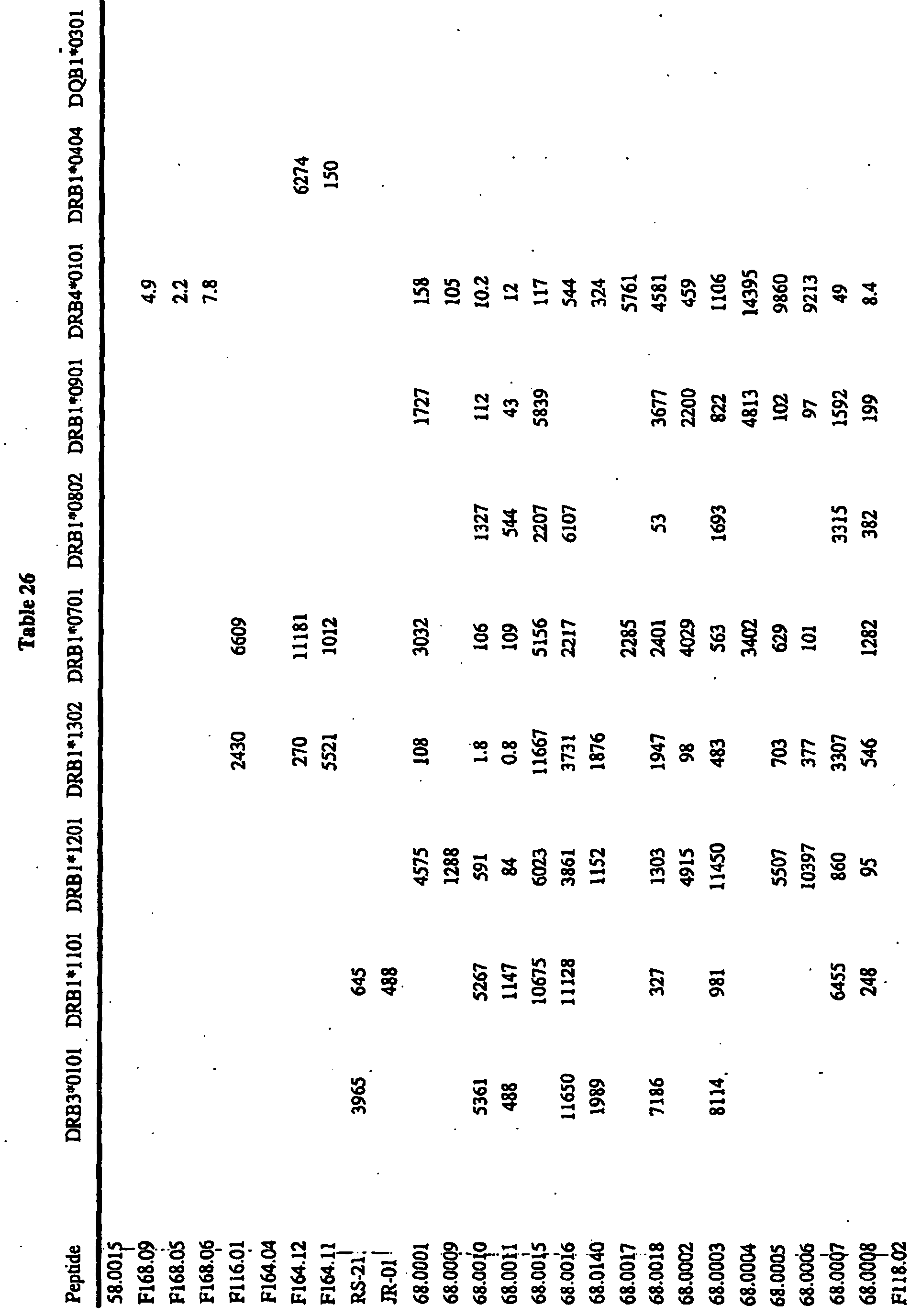

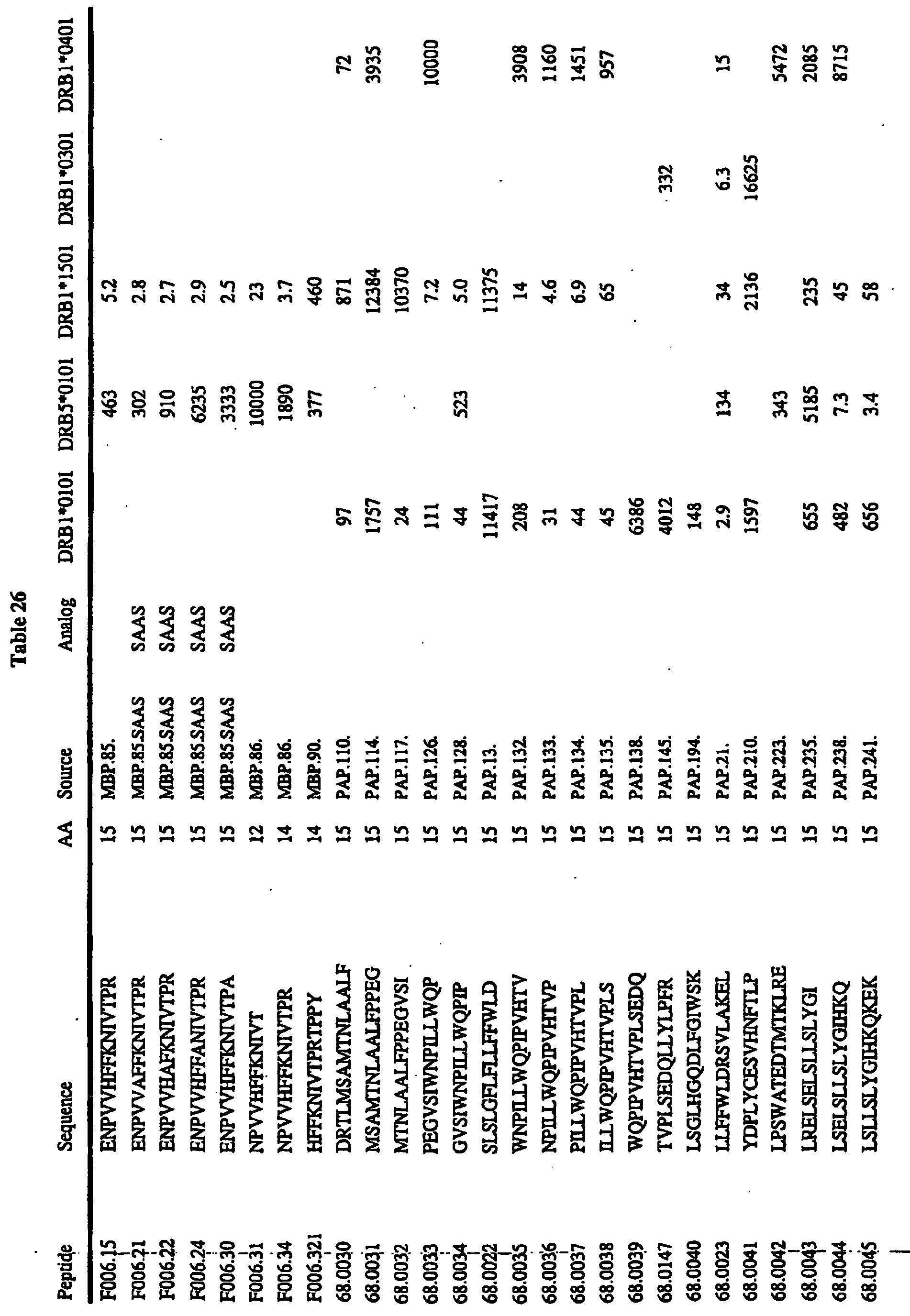

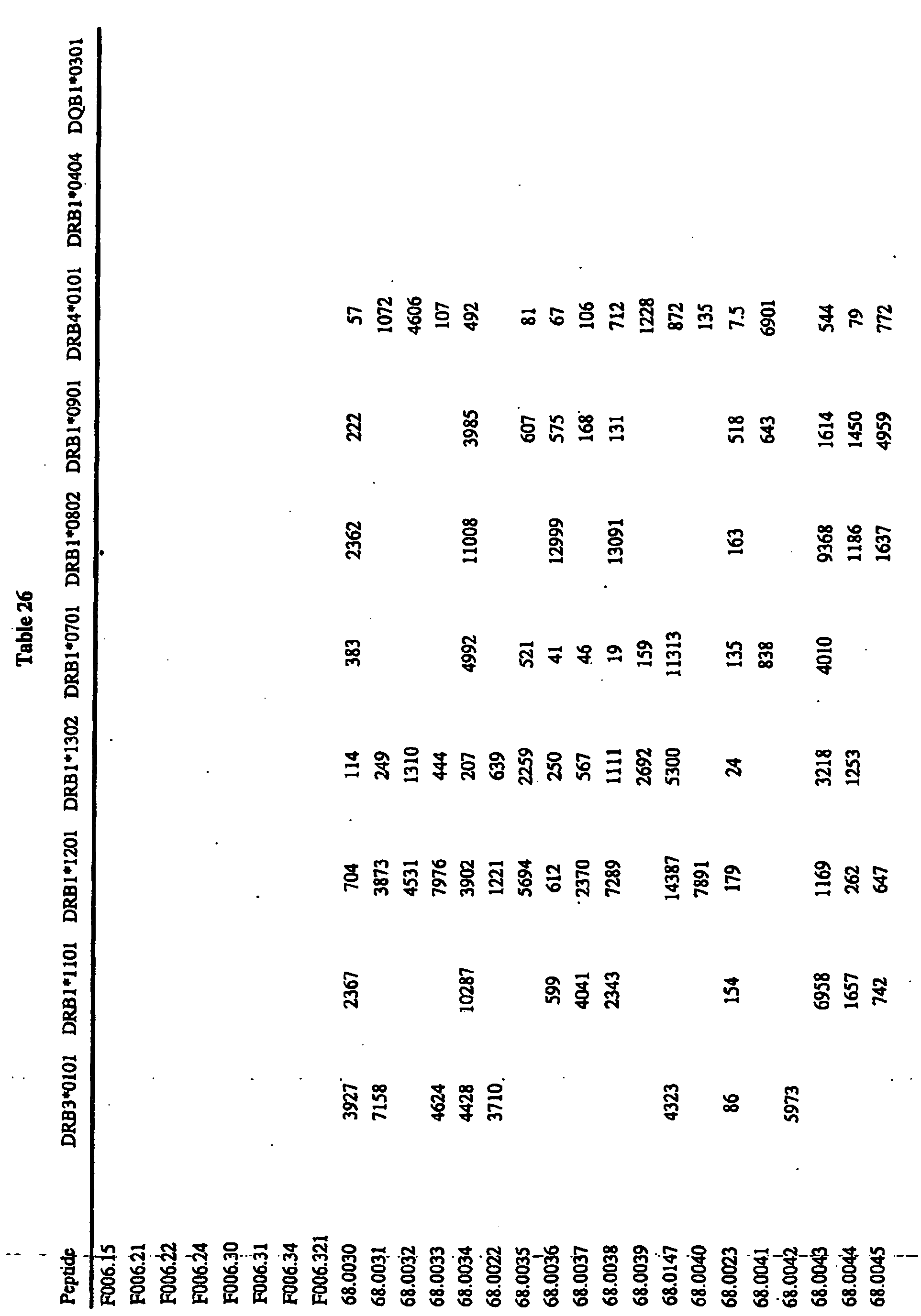

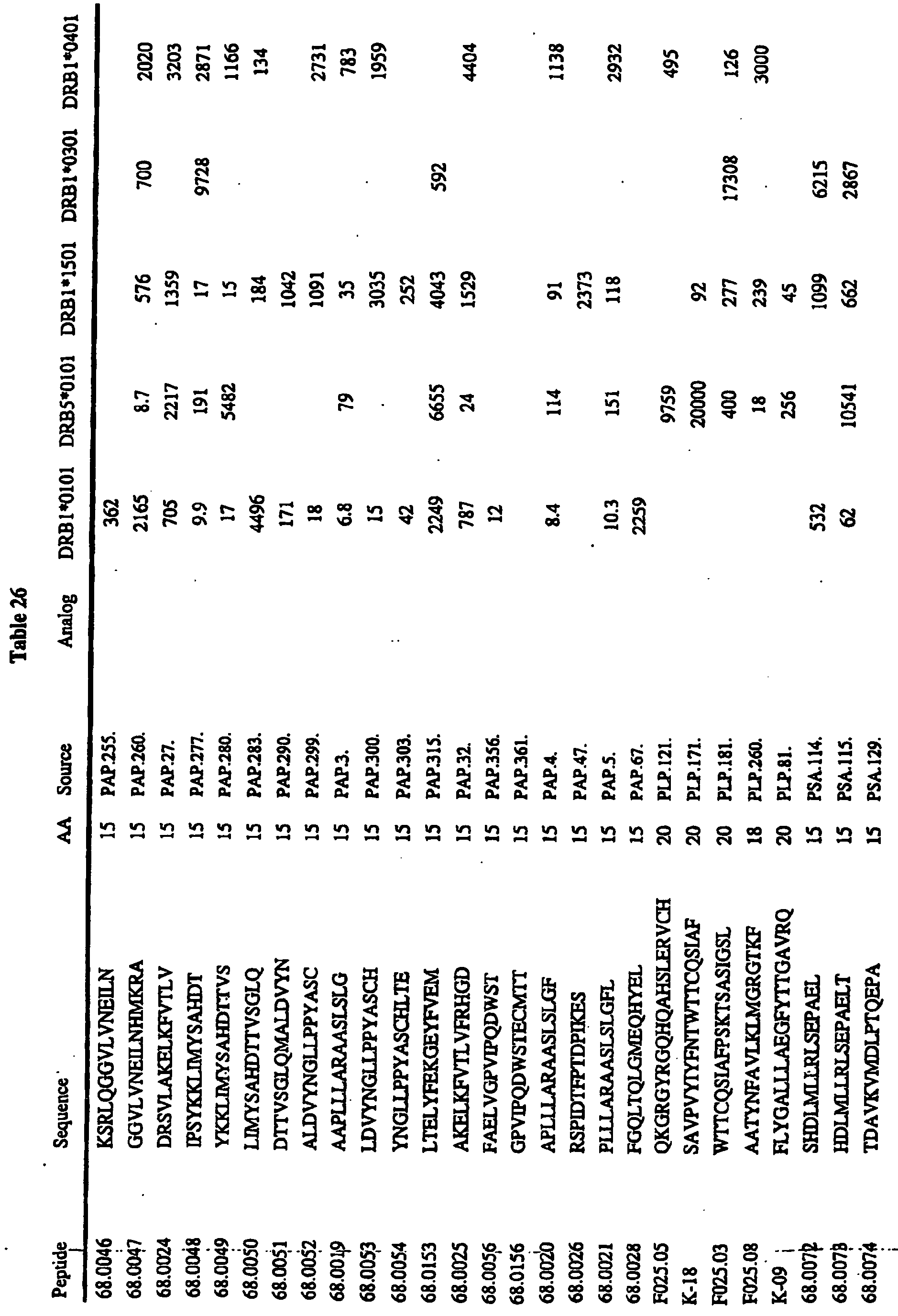

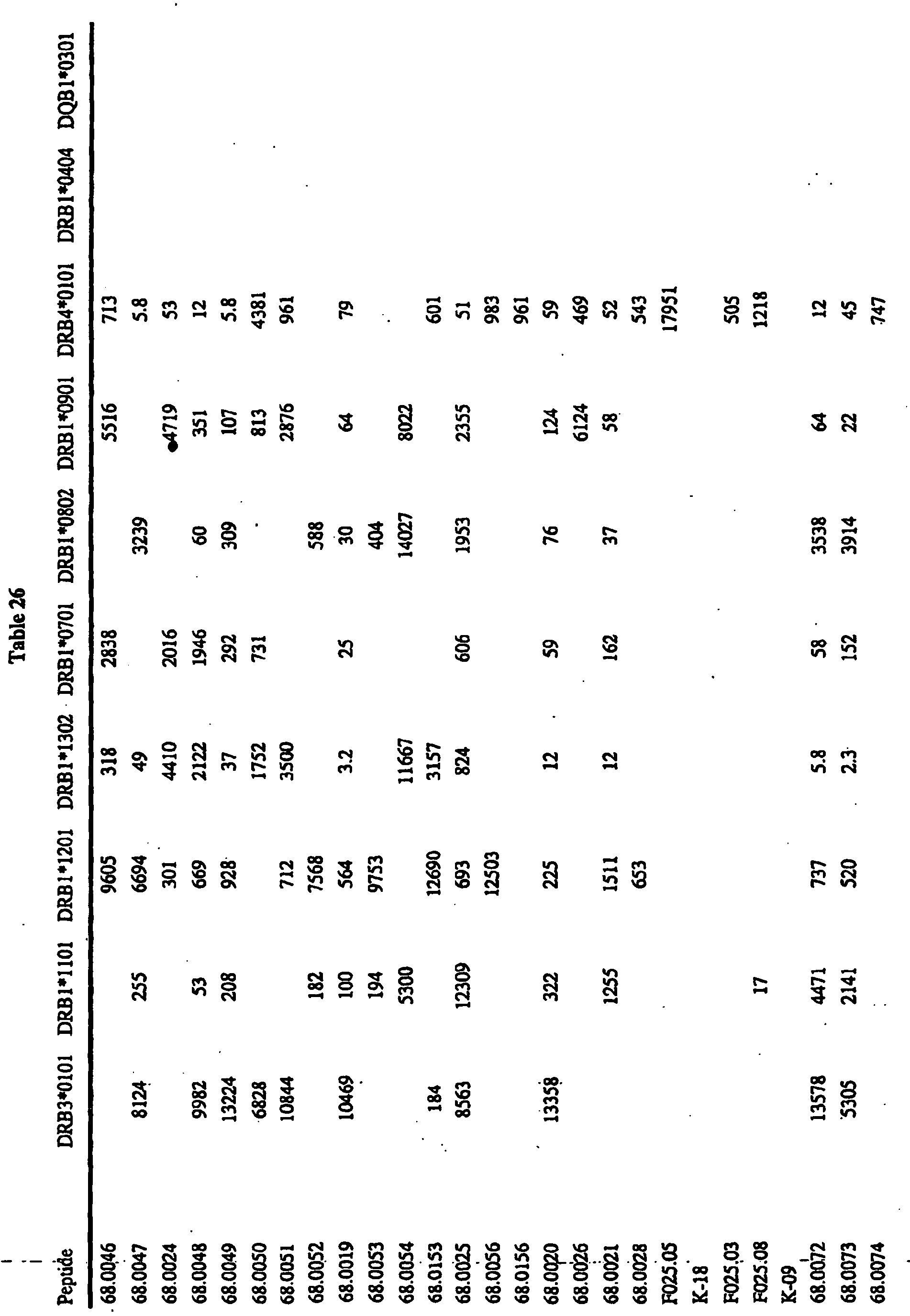

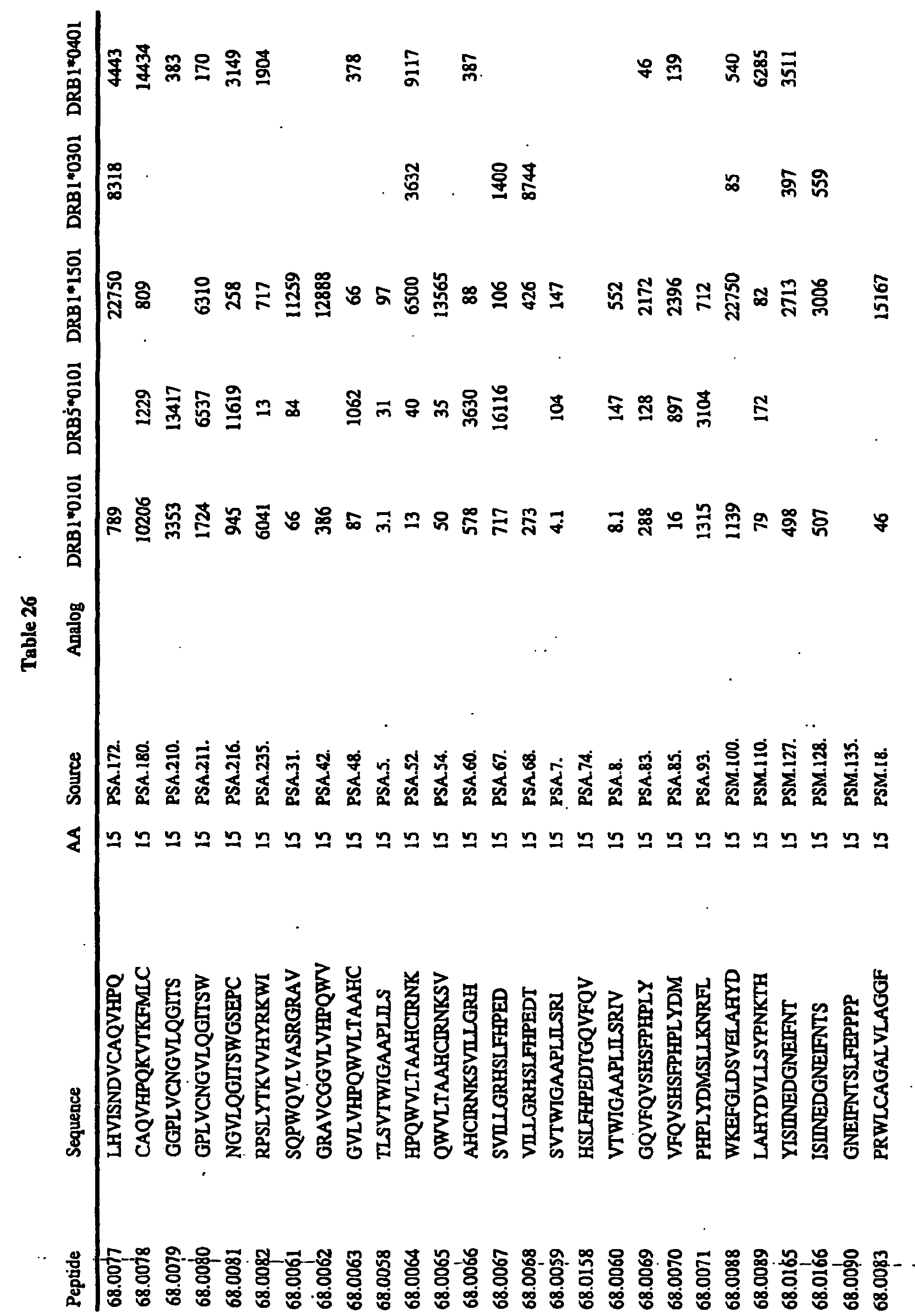

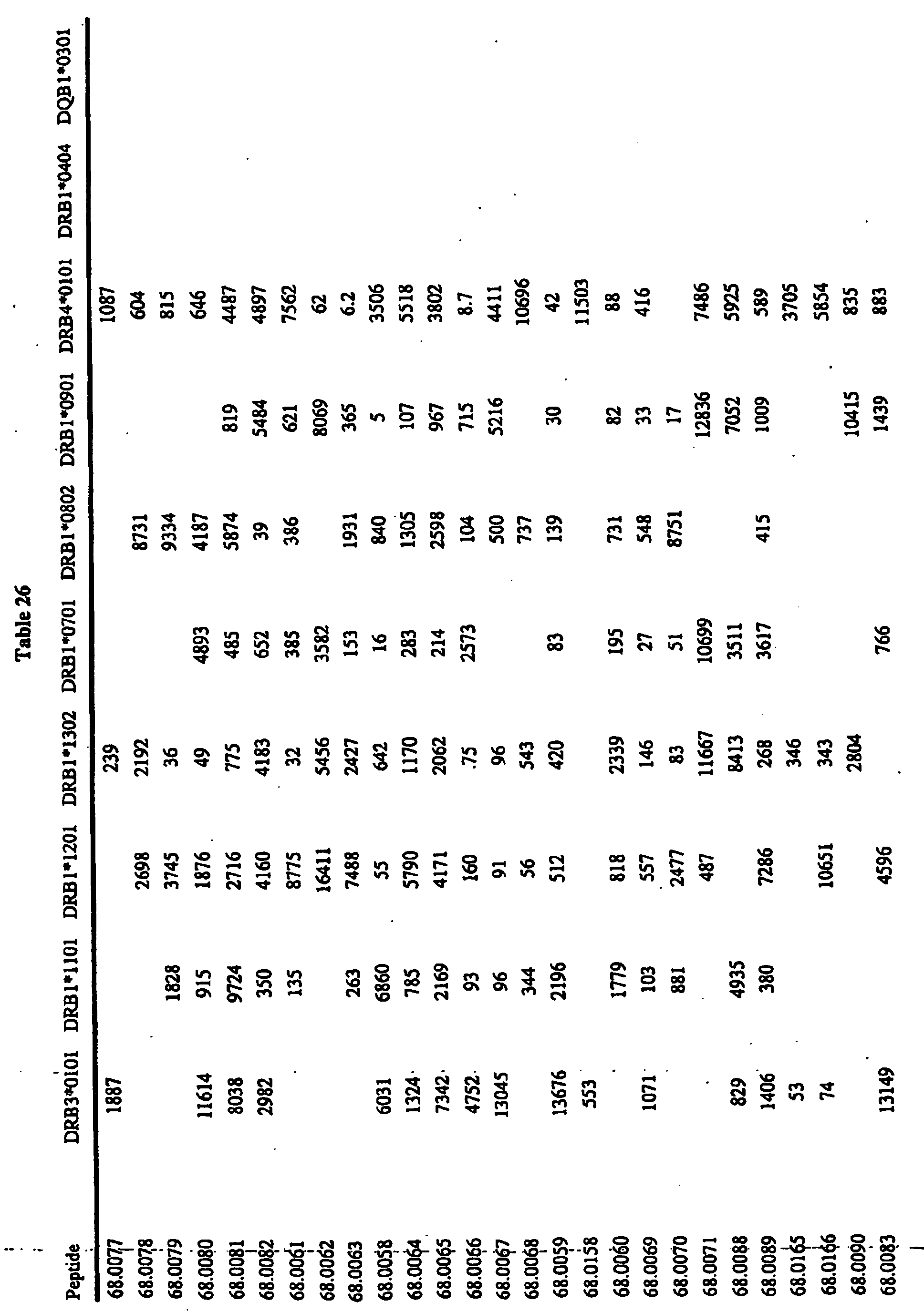

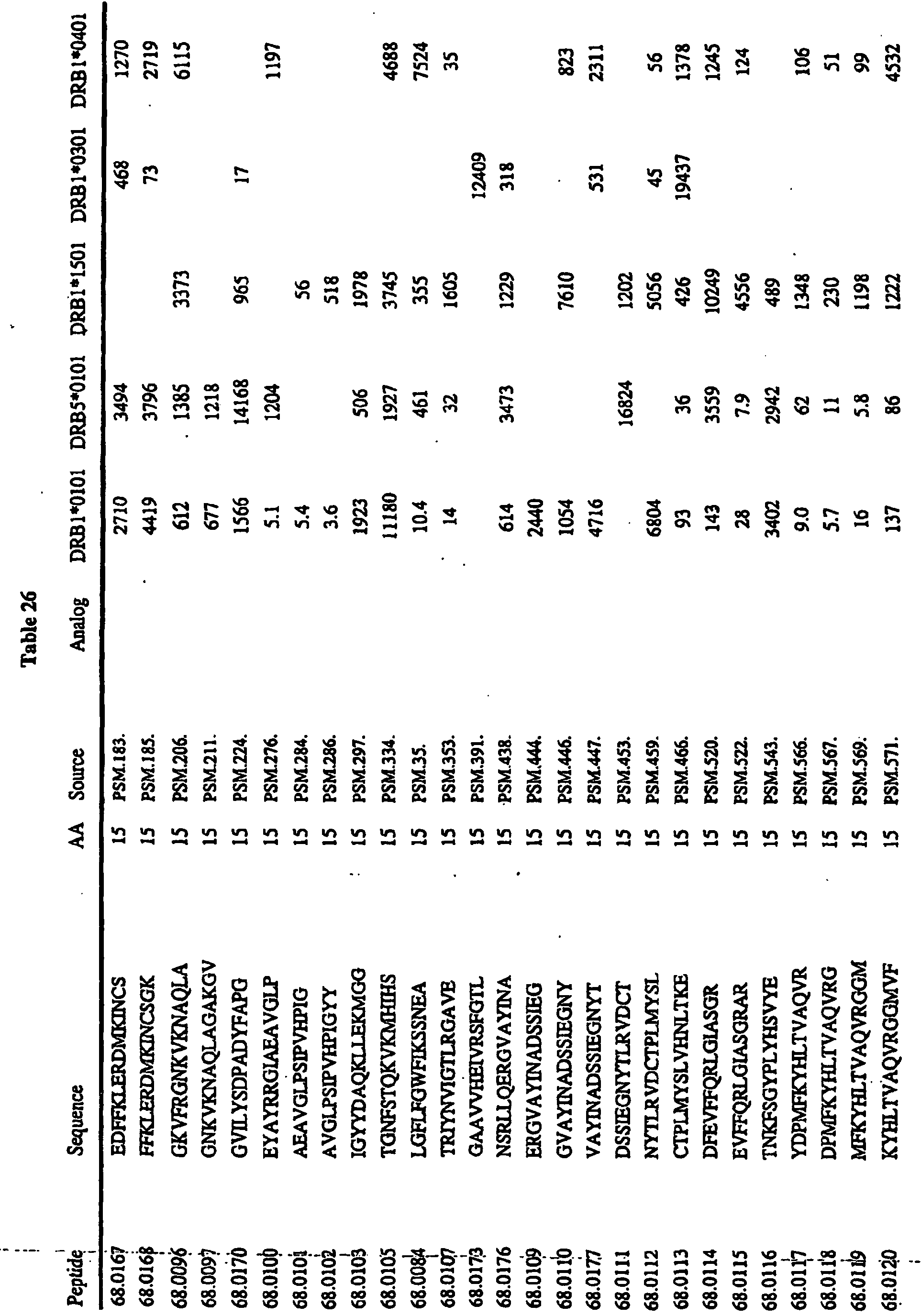

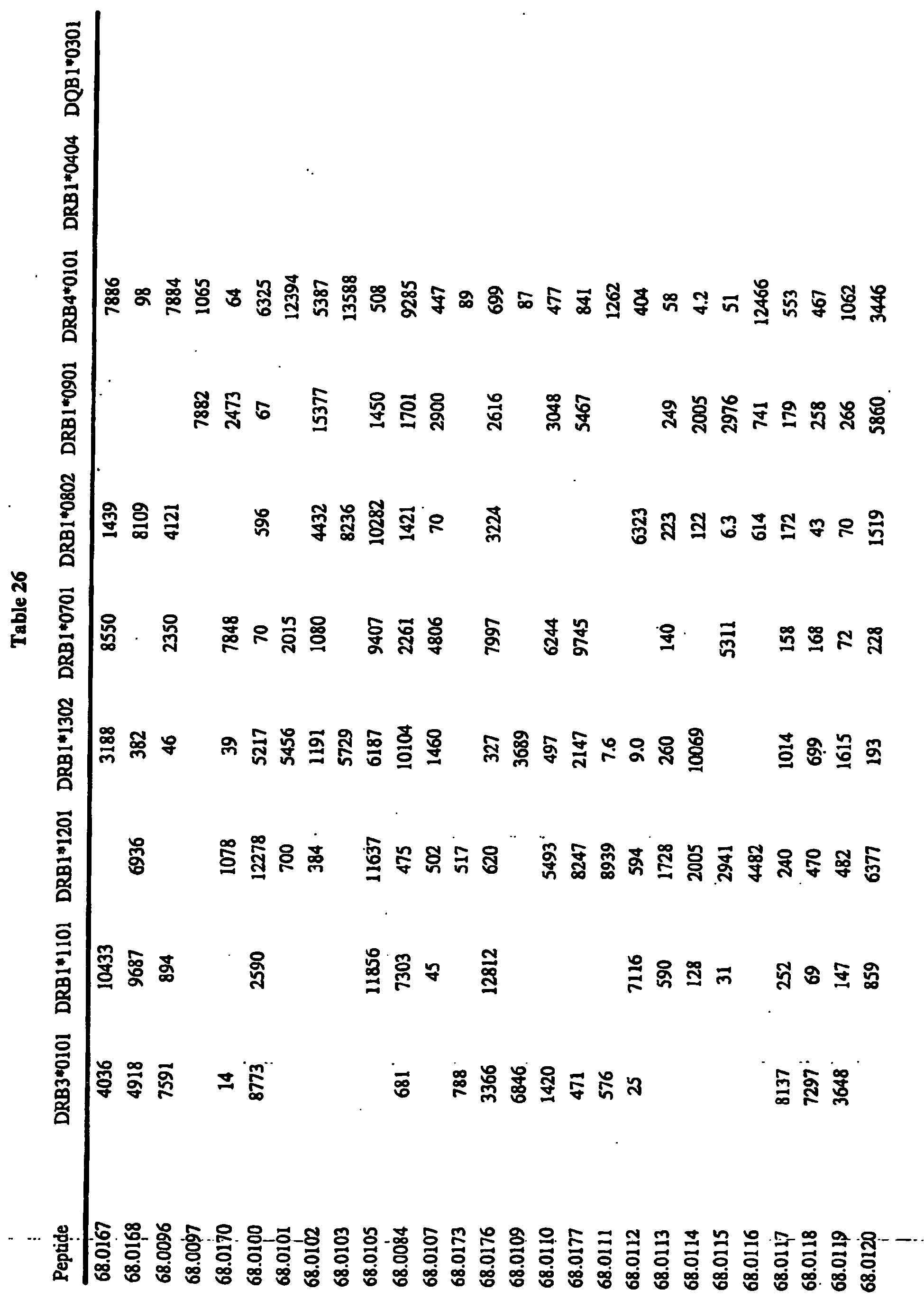

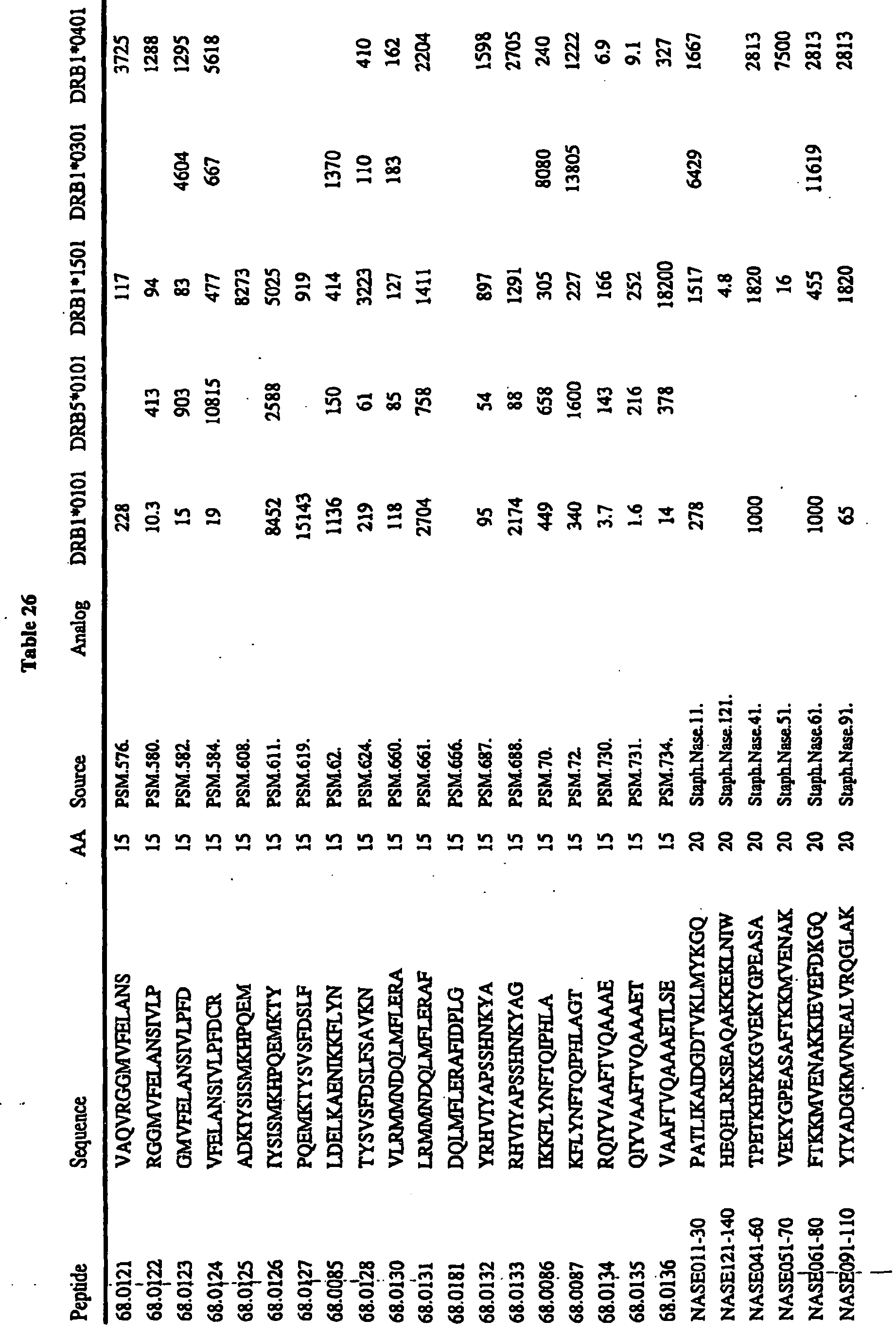

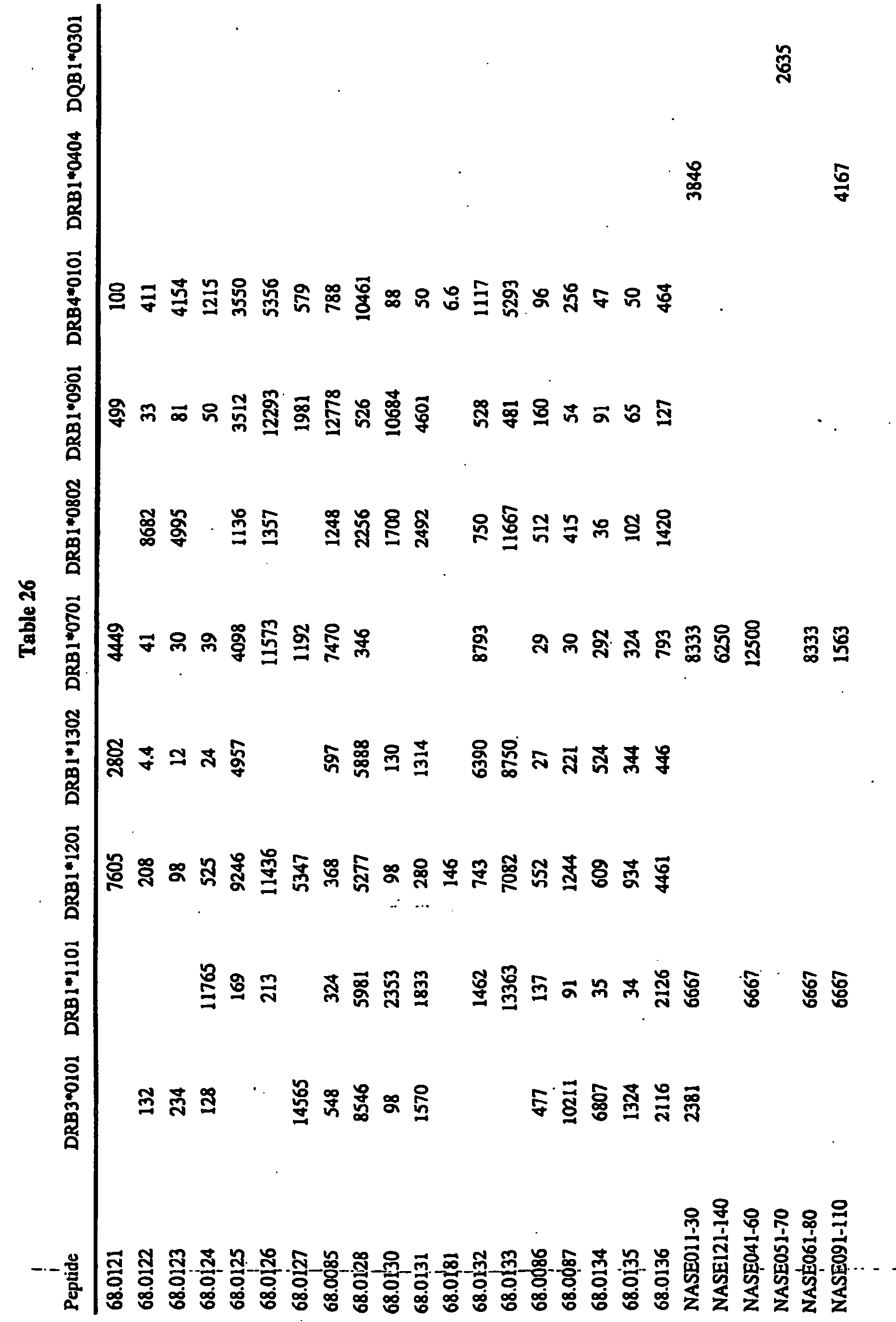

**TABLE 27a**

| | | | | | HLA-DR binding affinity (IC50 nM) | | | | |
|---|---|---|---|---|---|---|---|---|---|
| Peptide | Sequence | Source | Ag | Analog | DRB1 *0101 | DRB1 *0301 | DRB1 *0401 | DRB1 *0404 | DRB1 *0405 |
| PTS-04 | AAWSERAGEAMVLV | B.pertussis.247 | B.pertussis | | 625.0 | | 22500.0 | 500000.0 | |
| 582.02 | DNVLDHLTGRSC | B.pertussis.64 | B.pertussis | | 454.6 | 90000.0 | 22500.0 | 50000.0 | |
| PTS-10 | NH2-DNVLAHLTGRSCQ- COOH | B.pertussis.30 | B.pertussis | A | 122.0 | | | | |
| PTS-17 | NH2-DNVLDHLTGRSAQ- COOH | B.pertussis.30 | B.pertussis | A | 22.3 | | | | |
| PTS-18 | NH2-DNVLDHLTGRSCA- COOH | B.pertussis.30 | B.pertussis | A | 714.3 | | | | |
| 541.19 | HYTVDKSKPKVYQWRD | BeeVenom.109 | BeeVenom | | 1666.7 | | 450000.0 | 500000.0 | |
| 541.15 | INTKCYKLEHPVTGCG | BeeVenom85 | BeeVenom | | 227.3 | | 3750.0 | 500000.0 | |
| 541.04 | NELGRFKHTDACCRTH | BeeVenom.19 | BeeVenom | | 357.1 | | 450000.0 | 500000.0 | |
| 541.11 | NNDFYDNSADTISSYF | BeeVenom.61 | BeeVenom | | 1666.7 | | 918.4 | 500000.0 | |
| 541.20 | SKPKVYQWFDLRKY | BeeVenom.115 | BeeVenom | | 1250.0 | | 450000.0 | 2000.0 | |
| 541.13 | SSYFVGKMYFNLINTK | BeeVenom.73 | BeeVenom | | 714.3 | | 450000.0 | 500000.0 | |
| BMP | PDTRPAPGSTAPPAHGVTSAP DTR-NH2 | Breastmucin | Breastmucin | | 50000.0 | | 450000.0 | 50000.0 | |
| 1136.18 | ALPVFTWLALYFTSAK | Candida.aur1768.106 | Candida | | 178.7 | | 1485.2 | | 6675.9 |
| 1136.20 | ALYFTSAKIPQEWKPA | Candida.aur1768.114 | Candida | | 2886.8 | | 3763.1 | | 47.6 |
| 1136.15 | FATSFLIPLTSQFFLP | Candida.sur1768.90 | Candida | | 1.8 | | 5683.0 | | 518.5 |
| 1136.52 | GLFCRWSYTEIEKIDI | Candida.aur1768.338 | Candida | | 15811.4 | >9890.71 | 8079.6 | | 608.0 |
| 1136.53 | GLFSRWSYTEIEKIDI | Candida.aur1768.338 | Candida | | 25000.0 | | 1288.2 | | >8101.63 |
| 1136.48 | IGGAMLSLTVFEFTKY | Candida.aur1768.314 | Candida | | 388.1 | >9157.02 | 10978.9 | | 4976.8 |
| 1136.13 | IKLPIILAFATSFLIP | Candida.aur1768.82 | Candida | | 5.9 | | 2602.4 | | 16007.8 |
| 1136.43 | IMEVLFLSWLFPRFKF | Candida.aur1768.274 | Candida | | 208.3 | | 181.5 | | >25855.73 |
| 1136.67 | LTNNDQVSGINEEDEE | Candida.aur1768.418 | Candida | | >20412.41 | | >17450.25 | | >64231.72 |
| 1136.01 | MMASSILRSKIIQKPYQ | Candida.aur1768.1 | Candida | | 1644.9 | 9236.6 | >13693.06 | | >8101.63 |
| 1136.41 | PSLHSGSSIMVLFLS | Candida.aur1768.266 | Candida | | 735.4 | | >18541.93 | | 22391.7 |
| 1136.39 | SSIIFGAFPSLHSGSS | Candida.aur1768.258 | Candida | | 68.2 | | 28.2 | | 82.6 |
| 1136.10 | VSILFFVFVVFPASFF | Candida.aur1768.66 | Candida | | 2062.0 | >9157.02 | 637.2 | | 6088.8 |
| 58.0041 | IAKlTPDNNGTYACF | CEA.642 | CEA | A | | 51.7 | | | |
| 58.0022 | IPNTTVDNSGSYTCQ | CEA.286 | CEA | A | | 109.9 | | | |
| 58.0012 | IQNIIQDDTGFYTLH | CEA.109 | CEA | A | | 56.7 | | | |
| 58.0037 | LFNVTRDDARAYVCG | CEA.558 | CEA | A | | 56.8 | | | |
| 58.0017 | LFNVTRDDTASYKCE | CEA.202 | CEA | A | | 56.4 | | | |
| 58.0028 | LLSVTRDDVGPYECG | CEA.380 | CEA | A | | 560.6 | | | |
| 58.0015 | LWWVNNESLPVSPRL | CEA.177 | CEA | A | | 310.4 | | | |
| 58.0027 | LWWVNNESLPVSPRL | CEA.355 | CEA | A | | 2293 | | | |
| 58.0021 | QELFIPDITVNNSGS | CEA.282 | CEA | A | | 449.8 | | | |
| 58.0032 | QELFISDITEKNSGL | CEA.460 | CEA | A | | 302.3 | | | |
| 58.0009 | REIIYPDASLLIQNI | CEA.98 | CEA | A | | 126.4 | | | |
| 58.0020 | SCHAASDPPAQYSWF | CEA.258 | CEA | A | | 201.5 | | | |
| 58.0023 | TITVYADPPKPFITS | CEA.315 | CEA | A | | 378.0 | | | |
| 58.0005 | VLLLVHDLPQHLFGY | CEA.51 | CEA | A | | 4.9 | | | |
| 58.0042 | YACFVSDLATGRNNS | CEA.653 | CEA | A | | 19.5 | | | |
| CMP | PTTTPITTTTTVTPTPTPTGTQT YC-NH2 | Colonmucin | Colonmucin | | 50000.0 | | 1153.9 | 333.3 | |
| 722.01 | YIAFLKQATAK | Cyt.C | Cyt.C | | 104.2 | 900000.0 | 7500.0 | 10000.0 | |
| 500.02 | FLRRIRPKLK | Dynorphin.4 | Dynorphin | | | | | | |
| 199.02 | GFLRRIRPKLK | Dynorphin.3 | Dynorphin | | | | | | |
| 199.03 | LRRIRPKLK | Dynorphin.5 | Dynorphin | | | | | | |
| 199.01 | YGGFLRRIRPKLK | Dynorphin.1 | Dynorphin | | 50000.0 | 900000.0 | 450000.0 | 1923.1 | |
| 510.12 | DDNGPQDPDNTDDNG | EBV.LMP.260 | EBV | | 50000.0 | 51961.5 | 22500.0 | 25000.0 | |
| F166.01 | PYYTGEHAKAIGN | Flu.HA.308 | Flu | | 317.1 | | >14463.55 | >27317.92 | |
| 897.07 | FPQPQLPYSQPQPFRPQQPY- NH2 | Gliadin.61 NH2 | Gliadin | | | 900000.0 | | | |
| 897.25 | IPPYCTIAPFGIFGIN-NH2 | Gliadin.261 | Gliadin | | | 900000.0 | | | |
| F168.09 | IRNLALQTLPAMCNVY | Gliadin.235 | Gliadin | | | | | | |
| 897.04 | LGQQQPFPPQQPYPQPQPFP- NH2 | Gliadin.31 NH2 | Gliadin | | | 900000.0 | | | |
| 897.18 | LHQQQKQQQPSSQVSFQQP- NH2 | Gliadin.181 | Gliadin | | | 900000.0 | | | |
| 897.15 | LLQELCCQHLWQIPEQSQCQ- NH2 | Gliadin.151 | Gliadin | | | 900000.0 | | | |
| 897.13 | LQQHNIAHGRSQVLQQSTYQ- NH2 | Gliadin.131 | Gliadin | | | | | | |
| F168.04 | PQPFRPQQPYPQ | Gliadin.71 | Gliadin | | | | | | |
| F168.03 | PQPFRPQQPYPQPQPQ | Gliadin.71 | Gliadin | | | | | | |
| 897.08 | PQPFRPQQPYPQPQPQYSQP- NH2 | Gliadin.71 | Gliadin | | | 900000.0 | | | |
| 897.09 | PQPQPQYSQPQQPISQQQQQ- NH2 | Gliadin.81 | Gliadin | | | 900000.0 | | | |
| 897.19 | PSSQVSFQQPLQQYPLGQGS- NH2 | Gliadin.191 | Gliadin | | | 4691.6 | | | |
| F168.12 | QFEEIRNLALQT | Gliadin.231 | Gliadin | | | | | | |
| F168.11 | QFEEIRNLALQTLPAM | Gliadin.231 | Gliadin | | | | | | |
| 897.23 | QFEEIRNLALQTLPAMCNVY- NH2 | Gliadin.231 | Gliadin | | | 900000.0 | | | |
| F168.06 | QFLGQQQPFPPQ | Gliadin.29 | Gliadin | | | | | | |
| 897.22 | QGSVQPQQLPQFEEIRNLAL- NH2 | Gliadin.221 | Gliadin | | | 900000.0 | | | |
| 897.02 | QNPSQQQPQEQVPLVQQQF- NH2 | Gliadin.11 | Gliadin | | | 900000.0 | | | |
| 897.05 | QPYPQPQPFPSQQPYLQLQP- NH2 | Gliadin.41 | Gliadin | | | 900000.0 | | | |
| 897.12 | [?] QQLIFCMDVVLQQHNIAHGR- NH2 | Gliadin.121 | Gliadin | | | 9819.8 | | | |
| F168.18 | QVPLVQQQQFLG | Gliadin.21 | Gliadin | | | | | | |
| F168.07 | QVPLVQQQQFLGQQQP | Gliadin.21 | Gliadin | | | | | | |
| 897.03 | QVPLVQQQFLGQQQPFPPQ- NH2 | Gliadin.21 | Gliadin | | | | | | |
| 897.06 | SQQPYLQLQPFPQPQLPYSQ- NH2 | Gliadin.51 | Gliadin | | | 900000.0 | | | |
| F168.05 | VQQQQFLGQQQPFPPQ | Gliadin.25 | Gliadin | | | | | | |
| 897.01 | VRVPVPQLQPQNPSQQQPQE- NH2 | Gliadin.1 | Gliadin | | | 900000.0 | | | |
| 897.50 | YIPPYCTIAPFGIFGIN-NH2 | Gliadin.261 | Gliadin | | | >45000 | | | |
| 897.44 | YPSSQVSFQQPLQQYPLGQGS -NH2 | Gliadin.191 | Gliadin | | | 445.5 | | | |
| 897.47 | YQGSVQPQQLPQFEEIRNLAL- NH2 | Gliadin.221 | Gliadin | | | >30000 | | | |
| F160.26 | EKVPVSEVMGTTLAEMSTPE AT | gp100.377 | gp100 | | | | 10603.5 | 94.8 | |
| F160.02 | IYRRRLMKQDFSVPQLPHS | gp100.615 | gp100 | | | | 58.6 | 13.0 | |
| F160.07 | KRCLLHLAVIGALLAVGATK V | gp100.6 | gp100 | | | | >14411.53 | 408.8 | |
| F160.03 | LCQPVLPSPACQLVLHQILKG G | gp100.540 | gp100 | | | | >14411.5 | 732.0 | |
| F160.14 | MTPAEVSIVVLSGTTAAQVTT [?] T | gp100.400 | gp100 | | | | 1293 | 31.6 | |
| F160.16 | PGPVTAQVVLQAAIPLTSCGS S | gp100.283 | gp100 | | | | 2519.1 | 81.1 | |
| F160.01 | VSGLSIGTGRAMLGTHTMEV TVY | gp100.167 | gp100 | | | | 10452.1 | 384.2 | |
| F167.01 | VYVWKTTWGQYWQVLGGP VS | gp100.150 | gp100 | | 114.9 | >14248.07 | >3387.8 | 27886.2 | |
| F167.03 | WNRQLYPEWTEAQR | gp100.44 | gp100 | | 7800.3 | 361.8 | 145.6 | 538.4 | |
| F115.14 | AKRKTVTAMDVVYAL | H4.76 | H4 | | 1.3 | | | | |
| F115.02 | KRHRKVLRDNIQGITKPAIRR LAR | H4.16 | H4 | | 258.0 | | | | |
| F115.15 | KTVTAMDVVYALKRQ | H4.79 | H4 | | 26.0 | | | | |
| F115.10 | LRDNIQGITKPAIRR | H4.22 | H4 | | 16.4 | | | | |
| F115.11 | NIQGITKPAIRRLAR | H4.25 | H4 | | 234.7 | | | | |
| F115.03 | TYTEHAKRKTVTAMDVVYA LKRQG | H4.71 | H4 | | 75.9 | | | | |
| 590.23 | (2)KYVKQNTLKAT(SIC!) | HA.307 | HA | A | 15.2 | | 5.9 | | |
| 752.01 | (67)PKYVKQNTLKLAT | HA.307 | HA | | 3.9 | | 72.6 | 25000.0 | |
| 594.09 | AHAAHAA | HA. | HA | A | 263.2 | | 450000.0 | 500000.0 | |
| F049.01 | BIOTIN-A2- PRYVRQNTLRATC | HA | HA | | 6.5 | | 33.4 | | |
| 574.00 | CPKYVRSAKLRM | HA.302 | HA | | 1666.7 | 36742.4 | 450000.0 | 500000.0 | |
| 573.05 | GACPKYVKQN | HA.304 | HA | | 50000.0 | | 15000.0 | | |
| 573.04 | GACPKYVKQNTL | HA.304 | HA | | 50000.0 | | 1153.9 | | |
| 597.04 | GACPKYVKQNTLK | HA.304 | HA | | 50000.0 | | 3750.0 | | |
| 573.03 | GACPKYVKQNTLKL | HA.304 | HA | | 2500.0 | | 1184.2 | | |
| 30.1200 | HNTNGVTAASSH | HA.130 | HA | | 50000.0 | | 450000.0 | 500000.0 | |
| 573.08 | KQNTLKLATGMR | HA.311 | HA | | 50000.0 | | 15000.0 | | |
| 711.03 | LAKQNTLAKQNTLAKQNT | HA.307 | HA | A | 50000.0 | 900000.0 | 150.0 | 86.2 | |
| 573.09 | NTLKLATGMR | HA.313 | HA | | 50000.0 | | 15000.0 | | |
| 590.22 | P(2)YVKQNTLKAT(SIC!) | HA.307 | HA | A | 454.6 | | 20.5 | | |
| 864.10 | PFFVKQNILKLAT | HA.307 | HA | A | 7.2 | | 44.6 | 3571.4 | |
| 864.07 | PFFVKQNTLKLAT | HA.307 | HA | A | 1.4 | | 10.7 | 344.8 | |
| 864.06 | PFIVKQNTLKLAT | HA.307 | HA | A | 1.0 | | 19.6 | 79.6 | |
| 864.02 | PFYVKQNTLKLAT | HA.307 | HA | A | 1.6 | | 24.5 | 12500.0 | |
| 864.09 | PIFVKQNILKLAT | HA.307 | HA | A | 2.9 | | 56.4 | 3846.2 | |
| 864.05 | PIFVKQNTLKLAT | HA.307 | HA | A | 2.9 | | 32.1 | 649.4 | |
| 864.04 | PIIVKQNTLKLAT | HA.307 | HA | A | 7.6 | | 68.1 | 160.3 | |
| 864.01 | PIYVKQNTLKLAT | HA.307 | HA | A | 3.9 | | 23.8 | 10000.0 | |
| 590.10 | PK(1)VKQNTLKAT(SIC!) | HA.307 | HA | A | 454.6 | | 2368.4 | | |
| 590.21 | PK(2)VKQNTLKAT(SIC!) | HA.307 | HA | A | 454.6 | | 511.4 | | |
| 772.08 | PK(31)VKQNTLKLAT-NH2 | HA.307 | HA | A | 5.6 | | 136.4 | 8333.3 | |
| 611.11 | PKAVKQNTLKLAT | HA.307 | HA | A | 1000.0 | | 15000.0 | 25000.0 | |
| 601.09 | PKEVKQNTLKLAT | HA.307 | HA | A | 50000.0 | | 4500.0 | 500000.0 | |
| 841.05 | PKFQVETTKKLAT | HA. | HA | A | 185.2 | 900000.0 | 155.7 | 500000.0 | |
| 864.03 | PKIVKQNTLKLAT | HA307 | HA | A | 7.3 | | 92.4 | 130.9 | |
| 713.09 | PKKVKQNTLKLAT | HA.307 | HA | A | 2500.0 | | 9000.0 | 500000.0 | |
| 792.03 | PKQVKQNTLKLAT | HA.307 | HA | A | 1250.0 | | 9000.0 | 500000.0 | |
| 601.10 | PKSVKQNTLKLAT | HA.307 | HA | A | 50000.0 | | 57.7 | 500000.0 | |
| 713.14 | PKTVKQNTLKLAT | HA.307 | HA | A | 2500.0 | | 5625.0 | 500000.0 | |
| 590.09 | PKY(1)KQNTLKAT(SIC!) | HA.307 | HA | A | 72.5 | | 7.B | | |
| 833.08 | PKY(41)KQNTLKLAT | HA.307 | HA | A | 217.A | | 436.9 | 500000.0 | |
| 590.08 | PKYV(1)QNTLKAT(SIC!) | HA.307 | HA | A | 72.5 | | 375 | | |
| 590.19 | PKYV(2)QNTLKAT(SIC!) | HA.307 | HA | A | 138.9 | | 11.8 | | |
| 833.07 | PKYV(41)QNTLRLAT | HA.307 | HA | A | 50000.0 | | 576.9 | 500000.0 | |
| 590.07 | PKYVK(1)NTLKAT(SICl) | HA.307 | HA | A | 294.1 | | 225.0 | | |
| 590.18 | PKYVK(2)NTLKAT(SICl) | HA.307 | HA | A | 147.1 | | 463.9 | | |
| 713.10 | PKYVKKNTLKLAT | HA.307 | HA | A | 89.3 | | 3000.0 | 500000.0 | |
| 590.06 | PKYVKQ(1)TLKAT(SIC!) | HA.307 | HA | A | 50.0 | | 6.9 | | |
| 590.1 | PKYVKQ(2)TLKAT(SIC!) | HA.307 | HA | A | 116.3 | | 13.6 | | |
| 833.05 | PKYVKQ(41)TLKLAT | HA.307 | HA | A | 11.6 | | 61.6 | 25000.0 | |
| 590.05 | PKYVKQN(1)LKAT(SIC!) | HA.307 | HA | A | 50000.0 | | 450.0 | | |
| 590.16 | PKYVKQN(2)LKAT(SIC!) | HA.307 | HA | A | 454.6 | | 2368.4 | | |
| 772.06 | PKYVKQN(24)LKLAT | HA.307 | HA | A | 6.8 | | 88.2 | 25000.0 | |
| 772.01 | PKYVKQN(25)LKLAT | HA.307 | HA | A | 5.6 | | 1071.4 | 500000.0 | |
| 772.02 | PKYVKQN(26)LKLAT | HA.307 | HA | A | 166.7 | | 5625.0 | 500000.0 | |
| 772.03 | PKYVKQN(27)LKLAT | HA.307 | HA | A | 357.1 | | 3000.0 | 16666.7 | |
| 772.04 | PKYVKQN(28)LKLAT | HA.307 | HA | A | 20.8 | | 2812.5 | 25000.0 | |
| 772.07 | PKYVKQN(30)LKLAT | HA.307 | HA | A | 625.0 | | 15000.0 | 25000.0 | |
| 601.24 | PKYVKQNELKLAT | HA.307 | HA | A | 714.3 | | 15000.0 | 500000.0 | |
| 515.09 | PKYVKQNHLKLAT | HA.307 | HA | A | 96.2 | | 4500.0 | 500000.0 | |
| 864.08 | PKYVKQILKLAT | HA.307 | HA | A | 35.5 | | 156.8 | 5000.0 | |
| 713.12 | PKYVKQNKLKLAT | HA.307 | HA | A | 2500.0 | | 9000.0 | 500000.0 | |
| 863.07 | PKYVKQNNLKLAT | HA.307 | HA | A | 500.0 | | 3114.3 | 500000.0 | |
| 772.11 | PKYVKQNPLKLAT | HA.307 | HA | A | 4.2 | | 737.7 | 50000.0 | |
| 713.13 | PKYVKQNQLKLAT | HA.307 | HA | A | 45.5 | | 5625.0 | 500000.0 | |
| 590.04 | PKYVKQNT(1)KAT(SIC!) | HA.307 | HA | A | 102.0 | | 281.3 | | |
| 590.15 | PKYVKQNT(2)KAT(SIC!) | HA.307 | HA | A | 147.1 | | 264.7 | | |
| 833.03 | PKYVKQNT(41)KLAT | HA.307 | HA | A | 714.3 | | 7500.0 | 500000.0 | |
| 601.27 | PKYVKQNTKKLAT | HA.307 | HA | A | 13.9 | | 2142.9 | 500000.0 | |
| 590.03 | PKYVKQNTL(1)AT(SIC!) | HA307 | HA | A | 45.5 | | 6.9 | | |
| 590.14 | PKYVKQNTL(2)AT(SIC!) | HA.307 | HA | A | 66.7 | | 11.0 | | |
| 833.02 | PKYVKQNTL(41)LAT | HA.307 | HA | A | 56.8 | | 232.0 | 500000.0 | |
| 590.02 | PKYVKQNTLK(1)T(SIC!) | HA.307 | HA | A | 454.6 | | 2250.0 | | |
| 590.01 | PKYVKQNTLKAT(SIC!) | HA.307 | HA | A | 108.7 | | 21.4 | | |
| 713.06 | PKYVKQNTLKEAT | HA.307 | HA | A | 2500.0 | | 1097.6 | 500000.0 | |
| 863.08 | PKYVKQNTNKLAT | HA.307 | HA | A | 70.4 | | 1097.6 | 500000.0 | |
| 713.01 | PKYVKQNYLKLAT | HA.307 | HA | A | 192.3 | | 9000.0 | 25000.0 | |
| 175.00 | RTLYQNVGTYVSVGTSTLNK | HA.187 | HA | | 19.2 | 900000.0 | 450000.0 | 25000.0 | |
| 597.06 | VKQNTLKLATGMR | HA.310 | HA | | 50000.0 | | 7500.0 | | |
| 841.06 | YPKFQVETTKKLAT | HA | HA | A | 45.5 | | 111.4 | 500000.0 | |
| 841.04 | YPKFVKLNTKKLAT | HA | HA | A | 20.8 | | 365.9 | 5000.0 | |
| 771.04 | YPKFVKQRTLKLAT | HA | HA | A | 3.6 | | 46.9 | 357.1 | |
| 771.02 | YPKFVKQRTLKLAT-NH2 | HA | HA | | 4.6 | | 37.5 | 172.4 | |
| 771.06 | YPKFVKRNTLKLAT | HA.307 | HA | A | 14.3 | | 818.2 | 10000.0 | |
| 771.03 | YPKYVKQRTLKLAT | HA | HA | A | 2.8 | | 51.7 | 10000.0 | |
| 771.01 | YPKYVKQRTLKLAT-NH2 | HA | HA | A | 5.0 | | 60.0 | 1724.1 | |
| 771.07 | YPKYVKRNTLKLAT | HA.307 | HA | A | 23.8 | | 1956.5 | 12500.0 | |
| 841.02 | YPSFQVQTTLLLAT | HA | HA | A | 1.1 | | 2.7 | 12.2 | |
| 27.0278 | AAPFTQCGYPALMPL | HBV.pol.643 | HBV | | 160.8 | | >23985.08 | | |
| F039.09 | AILCWGELMTLA | HBV.core.58 | HBV | | 6299.4 | | 450000.0 | >7462.69 | |
| F076.05 | ALRQAILCWGELM | HBV.core.54 | HBV | | | | >9183.67 | >22727.27 | |
| F039.05 | ALRQAILCWGELMTLA | HBV.core.54 | HBV | | 505.8 | | 24549.5 | >7462-69 | |
| 799.04 | GYRWMCLRRFIIFLFILLLC | HBV.env.71 | HBV | | 2500.0 | 28460.5 | 424.5 | 12500.0 | |
| F039.02 | HHTALRQAILCWGELMTLA | XBV.core.51 | HBV | | 1127.1 | | 225000.0 | >7462.69 | |
| F039.03 | HTALRQAILCWGELMTLA | HBV.core.52 | HBV | | 512.7 | | 35575.6 | >7462.69 | |
| F039.10 | ILCWGELMTLA | HBV.core.59 | HBV | | 50000.0 | | >818.18 | >7462.69 | |
| F039.11 | LCWGELMTLA | HBV.core.60 | HBV | | 50000.0 | | >900 | >7462.69 | |
| F164.04 | LPETTVVRCRGRSPR | HBV.core.143 | HBV | | >1889.82 | | >504.69 | >22680.46 | |
| F039.06 | LRQAILCWGELMTLA | BBV.core.55 | HBV | | 896.6 | | 225000.0 | >7462.69 | |
| CF-09 | LSTLPETTVVRRRGRS | HBV.core.140 | HBV | | 50000.0 | | 9000.0 | 25000.0 | |
| F164.14 | MDIDPYKEFGASVELLSFL | HBVcore.1 | HBV | | 116.9 | | 837.6 | 27.6 | |
| F164.13 | MDIDPYKEFGASVELLSFLPS DFFP | HBV.core.1 | HBV | | 34.6 | | 2580.9 | 110.1 | |
| F164.16 | MDIDPYKEFGATVELLSFL | HBV.core.1 | HBV | | 1356.7 | | 308.0 | 108.8 | |
| F116.01 | MDIDPYKEFGATVELLSFLPS DFFP : . | HBV.core.1 | HBV | | 1562.5 | | 957.5 | | |
| F164.15 | MDIDPYKEFGATVELLSFLPS DFFP | HBV.core.1 | HBV | | 113.8 | | 145.4 | 17.3 | |
| F164.17 | MDIDPYKEFGATVQLLSFLPS DFFP : | HBV.core.1 | HBV | | 411.6 | | 33.9 | 10.2 | |
| F098.11 | MGLKFRQLLWF | HBV.core.94 | HBV | | 240.6 | | 3620.6 | 1350.7 | 206.3 |
| 1297.06 | PFLLAQFTSAICSVVRRA | HBV.pol.523 | HBV | | | | | | |
| F039.18 | PHHTALRQAILCW | HHV.core.50 | HBV | | 16666.7 | | 5489.4 | >20000 | |
| F039.17 | PHHTALRQAILCWG | HBV.core.50 | HBV | | 2130.1 | | 450000.0 | >20000 | |
| F039.16 | PHHTALRQAILCWGE | HBV.core.50 | HBV | | 11180.3 | | >600 | >20000 | |
| F039.15 | PHHTALRQAILCWGEL | HBV.core.50 | HBV | | 2391.8 | | 150000.0 | >20000 | |
| F039.14 | PHHTALRQAILCWGELM | HBV.core.50 | HBV | | 389.3 | | 5669.5 | >7462.69 | |
| F039.13 | PHHTALRQAILCWGELMT | HBV.core.50 | HBV | | 1725.2 | | 14230.3 | >7462.69 | |
| F039.12 | PHHTALRQAILCWGELMTL | HBV.core.50 | HBV | | 145.8 | | 10062.3 | >7462.69 | |
| 795.01 | PLGFFPDHQLDPAFGANSNNP DWDFNP · | HBV.PreS. | HBV | | 50000.0 | 900000.0 | 6428.6 | 500000.0 | |
| F039.08 | QAILCWGELMTLA | HBV.core.57 | HBV | | 1605.7 | | 20539.6 | >7462.69 | |
| CF-03 | RDLLDTASALYRREALESPEH | HBV.core.28 | HBV | | 50000.0 | | 7500.0 | 16666.7 | |
| CF-06 | RDLVVSYVNTNMGLKFRQLL | HBV.core.82 | HBV | | 1000.0 | | 2250.0 | 2941.1 | |
| F039.07 | RQAILCWGELMTLA | HBV.core.56 | HBV | | 3125.0 | | 225000.0 | >7462.69 | |
| 801.01 | RVRGLYFPAGGSSSGTVN | HBV.env.16 | HBV | | 294.1 | 900000.0 | 616.4 | 16666.7 | |
| | SLDSWWTSLNFLGGTTVCLG | HBV.env.31 | HBV | | 36.8 | 15667.0 | 1956.5. | 25000.0 | |
| 799.02 F039.04 | TALRQAILCWGELMTLA | HBV.core.53 | HBV | | 931.5 | | 43301.3 | >7462.69 | |
| F164.12 | TNMGLKFRQLLWFHI | HBV.core.91 | HBV | | >1889.82 | | >7367.12 | 6273.9 | |
| F164.11 | TNVGLKFRQLLWFHI | HBV.core.91 | HBV | | 1491.4 | | 835.7 | 149.6 | |
| F098.08 | TTVVRRRGRSPPRR | HBV.core.145 | HBV | | 98039.2 | | >100623.06 | >62.500 | >66149.51 |
| 800.04 | VGAGAFGLGFTPPHGGL | HBV.env.47 | HBV | | 5000.0 | 900000.0 | 26.5 | 3333.3 | |
| 764.01 | VSFGVWIRTPPA | HBV.core. | HBV | | 50000.0 | 900000.0 | 15000.0 | 5000.0 | |
| 857.01 | YPHHTALRQAILCWGELMTL A | HBV.core.50 | HBV | | 1000.0 | | 633.9 | 23622.8 | |
| F134.07 | GPGEGAVQWMNRLIAFASRG | HCV.NS4.291 | HCV | | 8.7 | | 5328.0 | | |
| Pape22 | GRHLIFCHSKRKCDELATKL | HCV.NS3.1388 | HCV | | >50000 | | >18750 | >62500 | >6614951 |
| F134.06 | LLFNILGGWVAAQLAAPGAA | HCV.NS4.191 | HCV | | 26.7 | | >3668.13 | | |
| 1283:40 | PAILSPGALVVGVVCA | HCV.NS4.1889 | HCV | | 50.9 | | 63469.7 | | |
| 221.06 | AFVAWRNRCK | HEL.107 | HEL | A | | | | | |
| 221.08 | AWAAWRNRCK | HEL.107 | HEL | A | | | | | |
| 221.10 | AWEAWRNRCK | HEL.107 | HEL | A | | | | | |
| 221.09 | AWLAWRNRCK | HEL.107 | HEL | A | | | | | |
| 221.15 | AWVAARNRCK | HEL.107 | HEL | A | | | | | |
| 221.16 | AWVAFRNRCK | HEL.107 | HEL | A | | | | | |
| 221.14 | AWVAQRNRCK | HEL.107 | HEL | A | | | | | |
| 221.19 | AWVAWANRCK | HEL.107 | HEL | A | | | | | |
| 221.17 | AWVAWENRCK | HEL.107 | HEL | A | | | | | |
| 221.18 | AWVAWKNRCK | HEL.107 | HEL | A | | | | | |
| 221.22 | AWVAWRARCK | HEL.107 | HEL | A | | | | | |
| 221.25 | AWVAWRNACK | HEL.107 | HEL | A | | | | | |
| 221.23 | AWVAWRNECK | HEL.107 | HEL | A | | | | | |
| 221.24 | AWVAWRNKCK | HEL.107 | HEL | A | | | | | |
| 221.32 | AWVAWRNRCA | HEL.107 | HEL | A | | | | | |
| 539.00 | AWVAWRNRCK | HEL.107 | HEL | | 50000.0 | >5000 | 3750.0 | 500000.0 | |
| 221.01 | AWVAWRNRCK | HEL.107 | HEL | | 50000.0 | | 3750.0 | 500000.0 | |
| 221.31 | AWVAWRNRCR | HEL.107 | HEL | A | | | | | |
| 221.28 | AWVAWRNREK | HEL.107 | HEL | A | | | | | |
| 221.29 | AWVAWRNRKK | HEL.107 | HEL | A | | | | | |
| 221.26 | AWVAWRNRQK | HEL.107 | HEL | A | | | | | |
| 221.27 | AWVAWRNRVK | HEL.107 | HEL | A | | | | | |
| 221.20 | AWVAWRQRCK | HEL.107 | HEL | A | | | | | |
| 221.21 | AWVAWRVRCK | HEL.107 | HEL | A | | | | | |
| 221.12 | AWVEWRNRCK | HEL.107 | HEL | A | | | | | |
| 221.11 | AWVSWRNRCK | HEL.107 | HEL | A | | | | | |
| 221.13 | AWVVWRNRCK | HEL.107 | HEL | A | | | | | |
| 565.01 | EFVAAKAAQK | HEL.107 | HEL | | 625.0 | 900000.0 | 11250.0 | 50000.0 | |
| 221.02 | EWVAWRNRCK | HEL.107 | HEL | A | | | | | |
| AP23 | LAAAMKRHGLDNYRGYSLG NWV | HEL.8 | HEL | | 1250.0 | 900000.0 | 22500.0 | 500000.0 | |
| AP12 | RNRCKGTDVQAWIRGCRL | HEL.112 | HEL | | 500.0 | 900000.0 | 22500.0 | 1087.0 | |
| AP38 | SVNCAKKIVSDGNGMN | HEL.91 | HEL | | 50000.0 | 9449.4 | 1125.0 | 8333.3 | |
| 221.04 | SWVAWRNRCK | HEL.107 | HEL | A | | | | | |
| 221.03 | VWVAWRNRCK | HEL.107 | HEL | A | | | | | |
| 560.02 | WRNAKWRNAKWRNAK | HEL.112 | HEL | A | 1000.0 | 900000.0 | 22500.0 | 500000.0 | |
| AP37 | YRGYSLGNWVCAAKFESNFN TQ | HEL.20 | HEL | | 555.6 | 900000.0 | 22500.0 | 500000.0 | |
| 58.0052 | CWMIDSDCRPRFREL | Her2/neu.958 | Her2/neu | A | | 5.1 | | | |
| 58.0045 | CYGLGMDDLREVRAV | Her2/neu.342 | Her2/neu | A | | 180.5 | | | |
| 58.0053 | FRELVSDFSRMARDP | Her2/neu.969 | Her2/neu | A | | 121.2 | | | |
| 58.0051 | IKWMALDSILRRRFT | Her2/neu.886 | Her2/neu | A | | 1.7 | | | |
| 58.0046 | LALIHHDTHLCFVHT | Her2/neu.465 | Her2/neu | A | | 17.0 | | | |
| 58.0043 | LTYLPTDASLSFLQD | Her2/neu.62 | Her2/neu | A | | 106.8 | | | |
| 58.0048 | QMRILKDTELRKVKV | Her2/neu.711 | Her2/neu | A | | 335.4 | | | |
| 1385.03 | SPYVSRLLGICLT | Her2/neu.777 | Her2/neu | | 12.8 | >21096.33 | >4494.22 | 172.5 | |
| 1385.04 | VPIKWMALESILRRRF | Her2/neu.884 | Her2/neu | | 12.1 | 117.7 | 1154.6 | 1035.9 | |
| 58.0047 | WDQLFRDPHQALLHT | Her2/neu.482 | Her2/neu | A | | 4.8 | | | |
| F159.13 | DRVHPVHAGPIA | HIV.gag.245 | HIV | | 7704.2 | | >7905.69 | 55555.6 | >11109.4 |
| F159.12 | DRVHPVHAGPIAPG | HIV.gag.245 | HIV | | 229.5 | | >6830.74 | 45454.6 | >9354.95 |
| F159.11 | DRVHPVHAGPIAPGQM | HIV.gag.245 | HIV | | 801.0 | | >5929.27 | 41666.7 | >8413.34 |
| F159.40 | DRVHPVHAGPIAPGQMRE | HIV.gag.245 | HIV | | 332.5 | | >5303.3 | 35714.3 | >7367.88 |
| F159.09 | DRVHPVHAGPIAPGQMREPR | HIV.gag.245 | HIV | | 1110.4 | | >4743.42 | 31250.0 | >6555.62 |
| F170.01 | DTEVHNVWATQACVPTDPNP | HIV.env.6 | HIV | | 753.1 | >7694.84 | >216.51 | 3104.3 | |
| F169.02 | EGLIHSQRRQDILDL | HIV.nef.98 | HIV | | 26418.8 | 2014.4 | >2598.08 | >83333.33 | |
| F170.06 | EPFRDYVDRFYKTLRAEQAS | HIV.gag.30 | HIV | | 15.2 | 1389.5 | 104.7 | 3733.3 | |
| F170.05 | IYKRWIILGLNKIVRMYSPV | HIV.gag.27 | HIV | | 1272.4 | >15389.68 | >435.44 | 5120.9 | |
| F170.02 | PKISFEPIPIHYCAPAGFAI | HIV.env.20 | HIV | | 228.9 | 2267.5 | 163.6 | 1487.1 | |
| F169.01 | TAATNAACAWLEA | HIV.nef.48 | HIV | | 9806.6 | >94868.33 | >2598.08 | 83612.0 | |
| F170.04 | TNNPPIPVGEIYKRWIILGL | HIV.gag.26 | HIV | | 13.9 | 87.8 | 298.0 | 155.7 | |
| F170.03 | VWGIKQLQARVLAVERYLKD | HIV.env.54 | HIV | | 2.3 | 3742.3 | >435.44 | 1078.5 | |
| 27.0374 | AVQMAVFIHNFKRKG | HIV1.pol.917 | HIV1 | | 1589.4 | | 1641.6 | | |
| 27.0299 | DQQLLGIWGCSGKLI | HIV1.env.755 | HIV1 | | 173.7 | | >22500 | | |
| 27.0287 | DQSLKPCVKLTPLCV | HIV1.env.126 | HIV1 | | 666.6 | | >22500 | | |
| 27.0284 | EDIISLWDQSLKPCV | HIV1.env.119 | HIV1 | | 103.6 | | >23985.08 | | |
| 190.20 | ERFAVNPGLLETSEGC | HTV1.gp.48 | HIV1 | | 250.0 | 900000.0 | 450000.0 | 7142.9 | |
| 190.15 | GARASVLSGGELDKWE | HIV1.gp.1 | HIV1 | | 833.3 | 900000.0 | 450000.0 | 500000.0 | |
| 190.16 | GGELDKWEKIRLRPGG | HIV1.gp.9 | HIV1 | | 2500.0 | 900000.0 | 450000.0 | 500000.0 | |
| RS-21 | GP41584-609 | HIV1.gp. | HIV1 | | 1250.0 | | | | |
| 27.0320 | IGGIGGFIKVRQYDQ | HIV1.pol.127 | HIV1 | | 22026.7 | | >18766.3 | | |
| 181.11 | ILKALGPAATLEEMMT(200.11 ) | HIV1 | HIV1 | | 22.1 | 900000.0 | 22500.0 | 1562.5 | |
| 200.10 | INEEAAEWERVHPVHA | HIV1.gp.73 | HIV1 | | 625.0 | 31819.8 | 450000.0 | 10000.0 | |
| 27.0348 | IQKLVGKLNWASQIY | HIV1.pol.436 | HIV1 | | 191.1 | | >16112.58 | | |
| 27.0286 | ISLWDQSLKPCVKLT | HIV1.env.122 | HIV1 | | 1269.7 | | >22500 | | |
| 190.19 | IVWASRELERFAVNPG | HIV1.gp.33 | HIV1 | | 2500.0 | 10392.3 | 450000.0 | 3846.2 | |
| 200.17 | IYKRWIILGLNKIVRN | HIV1.gp.129 | HIV1 | | 2500.0 | 900000.0 | 450000.0 | 500000.0 | |
| F091.13 | KQIINMWQEVGKAMYA | HIV1.env.428 | HIV1 | | 1813.7 | | 2477.2 | 98.1 | |
| 200.04 | KVVEEKAFSPEVIPMF | HIV1.gp.25 | HIV1 | | 2500.0 | 900000.0 | 450000.0 | 3125.0 | |
| 190.08 | LGKIWPSYKGRPGNFL | HIV1.gp.57 | HIV1 | | 2500.0 | 31819.8 | 9000.0 | 151.5 | |
| F091.08 | LKQIVKKLREQFGNNK | HIV1.env.342 | HIV1 | | 20412.4 | | >64951.91 | 62500.0 | |
| 27.0300 | LLGIWGCSGKLICTT | HIV1.env.758 | HIV1 | | 193.7 | | >23985.08 | | |
| 27.0300 | LSIVNRVRQGYSPLS | HIV1.env.877 | HIV1 | | 1157.1 | | 2793.9 | | |
| 200.01 | PIVQNLQGQMVHQAIS | HIV1.gp.1 | HIV1 | | 625.0 | 900000.0 | 450000.0 | 500000.0 | |
| F091.24 | PLGVAPTKAKRRVVQR | HIV1.env.671 | HIV1 | | 50000.0 | | >64951.91 | 12659.2 | |
| | PSLQTGSEELRSLYNT | HIV1.gp.65 | HIV1 | | 50000.0 | 12480.8 | 450000.0 | 500000.0 | |
| 190.23 F091.16 | QARILAVERYLKDQQL | HIV1.env.582 | HIV1 | | 1740.8 | | >64951.91 | 8751.8 | |
| 27.0352 | QGQWTYQIYQEPFKN | HIV1.pol.516 | HIV1 | | 4594.9 | | 13146.6 | | |
| 190.13 | QKQEPIDKELYPLTSL | HIV1.gp.97 | HIV1 | | 125.0 | >30000 | 450000.0 | 250.0 | |
| | QMAVFIHNFKRKGGI | HIV1.pol.919 | HIV1 | | 10331.1 | | 14711.1 | | |
| 27.0375 F091.07 | RIQRGPGRAFVTIGKL | HIV1.env.315 | HIV1 | | 14433.8 | | >64951.91 | >91287.09 | |
| 190.22 | RQILGQLQPSLQTGSE | HIV1.gp.57 | HIV1 | | 227.3 | 900000.0 | 7500.0 | 1000.0 | |
| F091.04 | SLKPCVKLTPLCVTLN | HIV1.env.115 | HIV1 | | 319.7 | | 26892.6 | 4247.1 | |
| F091.26 | SLWDQSLKPCVKLTPL | HIV1.env.825 | HIV1 | | 4902.9 | | >6495191 | >91287.09 | |
| 27.0359 | SQIIEQLIKKEKVYL | HIV1.pol.701 | HIV1 | | 5986.4 | | >16112.58 | | |
| F091.22 | SQNQQEKNEQELLELD | HIV1.env.654 | HIV1 | | 50000.0 | | >64951.91 | >91287.09 | |
| F091.11 | SSGGDPEIVMHSFNCG | HIV1.env.369 | HIV1 | | 25000.0 | | >64951.91 | >91287.09 | |
| 27.0334 | STKWRKLVDFRELNK | HIV1.pol.247 | HIV1 | | 618.4 | 1547.7 | >16112.58 | | |
| F091.12 | TITLPCRIKQFINMWQE | HIV1.env.413 | HIV1 | | 50000.0 | | 1636.6 | 826.7 | |
| 27.0292 | TVYYGVPVWKEATTT | HIVl.env.49 | HIV1 | | 3603.9 | | 3900.2 | | |
| F091.23 | VKIEPLGVAPTKAKRR | HIV1.env.667 | HIV1 | | 1796.1 | | 47434.2 | 729.3 | |
| 27.0301 | VLSIVNRVRQGYSPL | HIV1.env.876 | HIV1 | | 1624.8 | | 3241.0 | | |
| 27.0356 | VNIVTDSQYALGIIQ | HIV1.pol.675 | HIV1 | | 80.9 | | 4961.6 | | |
| F091.03 | WDQSLKPCVKLTPLCV | HIV1.env.112 | HIV1 | | 637.1 | | >64951.91 | >91287.09 | |
| 27.0335 | WRKLVDFRELNKRTQ | HIV1.pol.250 | HIV1 | | 4543.9 | | >16112.58 | | |
| F160.45 | STORKUSP45 | HPV16.e7. | HPV16 | A | | | >25339.89 | 237.2 | |
| 106.00 | APYTSTLLPPELSETP | HSV.gD.245 | HSV | | 555.6 | >45000 | 450000.0 | 25000.0 | |
| F095.05 | FRQLVHFVRDFAQLL | HSV | HSV | | | | | | |
| AP30 | SLKMADPNRFRGKDLP | HSV | HSV | | 1666.7 | 900000.0 | 450000.0 | 25000.0 | |
| 605.14 | ACRVNHVTLSQPKIVK | HumanB2-µglobulin.79 | HumaB2-µglobubin | | 5000.0 | 900000.0 | 5625.0 | 4166.7 | |
| 530.08 | CKKIPKVEMSDMSFSK | HumanB2-µglobulin.43 | HumanB2-µglobulin | | 50000.0 | 900000.0 | 7500.0 | 8333.3 | |
| 605.03 | HPAENGKSNFLNCYVS | HumanB2-µglobulin.13 | HumanB2-µglobulin | | 833.3 | 900000.0 | 1500.0 | 5555.6 | |
| 530.06 | HPPHIEIQMLKNGKKI | HumanB2-µglobulin.31 | HumanB2-µglobulin | | 1000.0 | 900000.0 | 450000.0 | 142.9 | |
| 530.01 | IQKTPQIQVYSRHPPE | HumanB2-µglobulin.1 | HumanB2-µglobulin | | 50000.0 | >30000 | 450000.0 | 500000.0 | |
| 530.07 | IQMLKNGKKIPKVEMS | HumaB2-µglobulin.37 | HumanB2-µglobulin | | 50000.0 | 1875.0 | 450000.0 | 50000.0 | |
| 605.01 | IQRTPKIQVYSRHPAE | HumanB2-µglobulin.1 | HumanB2-µglobulin | | 1666.7 | 900000.0 | 375.0 | 500000.0 | |
| 530.02 | IQVYSRHPPENGKPNI | HumanB2-µglobulin.7 | HumanB2-µglobulin | | 2500.0 | 900000.0 | 535.7 | 500000.0 | |
| 530.04 | KPNILNCYVTQFHPPH | HumauB2-µglobulin.19 | HumanB2-µglobulin | | 50000.0 | >30000 | 15000.0 | 147.1 | |
| 530.10 | SFSKDWSFYILAHTEF | HumanB2-µglobulin.55 | HumanB2-µglobulin | | 2500.0 | 900000.0 | 343.5 | 500000.0 | |
| 605.10 | SFSYDWSFYLLYYTEF | HumanB2-µglobulin.55 | HumanB2-µglobulin | | 50000.0 | 900000.0 | 833.3 | 500000.0 | |
| 605.11 | SFYLLYYTEFTPTEKD | HumanB2-µglobulin.61 | HumanB2-µglobulin | | 50000.0 | 4647.6 | 957.5 | 10000.0 | |
| 530.14 | TETDTYACRVKHDSMA | HumanB2-µglobulin.79 | HumanB2-µglobulin | | 50000.0 | 900000.0 | 450000.0 | 500000.0 | |
| 530.09 | VEMSDMSFSKDWSFYI | HumanB2-µglobulin.49 | HumanB2-µglobulin | | 2500.0 | 0.0 | 22500.0 | 500000.0 | |
| 544.02 | YGSDTITLPCRIKQFINMWQE | HumanB2-µglobulin.410 | HumanB2-µglobulin | | 50000.0 | 900000.0 | 1607.1 | 568.2 | |
| JR-01 | PEFLEQRRAAVDTYC | IEBs2 | IEBs2 | | 5000.0 | | 450000.0 | 500000.0 | |
| F073.01 | MRGSHHHHHHGSVD-II72-216 | Invariantchain | Ii | | | | 375.0 | 57.3 | |
| 734.01 | FVNQHLSGSHLVEALYLVSG- CONH2 | InsulinA | InsulinA | | 38.6 | >30000 | 6495.2 | | |
| 68.0002 | DLVLSIALSVGCTGA | Kallikrein2.3 | Kallikrein2 | | 1196.8 | | 73340.2 | | |
| 68.0005 | GQRVPVSHSFPHPLY | Kallikrein2.87 | Kallikrein2 | | 1563.3 | | >142302.49 | | |
| 68.0011 | HDLMLLRLSEPAKIT | Kallikrein2.119 | Kallikrein2 | | 15.7 | 1405.6 | 17231.4 | | |
| 68.0008 | HPLYNMSLLKHQSLR | Kallikrein2.98 | Kallikrein2 | | 232.1 | 13006.7 | 2806.6 | | |
| 68-0003 | HPQWVLTAAHCLKKN | Kallikrein2.56 | Kallilkein2 | | 22.0 | 1102.9 | 4922.1 | | |
| 68.0018 | KPAVYTKVVHYRKWI | Kallikrein2.239 | Kallikrein2 | | 5000.0 | | 8060.5 | | |
| 68.0140 | LHLLSNDMCARAYSE | Kallikrein2.176 | Kallikrein2 | | 2103.5 | 938.1 | 24058.1 | | |
| 68.0001 | MWDLVLSIALSVGCT | Kallikrein2.1 | kallikrein2 | | 205.2 | | 10386.4 | | |
| | NGVLQGITSWGPEPC | Kallikrein2.220 | Kallikrein2 | | 1092.9 | | 47434.2 | | |
| 68.0017 68.0009 | NMSLLKHQSLRPDED | Kallikrein2.102 | Kallikrein2 | | 3131.1 | | >183711.73 | | |
| 68.0015 | PEEFLRPRSLQCVSL | Kallikrein2.162 | Kallikrein2 | | 2001.1 | | >150000 | | |
| 68.0007 | PHPLYNMSLLKHQSL | Kallikrein2.97 | Kallikrein2 | | 19078.6 | | 4607.2 | | |
| 68.0016 | PRSLQCVSLHLLSND | Kallikrein2.168 | Kallikrein2 | | 1111.3 | | 90000.0 | | |
| 68.0004 | QWVLTAAHCLKKNSQ | Kallikrein2.58 | Kallikrein2 | | 895.1 | | >142302.49 | | |
| 68.0006 | RVPVSHSFPHPLYNM | Kallikrein2.89 | Kallikrein2 | | 66.7 | | >90000 | | |
| 68.0010 | SHDLMLLRLSEPAKI | Kallikrein2.118 | Kallikrein2 | | 55.9 | 2396.3 | 12622.4 | | |
| F090.02 | ATGFKQSSKALQRPV | Leukemia.15 | Leukemia | | 4921.1 | | >22786.64 | | |
| F090.01 | IVHSATGFKQSSKALQRPVAS DFEP | Leukemia.25 | Leukemia | | 1458.6 | | 5844.2 | | |
| F071.29 | IKYNGEEYLILSARD | M.leprae.79 | M.leprae | | | | 31980.1 | | |
| F071.26 | IYSKYGGTEIKYNGE | M.leprae.70 | M.leprae | | | | 12519.3 | | |
| F118.06 | LVIPENAKEKPQ | M.leprae.28 | M.leprae | | | | | | |
| F118.02 | LVIPENAKEKPQEGT | M.leprae.28 | M.leprae | | | | | | |
| F071.12 | LVIPENAKEKPQBGT | M.leprae.28 | M.leprae | | | | >64951.91 | | |
| F071.30 | NGEEYLILSARDVLA | M.leprae.82 | M.leprae | | | | 986.7 | | |
| F071.11 | PSGLVIPENAKEKPQ | M.leprae.25 | M.leprae | | | | >64951.91 | | |
| F071.22 | RIPVDVSEGDIVIYS | M.leprae.58 | M.leprae | | | | 45927.9 | | |
| 581.02 | RKHRIEDAVRNAKAAVEEGI VAG | M.leprae.437 | M.leprae | | 50000.0 | 871.5 | 5625.0 | 12500.0 | |
| F071.24 | SEGDIVIYSKYGGTE | M.leprae.64 | M.leprae | | | | >64951.91 | | |
| 572.03 | TLLQAAPALDKY | M.leprae.464 | M.leprae | | 135.1 | 900000.0 | 450000.0 | 25000.0 | |
| F071.03 | VAKVKIKPLEDKILV | M.leprae.1 | M.leprae | | | | 61237.2 | | |
| F071.04 | VKIKPLEDKILVQAG | M.leprae.4 | M.leprae | | | | 44126.1 | | |
| F071.17 | VVAVGPGRWDEDGAK | M.leprae.43 | M.leprae | | | | >64951.91 | | |
| 581.01 | YEKIGAELVKEVAKKTDDVA G | M.leprae.112 | M.leprae | | 27.8 | 900000.0 | 9000.0 | 500000.0 | |
| F160.21 | EKIWEELSVLEVFEGRED | MAGE.219 | MAGE | | | | 10741.3 | 320.2 | |
| F160.20 | ETSYVKNLHHMVKISGG | MAGE.280 | MAGE | | | | >16678.11 | 958.3 | |
| 58.0062 | EEKIWEDLSMLEVFE | MAGE2.218 | MAGE2 | A | | 257.2 | | | |
| 58.0057 | FPDLESDFQAAISRK | MAGE2.98 | MAGE2 | A | | 374.9 | | | |
| 58.0058 | LESVLRDCQDFFPVI | MAGE2.136 | MAGE2 | A | | 384.9 | | | |
| 58.0064 | MQDLVQDNYLEYRQV | MAGE2.247 | MAGE2 | A | | 118.6 | | | |
| 58.0043 | PRKLLMEDLVQENYL | MAGE2.242 | MAGE2 | A | | 614.3 | | | |
| 58.0065 | QDLVQEDYLEYRQVP | MAGE2.248 | MAGE2 | A | | 599.8 | | | |
| 58.0059 | QLVFGIDVVEVVPIS | MAGE2.159 | MAGE2 | A | | 770.1 | | | |
| 58.0071 | EEKIWEDLSVLEVFE | MAGE3.218 | MAGE3 | A | | 657.1 | | | |
| 58.0068 | LGSVVGDWQYFFPVI | MAGE3.136 | MAGE3 | A | | 89.9 | | | |
| 58.0074 | QHFVQEDYLEYRQVP | MAGE3.248 | MAGE3 | A | | 138.0 | | | |
| 58.0073 | TQHFVQDNYLEYRQV | MAGE3.247 | MAGE3 | A | | 204.2 | | | |
| F167.06 | LLKYRAREPVTKAEMLGSVV GNWQ | Mage6.121 | Mage6 | | 13.2 | 465.3 | 725.5 | 300.4 | |
| F167.08 | MVKISGGPRISYPLLHEWALR EGEE | Mage6.290 | Mage6 | | 319.4 | 7108.8 | >4242.64 | 14771.1 | |
| F167.07 | QVPGSDPACYEFLWGPRALIE TSY | Mage6.260 | Mage6 | | 35.3 | >11868.85 | >2772.68 | >20412.41 | |
| F160.27 | VGNWQYFFPVIFSKASDSL | MAGE6.140 | MAGE6 | | | | 5074.4 | 566.8 | |
| 520.09 | YKLNFYFDLLRAKL | Malaria | Malaria | | 1250.0 | 19655.6 | 22500.0 | 500000.0 | |
| 520.07 | YLDNIKDNVGKMED | Malaria | Malaria | | 50000.0 | >45000 | 5000.0 | 1562.5 | |
| F160.15 | AAGIGILTVILGVL | MART1.27 | MART1 | | | | >19300.26 | 92.9 | |
| 825.10 | AC- ASQKRPSQRHGSKYLATAST | MBP.1 | MBP | | 50000.0 | 900000.0 | 500.0 | 25000.0 | |
| 765.17 | AC- ASQKRPSQRSKYLATASTMD | MBP.1 | MBP | | 1178.5 | >30000 | 1527.4 | 25000.0 | |
| F006.16 | ANPVVHFFKNIVTPR | MBP.85 | MBP | A | | | | | |
| 825.01 | ASQKRPSQRHGSKYLATAST | MBP.1 | MBP | | 50000.0 | 900000.0 | 2368.4 | 50000.0 | |
| 765.15 | AYDAQGTLSKIFKLGGRDSR | MBP.141 | MBP | | 20.5 | 25980.8 | 2665.6 | 303.5 | |
| F006.14 | DENPVVHFFKN | MBP.84 | MBP | | | | | | |
| F006.13 | DENPVVHFFKNI | MHP.84 | MBP | | | | | | |
| F006.12 | DENPVVHFFKNIV | MBP.84 | MBP | | | | | | |
| F006.11 | DENPVVHFFKNIVT | MBP.84 | MBP | | | | | | |
| F006.10 | DENPVVHFFKNIVTPR | MBP.84 | MBP | | | | | | |
| F006.09 | DENPVVHFFKNIVTPRT | MBP.84 | MBP | | | | | | |
| F006.01 | DENPVVHFFKNIVTPRTPP | MBP.84 | MBP | | | | | | |
| F006.0202 | DENPVVHFFKNIVTPRTPPY | MBP.84 | MBP | | | | | | |
| F006.03 | DENPVVHFFRNIVTPRTPPY | MBP.84 | MBP | A | | | | | |
| F006.17 | EAPVVHFFKNIVTPR | MBP.85 | MBP | A | | | | | |
| F006.18 | ENAVVHFFKNIVTPR | MBP.85 | MBP | A | | | | | |
| F006.19 | ENPAVHFFKNIVTPR | MBP.85 | MBP | A | | | | | |
| F038.01 | ENPKVHFFKNIVTPR | MBP.85 | MBP | A | | | | | |
| F006.20 | ENPVAHFFKNIVTPR | MBP.85 | MBP | A | | | | | |
| F038.02 | ENPVKHFFKNIVTPR | MBP.85 | MBP | A | | | | | |
| F006.21 | ENPVVAFFKNIVTPR | MBP.85 | MBP | A | | | | | |
| F038.03 | ENPVVAFFKNIVTPR | MBP.85 | MBP | A | | | | | |
| F038.06 | ENPVVDFFKNIVTPR | MBP.85 | MBP | A | | | | | |
| F038.05 | ENPVVFFFKNIVTPR | MBP.85 | MBP | A | | | | | |
| F006.22 | ENPVVHAFKNIVTPR | MBP.85 | MBP | A | | | | | |
| F038.07 | ENPVVHAFKNIVTPR | MBP.85 | MBP | A | | | | | |
| F095.08 | ENPVVHAFRNIVTPR | MBP.85 | MBP | A | | | | | |
| F038.09 | ENPVVHDFKNIVTPR | MBP.85 | MBP | A | | | | | |
| F006.23 | ENPVVHFAKNIVTPR | MBP.85 | MBP | A | | | | | |
| F095.06 | ENPVVHFARNIVTPR | MBP.85 | MBP | A | | | | | |
| F006.24 | ENPVVHFFANIVTPR | MBP.85 | MBP | A | | | | | |
| F038.14 | ENPVVHFFANIVTPR | MBP.85 | MBP | A | | | | | |
| Cr-8 | ENPVVHFFANIVTPRTP | MBP.83 | MBP | A | | | | 8.6 | |
| F038.15 | ENPVVHFFDNIVTPR | MBP.85 | MBP | A | | | | | |
| F038.16 | ENPVVHFFHNIVTPR | MBP.85 | MBP | A | | | | | |
| F006.25 | ENPVVHFFKAIVTPR | MBP.85 | MBP | A | | | | | |
| Cr-9 | ENPVVHFFKAIVTPRTP | MBP.83 | MBP | A | | | | 11.4 | |
| F038.19 | ENPVVHFFKKIVTPR | MBP.85 | MBP | A | | | | | |
| F006.26 | ENPVVHFFKNAVTPR | MBP.85 | MBP | A | | | | | |
| Cr-10 | ENPVVHFFKNAVTPRTP | MBP.83 | MBP | A | | | | 10.7 | |
| F006.27 | ENPVVHFFKNIATPR | MBP.85 | MBP | A | | | | | |
| F006.28 | ENPVVHFFKNIVAPR | MBP.85 | MBP | A | | | | | |
| Cr-11 | ENPVVHFFKNIVAPRTP | MBP.83 | MBP | A | | | | 13.1 | |
| F006.29 | ENPVVHFFKNIVTAR | MBP.85 | MBP | A | | | | | |
| F006.30 | ENPVVHFFKNIVTPA | MBP.85 | MBP | A | | | | | |
| F006.36 | ENPVVHFFKNIVTPA | MBP.85 | MBP | A | | | | | |
| F006.15 | ENPVVHFFKNIVTPR | MBP.85 | MBP | | | | | | |
| Cr-7 | ENPVVHFFKNIVTPRTP | MBP.83 | MBP | | | | | 10.0 | |
| F006.04 | ENPVVHFFKNIVTPRTPPY | MBP.85 | MBP | | | | | | |
| F038.20 | ENPVVHFFKNKVTPR | MBP.85 | MBP | A | | | | | |
| F038.17 | ENPVVHFFLNIVTPR | MBP.85 | MBP | A | | | | | |
| F038.18 | ENPVVHFFRNIVTPR | MBP.85 | MBP | A | | | | | |
| F038.13 | ENPVVHFKKNIVTPR | MBP.85 | MBP | A | | | | | |
| F038.10 | ENPVVHHFKNIVTPR | MBP.85 | MBP | A | | | | | |
| F095.10 | ENPVVHHFRNIVTPR | MBP.85 | MBP | A | | | | | |
| F038:11 | ENPVVHLFKNIVTPR | MBP.85 | MBP | A | | | | | |
| F095.09 | ENPVVHLFRNIVTPR | MBP.85 | MBP | A | | | | | |
| F038.12 | ENPVVHWFKNIVTPR | MBP.85 | MBP | A | | | | | |
| F095.12 | ENPVVHYFANIVTPR | MBP.85 | MBP | A | | | | | |
| F095.11 | ENPVVHYFHNIVTPR | MBP.85 | MBP | A | | | | | |
| F038.08 | ENPVVHYFKNIVTPR | MBP.85 | MBP | A | | | | | |
| F095.13 | ENPVVHYFLNIVTPR | MBP.85 | MBP | A | | | | | |
| F095.07 | ENPVVHYFRNIVTPR | MBP.85 | MBP | A | | | | | |
| F038.04 | ENPVVKFFKNIVTPR | MBP.85 | MBP | A | | | | | |
| 613.02 | FFKNIVTPFFKNIVTP | MBP. | MBP | A | 833.3 | | 5000.0 | 6250.0 | |
| 825.07 | FSWGAEGQRPGFGYGGRASD | MBP.114 | MBP | | 50000.0 | 900000.0 | 2045.5 | 500000.0 | |
| 765.13 | GFGYGGRASDYKSAHKGFKG | MBP.121 | MBP | | 50000.0 | 900000.0 | 15000.0 | 500000.0 | |
| 825.06 | GKGRGLSLSRFSWGAEGQRP | MBP.104 | MBP | | 833.3 | 900000.0 | 725.8 | 2631.6 | |
| 825.04 | GSGKDSHHPARTAHYGSLPQ | MBP.55 | MBP | | 50000.0 | 900000.0 | 45000.0 | 500000.0 | |
| 765.03 | HARHGFLPRHRDTGILDSIG | MBP.21 | MBP | | 50000.0 | >30000 | 450000.0 | 500000.0 | |
| F006.321 | HFFKNIVTPRTPPY | MBP.90 | MBP | | | | | | |
| F006.322 | HFFKNIVTPRTPPY | MBP.90 | MBP | | | | | | |
| 765.16 | IFKLGGRDSRSGSPMARR | MBP.151 | MBP | | 29.2 | 900000.0 | 22500.0 | 500000.0 | |
| 765.06 | KRGSGKDSHTRTTHYGSLPQ | MBP.51 | MBP | | 5000.0 | >30000 | 11250.0 | 25000.0 | |
| 765.08 | KSQHGRTQDENPVVHFFKNI | MBP.71 | MBP | | 5000.0 | >30000 | 2500.0 | 6250.0 | |
| F006.31 | NPVVHFFKNIVT | MBP.86 | MBP | | | | | | |
| F006.37 | NPVVHFFKNIVTPA | MBP.86 | MBP | A | | | | | |
| F006.34 | NPVVHFFKNIVTPR | MBP.86 | MBP | | | | | | |
| F006.05 | NPVVHFFKNIVTPRTPPY | MBP.86 | MBP | | | | | | |
| F006.39 | PVVHFFKNIVT | MBP.87 | MBP | | | | | | |
| F006.38 | PVVHFFKNIVTPA | MBP.87 | MBP | A | | | | | |
| F006.35 | PVVHFFKNIVTPR | MBP.87 | MBP | | | | | | |
| F006.06 | PVVHFFKNIVTPRTPPY | MBP.87 | MBP | | | | | | |
| 825.11 | QKSHGRTQDENPVVHFFKNI | MBP.74 | MBP | | 1250.0 | 900000.0 | 900.0 | 1562.5 | |
| F121.03 | RASDYKSAHKGFKGVDAQGT | MBP.131 | MBP | | | | | | |
| F121.02 | RASDYKSAHKGLKGHDAQGT | MBP.131 | MBP | A | | | | | |
| 825.02 | RDTGILDSIGRFFGGDRGAP | MBP.33 | MBP | | 50000.0 | 1822.7 | 450000.0 | 500000.0 | |
| 765.04 | RDTGILDSIGRFFSGDRGAP | MBP.31 | MBP | | 50000.0 | 748.6 | 45000.0 | 25000.0 | |
| 825.03 | RFFGGDRGAPKRGSGKDSHH | MBP.43 | MBP | | 50000.0 | 45000.0 | 5000.0 | 500000.0 | |
| 765.05 | RFFSGDRGAPKRGSGKDSHT | MBP.41 | MBP | | 50000.0 | 7500.0 | 1046.8 | >16666.67 | |
| 825.05 | RTAHYGSLPQKSHGRTQDEN | MBP.65 | MBP | | 50000.0 | 900000.0 | 450000.0 | 500000.0 | |
| 825.09 | VDAQGTLSKIFKLGGRDSRS | MBP.144 | MBP | | 25.4 | 1383.0 | 9000.0 | 314.5 | |
| F112.01 | VDAQGTLSKIFKLGGRDSRS | MBP.144 | MBP | | | | | | |
| F112.04 | VDAQGTLSKLFKLGGRDSRS | MBP.144 | MBP | A | | | | | |
| F112.03 | VDAQGTLSRIFKLGGRDSRS | MBP.144 | MBP | A | | | | | |
| F006.08 | VHFFKNIVTPRTPPY | MBP.89 | MBP | | | | | | |
| 765.10 | VTPRTPPPSQGKGRGLSLSR | MBP.91 | MBP | | 50000.0 | 900000.0 | 5625.0 | 16666.7 | |
| F006.07 | VVHFFKNIVTPRTPPY | MBP.88 | MBP | | | | | | |
| 765.14 | YKSAHKGFKGAYDAQGTLSK | MBP.131 | MBP | | 3535.5 | >30000 | 86.8 | 16666.7 | |
| 825.08 | YKSAHKGFKGVDAQGTLSKI | MBP.134 | MBP | | 70.4 | 900000.0 | 5000.0 | 25000.0 | |
| 9.00 | ANERADLIAYLKQATK | Moth.CytochromeC.88 | Moth | | 1666.7 | >30000 | 450000.0 | 8333.3 | |
| | EFVVEFDLPGIKA | Mouse.Lysoyzme.38 | Mouse | | 50000.0 | 1773.3 | 330.9 | 500000.0 | |
| 847.02 593.02 | VITAFNEGLK | Mouse.Hemoglobin.67 | Mouse | | 50000.0 | 900000.0 | 22500.0 | 500000.0 | |
| 847.01 | YFFVVEFDLPGIKA | Mouse.Lysoyzme.38 | Mouse | | 50000.0 | | 40.9 | 2941.2 | |
| 753.03 | IAFNSGLEPGVVAEK-NH2 | MT.451 | MT | | 50000.0 | 900000.0 | 450000.0 | 500000.0 | |
| 753.02 | LLPLLEKVIGAGKPL-NH2 | MT.231 | MT | | 1666.7 | 900000.0 | 22500.0 | 12500.0 | |
| 829.01 | YKTIAYDEEARR | MT3 | MT | | 50000.0 | 142.8 | 22500.0 | 500000.0 | |
| 831.01 | TWQRDGEDQTQDTELVETRP AG | Naturallyprocessed | Naturallyprocessed | | 555.6 | | 592.1 | 1111.1 | |
| F009.04 | VDDTLFVRFDSDAASPREEPR | Naturallyprocessed | Naturallyprocessed | | | | 122.3 | 3194.4 | |
| F009.01 | VDDTLFVRFDSDATSPRKEPR | Naturallyprocessed | Naturallyprocessed | | | | 37.6 | 6201.7 | |
| F009.06 | VDDTQFVRFDSDAASPREEPR | Naturallyprocessed | Naturallyprocessed | | | | 68.1 | 2094.3 | |
| F009.02 | VDDTQFVRFDSDAASPRMAP R | Naturallyprocessed | Naturallyprocessed | | | | 44.6 | 1035.6 | |
| F009.05 | VDDTQFVRFDSDAASPRTEPR | Naturallyprocessed | Naturallyprocessed | | | | 93.6 | 1737.6 | |
| 536.00 | AHAAHAAHAAHAAHAA | Ova | Ova | A | 35.7 | 900000.0 | 22500.0 | 25000.0 | |
| N-3 | AVHAAHAEINEAGR | Ova.326 | Ova | | | | | | |
| 151.00 | HIATNAVLFFGR | Ova.370 | Ova | | 2500.0 | 90000.0 | 450000.0 | 500000.0 | |
| 144.01 | ISQAAHAAHAEINE | Ova.323 | Ova | A | | | | | |
| 84.04 | ISQADHAAHAEINE | Ova.323 | Ova | A | | | | | |
| 85.04 | ISQAVEAAHAEINE | Ova.323 | Ova | A | | | | | |
| 92.06 | ISQAVHAAHAEDNE | Ova.323 | Ova | A | | | | | |
| 92.05 | ISQAVHAAHAEIIE | Ova.323 | Ova | A | | | | | |
| 91.02 | ISQAVHAAHAQINE | Ova.323 | Ova | A | | | | | |
| 90.05 | ISQAVHAALAEINE | Ova.323 | Ova | A | | | | | |
| 144.08 | ISQAVHAANAEINE | Ova.323 | Ova | A | | | | | |
| 90.01 | ISQAVHAARAEINE | Ova.323 | Ova | A | | | | | |
| 747.01 | LKISQAVHAAHAEIN | Ova.321 | Ova | | | | | | |
| 705.45 | MVYLGAKDSTRTQINKVVRF | Ova.40 | Ova | | 384.6 | 900000.0 | 11250.0 | 8333.3 | |
| 521.00 | NVMEERKIKVYLPRM | Ova.271 | Ova | | 1666.7 | 24053.5 | 45000.0 | 25000.0 | |
| 560.04 | VHAAHAEINVHAAHA | Ova.327 | Ova | A | 1250.0 | >45000 | 500.0 | 7142.9 | |
| 560.03 | VHAAHAVHAAHAEIN | Ova.327 | Ova | A | 41.7 | 900000.0 | 22500.0 | 25000.0 | |
| 560.05 | VHAAHAVHAAHAVHA | Ova.327 | Ova | A | 12.2 | 900000.0 | 15000.0 | 1351.4 | |
| 594.03 | YTYTVHAAHAYTYT | Ova | Ova | | 45.5 | 5669.5 | 5625.0 | 25000.0 | |
| 112.06 | YTYTVHAAHAYTYT | Ova | Ova | | 45.5 | | 5625.0 | 25000.0 | |
| 58.0082 | LIRVEGDLRVEYLDD | p53.194 | p53 | A | | 8.7 | | | |
| 58.0081 | QHLIRVDGNLRVEYL | p53.192 | p53 | A | | 64.2 | | | |
| 58.0091 | RFEMFRDLNEALELK | p53.337 | p53 | A | | 876.0 | | | |
| 68.0019 | AAPLLLARAASLSLG | PAP.3 | PAP | | 6.8 | 35410.0 | 782.7 | | |
| 68.0025 | AKELKFVTLVFRHGD | PAP.32 | PAP | | 787.4 | 30000.0 | 4404.2 | | |
| 68.0052 | ALDVYNGLLPPYASC | PAP.299 | PAP | | 18.4 | | 2730.7 | | |
| 68.0020 | APLLLARAASLSLGF | PAP.4 | PAP | | 8.4 | 56250.0 | 1138.5 | | |
| 68.0024 | DRSVLAKELKFVTLV | PAP.27 | PAP | | 704.9 | | 3203.4 | | |
| 68.0030 | DRTLMSAMTNLAALF | PAP.110 | PAP | | 97.2 | 64285.7 | 71.8 | | |
| 68.0051 | DTTVSGLQMALDVYN | PAP.290 | PAP | | 171.2 | | 24885.1 | | |
| 68.0056 | FAELVGPVIPQDWST | PAP.356 | PAP | | 12.5 | | 16379.5 | | |
| 68.0028 | FGQLTQLGMEQHYEL | PAP.67 | PAP | | 2258.5 | | 18057.9 | | |
| 68.0047 | GGVLVNEILNHMKRA | PAP.260 | PAP | | 2165.4 | 699.6 | 2020.4 | | |
| 68.0156 | GPVIPQDWSTECMTT | PAP.361 | PAP | | | 52098.0 | | | |
| 68.0034 | GVSIWNPILLWQPIP | PAP.128 | PAP | | 44.2 | 56250.0 | 58094.8 | | |
| 68.0038 | ILLWQPIPVHTVPLS | PAP.135 | PAP | | 45.1 | >67840.05 | 957.0 | | |
| 68.0048 | IPSYKKLIMYSAHDT | PAP.277 | PAP | | 9.9 | 9727.6 | 2870.9 | | |
| 68.0046 | KSRLQGGVLVNEILN | PAP.255 | PAP | | 361.8 | | >129903.81 | | |
| 68.0053 | LDVYNGLLPPYASCH | PAP.300 | PAP | | 15.4 | | 1958.8 | | |
| 68.0050 | LIMYSAHDTTVSGLQ | PAP.283 | PAP | | 4496.4 | | 13a.a | | |
| 68.0023 | LLFFWLDRSVLAKEL | PAP.21 | PAP | | 2.9 | 63 | 14.8 | | |
| 68.0042 | LPSWATEDTMTKLRE | PAP.223 | PAP | | 20274.0 | | 5472.4 | | |
| 68.0043 | LRELSELSLLSLYGI | PAP.235 | PAP | | 654.7 | | 2084.6 | | |
| 68.0044 | LSELSLLSLYGIHKQ | PAP.238 | PAP | | 492.2 | >67840.05 | 8715.3 | | |
| 68.0040 | LSGLHGQDLFGIWSK | PAP.194 | PAP | | 147.7 | | >129903.81 | | |
| 68.0045 | LSLLSLYGNKQKEK | PAP.241 | PAP | | 656.4 | >67840.05 | 25000.0 | | |
| 68.0153 | LTELYFEKGEYFVEM | PAP.315 | PAP | | 2248.9 | 591.8 | 45288.4 | | |
| 68.0031 | MSAMTNLAALFPPEG | PAP.114 | PAP | | 1756.8 | | 3935.5 | | |
| 68.0032 | MTNLAALFPPEGVSI | PAP.117 | PAP | | 24.4 | | >225000 | | |
| 68.0036 | NPILLWQPIPVHTVP | PAP.133 | PAP | | 31.3 | >67840.05 | 1160.3 | | |
| 68.0033 | PEGVSIWNPILLWQP | PAP.126 | PAP | | 111.2 | | 10000.0 | | |
| 68.0037 | PILLWQPIPVHTVPL | PAP.134 | PAP | | 44.4 | >67840.05 | 1450.7 | | |
| 68.0021 | PLLLARAASLSLGFL | PAP.5 | PAP | | 10.3 | >67840.05 | 2932.4 | | |
| 68.0026 | RSPIDTITTDPIKES | PAP.47 | PAP | | >50000 | | 73660.4 | | |
| 68.0022 | SLSLGFLFLLFFWLD | PAP.13 | PAP | | 11416.8 | | 26501.2 | | |
| 68.0147 | TVPLSEDQLLYLPFR | PAP.145 | PAP | | 4012.0 | 332.2 | 60495.7 | | |
| 68.0035 | WNPILLWQPIPVHTV | PAP.132 | PAP | | 208.2 | >67840.05 | 3908.3 | | |
| 68.0039 | WQPIPVHTVPLSEDQ | PAP.138 | PAP | | 6385.9 | | >119903.81 | | |
| 68.0041 | YDPLYCESVHNFTLP | PAP.210 | PAP | | 1597.4 | 16625.5 | 50000.0 | | |
| 68.0049 | YKKUMYSAHDTTVS | PAP.280 | PAP | | 16.8 | 22677.9 | 1166.0 | | |
| 68.0054 | YNGLLPPYASCHLTE | PAP303 | PAP | | 41.6 | | 34811.9 | | |
| 1188.24 | AGGIAGGLALLACAG | Pf.SSP2.498 | Pf | | 359.7 | | 38031.9 | 6742.0 | >35906.62 |
| 1188.14 | ATSVLAGLLGNVSTV | Pf.EXP1.77 | Pf | | 19.6 | | 22116.3 | 943.9 | >35906.62 |
| 1188.28 | AVPLAMKLIQQLNLN | Pf.SSP2.68 | Pf | | 3768.9 | | 22278.3 | 5750.6 | 30661.1 |
| F14302 | EWSPCSVTCGNGIQVRIK | Pf.CSP.345 | Pf | | 771.9 | >53033.01 | 60893.1 | | |
| 27.0396 | PLALFFIIFNKESLA | Pf.EXP1.8 | Pf | | 7193 | | 1225.8 | | 4685.8 |
| F14309 | IEQYLKKIKNSISTEWSPCS | PLCSP.331 | Pf | | 72.7 | >53033.01 | 1102 | | |
| 118804 | IFHINGKIIKNSEKD | Pf.LSA1.18 | Pf | | 164.9 | 7595.4 | >60133.78 | >59761.43 | 103021 |
| 27.0401 | KHILYISFYFILVNL | Pf.LSA1.2 | Pf | | 3380.1 | | 892.8 | | >761525 |
| 1188.20 | LIDVHDLISDMIKKE | Pf.EXP1.47 | Pf | | 401.4 | 90662 | 1517.0 | 45454.6 | 5019.6 |
| F150.02 | NAREIIRLHSDASNKEKAL | Pf.SSP2. | Pf | | 98.1 | 49342.1 | 2191.4 | | |
| 27.0414 | NHAVPLAMKLLIQQLN | Pf.SSP2.66 | Pf | | 266.1 | | 3916.1 | | >7615.25 |
| F107.12 | TVLLGGVGLVLYNTE | Pf.EXP1.90 | Pf | | 10.3 | | 16198.4 | | >25333.33 |
| 1188.47 | VDLYLLMDCSGSIRR | Pf.SSP2.47 | Pf | | 3125.0 | | 3120.9 | 26688.0 | >35906.62 |
| 27.0410 | VKNVSQTNFKSLLRN | PfLSA1.89 | Pf | | 2937.8 | 1669.1 | 6441.2 | | 3182.2 |
| 1188.48 | VVILTDGIPDSIQDS | Pf.SSP2.157 | Pf | | 50000.0 | | 663.6 | 11707.3 | >35906.62 |
| F150.03 | YADSAWENVKNVIGPFMKAV | Pf.SSP2. | Pf | | 69.7 | >51961.52 | >4008.92 | | |
| 191.16 | ADLIAY'LKQATAK | Pigeon.CytochromeC.92 | Pigeon | | 80.7 | | | 12500.0 | |
| 12.04 | DLIAYLKQATAK | Pigeon.CytochromeC.88 | Pigeon | A | 217.A | | | 4166.7 | |
| 12.05 | ERADLIAYLKQATAK | Pigeon.CytochromeC.88 | Pigeon | A | 57.5 | | | 1612.9 | |
| 12.03 | IAYIKQATAK | Pigeon.CytochromeC.88 | Pigeon | A | 1000.0 | | | 12500.0 | |
| 191.10 | KAERADLIAY'LKQATA | Pigeon.CytochrameC.88 | Pigeon | | 2500.0 | | | 12500.0 | |
| 199.17 | LIAY'LKQATAK | Pigeon.CytochiomeC.94 | Pigeon | | 172.4 | | | 4545.5 | |
| F025.08 | AATYNFAVLKLMGRGTKF | PLP260 | PLP | | | >17256.71 | 3000.0 | | |
| K-09 | FLYGALLLAEGFYTTGAVRQ | PLP.81 | PLP | | | | | | |
| F050.02 | FLYGALLLAEGFYTTGAVRQ | PLP.81 | PLP | | | >37242.26 | 502.9 | | |
| K-28 | FNTWTTCQSIAFPS | PLP.178 | PLP | | | | | | |
| K-20 | LCADARMYGVLPWNAFPGK V | PLP.201 | PLP | | | | | | |
| K-05 | LTGTFKLIETYFSKNYQDYE | PLP.41 | PLP | | | | | | |
| F025.05 | QKGRGYRGQHQAHSLERVCH | PLP.121 | PLP | | | >15389-69 | 495.3 | | |
| K-18 | SAVPVYIYFNTWTTCQSIAF | PLP.171 | PLP | | | | | | |
| F050.05 | SAVPVYIYFNTWTTCQSIAF | PLP.171 | PLP | | | 27272.7 | 202.7 | | |
| K-16 | TAEFQMTFHLFIAAFVGAAA | PLP.231 | PLP | | | | | | |
| F025.63 | WTTCQSIAFPSKTSASIGSL | PLP.181 | PLP | | | 17307.7 | 126.0 | | |
| 68.0066 | AHCIRNKSVILLGRH | PSA.60 | PSA | | 578.1 | 29704.4 | 386.8 | | |
| 68.0078 | CAQVHPQKVTKFMLC | PSA.180 | PSA | | 10206.2 | | 14433.8 | | |
| 68.0079 | GGPLVCNGVLQGITS | PSA210 | PSA | | 3353.3 | | 382.9 | | |
| 68.0080 | GPLVCNGVLQGITSW | PSA.211 | PSA | | 1724.0 | | 169.9 | | |
| 68.0069 | GQVFQVSHSFPHPLY | PSA83 | PSA | | 288.4 | 45000.0 | 45.9 | | |
| 68.0062 | GRAVCGGVLVHPQWV | PSA.42 | PSA | | 386.5 | | >129903.81 | | |
| 68.0063 | GVLVHPQWVLTAAHC | PSA.48 | PSA | | 87.3 | 21320.1 | 378.4 | | |
| 68.0073 | HDLMLLRLSEPAELT | PSA.115 | PSA | | 62.1 | 2867.5 | 34833.4 | | |
| 68.0064 | HPQWVLTAAHCIRNK | PSA.52 | PSA | | 13.1 | 3631.5 | 9117.5 | | |
| 68.0158 | HSLFHPEDTGQVFQV | PSA.74 | PSA | | | 65260.2 | | | |
| 68.0077 | LHVISNDVCAQVHPQ | PSA.172 | PSA | | 789.0 | 8318.4 | 4443.4 | | |
| 68.0081 | NGVLQGITSWGSEPC | PSA216 | PSA | | 944.9 | 249423 | 3149.4 | | |
| 68.0071 | PHPLYDMSLLKNRFL | PSA.93 | PSA | | 13155 | | 116928.6 | | |
| 68.0065 | QWVLTAAHCIRNKSV | PSA.54 | PSA | | 49.8 | | 109141.0 | | |
| 68.0082 | RPSLYTKWHYRKWI | PSA.235 | PSA | | 6041.0 | 53785.3 | 1904.2 | | |
| 68.0072 | SHDLMLRLSEPAEL | PSA.114 | PSA | | 531.8 | 6214.7 | 22786.6 | | |
| 68.0061 | SQPWQVLVASRGRAV | PSA.31 | PSA | | 66.0 | >67940.05 | 42905.8 | | |
| 68.0067 | SVILLGRHSLFHPED | PSA67 | PSA | | 717.2 | 1399.7 | 71151.3 | | |
| 68.0059 | SVTWIGAAPLILSRI | PSA.7 | PSA | | 4.1 | >67840.05 | 17732.5 | | |
| 68.0074 | TDAVKVMDLPTQEPA | PSA.129 | PSA | | >50000 | | 225000 | | |
| 68.0058 | TLSVTWIGAAPLILS | PSA.5 | PSA | | 3.1 | >67840.05 | 40909.1 | | |
| 68.0070 | VFQVSHSFPHPLYDM | PSA.85 | PSA | | 155 | >63012.6 | 139.2 | | |
| 68.0068 | VILLGRHSLFHPBDT | PSA.68 | PSA | | 273.0 | 8743.6 | 46169.0 | | |
| 68.0060 | VTWIGAAPLn.SRN | PSA.8 | PSA | | 8.1 | >67940.05 | 45000.0 | | |
| 68.0125 | ADKIYSISMKHPQEM | PSM.608 | PSM | | 2230.7 | | 29867.8 | | |
| 68.0101 | AEAVGLPSIPVHPIG | PSM.284 | PSM | | 5.4 | | 55815.6 | | |
| 68.0102 | AVGLPSIPVHPIGYY | PSM.286 | PSM | | 3.6 | | 23586.4 | | |
| 68.0113 | CTPLMYSLVHNLTKE | PSA.466 | PSM | | 929 | 19437.0 | 1378.3 | | |
| 68.0114 | DFEVFFQRLGLASGR | PSM.520 | PSM | | 143.0 | | 1244.5 | | |
| 68.0118 | DPMFKYHLTVAQVRG | PSA.567 | PSM | | 5.7 | >63012.6 | 51.2 | | |
| 68.0181 | DQLMFLERAFIDPLG | PSM.666 | PSM | | | >14078.28 | | | |
| 66.0111 | DSSIEGNYTLRVDCT | PSM:453 | PSM | | 16666.7 | | 18898.2 | | |
| 68.0167 | EDFFKLEPDMKINCS | PSM.183 | PSM | | 2709.8 | 468.5 | 1269.5 | | |
| 68.0109 | ERGVAYINADSSIEG | PSM.444 | PSM | | 2439.8 | | 38031.9 | | |
| 68.0115 | EVFFQRLGIASGRAR | PSM.522 | PSM | | 28.0 | >63012.6 | 123.8 | | |
| 68.0100 | EYAYRRGIAEAVGLP | PSM.276 | PSM | | 5.1 | | 1196.9 | | |
| 68.0168 | FFKLERDMKINCSGK | PSM.185 | PSM | | 4419.2 | 73.0 | 2719.0 | | |
| 68.0173 | GAAVVHEIVRSFGTL | PSM.391 | PSM | | | 12409.1 | | | |
| 68.0096 | GKVFRGNKVKNAQLA | PSM.206 | PSM | | 611.6 | | 6115.2 | | |
| 68.0123 | GMVFELANSIVLPFD | PSM.582 | PSM | | 15.0 | 4603.7 | 1295.1 | | |
| 68.0090 | GNBBFNTSLFEPPPP | PSM.135 | PSM | | 20412.4 | | >60133.78 | | |
| 68.0097 | GNKVKNAQLAGAKGV | PSM.211 | PSM | | 677.0 | | 75000.0 | | |
| 68.0110 | GVAYINADSSIEGNY | PSM.446 | PSM | | 1054.1 | | 823.4 | | |
| 68.0170 | GVILYSDPADYFAPG | PSM.224 | PSM | | 1565.9 | 17.1 | 42232.7 | | |
| 68.0103 | IGYYDAQKLLEKMGG | PSM.297 | PSM | | 1923.1 | | 71151.3 | | |
| 68.0086 | IKKFLYNFTQIPHLA | PSM.70 | PSM | | 449.1 | 8079.6 | 240.4 | | |
| 68.0166 | ISIINEDGNEIFNTS | PSM.128 | PSM | | 506.7 | 559.2 | >51888.14 | | |
| 68.0126 | IYSISMKHPQEMKTY | PSM.611 | PSM | | 8451.5 | | 90000.0 | | |
| 68.0087 | KFLYNFTQIPHLAGT | PSM.72 | PSM | | 340.2 | 13805.4 | 1221.8 | | |
| 68.0120 | KYHLTVAQVRGGMVF | PSM.571 | PSM | | 136.9 | 33658.1 | 4531.8 | | |
| 68.0089 | LAHYDVLLSYPNKTH | PSM.110 | PSM | | 78.6 | 37532.6 | 6284.8 | | |
| 68.0083 | LDELKAENIKKFLYN | PSM.62 | PSM | | 1136.4 | 1369.9 | 272351.2 | | |
| 68.0084 | LGFLFGWFIKSSNEA | PSM.35 | PSM | | 10.4 | >63012.6 | 7524.1 | | |
| 68.0131 | LRMMNDQLMFLERAF | PSM.661 | PSM | | 2703.7 | | 2204.1 | | |
| 68.0119 | MFKYHLTVAQVRGGM | PSM.S69 | PSM | | 15.8 | 290323 | 98.7 | | |
| 68.0176 | NSRLLQERGVAYINA | PSM.438 | PSM | | 613.6 | 317.8 | 28624.2 | | |
| 68.0112 | NYTLRVDGTPLMYSL | PSM.459 | PSM | | 6804.1 | 45.1 | 55.8 | | |
| 68.0127 | PQEMKTYSVSFDSLF | PSM.619 | PSM | | 15142.7 | | 17010.0 | | |
| 68.0083 | PRWLCAGALVLAGGF | PSM.18 | PSM | | 46.5 | | >60133.78 | | |
| 68.0135 | QIYVAAFTVQAAAET | PSM.731 | PSM | | 1.6 | 26609.1 | 9.1 | | |
| 68.0122 | RGGMVFELANSIVLP | PSM580 | PSM | | 103 | 37117.9 | 1288.1 | | |
| 68.0133 | RHVIYAPSSHNKYAG | PSM.688 | PSM | | 2173.9 | | 2705.3 | | |
| 68.0134 | RQIYVAAFTVQAAAB | PSM.730 | PSM | | 3.7 | 283473 | 6.9 | | |
| 68.0105 | TGNFSTQKVKMHIHS | PSM.334 | PSM | | 11180.3 | | 4687.8 | | |
| 68.0116 | TNKFSGYPLYHSVYE | PSM.543 | PSM | | 3402.1 | | 31053.0 | | |
| 68.0107 | TRIYNVIGTLRGAVE | PSM.353 | ISM | | 14.3 | 33333.3 | 35.4 | | |
| 68.0128 | TYSVSFDSLFSAVKN | PSM.624 | PSM | | 219.1 | 110.1 | 410.0 | | |
| 68.0136 | VAAFTVQAAAETLSE | PSM.734 | PSM | | 14.5 | >63012.6 | 326.5 | | |
| 68.0121 | VAQVRGGMVFELANS | PSM.576 | PSM | | 228.2 | | 3724.7 | | |
| 68.0177 | VAYINADSSIEGNYT | PSM.447 | PSM | | 4716.5 | 530.9 | 2310.6 | | |
| 68.0124 | VFELANSIVLPFDCR | PSM.584 | PSM | | 19.2 | 667.4 | 5617.5 | | |
| 68.0130 | VLRMMNDQLMFLERA | PSM.660 | PSM | | 117.6 | 182.7 | 161.7 | | |
| 68.0088 | WKEFGLDSVELAHYD | PSM.100 | PSM | | 1138.8 | 84.8 | 540.1 | | |
| 68.0117 | YDPMIFKYHLIVAQVR | PSM.566 | PSM | | 9.0 | >63012.6 | 106.2 | | |
| 13.00 | NKALELFRKDIAAKYKELGY QG | Spermwhale.Myoglobin.132 | Spermwhale | | | 37.0 | 300.0 | | |
| 213.17 | RKDIAAKYKELGY | Spermwhale.Myoglobin.139 | Spermwhale | | | >6634.89 | 2250:0 | | |
| 68.0105 | YISIINEDGNEIFNT | PSM.127 | PSM | | 497.9 | 397.1 | 3510.5 | | |
| 68.0132 | YRHVIYAPSSHNKYA | PSM.687 | PSA | | 94.6 | >63012.6 | 1597.6 | | |
| F112.02 | VDAQGTLSRLFKLGGRDSRS | Rabbit.144 | Rabbit | | | | | | |
| 938.01 | KVNNVVSLKPEIIVDQEY | Rabiesvirus | Rabiesvirus | | 23.6 | | 7500.0 | 1752.5 | |
| F160.32 | STORKUSP32 | RAGE. | RAGE | | | | 392.6 | 91.5 | |
| F160.34 | STORKUSP34 | RAGE. | RAGE | | | | >24771.68 | 721.6 | |
| F160.36 | STORKUSP36 | RAGE. | RAGE | | | | 658.5 | 7.5 | |
| F160.37 | STORKUSP37 | RAGE. | RAGE | | | | 998.3 | 99.2 | |
| F160.39 | STORKUSP39 | RAGE. | RAGE | | | | >19733.81 | 40.3 | |
| F047.09 | DKLKQQRDTLSTQKET | RHD.81 | RHD | | | | | | |
| F047.16 | EQKSKQNIGALKQEL | RHD. | RHD | | | | | | |
| 938.06 | SGGTNYAQKFQGRVTMTRDT -NH2 [?] | Rheumatiodvector | Rheunrntiodvector | | 6682 | | 5625.0 | >16666.67 | |
| 938.08 | ELSRLSDDTAVYYCARAPG- NH2 | Rheumatiodvector | Rheumatiodvector | | 110.1 | | 304.0 | 10000.0 | |
| 938.09 | ELSRLTSDDTAVYYCAIAPG- NH2 | Rheumatiodvector | Rheumatiodvector | | 130.0 | | 653.6 | 2344.0 | |
| 938.10 | TISCSGSSSNIGSNTN-NH2 | Rheumatiodvector | Rheumatiodvector | | 50000.0 | | 147.1 | 2646.3 | |
| F015.05 | APYHFDLSGHA | Ryegms.Lolp1 | Ryegrass | | | | | | |
| 791.08 | APYHFDLSGHAF | Ryegrass.Lolp1.101 | Ryegrass | | | | 37.5 | | |
| 791.07 | APYHFDLSGHAFGS | Ryegmss.Lolp1.101 | Ryegrass | | | | 10.0 | | |
| 791.06 | APYHFDLSGHAFGSMA | Ryegrass.Lolp1.101 | Ryegrass | | | | 28.1 | | |
| 791.05 | APYHFDLSGHAFGSMAKK | Ryegraas.Lolp1.101 | Ryegrass | | | | 40.9 | | |
| 620.11 | APYHFDLSGHAFGSMAKKGE | Ryegxass.Lolp1 | Ryegrass | | 50000.0 | 25980.8 | 25.0 | 500000.0 | |
| 620.05 | CGYKDVDKAPFNGMTGCGN T | Ryegrass.Lolp1.41 | Ryegrass | | 50000.0 | 90.0 | 3750.0 | 500000.0 | |
| 807.03 | DLSGHAFGS | Ryegmss.Lolp1.106 | Ryegrass | | | | 450000.0 | | |
| 595.02 | EDVIPEGWKADTSYSAK | Ryegrass.Lotp1.80 | Ryegrass | | 50000.0 | | 60.8 | 500000.0 | |
| 620.14 | ELQFRRVKCKYPDDTKPTFH | Ryegrass.Lolp1.131 | Ryegrass | | 50000.0 | >30000 | 3750.0 | 500000.0 | |
| 620.13 | EQNVRSAGELELQFRRVKCK | Ryegrass.Lolp1.121 | Ryegrass | | 50000.0 | | 3750.0 | 500000.0 | |
| 620.20 | ESWGAVWRIDTPDKLTGPFT | Ryegrass.Lolp1 | Ryegrass | | 2500.0 | 3128.2 | 3750.0 | 333.3 | |
| 791.02 | FDLSGHAFGSMAKKGE | Ryegass.Lolp1.105 | Ryegrass | | | | 450000.0 | | |
| 620.13 | GDVVAVVDIKEKGKDKWIEL K | Ryegrass.Lolp1 | Ryegrass | | 50000.0 | 321.6 | 3750.0 | 50000.0 | |
| MA-05 | GEKRAYAASDPGRYC | Ryegrass.Lolp1 | Ryegrass | | 50000.0 | | 450000.0 | 500000.0 | |
| 620.19 | KGKDKWIELKESWGAVWRID | Ryegrass.Lolp1.181 | Ryegrass | | 1666.7 | >30000 | 3750.0 | 8333.3 | |
| 791.03 | LSGHAFGSMAKKGE | Ryegrass.Lolp1.107 | Ryegass | | | | 450000.0 | | |
| 620.23 | SEVEDVIPEGWKADTSYSAK | Ryegrass.Lo1p1.221 | Ryegrass | | 5000.0 | | 725.8 | 50000.0 | |
| MA-06 | VAYESSEIASKKAG | Ryegrass.Lolp1 | Ryegrass | | 50000.0 | | 714.3 | 12500.0 | |
| 620.16 | VEKGSNPNYLAILVKYVDGD | Ryegrass.Lolp1.151 | Ryegrass | | 200.0 | >30000 | 3750.9 | 500000.0 | |
| 791.09 | YHFDLSGHAFGS | Ryeglass.Lolp1,103 | Ryegrass | | | | 19.6 | | |
| 791.01 | YHFDLSGHAFGSMAKKGE | Ryegrass.Lolp1.103 | Ryegrass | | | | 38.1 | | |
| 620.15 | YPDDTKPTFHVEKGSNPNYL | Ryegass.Lolp1.141 | Ryegrass | | 1000.0 | >30000 | 1607.1 | 500000.0 | |
| F165.05 | KSDNQIKAVPASQALVA | Sm.eggantigen.235 | Sm | | 4.3 | | 86.7 | 58.0 | |
| F165.01 | PKSDNQIKAVPAS | Sm.eggantigen.234 | Sm | | 43505 | | >504.69 | 2858.8 | |
| F165.03 | PKSDNQIKAVPASQA | Sm.eggantigen.234 | Sm | | 19.2 | | 326.3 | 78.1 | |
| F165.02 | VRPKSDNQIKAVPAS | Smeggantigen.232 | Sm | | 5154.6 | | 3324.4 | 375.6 | |
| 213.16 | FRKDIAAKYKELGY | Spermwhale.Myoglobin.138 | Spenmwhale | | | 1080.2 | 1000.0 | | |
| 213.15 | LFRKDIAAKYKELGY | Spermwhale.Myoglobin.137 | Spermwhale | | | 427.2 | 737.7 | | |
| 542.00 | NKALELFRKDIAA | Spermwhale.Myoglobin.132 | Spermwhale | | | 3500.2 | 2250.0 | | |
| 213.12 | NKALELFRKDIAAK | Spennwhale.Myoglobin.132 | Spermwhale | | | 2547.6 | 2045.5 | | |
| 213.11 | NKALELFRKDIAAKY | Spermwhale.Myoglobin.132 | Spermwhale | | | 2393.4 | 775.9 | | |
| NASE061-80 | FTKKMVENAKKEEVEFDKGQ | Staph.Nase.61 | Staph. | | 1000.0 | 11619.0 | 28123 | 500000.0 | |
| F015.01 | GLAKVAYVYKP | Staph.Nase.101 | Staph. | | | | | | |
| NASE121-140 | HEQHLRKSEAQAKKEKLNIW | Staph.Nase.121 | Staph. | | 50000.0 | 90000.0 | 450000.0 | 16666.7 | |
| 191.26 | LVRQGLAKVAY | Staph.Nase.103 | Staph. | | 166.7 | | 450000.0 | 500000.0 | |
| NASE011-30 | PATUKAIDODTVKLMYKGQ | Staph.Nase.11 | Staph. | | 277.8 | 6428.6 | 1666.7 | 3846.2 | |
| NASE031-50 | PMTTRLLLVDTPETKHPKKG | Staph.Nase.31 | Staph. | | 104.2 | 45000.0 | 1216.2 | 574.7 | |
| 191.29 | QGLAKVAYVYK | Staph.Nase.106 | Staph | | 50000.0 | | 450000.0 | 500000.0 | |
| 191.28 | RQGLAKVAYVY | Staph..Nase.105 | Staph | | 50000.0 | | 450000.0 | 10000.0 | |
| NASE041-60 | TPETKHPKKGVEKYGPEASA | Staph..Nase.41 | Staph. | | 1000.0 | 900000.0 | 2812.5 | 500000.0 | |
| NASE051-70 | VEKYGPEASAFTKKMVENAK | Staph.Nase.51 | Staph. | | 50000.0 | 900000.0 | 7500.0 | 500000.0 | |
| 191.27 | VRQGLAKVAYV | Staph.Nase.104 | Staph | | 106.4 | | 450000.0 | 5000.0 | |
| NASE091.110 | YIYADGKMVNEALVRQGLAK | Staph..Nase.91 | Staph. | | 64.9 | | 2812.5 | 4166.7 | |
| 546.00 | FTKKMVENAKKIEVEFDKGQ | Staph.nuc..61 | Staph.nuc | | 50000.0 | 26762 | 22500.0 | 50000.0 | |
| 866.05 | HEQHLRKSEAQAKKEKLNIW | Staph.nuc..121 | Staph.nuc | | 50000.0 | 6428.6 | 22500.0 | 50000.0 | |
| 598.00 | PATLIKAIDGDTVKLMYKGQ | Staph.nuc..11 | Staph.nuc | | 1250.0 | 63.1 | 937.3 | 1388.9 | |
| 866.06 | QAKKEKLNIWSEDNADSGQ | Staph.nuc..131 | Staph.nuc | | 50000.0 | >45000 | 473.7 | 5000.0 | |
| 866.02 | VEKYGPEASAFTKKMVENAK | Staph.nuc..51 | Staph.nuc | | 5000.0 | >45000 | 9000.0 | 500000.0 | |
| 866.04 | YTYADGYd4VNEALVRQGLAK | Staph.nuc..91 | Staph.nuc | | 119.1 | 260.9 | 22500.0 | 16666.7 | |
| 835.03 | YGAVDSMGGVATYGAA-NH2 | Stp.dt..1 | Stp.dt. | | 59.5 | >15000 | 1216.2 | 259.1 | |
| F178.08 | AGTIAALNNSIGVLG | Subtilisin.69 | Subtilisin | | 43.1 | >25139.39 | 2234.7 | 114.4 | 857.1 |
| F178.10 | GSISYPARYANAMAV | Subtilisin.157 | Subtilisin | | 1389.5 | 17180.3 | 11296.9 | 6016.0 | 8624.5 |
| F178.06 | GTVAALDNSAGVLGV | Subtilisin.70 | Subtilisin | | 163.2 | 2991.9 | 964.4 | 10.4 | 1283.8 |
| F178.05 | GTVAALDNSIGVLGV | Subtilisin.70 | Subtilisin | | 15.1 | 236.4 | 133.3 | 17.1 | 757.2 |
| F178.03 | GTVAALNNSAGVLGV | Subtilisin.70 | Subtilisin | | 19.5 | 117022 | 598.1 | 16.3 | 1304.3 |
| F178.01 F178.0 | GTVAALNNSIGVLGV | Subtilisin.70 | Subtilisin | | 6.8 | 39992 | 156.0 | 7.1 | 186.9 |
| 9 | IAALNNSIGVLGVAP | Subtilisin.72 | Subtilisin | | 6.6 | 15938.9 | 283.5 | 10.0 | 1610.6 |
| F178.04 | NGIEWAIANNMDVAN | Subtilisin.109 | Subtilisin | | 7.7 | 21675.5 | 69.1 | 109.4 | 15.4 |
| F178.02 | NGIEWAIANNMDVIN | Subtilisin.109 | Subtilisin | | 8.3 | 32040.0 | 77.4 | 168.4 | 37.4 |
| F178.11 | SYPARYANAMAVGAT | Subtilisin.160 | Subtilisin | | 11.0 | >25139.39 | 142.4 | 6091.5 | 1437.1 |
| F1781.07 | TCSGVKVAVLDTGIS | Subtilisin.22 | Subtilisin | | >10270.85 | 35587.8 | >28708.41 | 2709.1 | 5964.0 |
| 573.13 | ANSKFIGITELKK | TetTox.834 | TetTox | | 50000.0 | | 11250.0 | | |
| 573.12 | IKANSKFIGITELKK | TetTox.832 | TetTox | | 50000.0 | | 11250.0 | | |
| 597.10 | ILMQYIKANS | TetTox.827 | TetTox | | 5000.0 | | 1875.0 | | |
| L-05; | QAIKANSKFIGITE | TetTox.830 | TetTox | A | 50000.0 | | 450000.0 | | |
| 650.05 | QEIKANSKFIGITE | TetTox.830 | TetTox | A | 5000.0 | 1748.3 | 11250.0 | 50000.0 | |
| 650.06 | QSIKANSKFIGITE | TetTox.830 | TetTox | A | 5000.0 | 4880.9 | 11250.0 | 50000.0 | |
| 650.21 | QYIKANQKFIGITE | TetTox.830 | TetTox | A | 5000.0 | 958.3 | 11250.0 | 50000.0 | |
| 650.29 | QYIKANSKFKGITE | TetTox.830 | TetTox | A | 5000.0 | 135.4 | 7500.0 | 500000.0 | |
| 650.14 | QYIKKNSKFIGITE | TetTox.830 | TefTox | A | 2000.0 | 36.0 | 11250.0 | 500000.0 500000.0 | |
| 650.16 | QYIKSNSKFIGITE | TetTox.830 | TetTox | A | 2000.0 | 4330.1 | 11250.0 | | |
| 650.13 | QYIRANSKFIGITE | TefTox.830 | TetTox | A | 100.0 | 798.0 | 11250.0 | 500000.0 | |
| 573.14 | SKFIGITELKK | TetTox.836 | TetTox | | 1250.0 | | 15000.0 | | |
| 48.02 | YNGQIGNDPNRDIL | TetTox. | TetTox | | 666.7 | | | | |
| 534.02 | DTPYLDITYHFVMQRLPL | Torp.Ac..califormica.195 | Torpedocalifornica | | 384.6 | 0.0 | 900.0 | 500000.0 | |
| 1385.07 | GELIGILNAAKVPAD | TPI.23 | TPI | | 9.0 | 96743 | 321.8 | 22.1 | |
| F167.11 | ALHIYMDCTMSQVQGSA | Tyrosinase.365 | Tyrosinase | | 442.3 | 91.5 | 17.8 | 31.8 | |
| F167.12 | ALHIYMNGTMSQVQGSA | Tyrosinase.365 | Tyrosinase | | 526.4 | 26631.2 | 3418.4 | 174.6 | |
| F089.22 | DQSYLQDSDPDSFQD | Tyrosinase.448 | Tyrosinase | A | | | 923.2 | | |
| F089.13 | DYSFLQDSDPDSFQD | Tyrosinase.448 | Tyrosinase | A | >50000 | | 563.6 | | |
| | DYSYFQDSDPDSFQD | Tyrosinase.448 | Tyrosinase | A | | | 507.2 | | |
| F089.25 1385.10 | DYSYLQDSDPDSFQD | Tyrosinase.448 | Tyrosinase | | >9128.71 | 39391.9 | 959.9 | >27277.24 | |
| F089.11 | DYSYLQDSVPDSFQD | Tyrosinase.448 | Tyrosinase | A | 71428.6 | | 156.0 | >62500 | >4546.62 |
| F089.24 | DYSYQQDSDPDSFQD | Tyrosinase.448 | Tyrosinase | A | | | 996.3 | | |
| 1385.09 | FLLHHAFVDSIFEQWLQRHRP | Tyrosinase.386 | Tyrosinase | | 10245.9 | 707.1 | >2758.62 | 11258.7 | |
| F089.18 | ILLSNAPLGPQFP | Tyrosinase.58 | Tyrosinase | | 869.5 | | 32142.9 | | |
| F089.20 | NILLSNAPLGPQFP | Tyrosinase.57 | Tyrosinase | | 60.0 | | 338.8 | | |
| F089.06 | QNFLLSNAPLGPQFP | Tyrosinase.56 | Tyrosinase | A | 5.1 | | 61.9 | 3803.0 | 217.8 |
| F089.28 | QNIFLSNAPLGPQFP | Tyrosinase.56 | Tyrosinase | A | | | 321.4 | | |
| F089.04 | QNILLSNAPQGPQFP | Tyrosinase.56 | Tyrosinase | A | 297.4 | | | | |
| F089.33 | QNILLSNAQLGPQFP | Tyrosinase.56 | Tyrosinase | A | | | 303.4 | | |
| F089.34 | QNILLSNAVLGPQFP | Tyrosinase.56 | Tyrosinase | A | | | 159.9 | | |
| F089.03 | QNILLSNQPLGPQFP | Tyrosinase.56 | Tyrosinase | A | 238.0 | | 7763.2; | | |
| F089.07 | QNILLSNVPLGPQFP | Tyrosinase.56 | Tyrosinase | A | 48.2 | | 12.7 | 32.1 | 162.0 |
| F089.30 | QNILQSNAPLGPQFP | Tyrosinase.56 | Tyrosinase | A | | | 758.6 | | |
| F089.32 | QNILVSNAPLGPQFP | Tyrosinase.56 | Tyrosinase | A | | | 619.9 | | |
| F089.17 | QNIQLSNAPLGPQFP | Tyrosinase.56 | Tyrosinase | A | | | 774.6 | | |
| F089.29 | QNIVLSNAPLGPQFP | Tyrosinase.56 | Tyrosinase | A | | | 99.5 | | |
| F089.19 | QNVLLSNAPLGPQFP | Tyrosinase.56 | Tyrosinase | A | 58.9 | | 9963 | | |
| F089.15 | SYLQDSDPDSFQD | Tyrosinase.450 | Tyrosinase | | >50000 | | 346.0 | | |
| F089.21 | SYLQDSVPDSFQD | Tyrosinase.450 | Tyrosinase | A | >50000 | | 63.0 | 166666.7 | 3084.7 |
| F167.09 | WPSVFYNRTCQCSCNF | Tyrosinase.80 | Tyrosinase | | 32258.1 | >17822.66 | >3801.38 | 36791.8 | |
| F167.10 | YGQMKNGSTPMFNDINIYDL | Tyrosinase.156 | Tyrosinase | | 1123.0 | 5706.9 | 14363 | 1086.3 | |
| F089.16 | YLQDSDPDSFQD | Tyrosinase.451 | Tyrosinase | | >50000 | | 567.4 | | |
| F089.14 | YSYLQDSEIPDSFQD | Tyrosinase.449 | Tyrosinase | | >50000 | | 247.7 | | |
| 604.01 | (AKA)6 | Unknown | u | | 625.0 | | 9000.0 | 500000.0 | |
| 848.01 | A(56)FAAAA(24)A(56)AA(57)- NH2 | Unknown | u | | 1.3 | | 1.1 | 2.8 | |
| 848.03 | A(56)FAAAA(24)L(56)AA(57)- NH2 | Unknown | u | | 6.4 | | 2.5 | 25.0 | |
| 848.05 | A(56)FAAAATA(56)AA(57)- NH2 | Unknown | u | | 23.0 | | 12.9 | 50.0 | |
| 848.07 | A(56)FAAAATL(56)AA(57)- NH2 | Unknown | u | | 2.5 | | 11.3 | 38.5 | |
| F042.01 | A(X)KQNTLKLAT | Unknown | u | A | 5.5 | | | | |
| 848.02 | AAFAAAA(24)A(56)AA(57)- NH2 | Unknown | u | | 1.1 | | 8.2 | 38.5 | |
| 848.04 | AAFAAAA(24)L(56)AA(57)- NH2          ; | Unknown | u | | 2.4 | | 2.0 | 31.3 | |
| 848.06 | AAFAAAATA(56)AA(57)-NH2 | Unknown | u | | 2.5 | | 2.7 | 9.6 | |
| 848.08 | AAFAAAATL(36)AA(57)-NH2 | Unknown | u | | 1.1 | | 4.1 | 7.9 | |
| 603.01 | AHAAHAAHAAHAAHAAY | Unknown | u | | 20.8 | | 9000.0 | 2173.9 | |
| F182.04 | EVIPMFSALSEGA | Unknown | u | | 22.0 | >51970.13 | 881.9 | 87.0 | 205.5 |
| 520.05 | EVWREEAYHAADIKD | Unknown | u | | 135.1 | 30000.0 | 4090.9 | 16666.7 | |
| 520.06 | EVWREEAYHAADIKDY | Unknown | u | | 250.0 | | 45000.0 | 500000.0 | |
| 852.04 | KYVKQNTLKLAT | Unknown | u | | 9.9 | | 34.6 | 25000.0 | |
| 852.05 | KYVKQNTLKLAT | Unknown | u | | 14.9 | | 30.0 | 500000.0 | |
| F182.09 | LNKIVRMYSPTSI | Unknown | u | | 10.9 | >61289.42 | 177.9 | 17.6 | 14.6 |
| F182.10 | NKIVRMYSPTSIL | Unknown | u | | 11.0 | >51970.13 | 135.0 | 40.8 | 90.8 |
| F042.06 | P(X)KQNTLAT | Unknown | u | A | 1.7 | | | | |
| F042.07 | P(X)KQNTLKLAT | Unknown | u | A | 14.9 | | | | |
| F182.03 | PBVIPMFSALSEG | Unknown | u | | 17.1 | >51970.13 | 123.2 | 20.8 | 22.5 |
| F182.61 | PIVQNIQGQMVHQ | Unknown | u | | 28.0 | >59528.21 | 227735.0 | 11756.6 | 4074.0 |
| F042.02 | PKFV(X)TLKLAT | Unknown | u | A | 6.2 | | | | |
| F042.05 | PKFVKQNTL(X)T | Unknown | u | A | 7.9 | | | | |
| | PKFVQ(X)KLAT | Unknown | u | A | 6.9 | | | | |
| F042.03 F182.08 | QEQIGWMTNNPPI | Unknown | u | | 1765.4 | >51970.13 | 1958.I | 240.6 | 158.5 |
| 832.01 | TFGLQLELTEGMRFDKG | Unknown | u | | 50000.0 | 161.0 | 918.4 | 16666.7 | |
| 173.00 | TYQRTRALVTG | Unknown | u | | 2500.0 | 900000.0 | 450000.0 | 500000.0 | |
| F182.02 | VQNIQGQMVHQAI | Unknown | u | | 138.2 | >51970.13 | 1338.7 | 240.1 | 2952.5 |
| 835.01 | YIDVWLGGLAENFLP-NH2 | Unknown | u | | 50000.0 | 90000.0 | 15000.0 | 50000.0 | |
| 785.02 | YKKSIQFHWKNSNQIKBLG | Unknown | u | | 217.4 | | 2142.9 | 1562.5 | |
| 843.03 | YKLNDRAPSRRSL | Naturallyprocessed | u | | 50000.0 | | 450000.0 | 500000.0 | |
| F002.02 | YKPVSQLRLATPLLLRPL | Unknown | u | | 03 | 58.5 | 851.9 | 86.9 | |
| 835.02 | YLDPLIRGLLARPAKLQV- NH2 | Unknown | u | | 1.5 | 900000.0 | 3750.0 | 44.3 | |
| F002.01 | YLPKPPKPVSKLRLATPLLLQ ALPL | Unknown | u | | 0.8 | 565.8 | 1227.5 | 435.2 | |
| 824.07 | (14)AAAKTAAAFA-NH2 | Artificialsequence | | | 1.8 | | 0.9 | 4.4 | |
| 824.08 | (15)AAAKTAAAFA-NH2 | Artificialsequence | | | 1.4 | | 0.9 | 3.1 | |
| 820.03 | (15A)RQTTLKAAA-NH2 | Artificialsequence | | A | 4.2 | | 1.8 | 4.3 | |
| 820.05 | (15A)RQTTLICAAA-NH2 | Artificialsequence | | A | 2500.0 | | 153 | 1851.9 | |
| 824.09 | (16)AAAKTAAAFA-NH2 | Artificialsequence | | | 10.9 | | 1.0 | 50.3 | |
| 820.04 | (16A)RQTTLKAAA-NH2 | Artificialsequence | | A | 454.6 | | 7.1 | 37.0 | |
| 853.01 | (39)AAAATKAA(35)-NH2 | Artificialsequence | | A | 250.0 | | 1184.2 | 25000.0 | |
| 853.02 | (39)AAAATKAA(36)-NH2 | Artificialsequence | | A | 8333 | | 818.2 | 50000.0 | |
| 853.03 | (39)AAAATKAA(37)-NH2 | Artificialsequence | | A | 131.6 | | 957.5 | 25000.0 | |
| 824.37 | (39)AAAATKAAAA | Artificialsequence | | | 156.3 | | 616.4 | 12500.0 | |
| 856.04 | (39)AAAKTAAA(35)-NH2 | Artificialsequence | | A | 76.9 | | 2.5 | 294.1 | |
| 824.03 | (39)-AAAKTAAAFA-NH2 | Artificialsequence | | | 5.6 | 900000.0 | 1.0 | 13.2 | |
| 824.21 | (39)AAAKTAAAF-NH2 | Artificialsequence | | | 555.6 | | 25.1 | 1282.1 | |
| 824.26 | (39)AAKTAAAFA-NH2 | Artificialsequence | | | 714.3 | | 600.0 | 2083.3 | |
| 824.02 | (40)-AAAKTAAAFA-NH2 | Artificialsequence | | | 52.1 | 900000.0 | 11.4 | 22.7 | |
| 717.63 | (42)YARFQSQTTLKAKT-NH2 | Artificialsequence | | | 21.8 | 22500.0 | 7.7 | 38.5 | |
| 787.34 | (43)AADFFFFFFFFDA-(NH2) | Artificialsequence | | | 50000.0 | | 483.9 | 500000.0 | |
| 787.43 | (43)AAFGIDIFGFKIA-(NH2) | Artificialsequence | | | 50000.0 | | 803.6 | 500000.0 | |
| 824.11 | (45)AAAKTAAAFA-NH2 | Artificialsequence | | | 833.3 | | 23.2. | 9.1 | |
| 824.28 | (46)AAAATKAAAA | Artificialsequence | | | 17.9 | | 6923 | 16666.7 | |
| 824.12 | (46)AAAKTAAAFA-NH2 | Artificialsequence | | | 9.6 | | 0.6 | 16.7 | |
| 824.29 | (47)AAAATKAAAA | Artificialsequence | | | 263.2 | | 2812.5 | 33333.3 | |
| 824.13 | (47)AAAKTAAAFA-NH2 | Artificialsequence | | | 83.3 | | 3.8 | 127.9 | |
| 824.14 | (48)AAAKTAAAFA-NH2 | Artificialsequence | | | 625.0 | | 5.9 | 20.6 | |
| 824.31 , | (49)AAAATKAAAA | Artificialsequence | | | 15.2 | | 36.3 | 684.9 | |
| 862.06 | (49)AAAKTAA(64)A-NH2 | Artificialsequence | | | 263.2 | | 5.2 | 505.1 | |
| 856.03 | (49)AAAKTAAA(35)-NH2 | Artificialsequence | | A | 20.4 | | 1.3 | 33.3 | |
| 862.07 | (49)AAAKTAAA(64)-NH2 | Artificialsequence | | | 9.2 | | 0.9 | 12.8 | |
| 862.01 | (49)AAAKTAAAAA-NH2 | Artificialsequence | | | 6.2 | | 0.5 | 5.8 | |
| 824.15 | (49)AAAKTAAAFA-NH2 | Artificialsequence | | | 2.6 | | 0.5 | 2.0 | |
| 824.23 | (49)AAKTAAAFA-NH2 | Artificialsequence | | | 312.5 | | 5000.0 | 2173.9 | |
| 824.32 | (50)AAAATKAAAA | Artificialsequence | | | 500.0 | | 5000.0 | 520.8 | |
| 824.16 | (50)AAAKTAAAFA-NH2 | Artificialsequence | | | 128.2 | | 2.5 | 1.2 | |
| 824.45 | (51)AAAATKAAAA | Artificialsequence | | | 49.5 | | 175.1 | 381.7 | |
| 824.43 | (51)AAAKTAAAFA-NH2 | Artificialsequence | | | 13.2 | | 1.7 | 6.5 | |
| 824.44 | (52)AAAKTAAAFA-NH2 | Artificialsequence | | | 83.3 | | 17.5 | 43.5 | |
| 824.51 | (53)AAAATKAAAA | Artificialsequence | | | 666.7 | | 2250.0 | 7142.9 | |
| 824.47 | (53)AAAKTAAAFA-NH2 | Artificialsequence | | | 208.3 | | 5.5 | 38.5 | |
| 824.52 | (54)AAAATKAAAA | Artificialsequence | | | 454.6 | | 584.4 | 2000.0 | |
| 824.41 | (54)AAAKTAAAFA-NH2 | Artificialsequence | | | 172.4 | | 5.5 | 32.7 | |
| 824.50 | (55)AAAKTAAAFA-NH2 | Artificialsequence | | | 1250.0 | | 149.0 | 13.3 | |
| 601.42 | (65)(66)PKFVRQNTLKLAT | Artificialsequence | | | 7.4 | | 47.2 | 400.0 | |
| 752.03 | (67)AAYAAAAAAKAA-NH2 | Artificialsequence | | A | 16.3 | | 102.3 | 500000.0 | |
| 752.02 | (67)FAAAAAAKAA-NH2 | Artificialsequence | | A | 7.4 | | 143.8 | 500000.0 | |
| 838.07 | (CP)-QSQTTLKAKT-NH2 | Artificialsequence | | | 166.7 | 900000.0 | 0.8 | 11.6 | |
| 859.03 | (CP)YAAFQRQTTLKAAA-NH2 | Artiticialsequence | | | 7.7 | 900000.0 | 0.6 | 1.2 | |
| Sandoz374 | (NAF)AAAKTAAAFA-NH2 | Artificialsequence | | | 20.0 | | 4.5 | 49.5 | |
| 934.15 | (X2)KSSQYIKANSKFIGITEAA AFLPSDFFPSV | Artificialsequence | | A | 559.0 | | 3198.0 | >50000 | |
| 803.11 | A(14)AAAKTAAAAA-CONH2 | Artificialsequence | | | 1.3 | 45000.0 | 1.6 | 9.4 | |
| 838.02 | A(14)AAAKTAAAA-NH2 | Artificialsequence | | | 61.0 | | 2.7 | 33.3 | |
| | A(14)AAAKTAAAFA-NH2 | Artificialsequence | | A | 50000.0 | | 290.3 | 25000.0 | |
| 760.73 838.01 | A(14)AAAKTAAA-NH2 | Artificialsequence | | | 39.4 | | 2.5 | 53.8 | |
| 838.04 | A(14)AAAKTAAA-NH2 | Artificialsequence | | | 416.7 | | 25.7 | 8333.3 | |
| 803.10 | A(14)AAAKTAA-CONH2 | Artificialsequence | | | 50000.0 | | 109.8 | 500000.0 | |
| 838.03 | A(14)AAAKTAA-NH2 | Artificialsequence | | | 2500.0 | | 5.6 | 2500.0 | |
| 736.21 | AA(10)AAAAAAKAAA-NH2 | Artificialsequence | | A | 1.9 | | 14.1 | 25000.0 | |
| 736.23 | AA(12)AAAAAAKAAA-NH2 | Artificialsequence | | A | 4.6 | | 7.5 | 8333.3 | |
| 839.29 | AA(14)A(37)ATKAAAA | Artificialsequence | | | 625.0 | | 2812.5 | 16666.7 | |
| 828.03 | AA(14)AAAA(24)KAAAA-NH2 | Artificialsequence | | A | 13 | 10000.0 | 3.2 | 24.4 | |
| 736.25 | AA(14)AAAAAAKAAA-NH2 | Artificialsequence | | A | 1.9 | | 4.4 | 50.5 | |
| 828.01 | AA(14)AAAAPKAAAA-NH2 | Artificialsequence | | A | 0.9 | | 16.7 | 467.3 | |
| 906.30 | AA(14)AAAATEKAAA-NH2 | Artificialsequence | | | 6.4 | 6240.4 | 14.9 | 20.4 | |
| 906.33 | AA(14)AAAATFKAAA-NH2 | Artificialsequence | | | 1.0 | 2383.3 | 1.1 | 3.3 | |
| 90634 | AA(14)AAAATIKAAA-NH2 | Artificialsequence | | | 0.5 | 1505.9 | 2.3 | 3.2 | |
| 839.17 | AA(14)AAAATK(36)AA | Artificialsequence | | | 5000.0 | | 750.0 | 500000.0 | |
| 839.31 | AA(14)AAAATK(37)AA | Artificialsequence | | | 555.6 | | 937.5 | 4545.5 | |
| 839.06 | AA(14)AAAATKA(35)A | Artificialsequence | | | 1666.7 | | 441.2 | 500000.0 | |
| 839.18 | AA(14)AAAATKA(36)A | Artificialsequence | | | 217.4 | | 80.9 | 12500.0 | |
| 839.32 | AA(14)AAAATKA(37)A | Artificialsequence | | | 113.6 | | 30.0 | 1612.9 | |
| 839.20 | AA(14)AAAATKAA(35)-NH2 | Artificialsequence | | | 1.8 | | 1.7 | 41.7 | |
| 839.24 | AA(14)AAAATKAA(36)-NH2 | Artificialsequence | | | 0.8 | | 1.9 | 45.5 | |
| 839.35 | AA(14)AAAATKAA(37)-NH2 | Artificialsequence | | | 0.7 | | 5.6 | 25.0 | |
| 839.25 | AA(14)AAAATKAAAA | Artificialsequence | | | 1.1 | | 3.2 | 29.4 | |
| 828.11 | AA(14)AAAATKAAAA-NH2 | Artificialsequence | | A | 1.5 | 45000.0 | 2.8 | 11.4 | |
| 906.29 | AA(14)AAAATKKAAA-NH2 | Artificialsequence | | | 3.2 | >16431.68 | 4.7 | 43.1 | |
| 906.35 | AA(14)AAAATLEAAA-NH2 | Artificialsequence | | | 1.7 | 2675.1 | 4.0 | 5.0 | |
| 906.38 | AA(14)AAAATLFAAA-NH2 | Artificialsequence | | | 2.8 | >16431.68 | 3.0 | 6.8 | |
| 760.57 | AA(14)AAAATLKAAA-NH2 | Artificialsequence | | A | 1.4 | 1165.6 | 2.1 | 3.5 | |
| 906.47 | AA(14)AAAATLKAEA-NH2 | Artificialsequence | | | 33 | 7862.2 | 2.8 | 8.2 | |
| 906.50 | AA(14)AAAATLKAFA-NH2 | Artificialsequence | | | 0.5 | 117.4 | 1.9 | 3.3 | |
| 906.48 | AA(14)AAAATLKAQA-NH2 | Artificialsequence | | | 0.5 | 2433.3 | 2.3 | 5.5 | |
| 906.49 | AA(14)AAAATLKAVA-NH2 | Artificialsequence | | | 0.6 | 503.5 | 3.2 | 3.5 | |
| 906.41 | AA(14)AAAATLKEAA-NH2 | Artificialsequence | | | 240.6 | 4129.5 | 9.2 | 229.7 | |
| 906.44 | AA(14)AAAATLKFAA-NH2 | Artificialsequence | | | 2.2 | 59.6 | 2.9 | 37.1 | |
| 906.45 | AA(14)AAAATLKIAA-NH2 | Artificialsequence | | | 1.1 | 921.1 | 2.2 | 21.1 | |
| 906.40 | AA(14)AAAATLKKAA-NH2 | Artificialsequence | | | 79.1 | 386.4 | 11.3 | 29.9 | |
| 906.42 | AA(14)AAAATLKQAA-NH2 | Artificialsequence | | | 11.8 | 1214.9 | 5.2 | 7.3 | |
| 906.43 | AA(14)AAAATLKVAA-NH2 | Artificialsequence | | | 0.5 | 285.8 | 1.8 | 15.7 | |
| 906.36 | AA(14)AAAATLQAAA-NH2 | Artificialsequence | | | 2.6 | 1019.3 | 2.9 | 4.9 | |
| 906.39 | AA(14)AAAATLRAAA-NH2 | Artificialsequence | | | 1.1 | 790.2 | 3.9 | 3.1 | |
| 906.31 | AA(14)AAAATQKAAA-NH2 | Artificialsequence | | | 0.6 | 3602.9 | 4.3 | 12.4 | |
| 906.32 | AA(14)AAAATVKAAA-NH2 | Artificialsequence | | | 0.3 | 3834.1 | 3.5 | 3.4 | |
| 906.24 | AA(14)AAAETLKAAA-NH2 | Artificialsequence | | | 1.5 | 1004.8 | 4.0 | 4.5 | |
| 906.27 | AA(14)AAAFTLKAAA-NH2 | Artificialsequence | | | 0.5 | 1252.7 | 2.4 | 1.3 | |
| 856.02 | AA(14)AAKTAAA(35)-NH2 | Artificialsequenre | | A | 1.6 | | 2.1 | 7.8 | |
| 760.50 | AA(14)AAAKTAAAAA-NH2 | Artificialsequence | | A | 3.1 | 2345.8 | 2.8 | 7.0 | |
| 906.23 | AA(14)AAAKTLKAAA-NH2 | Artificialsequence | | | 0.8 | 672.5 | 2.0 | 4.2 | |
| 906.25 | AA(14)AAAQTLKAAA-NH2 | Artificialsequence | | | 1.6 | 581.1 | 2.3 | 4.5 | |
| 906.28 | AA(14)AAATTLKAAA-NH2 | Artificialsequence | | | 1.8 | 1058.9 | 4.5 | 7.6 | |
| 906.26 | AA(14)AAAVTLKAAA-NH2 | Artificialsequence | | | 1.1 | 680.7 | 3.0 | 2.9 | |
| 906.19 | AA(14)AAEATLKAAA-NH2 | Artificialsequence | | | 3.6 | 381.9 | 4.6 | 7.2 | |
| 906.22 | AA(14)AAFATLKAAA-NH2 | Artificialsequence | | | 1.7 | 23237.9 | 2.5 | 3.0 | |
| 906.18 | AA(14)AAKATLKAAA-NH2 | Artificialsequence | | | 7.0 | >900 | 3.0 | 29.9 | |
| 906.20 | AA(14)AAQATLKAAAA-NH2 | Artificialsequence | | | 1.0 | 17170.7 | 2.5 | 4.8 | |
| 906.21 | AA(14)AAVATLKAAA-NH2 | Artificialsequence | | | 0.7 | 4305.3 | 3.0 | 4.1 | |
| 906.14 | AA(14)AEAATLKAAA-NH2 | Artificialsequence | | | 6.5 | 3170.1 | 4.2 | 15.1 | |
| 906.17 | AA(14)AFAATLKAAA-NH2 | Artificialsequence | | | 1.6 | 461.8 | 5.0 | 5.2 | |
| 906.13 | AA(14)AKAATLKAAA-NH2 | Artificialsequence | | | 2.6 | 930.8 | 6.1 | 5.0 | |
| 906.15 | AA(14)AQAATLKAAA-NH2 | Artificialsequence | | | 2.7 | 1642.4 | 4.1 | 8.5 | |
| 906.16 | AA(14)AVAATLKAAA-NH2 | Artificialsequence | | | 0.7 | 219.9 | 5.3 | 4.3 | |
| 906.07 | AA(14)EAAATLKAAA-NH2 | Artificialsequence | | | 3.0 | 1373.8 | 3.3 | 3.9 | |
| 906.10 | AA(14)FAAATLKAAA-NH2 | Artificialsequence | | | 1.3 | 793.1 | 5.0 | 2.7 | |
| 906.11 | AA(14)IAAATLKAAA-NH2 | Artificialsequence | | | 0.4 | 70.1 | 2.9 | 3.4 | |
| 906.06 | AA(14)KAAATLKAAA-NH2 | Artificialsequence | | | 0.8 | 597.6 | 1.7 | 6.4 | |
| 906.12 | AA(14)LAAATLKAAA-NH2 | Artificialsequence | | | 0.2 | 266.1 | 3.7 | 3.4 | |
| 906.08 | AA(14)QAAATLKAAA-NH2 | Artificialsequence | | | 1.7 | 653.1 | 6.8 | 4.7 | |
| 906.09. | AA(14)VAAATLKAAA-NH2 | Artificialsequence | | | 0.6 | 107.5 | 2.7 | 5.6 | |
| 906.56 | AA(14)VVAATLKAFA | Artificialsequence | | | 0.5 | 27.2 | 1.5 | 4.5 | |
| 828.13 | AA(15)AAAATKAAAA-NH2 | Artificialsequence | | A | 1.5 | | 6.2 | 152.0 | |
| 819.01 | AA(15)AAKTAAAFA-NH2 | Artificialsequence | | A | 0.4 | | 5.8 | 3.1 | |
| 819.02 | AA(15)AAKTGGGFG-NH2 | Artificialsequence | | A | 3.3 | | 2.0 | 114.4 | |
| 819.04 | AA(15)GGKGGGGFG-NH2 | Artificialsequence | | A | 18.4 | | 576.9 | 50000.0 | |
| 819.03 | AA(15)GGKTAAAFA-NH2 | Artificialsequence | | A | 0.4 | | 1.5 | 50.0 | |
| 819.05 | AA(15)GGKTGGGFG-NH2 | Artificialsequence | | A | 94.3 | | 16.7 | 5555.6 | |
| 736.28 | AA(17)AAAAAAKAAA-NH2 | Artificialsequence | | A | 23.8 | | 15000.0 | 500000.0 | |
| 736.33 | AA(2)AAAAAAKAAA-CONH2 | Artificialsequence | | A | 87.7 | | 450000.0 | 500000.0 | |
| 773.08 | AA(31)AAAAAAAKAAA-NH2 | Artificialsequence | | | 6.4 | | 37.5 | 500000.0 | |
| 736.17 | AA(6)AAAAAAKAAA-NH2 | Artificialsequence | | A | 5.0 | | 409.1 | 442.5 | |
| 736.18 | AA(7)AAAAAAKAAA-NH2 | Artificialsequence | | A | 1.3 | | 11.3 | 925.9 | |
| 736.19 | AA(8)AAAAAAKAAA-NH2 | Artificialsequence | | A | 2.4 | | 18.9 | 649.4 | |
| 736.20 | AA(9)AAAAAAKAAA-NH2 | Artificialsequence | | A | 3.1 | | 23.7 | 50000.0 | |
| 736.34 | AA(TIC)AAAAAAKAAA- CONH2 | Artificialsequence | | A | 6.9 | | 32143 | 10000.0 | |
| 871.14 | AAAAKAATLKAAA-NH2 | Artificialsequence | | | 5000.0 | | 288.5 | 463.0 | |
| 760.27 | AAAFAAAKTAAAFA-NH2 | Artificialsequence | | A | 1.4 | 398.9 | 1.5 | 17.9 | |
| 730.04 | AAAKAAAAAA(10)AA- CONH2 | Artificialsequence | | A | 172.4 | | 1250.0 | 5000.0 | |
| 730.02 | AAAKAAAAAAFAA-CONH2 | Artificialsequence | | A | 71.43 | | 937.5 | 500000.0 | |
| 702.02 | AAAKAAAAAAYAA | Artificialsequence | | A | 833.3 | 900000.0 | 1285.7 | 500000.0 | |
| 702.06 | AAAKAAAAAAYAA-COHN2 | Artificialsequence | | A | 74.6 | 900000.0 | 204.6 | 500000.0 | |
| 789.05 | AAAKAAAAAFAAA | Artificialsequence | | | 833.3 | | 633.8 | 5555.6 | |
| 730.07 | AAAKATAAAA(23)AA- CONH2 | Artificialsequence | | A | 125.0 | | 166.7 | 25000.0 | |
| 787.06 | AADFGIFIDFILA-(NH2) | Artificialsequence | | | 1000.0 | | 172.4 | 500000.0 | |
| 736.10 | AAEAAAAAAKAAA-NH2 | Artificialsequence | | A | 1000.0 | | 22500.0 | 500000.0 | |
| 736.07 | AAFAAAAAAKAAA-NH2 | Artificialsequence | | A | 1.9 | | 15.5 | 961.5 | |
| 761.03 | AAFAAAAAARLFA-NH2 | Artificialsequence | | | A 1.1 | | 155.2 | 3846.2 | |
| 828.07 | AAFAAAAB(24)KAAAA-NH2 | Artificialsequence | | A | 13.6 | | 7.6 | 273.2 | |
| 760.71 | AAFAAAATAKAAA | Artificialsequence | | A | 1.3 | 45000.0 | 2.3 | 11.4 | |
| 760.41 | AAFAAAATAKAAA-NH2 | Artificialsequence | | A | 1.3 | 90000.0 | 1.2 | 8.1 | |
| 760.72 | AAFAAAATLKAAA | Artificialsequence | | A | 1.9 | 4803.8 | 1.4 | 2.4 | |
| 760.43 | AAFAAAATLKAAA-NH2 | Artificialsequence | | A | 1.5 | 2686.9 | 1.4 | 2.9 | |
| 871.12 | AAFAAAATLKAKA-NH2 | Artificialsequence | | | 2.9 | | 4.0 | 12.8 | |
| 871.11 | AAFAAAATLKKAA-NH2 | Artificialsequence | | | 500.0 | | 22.5 | 135.1 | |
| 760.37 | AAFAAAKTAAAAA-NH2 | Artificialsequence | | A | 2.3 | 15000.0 | 1.7 | 9.1 | |
| 803.08 | AAFAAAKTAAAFA-CONH2 | Artificialsequence | | | 1.0 | | 1.7 | 8.9 | |
| 760.15 | AAFAAAKTAAAFA-NH2 | Artificialsequence | | A | 1.3 | 1062.6 | 1.1 | 6.2 | |
| 760.33 | AAFAAAKTAAAFA-NH2 | Artificialsequence | | A | 22 | 1066.0 | 1.5 | 5.8 | |
| 760.17 | AAFAAAKTAAAFE-NH2 | Artificialsequence | | | A 1.2 | | 2.1 | 7.3 | |
| 803.13 | AAFAAAKTAAAKA-NH2 | Artificialsequence | | | 2.8 | | 6.5 | 8.2 | |
| 760.47 | AAFAAAKTLAAAA-NH2 | Artificialsequence | | A | 1.9 | 7252.4 | 0.9 | 5.0 | |
| 871.10 | AAFAAAKTLKAAA-NH2 | Artificialsequence | | | 2.0 | | 43 | 7.1 | |
| 871.09 | AAFAAKATLKAAA-NH2 | Artificialsequence | | | 43.1 | | 1.1 | 73.1 | |
| 760.70 | AAFAANKNAAFAA-CONH2 | Artificialsequence | | | 108.7 | | 62.9 | 25000.0 | |
| 760.68 | AAFAAQKQAAFAA-CONH2 | Artificialsequence | | | 500.0 | | 6428.6 | 25000.0 | |
| 760.69 | AAFAATKTAAFAA-CONH2 | Artificialsequence | | | 185.2 | | 29.4 | 8333.3 | |
| 760.64 | AAFAKAATAKAAA-CONH2 | Artificialsequence | | A | 2.4 | 45000.0 | 2.7 | 14.3 | |
| 760.63 | AAFAKAATLKAAA-CONH2 | Artificialsequence | | A | 3.3 | 8017.8 | 4.0 | 9.1 | |
| 871.06 | AAFAKAATLKAKA-NH2 | Artificialsequence | | | 12.8 | | 3.2 | 19.2 | |
| 871.05 | AATAKAATLKKAA-NH2 | Artificialsequence | | | 500.0 | | 20.5 | 150.2 | |
| 871.04 | AAFAKAKTLKAAA-NH2 | Artificialsequence | | | 8.2 | | 3.6 | 33.3 | |
| 871.03 | AAFAKKATLKAAA-NH2 | Artificialsequence | | | 312.5 | | 16.7 | 191.6 | |
| 787.05 | AAFFGIFKIGKFA-(NH2) | Artificialsequence | | | 833.3 | | 1097.6 | 500000.0 | |
| 787.11 | AAFGIKIFQFKIA-(NH2) | Artificialsequence | | | 50000.0 | | 671.6 | 500000.0 | |
| 871.02 | AAFKKAATLKAAA-NH2 | Artificialsequence | | | 5.0 | | 2.5 | 7.5 | |
| 770.04 | AAFPPPPTLKAAA-NH2 | Artificialsequence | | | 714.3 | | 15000.0 | | |
| 793.03 | AAFVSQTTLKAAA | Artificialsequence | | A | 2.3 | | 2.0 | 5.5 | |
| 736.06 | AAHAAAAAAKAAA-NH2 | Artificialsequence | | A | 17.9 | | 45000.0 | 500000.0 | |
| 787.03 | AAIGFFFFKKGIA-(NH2) | Artificialsequence | | | 50000.0 | | 252.8 | 500000.0 | |
| 787.07 | AAIGGIFIKKDA-(NH2) | Artificialsequence | | | 50000.0 | | 957.5 | 500000.0 | |
| 736.08 | AALAAAAAAKAAA-NH2 | Artificialsequence | | A | 1.9 | | 69.2 | 151.5 | |
| 760.09 | AALKATAAAAYAA-NH2 | Artificialsequence | | A | 500.0 | | 1406.3 | 500000.0 | |
| 736.09 | AAWAAAAAAKAAA-NH2 | Artificialsequence | | A | 1.7 | | 23.7 | 8333.3 | |
| 758.01 | AAYA(4)A(4)AAKAAA | Artificialsequence | | A | 38.5 | | 4500.0 | 500000.0 | |
| 758.02 | AAYAA(4)A(4)AKAAA | Artificialsequence | | A | 5.9 | | 535.7 | 500000.0 | |
| 773.06 | AAYAAAA(24)AKAAA-NH2 | Artificialsequence | | | 10.2 | | 3.0 | 128.2 | |
| 773.02 | AAYAAAA(26)AKAAA-NH2 | Artificialsequence | | | 161.3 | | 15.0 | 500000.0 | |
| 773.03 | AAYAAAA(27)AKAAA-NH2 | Artificialsequence | | | 128.2 | | 321.4 | 500000.0 | |
| 773.04 | AAYAAAA(28)AKAAA-NH2 | Artificialsequence | | | 5.8 | | 11.8 | 12500.0 | |
| 773.05 | AAYAAAA(29)AKAAA-NH2 | Artificialsequence | | | 12.8 | | 6.9 | 23000.0 | |
| 773.07 | AAYAAAA(30)AKAAA-NH2 | Artificialsequence | | | 142.9 | | 2142.9 | 500000.0 | |
| 702.03 | AAYAAAAAAKAAA-CONH2 | Artificialsequence | | A | 2.6 | 900000.0 | 6.8 | 500000.0 | |
| 773.09 | AAYAAAAPAKAAA-CONH2 | Artificialsequence | | | 1.0 | | 33.8 | 25000.0 | |
| 736.04 | AAYAAAATAAAKA-NH2 | Artificialsequence | | A | 2.5 | | 2.3 | 1724.1 | |
| 736.03 | AAYAAAATAKAAA-NH2 | Artificialsequence | | A | 3.9 | | 3.1 | 2777.8 | |
| 736.09 | AAYAAAAYAAAKA-NH2 | Artificialsequence | | A | 166.7 | | 28.1 | 500000.0 | |
| 736.14 | AAYAAJJAAKAAA-NH2 | Artificialsequence | | A | 1000.0 | | 3214.3 | 500000.0 | |
| 871.13 | AAYAKAATLKAAA-NH2 | Artificialsequence | | | 5.5 | | 4.0 | 213.7 | |
| 760.58 | AC-AA(14)AAAATLKAAA- NH2 | Artificialsequence | | A | 2.0 | 727.8 | 1.7 | 2.8 | |
| 760.51 | AC-AA(14)AAAKTAAAAA- NH2 | Artificialsequence | | A | 7.6 | 4201.7 | 17.3 | 29.4 | |
| 730.13: | AC-AAAKAAAAAA(23)AA- CONH2 | Artificialsequence | | A | 1000.0 | | 548.8 | 1136.4 | |
| 702.08 | AC-AAAKAAAAAAYAA | Artificialsequence | | A | 625.0 | | 1956.5 | | |
| 702.12 | AC-AAAKAAAAAAAYAA- CONH2 | Artificialsequence | | A | 1000.0 | | 692.3 | 16666.7 | |
| 760.42 | AC-AAFAAAATAKAAA-NH2 | Artificialsequence | | A | 2.5 | 25980.8 | 3.5 | 2.1 | |
| 760.44 | AC-AAFAAAATLKAAA-NH2 | Artificialsequence | | A | 3.5 | 2358.6 | 3.3 | 18.5 | |
| 760.38 | AC-AAFAAAKTAAAAA-NH2 | Artificialsequence | | A | 3.9 | | 1.3 | 19.9 | |
| 760.34 | AC-AAFAAAKTAAAFA-NH2 | Artificialsequence | | A | 1.7 | 1530.9 | 2.5 | 13.5 | |
| 760.48 | AC-AAFAAAKTLAAAA-NH2 | Artificialsequence | | A | 17.0 | | 16.4 | 111.1 | |
| 760.67 | AC-AAFAANKNAAFAA- CONH2 AC-AAFAA[?][?][?]AAFAA[?] | Artificialsequence | | | 500.0 | | 44.6 | 25000.0 | |
| 760.65 | AC-AAFAAQKQAAFAA- CONH2 | Artificialsequence | | | 2500.0 | | 5000.0 | 25000.0 | |
| 760.66 | AC-AAFAATKTAAFAA- CONH2 | Artificialsequence | | | 454.6 | | 38.5 | 25000.0 | |
| 806.06 | AC-YAA(10)LFLSAARKRA- NH2 | Artificialsequence | | A | 5.7 | | 300.0 | 500000.0 | |
| 760.60 | AC-YAA(14)AAAATLKAAA- NH2 | Artificialsequence | | A | 3.1 | 2253.5 | 6.4 | 12.5 | |
| 760.53 | AC-YAA(14)AAAKTAAAAA- NH2 | Artificialsequence | | A | 1.2 | 18000.0 | 1.2 | 20.8 | |
| 760.55 | AC-YAAFAAAATAKAAA-NH2 | Artificialsequence | | A | 28.9 | 900000.0 | 9.6 | 125.0 | |
| 760.46 | AC-YAAFAAAATLKAAA-NH2 | Artificialsequence | | A | 4.0 | 12857.1 | 2.0 | 9.5 | |
| 760.40 | AC-YAAFAAAKTAAAAA-NH2 | Artificialsequence | | A | 14.3 | 900000.0 | 16.7 | 30.3 | |
| 760.36 | AC-YAAFAAAKTAAAFA-NH2 | Artificialsequence | | A | 6.9 | 10000.0 | 6.4 | 17.9 | |
| 760.62 | AC-YAAFAAAKTLAAAA-NH2 | Artificialsequence | | A | 7.5 | 18000.0 | 1.8 | 4.4 | |
| 805.03 | AC-YARFLALTTLRARA- CONH2 | Artificialsequence | | A | 1.7 | | 2.3 | 10.0 | |
| 906.02 | AE(14)AAAATLKAAA-NH2 | Artificialsequence | | | 3.7 | 512.2 | 4.2 | 12.0 | |
| 760.13 | AEFAAAKTLAAFA-NH2 | Artificialsequence | | A | 1.7 | | 2.4 | 5.1 | |
| 906.05 | AF(14)AAAATLKAAA-NH2 | Artificialsequence | | | 0.9 | 554.9 | 1.7 | 1.8 | |
| 851.09 | AFAAAKTAA(71) | Artificialsequence | | | 1666.7 | | 5.6 | 657.9 | |
| 803.05 | AFAAAKTAAAA-CONH2 | Artificialsequence | | | 151.5 | | 6.5 | 2777.8 | |
| 803.04 | AFAAAKTAAA-NH2 | Artificialsequence | | | 5000.0 | | 200.9 | 50000.0 | |
| 760.05 | AFLRAAAAAFAAY-NH2 | Artificialsequence | | A | 625.0 | | 1153.9 | 500000.0 | |
| 906.01 | AK(14)AAAATLKAAA-NH2 | Artificialsequence | | | 1.7 | 786.0 | 2.0 | 5.0 | |
| 965.16 | AK(14)VAAATLKAAA-NH2 | Artificialsequence | | | 4.2 | 558.2 | 7.2 | 71.0 | |
| 965.15 | AK(14)VAAHTLKAAA-NH2 | Artificialsequence | | | 2.1 | 514.5 | 3.2 | 17.4 | |
| 965.14 | AK(14)VAAKTLYAAA-NH2 | Artificialsequence | | | 5.9 | 296.1 | 8.5 | 58.2 | |
| 965.08 | AK(14)VAANTLKAAA-NH2 | Artificialsequence | | | 2.5 | 105.4 | 3.0 | 25.4 | |
| 965.08 | AK(14)VAANTLKAAA-NH2 | Artificialsequence | | | 0.7 | 484.5 | 1.3 | 4.8 | |
| 965.10 | AK(14)VAAWTLKAAA-NH2 | Artificialsequence | | | 23 | 55.0 | 3.7 | 14.3 | 58.8 |
| 965.17 | AK(14)VAAWTLKAAA-NH2 | Artificialsequence | | | 2.0 | 678.4 | 3.8 | 34.0 | |
| 965.09 | AK(14)VAAYTLKAAA-NH2 | Artificialsequence | | | 1.7 | 69.4 | 2.2 | 12.8 | |
| 965.09 | AK(14)VAAYTLKAAA-NH2 | Artificialsequence | | | 0.6 | 1129.3 | 1.1 | 7.1 | |
| 965.07 | AK(14)VKAHTLKAAA-NH2 | Artificialsequence | | | 1.6 | 989.1 | 1.8 | 10.1 | |
| 965.07 | AK(14)VKAHTLKAAA-NH2 | Artificialsequence | | | 1.3 | 1458.0 | 1.4 | 6.7 | |
| 965.01 | AK(14)VKANTLKAAA-NH2 | Artificialsequence | | | 1.5 | 83.6 | 3.1 | 4.0 | |
| 965.01 | AK(14)VKANTLKAAA-NH2 | Artificialsequence | | | 1.3 | 9253 | 3.5 | 5.9 | |
| 965.02 | AK(14)VKAWTLKAAA-NH2 | Artificialsequence | | | 0.9 | 223.0 | 1.6 | 4.8 | |
| 965.02 | AK(14)VKAWTLKAAA-NH2 | Artificialsequence | | | 0.7 | 2574.6 | 0.8 | 1.5 | |
| 965.03 | AK(14)VWANTLKAAA-NH2 | Artificialsequence | | | 1.7 | 107.4 | 2.2 | 5.8 | |
| 965.03 | AK(14)VWANTLKAAA-NH2 | Artificialsequence | | | 0.4 | 275.6 | 1.0 | 3.5 | |
| 965.04 | AK(14)VWAYTLKAAA-NH2 | Artificialsequence | | | 0.5 | 27.8 | 0.6 | 2.8 | |
| 965.04 | AK(14)VWAYTLKAAA-NH2 | Artificialsequence | | | 0.3 | 724.1 | 1.1 | 3.6 | |
| 965.05 | AK(14)VYAWTLKAAA-NH2 | Artificialsequence | | | 0.5 | 58.8 | 0.3 | 4.4 | |
| 965.05 | AK(14)VYAWTLKAAA-NH2 | Artificialsequence | | | 0.8 | 3368.2 | 0.8 | 6.1 | |
| 537.00 | AKAAKAAKAAKAAKAA | Artificialsequence | | | 1250.0 | 900000.0 | 22500.0 | 500000.0 | |
| 871.07 | AKFAAAATLKAAA-NH2 | Artificialsequence | | | 2.9 | | 1.3 | 10.9 | |
| 803.14 | AKFAAAKTAAAKA-NH2 | Artificialsequence | | | 4.3 | | 4.2 | 33.3 | |
| Sandoz339 | AKFAAALTLAAPA-NH2 | Artificialsequence | | | 19.2 | | 7.1 | 6.3 | |
| Sandoz338 | AKFAAALTLAQAA-NH2 | Artificialsequence | | | 238.1 | | 32.1 | 35.7 | |
| 1303.02 | AKFIAADTLKAAA | Artificialsequence | | A | | 991.8 | | | |
| 1303.01 | AKFIAAWTLKAAA | Artificialsequence | | A | | 495.9 | 5.2 | | |
| 1024.03 | AKFVAAWTLKAAA-NH2 | Artificialsequence | | | 1.2 | 599.2 | 1.8 | 8.2 | 11.5 |
| 1024.06 | AKFVAAWTLKAAA-NH2 | Artificialsequence | | | 3.9 | 805.7 | 5.2 | | 49.1 |
| 1024.04 | AKFVAAYTLKAAA-NH2 | Artificialsequence | | | 0.9 | 860.1 | 4.8 | 7.0 | |
| 1303.04 | AKFVADWTLKAAA | Artificialsequence | | A | 78.6 | 38.5 | 28.8 | | |
| 1303.03 | AKFVIAWTLKAAA | Artificialsequence | | A | 1.8 | 132.9 | 11.2 | | |
| 1024.01 | AKFVWANTLKAAA-NH2 | Artificialsequence, | | | 4.0 | 757.7 | 1.6 | 8.1 | |
| 1024.62 | AKFVYANTLKAAA-NH2 | Artificialsequence | | | 3.3 | 888.1 | 6.1 | 12.4 | |
| 1303.08 | AKIVAAWTLKAAA | Artificialsequence | | A | 7.1 | 409.6 | 20.3 | | |
| 1303.06 | AKIVADWTLKAAA | Astificialsequence | | A | 2506.3 | 80.0 | 146.3 | | |
| 1303.09 | AKLVAAWTLKAAA | Artificialsequence | | A | | 943.5 | | | |
| 1303.10 | AKMVAAWTLKAAA | Artificialsequence | | A | 17.8 | 819.1 | 19.1 | | |
| 1303.07 | AKMVADWTLKAAA | Artificialsequence | | A | 3657.6 | 98.6 | 143.0 | | |
| 906.03 | AQ(14)AAAATLKAAA-NH2 | Artificialsequence | | | 1.7 | 296.1 | 2.4 | 2.1 | |
| 965.06 | AR(14)VRANTLKAAA-NH2 | Artificialsequence | | | 0.8 | 1715.6 | 1.4 | 4.8 | |
| 820.07 | AR(15A)RQTTLKCAAA-NH2 | Artificialsequence | | A | 1.3 | | 3.3 | 5.1 | |
| 788.06 | ARFQRQTTLKAAA | Artificialsequence | | A | 3.9 | | 6.1 | 20.0 | |
| 781.08 | ARFQRQTTLKAAA-NH2 | Artificialsequence | | A | 2.4 | | 2.0 | 7.8 | |
| 640.05 | ARRLKARRLKAIY | Artificialsequence | | | 50.0 | 900000.0 | 22500.0 | 12500.0 | |
| 906.04 | AV(14)AAAATLKAAA-NH2 | Artificialsequence | | | 0.4 | 334.5 | 3.2 | 3.8 | |
| 906.51 | AV(14)AVAATLKAFA | Artificialsequence | | | 0.5 | 15.1 | 1.1 | 2.5 | |
| 906.55 | AV(14)VFAATLKAFA | Artificialsequence | | | 0.7 | 196.5 | 1.8 | 7.6 | |
| 906.52 | AV(14)VVAATLKAAA | Artificialsequence | | | 0.5 | 88.5 | 1.8 | 3.8 | |
| 906.53 | AV(14)VVAATLKAFA | Artificialsequence | | | 0.4 | 46.4 | 1.1 | 2.2 | |
| 640.01 | AWRNRAKAWRNRAKAWRN RAK | Artificialsequence | | | 1666.7 | 900000.0 | 15000.0 | 500000.0 | |
| 640.03 | AWRNRAKAWRNRAKGTD | Artificialsequence | | | 1666.7 | 900000.0 | 15000.0 | 500000.0 | |
| 758.03 | AYAAA(4)A(4)KAAA | Artificialsequence | | A | 92.6 | | 264.7 | 500000.0 | |
| S5004 | AYZAYAYTLKAAA | Artificialsequence | | | 18.8 | | 25.0 | 32.1 | |
| S5003 | BOC-AYZAYAYTLKAAA | Artificialsequence | | | 72.5 | | 10.2 | 73.5 | |
| S1395 | BOCXFAXAXTLKAAA | Artificialsequence | | | 20.8 | | 12.5 | 62.5 | |
| 631.02 | DDYVKQYTKQYTKQNTLKK | Artificialsequence | | | 50000.0 | | 900.0 | 500000.0 | |
| 631.03 | DDYVKQYTKQYTKQNTLKK | Actificialsequence | | | 238.1 | >30000 | 11250.0 | 500000.0 | |
| 760.08 | EFAAATKAAAFAAY-NH2 | Artificialsequence | | A | 8333 | | 2142.9 | 500000.0 | |
| 819.06 | GG(15)GGKGGGGFG-NH2 | Artificialsequence | | A | 58.8 | | 403.4 | 3571.4 | |
| 736.15 | JJYJJAAAAKAAA-NH2 | Artificialsequence | | A | 50000.0 | | 22500.0 | 500000.0 | |
| 640.06 | KNRAKNRAKNRAKNRAK | Artificialsequence | | | 1666.7 | 900000.0 | 15000.0 | 500000.0 | |
| 640.07 | KRLKRLKRLKRLKRL | Artificialsequence | | | 1666.7 | 314.2 | 22500.0 | 6250.0 | |
| 965.19 | KSSAK(14)VAAWTLKAAA- NH2 | Artificialsequence | | | 11.3 | | 4.1 | 41.7 | 34.8 |
| 784.08 | PKYDKQGGLKIAT | Artificialsequence | | A | 151.5 | | 22500.0 | 500000.0 | |
| 784.05 | PKYFKQFRLKIAT | Artificialsequence | | A | 1000.0 | | 15000.0 | 25000.0 | |
| 784.13 | PKYFKQIGLKRAT | Artificialsequence | | A | 200.0 | | 633.8 | 25000.0 | |
| 784.04 | PKYGKQRFLKIAT | Artificialsequence | | A | 5000.0 | | 900.0 | 10000.0 | |
| 784.15 | PKYIKQDGLKGAT | Artificialsequence | | A | 31.3 | | 11.8 | 16666.7 | |
| 784.09 | PKYIKQFFLKRAT | Artificialsequence | | A | 50000.0 | | 22500.0 | 746.3 | |
| 784.01 | PKYIKQIILKIAT | Artificialsequence | | A | 1250.0 | | 500.0 | 416.7 | |
| 784.10 | PKYRKQFILKGAT | Artificialsequence | | A | 104.2 | | 23.7 | 500000.0 | |
| 736.01 | PNYAAAAAAKAAA-NH2 | Artificialsequence | | A | 1.7 | | 25.0 | 500000.0 | |
| 736.31 | PNYAAAAAAKAA-CONK2 | Artificialsequence | | A | 3.1 | | 30.0 | 50000.0 | |
| 781.07 | RFQRQTTLKAAA-NH2 | Artificialsequence | | A | 10.9 | | 3.5 | 9.1 | |
| 716.02 | TAIKKLTTQRQFRAY | Artificialsequence | | A | 1000.0 | >45000 | 2500.0 | 25000.0 | |
| 717.11 | TKQKLTTQSQFRAY-NH2 | Artificialsequence | | A | 1666.7 | 900000.0 | 9000.0 | 500000.0 | |
| S5006 | XFAXAXTLKAAA | Artificialsequence | | | 50.0 | | 11.8 | 166.7 | |
| Sandoz364 | YA(AMK)FAAAKTAAAFA- NH2 | Artificialsequence | | | 96.2 | | 6.1 | 200.0 | |
| Sandoz366 | YA(K1)FQ(K1)QTTL(K1)AAA- OH | Artificialsequence | | | 403 | | 7.1 | 35.7 | |
| 806.05 | YAA(10)LFLSAARKRA-NH2 | Artificialsequence | | A | 8.1 | 36742.4 | 250.0 | 500000.0 | |
| 828.04 | YAA(14)AAAA(24)KAAAA- NH2 | Artificialsequence | | A | 37 | 900000.0 | 33 | 24.4 | |
| 828.02 | YAA(14)AAAAPKAAAA-NH2 | Artificialsequence | | A | 0.8 | | 20.5 | 675.7 | |
| 828.12 | YAA(14)AAAATKAAAA-NH2 | Artificialsequence | | A | 1.1 | 45000.0 | 2.9 | 6.9 | |
| 760.59 | YAA(14)AAAATLKAAA-NH2 | Artificialsequence | | A | 1.4 | 353.1 | 2.0 | 3.0 | |
| 760.52 | YAA(14)AAAKTAAAAA-NH2 | Artificialsequence | | A | 0.4 | 1000.8 | 0.9 | 1.5 | |
| 906.54 | YAA(14)AVAATlKAAA | Artificialsequence | | | 3.2 | 3232.9 | 2.7 | 7.5 | |
| 828.14 | YAA(15)AAAATKAAAA-NH2 | Artificialsequence | | A | 1.2 | | 3.0 | 71.0 | |
| Sandoz363 | YAA(AMF)AAAKTAAAFA- NH2 | Artificialsequence | | | 172.4 | | 10.7 | 55.0 | |
| 760.26 | YAAA1CAAAAAAFAA-NH2 | Artificialsequence | | A | 555.6 | | 5625.0 | 500000.0 | |
| Sandoz368 | YAAFAAA(DHPS)TAAAFA- NH2 | Artificialsequence | | | 20.0 | | 0.8 | 33.3 | |
| Sandoz367 | YAAFAAA(K1)TAAAFA-NH2 | Artificialsequence | | | 7.0 | | 0.9 | 10.2 | |
| 828.08 | YAAFAAAA(24)KAAAA-NH2 | Artificialsequence | | A | 12.3 | | 6.3 | 144.5 | |
| 760.25 | YAAFAAAAAAKAAA-NH2 | Artificialsequence | | A | 4.6 | | 15.5 | 1388.9 | |
| 761.04 | YAAFAAAAAARLFA-NH2 | Artificialsequence | | A | 18.5 | | 375.0 | 16666.7 | |
| 761.02 | YAAFAAAAAQRLFA-NH2 | Artificialsequence | | A | 1.1 | | 52.9 | 5555.6 | |
| 760.54 | YAAFAAAATAKAAA-NH2 | Artificialsequence | | A | 1.9 | 15212.8 | 1.3 | 7.5 | |
| 760.45 | YAAFAAAATLKAAA-NH2 | Artificialsequence | | A | 0.6 | 2070.2 | 1.2 | 4.9 | |
| 760.39 | YAAFAAAKTAAAAA-NH2 | Artificialsequence | | A | 1.8 | 8181.8 | 1.3 | 3.9 | |
| 760.39 | YAAFAAAKTAAAAA-NH2 | Artificialsequence | | A | 6.5 | 30000.0 | 10.2 | 73.5 | |
| 760.39 | YAAFAAAKTAAAAA-NH2 | Artificialsequence | | A | 1.2 | 4285.7 | 1.1 | 6.4 | |
| 760.16 | YAAFAAAKTAAAFA-NH2 | Artificialsequence | | A | 0.7 | | 0.7 | 5.0 | |
| 760.35 | YAAFAAAKTAAAFA-NH2 | Artificialsequence | | A | 13.5 | 2214.3 | 15.5 | 53.8 | |
| Sandoz362 | YAAFAAAKTAAAFA-NH2 | Artificialsequence | | | 1.9 | | 4.6 | 7.0 | |
| 760.18 | YAAFAAAKTAAAFE-NH2 | Artificialsequence | | A | 1.0 | | 1.3 | 7.7 | |
| 760.61 | YAAFAAAKTLAAAA-NH2 | Artificialsequence | | A | 1.5 | 4824.5 | 2.0 | 5.8 | |
| 788.05 | YAAFQRQTTLKAAA | Artificialsequence | | A | 2.6 | | 1.6 | 17.9 | |
| 788.04 | YAAFQSQTILKAAA | Artificialsequence | | A | 1.9 | | 1.9 | 6.9 | |
| 793.02 | YAAFVRQTTLKAAA | Artificialsequence | | A | 2.6 | | 2.4 | 7.6 | |
| 793.01 | YAAFVSQTTLKAAA | Artificialsequence | | A | 1.9 | | 1.3 | 5.1 | |
| 760.14 | YAEFAAAKTLAAFA-NH2 | Artificialsequence | | A | 2.3 | | 2.7 | 7.9 | |
| 781.16 | YAK(14)QRQTTLKAAA- CONH2 | Artificialsequence | | A | 5.0 | | 4.7 | 16.1 | |
| Sandoz370 | YAKFAAAKTAAAA(TR) | Artificialsequence | | | 6.4 | | 6.6 | 19.2 | |
| 717.08 | YAKFKSTTKKRIKS-NH2 | Artificialsequence | | A | 50000.0 | 900000.0 | 450000.0 | 500000.0 | |
| 1341.01 | YAKFVAAWTLKAAA | Artificialsequence | | A | | | | | |
| 717.3.5 | YAR(10)QSQTTLKAKT-NH2 | Artificialsequence | | A | 3.9 | | 5.8 | 80.7 | |
| 781.20 | YAR(14)QKQTTLKAAA | Artificialsequence | | A | 8.9 | 900000.0 | 4.0 | 10.0 | |
| 788.01 | YAR(14)QRQTTLKAAA | Artificialsequence | | A | 31.3 | | 63.4 | 1041.7 | |
| 781.15 | YAR(14)QRQTTLKAAA- CONH2 | Artificialsequence | | A | 3.9 | | 2.5 | 15.6 | |
| 782.03 | YAR(15)ASQTTLKAKT-NH2 | Artificialsequence | | | 1.5 | | 2.6 | 5.2 | |
| 782.07 | YAR(15)ASQTTLKAKT-NH2 | Artificialsequence | | | 41.7 | | 1.7 | 29.4 | |
| 820.02 | YAR(15A)RQTTLKAAA-NH2 | Artificialsequence | | A | 1.6 | | 2.0 | 3.8 | |
| 820.11 | YAR(15A)RQTTLKAAA-NH2 | Artificialsequence | | A | 4.4 | | 3.8 | 4.3 | |
| 820.01 | YAR(15A)SQTTLKAKT-NH2 | Artificialsequence | | A | 2.4 | | 4.4 | 5.2 | |
| 820.10 | YAR(15A)SQTTLKAKT-NH2 | Artificialsequence | | A | 10.7 | | 5.1 | 25.6 | |
| 782.04 | YAR(16)ASQTTLKAKT-NH2 | Artificialsequence | | | 192.3 | 900000.0 | 3.5 | 92.6 | |
| 717.34 | YAR(9)QSQTTLKAKT-NH2 | Artificialsequence | | A | 27.8 | | 4.4 | 41.7 | |
| 717.43 | YAREQSQTTLKAKT-NH2 | Artificialsequence | | A | 50000.0 | | 750.0 | 500000.0 | |
| 782.06 | YARFGGQTTLKAKT | Artificialsequence | | | 357.1 | | 6.1 | 56.2 | |
| 805.01 | YARFLALTTLRARA-CONH2 | Artificialsequence | | A | 1.0 | | 1.6 | 3.4 | |
| 805.02 | YARFLALTTLRARA-CONH2 | Artificialsequence | | A | 2.0 | | 5.8 | 12.5 | |
| 717.36 | YARFQSQT(24)LKAKT-NH2 | Artificialsequence | | A | 4.6 | | 5.8 | 12.8 | |
| 717.18 | YARFQSQTELKAKT-NH2 | Artificialsequence | | A | 714.3 | | 264.7 | 16666.7 | |
| 782.01 | YARFQSQTTL(32)AKT-NH2 | Artificialsequence | | | 3.6 | 900000.0 | 1.7 | 15.2 | |
| 717.24 | YARFQSQTTLKEKT-NH2 | Artificialsequence | | A | 50000.0 | | 30.0 | 2083.3 | |
| 715.01 | YARRLKAIFARRLKA | Artificialsequence | | | 38.5 | | 11250.0 | | |
| 788.03 | YASFQSQTTLKAAA | Artificialsequence | | A | 3.1 | | 3.1 | 9.3 | |
| 788.02 | YASFVSQTTLKAAA | Artificialsequence | | A | 1.7 | | 2.9 | 12.8 | |
| 785.01 | YKKSIQFHWKNSNQIKILGNQ GSFLTKGPS | Artificialsequence | | | 5.7 | 11078.2 | 5.6 | 23.8 | |
| 717.09 | YPKFVKQNTLKAAT-NH2 | Artificialsequence | | A | 555.6 | 900000.0 | 5000.0 | 16666.7 | |
| 806.01 | YQFIKANSKFKGKFK-NH2 | Artificialsequence | | A | 1666.7 | | 535.7 | 500000.0 | |
| 768.01 | YSSFSSSSSSKSSS | Artificialsequence | | A | 1000.0 | | 7.9 | | |
| 768.02 | YSSFSSSSSSKSSS-NH2 | Artificialsequence | | A | 714.3 | 900000.0 | 5.8 | 192.3 | |
| 768.03 | YSSFSSSSSSSSSS | Artificialsequence | | A | 50000.0 | | 6.9 | | |
| 768.04 | YSSFSSSSSSSSSS-NH2 | Artificialsequence | | A | 5000.0 | | 5.8 | 416.7 | |
| 564.01 | YVKADYVKADYVKADYVK | Artificialsequence | | | 1666.7 | | 957.5 | 500000.0 | |
| 718.01 | YVKQNTLAFVKQNTLA | Artificialsequence | | A | 172.4 | | 239.4 | 5000.0 | |
| 631.01 | YVKQYTKQYTKQNTLK | Artificialsequence | | | 50000.0 | | 4500.0 | 500000.0 | |
| S1396 | ZAAFAAAATLKAAA | Artificialsequence | | | 25.0 | | 11.5 | 54.4 | |
| S1399 | ZAAFAAAXTAYAYA | Artificialsequence | | | 33.3 | | 7.8 | 122.0 | |
| S1400 | ZAAFAXAATAYAYA | Artificialsequence | | | 18.5 | | 9.0 | 75.8 | |
| S5005 | ZAAFAXAATLKAAA | Artificialsequence | | | 16.1 | | 7.3 | 24.6 | |

**TABLE 27b**

| | | HLA-DR binding affinity (IC50 nM) | | | | | | | | | | |
|---|---|---|---|---|---|---|---|---|---|---|---|---|
| Peptide | Sequence | DRB1 *0701 | DRB1 *0802 | DRB1 *0901 | DRB1 *1101 | DRB1 *1201 | DRB1 *1302 | DRB1 *1501 | DRB3 *0101 | DRB4 *0101 | DRB5 *0101 | DQB1 *0301 |
| PTS-04 | AAWSERAGEAMVLV | >25000 | | | 10000.0 | | | 91000.0 | >10000 | | | 256.6 |
| 582.02 | DNVLDHLTGRSC | >25000 | | | 10000.0 | | | 91000.0 | 11111.1 | | | >25000 |
| PTS-10 | NH2-DNVLAHLTGRSCQ- COOH | | | | | | | 91000.0 | | | | |
| PTS-17 | NH2-DNVLDHLTGRSAQ- COOH | | | | | | | 9100.0 | | | | |
| PTS-18 | NH2-DNVLDHLTGRSCA- COOH | | | | | | | 91000.0 | | | | |
| 541.19 | HYTVDKSKPKVYQWRD | 250000.0 | | | 1250.0 | | | 700.0 | 2985.4 | | | |
| 541.15 | INTKCYKLEHPVTGCG | 250000.0 | | | 1250.0 | | | 1820.0 | >7692.31 | | | |
| 541.04 | NELGRFKHTDACCRTH | 1550.4 | | | 266.7 | | | 28.4 | >9090.91 | | | |
| 541.11 | NNDFYDNSADTISSYF | 5000.0 | | | 1250.0 | | | 1820.0 | 1807.8 | | | |
| 541.20 | SKPKVYQWFDLRKY | 1666.7 | | | 222.2 | | | 1011.1 | 2565.0 | | | |
| 541.13 | SSYFVGKMYFNLINTK | 250000.0 | | | 339.0 | | | 606.7 | >7692.31 | | | |
| BMP | PDTRPAPGSTAPPAHGVTSAP DTR-NH2 | >25000 | | | 10000.0 | | | 91000.0 | >25000 | | | 10206.2 |
| 1136.18 | ALPVFTWLALYFTSAK | 1181.1 | 20104.9 | | 4847.9 | | 1666.7 | 598.7 | | 2790.5 | 1182.6 | |
| 1136.20 | ALYFTSAKIPQEWKPA | 381.6 | 134.6 | | 59.0 | | >8750 | 7690.9 | | >15681.4 | 8207.6 | |
| 1136.15 | FATSFLIPLTSQFFLP | 41.8 | 21304.4 | | 9523.8 | | 8750.0 | 48.0 | | 592.0 | 42.9 | |
| 1136.52 | GLFCRWSYTEIEKIDI | 555.7 | 30625.0 | 2420.8 | >6405.13 | >2580.87 | 17500.0 | 5698.6 | | 791.3 | 1690.3 | |
| 1136.53 | GLFSRWSYTEIEKIDI | 298.7 | 11650.2 | | >9428.09 | | 4341.2 | >30333.33 | | 1607.4 | >47140.45 | |
| 1136.48 | IGGAMLSLTVFEFTKY | 1946.3 | >3357.42 | 12364.1 | >7427.81 | 123.7 | 11666.7 | 15166.7 | | 40312.8 | >2439.21 | |
| 1136.13 | IKLPIILAFATSFLIP | 112.4 | 16257.4 | | >7767.36 | | 53.4 | 0.8 | | 1676.4 | 63.1 | |
| 1136.43 | IMEVLFLSWLFPRFKF | 104.4 | >34648.23 | | 9090.9 | | 1144.0 | 538.1 | | >16018.68 | >47140.45 | |
| 1136.67 | LTNNDQVSGINEEDEE | >33407.66 | >34648.23 | | >6405.13 | | 576.2 | >37150.59 | | >42758.17 | >47140.45 | |
| 1136.01 | MMASSILRSKIIQKPYQ | >27777.78 | 245.3 | 24607.0 | 2112.9 | >2651.58 | >8750 | 1690.4 | | >10826.47 | 85.2 | |
| 1136.41 | PSLHSGSSIMEVLFLS | 13325.0 | >34648.23 | | >7767.36 | | >8750 | >37150.59 | | 6080.1 | >81649.66 | |
| 1136.39 | SSIIFGAFPSLHSGSS | 1228.7 | 15765.5 | | 1598.0 | | 1909.4 | 761.0 | | 447.5 | 470.6 | |
| 1136.10 | VSILFFVFVVFPASFF | 1041.7 | 13876.0 | 3413.7 | 5834.6 | >2904.73 | 4044.6 | >9989.28 | | 8004.0 | 7372.1 | |
| 58.0041 | IAKITPDNNGTYACF | | | | | | | | | | | |
| 58.0022 | IPNTTVDNSGSYTCQ | | | | | | | | | | | |
| 58.0012 | IQNIIQDDTGFYTLH | | | | | | | | | | | |
| 58.0037 | LFNVTRDDARAYVCG | | | | | | | | | | | |
| 58.0017 | LFNVTRDDTASYKCE | | | | | | | | | | | |
| 58.0028 | LLSVTRDDVGPYECG | | | | | | | | | | | |
| 58.0015 | LWWVNNESLPVSPRL | | | | | | | | | | | |
| 58.0027 | LWWVNNESLPVSPRL | | | | | | | | | | | |
| 58.0021 | QELFIPDITVNNSGS | | | | | | | | | | | |
| 58.0032 | QELFISDITEKNSGL | | | | | | | | | | | |
| 58.0009 | REIIYPDASLLIQNI | | | | | | | | | | | |
| 58.0020 | SCHAASDPPAQYSWF | | | | | | | | | | | |
| 58.0023 | TTTVYADPPKPFTS | | | | | | | | | | | |
| 58.0005 | VLLLVHDLPQHLFGY | | | | | | | | | | | |
| 58.0042 | YACFVSDLATGRNNS | | | | | | | | | | | |
| CMP | PTTTPITTTTTTVTPTPTPTGTQT YC-NH2 | >25000 | | | 10000.0 | | | 91000.0 | >25000 | | | >25000 |
| 722.01 | YIAFLKQATAK | >25000 | | | 3333.3 | | | 91000.0 | >50000 | | | >25000 |
| 500.02 | FLRRIRPKLK | | | | 2857.1 | | | 11.1 | | | | |
| 199.02 | GFLRRIRPKLK | | | | 625.0 | | | 6.1 | | | | |
| 199.03 | LRRIRPKLK | | | | 4000.0 | | | 21.7 | | | | |
| 199.01 | YGGFLRRIRPKLK | >25000 | | | 274.0 | | | 4.1 | >16666.67 | | | 10206.2 |
| 510.12 | DDNGPQDPDNTDDNG | 25000.0 | | | 10000.0 | | | 7583 | >7692.31 | | | >25000 |
| F166.01 | PYYTGEHAKAIGN | >16137.43 | | | | | >10103.63 | | | | | |
| 897.07 | FPQPQLPYSQPQPFRPQQPY- NH2 | 250000.0 | | | 6666.7 | | | | 1000000.0 | 9.7 | | |
| 897.25 | IPPYCTIAPFGIFGIN-NH2 | 147.1 | | | 200000.0 | | | | 1000000.0 | 4833.3 | | |
| F168.09 | IRNLALQTLPAMCNVY | | | | | | | | | 4.9 | | |
| 897.04 | LGQQQPFPPQQPYPQPQPFP- NH2 | 250000.0 | | | 200000.0 | | | | >33333.33 | 252.0 | | |
| 897.18 | LHQQQKQQQQPSSQVSFQQP- NH2 | 250000.0 | | | 200000.0 | | | | 1000000.0 | 223.1 | | |
| 897.15 | LLQELCCQHLWQIPEQSQCQ- NH2 | 25000.0 | | | 200000.0 | | | | 50000.0 | 31.4 | | |
| 897.13 | LQQHNLAHGRSQVLQQSTYQ- NH2 | | | | | | | | | 607.0 | | |
| F168.04 | PQPFRPQQPYPQ | | | | | | | | | 2.4 | | |
| F168.03 | PQPFRPQQPYPQPQPQ | | | | | | | | | 3.6 | | |
| 897.08 | PQPFRPQQPYPQPQPQYSQP- NH2 | 250000.0 | | | 2468.3 | | | | 1000000.0 | 7.6 | | 25000.0 |
| 897.09 | PQPQPQYSQPQQPISQQQQ- NH2 | 250000.0 | | | 200000.0 | | | | 1000000.0 | 573.3 | | 14433.8 |
| 897.19 | PSSQVSFQQPLQQYPLGQGS- NH2 | 250000.0 | | | 11547.0 | | | | 1000000.0 | 6.9 | | |
| F168.12 | QFEEIRNLALQT | | | | | | 84.0 | | | | | |
| F168.11 | QFEEIRNLALQTLPAM | | | | | | | | | 149.9 | | |
| 897.23 | QFEEIRNLALQTLPAMCNVY- NH2 | 656.5 | | | 1230.9 | | | | 1000000.0 | 57.7 | | |
| F168.06 | QFLGQQQPFPPQ | | | | | | | | | 7.8 | | |
| 897.22 | QGSVQPQQLPQFEEIRNLAL- NH2 | 158.5 | | | 1490.7 | | | | 1000000.0 | 873.6 | | |
| 897.02 | QNPSQQQPQEQVPLVQQQQF- NH2 | 250000.0 | | | 200000.0 | | | | 1000000.0 | 213.8 | | |
| 897.05 | QPYPQPQPFPSQQPYLQLQP- NH2 | 250000.0 | | | 200000.0 | | | | 1000000.0 | 23.5 | | |
| 897.12 | QQLIFCMDVVLQQHNIAHGR- NH2 | 8333.3 | | | 20000.0 | | | | 7715.5 | 40.1 | | 14433.8 |
| F168.08 | QVPLVQQQQFLG | | | | | | | | | 70.3 | | |
| F168.07 | QVPLVQQQQFLGQQQP | | | | | | | | | 0.8 | | |
| 897.03 | QVPLVQQQFLGQQQPFPPQ- NH2 | | | | | | | | | 21.9 | | |
| 897.06 | SQQPYLQLQPFPQPQLPYSQ- NH2 | 3149.7 | | | 228.7 | | | | 1000000.0 | 49.0 | | |
| F168.05 | VQQQQFLGQQQPFPPQ | | | | | | | | | 2.2 | | |
| 897.01 | VRVPVPQLQPQNPSQQQPQE- NH2 | 250000.0 | | | 200000.0 | | | | 1000000.0 | 7.9 | | |
| 897.50 | YIPPYCTIAPFGIFGIN-NH2 | 0.0 | | | | | | | >14285.71 | | | |
| 897.44 | YPSSQVSFQQPLQQYPLGQGS -NH2 | 250000.0 | | | | | | | >11111.11 | | | |
| 897.47 | YQGSVQPQQLPQFEEIRNLAL- NH2 | 0.0 | | | | | | | >11111.11 | | | |
| F160.26 | T PGPVTAQVVLQAAIPLTSC[?] S | | | | | | | | | | | |
| F160.02 | IYRRRLMKQDFSVPQLPHS | | | | | | | | | | | |
| F160.07 | K KRCLLHLAVIGALLAVGATK V | | | | | | | | | | | |
| F160.03 | LCQPVLPSPACQLVLHQILKG G | | | | | | | | | | | |
| F160.14 | MTPAEVSIVVLSGTTAAQVTT T | | | | | | | | | | | |
| F160.16 | PGPVTAQVVLQAAIPLTSCGS S | | | | | | | | | | | |
| F160.01 | VSGLSIGTGRAMLGTHTMEV TVY | | | | | | | | | | | |
| F167.01 | VYVWKTTWGQYWQVLGGP VS | 402.7 | | | | | 13011.2 | | | >14500 | | |
| F167.03 | WNRQLYPEWTEAQR | 162.3 | | | | | 4229.0 | | | 21.5 | | |
| F.115.14 | AKRKTVTAMDVVYAL | | | | | | | | | | | |
| F115.02 | KRHRKVLRDNIQGITKPAIRR LAR | | | | | | | | | | | |
| F115.15 | KTVTAMDVVYALKRQ | | | | | | | | | | | |
| F115.10 | LRDNIQGITKPAIRR | | | | | | | | | | | |
| F115.11 | NIQGITKPAIRRLAR | | | | | | | | | | | |
| F115.03 | TYTEHAKRKTVTAMDVVYA LKRQG | | | | | | | | | | | |
| 590.23 | (2)KYVKQNTLKAT(SIC!) | | | | 238.1 | | | 12.0 | | | | |
| 752.01 | (67)PKYVKQNTLKLAT | 156.9 | | | 210.5 | | | 20.7 | >50000 | | | >25000 |
| 594.09 | AHAAHAAHAAHAAHAA | >8838.83 | | | 200000.0 | | | 91000.0 | | | | 58.3 |
| F,049.01 | BIOTIN-A2- PRYVRQNTLRATC | | | | 60.2 | | | | | | | |
| 574.00 | CPKYVRSAKLRM | 36.4 | | | 227.3 | | | 1300.0 | >16666.67 | | | >25000 |
| 573.05 | GACPKYVKQN | >6250 | | | 5000.0 | | | 91000.0 | | | | |
| 573.04 | GACPKYVKQNTL | 735.3 | | | 5000.0 | | | 91000.0 | | | | |
| 597.04 | GACPKYVKQNTLK | 20.8 | | | 5000.0 | | | 479.0 | | | | |
| 573.03 | GACPKYVKQNTLKL | 25000.0 | | | 5000.0 | | | 3033.3 | | | | |
| 30.1200 | HNTNGVTAASSH | >2500 | | | 10000.0 | | | 30333 | >16666.67 | | | 299.8 |
| 573.08 | KQNTLKLATGMR | 806.5 | | | 5000.0 | | | 4550.0 | | | | |
| 711.03 | LAKQNTLAKQNTLAKQNT | >25000 | | | 2857.1 | | | 6.1 | 25000.0 | | | >25000 |
| 573.09 | NTLKLATGMR | 1315.8 | | | 5000.0 | | | 1516.7 | | | | |
| 590.22 | P(2)YVKQNTLKAT(SIC!) | | | | 15385 | | | 94.8 | | | | |
| 864.10 | PFFVKQNILKLAT | | | | | | | | | | | |
| 864.07 | PFFVKQNTLKLAT | | | | | | | | | | | |
| 864.06 | PFIVKQNTLKLAT | | | | | | | | | | | |
| 864.02 | PFYVKQNTLKLAT | | | | | | | | | | | |
| 864.09 | PIFVKQNILKLAT | | | | | | | | | | | |
| 864.05 | PIFVKQNTLKLAT | | | | | | | | | | | |
| 864.04 | PIIVKQNTLKLAT | | | | | | | | | | | |
| 864.01 | PIYVKQNTLKLAT | | | | | | | | | | | |
| 590.10 | PK(1)VKQNTLKAT(SIC!) | | | | 1538.5 | | | 606.7 | | | | |
| 590.21 | PK(2)VKQNTLKAT(SIC!) | | | | 1538.5 | | | 650.0 | | | | |
| 772.08 | PK(31)VKQNTLKLAT-NH2 | | | | | | | | | | | |
| 611.11 | PKAVKQNTLKLAT | 250000.0 | | | 6666.7 | | | 505.6 | | | | |
| 601.09 | PKEVKQNTLKLAT | 250000.0 | | | 10000.0 | | | 91000.0 | | | | |
| 841.05 | PKFQVETTKKLAT | 250000.0 | | | 20000.0 | | | 650.0 | | | 1533.9 | |
| 864.03 | PKIVKQNTLKLAT | | | | | | | | | | | |
| 713.09 | PKKVKQNTLKLAT | 250000.0 | | | 200000.0 | | | 2275.0 | | | | |
| 792.03 | PKQVKQNTLKLAT | 250000.0 | | | 20000.0 | | | 4550.0 | >50000 | | | |
| 601.10 | PKSVKQNTLKLAT | 250000.0 | | | 10000.0 | | | 9100.0 | | | | |
| 713.14 | PKTVKQNTLKLAT | 250000.0 | | | 200000.0 | | | 2275.0 | | | | |
| 590.09 | PKY(1)KQNTLKAT(SIC!) | | | | 256.4 | | | 395.7 | | | | |
| 833.08 | PKY(41)KQNTLKLAT | | | | | | | | | | | |
| 590.08 | PKYV(1)QNTLKAT(SIC!) | | | | 181.8 | | | 700.0 | | | | |
| 590.19 | PKYV(2)QNTLKAT(SIC!) | | | | 1000.0 | | | 758.3 | | | | |
| 833.07 | PKYV(41)QNTLKLAT | | | | | | | | | | | |
| 590.07 | PKYVK(1)NTLKAT(SIC!) | | | | 322.6 | | | 45.5 | | | | |
| 590.18 | PKYVK(2)NTLKAT(SIC!) | | | | 1000.0 | | | 154.2 | | | | |
| 713.10 | PKYVKKNTLKLAT | 3571.4 | | | 107.0 | | | 52.9 | | | | |
| 590.06 | PKYVKQ(1)TLKAT(SIC!) | | | | 87.0 | | | 137.9 | | | | |
| 590.17 | PKYVKQ(2)TLKAT(SIC!) | | | | 219.8 | | | 758.3 | | | | |
| 833.05 | PKYVKQ(41)TLKLAT | | | | | | | | | | | |
| 590.05 | PKYVKQN(1)LKAT(SIC!) | | | | 800.0 | | | 182.0 | | | | |
| 590.16 | PKYVKQN(2)LKAT(SIC!) | | | | 1538.5 | | | 535.3 | | | | |
| 772.06 | PKYVKQN(24)LKLAT | | | | | | | | | | | |
| 772.01 | PKYVKQN(25)LKLAT | | | | | | | | | | | |
| 772.02 | PKYVKQN(26)LKLAT | | | | | | | | | | | |
| 772.03 | PKYVKQN(27)LKLAT | | | | | | | | | | | |
| 772.04 | PKYVKQN(28)LKLAT | | | | | | | | | | | |
| 772.07 | PKYVKQN(30)LKLAT | | | | | | | | | | | |
| 601.24 | PKYVKQNELKLAT | 446.4 | | | 200000.0 | | | 3033.3 | | | | |
| 515.09 | PKYVKQNHLKLAT | 714.3 | | | 4000.0 | | | 1011.1 | | | | |
| 864.08 | PKYVKQMLKLAT | | | | | | | | | | | |
| 713.12 | PKYVKQNKLKLAT | 301.2 | | | 31.0 | | | 1516.7 | | | | |
| 863.07 | PKYVKQNNLKLAT | 431.0 | | | 1481.5 | | | 1011.1 | | | | |
| 772.11 | PKYVKQNPLKLAT | | | | | | | | | | | |
| 713.13 | PKYVKQNQLKLAT | 312.5 | | | 2857.1 | | | 1137.5 | | | | |
| 590.04 | PKYVKQNT(1)KAT(SIC!) | | | | 1000.0 | | | 395.7 | | | | |
| 590.15 | PKYVKQNT(2)KAT(SIC!) | | | | 1052.6 | | | 185.7 | | | | |
| 833.03 | PKYVKQNT(41)KLAT | | | | | | | | | | | |
| 601.27 | PKYVKQNTKKLAT | 1388.9 | | | 16.7 | | | 24.4 | | | | |
| 590.03 | PKYVKQNLT(1)AT(SIC!) | | | | 238.1 | | | 395.7 | | | | |
| 590.14 | PKYVKQNTL(2)AT(SIC!) | | | | 294.1 | | | 116.7 | | | | |
| 833.02 | PKYVKQNTL(41)LAT | | | | | | | | | | | |
| 590.02 | PKYVKQNTLK(1)T(SIC!) | | | | 1538.5 | | | 260.0 | | | | |
| 590.01 | PKYVKQNTLKAT(SIC!) | | | | 219.8 | | | 455.0 | | | | |
| 713.06 | PKYVKQNTLKEAT | 862.1 | | | 200000.0 | | | 1516.7 | | | | |
| 863.08 | PKYVXQNTNKLAT | 25000.0 | | | 2000.0 | | | 60.7 | | | | |
| 713.01 | PKYVKQNYLKLAT | 80.1 | | | 2222.2 | | | 700.0 | >50000 | | | >25000 |
| 175.00 | RTLYQNVGTYVSVGTSTLNK | >12500 | | | 1538.5 | | | 3033.3 | >11111.11 | | | 6455.0 |
| 597.06 | VKQNTLKLATGMR | >11180.34 | | | 769.2 | | | 91000.0 | | | | |
| 841.06 | YPKFQVEITKXLAT | | | | | | | | | | | |
| 841.04 | YPKFVKLN7XKLAT | | | | | | | | | | | |
| 771.04 | YPKFVKQRTLKLAT | 254.9 | | | | | | | | | | |
| 771.02 | YPKFVKQRTLKLAT-NH2 | 94.6 | | | | | | | | | | |
| 771.06 | YPKFVKRNTLKLAT | 5000.0 | | | | | | | | | | |
| 771.03 | YPKYVKQRTLKLAT | 134.7 | | | | | | | | | | |
| 771.01 | YPKYVKQRTLKLAT-NH2 | 247.5 | | | | | | | | | | |
| 771.07 | YPKYVKRNLKLAT | 8333.3 | | | | | | | | | | |
| 841.02 | YPSFQVQTTLLLAT | | | | | | | | | | | |
| 27.0278 | AAPFTQCGYPALMPL | 3186.5 | | | 3524.0 | | | 267.7 | | | 666.1 | |
| F039.09 | AILCWGELMTLA | | | | >100000 | | | 9.5 | | | 10000.0 | |
| F076.05 | ALRQAILCWGBLM | | | | >28571.43 | | | | | | >377.36 | |
| F039.05 | ALRQAILCWGELMTLA | | | | >100000 | | | 11.6 | | | 5263.2 | |
| 799.04 | GYRWMCLRRFIIFLFILLLC | 2272.7 | | | 4000.0 | | | 1820.0 | >25000 | | | |
| F039.02 | HHTALRQAILCWGELMTLA | | | | >66666.67 | | | 29.9 | | | 3921.6 | |
| F039.03 | HTALRQAILCWGELMTLA | | | | >66666.67 | | | 27.0 | | | 7407.4 | |
| F039.10 | ILCWGELMTLA | | | | >100000 | | | 127.7 | | | 5882.4 | |
| F039.11 | LCWGBLMTTLA | | | | >100000 | | | 829.3 | | | >9523.81 | |
| F164.04 | LPETTVVRCRGRSPR | >10640.71 | | | | | >8750 | | | | | |
| F039.06 | LRQAILCWGELMTLA | | | | >100000 | | | 9.9 | | | 555.6 | |
| CF-09 | LSTLPETTVVRRRGRS | >1923.08 | | | 235.3 | | | 9100.0 | 12500.0 | | | >25000 |
| F164.14 | MDIDPYKEFGASVELLSFL | 566.0 | | | | | 2455.6 | | | | | |
| F164.13 | MDIDPYKEFGASVELLSFLPS DFFP | 184.3 | | | | | >8750 | | | | | |
| F164.16 | MDIDPYKEFGATVELLSFL | 8394.9 | | | | | 4005.4 | | | | | |
| F116.01 | MDIDPYKEFGATVELLSFLPS DFFP | 6608.8 | | | | | 2429.9 | | | | | |
| F164.15 | MDIDPYKEFGATVELLSFLPS DFFP | 1914.3 | | | | | 722.7 | | | | | |
| F164.17 | MDIDPYKEFGATVQLLSFLPS DFFP | 777.4 | | | | | 2819.9 | | | | | |
| F098.11 | MGLKFRQLLWF | 1174.0 | 255.0 | 11582.8 | 13.2 | 784.3 | 1795.8 | 76.1 | | | 1483.2 | |
| 1297.06 | PFLLAQFTSAICSWRRA | | | | | | | | 1603.9 | 391.2 | | |
| F039.18 | PHHTALRQALLCW | | | | >28571.43 | | | 207.5 | | | >7407.41 | |
| F039.17 | PHHTALRQALLCWG | | | | >28571.43 | | | 26.5 | | | 5128.2 | |
| F039.16 | PHHTALRQALLCWGE | | | | >25000 | | | 1685.2 | | | 6451.6 | |
| F039.15 | PHHTALRQALLCWGEL | | | | >25000 | | | 239.1 | | | >6060.61 | |
| F039.14 | PHHTALRQALLCWGELM | | | | 18181.8 | | | 136.8 | | | >5714.29 | |
| F039.13 | PHHTALRQAILCWGELMT | | | | 16666.7 | | | 126.1 | | | 3389.8 | |
| F039.12 | PHHTALRQAILCWGELMTL | | | | >100000 | | | 18.3 | | | 5000.0 | |
| 795.01 | PLGFFPDHQLDPAFGANSNNP DWDFNP | 250000.0 | | | 200000.0 | | | 91000.0 | 310.6 | | | 25000.0 |
| F039.08 | QAILCWGELMTLA | | | | >100000 | | | 12.1 | | | >10526.32 | |
| CF-03 | RDLLDTASALYRREALESPEH | 271.7 | | | 200000.0 | | | 364.0 | >33333.33 | | | 4225.8 |
| CF-06 | RDLVVSYVNTNMGLKFRQLL | >1470.59 | | | 200000.0 | | | 3033.3 | 6250.0 | | | >25000 |
| F039.07 | RQAILCWGELMTLA | | | | >100000 | | | 8.9 | | | 3125.0 | |
| 801.01 | RVRGLYFPAGGSSSGTVN | 8333.3 | | | 20000.0 | | | 535.3 | >25000 | | | |
| 799.02 | SLDSWWTSLNFLGGTTVCLG | 4166.7 | | | 20000.0 | | | 91000.0 | >25000 | | | |
| F039.04 | TALRQAILCWGELMTLA | | | | >100000 | | | 14.0 | | | >8000 | |
| F164.12 | TNMGLKFRQLLWFHI | 11180.7 | | | | | 269.7 | | | | | |
| F164.11 | TNVGLKFRQLLWFHI | 1012.3 | | | | | 5520.5 | | | | | |
| F098.08 | ZTWRRRGRSPRRR | >102062.07 | 4516.4 | >60436.72 | 2756.5 | >6708.41 | 35000.0 | 15166.7 | | | 530.7 | |
| 800.04 | VGAGAFGLGFTPPHGGL | 250000.0 | | | 200000.0 | | | 9100.0 | >25000 | | | |
| 764.01 | VSFGVWIRTPPA | 4724.6 | | | 4000.0 | | | 91000.0 | 13609.2 | | | >25000 |
| 857.01 | YPHHTALRQAILCWGELMTL A | 35355.3 | | | 10767.6 | | | 112.2 | | 429.9 | 5455.5 | |
| F134.07 | GPGEGAVQWMNRLIAFASRG | 6305.2 | | | 88.3 | | | | | | | |
| Pape22 | GRHLIFCHSKRKCDELATKL | >102062.07 | >48048.45 | >60436.72 | 17407.8 | >6708.41 | 5000.0 | 827.3 | | | 2073.9 | |
| F134.06 | LLFNILGGWVAAQLAAPGAA | 56433.4 | | | 7057.2 | | | | | | | |
| 1283.40 | PAILSPGALVVGVVCA | 3429.9 | | | | | | | | | | |
| 221.06 | AFVAWRNRCK | | | | | | | 5.2 | | | | |
| 221.08 | AWAAWRNRCK | | | | | | | 50.6 | | | | |
| 221.10 | AWEAWRNRCK | | | | | | | 60.7 | | | | |
| 221.09 | AWLAWRNRCK | | | | | | | 19.8 | | | | |
| 221.15 | AWVAARNRCK | | | | | | | 2.1 | | | | |
| 221.16 | AWVAFRNRCR | | | | | | | 5.1 | | | | |
| 221.14 | AWVAQRNRCK | | | | | | | 10.1 | | | | |
| 221.19 | AWVAWANRCK | | | | | | | 26.0 | | | | |
| 221.17 | AWVAWENRCK | | | | | | | 56.9 | | | | |
| 221.18 | AWVAWKNRCK | | | | | | | 11.1 | | | | |
| 221.22 | AWVAWRARCK | | | | | | | 7.6 | | | | |
| 221.25 | AWVAWRNACK | | | | | | | 13.6 | | | | |
| 221.23 | AWVAWRNECK | | | | | | | 53.5 | | | | |
| 221.24 | AWVAWRNKCK | | | | | | | 36.4 | | | | |
| 221.32 | AWVAWRNRCA | | | | | | | 433.3 | | | | |
| 539.00 | AWVAWRNRCK | >12500 | | | 5000.0 | | | 20.2 | >11111.11 | | | |
| 221.01 | AWVAWRNRCK | >12500 | | | 5000.0 | | | 20.2 | >11111.11 | | | >25000 |
| 221.31 | AWVAWRNRCR | | | | | | | 86.7 | | | | |
| 221.28 | AWVAWRNREK | | | | | | | 10.7 | | | | |
| 221.29 | AWVAWRNRKK | | | | | | | 3.3 | | | | |
| 221.26 | AWVAWRNRQK | | | | | | | 2.0 | | | | |
| 221.27 | AWVAWRNRVK | | | | | | | 3.4 | | | | |
| 221.20 | AWVAWRQRCK | | | | | | | 606.7 | | | | |
| 221.21 | AWVAWRVRCK | | | | | | | 162.5 | | | | |
| 221.12 | AWVEWRNRCK | | | | | | | 124.7 | | | | |
| 221.11 | AWVSWRNRCK | | | | | | | 36.4 | | | | |
| 221.13 | AWWWRNRCK | | | | | | | 45.5 | | | | |
| 565.01 | EFVAAKAAQK | >25000 | | | 1250.0 | | | 1.4 | >50000 | | | 3571.4 |
| 221.02 | BWVAWRNRCK | | | | | | | 19.0 | | | | |
| AP23 | LAAAMKRHGLDNYRGYSLG NWV | 5000.0 | | | 83.3 | | | 178.4 | >5263.16 | | | >25000 |
| AP12 | RNRCKGTDVQAWIRGCRL | 3571.4 | | | 2500.0 | | | 178.4 | >6250 | | | >25000 |
| AP38 | SVNCAKKIVSDGNGMN | >8333.33 | | | 666.7 | | | 4550.0 | 2306.3 | | | >25000 |
| 221.04 | SWVAWRNRCK | | | | | | | 21.7 | | | | |
| 221.03 | VWVAWRNRCK | | | | | | | 47.9 | | | | |
| 560.02 | WRNAKWRNAKWRNAK | >12500 | | | 5000.0 | | | 505.6 | >14285.71 | | | >25000 |
| AP37 | YRGYSLGNWVCAAKFESNFN TQ | >6250 | | | 5000.0 | | | 1011.1 | >5263.16 | | | 25000.0 |
| 58.0052 | CWMIDSDCRPRFREL | | | | | | | | | | | |
| 58.0045 | CYGLGMDHLTEVRAV | | | | | | | | | | | |
| 58.0053 | FRELVSDFSRMARDP | | | | | | | | | | | |
| 58.0051 | IKWMALDSILRRRFT | | | | | | | | | | | |
| 58.0046 | LALIHHDTHLCFVHT | | | | | | | | | | | |
| 58.0043 | LTYLPTDASLSFLQD | | | | | | | | | | | |
| 58.0048 | QMRILKDTELRKVKV | | | | | | | | | | | |
| 1385.03 | SPYVSRLLGICLT | 886.5 | | | | | 4207.3 | | 3414.1 | 831.4 | | |
| 1385.04 | VPIKWMALESILRRRF | 1300.9 | | | | | 2815.1 | | 1430.6 | 46.9 | | |
| 58.0047 | WDQLFRDPHQALLHT | | | | | | | | | | | |
| F159.13 | DRVHPVHAGPIA | 98.5 | >9500.69 | | >53452.25 | | 528.8 | 19198.3 | | | >12909.94 | |
| F159.12 | DRVHPVHAGPIAPG | 93.2 | >7836.24 | | >50000 | | 342.3 | 724.3 | | | 11809.5 | |
| F159.11 | DRVHPVHAGPIAPGQM | 265.3 | >6971.6 | | >33333.33 | | 655.6 | 1102.5 | | | 1845.0 | |
| F159.10 | DRVHPVHAGPIAPGQMRE | 399.8 | 14869.0 | | >31622.78 | | 591.1 | 706.9 | | | 6288.4 | |
| F159.09 | DRVHPVHAGPIAPGQMREPR | 223.2 | >5591.33 | | >29488.39 | | 1246.7 | 3572.6 | | | >7905.69 | |
| F170.01 | DTEVHNVWATQACVPTDPNP | >3240.47 | >5658.03 | >2528.25 | >3232.54 | | >4183.3 | 5055.6 | >6537.23 | 5493.0 | >533.76 | |
| F169.02 | GLlHSQRRQDILDL | >39528.47 | >70725.41 | >30316.95 | 1843.8 | | 837.9 | 505.6 | >77821.01 | 11901.1 | 1364.0 | |
| F170.06 | EPFRDYVDRFYKTLRAEQAS | 1761.7 | 201.7 | 3463.5 | 100.7 | | >7826.24 | 162.5 | >12903.23 | 2902.6 | 690.6 | |
| F170.05 | FYKRWIILGLNKIVRMYSPV | 659.0 | 151.5 | >5056.5 | >6428.24 | | 9589.0 | 10111.1 | >12903.23 | 659.7 | >1054.09 | |
| F170.02 | PKISFEPIPIHYCAPAGFAI | 389.3 | 15312.5 | 214.0 | 14285.7 | | 758.2 | 350.0 | >6537.23 | 78.3 | >533.76 | |
| F169.01 | TAATNAACAWLEA | 18351.3 | >70725.41 | 1827.4 | >37796.45 | | 974.7 | 3137.9 | >77821.01 | 59094.5 | >6565.32 | |
| F170.04 | TNNPPPVGEIYKRWIILGL | 912.9 | 200.8 | 1616.9 | 98.6 | | 11.9 | 20.2 | 7624.9 | 442.7 | 44.7 | |
| F170.03 | VWGIKQLQARVLAVERYLKD | 200.7 | 796.7 | 2451.5 | 541.3 | | 134.6 | 16.9 | 5951.0 | 372.8 | 39.7 | |
| 27.0374 | AVQMAVFIHNFKRKG | 2701.6 | | | 73.6 | | | 3.5 | | | 7.9 | |
| 21.0299 | DQQLLGIWGCSGKLI | 1437.7 | | | 3090.0 | | | 606.7 | | | 2026.7 | |
| 27.0287 | DQSLKPCVKLTPLCV | 2339.9 | | | 4025.1 | | | 5055.6 | | | 11063.2 | |
| 27.0284 | EDIISLWDQSLKPCV | >27277.24 | | | >18257.42 | | | 5352.9 | | | 11985.1 | |
| 190.20 | ERFAVNPGLLETSEGC | >12500 | | | 2500.0 | | | 1300.0 | >33333.33 | | | 1976.4 |
| 190.15 | GARASVLSGGELDKWE | >12500 | | | 10000.0 | | | 1300.0 | >14285.71 | | | 315.5 |
| 190.16 | GGELDKWEKIRLRPGG | >12500 | | | 10000.0 | | | 252.8 | >33333.33 | | | >25000 |
| RS-21 | GP41584-609 | >12500 | | | 645.2 | | | 9100.0 | 3965.3 | | | >25000 |
| 27.0320 | IGGIGGFIKVRQYDQ | 8059.6 | | | 1409.9 | | | 45.5 | | | 112.2 | |
| 1.81.11 | ILKALGPAATLEEMMT(200.11 ) | 12500.0 | | | 200000.0 | | | 91000.0 | >50000 | | | 25000.0 |
| 200.10 | INEEAAEWERVHPVHA | >12500 | | | 10000.0 | | | 1300.0 | 16666.7 | | | >25000 |
| 27.0348 | IQKLVGKLNWASQIY | 5936.9 | | | 4573.0 | | | 6500.0 | | | >7273.93 | |
| 27.0286 | ISLWDQSLKPCVKLT | >14153.46 | | | 8486.2 | | | 313.8 | | | 1179.1 | |
| 190.19 | IVWASRELERFAVNPG | >12500 | | | 339.0 | | | 1300.0 | >33333.33 | | | 3125.0 |
| 200.17 | IYKRWIILGLNKIVRN | >12500 | | | 10000.0 | | | 364.0 | >33333.33 | | | >25000 |
| F091.13 | KQIINMWQEVGKAMYA | 11763.0 | | | 22360.7 | | | 2527.8 | | 61.1 | 5661.4 | |
| 200.04 | KVVEEKAFSPEVIPMF | 12500.0 | | | 10000.0 | | | 606.7 | >33333.33 | | | >25000 |
| 190.08 | LGKIWPSYKGRPGNFL | >12500 | | | 277.8 | | | 1300.0 | 20000.0 | | | 715.5 |
| F091.08 | LKQIVKKLREQFGNNK | >45643.55 | | | 6900.7 | | | 206.8 | | 601.4 | 492.1 | |
| 27.0300 | LLGIWGCSGKLICTT | 3934.7 | | | >11785.11 | | | 1820.0 | | | 4279.4 | |
| 27.0302 | LSIVNRVRQGYSPLS | 16606.8 | | | 27.8 | | | 284.4 | | | 668.2 | |
| 200.01 | PNQNLQGQMVHQAIS | >12500 | | | 10000.0 | | | 1300.0 | >33333.33 | | | >25000 |
| F091.24 | PLGVAPTKAKRRWQR | 38575.8 | | | 22360.7 | | | 19.0 | | 2371.8 | 57.4 | |
| 190.23 | PSLQTGSEELRSLYNT | >12500 | | | 10000.0 | | | 758.3 | >33333.33 | | | 25000.0 |
| F091.16 | QARILAVERYLKDQQL | 26352.3 | | | 676.5 | | | 479.0 | | 246.2 | 1081.5 | |
| 27.0352 | QGQWTYQIYQEPFKN | 9940.4 | | | >30860.67 | | | 606.7 | | | 1363.5 | |
| 190.13 | QKQEPWKELYPLTSL | >12500 | | | 10000.0 | | | 1300.0 | >33333.33 | | | >25000 |
| 27.0375 | QMAVFIHNFKRKGGI. | 7008.2 | | | 19.1 | | | 7.6 | | | 17.4 | |
| F091.07 | RIQRGPGRAFVTIGKL | >45643.55 | | | 429.0 | | | 1096.4 | | 4207.8 | 2424.6 | |
| 190.22 | RQILGQLQPSLQTGSE | >12500 | | | 10000.0 | | | 1300.0 | >33333.33 | | | 17677.7 |
| F091.04 | SLKPCVKLTPLCVTLN | 1159.4 | | | 449.5 | | | 758.3 | | 508.7 | 1707.5 | |
| F091.26 | SLWDQSLKPCVKLTPL | >45643.55 | | | 4255.3 | | | 413.6 | | 2774.5 | 910.0 | |
| 27.0359 | SQIIEQLIKKEKVYL | >25649.46 | | | 428.5 | | | 293.6 | | | 650.2 | |
| F091.22 | SQNQQEKNEQELLELD | >45643.55 | | | >51639.78 | | | 758.3 | | >64845.97 | 1666.7 | |
| F091.11 | SSGGDPEIVMHSFNCG | >45643.35 | | | >51639.78 | | | 22750.0 | | 503.3 | >21821.79 | |
| 27.0334 | STKWRKLVDFRELNK | 2183.7 | 1753.2 | 21061.5 | 124.8 | >7407.41 | >3818.81 | 429.6 | | | 509.1 | |
| F091.12 | TTTLPCRIKQFINMWQE | 24397.5 | | | 47140.5 | | | 8272.7 | | 730.7 | 18181.8 | |
| 27.0292 | TVYYGVPVWKEATTT | 1997.6 | | | 2275.4 | | | 568.8 | | | 2098.8 | |
| F091.23 | VKIEPLGVAPTKARRR | >45643.55 | | | >51639.78 | | | 6.1 | | 5913.4 | 13.3 | |
| 27.0301 | VLSIVNRVRQGYSPL | 2756.6 | | | 3.7 | | | 5.4 | | | 11.9 | |
| 27.0356 | VNIVTDSQYALGIIQ | 2299.5 | | | >25197.63 | | | 6500.0 | | | >7273.93 | |
| F091.03 | WDQSLKPCVKLIPLCV | 3140.7 | | | 20203.1 | | | 3137.9 | | 4515.3 | 6896.6 | |
| 27.0335 | WRKLVDFRBLNKRTQ | >25649.46 | | | 69.0 | | | 5352.9 | | | >5939.14 | |
| F160.45 | STORKUSP45 | | | | | | | | | | | |
| 106.00 | APYTSTLLPPELSETP | >1923.08 | | | 1818.2 | | | 4550.0 | >12500 | | | >25000 |
| F095.05 | FRQLVHFVRDFAQLL | | | | | | | 399.1 | | | 869.6 | |
| AP30 | SLKMADPNRFRGKDLP | >25000 | | | 1818.2 | | | 700.0 | >12500 | | | >25000 |
| 605.14 | ACRVNHVTLSQPKIVK | 312.5 | | | 200000.0 | | | 1011.1 | | | | 7216.9 |
| 530.08 | GKKIPKVEMSDMSFSK | >12500 | | | 200000.0 | | | 910.0 | >7142.86 | | | >25000 |
| 605.03 | HPAENGKSNFLNCYVS | 2500.0 | | | 200000.0 | | | 91000.0 | | | | >25000 |
| 530.06 | HPPHIEIQMLKNGKKI | 8333.3 | | | 350.9 | | | 11.5 | >7142.86 | | | >25000 |
| 530.01 | IQKTPQIQVYSRHPPE | >12500 | | | 833.3 | | | 91000.0 | >7142.86 | | | >25000 |
| 530.07 | IQMLKNGKKIPKVEMS | 8333.3 | | | 740.7 | | | 15.4 | >7142.86 | | | >25000 |
| 605.01 | IQRTPKIQVYSTHPAE | 12500.0 | | | 2000.0 | | | 1516.7 | | | | >25000 |
| 530.02 | IQVYSRHPPENGKPNI | >12500 | | | 20000.0 | | | 91000.0 | >7142.86 | | | >25000 |
| 530.04 | KPNILNCYVTQFHPPH | >12500 | | | 200000.0 | | | 4550.0 | >7142.86 | | | >25000 |
| 530.10 | SFSKDWSFYILAHTEF | 1206.3 | | | 2857.1 | | | 91000.0 | 4543 | | | >25000 |
| 605.10 | SFSYDWSFYLLYYTEF | 250000.0 | | | 200000.0 | | | 395.7 | | | | >25000 |
| 605.11 | SFYLLYYTEFTPTEKD | 2777.8 | | | 200000.0 | | | 91000.0 | | | | 17677.7 |
| 530.14 | TETDTYACRVKHDSMA | 925.9 | | | 200000.0 | | | 91000.0 | >7142.86 | | | >25000 |
| 530.09 | VEMSDMSFSKDWSFYI | 438.6 | | | 20000.0 | | | 91000.0 | 322.6 | | | >25000 |
| 544.02 | YGSDTITLPCRIKQFINMWQE | 1000.0 | | | 909.1 | | | 91000.0 | >9129.09 | | | >25000 |
| FR-01 | PEFLEQRRAAVDTYC | 250000.0 | | | 487.8 | | | 91000.0 | | | | >12500 |
| F073.01 | MRGSHHHHHHGSVD-II72-216 | 147.3 | | | 983.0 | | | 63.0 | | 18.4 | 13.6 | |
| 734.01 | FVNQHLSGSHLVEALYLVSG- CONH2 | 216.5 | | | 200000.0 | | | 9100.0 | | | 20000.0 | |
| 68.0002 | DLVLSIALSVGCTGA | 4029.1 | >141450.82 | 2200.2 | >115470.05 | 4914.8 | 97.5 | 18200.0 | >11629.26 | 459.5 | >100000 | |
| 68.0005 | GQRVPVSHSFPHPLY | 628.7 | >141450.82 | 102.1 | >57735.03 | 5507.3 | 702.5 | 3960.3 | >11629.26 | 9860.5 | >100000 | |
| 68.0011 | HDLMLLRLSEPAKIT | 109.3 | 544.2 | 43.1 | 1146.7 | 84.4 | 0.8 | 115.1 | 488.0 | 11.6 | 211.2 | |
| 68.0008 | HPLYMMSLLKHQSLR | 1282.0 | 381.7 | 199.2 | 248.2 | 95.2 | 546.3 | 471.5 | >11629.26 | 8.4 | 219.0 | |
| 68.0003 | HPQWVLTAAHCLKKN | 563.4 | 1693.2 | 822.4 | 980.8 | 11450.4 | 482.8 | 1219.5 | 8114.3 | 1105.8 | 11.0 | |
| 68.0018 | KPAVYTKVVHYRKWI | 2401.0 | 53.2 | 3676.7 | 327.0 | 1303.0 | 1947.5 | 401.1 | 7186.5 | 4581.2 | 22.7 | |
| 68.0140 | LHLLSNDMCARAYSE | 27685.5 | 50230.1 | 59904.2 | 26011.5 | 1152.5 | 1876.4 | >2030.13 | 1988.8 | 323.8 | 28817.3 | |
| 68.0001 | MWDLVLSIALSVGCT | 3031.7 | 23045.8 | 1727.3 | 81096.0 | 4574.6 | 107.7 | 11375.0 | 15204.5 | 157.5 | 70710.7 | |
| 68.0017 | NGVLQGITSWGPEPC | 2285.2 | 52234.2 | 50111.5 | 32444.3 | >3132.18 | >14288.69 | 834.9 | >10217.64 | 5760.9 | >100000 | |
| 68.0009 | NMSLLKHQSLRPDED | 20619.7 | 26496.2 | 96824.6 | 25819.9 | 1287.6 | >20207.26 | >23496.1 | >11629.26 | 104.9 | >100000 | |
| 68.0015 | PEEFLRPRSLQCVSL | 5155.9 | 2207.1 | 5839.1 | 10675.2 | 6073.3 | 11666.7 | 3193.5 | >10463.53 | 117.0 | 57537.0 | |
| 68.0007 | PHPLYNMSLLKHQSL | 20690.6 | 3315.4 | 1591.6 | 6455.0 | 859.8 | 3307.2 | 3873.2 | >11629.26 | 49.4 | 1901.1 | |
| 68.0016 | PRSLQCVSLHLLSND | 2216.7 | 6106.8 | 28306.9 | 11128.3 | 3861.0 | 3731.0 | 1596.7 | 11649.6 | 544.1 | 46415.9 | |
| 68.0004 | QWVLTAAHCLKKNSQ | 3402.1 | 99000.0 | 4812.5 | 14213.4 | >10721.94 | >24748.74 | >30333.33 | >11629.26 | 14395.4 | 382.2 | |
| 68.0006 | RVPVSHSFPHPLYNM | 101.1 | 100020.8 | 96.8 | >57735.03 | 10397.4 | 376.9 | 5517.7 | >11629.26 | 9213.5 | 11649.7 | |
| 68.0010 | SHDLMLLRLSEPAKI | 106.4 | 1326.9 | 111.5 | 5267.4 | 591.4 | 1.8 | 365.4 | 5360.8 | 10.2 | 2031.1 | |
| F090.02 | ATGFKQSSKALQRPV | | | | 12.0 | | | | | | | |
| F090.01 | IVHSATGFKQSSKALQRPVAS DFEP | | | | 16.4 | | | | | | | |
| F071.29 | IKYNGEEYLILSARD | | | | 3592.1 | | | 322.1 | | 1589.2 | 1443.4 | |
| F071.26 | IYSKYGGTEIKYNGE | | | | >100000 | | | 3625.5 | | >93715.70 | 457.9 | |
| F118.06 | LVIPINAKEKPQ | | | | | | | 535.3 | | | 1217.8 | |
| F118.02 | LVIPENAKEKPQEGT | | | | | | | 827.3 | | | 1876.3 | |
| F071.12 | LVIPENAKEKPQEGT | | | | 66666.7 | | | 56.9 | | >713513.06 | 174.1 | |
| F071.30 | NGEEYLILSARDVLA | | | | 6052.3 | | | 5732.5 | | 1691.3 | 590.5 | |
| F071.11 | PSGLVIPENAKEKQ | | | | >100000 | | | 53.5 | | >61828.21 | 138.6 | |
| F071.22 | RIPVDVSEGDIVIYS | | | | >100000 | | | 15.3 | | >61828.21 | >51639.78 | |
| 581.02 | RKHRIEDAVRNAKAAVEEGI VAG | 892.9 | | | 200000.0 | | | 91000.0 | 1000000.0 | | | 3726.8 |
| F071.24 | SEGDIVIYSKYGGTE | | | | 3479.5 | | | 144.3 | | >71393.06 | 40824.8 | |
| 572.03 | TLLQAAPALDKY | 1785.7 | | | 10000.0 | | | 18200.0 | >15429.72 | | 40000.0 | >25000 |
| F071.03 | VAKVKIKPLEDKILV | | | | 4789.8 | | | 180.5 | | 5919.6 | 3722.9 | |
| F071.04 | VKIKPLEDKILVQAG | | | | 2309.4 | | | 471.2 | | 483.3 | 28284.3 | |
| F071.17 | VVAVGPGRWDEDGAK | | | | >100000 | | | 650.0 | | >71393.06 | 1490.7 | |
| 581.01 | YEKIGAELVKEVAKKTDDVA G | 312.5 | | | 200000.0 | | | 2275.0 | >20000 | | | >25000 |
| F160.21 | EKIWEELSVLEVFEGRED | | | | | | | | | | | |
| F160.20 | ETSYVKNLHHMVKISGG | | | | | | | | | | | |
| 58.0062 | EEKIWEDLSMLEVFE | | | | | | | | | | | |
| 58.0057 | FPDLESDFQAAISRK | | | | | | | | | | | |
| 58.0058 | LESVLRDCQDFFPVI | | | | | | | | | | | |
| 58.0064 | MQDLVQDNYLEYRQV | | | | | | | | | | | |
| 58.0063 | PRKLLMEDLVQENYL | | | | | | | | | | | |
| 58.0065 | QDLVQEDYLEYRQVP | | | | | | | | | | | |
| 58.0059 | QLVFGIDVVEVVPIS | | | | | | | | | | | |
| 58.0071 | EEKIWEDLSVLEVFE | | | | | | | | | | | |
| 58.0068 | LGSVVGDWQYFFPVI | | | | | | | | | | | |
| 58.0074 | QHFVQEDYLEYRQVP | | | | | | | | | | | |
| 58.0073 | TQHFVQDNYLEYPQV | | | | | | | | | | | |
| F167.06 | LLKYRAREPVTKAEMLGSVV GNWQ | 974.2 | | | | | 117.0 | | | 143 | | |
| E167.08 | MVKISGGPRISYPLLHEWALR EGEE | 161.1 | | | | | 9.9 | | | >11245.72 | | |
| F167.07 | [?][?][?][?] QVPGSDPACYEFLWGPRALIE TSY | >7816.32 | | | | | >6092.72 | | | 27878.8 | | |
| F160.27 | VGNWQYFFPVIFSKASDSL | | | | | | | | | | | |
| 520.09 | YKLNFYFDLLRAKL | 1470.6 | | | 1250.0 | | | 91000.0 | 6666.7 | | | >25000 |
| 520.07 | YLDNIKDNVGKMED | 1470.6 | | | 33333 | | | 91000.0 | >13363.62 | >1526.32 | | >25000 |
| F160.15 | AAGIGILTVILGVL | | | | | | | | | | | |
| 825.10 | AC- ASQKRPSQRHGSKYLATAST | 2362.3 | | | 200000.0 | | | 91000.0 | | | | |
| 765.17 | AC- ASQKRPSQRSKYLATASTMD | 299.8 | | | 200000.0 | | | 3033.3 | | | 8165.0 | |
| F006.16 | ANPVVHFFKNIVTPR | | | | | | | 1.3 | | | 315.5 | |
| 825.01 | ASQKRPSQRHGSKYLATAST | 898.0 | | | 200000.0 | | | 91000.0 | | | | |
| 765.15 | AYDAQGTLSKIFKLGGRDSR | 2192.7 | | | 37.7 | | | 45.3 | | | 110.0 | |
| F006.14 | DENPVVHFFKN | | | | | | | 959.2 | | | >6666.67 | |
| F006.13 | DENPVVHFFKNI | | | | | | | 15.7 | | | 4472.1 | |
| F006.12 | DENPVVHFFKNIV | | | | | | | 6.5 | | | 2020.3 | |
| F006.11 | DENPVVHFFKNIVT | | | | | | | 4.2 | | | 691.3 | |
| F006.10 | DENPVVHFFKNIVTPR | | | | | | | 10.7 | | | 1529.4 | |
| F006.09 | DENPVVHFFKNIVTPRT | | | | | | | 26.8 | | | 2981.4 | |
| F006.01 | DENPVVHFFKNIVTPRTPP | | | | | | | 3.0 | | | 54.7 | |
| F006.0202 | DENPVVHFFKNIVTPRTRPPY | | | | | | | 12.2 | | | 26.3 | |
| F006.03 | DENPVVHFFRNIVTPRTPPY | | | | | | | 1.4 | | | 19.5 | |
| F006.17 | EAPVVHFFKKNIVTPR | | | | | | | 2.8 | | | 691.7 | |
| F006.18 | ENAVVHFFKNIVTPR | | | | | | | 4.6 | | | 423.9 | |
| F006.19 | ENPAVHFFKNIVTPR | | | | | | | 1.6 | | | 678.1 | |
| F038.01 | ENPKVHFFKNIVTPR | | | | | | | 7.3 | | | 15.4 | |
| F006.20 | ENPVAHFFKNIVTPR | | | | | | | 156.0 | | | 482.8 | |
| F038.02 | ENPVKHFFKNIVTPR | | | | | | | 405.4 | | | 869.6 | |
| F006.21 | ENPVVAFFKNIVTPR | | | | | | | 2.8 | | | 301.5 | |
| F038.03 | ENPVVAFFKNVTPR | | | | | | | 5.2 | | | 11.1 | |
| F038.06 | ENPVVDFFKNIVTPR | | | | | | | 75.8 | | | 166.7 | |
| F038.05 | ENPVVFFFKNIVTPR | | | | | | | 2.1 | | | 4.4 | |
| F006.22 | ENPVVHAFKNIVTPR | | | | | | | 2.7 | | | 910.0 | |
| F038.07 | ENPVVHAFKNIVTPR | | | | | | | 2.1 | | | 4.7 | |
| F095.08 | ENPVVHAFRNIVTPR | | | | | | | 219 | | | 42.6 | |
| F038.09 | ENPVVHDFKNIVTPR | | | | | | | 451.6 | | | 909.1 | |
| F006.23 | ENPVVHFAKNIVTPR | | | | | | | 233.2 | | | 6324.6 | |
| F095.06 | ENPVVHFARNIVTPR | | | | | | | 975.1 | | | 2020.2 | |
| F006.24 | ENPVVHFFANIVTPR | | | | | | | 2.9 | | | 6235.3 | |
| F038.14 | ENPVVHFFANIVTPR | | | | | | | 3.6 | | | 7.7 | |
| Cr-8 | ENPVVHFFNIVTPRTP | | | | | | | 126.4 | | | | |
| F038.15 | ENPVVHFFDNIVTPR | | | | | | | 344.9 | | | 740.7 | |
| F038.16 | ENPVVBFFHNIVTPR | | | | | | | 6.4 | | | 133 | |
| F006.25 | ENPVVHFFKAIVTPR | | | | | | | 4.0 | | | 209.7 | |
| Cr-9 | ENVVHFFKAIVTPRTP | | | | | | | 12.5 | | | | |
| F038.19 | ENVVHFFKKIVTPR | | | | | | | 650.0 | | | 1428.6 | |
| F006.26 | ENPVVHFFKNAVTPR | | | | | | | 2.6 | | | 1616.9 | |
| Cr-10 | ENPVVHFFKNAVTPRTP | | | | | | | 82.7 | | | 188.9 | |
| F006.27 | ENPVVHFFKNIATPR | | | | | | | 4.1 | | | 1020.6 | |
| F006.28 | ENPVVHFFKNIVAPR | | | | | | | 0.7 | | | 66.2 | |
| Cr-11 | ENPVVHFFKNIVAPRTP | | | | | | | 16.0 | | | 37.0 | |
| F006.29 | ENPVVHFFKNIVTAR | | | | | | | 1.0 | | | 495.1 | |
| F006.30 | ENPVVHFFKNIVTPA | | | | | | | 2.5 | | | 3333.3 | |
| F006.36 | ENPVVHFFKNIVTPA | | | | | | | 6.9 | | | 20000.0 | |
| F006.15 | ENPVVHFFKNIVTPR | | | | | | | 5.2 | | | 462.5 | |
| Cr-7 | ENPVVHFFKNIVTPRTP | | | | | | | 43.3 | | | | |
| F006.04 | ENPVVHFFKNIVTPRTPPY | | | | | | | 17.4 | | | 573.1 | |
| F038.20 | ENPVVHFFKNKVTPR | | | | | | | 18.5 | | | 37.7 | |
| F038.17 | ENPVVHFFLNIVTPR | | | | | | | 3.4 | | | 6.3 | |
| F038.18 | ENVVHFFRNIVTPR | | | | | | | 2.0 | | | 4.3 | |
| F038.13 | ENPVVHFKKNIVTPR | | | | | | | 339.1 | | | 714.3 | |
| F038.10 | ENPVVHHFKNIVTPR | | | | | | | 7.9 | | | 16.7 | |
| F095.10 | ENPVVHHFRNIVTPR | | | | | | | 16.6 | | | 33.9 | |
| F038.11 | ENPVVHLFKNIVTPR | | | | | | | 8.4 | | | 16.7 | |
| F095.09 | ENPVVHLFRNIVTPR | | | | | | | 5.5 | | | 11.8 | |
| F038.12 | ENPVVHWFKNIVTPR | | | | | | | 19.7 | | | 42.6 | |
| F095.12 | ENPVVHYFANIVTPR | | | | | | | 18.3 | | | 40.0 | |
| F0095.11 | ENPVVHYFHNIVTPR | | | | | | | 14.2 | | | 28.2 | |
| F038.08 | ENPVVHYFKNIVTPR | | | | | | | 2.1 | | | 4.7 | |
| F095.13 | ENPVVHYFLNIVTPR | | | | | | | 18.1 | | | 38.5 | |
| F095.07 | ENPVVHYFRNIVTPR | | | | | | | 49.2 | | | 48.8 | |
| F038.04 | ENPVVKFFKNIVTPR | | | | | | | 7.0 | | | 15.4 | |
| 613.02 | FFKNIVTPFFKNIVTP | 2272.7 | | | | | | | | | | |
| 825.07 | FSWGAEGQRPGFGYGGRASD | >10206.21 | | | 200000.0 | | | 91000.0 | | | | |
| 765.13 | GFGYGGRASDYKSAHKGFKG | >10206.21 | | | 138.9 | | | 61.9 | | | | |
| 825.06 | GKGRGLSLSRFSWGAEGQRP | 4724.6 | | | 1666.7 | | | 3033.3 | | | | |
| 825.04 | GSGKDSHHPARTAHYGSLPQ | >10206.21 | | | 200000.0 | | | 91000.0 | | | | |
| 765.03 | HARHGFLPRHRDTGILDSIG | >10206.21 | | | 4000.0 | | | 91000.0 | | | | |
| F006.321 | HFFKNIVTPRTPPY | | | | | | | 460.2 | | | 377.4 | |
| F.006.322 | HFFKNIVTPRTPPY | | | | | | | 2144.9 | | | 571.7 | |
| 765.16 | EFKLGGRDSRSGSPMARR | >12500 | | | 8000.0 | | | 1011.1 | | | | |
| 765.06 | KRGSGKDSHTRTTHYGSLPQ | >10206.21 | | | 14142.1 | | | 479.0 | | | 800.0 | |
| 765.08 | KSQHGRTQDENPVVFFKNI | >10206.21 | | | 10000.0 | | | 9100.0 | | | | |
| F006.31 | NPVVHFFKNIVT | | | | | | | 23.4 | | | 10000.0 | |
| F006.37 | NPVVHFFKNIVTPA | | | | | | | 4.8 | | | 13.3 | |
| F006.34 | NPVVHFFKNIVTPR | | | | | | | 3.7 | | | 1889.8 | |
| F006.05 | NPVVHFFKNIVTPRTPPY | | | | | | | 7.7 | | | 124.0 | |
| F006.39 | PVVHFFKNIVT | | | | | | | 348.0 | | | 714.3 | |
| F006.38 | PVVHFFKNIVTPA | | | | | | | 15.0 | | | 20000.0 | |
| F006.35 | PVVHFFKNIVTPR | | | | | | | 18.6 | | | 8165.0 | |
| F006.06 | PVVHFFKNIVTPRTPPY | | | | | | | 4.8 | | | 45.3 | |
| 825.11 | QKSHGRTQDENPVVHFFKNI | 10000.0 | | | 13333.3 | | | 1300.0 | | | | |
| F121.03 | RASDYKSAHKGFKGVDAQGT | | | | 449.5 | | | | | | | |
| F121.02 | RASDYKSAHKGLKGHDAQGT | | | | 149.1 | | | | | | | |
| 825.02 | RDTGILDSIGRFFGGDRGAP | >10206.21 | | | 169.5 | | | 91000.0 | | | | |
| 765.04 | RDTGILDSIGRFFSGDRGAP | 250000.0 | | | 265.8 | | | 3033.3 | | | 5773.5 | |
| 825.03 | RFFGGDRGAPKRGSGKDSHH | >10206.21 | | | 200000.0 | | | 4550.0 | | | | |
| 765.05 | RFFSGDRGAPKRGSGKDSHT | 250000.0 | | | 200000.0 | | | 910.0 | | | 2000.0 | |
| 825.05 | RTAHYGSLPQKSHGRTQDEN | >10206.21 | | | 3333.3 | | | 91000.0 | | | | |
| 825.09 | VDAQGTLSKIFKLGGRDSRS | 1170.7 | | | 18.2 | | | 3033.3 | 769.2 | | | |
| F112.01 | VDAQGTLSKIFKLGGRDSRS | | | | 37.7 | | | | | | | |
| F112.04 | VDAQGTLSKLFKLGGRDSRS | | | | 57.1 | | | | | | | |
| F112.03 | VDAQGTLSRIFKLGGRDSRS | | | | 33.9 | | | | | | | |
| F006.08 | VHFFKNIVTPRTPPY | | | | | | | 63.7 | | | 49.7 | |
| 765.10 | VTPRTPPPSQGKGRGLSLSR | >10206.21 | | | 10000.0 | | | 91000.0 | | | | |
| F006.07 | VVHFFKNIVTPRTPPY | | | | | | | 10.3 | | | 46.1 | |
| 765.14 | YKSAHKGFKGAYDAQGTLSK | 250000.0 | | | 33333 | | | 21.7 | | | 50.3 | |
| 825.08 | YKSAHKGFKGVDAQGTLSKI | 108.3 | | | 2000.0 | | | 606.7 | | | | |
| 9.00 | ANERADLIAYLKQATK | >8333.33 | | | 1333.3 | | | 171.7 | >7142.86 | | | >25000 |
| 847.02 | EFVVEFDLPGIKA | 250000.0 | | | 200000.0 | | | 91000.0 | 14285.7 | | | >25000 |
| 593.02 | VITAFNEGLK | >3125 | | | 2222.2 | | | 91000.0 | >50000 | | | >25000 |
| 847.01 | YEFVVEFDLPGIKA | 4166.7 | | | 20000.0 | | | 91000.0 | | | | |
| 753.03 | IAFNSGLEPGVVAEK-NH2 | 250000.0 | | | 6666.7 | | | >15166.67 | | | 200000.0 | >25000 |
| 753.02 | LLPLLEKVIGAGKPL-NH2 | 250000.0 | | | 6666.7 | | | 313.8 | >50000 | | | 188.0 |
| 829.01 | YKTIAYDEEARR | 250000.0 | | | 200000.0 | | | >18200 | >50000 | | 200000.0 | >25000 |
| 831.01 | TWQRDGEDQTQDTELVETRP AG | | | | | | | | | | | |
| F009.04 | VDDTLFVRFDSDAASPREEPR | | | | | | | | | | | |
| F009.01 | VDDTLFVRFDSDATSPRKEPR | | | | | | | | | | | |
| F009.06 | VDDTQFVRFDSDAASPREEPR | | | | | | | | | | | |
| F009.02 | VDDTQFVRFDSDAASPRMAP R | | | | | | | | | | | |
| F009.05 | VDDTQFVRFDSDAASPRTEPR | | | | | | | | | | | |
| 536.00 | AHAAHAAHAAHAAHAA | >12500 | | | 10000.0 | | | 1516.7 | >7142.86 | | | 33.0 |
| N-3 | AVHAAHAEINEAGR | | | | | | | | | | | 964.4 |
| 151.00 | HIATNAVLFFGR | >25000 | | | 2500.0 | | | 91000.0 | >16666.67 | | | >25000 |
| 144.01 | ISQAAHAAHAEINE | | | | | | | | | | | 438.6 |
| 84.04 | ISQADHAAHAEINE | | | | | | | | | | | |
| 85.04 | ISQAVEAAHAEINE | | | | | | | | | | | 643.8 833.8 |
| 92.06 | ISQAVHAAHAEDNE | | | | | | | | | | | 855.0 |
| 92.05 | ISQAVHAAHAEIIE | | | | | | | | | | | 735.3 |
| 91.02 | ISQAVHAAHAQINE | | | | | | | | | | | 543.5 |
| 90.05 | ISQAVHAALAEINE | | | | | | | | | | | 892.9 |
| 144.08 | ISQAVHAANAEINE | | | | | | | | | | | 836.6 |
| 90.01 | ISQAVHAARAEINE | | | | | | | | | | | 576.6 |
| 747.01 | LKISQAVHAAHAEIN | 3.5 | | | | | | | | | | |
| 705.05 | MVYLGAKDSTRTQINKVVRF | >6250 | | | 5000.0 | | | 910.0 | >33333.33 | | | >25000 |
| 521.00 | NVMEERKIKVYLPRM | >25000 | | | 2000.0 | | | 91000.0 | >14285.71 | | | >25000 |
| 560.04 | VHAAHAEINVHAAHA | >12500 | | | 200000.0 | | | 91000.0 | >14285.71 | | | 300.3 |
| 560.03 | VHAAHAVHAAHAEIN | >12500 | | | 200000.0 | | | 91000.0 | >14285.71 | | | 37.7 |
| 560.05 | VHAAHAVHAAHAVHA | 324.7 | | | 200000.0 | | | 758.3 | >14285.71 | | | 41.6 |
| 594.03 | YTYTVHAAHAYTYT | 48.1 | | | 5000.0 | | | 700.0 | 14285.7 | | | |
| 112.06 | YTYTVHAAHAYTYT | 48.1 | | | 5000.0 | | | 700.0 | >14285.71 | | | 328.4 |
| 58.0082 | LIRVEGDLRVEYLDD | | | | | | | | | | | |
| 5.8.0081 | QHLIRVDGNLRVEYL | | | | | | | | | | | |
| 58.0091 | RFEMFRDLNEALELK | | | | | | | | | | | |
| 68.0019 | AAPLLLARAASLSLG | 24.8 | 303 | 64.4 | 99.8 | 563.8 | 3.2 ' | 35.0 | 10469.0 | 78.8 | 79.0 | |
| 68.0025 | AKELKFVTLVFRHGD | 605.8 | 1952.8 | 2354.9 | 12309.2 | 693.3 | 824.0 | 1529.5 | 8563.1 | 51.4 | 23.9 | |
| 68.0052 | ALDVYNGLLPPYASC | >28845.61 | 588.5 | 86602.5 | 182.4 | 7568.1 | >24748.74 | 1090.8 | >13969.43 | >9459.77 | 115470.1 | |
| 68.0020 | APLLLARAASLSLGF | 58.9 | 75.9 | 124.2 | 322.1 | 224.7 | 11.8 | 91.4 | 13358.3 | 58.9 | 113.9 | |
| 68.0024 | DRSVLAKELKFVTLV | 2016.1 | 15814.7 | 47193 | 20965.7 | 300.7 | 4409.6 | 1359.0 | > 10217.64 | 53.2 | 221.2 | |
| 68.0030 | DRTLMSAMTNLAALF | 382.6 | 2361.5 | 221.6 | 2366.9 | 704.3 | 114.2 | 870.9 | 3927.1 | 57.4 | 26137.9 | |
| 68.0051 | DTTVSGLQMALDVYN | 14705.9 | >110162.86 | 2875.6 | >19640.02 | 712.5 | 3500.0 | 1042.3 | 10843.6 | 960.7 | >200000 | |
| 68.0056 | FAELVGPVIPQDWST | 24056.3 | >110162.86 | 39471.8 | >19640.02 | 12503.5 | >17500 | >45500 | >13968.43 | 983.2 | >200000 | |
| 68.0028 | FGQLTQLGMEQHYEL | >51031.04 | 109567.3 | >167705.1 | 27216.6 | 652.8 | >35000 | >18200 | >10217.64 | 543.3 | 100000.0 | |
| 68.0047 | GGVLVNEILNHMKRA | 29461.8 | 3238.7 | 54410.7 | 254.6 | 6694.4 | 48.7 | 575.6 | 8124.1 | 5.8 | 8.7 | |
| 68.0156 | GPVIPQDWSTECMTT | | | | | >8184.79 | | | 202963 | 961.1 | | |
| 68.0034 | GVSIWNPILLWQPIP | 4992.0 | 11008.0 | 3984.9 | 10286.9 | 3902.2 | 207.3 | 5.0 | 4427.9 | 492.0 | 522.6 | |
| 68.0038 | ILLWQPIPVHTVPLS | 18.8 | 13091.1 | 131.0 | 2343.5 | 7288.6 | 1111.3 | 65.5 | >8824.57 | 712.3 | 28768.5 | |
| 68.0048 | IPSYKKLIMYSAHDT | 1946.3 | 60.5 | 350.5 | 52.8 | 669.1 | 2122.2 | 17.3 | 9982.0 | 12.0 | 191.1 | |
| 68.0046 | KSRLQGGVLVNEILN | 2837.6 | >49000 | 5516.2 | >29814.24 | 9605.1 | 318.3 | >26269.44 | >13968.43 | 713.0 | >100000 | |
| 68.0053 | LDVYNGLLPPYASCH | >28845.61 | 404.3 | 31277.2 | 194.3 | 9752.9 | >24748.74 | 3035.0 | >13968.43 | >10496.41 | 25819.9 | |
| 68.0050 | LIMYSAHDTTVSGLQ | 730.9 | 24812.1 | 813.4 | >29814.24 | >12214.98 | 1752.2 | 183.7 | 6827.8 | 4381.2 | >100000 | |
| 68.0023 | LLFFWLDRSVLAKEL | 135.0 | 163.2 | 518.4 | 154.2 | 178.6 | 23.9 | 33.7 | 85.8 | 7.5 | 134.5 | |
| 68.0042 | LPSWATEDTMTKLRE | >33407.66 | >49000 | >108253.18 | >28114.42 | >8417.51 | >24748.74 | >31547.94 | 5972.7 | >10717.79 | 343.5 | |
| 68.0043 | LRELSELSLLSLYCI | 4009.8 | 9367.8 | 1614.0 | 6958.4 | 1169.0 | 3217.7 | 234.8 | >13968.43 | 544.0 | 5184.8 | |
| 68.0044 | LSELSLLSLYGIHKQ | 20906.1 | 1186.3 | 1450.2 | 1657.2 | 262.1 | 1253.2 | 45.0 | >10746.91 | 79.4 | 7.3 | |
| 68.0040 | LSGLHGQDLFGIWSK | >33407.66 | >49000 | >108253.18 | 30151.1 | 7890.6 | >24748.74 | 32173.4 | >8824.57 | 134.8 | 81649.7 | |
| 68.0045 | LSLLSLYGIHKQKEK | >33407.66 | 1636.9 | 4959.5 | 742.1 | 646.5 | >24748.74 | 58.4 | >13968.43 | 771.9 | 3.4 | |
| 68.0153 | LTELYFEKGEYFVEM | 13061.7 | 18840.8 | 26949.3 | >18736.1 | 12690.0 | 3157.4 | 4042.7 | 184.4 | 600.5 | 6655.5 | |
| 68.0031 | MSAMTNLAALFPPEG | 36084.4 | 73870.3 | >144439.26 | >200000 | 3873.3 | 249.5 | 12383.5 | 7157.7 | 1072.0 | 63245.6 | |
| 68.0032 | MTNLAALFPPEGVSI | >68790.15 | 39231.4 | 22821.8 | 141421.4 | 4530.8 | 1309.8 | 10370.4 | >8824.57 | 4605.8 | 141421.4 | |
| 68.0036 | NPILLWQPIPVHTVP | 41.4 | 12998.7 | 5753 | 599.1 | 612.4 | 250.0 | 4.6 | >8824.57 | 67.1 | 25000.0 | |
| 68.0033 | PEGVSIWNPILLWQP | 15030.0 | 28577.4 | 103095.8 | 30860.7 | 7975.9 | 444.3 | 7.2 | 4623.6 | 107.3 | 22222.2 | |
| 68.0037 | PILLWQPIPVHTVPL | 45.6 | 21244.2 | 168.3 | 4040.6 | 2370.0 | 567.3 | 6.9 | >8824.57 | 106.2 | 41491.3 | |
| 68.0021 | PLLLARAASLSLGFL | 162.1 | 36.6 | 57.9 | 1254.6 | 1511.2 | 12.4 | 117.7 | >8602.89 | 52.0 | 150.7 | |
| 68.0026 | RSPIDTFPTDPIKES | >51031.04 | >109567.33 | 6123.7 | >200000 | >3132.18 | >24748.74 | 2373.5 | >10217.64 | 468.9 | 28571.4 | |
| 68.0022 | SLSLGFLFLLFFWLD | 227273 | >5809592 | 24620.0 | 100000.0 | 1220.9 | 639.5 | 11375.0 | 3710.2 | >9602.54 | 66666.7 | |
| 68.0147 | TVPLSEDQLLYLPFR | 11312.7 | 42162.0 | 37369.2 | 26455.0 | 14387.2 | 5300.1 | >2030.13 | 4322.9 | 872.5 | 27220.8 | |
| 68.0035 | WNPILLWQPIPVHTV | 520.5 | 115494.1 | 607.1 | 19640.0 | 5694.0 | 2259.2 | 14.0 | >8824.57 | 80.6 | 100000.0 | |
| 68.0039 | WQPIPVHTVPLSEDQ | 159.4 | >49000 | 17517.8 | >28114.42 | >8417.51 | 2692.3 | >31547.94 | >8824.57 | 1228.2 | >100000 | |
| 68.0041 | YDPLYCESVHNFTLP | 838.4 | 30867.1 | 643.1 | 30151.1 | >8417.51 | >35000 | 2136.1 | >8824.57 | 6900.7 | 28768.5 | |
| 68.0049 | YKKLIMYSAHDTTRVS | 291.8 | 308.8 | 107.5 | 207.7 | 927.6 | 37.4 | 14.5 | 13224.0 | 5.8 | 5482.3 | |
| 68.0054 | YNGLLPPYASCHLTE | >54554.47 | 14026.8 | 8022.4 | 5300.4 | >12214.98 | 11666.7 | 252.1 | >13968.43 | >10496.41 | 100000.0 | |
| 1188.24 | AGGIAGGLALLACAG | >72168.78 | 138871.0 | | >63245.55 | >1086432 | | 7000.0 | | >74877.68 | >13074.41 | |
| 1188.14 | ATSVLAGLLGNVSTV | >72168.78 | 95227.4 | | 25000.0 | 10819.9 | | 18200.0 | | 16307.3 | >29814.24 | |
| 1188.28 | AVPLAMKLIQQLNLN | 327.7 | 1305.2 | | 100000.0 | 306.2 | | 15166.7 | | 1659.9 | >33333.33 | |
| F143.02 | EWSPCSVTCGNGIQVRIK | 1355.0 | 44208.9 | | >50000 | >225000 | 1374.0 | >64346.72 | | | >66666.67 | |
| 27.0396 | FLALFFIIFNKESLA | 1994.4 | 23400.9 | >20044.59 | 1004.1 | >2412.22 | 1840.7 | | 4333.3 | | >6655.58 | |
| F143.09 | IEQYLKKIKNSISTEWSPCS | 250.4 | 426.5 | | 1961.0 | 20044.5 | 211.1 | 376.0 | | | 2483.4 | |
| 1188.04 | IFHINGKIIKNSEKD | 16855 | 1267.6 | 9377.5 | 539.2 | 3381.2 | 300.5 | 4203 | >1147.08 | 48502.0 | 349.9 | |
| 17.0401 | KHILYISFYFILVNL | 1533.6 | 25198.9 | | 9578.0 | >2412.22 | | 4333.3 | | | >8908.71 | |
| 1188.20 | LIDVHDLISDMIKKE | 2602.2 | 5290.8 | 1734.6 | 6085.8 | >10864.32 | 9668.5 | 1491.8 | | 646.8 | 2679.0 | |
| F150.02 | NAREIIRLHSDASKNKEKAL | >47245.56 | 24424.4 | | 2064.7 | | >17500 | 797.9 | | | 2653.1 | |
| 27.0414 | NHAVPLAMKLIQQLN | 4536.7 | 305.2 | 5991.9 | 1174.1 | 220.8 | 2010.7 | 3640.0 | | | 8074.6 | |
| F107.12 | TYLLGGVGLVLYNTE | 35816.8 | >173241.16 | | >81649.66 | >5590.06 | | 18200.0 | | | >40000 | |
| 1188.47 | VDLYLLMDCSGSIRR | 5597.2 | 73283.2 | | >50000 | >10864.32 | | 947.9 | | >52946.51 | 2522.9 | |
| 27.0410 | VKNVSQTNFKSLLRN | 3469.0 | 361.1 | >20044.59 | 186.5 | >2412.22 | 1013.5 | 3198.6 | | | 1612.4 | |
| 1188.48 | VVILTDGIPDSIQDS | >72168.78 | >141450.82 | | >50000 | >10864.32 | | 13166.7 | | 5138.6 | >33333.33 | |
| F150.03 | YADSAWENVKNVIGPFMKAV | 611.0 | 4751.3 | | 21344.7 | | >17500 | 520.8 | | | 633.8 | |
| 191.16 | ADLIAYLKQATAK | | | | 133.3 | | | 3033.3 | | | | |
| 12.04 | DLIAYLKQATAK | | | | 95.2 | | | 1300.0 | | | | |
| 12.05 | ERADLIAYLKQATAK | | | | 100.0 | | | 185.7 | | | | |
| 12.03 | IAYLKQATAK | | | | 1333.3 | | | 827.3 | | | | |
| 191.10 | KAERADLLAY'LKQATA | | | | 714.3 | | | 165.5 | | | | |
| 199.17 | LIAY'LKQATAK | | | | 181.8 | | | 1820.0 | | | | |
| F025.08 | AATYNFAVLKLMGRGTKF | | | | 16.7 | | | 239.0 | 70028.0 | 1217.9 | 17.9 | |
| K-09 | FLYGALLLAEGFYTTGAVRQ | | | | | | | 44.6 | | | 256.4 | |
| F050.02 | FLYGALLLAEGFYTTGAVRQ | | | | 25197.6 | | | | 91324.2 | 1000.6 | | |
| K-28 | FNTWTTCQSIAFPS | | | | | | | >9100 | | | 99.2 | |
| K-20 | LCADARMYGVLPWNAFPGK V | | | | | | | 44.7 | | | 5773.5 | |
| K-05 | LTGTEKLIETYFSKNYQDYE | | | | | | | 195.8 | | | 20000.0 | |
| F025.05 | QKGRGYRGQHQAHSLERVCH | | | | 30151.1 | | | >9100 | >250000 | 17950.6 | 9759.0 | |
| K-18 | SAVPVYIYFNTWTTCQSIAF | | | | | | | 92.0 | | | 20000.0 | |
| F050.05 | SAVPVYIYFNTWTTCQSIAF | | | | 33333.3 | | | | 15760.4 | 72500.0 | | |
| K-16 | TAEFQMTFHLFIAAFVGAAA | | | | | | | 548.8 | | | >20000 | |
| F025.03 | WTTCQSIAFPSKTSASIGSL | | | | 40000.0 | | | 276.9 | 37453.2 | 504.8 | 400.5 | |
| 68.0066 | AHCIRNKSVILLGRH | 2573.4 | 104.0 | 715.1 | 932 | 159.5 | 75.1 | 88.4 | 4752.4 | 8.7 | 3630.5 | |
| 68.0078 | CAQVHPQKVTKFMLC | 32274.9 | 8730.5 | 34893.2 | 18490.0 | 2697.7 | 2191.8 | 808.9 | >5543.24 | 604.1 | 1229.0 | |
| 68.0079 | GGPLVCNGVLQGITS | >29880.72 | 9333.8 | 16308.2 | 1827.9 | 3744.7 | 35.9 | 30333.3 | >3547.48 | 815.0 | 13417.3 | |
| 68.0080 | GPLVCNGVLQGITSW | 4893.0 | 4187.0 | 32639.6 | 914.6 | 1875.7 | 49.3 | 6309.7 | 11614.4 | 646.0 | 6537.2 | |
| 68.0069 | GQVFQVSHSFPHPLY | 27.0 | 548.4 | 332 | 102.9 | 557.4 | 145.7 | 2172.2 | 1070.7 | 415.9 | 127.6 | |
| 68.0062 | GRAVCGGVLVHPQWV | 3581.7 | >110162.86 | 8068.7 | >19640.02 | 16411.4 | 5455.9 | 12888.3 | >10756.16 | 62.1 | 100000.0 | |
| 68.0063 | GVLVHPQWVLTAAHC | 153.3 | 1931.0 | 364.5 | 262.5 | 7487.5 | 2426.8 | 65.7 | >10133.77 | 6.2 | 1061.9 | |
| 68.0073 | HDLMLLRLSEPAELT | 1521 | 3913.9 | 22.3 | 2141.0 | 520.0 | 2.3 | 661.8 | 5305.0 | 44.9 | 10540.9 | |
| 68.0064 | HPQWVLTAAHCIRNK | 282.5 | 1305.2 | 106.7 | 784.7 | 5790.0 | 1169.8 | 6500.0 | 1324.0 | 5518.4 | 40.1 | |
| 68.0158 | HSLFHPEDTGQVFQV | | | | | >8184.79 | | | 552.6 | 11502.9 | | |
| 68.0077 | LHVISNDVCAQVHPQ | 17451.2 | >122500 | 32670.5 | >4472136 | >7118 | 239.0 | 22750.0 | 18&6.7 | 1086.7 | >200000 | |
| 68.0081 | NGVLQGITSWGSEPC | 485.4 | 5874.3 | 819.3 | 9724.3 | 2716.4 | 775.0 | 257.6 | 8037.9 | 4487.5 | 11619.1 | |
| 68.0071 | PHPLYDMSLLKNRFL | 10699.0 | 29812.7 | 12835.5 | >4472136 | 486.7 | 11666.7 | 711.8 | >5543.24 | 7486.1 | 3103.9 | |
| 68.0065 | QWVLTAAHCIRNKSV | 213.8 | 2598.0 | 966.8 | 2169.4 | 4170.5 | 2062:4 | 13565.5 | 7341.6 | 3801.6 | 34.7 | |
| 68.0082 | RPSLYTKVVHYRKWI | 652.5 | 38.7 | 5483.8 | 350.4 | 4160.1 | 4183.3 | 717.0 | 2981.8 | 4896.6 | 13.4 | |
| 68.0072 | SHDLMLLRLSEPAEL | 58.1 | 3537.8 | 64.4 | 4470.8 | 736.7 | 5.8 | 1098.5 | 13577.7 | 11.6 | 100000.0 | |
| 68.0061 | SQPWQVLVASRGRAV | 384.7 | 383.9 | 621.3 | 135.2 | 8775.4 | 32.1 | 11258.9 | >10756.16 | 7561.8 | 83.5 | |
| 68.0067 | SVILLGRHSLFHPED | 26088.3 | 500.1 | 5216.5 | 96.1 | 91.2 | 96.3 | 105.7 | 13044.6 | 4410.6 | 16116.5 | |
| 68.0059 | SVTWIGAAPLILSRI | 82.9 | 139.4 | 30.4 | 2195.8 | 511.9 | 419.8 | 147.5 | 13676.2 | 41.7 | 104.1 | |
| 68.0074 | TDAVKVMDLPTQEPA | >28306.93 | 20874.7 | >75000 | >44721.36 | >7118 | >35000 | >37150.59 | >5543.24 | 747.2 | >141421.36 | |
| 68.0058 | TLSVTWIGAAPLILS | 15.7 | 839.7 | 5.4 | 6859.9 | 55.3 | 642.2 | 97.3 | 6030.6 | 3506.5 | 31.2 | . |
| 68.0070 | VFQVSHSFPHPLYDM | 50.6 | 8751.4 | 17.5 | 881.2 | 2477.4 | 83.2 , | 2395.6 | 23430.2 | >8574.92 | 897.2 | |
| 68.0068 | VILLGRHSLFHPEDT | 30624.8 | 737.2 | 18519.9 | 343.6 | 56.4 | 543.4 | 426.4 | >10756.16 | 10695.9 | 100000.0 | |
| 68.0060 | VTWIGAAPLILSRIV | 195.4 | 730.7 | 82.1 | 1779.4 | 818.5 | 2338.5 | 551.6 | >10133.77 | 88.3 | 147.1 | |
| 68.0125 | ADKIYSISMKHPQEM | 4098.4 | 1135.6 | 3512.2 | 169.5 | 9245.9 | 4957.0 | 8272.7 | >15552.1 | 3550.1 | 26726.1 | |
| 68.0101 | AEAVGLPSIPVHPIG | 2015.2 | >282901.63 | 23101.5 | >43643.58 | 699.6 | 5455.9 | 55.9 | >11642.8 | 12393.6 | 69336.1 | |
| 68.0102 | AVGLPSIPVHPIGYY | 1079.9 | 4431.7 | 15376.7 | 33333.3 | 384.3 | 1190.7 | 518.4 | >11642.8 | 5386.6 | 38517.1 | |
| 68.0113 | CTPLMYSLVHNLTKE | 139.7 | 223.4 | 249.3 | 589.5 | 1727.7 | 259.8 | 426.1 | 18348.6 | 58.2 | 35.9 | |
| 68.0114 | DFEVFFQRLGIASGR | 21926.5 | 122.3 | 2005.2 | 128.4 | 2005.3 | 10069.0 | 10248.9 | 30740.9 | 4.2 | 3559.1 | |
| 68.0118 | DPMFKYHLTVAQVRG | 167.6 | 42.8 | 257.7 | 68.6 | 470.2 | 699.4 | 230.0 | 7297.1 | 466.8 | 11.5 | |
| 68.0181 | DQLMFLERAFIDPLG | | | | | 146.3 | | | 17114.5 | 6.6 | | |
| 68.0111 | DSSIEGNYTLRVDCT | 14457.9 | >163333.33 | >100222.97 | >40548.01 | 8939.2 | 7.6 | 1202.3 | 575.7 | 1261.8 | 16823.9 | |
| 68.0167 | EDFFKLERDMKINCS | 8549.9 | 1439.3 | >52337.75 | 10433.3 | >10789.89 | 3188.2 | >2432.11 | 4036.4 | 7886.1 | 3494.5 | |
| 68.0109 | ERGVAYINADSSIEG | 34020.7 | >163333.33 | 25515.5 | >40548.01 | >7055.5 | 36893 | 30333.3 | 6846.0 | 87.4 | 200000.0 | |
| 68.0115 | EVFFQRLGIASGRAR | 5310.6 | 6.3 | 2976.2 | 30.9 | 2940.6 | 17500.0 | 4555.7 | >13118.19 | 51.2 | 7.9 | |
| 68.0100 | EYAYRRGIAEAVGLP | 70.5 | 596.5 | 66.6 | 2589.8 | 12278.3 | 5217.5 | >45500 | 8773.5 | 6324.6 | 1204.4 | |
| 68.0168 | FFKLERDMKINCSGK | >11622.71 | 8109.0 | >52337.75 | 9687.3 | 6935.8 | 381.7 | >2432.11 | 4917.9 | 98.4 | 3795.7 | |
| 68.0173 | GAAVVHEIVRSFGTL | | | | | 517.3 | | | 788.3 | 88.7 | | |
| 68.0096 | GKVFRGNKVKNAQLA | 2349.8 | 4120.7 | 3127.2 | 894.1 | >10595.71 | 45.8 | 3372.7 | 7591.3 | 7884.3 | 1385.1 | |
| 68.0123 | GMVFELANSIVLPFD | 29.6 | 4994.8 | 81.4 | >25819.89 | 97.5 | 11.9 | 82.8 | 234.0 | 4154.0 | 902.6 | |
| 68.0090 | GNEIFNTSLFEPPPP | >29880.72 | >126517.46 | 10414.7 | >20380.71 | >10595.71 | 2804.4 | >91000 | >12809.02 | 835.0 | >115470.05 | |
| 68.0097 | GNKVKNAQLAGAKGV | >72168.78 | 28904.2 | 7881.7 | >26726.12 | >10595.71 | >20207.26 | >45500 | >12103.61 | 1064.9 | 1217.9 | |
| 68.0110 | GVAYINADSSIEGNY | 6244.5 | 23359.8 | 3047.6 . | >36514.84 | 5493.2 . | 496.8 | 7609.8 | 1420.1 | 476.5 | 66666.7 | |
| 68.0170 | GVILYSDPADYFAPG | 7848.0 | 106290.7 | 2472.8 | >18736.1 | 1078.3 | 39.4 | 964.9 | 14.2 | 64.1 | 14167.9 | |
| 68.0103 | IGYYDAQKLLEKMGG | >72168.78 | 8235.6 | 47245.6 | >26726.12 | >7751.94 | 5729.1 | 1978.3 | 17304.0 | 13587.6 | 506.4 | |
| 68.0086 | IKKFLYNFTQIPHLA | 28.8 | 512.5 | 159.9 | 136.8 | 551.7 | 273 | 305.2 | 477.4 | 96.4 | 658.5 | |
| 68.0166 | ISIINEDGNEIFNTS | >11622.71 | >81666.67 | >52337.75 | >18736.1 | 10650.6 | 343.1 | 3005.9 | 73.6 | 5853.6 | >34616.84 | |
| 68.0126 | IYSISMKHPQEMKTY | 11573.2 | 1356.9 | 12292.8 | 212.9 | 11435.8 | >35000 | 5024.7 | >15552.1 | 5356.2 | 2588.2 | |
| 68.0087 | KFLYNFRQIPHLAGT | 29.7 | 415.0 | 54.5 | 90.8 | 1244.5 | 220.7 | 227.1 | 10211.4 | 256.3 | 16002 | |
| 68.0120 | KYHLIVAQVRGGMVF | 228.4 | 1519.1 | 5860.1 | 858.6 | 6376.7 | 192.9 | 1221.5 | >15552.1 | 3445.6 | 86.1 | |
| 68.0089 | LAHYDVLLSYPNKTH | 3617.2 | 414.9 | 1009.0 | 380.1 | 7286.3 | 268.2 | 82.3 | 1405.7 | 588.5 | 172.4 | |
| 68.0085 | LDELKAENKKFLYN | 7470.2 | 1248.2 | 12778.1 | 324.3 | 368.1 | 597.0 | 413.8 | 547.8 | 787.9 | 149.5 | |
| 68.0084 | LGFLFGWFIKSSNEA | 2261.4 | 1420.6 | 1700.6 | 7303.0 | 474.6 | 10103.6 | 354.8 | 680.8 | 9285.1 | 460.9 | |
| 68.0131 | LRMMNDQLMFLERAF | 17506.9 | 2492.0 | 4600.6 | 1832.9 | 279.9 | 1314.0 | 1411.2 | 1569.6 | 49.8 | 757.8 | |
| 68.0119 | MFKYHLTVAQVRGGM | 72.2 | 70.4 | 265.8 | 147.1 | 481.6 | 1615.2 | 1197.8 | 3647.9 | 1061.8 | 5.8 | |
| 68.0176 | NSRLLQERGVAYINA | 7996.7 | 3224.4 | 2616.1 | 12812.3 | 619.9 | 327.5 | 1229.3 | 3366.4 | 698.5 | 3472.8 | |
| 63.0112 | NYTLRVDCTPLMYSL | 24596.7 | 6322.7 | 48412.3 | 7115.7 | 593.9 | 9.0 | 5055.6 | 25.4 | 404.1 | 66666.7 | |
| 63.0127 | PQEMKTYSVSFDSLF | 1192.4 | >82825.12 | 1980.9 | >44721.36 | 5347.0 | 24748.7 | 919.2 | 14564.5 | 579.3 | 100000.0 | |
| 63.0083 | PRWLCAGALVLAGGF | 766.0 | 26530.7 | 1438.6 | >20380.71 | 4596.1 | 20207.3 | 15166.7 | 13149.2 | 883.1 | 40824.8 | |
| | QIYVAAFTVQAAAET | 323.7 | 101.7 | 64.5 | 33.8 | 933.7 | 343.6 | 251.8 | 1323.8 | 50.5 | 216.1 | |
| 68.0135 68.0122 | RGGMVFELANSIVLP | 41.5 | 8682.4 | 33.1 | >25819.89 | 208.0 | 4.4 | 94.2 | 131.7 | 411.4 | 413.4 | |
| 68.0133 | RHVIYAPSSHNKYAG | 31250.0 | 11666.7 | 481.2 | 13363.1 | 7082.2 | 8750.0 | 1290.8 | >12475.05 | 5293.4 | 87.6 | |
| 68.0134 | RQIYVAAFTVQAAAE | 291.9 | 36.1 | 90.9 | 34.6 | 609.0 | 524.4 | 166.3 | 6807.4 | 47.2 | 142.6 | |
| 68.0105 | TGNFSTQKVKMHIHS | 9407.4 | 10281.7 | 1450.5 | 11856.3 | 11636.9 | 6187.2 | 3744.6 | >11642.8 | 508.5 | 1926.9 | |
| 63.0116 | TNKFSGYPLYHSVYE | 30853.4 | 613.6 | 740.7 | 33333.3 | 4482.1 | >24748.74 | 489.3 | >15552.1 | 12465.6 | 2942.5 | |
| 68.0107 | TRIYNVIGTLRGAVE | 4806.0 | 70.4 | 2900.4 | 45.4 | 502.4 | 1459.6 | 1605.3 | 17550.0 | 447.4 | 31.6 | |
| 68.0128 | TYSVSFDSLFSAVKN | 345.5 | 2256.0 | 526.2 | 5981.5 | 5276.7 | 5888.4 | 3223.4 | 8546.3 | 10461.3 | 61.1 | |
| 68.0136 | VAAFTVQAAAETLSE | 792.9 | 1420.0 | 127.5 | 2126.2 | 4460.8 | 445.9 | 18200.0 | 2116.0 | 464.1 | 377.7 | |
| 69.0121 | VAQVRGGMVFELANS | 4448.6 | >98000 | 499.1 | >44721.36 | 7605.2 | 2802.2 | 116.5 | >15552.1 | 99.9 | 64366.0 | |
| 68.0177 | VAYINADSSIEGNYT | 9744.9 | 105831.5 | 5467.3 | >18736.1 | 8247.2 | 2147.4 | >243211 | 471.3 | 841.5 | >34616.84 | |
| 68.0124 | VFELANSIVLPFDCR | 39.0 | 36122.8 | 49.9 | 11764.7 | 525.2 | 23.7 | 477.2 | 128.0 | 1215.0 | 10814.8 | |
| 68.0130 | VLRMMNDQLMFLERA | 17334.4 | 1699.9 | 10683.8 | 2353.0 | 98.5 | 129.8 | 126.8 | 97.9 | 88.1 | 85.0 | |
| 68.0088 | WKEFGLDSVELAHYD | 3511.0 | 19970.9 | 7051.7 | 4935.0 | >10595.71 | 8413.1 | 22750.0 | 829.1 | 5925.1 | 89442.7 | |
| 68.0117 | YDPMFKYHLTVAQVR | 158.2 | 171.9 | 178.7 | 252.5 | 239.7 | 1014.3 | 1349.5 | 6137.4 | 5527 | 62.4 | |
| 68.0165 | YISINEDGNEIFNT | 23719.2 | >81666.67 | 83056.5 | >18736.1 | >10438.01 | 345.8 | 2713.2 | 52.9 | 3705.3 | 72992.7 | |
| 68.0132 | YRHVIYAPSSHNKYA | 8792.9 | 750.0 | 528.4 | 1461.8 | 742.7 | 6390.1 | 896.8 | 61349.7 | 1117.4 | 54.1 | |
| F1112.02 | VDAQGTLSRLFKLGGRDSRS | | | | 25.3 | | | | | | | |
| 938.01 | KVNNQVVSLKPEIIVDQEY | 2041.2 | | | >10000 | | | | | | | |
| F160.32 | STORKUSP32 | | | | | | | | | | | |
| F160.34 | STORKUSP34 | | | | | | | | | | | |
| F160.36 | STORKUSP36 | | | | | | | | | | | |
| F160.37 | STORKUSP37 | | | | | | | | | | | |
| F160.39 | STORKUSP39 | | | | | | | | | | | |
| F047.09 | DKLKQQRDTLSTQKET | >12578.87 | | | | | | | | 611.4 | | |
| F047.16 | EQKSKQNIGALKQEL | >12996.88 | | | | | | | | 110.7 | | |
| 938.06 | SGGTNYAQKFQGRVTMTRDT -NH2 | 8838.8 | | | 1571.4 | | | | | | | |
| 938.08 | ELSRLSDDTAVYYCARAPG- NH2 | 4166.7 | | | 40.8 | | | | | | | |
| 938.09 | ELSRLTSDDTAVYYCAIAPG- NH2 | 2311.3 | | | 3162.3 | | | | | | | |
| 938.10 | TISCSGSSSNIGSNTVN-NH2 | 17677.7 | | | >6666.67 | | | | | | | |
| F015.05 | APYHFDLSGHA | | | | | | | | | | | 208.3 |
| 791.08 | APYHFDLSGHAF | | | | | | | | | | | 603.7 |
| 791.07 | APYHFDLSGHAFGS | | | | | | | | | | | 714.3 |
| 791.06 | APYHFDLSGHAFGSMA | | | | | | | | | | | 54.0 |
| 791.05 | APYHFDLSGHAFGSMAKK | | | | | | | | | | | |
| 610.11 | APYHFDLSGHAFGSMAKKGE | >12500 | | | 1000.0 | | >5051.81 | 9592.2 | 172.4 | | 20000.0 | 101.4 90.0 |
| 620.05 | CGYKDVDKAPFNGMTGCGN T | >12500 | | | 200000.0 | | | 91000.0 | >20000 | | | >25000 |
| 807.03 | DLSGHAFGS | | | | | | | | | | | 928.5 |
| 595.02 | EDVIPEGWKADTSYSAK | >8333.33 | | | | | | | | | | |
| 620.14 | ELQFRRVKCKYPDDTKPTFH | 8333.3 | | | 200000.0 | | | 202.2 | >25000 | | | >25000 |
| 620.13 | EQNVRSAGELELQFRRVKCK | 5000.0 | | | 2000.0 | | | 479.0 | >25000 | | | >25000 |
| 620.20 | ESWGAVWRIDTPDKLTGPFT | >12500 | | | 444.4 | | 2268.7 | 1011.1 | 18.9 | | | 186.0 |
| 791.02 | FDLSGHAFGSMAKKGE | | | | | | | | | | | 143.9 |
| 620.18 | GDVVAVVDIKEKGKDKWIEL K | >12500 | | | 200000.0 | | | 4550.0 | >25000 | | | >25000 |
| MA-05 | GEKRAYAASDPGRYC | 6250.0 | | | 2222.2 | | | 910.0 | 408.2 | | | 17677.7 |
| 620.19 | KGKDKWIELKESWGAVWRID | 5000.0 | | | 3333.3 | | | 3033.3 | 55.6 | | | 295.9 |
| 791.03 | LSGHAFGSMAKKGE | | | | | | | | | | | 443.0 |
| 620.23 | SEVEDVIPEGWKADTSYSAK | 4166.7 | | | 6666.7 | | | 91000.0 | 1041.7 | | | >25000 |
| MA-06 | VAYESSEIASKKAG | >8333.33 | | | 200000.0 | | | 15.4 | >16666.67 | | | 25000.0 |
| 620.16 | VEKGSNPNYLAILVKYVDGD | 61.0 | | | 2000.0 | | | 3033.3 | >25000 | | | 156.0 |
| 791.09 | YHFDLSGHAFGS | | | | | | | | | | | 944.9 |
| 791.01 | YHFDLSGHAFGSMAKKGB | | | | | | | | | | | 171.1 |
| 620.15 | YPDDTKPTKPTFHVEKGSNPNYL | >12500 | | | 200000.0 | | | 3033.3 | >25000 | | | 1178.5 |
| F165.05 | KSDNQIKAVPASQALVA | 59.0 | | | | | 40.7 | | | | | |
| F165.01 | FKSDNQIKAVPAS | 5989.5 | | | | | >8750 | | | | | |
| F165.03 | PKSDNQIKAVPASQA | 1107.8 | | | | | 30.0 | | | | | |
| F165.02 | VRPKSDNQIKAVPAS | 1582.1 | | | | | 1197.7 | | | | | |
| 213-16 | FRKDIAAKYKELGY | | | | 83.3 | | | 3033.3 | 169.5 | | | |
| 213.15 | LFRKDIAAKYKELGY | | | | 66.7 | | | 1820.0 | 256.4 | | | |
| 542.00 | NKALELFRKDIAA | | | | 909.1 | | | 91000.0 | 16666.7 | | | |
| 413.12 | NKALELFRKDIAAK | | | | 142.9 | | | 91000.0 | 8333.3 | | | |
| 213.11 | NKALELFRKDIAAKY | | | | 105.3 | | | 91000.0 | 0.0 | | | |
| 13.00 | NKALELFRKDIAAKYKELGY QG | | | | 80.0 | | | 650.0 | 178.6 | | | |
| 213.17 | RKDIAAKYKELGY | | | | 142.9 | | | 91000.0 | >20000 | | | |
| NASE061-80 | FTKKMVENAKKIEVEFDKGQ | 8333.3 | | | 6666.7 | | | 455.0 | >25000 | | | >25000 |
| F015.01 | GLAKVAYVYKP | | | | | | | | | | | 962.3 |
| NASE121-140 | HEQHLRKSEAQAKKEKLNIW | 6250.0 | | | 200000.0 | | | 4.8 | >5555.56 | | | >25000 |
| 191.26 | LVRQGLAKVAY | | | | 200000.0 | | | 4550.0 | | | | 533.0 |
| NASE011-30 | PATLIKAIDGDTVKLMYKGQ | 8333.3 | | | 6666.7 | | | 1516.7 | 2381.0 | | | >25000 |
| NASE031-50 | PMTFRLLLVDTPETKHPKKG | >12500 | | | 6666.7 | | | 1820.0 | >5555.6 | | | >25000 |
| 191.29 | QGLAKVAYVYK | | | | 1333.3 | | | 4550.0 | | | | 75.2 |
| 191.28 | RQGLAKVAYVY | | | | 200000.0 | | | 4550.0 | | | | 988.2 |
| NASE041- 60 | TPETKHPKKGVEKYGPEASA | 12500.0 | | | 6666.7 | | | 1820.0 | >11785.5 | | | >25000 |
| NASE051- 70 | VEKYGPEASAFTKKMVENAK | 12500.0 | | | 20000.0 | | | 15.6 | 16666.7 | | | 2635.2 |
| 191.27 | VRQGLAKVAYV | | | | 200000.0 | | | 4550.0 | | | | 637.1 |
| NASE091- 110 | YIYADGKMVNEALVRQGLAK | 1562.5 | | | 6666.7 | | | 1820.0 | >5555.56 | | | 12500.0 |
| 546.00 | FTKKMVENAKKIEVEFDKGQ | 250000.0 | | | 3333.3 | | | 116.7 | | | | >25000 |
| 866.05 | HEQHLRKSEAQAKKEKLNIW | 25000.0 | | | 200000.0 | | | 7.6 | | | 16.7 | |
| 598.00 | PATLIKAIGDTVKLMYKGQ | 250000.0 | | | 2222.2 | | | 568.8 | | | | >25000 |
| 866.06 | QAKKEKLNIWSEDNADSGQ | 250000.0 | | | 200000.0 | | | 91000.0 | | | | |
| 866.02 | VEKYGPEASAFTKKMVENAK | 250000.0 | | | 200000.0 | | | 85.9 | | | | |
| 866.04 | YIYADGKMVNEALVRQGLAK | 8333.3 | | | 20000.0 | | | 30333 | | | | 25000.0 |
| 835.03 | YGAVDSILGGVATYGAA-NH2 | 250000.0 | | | 200000.0 | | | 91000.0 | | | | 26.9 |
| F178.08 | AGTIAALNNSIGVLG | 4848.3 | 4873.6 | 4351.8 | 6001.8 | 2069.8 | 14.9 | 204.3 | 3870.9 | 175.9 | 99711.3 | 6099.4 |
| F178.10 | GSISYPARYANAMAV | 688.0 | 36795 | 1575.8 | 22091.7 | >7434.33 | 2701.3 | 832.5 | >4156.45 | 6815.0 | 100930.8 | 6125.0 |
| F178.06 | GTVAALDNSAGVLGV | 5286.3 | 36537.9 | 3850.5 | 5734.7 | 1466.7 | 122.6 | 983.0 | 699.9 | 404.9 | 19777.6 | 220.6 |
| F178.05 | GTVAALDNSIGVLGV | 376.6 | 22837.4 | 328.5 | 9283.1 | 651.2 | 26.1 | 60.4 | 45.6 | 48.5 | 27201.0 | 1467.1 |
| F178.03 | GTVAALNNSAGVLGV | 1178.0 | 12106.6 | 549 | 3925.6 | 577.4 | 3.5 | 140.2 | 1788.8 | 53.5 | 24476.1 | 1053.6 |
| F178.01 | GTVAALNNSIGVLGV | 213.8 | 2519.7 | 56.8 | 3330.3 | 74.0 | 1.7 | 21.0 | 1468.0 | 41.7 | 13528.6 | 491.9 |
| F178.09 | IAALNNSIGVLGVAP | 851.3 | 2926.4 | 131.1 | 3058.5 | 194.5 | 3.0 | 273.9 | 1654.6 | 6.9 | 14400.4 | 4843.7 |
| F178.04 | NGIEWAIANNMDVAN | 148.6 | 3188.2 | 74.7 | 1742.4 | 503.0 | 17.8 | 214.3 | 95.9 | 603.7 | 1044.3 | 3305.5 |
| F178.02 | NGIEWAIANNMDVIN | 109.5 | 2209.2 | 45.9 | 3910.6 | 932.5 | 14.0 | 561.7 | 116.0 | 719.2 | 983.1 | 2510.3 |
| F178.11 | SYPARYANAMAVGAT | 129.6 | 2415.5 | 196.2 | 5392.5 | >7476.32 | 465.1 | 113.4 | 10999.9 | 10139.5 | 2044.3 | 71.2 |
| F178.07 | TGSGVKVAVLDTGIS | >18494.24 | 177601.5 | >30905.75 | >19820.12 | >6676.56 | 2755.6 | >4297.05 | 1217.5 | 11.3 | >30573.62 | 2653.0 |
| 573.13 | ANSKFIGITELKK | 250000.0 | | | 10000.0 | | | 568.8 | >11111.11 | | | |
| 573.12 | IKANSKFIGITELKK | 2500.0 | | | 33.9 | | | 178.4 | 7142.9 | | | |
| 597.10 | ILMQYIKANS | 12500.0 | | | 200000.0 | | | 91000.0 | | | | |
| L-05 | QAIKANSKFIGITE | | | | 6666.7 | | | 65.0 | >3703.7 | | | |
| 650.05 | QEIKANSKFIGITE | 12500.0 | | | 57.1 | | | 267.7 | >50000 | | | >25000 |
| 650.06 | QSIKANSKFIGITE | 5000.0 | | | 40.0 | | | 131.9 | | | | |
| 650.21 | QYIKANQKFIGITE | 1315.8 | | | 26.3 | | | 20.2 | 14285.7 | | | >25000 |
| 650.29 | QYIKANSKFKGITE | 5000.0 | | | 35.1 | | | 2.3 | >50000 | | | >25000 |
| 650.14 | QYIKKNSKFIGITE | 531.9 | | | 46.5 | | | 2600.0 | >50000 | | | >25000 |
| 650.16 | QYIKSNSKFIGITE | 20.8 | | | 57.1 | | | 32.5 | | | | |
| 650.13 | QYIRANSKFIGITE | 8.9 | | | | | | 9.5 | 18896.5 | | | >25000 |
| 573.14 | SKFIGTELKK | 781.3 | | | 645.2 | | | 2275.0 | >14285.71 | | | |
| 548.02 | YNGQIGNDPNRDIL | | | | | | | | 0.0 | | | |
| 534.02 | DIPYLDITYHFVMQRLPL | 390.6 | | | 200000.0 | | | 91000.0 | 800.0 | | | >25000 |
| 1385.07 | GELIGILNAAKVPAD | 516.9 | | | | | 13.0 | | >2599.36 | 931.9 | | |
| F167.11 | ALHIYMDGTMSQVQGSA | >10879.85 | | | | | 2933.4 | | | >16680.72 | | |
| F167.12 | ALHIYMNQTMSQVQGSA | >9731.24 | | | | | 65.5 | | | >56873.68 | | |
| F089.22 | DQSYLQDSDPDSFQD | | | | | | | | | | | |
| F089.13 | DYSFLQDSDPDSFQD | | | | >66666.67 | | | | | | | |
| F089.25 | DYSYFQDSDPDSFQD | | | | | | | | | | | |
| 1385.10 | DYSYLQDSDPDSFQD | >11037.77 | | | | | >10552.9 | | 6582.4 | 31455.1 | | |
| F089.11 | DYSYLQDSVPDSFQD | >102062.07 | >48048.45 | >79950.27 | >66666.67 | >6708.41 | 17500.0 | >37150.59 | | | >36514.84 | |
| F089.24 | DYSYQQDSDPDSFQD | | | | | | | | | | | |
| 1385.09 | FLLHHAFVDSIFEQWLQRHRP | 578.6 | | | | | >6390.1 | | >2196.88 | 21.6 | | |
| F089.18 | ILLSNAPLGPQFP | | | | >66666.67 | | | | | | | |
| F089.20 | NILLSNAPLGPQFP | | | | 49508.7 | | | | | | | |
| F089.06 | QNFLLSNAPLGPQFP | 78.1 | 169.1 | 92.0 | 1677.1 | >6708.41 | 1.6 | 1184.7 | | | 1291.0 | |
| F089.28 | QNIFLSNAPLGPQFP | | | | | | | | | | | |
| F089.04 | QNILLSNAPQGPQFP | | | | 33256.7 | | | | | | | |
| F089.33 | QNILLSNAQLGPQFP | | | | | | | | | | | |
| F089.34 | QNILLSNAVLGPQFP | | | | | | | | | | | |
| F089.03 | QNILLSNQPLGPQFP | | | | 6234.6 | | | | | | | |
| F089.07 | QNILLSNVPLGPQFP | 869.1 | 5853.2 | 1143.8 | 26263.3 | | 3.9 | 250.2 | | | >36514.84 | |
| F089.30 | QNILQSNAPLGPQFP | | | | | | | | | | | |
| F089.32 | QNILYSNAPLGPQFP | | | | | | | | | | | |
| P089.27 | QNIQLSNAPLGPQFP | | | | | | | | | | | |
| F089.29 | QNIVLSNAPLGPQFP | | | | | | | | | | | |
| F089.19 | QNVLLSNAPLGPQFP | | | | 24784.1 | | | | | | | |
| F089.15 | SYLQDSDPDSFQD | | | | >66666.67 | | | | | | | |
| F089.21 | SYLQDSVPDSFQD | >102062.07 | >49048.45 | >60436.72 | >66666.67 | >6708.41 | >17500 | >45500 | | | >36514.94 | |
| F167.09 | WPSVFYNRTCQCSGNF | 14367.8 | | | | | 391.8 | | | >17950.55 | | |
| F167.10 | YGQMKNGSTPMFNDINTYDL | 1186.5 | | | | | 172.2 | | | 6392.5 | | |
| F089.16 | YLQDSDPDSFQD | | | | >66666.67 | | | | | | | |
| F089.14 | YSYLQDSDPDSFQD | | | | >66666.67 | | | | | | | |
| 604.01 | (AKA)6 | >12500 | | | | | | | | | | |
| 848.01 | A(56)FAAAA(24)A(56)AA(57)- NH2 | 286.9. | | | 2.0 | | | 1.4 | | | | |
| 848.03 | A(56)FAAAA(24)L(56)AA(57)- NH2 | 174.1 | | | 26.6 | | | 4.6 | | | | |
| 848.05 | A(56)FAAAATA(56)AA(57)- NH2 | 537.8 | | | 79.7 | | | 5.1 | | | | |
| 848.07 | A(56)FAAAATL(56)AA(57)- NH2 | 235.3 | | | 16.7 | | | 5.4 | | | | |
| F042.01 | A(X)KQNTLKLAT | | | | | | | | | | | |
| 848.02 | AAFAAAA(24)A(56)AA(57)- NH2 [?] | 145.7 | | | 36.0 | | | 2.5 | | | | |
| 848.04 | AAFAAAA(24)L(56)AA(57)- NH2 | 137.5 | | | 143 | | | 1.6 | | | | |
| 848.06 | AAFAAAATA(56)AA(57)-NH2 | 596.8 | | | 18.2 | | | 13.3 | | | | |
| 848.08 | AAFAAAATL(56)AA(57)-NH2 | 133.4 | | | 10 | | | 4.1 | | | | |
| 603.01 | AHAAHAAHAAHAAHAAY | >12500 | | | | | | | | | | |
| F182.04 | EVIPMFSALSEGA | 15001.0 | | 5769.3 | 10733.4 | | | | | | 52426.9 | |
| 520.05 | EVWREEAYRAADIKD | 25000.0 | | | 10000.0 | | | 3033.3 | >12599.22 | | | >25000 |
| 520.06 | BVWRBEAYHAADIKDY | | | | | | | | | | | |
| 852.04 | KYVKQNTLKLAT | | | | | | | | | | | |
| 852.05 | KYVKQNTLKLAT | | | | | | | | | | | |
| F182.09 | LNKIVRMYSPTSI | 1030.8 | | 7820.2 | 138.9 | | | | | | 971.4 | |
| F182.10 | NKIVRMYSFTSIL | 274.9 | | 250.7 | 306.1 | | | | | | 11409 | |
| F042.06 | PMKQN7LKLAT | | | | | | | | | | | |
| F042.07 | P(X)KQNTLKLAT | | | | | | | | | | | |
| F182.03 | PEVIPMFSALSEG | 23900.6 | | 132744.2 | 3185.4 | | | | | | 11281.7 | |
| F182.01 | PIVQNIQGQMVHQ | 15399.3 | | 665242.5 | >74649.21 | | | | | | 70687.3 | |
| F042.02 | PKFV(X)TLKLAT | | | | | | | | | | | |
| F042.05 | PKTVKQNTL(X)T | | | | | | | | | | | |
| F042.03 | PKFVQ(X)KLAT | | | | | | | | | | | |
| F182.08 | QEQIQWMTNNPPI | 4044.9 | | 313909.2 | >74649.21 | | | | | | 158188.1 | |
| 832.01 | TFGLQLELTEGMRFDKG | 289.2 | | | 20000.0 | | | 4550.0 | | | | |
| 173.00 | TYQRTRALVTG | 51.6 | | | 4000.0 | | | 91000.0 | >16666.67 | | | >25000 |
| F182.02 | VQNIQGQMVHQAI | 22807.6 | | 446065.2 | >74649.21 | | | | | | 280386.1 | |
| 833.01 | YLDPLIRGLLARPAKLQV- NH2 | 250000.0 | | | 200000.0 | | | 91000.0 | | | | 269.9 |
| 785.02 | YKKSIQFHWKNSNQIKILG | | | | | | | | | | | |
| 843.03 | YKLNDRADSRRSL | 250000.0 | | | 227.3 | | | 91000.0 | | | | |
| F002.02 | YKPVSQLRLATPLLLRPL | 14.8 | | | 434.8 | | | 0.9 | | 51.0 | 21.1 | |
| 835.02 | YLDPLIRGLLARPAKLQV- NH2 | 250000.0 | | | 101.0 NH2 | | | 216.7 | 1000000.0 | | | 10206.2 |
| F002.01 | YLPKPPKPVSKLRLATPLLLQ ALPL | 22.8 | | | 1451.0 | | | 48.2 | | 99.4 | 933 | |
| 824.07 | (14)AAAKTAAAFA-NH2 | 2551.6 | | | 3.2 | | | 5.9 | | | | |
| 824.08 | (15)AAAKTAAAFA-NH2 | 4419.4 | | | 1.3 | | | 1.6 | | | | |
| 820.03 | (15A)RQTTLKAAA-NH2 | 250000.0 | | | 1.8 | | | 4.3 | | | | |
| 920.05 | (15A)RQTTLKAAA-NH2 | | | | | | | 4550.0 | | | | |
| 824.09 | (16)AAAKTAAAFA-NH2 | 25000.0 | | | 64.5 | | | 325.0 | | | | |
| 820.04 | (16A)RQTTLKAAA-NH2 | 250000.0 | | | 76.9 | | | 1920.0 | | | | |
| 853.01 | (39)AAAATKAA(35)-NH2 | | | | | | | | | | | |
| 853.02 | (39)AAAATKAA(36)-NH2 | | | | | | | | | | | |
| 853.03 | (39)AAAATKAA(37)-NH2 | | | | | | | | | | | |
| 824.37 | (39)AAAATKAAAA | | | | | | | | | | | |
| 824.04 | (39)AAAKTAAA(35)-NH2 | | | | | | | | | | | |
| 824.03 | (39)-AAAKTAAAFA-NH2 | 250000.0 | | | 65.8 | | | 1516.7 | 1000000.0 | | | |
| 824.21 | (39)AAAKTAAAF-NH2 | | | | | | | | | | | |
| 824.26 | (39)AAKTAAAFA-NR2 | | | | | | | | | | | |
| 824.02 | (40)-AAAKTAAAFA-NH2 | 250000.0 | | | 769.2 | | | 4550.0 | 1000000.0 | | | |
| 717.63 | (42)YARFQSQTTLKAKT-NH2 | 7905.7 | | | 50.9 | | | 137.9 | >4347.83 | | | >25000 |
| 787.34 | (43)AADFFFFFFFFDA-(NH2) | | | | | | | | | | | |
| 787.43 | (43)AAFGIDIFGFKIA-(NH2) | | | | | | | | | | | |
| 824.11 | (45)AAAKTAAAFA-NH2 | | | | | | | | | | | |
| 824.28 824.12 | (46)AAAATKAAAA (46)AAAKTAAAFA-NH2 | 25000.0 | | | 129.0 | | | 827.3 | | | | |
| 824.29 | (47)AAAATKAAAA | | | | | | | | | | | |
| 824.13 | (47)AAAKTAAAFA-NH2 | 25000.0 | | | 1176.5 | | | 3033.3 | | | | |
| 824.14 | (48)AAAKTAAAFA-NH2 | | | | | | | | | | | |
| 824.31 | (49)AAAATKAAAA | 9333.3 | | | 500.0 | | | 1516.7 | | | | |
| 862.06 | (49)AAAKTAA(64)A-NH2 | | | | | | | | | | | |
| 856.03 | (49)AAARTAAA(35)·NH2 | | | | | | | | | | | |
| 862.07 | (49)AAAKTAAA(64)-NH2 | | | | | | | | | | | |
| 862.01 | (49)AAAKTAAAAA-NH2 | | | | | | | | | | | |
| 64.15 | (49)AAAKTAAAFA-NH2 | 8333.3 | | | 12.5 | | | 30.6 | | | | 27.2 |
| 824.23 | (49)AAKTAAAFA-NH2 | | | | | | | | | | | |
| 824.32 | (50)AAAATICAAAA | | | | | | | | | | | |
| 824.16 | (50)AAAKTAAAFA-NH2 | >17677.67 | | | 1428.6 | | | 2275.0 | | | | |
| 824.45 | (51)AAAATKAAAA | 12500.0 | | | 869.6 | | | 1011.1 | | | | |
| 814.43 | (51)AAAKTAAAFA-NH2 | 25000.0 | | | 370.4 | | | 154.2 | | | | |
| 824.44 | (52)AAAKTAAAFA-NH2 | 12500.0 | | | 285.7 | | | 910.0 | | | | |
| 824.51 | (53)AAAATKAAAA | | | | | | | | | | | |
| 824.47 | (53)AAAKTAAAFA-NH2 | 25000.0 | | | 7692 | | | 3033.3 | | | | |
| 814.52 | (54)AAAATKAAAA | | | | | | | | | | | |
| 824.48 | (54)AAAKTAAAFA-NH2 | 15000.0 | | | 2000.0 | | | 2275.0 | | | | |
| 824.50 | (SS)AAAKTAAAFA-NH2 | | | | | | | | | | | |
| 601.42 | (6S)(66)PKFVKQNTLKLAT | 129.5 | | | 588.2 | | | 11.7 | | | | |
| 752.03 | (67)AAYAAAAAAKAA-NH2 | >12500 | | | 666.7 | | | 433 | 33333.3 | | | 357.1 |
| 752.02 | (67)FAAAAAAKAA-NH2 | >25000 | | | 5000.0 | | | 126.4 | 50000.0 | | | 143.5 |
| 858.07 | (CP)-QSQTTLKAKT-NH2 | 250000.0 | | | 117.7 | | | 1011.1 | | | | |
| 859.03 | (CP)YAAFQRQTTLKAAA-NH2 | 250000.0 | | | 2.9 | | | 123.0 | | | 297.7 | |
| Sandoz374 | (NAF)AAAKTAAAFA-NH2 | | | | | | | | 4000.0 | | | |
| 934.15 | (X2)KSSQYIKANSKFIGITEAA AFLPSDFFPSV | 1666.7 | | | 2390.5 | | | 568.8 | | 23678.4 | 1296.4 | |
| 803.11 | A(14)AAAKTAAAAA-CONH2 | 892.9 | | | 10.5 | | | 22.6 | >5000 | | | 67.6 |
| 838.02 | A(14)AAAKTAAAA-NH2 | 454.6 | | | 108.7 | | | 46.0 | | | | |
| 760.73 | A(14)AAAKTAAAFA-NH2 | | | | | | | | | | | |
| 838.01 | A(14)AAAKTAAA-NH2 | 96.2 | | | 42.7 | | | 54.3 | | | | |
| 838.04 | A(14)AAAKTAAA-NH2 | | | | | | | | | | | |
| 863.10 | A(14)AAAKTAA-CONH2 | | | | | | | | | | | >8333.3 |
| 838.03 | A(14)AAAKTAA-NH2 | | | | | | | | | | | |
| 736.21 | AA(10)AAAAAAKAAA-NH2 | 190.8 | | | 153.9 | | | 13.4 | 50000.0 | | | 82.2 |
| 736.23 | AA(12)AAAAAAKAAA-NH2 | | | | | | | | | | | |
| 839.29 | AA(14)A(37)ATKAAAA | | | | | | | | | | | |
| 828.03 | AA(14)AAAA(2A)KAAAA-NH2 | 92.3 | | | 6.6 | | | 2.6 | >4545.45 | | | 193.9 |
| 736.25 | AA(14)AAAAAAKAAA-NH2 | 297.6 | | | 22.2 | | | 13.4 | | | | |
| 828.01 | AA(14)AAAAPKAAAA-NH2 | 102.0 | | | 8.9 | | | 3.2 | | | | 47.4 |
| 906.30 | AA(14)AAAATEKAAA-NH2 | 773.7 | | | 55.4 | | | 11.8 | 756.5 | | 26.2 | 252.0 |
| 906.30 | AA(14)AAAATFKAAA-NH2 | 44.8 | | | 33 | | | 0.8 | 4767.4 | | 1.9 | 31.0 |
| 906.34 | AA(14)AAAATIKAAA-NH2 | 80.9 | | | 3.2 | | | 1.1 | 896.2 | | 2.3 | 69.2 |
| 839.17 | AA(14)AAAATK(36)AA | | | | | | | | | | | |
| 839.31 | AA(14)AAAATK(37)AA | | | | | | | | | | | |
| 839.06 | AA(14)AAAATKA(33)A | | | | | | | | | | | |
| 839.18 | AA(14)AAAATKA(36)A | | | | | | | | | | | |
| 839.32 | AA(14)AAAATKA(37)A | | | | | | | | | | | |
| 839.20 | AA(14)AAAATKAA(3S)-NH2 | 58.1 | | | 16.7 | | | 3.5 | | | | |
| 839.24 | AA(14)AAAATKAA(36)-NH2 | 20833 | | | 6.7 | | | 1A | | | | |
| 839.35 | AA(14)AAAATKAA(37)-NH2 | | | | | | | | | | | |
| 839.25 | AA(14)AAAATKAAAA | 201.6 | | | 9.1 | | | 1.9 | | | | |
| 828.11 | AA(14)AAAATKAAAA-NH2 | 192.3 | | | 7.4 | | | 7.6 | >4545.45 | | | 70.0 |
| 906.29 | AA(14)AAAATKKAAA-NH2 | 317.8 | | | 7.8 | | | 13 | >19611.69 | | 2.9 | 471.7 |
| 906.35 | AA(14)AAAATLEAAA-NH2 | 169.6 | | | 11.3 | | | 7.0 | 1494.0 | | 17.3 | 67.8 |
| 906.38 | AA(14)AAAATLFAAA-NH2 | 62.4 | | | 19.3 | | | 1.8 | 2719.6 | | 4.1 | 27.8 |
| 760.57 | AA(14)AAAATLKAAA-NH2 | 171.1 | | | 6.9 | | | 492.1 | 2827.2 | 80.9 | 4.3 | 99.2 |
| 906.47 | AA(14)AAAATLKAEA-NH2 | 147.8 | | | 26.8 | | | 2.4 | 1792.9 | | 5.7 | 103.9 |
| 906.50 | AA(14)AAAATLKAFA-NH2 | 793 | | | 10.1 | | | 21.2 | 459.8 | 30.1 | 3.7 | 35.5 |
| 906.48 | AA(14)AAAATLKAQA-NH2 | 102.9 | | | 20.1 | | | 1.0 | 443.5 | | 2.2 | 327 |
| 906.49 | AA(14)AAAATLKAVA-NH2 | 29.3 | | | 4.6 | | | 1.0 | 604.6 | | 2.2 | 29.5 |
| 906.41 | AA(14)AAAATLKBAA-NH2 | 252.6 | | | 295.2 | | | 13.0 | >19611.69 | | 31.0 | 449.0 |
| 906.44 | AA(14)AAAATLKFAA-NH2 | 26.2 | | | 117.7 | | | 3217.3 | 21.6 | 845.7 | 2.3 | 277.8 |
| 906.45 | AA(14)AAAATLKIAA-NH2 | 28.5 | | | 30.6 | | | 1.2 | 54.4 | | 2.6 | 93.9 |
| 906.40 | AA(14)AAAATLKKAA-NH2 | 625.0 | | | 39.3 | | | 5253.9 | >1754.39 | 8202.4 | 0.6 | 61.0 |
| 906.42 | AA(14)AAAATLKQAA-NH2 | 275.5 | | | 29.4 | | | 2.7 | 5661.2 | | 13.4 | 806.5 |
| 906.43 | AA(14)AAAATLKVAA-NH2 | 49.5 | | | 11.9 | | | 54.2 | 85.5 | 2092.9 | 2.0 | 43.4 |
| 906.36 | AA(14)AAAATLQAAA-NH2 | 120.6 | | | 17.1 | | | 1.6 | 5423.3 | | 3.8 | 46.1 |
| 906.39 | AA(14)AAAATLRAAA-NH2 | | | | | | | | | | 1.2 | 48.0 |
| 906.31 | AA(14)AAAATQKAAA-NH2 | 97.0 114.9 | | | 6.9 9.0 | | | 0.5 | 5263.2 1465.7 | | 1.9 | 89.0 |
| 906.32 | AA(14)AAAATVKAAA-NH2 | 58.3 | | | 4.2 | | | 0.9 1.3 | 2917.9 | | 3.0 | 55.5 |
| 906.24 | AA(14)AAAETLKAAA-NH2 | 423.1 | | | 11.4 | | | 2.8 | 343.4 | | 6.3 | 82.6 |
| 906-27 | AA(14)AAAFTLKAAA-NH2 | 67.6 | | | 2.6 | | | 1.6 | 3823.7 | | 3.5 | 73.7 |
| 856.02 | AA(14)AAAKTAAA(35)-NH2 | | | | | | | | | | | |
| 760.50 | AA(14)AAAKTAAAAA-NH2 | 753.1 | | | 6.1 | | | 743.0 0 | 2416.8 | 95.3 | 1250.0 | 56.5 |
| 906.23 | AA(14)AAAKTLKAAA-NH2 | 29.9 | | | 2.2 | | | 0.7 | 4445.6 | | 2.8 | 52.9 |
| 906-25 | AA(14)AAAQTLKAAA-NH2 | 154.1 | | | 3.3 | | | 0.8 | 4545.5 | | 1.8 | 71.2 |
| 906.28 | AA(14)AAATTLKAAA-NH2 | 75.2 | | | 53 | | | 0.6 | 2461.8 | | 1.4 | 50.0 |
| 906.26 | AA(14)AAAVTLKAAA-NH2 | 69.7 | | | 4.1 | | | 0.7 | 104.2 | | 1.5 | 46.6 |
| 906.19 | AA(14)AAEATLKAAA-NH2 | >1388.89 | | | 6.8 | | | 6434.7 | 315.7 | 92.4 | 10.0 | 1136.4 |
| 906.22 | AA(14)AAFATLKAAA-NH2 | 70.8 | | | 4.1 | | | 1.5 | 2858.3 | | 3.7 | 3125.0 |
| 906.18 | AA(14)AAKATLKAAA-NH2 | 25000.0 | | | 1.6 | | | 1.6 | >19611.69 | | 3.8 | 581.4 |
| 906.20 | AA(14)AAQATLKAAANH2 | 2727.7 | | | 4.2 | | | 1.5 | >19611.69 | | 3.4 | 1087.0 |
| 906.21 | AA(14)AAVATLKAAA-NH2 | 45.4 | | | 2.5 | | | 0.7 | 3261.7 | | 1.6 | 7143 |
| 906.14 | AA(14)AEAATLKAAA-NH2 | 331.1 | | | 14.6 | | | 15.1 | 11180.7 | | 24.4 | 3571.4 |
| 916.17 | AA(14)AFAATLKAAA-NH2 | 33.8 | | | 3.3 | | | 20.5 | 1145.9 | 22.8 | 1.2 | 137.8 |
| 906.13 | AA(14)AKAATLKAAA-NH2 | 97.6 | | | 3.3 | | | 629.0 | 22361.4 | 605.4 | 3.4 | >1336.31 |
| 906.15 | AA(14)AQAATLKAAA-NH2 | 104.2 | | | 5.7 | | | 0.8 | 21838.8 | | 1.9 | 581.4 |
| 906.16 | AA(14)AVAATLKAAA-NH2 | 240.2 | | | 7.4 | | | 75.8 | 1744.5 | 13.5 | 1.3 | 3571.4 |
| 906.07 | AA(14)EAAATLKAAA-NH2 | 221.8 | | | 4.4 | | | 7.6 | 3760.8 | | 18.0 | 114.5 |
| 906.10 | AA(14)FAAATLKAAA-NH2 | 32.8 | | | 4.8 | | | 1.8 | 501.4 | | 4.1 | 159.8 |
| 906.11 | AA(14)IAAATLKAAA-NH2 | 30.6 | | | 4.2 | | | 20.5 | 173.1 | 42.A | 1.4 | 188.7 |
| 906.06 | AA(14)KAAATLKAAA-NH2 | 53.5 | | | 2.8 | | | 1.8 | 318.7 | | 4.3 | 57.7 |
| 906.12 | AA(14)LAAATLKAAA-NH2 | 47.5 | | | 4.8 | | | 44.6 | 305.6 | 41.7 | 1.6 | 97.4 |
| 906.08 | AA(14)QAAATLKAAA-NH2 | 171.9 | | | 5.5 | | | 1.A | 443.0 | | 3.2 | 123.1 |
| 906.09 | AA(14)VAAATLKAAA-NH2 | 77.2 | | | 2.6 | | | 14.3 | 100.0 | 39.0 | 1.9 | 86.0 |
| 906.56 | AA(14)VVAATLKAFA | 64.6 | | | 3.8 | | | 6.2 | 844.8 | 12-3 | 8.9 | |
| 828.13 | AA(15)AAAA1KAAAA-NH2 | 47.5 | | | 8.3 | | | 0.5 | | | | |
| 819.01 | AA(15)AAKTAAAFA-NH2 | 7.8 | | | 1.2 | | | 0.9 | | | | |
| 819.02 | AA(15)AAKTGGGFG-NH2 | 136.1 | | | 2.5 | | | 1.9 | | | | |
| 819.04 | AA(15)GGKGGGGFG-NH2 | | | | | | | | | | | |
| 819.03 | AA(15)GGKTAAAFA-NH2 | 2273.3 | | | 2.0 | | | 9.1 | | | | |
| 819.05 | AA(15)GGKTGGGFG-NH2 | | | | | | | | | | | |
| 736.28 | AA(17)AAAAAAKAAA-NH2 | | | | | | | | | | | |
| 736.33 | AA(2)AAAAAAKAAA-CONH2 | | | | | | | | | | | |
| 773.08 | AA(31)AAAAAAKAAA-NN2 | | | | | | | | | | | |
| 736.17 | AA(6)AAAAAAKAAA-NH2 | | | | | | | | | | | |
| 736.18 | AA(7)AAAAAAKAAA-NH2 | | | | | | | | | | | |
| 736.19 | AA(8)AAAAAAKAAA-NH2 | | | | | | | | | | | |
| 736.20 | AA(9)AAAAAAKAAA-NH2 | 2083.3 | | | 181.8 | | | 53.5 | 50000.0 | | | 224.7 |
| 736.34 | AA(TIC)AAAAAAKAAA- CONH2 | | | | | | | | | | | |
| 871.14 | AAAAKAATLKAAA-NH2 | | | | | | | | | | | 493.6 |
| 760.27 | AAAFAAAKTAAAFA-NH2 | 29.4 | | | 16.7 | | | 6.5 | 1000000.0 | | | 36.1 |
| 730.04 | AAAKAAAAAA(10)AA- CONH2 [?] | | | | | | | | | | | |
| 730.02 | AAAKAAAAAAFAA-CONH2 | 1785.7 | | | 20000.0 | | | 1516.7 | >50000 | | | >25000 |
| 702.02 | AAAKAAAAAAYAA | 12500.0 | | | 200000.0 | | | 1300.0 | 6250.0 | | | 423.7 |
| 702.06 | AAAKAAAAAAYAA-COHN2 | 3125.0 | | | 2222.2 | | | 364.0 | 16666.7 | | | 25000.0 |
| 789.05 | AAAKAAAAAPAAA | | | | | | | | | | | |
| 730.07 | AAAKATAAAA(23)AA- CONH2 | >6250 | | | 1818.2 | | | 395.7 | >50000 | | | 1087.0 |
| 787.06 | AADFGIFIDFILA-(NH2) | | | | | | | | | | | |
| 736.10 | AABAAAAAAKAAA-NH2 | | | | | | | | | | | |
| 736.07 | AAFAAAAAAKAAA-NH2 | | | | | | | | | | | |
| 761.03 | AAFAAAAAARLFA-NH2 | | | | | | | | | | | |
| 828.07 | AAFAAAAB(24)KAAAA-NH2 | 1041.7 | | | | | | 5.4 | | | | 162.3 |
| 760.71 | AAFAAAATAKAAA | 752.4 | | | 22.8 | | | 26.0 | >4545.45 | | | 91.4 |
| 760.41 | AAFAAAATAKAAA-NH2 | 147.1 | | | 12.5 | | | 13.4 | >4545.45 | | | 36.6 |
| 760.72 | AAFAAAATLKAAA | 321.5 | | | 13.3 | | | 10.7 | >4545.45 | | | 18.0 |
| 760.43 | AAFAAAATLKAAA-NH2 | 64.1 | | | 9.5 | | | 8.3 | >4545.45 | | | 37.9 |
| 871.12 | AAFAAAATLKAKA-NH2 | | | | | | | | | | | 44.2 |
| 871.11 | AAFAAAATLKKAA-NH2 | | | | | | | | | | | 771.5 |
| 760.37 | AAFAAAKTAAAAA-NH2 | 280.3 | | | 5.6 | | | 23.2 | >4545.45 | | | 39.6 |
| 803.08 | AAFAAAKTAAAFA-CONH2 | | | | | | | | | | | 35.0 |
| 760.15 | AAFAAAKTAAAFA-NH2 | 67.5 | | | 4.6 | | | 11.2 | 20000.0 | | | 49.1 |
| 760.33 | AAFAAAKTAAAFA-NH2 | 98.9 | | | 7.7 | | | 10.5 | >4545.45 | | | 47.4 |
| 760.17 | AAFAAAKTAAAFE-NH2 | | | | | | | | | | | |
| 803.13 | AAFAAAKTAAAKA-NH2 | | | | | | | | | | | |
| 760.47 | AAFAAAKTLAAAA-NH2 | 180.7 | | | 5.1 | | | 14.9 | >4545.45 | | | 22.7 |
| 891.10 | AAFAAAKTLKAAA-MH2 | | | | | | | | | | | 47.6 |
| 841.09 | AAFAAKATLKAAA-NH2 | | | | | | | | | | | 1767.8 |
| 760.70 | AAFAANKNAAFAA-CONH2 | 862.1 | | | 7143 | | | 182.0 | | | | |
| 760.68 | AAFAAQKQAAFAA-CONH2 | 250000.0 | | | 1176.5 | | | 57.2 | | | | |
| 760.69 | AAFAATKTAAFAA-CONH2 | 8.3 | | | 222.2 | | | 11.5 | | | | |
| 760.64 | AAFAKAATAKAAA-CONH2 | 641.0 | | | 5.9 | | | 36.4 | >4545.45 | | | 83.0 |
| 760.63 | AAFAKAATLKAAA-CONH2 | 115.2 | | | 4.2 | | | 10.2 | >4545.45 | | | 1222.8 |
| 871.06 | AAFAKAATTLKAKA-MH2 | | | | | | | | | | | 2351.6 |
| 871.05 | AAFAKAATLKKAA-NH2 | | | | | | | | | | | 1062.1 |
| 871.04 | AAFAKAKTLKAAA-NH2 | | | | | | | | | | | 1511.8 |
| 871.03 | AAFAKKATLKAAA-NH2 | | | | | | | | | | | >12500 |
| 787.05 | AAFFGIFKIGKFA-(NH2) | | | | | | | | | | | |
| 787.11 | AAFGIKIFGFKIA-(NH2) | | | | | | | | | | | |
| 871.02 | AAFKKAATLKAAA-NH2 | | | | | | | | | | | 25000.0 |
| 770.04 | AAFPPPPTLKAAA-NH2 | | | | | | | | | | | 25000.0 |
| 793.03 | AAFVSQTTLKAAA | | | | | | | | | | | |
| 736.06 | AAHAAAAAAKAAA-NH2 | | | | | | | | | | | |
| 787.03 | AAIGFFFFKKGIA-(NH2) | | | | | | | | | | | |
| 787.07 | AAIGGIFIFKKDA-(NH2) | | | | | | | | | | | |
| 736.08 | AALAAAAAAKAAA-NH2 | 1315.8 | | | 2222.2 | | | 30.3 | | | | |
| 760.09 | AALKATAAAYAA-NH2 | | | | | | | | | | | |
| 736.09 | AAWAAAAAAKAAA-NH2 | | | | | | | | | | | |
| 758.01 | AAYA(4)A(4)AAKAAA | | | | | | | | | | | |
| 758.02 | AAYAA(4)A(4)AKAAA | | | | | | | | | | | |
| 773.06 | AAYAAAA(24)AKAAA-NH2 | | | | | | | | | | | |
| 773.02 | AAYAAAA(26)AKAAA.NH2 | | | | | | | | | | | |
| 713.03 | AAYAAAA(27)AKAAA-NH2 | | | | | | | | | | | |
| 773.04 | AAYAAAA(28)AKAAA-NH2 | | | | | | | | | | | |
| 773.05 | AAYAAAA(29)AKAAA-NH2 | | | | | | | | | | | |
| 773.07 | AAYAAAA(30)AKAAA-NH2 | | | | | | | | | | | |
| 702.03 | AAYAAAAAAKAAA.CONH2 | 129.9 | | | 9J | | | 7.0 | 3030.3 | | | 47.9 |
| 713.09 | AAYAAAAPAKAAA-CONH2 | | | | | | | | | | | |
| 736.04 | AAYAAAATAAAKA-NH2 | | | | | | | | | | | |
| 736.03 | AAYAAAATAKAAA-NH2 | | | | | | | | | | | |
| 736.05 | AAYAAAAYAAAKA-NH2 | | | | | | | | | | | |
| 736.14 | AAYAAJJAAKAAA-NH2 | | | | | | | | | | | |
| 871.13 | AAYAKAATLKAAA-NH2 | | | | | | | | | | | 481.6 |
| 760.58 | AC-AA(14)AAAATLKAAA- NH2 | 289.0 | | | 7.4 | | | 3.5 | >4545.45 | | | 70.7 |
| 760.51 | AC-AA(14)AAAKTAAAAA- NH2 | 315.5 | | | 56.2 | | | 91.0 | >4545-45 | | | 3965 |
| 730.13 | AC-AAAKAAAAAA(23)AA- CONH2 | | | | | | | | | | | |
| 702.08 | AC-AAAKAAAAAAYAA | | | | | | | | | | | |
| 702.12 | AC-AAAKAAAAAAYAA- CONH2 | 2777.8 | | | 5000.0 | | | 4550.0 | | | | |
| 760.42 | AC-AAFAAAATAKAAA-NH2 | 533.9 | | | 26.2 | | | 27.2 | >4545.45 | | | 50.3 |
| 760.44 | AC-AAFAAAATLKAAA-NH2 | 1075.8 | | | 13.3 | | | 16.2 | >4545.45 | | | 68.8 |
| 760.38 | AGAAFAAAKTAAAAA-NH2 | | | | | | | | >4347.83 | | | |
| 760.34 | AC-AAFAAAKTAAAFA-NH2 | 203.7 | | | 20.0 | | | 67.4 | >4545.45 | | | 472.0 |
| 760.48 | AC-AAFAAAKTLAAAA-NH2 | 5000.0 | | | 25.2 | | | 202.2 | | | | |
| 760.67 | AC-AAFAANKNAAFAA- CONH2 | 1190.5 | | | 909.1 | | | 246.0 | | | | |
| 760.65 | AC-AAFAAQKQAAFAA- CONH2 : | 250000.0 | | | 1176.5 | | | 455.0 | | | | |
| 760.66 | AC-AAFAATKTAAFAA- CONH2 | 50.0 | | | 555.6 | | | 97.9 | | | | |
| 806.06 | AC-YAA(10)LFLSAARKRA- NH2 | 1679.2 | | | 12.5 | | | 0.5 | | | | |
| 760.60 | AC-YAA(14)AAAATLKAAA- NH2 | 705.7 | | | 14.3 | | | 7.6 | >4347.83 | | | 85.5 |
| 760.53 | AC-YAA(14)AAAKTAAAAA- NH2 | 1010.6 | | | 11.8 | | | 56.5 | >4347.83 | | | 81.3 |
| 760.55 | AC-YAAFAAAATAKAAANH2 | 25000.0 | | | 384.6 | | | 84.3 | 4347.8 | | | 131.0 |
| 760.46 | AC-YAAFAAAATLKAAA-NH2 | 1000.8 | | | 20.0 | | | 11.6 | >4347.83 | | | 48.1 |
| 760.40 | AC-YAAFAAAKTAAAAA-NH2 | 1250.0 | | | 38.6 | | | 93.8 | >4347.83 | | | 33.5 |
| 760.36 | AGYAAFAAAKTAAAFA-NH2 | 1828.2 | | | 64.9 | | | 66.4 | >4347.83 | | | 63.5 |
| 760.62 | AC-YAAFAAAKTLAAAA-NH2 | 249.6 | | | 5.4 | | | 17.1 | >4347.83 | | | 97.3 |
| 805.03 | AC-YARFLALTTLRARA- CONH2 | | | | | | | | | | | 12500.0 |
| 906.02 | AE(14)AAAATLKAAA-NN2 | 181.8 | | | 5.8 | | | 2.1 | 2942.5 | | 4.6 | 50.2 |
| 7.60.13 | AEFAAAKTLAAFA-NH2 | | | | | | | | | | | |
| 906.05 | AF(14)AAAATLKAAA-NH2 | 35.7 | | | 3.4 | | | 0.8 | 1335.1 | | 1.9 | 39.5 |
| 851.09 | AFAAAKTAA(71) | | | | | | | | | | | |
| 803.05 | AFAAAKTAAAA-CONH2 | | | | | | | | | | | 74.2 |
| 803.04 | AFAAAKTAAA-NH2 | | | | | | | | | | | 757.6 |
| 760.05 | AFLRAAAAAAFAAY-NH2 | | | | | | | | | | | |
| 906.01 | AK(14)AAAATLKAAA-NH2 | 88.9 | | | 4.6 | | | 1.2 | 1346.0 | | 2.9 | 42.3 |
| 963.16 | AK(14)VAAATLKAAA-NH2 | 234.2 | | | 23.2 | | | 2.8 | | 88.5 | 10.9 | |
| 965.15 | AK(14)VAAHTLKAAA-MH2 | 168.6 | | | 30.1 | | | 2.5 | | 50.3 | 4.0 | |
| 965.14 | AK(14)VAAKTLKAAA-NH2 | 232.9 | | | 34.5 | | | 3.6 | | 63.2 | 8.6 | |
| 965.08 | AK(14)VAANTLKAAA-NH2 | 198.9 | | | 8.9 | | | 1.7 | | 100.1 | 3.6 | |
| 965.08 | AK(14)VAANTLKAAA-NH2 | 146.8 | | | 13.7 | | | 3.3 | | | 5.5 | |
| 965.10 | AK(14)VAAWTLKAAA-NH2 | 157.7 | 97.1 | 127.6 | 16.1 | >2852.25 | 220.0 | 45.1 | 268.6 | 104.9 | 16.9 | 42.8 |
| 965.17 | AK(14)VAAWTLKAAA-NH2 | 151.3 | | | 15.4 | | | 2.7 | | 201.1 | 7.9 | |
| 965.09 | AK(14)VAAYTLKAAA-NH2 | 247.5 | | | 8.2 | | | 3.4 | | 53.3 | 8.5 | |
| 965.09 | AK(14)VAAYTLKAAA-NH2 | 57.8 | | | 4.9 | | | 2.9 | | | 6.7 | |
| 905.07 | AK(14)VKAHTLKAAA-NH2 | 174.0 | | | 10.5 | | | 2.3 | | | 5.4 | |
| 965.07 | AK(14)VKAHTLKAAA-NH2 | 581.6 | | | 5.3 | | | 3.0 | | | 6.7 | |
| 965.01 | AK(14)VKANTLKAAA-NH2 | 492.2 | | | 13.2 | | | 5.7 | | | 13.4 | |
| 965.01 | AK(14)VKANTLKAAA-NH2 | 875.4 | | | 2.4 | | | 4.8 | | | 11.3 | |
| 965.02 | AK(14)VKAWTLKAAA-NH2 | 93.1 | | | 7.3 | | | 2.6 | | | 5.7 | |
| 965.02 | AK(14)VKAWTLKAAA-NH2 | 357.1 | | | 1.1 | | | 4.1 | | | 9.4 | |
| 965.03 | AK(14)VWANTLKAAA-NH2 | 206.9 | | | 8.8 | | | 2.5 | | 261.2 | 5.8 | |
| 965.03 | AK(14)VWANTLXAAA-NH2 | 291.5 | | | 3.3 | | | 239.8 | 4134.5 | | 11.0 | 3227.5 |
| 965.04 | AK(14)VWAYTLKAAA-NH2 | 157.4 | | | 3.5 | | | 1.2 | | | 2.6 | |
| 965.04 | AK(14)VWAYTLKAAA-NH2 | 673.0 | | | 4.4 | | | 4.3 | | | 10.1 | |
| 965.05 | AK(14)VYAWTLKAAA-NH2 | 885.0 | | | 5.7 | >20454.55 | | 3.3 | | | 7.3 | |
| 965.05 | AK(14)VYAWTLKAAA-NH2 | 252.5 | | | 7.0 | | | 4.1 | | | 9.1 | |
| 537.00 | AKAAKAAKAAKAAKAA | 12500.0 | | | 363.6 | | | 16.6 | >7142.86 | | | 12500.0 |
| 871.07 | AKFAAAATLKAAA-NH2 | | | | | | | | | | | 62.6 |
| 803.14 | AKFAAAKTAAAKAAA-NH2 | | | | | | | | | | | |
| Sandoz339 | AKFAAALTLAAPA-NH2 | | | | | | | | >4545.45 | | | 84.7 |
| Sandoz338 | AKFAAALTLAQAA-NH2 | | | | | | | | >4545.45 | | | 7216.9 |
| 1303.02 | AKFIAADTLKAAA | | | | | | | | | | | |
| 1303.01 | AKFIAAWTLKAAA | | | | | | | | | 3164.2 | | |
| 1024.03 | AKFVAAWTLKAAA-NH2 | 270.4 | 12.0 | 178.4 | 19.8 | 5487.8 | 290.6 | 29.0 | 213.9 | 798.2 | 16.2 | 44.5 |
| 1024.06 | AKFVAAWTLKAAA-NH2 | 100.9 | 9.5 | 69.9 | 7.3 | | 572.0 | 50.4 | | 5362.1 | 30.4 | |
| 1024.04 | AKFVAAYTLKAAA-NH2 | 362.4 | | | 15.3 | | | 59.0 | 300.6 | 1421.8 | 13.6 | 30.8 |
| 1303.04 | AKFVADWTLKAAA | 15212.8 | 281.4 | | 263.9 | >29355.39 | 248.8 | 8974.4 | | >72500 | 1854.8 | |
| 1303.03 | AKFVIAWTLKAAA | 178.2 | 20.4 | | 27.8 | 30000.0 | 234.1 | 7.7 | | 10103.7 | 13.1 | |
| 1024.01 | AKFVWANTLKAAA-NH2 | 264.9 | | | 15.3 | | | 1174.8 | 415.2 | 5919.6 | 47.6 | 12500.0 |
| 1024.02 | AKFVYAMLKAAA-KH2 | 322.4 | | | 33.6 | | | 1985.8 | 6250.0 | 1764.9 | 21.7 | 12500.0 |
| 1303.08 | AKIVAAWTLKAAA | 302.8 | 99.2 | | 176.4 | 13803 | 134.5 | 76.1 | | 5515.1 | 190.2 | |
| 1303.06 | AKIVADWTLKAAA | >47245.56 | 6381.7 | | 3379.4 | >28355.39 | 145.3 | 653.3 | | >7250 | >66666.67 | |
| 1303.09 | AKLVAAWTLKAAA | | | | | | | | | | | |
| 1303.10 | AKMVAAWTLKAAA | 396.8 | 100.0 | | 265.1 | 90000.0 | 277.5 | 160.7 | | 7934.4 | 178.7 | |
| 1303.07 | AKMVADWTLKAAA | >45950-91 | 4289.6 | | 5310.6 | >28355-39 | 173.8 | 4338.5 | | >72500 | 61728.4 | |
| 906.03 | AQ(14)AAAATLKAAA-NH2 | 53.2 | | | 3.7 | | | 0.8 | 1136.4 | 34.1 | 92.2 | |
| 965.06 | AR(14)VRANTLKAAA-NH2 | 84.0 | | | 4.4 | | | 2.9 | | | 6.6 | |
| 820.07 | AR(15A)RQTTLKAAA-NH2 | 1250.0 | | | 1.6 | | | 0.5 | | | | |
| 788.06 | ARFQRQTTLKAAA | | | | | | | | | | | |
| 781.08 | ARFQRQTTLKAAA-NH2 | | | | | | | | | | | |
| 640.05 | ARRLKARRLKAIY | >12500 | | | 10000.0 | | | 650.0 | >16665.67 | | | >25000 |
| 906.04 | AV(14)AAAATLKAAA-NH2 | 122.9 | | | 3.1 | | | 123.8 | 5564.2 | 10.8 | 2.2 | 69.5 |
| 906.51 | AV(14)AVAATLKAFA | 77.8 | | | 3.6 | | | 38.9 | 411.3 | 1.3 | 8.6 | |
| 906.55 | AV(14)VFAATLKAFA | 91.4 | | | 7.0 | | | 18.1 | 3603.7 | 25.5 | 16.5 | |
| 906.52 | AV(14)WAATLKAAA | 135.0 | | | 5.4 | | | 62.2 | 221.7 | 46.6 | 11.2 | |
| 906.53 | AV(14)WAATLKAFA | 86.2 | | | 6.3 | | | 26.6 | 133.2 | 7.8 | 14.0 | |
| 640.01 | AWRNRAKAWRNRAKAWRN RAK | >9333.33 | | | 714.3 | | | 182.0 | >10000 | | | 1785.7 |
| 640.03 | AWRNRAKAWRNRAKGTD | >12500 | | | 5000.0 | | | 104.6 | >12500 | | | 2512.6 |
| 718.03 | AYAAA(4)A(4)KAAA | | | | | | | | | | | |
| S5004 | AYZAYAYITKAAA | | | | | | | | | | | |
| S5003. | BOC-AYZAYAYTLKAAA | | | | | | | | | | | |
| S1395 | BOCXFAXAXTLKAAA | | | | | | | | 3030.3 | | | |
| 631.02 | DDYVKQYTKQYTKQNTLKK | 12500.0 | | | 1111.1 | | | 16.0 | >11111.11 | | | >25000 |
| 631.03 | DDYVKQYTKQYTKQNTLKK | >25000 | | | 645.2 | | | 175.0 | >11111.11 | | | |
| 760.08 | EFAAATKAAAFAAY-NH2 | | | | | | | | | | | |
| 819.06 | GG(15)GGKGGGGFG-NH2 | | | | | | | | | | | |
| 736.13 | JJYJJAAAAKAAA-NH2 | >12500 | | | 200000.0 | | | 91000.0 | >50000 | | | 90.7 |
| 640.06 | KNRAKNRAKNRAKNRAK | >12500 | | | 200000.0 | | | 197.8 | >12500 | | | >25000 |
| 640.07 | KRLKRLKRLKRLKRL | >12500 | | | 87.0 | | | 168.5 | 5882.4 | | | >25000 |
| 965.19 | KSSAK(14)VAAWTLKAAA- NH2 | 514.6 | 29.3 | | 6.8 | | | 10.7 | | | 24.8 | |
| 784.08 | PKYDKQOGLKIAT | | | | | | | | | | | |
| 784.05 | PKYFKQFRLKIAT | | | | | | | | | | | |
| 784.13 | PKYFKQIGLKRAT | | | | | | | | | | | |
| 784.04 | PKYGKQRFLKIAT | | | | | | | | | | | |
| 784.15 | PKYIKQDGLKGAT | | | | | | | | | | | |
| 784.09 | PKYIKQFFLKRAT | | | | | | | | | | | |
| 784.01 | PKYIKQIILKIAT | | | | | | | | | | | |
| 784.10 | PKYRKQFILKGAT | | | | | | | | | | | |
| 736.01 | PNYAAAAAAKAAA-NH2 | >4166.67 | | | | | | | | | | |
| 73631 | PNYAAAAAAKCAA-OONH2 | | | | | | | | | | | |
| 781.07 | RFQRQTTLKAAA-NH2 | | | | | | | | | | | |
| 716.02 | TAIKLTTQRQFRAY | >14433.76 | | | 200000.0 | | | 4550.0 | 14493 | | | |
| 717.11 | TKQKLTTQSQFRAY-NH2 | >14433.76 | | | 200000.0 | | | 91000.0 | >3333333 | | | |
| S5006 | XFAXAXTLKAAA | | | | | | | | | | | |
| Sandoz364 | YA(AMK)FAAAKTAAAFA- NH2 | | | | | | | | 4545.5 | | | |
| Sandoz366 | YA(K1)FQ(K1)QTTL(K1)AAA- OH | | | | | | | | 4347.8 | | | |
| 806.05 | YAA(10)LFLSAARKRA-NH2 | 2777.8 | | | 5.6 | | | 2.6 | | | | 1923.1 |
| 828.04 | YAA(14)AAAA(24)KAAAA- NH2 | 121.4 | | | 31.8 | | | 3.7 | >4347.83 | | | 79.4 |
| 828.02 | YAA(14)AAAAPKAAAA-NH2 | 192.3 | | | 10.3 | | | 4.7 | | | | 36.8 |
| 828.12 | YAA(14)AAAATKAAAA-NH2 | 115.7 | | | 7.7 | | | 5.2 | >4347.83 | | | 37.1 |
| 760.59 | YAA(14)AAAATLKAAA-NH2 | 256.2 | | | 7.1 | | | 4.8 | >4347.83 | | | 30.5 |
| 760.52 | YAA(14)AAAKTAAAAA-NH2 | 406.5 | | | 6.9 | | | 14.5 | 2439.0 | 64.1 | | 20.8 |
| 906.54 | YAA(14)AVAATLKAAA | 716.3 | | | 9.5 | | | 10.6 | 20000.0 | 48.7 | 235 | |
| 828.14 | YAA(15)AAAATKAAAA-NH2 | 75.8 | | | 8.3 | | | 0.3 | | | | |
| Sandoz363 | YAA(AMF)AAAKTAAAFA- NH2 | | | | | | | | 4545.5 | | | |
| 760.26 | YAAAKAAAAAAFAA-NH2 | | | | | | | | | | | |
| Sandoz368 | YAAFAAA(DHPS)TAAAFA- NH2 | | | | | | | | | | | 452 |
| Sandoz367 | YAAFAAA(K1)TAAAFA-NH2 | | | | | | | | 4000.0 | | | 82.8 |
| 828.08 | YAAFAAAA(24)KAAAA-NH2 | 1190.5 | | | 129.9 | | | 3.3 | | | | 72.1 |
| 760.25 | YAAFAAAAAAKAAA-NH2 | 266.0 | | | 487.8 | | | 75.8 | 1000000.0 | | | 409.8 |
| 761.04 | YAAFAAAAAARLFA-NH2 | | | | | | | | | | | |
| 761.02 | YAAFAAAAAQRLFA-NH2 | | | | | | | | | | | |
| 740.54 | YAAFAAAATAKAAA-NH2 | 575.1 | | | 15.4 | | | 14.4 | >4347.83 | | | 11.9 |
| 760.45 | YAAFAAAATLKAAA-NH2 | 279.7 | | | 9.5 | | | 43 | >4347.83 | | | 28.3 |
| 760.39 | YAAFAAAKTAAAAA-NH2 | 563.8 | | | 12.5 | | | 29.2 | >4347.83 | | | 79.9 |
| 760.39 | YAAFAAAKTAAAAA-NH2 | 1562.5 | | | 18.2 | | | 91.0 | | | | |
| 760.39 | YAAFAAAKTAAAAA-NH2 | | | | 46.3 | | | | | | | |
| 760.16 | YAAFAAAKTAAAPA-NH2 | 33.7 | | | 2.6 | | | 4.3 | 33333.3 | 30.0 | | 35.6 |
| 760.35 | YAAFAAAKTAAAFA-NH2 | 1275.8 | | | 13.3 | | | 46.7 | >4347.83 | 168.1 | | 255.2 |
| Sandoz362 | YAAFAAAKTAAAFA-NH2 | | | | | | | | >4347.83 | | | 38.9 |
| 760.18 | YAAFAAAKTAAAFE-NH2 | | | | | | | | | | | |
| 760.61 | YAAFAAAKTLAAAA-NH2 | 134.7 | | | 5.0 | | | 20.0 | >4347.83 | | | 37.1 |
| 788.05 | YAAFQRQTTLKAAA | | | | | | | | | | | |
| 788.04 | YAAFQSQTTLKAAA | | | | | | | | | | | |
| 793.02 | YAAFVRQTTLKAAA | | | | | | | | | | | |
| 793.01 | YAAFVSQTTLKAAA | | | | | | | | | | | |
| 760.14 | YAEFAAAKTLAAFA-NH2 | | | | | | | | | | | |
| 781.16 | YAK(14)QRQTTLKAAA- CONH2 | >25000 | | | 3.6 | | | 31.4 | >50000 | | | >25000 |
| Sandoz370 | YAKFAAAKTAAAA(TR) | | | | | | | | >4545.45 | | | 38.9 |
| 717.08 | YAKFKSTTKKRKS-NH2 | 785.7 | | | 87.0 | | | 70.0 | >33333.33 | | | |
| 1341.01 | YAKFVAAWTLKAAA | | | | | | | | 2502.4 | 395.8 | | |
| 717.35 | YAR(10)QSQTTLKAKT-NH2 | 25000.0 | | | 8.0 | | | 323 | | | | |
| 781.20 | YAR(14)QKQTTLKAAA | >12500 | | | 7.1 | | | 615 | | | | |
| 788.01 | YAR(14)QRQTTLKAAA | | | | | | | | | | | |
| 781.15 | YAR(14)QRQTTLKAAA- CONH2 | >25000 | | | 2.6 | | | 75.8 | >50000 | | | >25000 |
| 782.03 | YAR(15)ASQTTLKAKT-NH2 | 1195.9 | | | 3.9 | | | 1.7 | | | | >25000 |
| 782.07 | YAR(15)ASQTTLKAKT-NH2 | | | | | | | | | | | |
| 820.02 | YAR(15A)RQTTLKAAA-NH2 | 8333.3 | | | 1.2 | | | 0.4 | | | | |
| 820.11 | YAR(15A)RQTTLKCAAA-NH2 | 250000.0 | | | 1.2 | | | 5.4 | | | | |
| 820.01 | YAR(15A)SQTTLKAKT-NH2 | 1785.7 | | | 2.5 | | | 0.7 | | | | |
| 820.10 | YAR(15A)SQTTLKAKT-NH2 | 250000.0 | | | 4.2 | | | 22.8 | | | | |
| 782.04 | YAR(16)ASQTTLKAKT-NH2 | 25000.0 | | | 800.0 | | | 76.5 | | | | |
| 717.34 | YAR(9)QSQTTLKAKT-NH2 | 2988.1 | | | 47.6 | | | 118.2 | | | | |
| 717.43 | YAREQSQTTLKAKT-NH2 | >25000 | | | 200000.0 | | | 4550.0 | | | | |
| 782.06 | YARFGGQTTLKAKT | | | | | | | | | | | |
| 805.01 | YARFLALTTLRARA-CONH2 | | | | | | | | | | | >25000 |
| 805.02 | YARFLALTTLRARA-CONH2 | | | | | | | | | | | >25000 |
| 717.36 | YARFQSQT(24)LKAKT-NH2 | >25000 | | | 14.3 | | | 12.5 | | | | |
| 717.18 | YARFQSQTELKAKT-NH2 | 3608.4 | | | 4000.0 | | | 379.2 | | | | |
| 782.01 | YARFQSQTTL(32)AKT-NH2 | 25000.0 | | | 57.1 | | | 151.7 | >4347.83 | | | >25000 |
| 717.24 | YARFQSQTTLKEKT-NH2 | 4166.7 | | | 6666.7 | | | 193.6 | | | | |
| 715.01 | YARRLKAIFARRLKA | 0.0 | | | | | | | | | | |
| 788.03 | YASFQSQTTLKAAA | | | | | | | | | | | |
| 788.02 | YASFVSQTTLKAAA | | | | | | | | | | | |
| 785.01 | YKKSIQFHWKNSNQIKILGNQ GSFLTKGPS | 93.7 | | | 170.9 | | | 48.4 | | | | |
| 717.09 | YPKFVKQNTLKAAT-NH2 | >7216.88 | | | 1666.7 | | | 606.7 | >33333.33 | | | |
| 806.01 | YQHKANSKFKGKFK-NH2 | 5000.0 | | | 8.7 | | | 2.4 | | | | |
| 768.01 | YSSFSSSSSSKSSS | | | | | | | | | | | 20833 |
| 768.02 | YSSFSSSSSSKSSS-NH2 | 347.2 | | | 20000.0 | | | 700.0 | | | 1538.5 | 342.5 |
| 768.03 | YSSFSSSSSSSSSS | | | | | | | | | | | 2777.8 |
| 768.04 | YSSFSSSSSSSSSS-NH2 | | | | | | | | | | | |
| 564.01 | YVKADYVKADYVKADYVK | >25000 | | 0 | 200000.0 | | | 1137.5 | | | | |
| 718.01 | YVKQNTLAFVKQNTLA | 12500.0 | | | 200000.0 | | | 650.0 | | | | |
| 631.01 | YVKQYTKQYTKQNTLK | 2083.3 | | | 689.7 | | | 364.0 | >14285.71 | | | >25000 |
| S1396 | ZAAFAAAATLKAAA | | | | | | | | | | | |
| S1399 | ZAAFAAAXTAYAYA | | | | | | | | | | | |
| S1400 | ZAAFAXAATAYAYA | | | | | | | | | | | |
| S5005 | ZAAFAXAATLKAAA | | | | | | | | | | | |

**Table 28a**

| Peptide | AA | Sequence | Source |
|---|---|---|---|
| 008.00 | 16 | SALLSSDITASVNCAK | HEL 81-96 |
| 200.06 | 16 | SALSEGATPQDLNTML | HIV gp25 41-56 |
| 213.10 | 16 | NKALELFRKDLAAKYK | Sp. W. myo. 132 |
| 506.01 | 20 | NKAELFRKDIAAKYKELGY | SW Myo 132-151 |
| 506.03 | 18 | ALELFRKDIAAKYKELGY | Sp. W myo. 134 |
| 506.05 | 16 | ELFRKDIAAKYKELGY | Sp. W myo. 136 |
| 570.01 | 16 | MAKTIAYDEEARRGLE | Heat Shock Prot |
| 705.06 | 20 | KVYLPRMKMEEKYNLTSVLM | Ova 279-298 |
| 717.04 | 14 | YASFVKTTTLRKFT-NH2 | DR2 combinatorial |
| 857.02 | 20 | PHHTALRQAILCWGELMTLA | HBV.core.50 |
| 865.01 | 15 | YKMKMVHAAHAKMKM | OVA KM core ext. |
| F050.03 | 20 | GFYTTGAVRQIFGDYKTTIC | PLP91-110 |
| F089.01 | 15 | QNILLSNAPLGPQFP | Tyrosinase 56 |
| F098.03 | 20 | AAYAAQGYKVLVLNPSVAAT | HCV.NS3.1242 |
| F098.04 | 20 | GYKVLVLNPSVAATLGFGAY | HCV NS3 1248 |
| F098.05 | 14 | GYKLVLVLNPSVAAT | HCV NS3 1248 |
| F098.06 | 19 | SYVNTNMGLKFRQLLWFHI | HBV Core 87 |
| F098.10 | 12 | GLKFRQLLWFHI | HBV Core 94 |
| F134.04 | 20 | TLHGPTPLLYRLGAVQNEIT | HCV NS4 1-20 |
| F134.05 | 20 | NFLSGIQYLAGLSTLPGNPA | HCV.NS4.151 |
| F134.08 | 21 | GEGAVQWMNRLIAFASRGNHV | HCV.NS4.293 |
| IA-p5 | 17 | KPVSQMRMATPLLMRPM | Mouse.Ii.85 |
| Tr-28 p1 | 24 | LPKPPKPVSKMRMATPLLMQALPM | Human.Ii.80 |
| 27.0279 | 15 | EYLVSFGVWIRTPPA | HBV.nuc.117 |
| 27.0280 | 15 | GVWIRTPPAYRPPNA | HBV.nuc.123 |
| 27.0281 | 15 | RHYLHTLWKAGILYK | HBV.pol.145 |
| 27.0283 | 15 | VPNLQSLTNLLSSNL | HBV POL 409 |
| 27.0288 | 15 | WVTVYYGVPVWKEAT | HIV1.env.47 |
| 27.0293 | 15 | YYGVPVWKEATTTLF | HW1 ENV 51 |
| 27.0294 | 15 | VPVWKBATTTLFCAS | HIV1.env.54 |
| 27.0295 | 15 | LSGIVQQQNNLLRAI | HIV1.env.711 |
| 27.0296 | 15 | QQHLLQLTVWGIKQL | HIV1 ENV 728 |
| 27.0297 | 15 | QHLLQLTVWGIKQLQ | HIV1.env.729 |
| 27.0298 | 15 | LLQLTVWGIKQLQAR | HIV1 ENV731 |
| 27.0304 | 15 | QGQMVHQAISPRTLN | HIV1.gag.171 |
| 27.0307 | 15 | SPEVIPMFSALSEGA | HIV1 GAG 197 |
| 27.0310 | 15 | QEQIGWMTNNPPIPV | HIV1 GAG 276 |
| 27.0311 | 15 | GBIYKRWIILGLNKI | HIV1.gag.294 |
| 27.0312 | 15 | YKRWIILGLNKIVRM | HIV1 GAG 297 |
| 27.0313 | 15 | KRWIILGLNKIVRMY | HIV1.gag.298 |
| 27.0314 | 15 | WIILGLNKIVRMYSP | HIV1.gag.300 |
| 27.0315 | 15 | VKNWMTETLLVQNAN | HIV1 GAG 348 |
| 27.0322 | 15 | GTVLVGPTPVNIIGR | HIV1 POL 153 |
| 27.0324 | 15 | PVNIIGRNLLTQIGC | HIV1 POL 161 |
| 27.0326 | 15 | GRNLLTQIGCTLFP | HIV1.pol.166 |
| 27.0328 | 15 | TLNFPISPIETVPVK | HIV1POL 176 |
| 27.0329 | 15 | NFPISPIETVPVKLK | HIV1.pol.178 |
| 27.0341 | 15 | FRKYTAFTIPSINNE | HIV1.pol.303 |
| 27.0344 | 15 | SPAIFQSSMTKILEP | HIV1.pol.335 |
| 27.0345 | 15 | PAIFQSSMTKILEPF | HIV1POL 336 |
| 27.0349 | 15 | QKLVGKLNWASQIYA | HIV1POL 437 |
| 27.0350 | 15 | VGKLNWASQIYAGIK | HIV1.pol.440 |
| 27.0351 | 15 | NREILKEPVHGVYYD | HIV1.pol.485 |
| 27.0353 | 15 | IPEWEFVNTPPLVKL | HIV1 POL 593 |
| 27.0354 | 15 | WEFVNTPPLVKLWYQ | HIV1.pol.596 |
| 27.0360 | 15 | EQLIKKEKVYLAWVP | HIV1POL 705 |
| 27:0361 | 15 | EKVYLAWVPAHKGIG | HIV1.pol.711 |
| 27.0364 | 15 | HSNWRAMASDFNLPP | HIV1.pol.758 |
| 27.0370 | 15 | ASGYIEAEVIPAETG | HIV1 POL 822 |
| 27.0372 | 15 | AEHLKTAVQMAVFIH | HIV1.pol.911 |
| 27.0373 | 15 | KTAVQMAVFIHNFKR | HIV1.pol.915 |
| 27.0377 | 15 | QKQITKIQNFRVYYR | HIV1.pol.956 |
| 27.0379 | 15 | KLLWKGEGAVVIQDN | HTV1.pol.982 |
| 27.0381 | 15 | ENRWQVMIVWQVDRM | HIV1.vif.2 |
| 27.0382 | 15 | VBABRILQQLLFIH | HIV1 VPR 57 |
| 27.0384 | 15 | FNVVNSSIGLIMVLS | Pf CSP 413 |
| 27.0387 | 15 | MNYYGKQENWYSLKK | Pf CSP 53 |
| 27.0388 | 15 | MRKLAILSVSSFLFV | Pf.CSP.2 |
| 27.0390 | 15 | NSSIGLIMVLSFLFL | Pf CSP 417 |
| 27.0392 | 15 | SSVFNVVNSSIGLIM | Pf.CSP.410 |
| 27.0393 | 15 | MKILSVFFLALFFII | Pf EXP1 1 |
| 27.0398 | 15 | FILVNLLIFHHINGICI | Pf LSA1 11 |
| 27.0400 | 15 | HILYISFYFILVNLL | Pf LSA13 |
| 27.0402 | 15 | LLIFHINGKIIKNSE | Pf LSA1 16 |
| 27.0403 | 15 | LVNLLIFHINGKIIK | Pf LSA1 13 |
| 27.0406 | 15 | NLUFHINGKIIKNS | Pf LSA1 15 |
| 27.0408 | 15 | QTNFKSLLRNLGVSE | Pf LSA1 94 |
| 27.0412 | 15 | AYKFVVPGAATPYAG | Pf SSP2514 |
| 27.0415 | 15 | NVKYLVIVFLIFFDL | Pf SSP2 6 |
| 27.0417 | 15 | VKNVIGPFMKAVCVE | Pf.SSP2.223 |
| 27.0418 | 15 | WENVKNVIGPFMKAV | PfSSP2220 |
| 1186.04 | 15 | CSVVRRAFPHCLAFS | HBV.pol.534. |
| 1186.06 | 15 | FVQWFVGLSPTVWLS | HBV ENV 342 |
| 1186.10 | 15 | LAQFTSAICSVVRRA | HBV.pol.526 |
| 1186.15 | 15 | LVPFVQWFVGLSPTV | HBV.env.339 |
| 1186.18 | 15 | NLSWLSLDVSAAFYH | HBV POL 422 |
| 1186.25 | 15 | SFGVWIRTPPAYRPP | HBV.nuc.121 |
| 1186.26 | 15 | SPFLLAQFTSAICSV | HBV.pol.522 |
| 1186.27 | 15 | SSNLSWLSLDVSAAF | HBV POL 420 |
| 1188.01 | 15 | DKELTMSNVKNVSQT | Pf LSA1 81 |
| 1188.13 | 15 | AGLLGNVSTVLLGGV | Pf.EXP1.82 |
| 1188.16 | 15 | KSKYKLATSVLAGLL | Pf.EXP1.71 |
| 1188.32 | 15 | GLAYKFVVPGAATPY | Pf.SSP2.512 |
| 1188.34 | 15 | HNWVNHAVPLAMKLI | Pf.SSP2.62 |
| 1188.35 | 15 | IGPFMKAVCVEVEKT | Pf SSP2 227 |
| 1188.38 | 15 | KYKIAGGIAGGLALL | Pf.SSP2.494 |
| 1188.45 | 15 | RHNWVNHAVPLAMKL | Pf SSP2 61 |
| F091.15 | 16 | IKQFINMWQEVGKAMY | HIV1.env.566 |
| F107.03 | 15 | LQSLTNLLSSNLSWL | HBV.pol.412 |
| F107.04 | 15 | PFLLAQFTSAICSVV | HBV.pol.523 |
| F107.09 | 15 | KYKLATSVLAGLLGN | Pf EXP1 73 |
| F107.10 | 15 | LAGLLGNVSTVLLGG | Pf EXP1 81 |
| F107.11 | 15 | RHPFKIGSSDPADNA | Pf EXP1 107 |
| F107.14 | 15 | ANQLVVILTDGIPDS | PfSSP2153 |
| F107.17 | 15 | KFVVPGAATPYAGEP | Pf SSP2 516 |
| F107.23 | 15 | VFNVVNSSIGLIMVL | Pf CSP 412 |
| 35.0093 | 15 | VGPLTVNEKRRLKLI | HBV.pol.96 |
| 35.0096 | 15 | ESRLVVDFSQFSRGN | HBV.pol.387 |
| 35.0100 | 15 | LCQVFADATPTGWGL | HBV.pol.683 |
| 35.0106 | 15 | VVVVATDALMTGYTG | HCV.1437 |
| 35.0107 | 15 | TVDFSLDPTFTIETT | HCV.1466 |
| 35.0125 | 15 | AETFYVDGAANRETK | HIV.pol.619 |
| 35.0127 | 15 | EVNIVTDSQYALGII | HIV.pol.674 |
| 35.0131 | 15 | WAGIKQEFGIPYNPQ | HIV.pol.874 |
| 35.0133 | 15 | GAVVIQDNSDIKVVP | HIV.pol.989 |
| 35.0135 | 15 | YRKIRQLRQRKIDRLID | HIV.vpu.31 |
| 35.0171 | 15 | PDSIQDSLKESRKLN | Pf.SSP2.165 |
| 35.0172 | 15 | KCNLYADSAWENVKN | Pf.SSP2.211 |
| 1280.02 | 15 | IGTVLVGPTPVNIIG | HIV.pol.152 |
| 1280.03 | 15 | KVYLAWVPAHKGIGG | HIV.pol.712 |
| 1280.04 | 15 | TKELQKQITKIQNFR | HIV.pol.952 |
| 1280.06 | 15 | AGFFLLTRILTIPQS | HBV.env.180 |
| 1280.08 | 15 | GFFLLTRILTIPQSL | HBV ENV 181 |
| 1280.09 | 15 | GTSFVYVPSALNPAD | HBV.pol.774 |
| 1280.12 | 15 | IIFLFILLLCLIFLL | HBV ENV 244 |
| 1280.13 | 15 | KFAVPNLQSLTNLLS | HBV POL 406 |
| 1280.15 | 15 | LHLYSHPIILGFRKI | HBV.pol.501 |
| 1280.16 | 15 | LLCLIFLLVLLDYQG | HBV ENV 251 |
| 1280.21 | 15 | VGLLGFAAPFTQCGY | HBV POL 637 |
| 1280.22 | 15 | FYFILVNLLIFHING | Pf LSA19 |
| 1280.23 | 15 | KSLLRNLGVSENlFL | Pf.LSA1.98 |
| 1280.25 | 15 | RGYYIPHQSSLPQDN | Pf.LSA1.1669 |
| 1283.02 | 15 | VYLLPRRGPRLGVRA | HCV Core 34 |
| 1283.10 | 15 | GHRMAWDMMMNWSPT | HCV E1 315 |
| 1283.11 | 15 | CGPVYCFTPSPVVVG | HCV.NS1/E2.506 |
| 1283.12 | 15 | VYCFTPSPVVVGTTD | HCV NS1/E2 509 |
| 1283.13 | 15 | GNWTGCTWMNSTGFT | HCV.NS1/E2.550 |
| 1283.14 | 15 | FTTLPALSTGLIHLH | HCV NS1/E2 684 |
| 1283.16 | 15 | SKGWRLLAPITAYAQ | HCV.NS3.1025 |
| 1283.17 | 15 | DLYLVTRHADVIPVR | HCV NS3 1134 |
| 1283.20 | 15 | AQGYKVLVLNPSVAA | HCV.NS3.1251 |
| 1283.21 | 15 | GYKVLVLNPSVAATL | HCV.NS3.1253 |
| 1283.22 | 15 | VLVLNPSVAATLGFG | HCV.NS3.1256 |
| 1283.24 | 15 | GARLVVLATATPPGS | HCV NS3 1345 |
| 1283.26 | 15 | DVVVVATDALMTGYT | HCV.NS3.1436 |
| 1283.30 | 15 | FTGLTHIDAHFLSQT | HCV.NS3.1567 |
| 1283.31 | 15 | YLVAYQATVCARAQA | HCV.NS3.1591 |
| 1283.33 | 15 | LEVVTSTWVLVGGVL | HCV NS4 1658 |
| 1283.34 | 15 | TWVLVGGVLAALAAY | HCV NS4 1664 |
| 1283.36 | 15 | AKHMWNFISGIQYLA | HCV.NS4.1767 |
| 1283.37 | 15 | IQYLAGLSTLPGNPA | HCV NS41777 |
| 1283.44 | 15 | MNRLIAFASRGNHVS | HCV.NS4.1921 |
| 1283.50 | 15 | SYTWTGALITPCAAE | HCV.NS5.2456 |
| 1283.55 | 15 | GSSYGFQYSPGQRVE | HCV.NS5.2641 |
| 1283.57 | 15 | LELITSCSSNVSVAH | HCV.NS5.2813 |
| 1283.61 | 15 | ASCLRKLGVPPLRVW | HCV.NS5.2939 |
| 1298.02 | 15 | VGNFTGLYSSTVPVF | HBV.pol.53 |
| 1298.03 | 15 | TNFLLSLGIHLNPNK | HBV.pol.568 |
| 1298.04 | 15 | KQCFRKLPVNRPIDW | HBV.pol.615 |
| 1298.06 | 15 | KQAFTFSPTYKAFLC | HBV.pol.661 |
| 1298.07 | 15 | AANWILRGTSFVYVP | HBV.pol.764 |
| 1298.08 | 15 | PDRVHFASPLHYAWR | HBV.pol.824 |
| 1298.10 | 15 | IRPWSTQLLLNGSL | WVl,env.333 |
| 1298.11 | 15 | RSELYKYKVVKIEPL | HIV1.env.637 |
| 1298.13 | 15 | DRFYKTLRAEQASQE | HIV1 GAG 333 |
| 1298.16 | 15 | KVILVAVHVASGYIE | HIV1.pol.813 |
| F125.02 | 17 | LVNLLIFHHINGKIIKNS | Pf.LSA1.13 |
| F125.04 | 16 | RHNWVNHAVPLAMKLI | Pf.SSP2.61 |

| | | | |
|---|---|---|---|
| -- indicates binding affinity >15,000nM. | | | |

**Table 28b**

| **IC₅₀ nM** | | | | | | | | | | | | | | | |
|---|---|---|---|---|---|---|---|---|---|---|---|---|---|---|---|
| Sequence | DR1 | DR2 w2B1 | DR2w 2B2 | DR3 | DR4 w4 | DR4 w15 | DR5 w11 | DR5 w12 | DR6 w19 | DR7 | DR8 w2 | DR52a | DR9 | DR w53 | Cross-reactivity |
| SALLSSDITASVNCAK | >1667 | | >5000 | 244 | 84 | | >10000 | | | >8333 | | 9592 | | | 2 |
| SALSEGATPQDLNTML | 417 | | 833 | 587 | - | | 323 | | | - | | - | | | 4 |
| NKALELFRKDIAAKYK | | | 1667 | 233 | 529 | | 182 | | | | | 261 | | | 4 |
| NKALELFRKDIAAKYKELGY | >1250 | | 1429 | 240 | 306 | | 42 | | | - | | 392 | | | 4 |
| ALELFRKDIAAKYKELGY | | | 1818 | 150 | 237 | | 364 | | | | | 336 | | | 4 |
| ELFRKDIAAKYKELGY | | | 1667 | 297 | 300 | | 125 | | | | | 470 | | | 4 |
| MAKTIAYDEEARRGLE | - | | >5000 | 283 | - | | - | | | - | | | | | 1 |
| KVYLPRMKMEEKYNLTSVLM | 1000 | | 3333 | 335 | >11250 | | 1053 | | | 714 | | - | | | 3 |
| YASFVKTTTLRKFT-NH2 | 500 | | 3.6 | 178 | 12 | | 22 | | | 31 | | - | | | 6 |
| PHHTALRQAILCWGELMTLA | 70 | 8.3 | 211 | - | 85 | | 263 | - | - | 676 | 196 | 2701 | | | 7 |
| YKMKMVHAAHAKMKM | 3.6 | | 8.7 | 208 | 92 | | 45 | | | 62 | | | | | 6 |
| GFYTTGAVRQIFGDYKTTIC | | | | 91 | 346 | | - | | | | | 7833 | | 48 | 3 |
| QNILLSNAPLGPQFP | | | | | 225 | | | | | | | | | | 1 |
| AAYAAQGYKVLVLNPSVAAT | 29 | 48 | 488 | 1765 | 17 | 1226 | 105 | 3239 | 11 | 96 | 61 | 6528 | 242 | 77 | 10 |
| GYKVLVLNPSVAATLGFGAY | 3.6 | 41 | 8000 | | 10 | 1520 | 241 | 9613 | 4.1 | 23 | 80 | | 20 | | 8 |
| GYKVLVLNPSVAAT | 1.4 | 38 | 3704 | | 8 | 141 | 74 | 4585 | 3.5 | 125 | 21 | | 268 | | 9 |
| SYVNTNMGLKFRQLLWFHI | 417 | 123 | 2899 | | 6618 | 6441 | 41 | 1987 | 13 | 833 | 377 | . | 9259 | | 6 |
| GLKFRQLLWFHI | 179 | 34 | 1053 | | 4091 | 1027 | 32 | 5960 | 1591 | 641 | 123 | | 4688 | | 5 |
| TLHGPTPLLYRLGAVQNEIT | 17 | | | | 1286 | | 690 | | | 2273 | | | | | 2 |
| NFISGIQYLAGISTLPGNPA | 10 | | 606 | | 85 | | 74 | | | - | 70 | | | | 5 |
| GEGAVQWMNRLLAFASRGNHV | 3.3 | 29 | 182 | - | 375 | | 14 | - | 26 | 227 | 158 | | | | 8 |
| KPVSQMRMATPLLMRPM | 1.2 | | 15 | 52 | 49 | 88 | 111 | | | 25 | 169 | - | | 13 | 9 |
| LPKPPKPVSKMRMATPLLMQA LPM | 0.90 | 14 | 31 | 79 | 141 | 200 | 444 | 392 | 0.70 | 40 | 258 | - | 54 | 8 | 13 |
| EYLVSFGVWIRTPPA | 8.8 | 15 | 435 | - | 4091 | 792 | 1818 | - | 2333 | 58 | 6901 | | 87 | | 6 |
| GVWIRTPPAYRPPNA | 14 | 217 | 2.8 | - | 13 | 67 | 42 | - | >8750 | 114 | 93 | - | 1667 | 13488 | 8 |
| RHYLHTLWKAGILYK | 17 | 5.4 | 35 | - | 2250 | 1462 | 42 | 149 | 745 | 61 | 27 | 11191 | 174 | 784 | 10 |
| VPNLQSLTNLLSSNL | 83 | 3033 | - | - | 237 | | 1250 | 10643 | 5000 | - | 4083 | | - | | 2 |
| WVTVYYGVPVWKEAT | 63 | 2 | 286 | - | 2500 | 4.6 | - | - | >8750 | 807 | 10000 | | 163 | | 6 |
| YYGVPVWKEATTTLF | 455 | - | 7407 | - | 1452 | | 13333 | - | >8750 | 109 | 13243 | | 197 | | 3 |
| VPVWKEATTTLFCAS | 192 | - | 385 | - | 409 | 422 | 9524 | - | 8750 | 397 | 5698 | | 160 | | 6 |
| LSGIVQQQNNLLRAI | 54 | 57 | 61 | 10345 | 300 | 2000 | - | - | 46 | - | - | | - | | 5 |
| QQHLLQLTVWGIKQL | 4.5 | 12 | 345 | - | 1364 | 141 | 5556 | 608 | 194 | 64 | 2333 | | 147 | | 8 |
| QHLLQLTVWGIKQLQ | 6.1 | 21 | 690 | - | 1184 | 346 | 2128 | 1064 | 350 | 44 | 907 | - | 375 | 414 | 9 |
| LLQLTVWGIKQLQAR | 60 | | 2083 | | 2368 | | 267 | | | 1389 | | | | | 2 |
| QGQMVHQAISPRTLN | 73 | 65 | 13 | - | 54 | 400 | - | - | 412 | 455 | 7313 | - | 117 | 135 | 9 |
| SPEVIPMFSALSEGA | 109 | 325 | 5882 | - | 281 | | 2667 | - | 5000 | - | 3769 | | 5769 | | 3 |
| QEQIGWMTNNPPIPV | 714 | 4550 | 8333 | 13044 | 38 | | - | - | 1.0 | 1923 | - | | - | | 3 |
| GEIYKRWIILGLNKI | 82 | 138 | 225 | - | 1500 | 380 | 213 | 1656 | 98 | 192 | 63 | 1205 | 536 | 161 | 10 |
| YKRWIILGLNKIVRM | 4.2 | 5.7 | 26 | 273 | 608 | 158 | 65 | 199 | 0.90 | 208 | 91 | | 121 | | 12 |
| KRWIILGLNKIVRMY | 4.2 | 5.1 | 24 | 188 | 570 | 404 | 54 | 347 | 0.40 | 379 | 49 | 1808 | 58 | 187 | 13 |
| WIILGLNKIVRMYSP | 125 | 28 | 182 | 273 | 1452 | 1310 | 54 | 1242 | 1.9 | 2841 | 175 | | - | | 7 |
| VKNWMTETULVQNAN | 1515 | 700 | 2597 | - | 938 | | - | - | 1094 | 893 | - | | - | | 3 |
| GTVLVGPTPVNIIGR | 758 | 1492 | >14285 | - | - | | 4651 | - | 121 | 305 | - | | 4167 | | 3 |
| PVNIIGRNLLTQIGC | 848 | | >14285 | | 10465 | | 202 | | | - | | | | | 2 |
| GRNLLTQIGCTLNFP | 25 | 182 | >14285 | - | 500 | >8261 | 3030 | - | 27 | 4167 | - | | - | | 4 |
| TLNFPISPIETVPVK | 263 | - | >14285 | - | 1286 | | - | 8054 | 233 | 391 | - | | - | | 3 |
| NFPISPIETVPVKLK | 104 | 7000 | 9091 | - | 556 | 4000 | - | 6478 | 184 | 167 | - | | - | | 4 |
| FRKYTAFTIPSINNE | 185 | 70 | 4167 | - | 265 | 136 | 1818 | - | >8750 | 30 | 803 | - | 40 | - | 7 |
| SPAIFQSSMTKILEP | 357 | 217 | 667 | - | 3214 | 109 | 741 | - | 13 | 68 | 3267 | 887 | 33 | 14500 | 9 |
| PAIFQSSMTKILEPF | 156 | 284 | 1000 | - | 7759 | 59 | 303 | - | 32 | 34 | 3500 | | 82 | | 8 |
| QKLVGKLNWASQIYA | 128 | 989 | 3636 | 8108 | 10227 | >8261 | 5714 | 2709 | 2.3 | 510 | 223 | | 375 | | 6 |
| VGKLNWASQIYAGIK | 357 | 178 | 13 | - | 7258 | >8261 | 4348 | 1064 | 2.2 | 481 | 5104 | | 68 | | 6 |
| NREILKEPVHGVYYD | 17 | 61 | >14285 | 2500 | 237 | 1267 | - | 12957 | 292 | 7576 | - | | 3571 | | 4 |
| IPEWEFVNTPPLVKL | 4.5 | 1023 | 11 | 3261 | 68 | 24 | 241 | 5519 | 152 | 18 | 189 | | 29 | | 9 |
| WEFVNTPPLVKLWYQ | 7.2 | 222 | 2.1 | 13636 | 25 | 20 | 318 | 1355 | 90 | 15 | 350 | 8546 | 40 | 527 | 11 |
| EQLIKKEKVYLAWVP | 7.8 | 114 | 3390 | 13636 | 11 | | 3448 | - | >116 67 | - | - | | - | | 3 |
| EKVYLAWVPAHKGIG | 3.6 | 21 | 4.9 | 3226 | 8.3 | 27 | 37 | 6478 | 3500 | 18 | 31 | - | 144 | 14 | 10 |
| HSNWRAMASDFNLPP | 33 | - | 125 | - | 9.6 | 15 | 95 | - | 4375 | 472 | 1960 | - | 872 | 951 | 8 |
| ASGYIEAEVIPAETG | 217 | - | >9523 | - | 196 | | 10000 | - | 5833 | 2083 | 5052 | | 1563 | | 2 |
| AEHLKTAVQMAVFIH | 200 | 4333 | 6452 | - | 3462 | 224 | - | 7268 | 66 | 403 | 9608 | | 142 | | 5 |
| KTAVQMAVFIHNFKR | 161 | 650 | 690 | - | 818 | 452 | 182 | - | 125 | 1786 | 1441 | 14688 | 2586 | 1000 | 8 |
| QKQITKIQNFRVYYR | 2.9 | 3.4 | 80 | - | 321 | 49 | 53 | 124 | 25 | 25 | 75 | - | 577 | 611 | 12 |
| KLLWKGEGAVVIQDN | 11 | 8273 | 3448 | - | 600 | 1900 | 3333 | - | 78 | 104 | 1089 | | 357 | | 5 |
| ENRWQVMIVWQVDRM | 2 | 1542 | 5556 | - | 652 | 20 | 6250 | - | 1944 | 208 | 327 | | 259 | | 6 |
| VEAIIRILQQLLFIH | 147 | | 2667 | | 1216 | | 556 | | | 1136 | | | | | 2 |
| FNVVNSSIGLIMVLS | 66 | 350 | >9523 | - | 14063 | >1407 4 | - | - | 65 | 281 | - | | 469 | | 5 |
| MNYYGKQENWYSLKK | 6.4 | 9100 | 435 | - | 21 | 292 | 351 | - | 3182 | 3788 | 539 | | - | | 6 |
| MRKLAILSVSSFLFV | 50 | 18 | 1539 | - | 5769 | 1407 | 541 | 2483 | 38 | 500 | - | | 682 | | 6 |
| NSSIGLIMVLSFLFL | 1250 | 364 | 8333 | - | 3750 | 10857 | - | 8765 | 3889 | 362 | - | | - | | 2 |
| SSVFNVVNSSIGLIM | 42 | 314 | 2500 | - | 450 | 1652 | 1177 | - | 10 | 33 | 891 | | 63 | | 7 |
| MKILSVFFLALFFII | 2941 | 535 | >9523 | - | 1216 | >8261 | >2000 0 | - | 1842 | 694 | - | | 12500 | | 2 |
| FILVNLLIFHINGKI | 1389 | 700 | 13333 | - | 1406 | >8085 | 2632 | - | 1029 | 521 | 11667 | | 6818 | | 2 |
| HILYISFYFILVNLL | 3125 | 827 | - | - | 1667 | >12667 | 18182 | - | 2500 | 357 | - | | - | | 2 |
| LLIFHINGKIIKNSE | 8.8 | 31 | 80 | 682 | 7500 | >12667 | 56 | 12957 | 106 | 192 | 350 | | 500 | | 9 |
| LVNLLIFHINGKIIK | 78 | 13 | 2857 | - | 4091 | >12667 | 74 | 7268 | 66 | 208 | 1690 | | 417. | | 6 |
| NLLIFHINGKIIKNS | 3.6 | 6.1 | 71 | 1200 | 5769 | 2375 | 83 | 6082 | 19 | 49 | 153 | | 278 | | 8 |
| QTNFKSLLRNLGVSE | 91 | 8273 | 5405 | - | 2500 | 1900 | 51 | - | 47 | 7813 | 69 | | - | | 4 |
| AYKFVVPGAATPYAG | 14 | >10111 | 323 | 18750 | 281 | 10556 | 31 | 2483 | 21 | 139 | 53 | | 577 | | 8 |
| NVKYLVIVFLIFFDL | 2000 | 650 | >6897 | - | 750 | >12667 | 6667 | - | 2188 | 714 | 9074 | | - | | 3 |
| VKNVIGPFMKAVCVE | 56 | 212 | 250 | - | - | >12667 | 476 | 3239 | 32 | 424 | 2130 | - | 862 | 5859 | 7 |
| WENVKNVIGPFMKAV | 79 | 54 | 290 | - | - | 8636 | - | - | 4375 | 1563 | - | | 9375 | | 3 |
| CSVVRRAPPHCLAFS | 50 | 89 | 260 | - | - | >12667 | - | - | 65 | 424 | 1960 | | 63 | 1261 | 6 |
| FVQWFVGLSPTVWLS | 11 | 2600 | 1250 | - | 3462 | | 2778 - | | 184 | 362 | 2722 | | 1829 | - | 3 |
| LAQFTSAICSVVRRA | 417 | 1400 | 133 | - | 3000 | 1357 | 2632 | - | 3500 | 893 | 3267 | | 852 | 3053 | 4 |
| LVPFVQWFVGLSPTV | 385 | 13 | 1429 - | | 300 | 27 | 53 | 452 | 1944 | 2717 | 74 | 1679 | 30 | 20 | 9 |
| NLSWLSLDVSAAFYH | 36 | - | - | 395 | 155 | | 6061 | 14900 | 106 | 6098 | 3267 | 63 | 1210 | 7.4 | 6 |
| SFGVWIRTPPAYRPP | 532 | 827 | 47 | - | 577 | 603 | 769 | - | - | 1042 | 196 | - | 938 | - | 8 |
| SPFLLAQFTSAICSV | 42 | 455 | 2353 | - | 608 | 2000 | - | - | 65 | 758 | - | | 1974 | 290 | 6 |
| SSNLSWLSLDVSAAF | 36 | 3033 | - | 200 | 167 | | 4348 | 8514 | 35 | 6410 | 1065 | 22 | 6818 | 3.2 | 6 |
| DKELTMSNVKNVSQT | 2778 | - | - | - | - | 5135 | 6667 | - | 81 | 610 | 4455 | | 1056 | - | 2 |
| AGLLGNVSTVLLGGV | 116 | 379 | - | - | 6923 | 1056 | -- | - | 0.80 | 58 | - | | 142 | - | 5 |
| KSKYKLATSVLAGLL | 3.6 | 1247 | 24 | - | 7.1 | 47 | 30 | - | 427 | 13 | 45 | 1567 | 28 | 4143 | 9 |
| GLAYKFVVPGAATPY | 3.1 | - | 29 | - | 45 | 1407 | 11 | 851 | 7.1 | 167 | 20 | 47000 | 125 | 3053 | 9 |
| HNWVNHAVPLAMKLI | 14 | 364 | 143 | 1304 | 12 | 950 | 2703 | 497 | 3.7 | 66 | 68 | - | 19 | 3053 | 10 |
| IGPFMKAVCVEVEKT | 694 | - | 465 | - | - | 4419 | - | - | 11667 | 13158 | - | | 915 | 829 | 4 |
| KYKIAGGIAGGLALL | 132 | - | 417 | 12000 | 3750 | - | 87 | 828 | 15 | 3968 | 31 | | 289 | - | 7 |
| RHNWVNHAVPLAMKL | 26 | 260 | 125 | 750 | 9.0 | 1056 | 2532 | 12417 | 3.9 | 57 | 27 | | 16 | 1349 | 9 |
| IKQFINMWQEVGKAMY | 128 | 217 | 206 | - | 375 | 271 | 4878 | - | 1000 | - | 350 | - | 5769 | 112 | 8 |
| LQSLTNLISSNLSWL | 2.0 | 21 | 1000 | - | 9.4 | 47 | 294 | 397 | 135 | 167 | 557 | - | 682 | 1813 | 11 |
| PFLLAQFTSAICSVV | 28 | 337 | 4762 | - | 563 | 317 | 1667 | - | 44 | 325 | 845 | - | 1271 | 1349 | 7 |
| KYKLATSVLAGLLGN | 4.2 | 5056 | 286 | -- | 14 | 317 | 952 | - | 467 | 39 | 377 | | 29 | | 9 |
| LAGLLGNVSTVLLGG | 119 | 827 | - | 38462 | 2813 | 1652 | - | - | 5.5 | 66 | - | | 136 | | 5 |
| RHPFKIGSSDPADNA | 1136 | - | - | - | 96 | 13103 | 3571 | - | 11667 - | | 1290 | | 790 | | 2 |
| ANQLVVILTDGIPDS | 1316 | 11375 | - | - | 978 | 6129 | - | - | 500 | 4630 | - | | 1786 | | 2 |
| KFVVPGAATPYAGEP | 217 | - | - | 4839 | 375 | 5672 | 20000 | 3465 | 76 | 14706 | 7656 | | 300 | | 4 |
| VFNVVNSSIGLIMVL | 161 | 4333 | - | - | 5696 | 6786 - | | - | 14 | 139 | - | | 150 | | 4 |
| VGPLTVNEKRRLKLI | 8333 | 4136 | 4255 | 136 | >8182 | | 6667 | - | 3889 | - | 5326 | >3615 | | 8169 | 1 |
| ESRLVVDFSQFSRGN | 7143 | 1230 | - | 115 | 188 | | - | - | 875 | - | - | 2474 | | 8657 | 3 |
| LCQVFADATPTGWGL | 7143 | - | - | 67 | 489 | | - | - | 1944 | - | - | 1808 | | 1036 | 2 |
| VVVVATDALMTGYTG | 1042 | 1936 | 14286 | 273 | >8182 | | - | - | 1207 | 625 | - | >3615 | | 817 | 3 |
| TVDFSLDPTFTIETT | 5556 | - | >10000 | 150 | 1957 | | - | - | 169 | 8333 | >30625 | 24 | | 3625 | 3 |
| AETFYVDGAANRETK | 2381 | - | 4348 | 769 | >8182 | | - | - | - | - | 14000 | 1068 | | - | 1 |
| EVNIVTDSQYALGII | - | 2677 | - | 732 | 7627 | | - | - | 324 | - | - | 118 | | - | 3 |
| WAGIKQEFGIPYNPQ | 2778 | 5056 | 125 | 300 | >10000 | | - | 805 | 285 | - | - | >3615 | | 951 | 5 |
| GAVVIQDNSDIKVVP | 1515 | 325 | 14286 | 1000 | - | | - | - | 78 | - | - | >3615 | | 725 | 4 |
| YRKILRQRKIDRLID | 2083 | 123 | 488 | 23 | >10000 | | 133 | 4257 | 2188 | - | 1815 | >3615 | | 36 | 5 |
| PDSIQDSLKESRKLN | - | 2275 | >11111 | 357 | >10000 | | - | - | - | - | - | >3615 | | - | 1 |
| KCNLYADSAWENVKN | - | - | - | 857 | >10000 | | - | - | 11667 | - | - | - | | - | 1 |
| IGTVLVGPTPVNIIG | 417 | 535 | - | | 56250 | 12667 | - | | 88 | 352 | - | | 2344 | | 4 |
| KVYLAWVPAHKGIGG | 8.3 | 25 | 24 | - | 141 | 165 | 71 | 12417 | 2500 | 179 | 196 | - | 250 | 290 | 10 |
| TKELQKQITKIQNFR | 704 | 433 | 571 | | 833 | 1900 | 377 | | 700 | 4546 | 1960 | | - | | 6 |
| AGFFLLTRILTIPQS | 1.1 | 217 | 1053 - | | 8.5 | 253 | 6 | 4257 | 10 | 8 | 189 | 4273 | 58 | 699 | 10 |
| GFFLLTRILT1PQSL | 12 | 607 | 1818 | | 15 | 84 | 9 | | 45 | 7 | 31 | | 136 | | 9 |
| GTSFVYVPSALNPAD | 14 | 650 | 400 | - | 118 | 93 | 426 | - | 35000 | 93 | 803 | - | 221 | - | 9 |
| IIFLFILLLCLIFLL | | | | | | | | | | | | | | | 0 |
| KFAVPNLQSLTNLLS | 278 | - | - | | 346 | | 4651 | | 398 | - | | | - | | 3 |
| LHLYSHPIILGFRKI | 227 | 268 | 500 | - | 66 | 238 | 488 | 9613 | 17500 | 41667 | 803 | - | 1531 | 102 | 8 |
| LLCLIFLLVLLDYQG | | | | | | | | | | | | | | | 0 |
| VGLLGFAAPFTQCGY | 106 | 29 | - | | - | | - | | - | 17857 | | | 132 | | 3 |
| FYFILVNLLIFHING | | | | | | | | | | | | | | | 0 |
| KSLLRNLGVSENIFL | 333 | 1034 | - | | 2143 | 469 | 6061 | | 6.1 | 325 | - | | 47 | | 5 |
| RGYYIPHQSSLPQDN | 17 | - | 6897 | | 11 | 136 | 3125 | | 8750 | 147 | 3267 | | 500 | | 5 |
| VYLLPRRGPRLGVRA | 357 | 23 | 556 | | - | | 1667 | | - | - | | | - | | 3 |
| GHRMAWDMMMNWSPT | 179 | 6067 | 4546 | | 281 | | 2532 | | 438 | 14706 | | | 3261 | | 3 |
| CGPVYCFTPSPVVVG | 19 | 3640 | - | | 173 | 95 | - | | - | 93 | - | | 174 | | 5 |
| VYCFTPSPVVVGTTD | 278 | - | - | | 489 | 667 | 3571 | | - | 338 | 14000 | | 417 | | 5 |
| GNWFGCTWMNSTGFT | 139 | 284 | - | | 107 | 1520 | 952 | | - | 1000 | 14000 | | 2778 | | 5 |
| FTTLPALSTGLIHLH | 1.2 | 2528 | 12500 | | 6338 | | 1539 | | - | 625 | | | 2419 | | 2 |
| SKGWRLLAPITAYAQ | 0.40 | 125 | 23 | - | 21 | 152 | 5 | 10276 | - | 962 | 54 | - | 1191 | 527 | 9 |
| DLYLVTRHADVIPVR | 617 | 414 | 18182 | | - | | 2632 | | 7000 | 309 | | | 1210 | | 3 |
| AQGYKVLVLNPSVAA | 6.0 | 650 | - | 10000 | 7.1 | 224 | 74 | 2980 | 5.9 | 833 | 175 | - | 375 | 276 | 10 |
| GYKVLVLNPSVAATL | 4.5 | 350 | - | 3061 | 4.7 | 567 | 143 | 5731 | 5.1 | 89 | 288 | - | 54 | 1933 | 9 |
| VLVLNPSVAATLGFG | 2.8 | 758 | - | | 21 | 10857 | 1429 | | 11 | 17 | - | | 23 | | 6 |
| GARLVVLATATPPGS | 167 | 10111 | - | | 51 | | 2128 | | 8750 | 568 | | | 11194 | | 3 |
| DVVVVATDALMTGYT | 455 | 827 | - | | 2500 | 5278 | - | | 250 | 275 | - | | - | | 4 |
| FTGLTHIDAHFLSQT | 6.6 | 15 | 154 | | - | 12667 | 2410 | | - | 179 | 980 | | 13393 | | 5 |
| YLVAYQATVCARAQA | 6.8 | 379 | 571 | | 80 | 1357 | 1418 | | 1207 | 2778 | 7903 | | 278 | | 5 |
| LEVVTSTWVLVGGVL | 417 | 1167 | - | | 1607 | | - | | 761 | 156 | | | 6250 | | 3 |
| TWVLVGGVLAALAAY | 6.5 | 8273 | - | | - | | - | | - | 439 | | | 12931 | | 2 |
| AKHMWNFISGIQYLA | 33 | 607 | 351 | | 11250 | 633 | 2632 | | 8750 | 109 | 3063 | | 278 | | 6 |
| IQYLAGLSTLPGNPA | 1.4 | 212 | 2128 | | 12 | | 12 | | - | 11905 | | | 1364 | | 4 |
| MNRLLAFASRGNHVS | 66 | 5 | 1539 | 51724 | 5696 | 585 | 46 | 14191 | 7.3 | 227 | 102 | - | 313 | 6374 | 8 |
| SYTWTGALITPCAAE | 192 | 13000 | 13333 | | 662 | 1727 | 6452 | | - | 51 | 3769 | | 1000 | | 4 |
| GSSYGFQYSPGQRVE | 11 | - | 667 | - | 375 | 745 | - | - | 19 | 156 | - | 6620 | 68 | - | 7 |
| LELITSCSSNVSVAH | 204 | 455 | - | | 517 | 1086 | - | | 69 | 714 | - | | 2273 | | 5 |
| ASCLRKLGVPPLRVW | 5.0 | 16 | 217 | - | 5625 | 78 | 645 | 157 | 2500 | 862 | 671 | - | 8621 | 5273 | 8 |
| VGNFTGLYSSTVPVF | 2.9 | 910 | 12500 | | 3214 | 224 | 5714 | | 60 | 45 | 11136 | | 242 | | 6 |
| TNFLLSLGIHLNPNK | 1.4 | 222 | 167 | | 2046 | 1056 | 3774 | | 219 | | 114 - | | 197 | | 6 |
| KQCFRKLPVNRPIDW | 3.3 | 4136 | 952 | - | 38 | 45 | 1539 | - | 814 | 63 | 845 | - | 3000 | 3053 | 7 |
| KQAFTFSPTYKAFLC | 9.4 | 38 | 143 | - | 41 | 173 | 83 | - | 175 | 76 | 408 | 4845 | 139 | 322 | 11 |
| AANWILRGTSFVYVP | 54 | 379 | 3279 | - | 882 | 1520 | 1429 | 805 | 140 | 43 | 196 | - | 278 | 6517 | 8 |
| PDRVHFASPLHVAWR | 98 | 314 | - | | - | 7037 | - | | 184 | 309 | 14000 | | 313 | | 5 |
| IRPVVSTQLLLNGSL | 568 | 233 | - | | 1875 | 4750 | - | | 71 | 74 | - | | 5769 | | 4 |
| RSELYKYKVVKIEPL | 758 | 284 | 14286 | | - | 2000 | 4762 | | 350 | 139 | 446 | | 441 | | 6 |
| DRFYKTLRAEQASQE | 94 | 5688 | 400 | | 300 | | 465 | | - | 8929 | | | - | | 4 |
| KVILVAVHVASGYIE | 7.7 | 4333 | - | | 455 | 2923 | 2174 | | 714 | 156 | 2130 | | 1667 | | 4 |
| LVNLLIFHINGKIIKNS | 7.6 | 17 | 182 | 20000 | | 1810 | 357 | 11462 | 200 | | 446 | | | | 6 |
| RHNWVNHAVPLAMKLI | 3.6 | 260 | 23 | 8824 | | 317 | 1333 | 438 | 36 | | 70 | | | | 7 |

**Table 29a**

| No. amino acids | Sequence | Motif | Source | % Conserv (Total) | % Conserv (Core) |
|---|---|---|---|---|---|
| 20 | FPQPQLPYSQPQPFRPQQPY | DR sup | Gliadin 61-80 | | |
| 16 | IPPYCTIAPFGIFGTN | DR sup | Gliadin 261-276 | | |
| 20 | LGQQQPFPPQQPYPQPQPFP | DR sup | Gliadin 31-50 | | |
| 20 | LHQQQKQQQQPSSQVSFQQP | DR sup | Gliadin 181-200 | | |
| 20 | LLQELCCQHLWQIPEQSQCQ | DR sup | Gliadin 151-170 | | |
| 20 | LQQHNIAHGRSQVLQQSTYQ | DR sup | Gliadin 131-150 | | |
| 20 | PQPFRPQQPYPQPQPQYSQP | DR sup | Gliadin 71-90 | | |
| 20 | PQPQPQYSQPQQPISQQQQQ | DR sup | Gliadin 81-100 | | |
| 20 | PSSQVSFQQPLQQYPLGQGS | DR sup | Gliadin 191-210 | | |
| 20 | QFEEIRNLALQTLPAMCNVY | DR sup | Gliadin 231-250 | | |
| 20 | QGSVQPQQLPQFEEIRNLAL | DR sup | Gliadin 221-240 | | |
| 20 | QNPSQQQPQEQVPLVQQQQF | DR sup | Gliadin 11-30 | | |
| 20 | QPYPQPQPFPSQQPYLQLQP | DR sup | Gliadin 41-60 | | |
| 20 | QQLIFCMDVVLQQHNIAHGR | DR sup | Gliadin 121-140 | | |
| 20 | QVPLVQQQQFLGQQQPFPPQ | DR sup | Gliadin 21-40 | | |
| 20 | SQQPYLQLQPFPQPQLPYSQ | DR sup | Gliadin 51-70 | | |
| 20 | VRVPVPQLQPQNPSQQQPQE | DR sup | Gliadin 1-20 | | |
| 20 | IRNLALQTLPAMCNVY | DR sup | gliadin 235 | | |
| 12 | PQPFRPQQPYPQ | DR sup | gliadin 71 | | |
| 16 | PQPFRPQQPYPQPQPQ | DR sup | gliadin 71 | | |
| 12 | QFEEIRNLALQT | DR sup | gliadin 231 | | |
| 16 | QFEEIRNLALQTLPAM | DR sup | gliadin 231 | | |
| 20 | QFLGQQQPFPPQ | DR sup | gliadin 29 | | |
| 12 | QVPLVQQQQFLG | DR sup | gliadin 21 | | |
| 16 | QVPLVQQQQFLGQQQP | DR sup | gliadin 21 | | |
| 20 | VQQQQFLGQQQPFPPQ | DR sup | gliadin 25 | | |
| 16 | aHAAHAAHAAHAAHAa | DR sup | d protected AHA reiterative | | |
| 12 | CPKYVRSAKLRM | DR sup | HA 302-313 (PR8) | | |
| 12 | GACPKYVKQNTL | DR sup | HA 304-315 | | |
| 13 | GACPKYVKQNTLK | DR sup | HA 304-316 | | |
| 12 | KQNTLKLATGMR | DR sup | HA 311-322 | | |
| 18 | LAKQNTLAKQNTLAKQNT | DR sup | HA 307-319 reiterative | | |
| 13 | PKAVKQNTLKLAT | DR sup | HA 307-319 analog | | |
| 13 | PKSVKQNTLKLAT | DR sup | HA 307-319 analog | | |
| 13 | PKYDKQGGLKIAT | DR sup | HA multivariate | | |
| 13 | PKYFKQFRLKIAT | DR sup | HA multivariate | | |
| 13 | PKYGKQRFLKIAT | DR sup | HA multivariate | | |
| 13 | PKYIKQIILKIAT | DR sup | HA multivariate | | |
| 13 | PKYVKKNTLKLAT | DR sup | HA 307-319 analog | | |
| 13 | PKYVKQNKLKLAT | DR sup | HA 307-319 analog | | |
| 13 | PKYVKQNTKKLAT | DR sup | HA 307-319 analog | | |
| 13 | PKYVKQNTLKEAT | DR sup | HA 307-319 analog | | |
| 13 | PKYVKQNTLKLAT | DR sup | HA 307-319 | | |
| 13 | PKYVKQNTLKIAT | DR sup | HA 307-319 analog | | |
| 13 | PKYVKQNTLKIAT | DR sup | HA 307-319 analog | | |
| 13 | PKYVKQNTIKLAT | DR sup | HA 307-319 analog | | |
| 13 | PKYVKQnTLKLAT | DR sup | HA 307-319 analog | | |
| 13 | pKYVKQNTLKLAT | DR sup | HA 307-319 analog | | |
| 13 | PKYVKQNTLKNAT | DR sup | HA 307-319 analog; Asn scan | | |
| 13 | PKYVKQNTNKLAT | DR sup | HA 307-319 analog; Asn scan | | |
| 20 | RTLYQNVGTYVSVGTSTLNK | DR sup | HA 187-206 | | |
| 13 | VKQNTLKLATGMR | DR sup | HA 310-322 | | |
| 14 | YPKYVKRNTLKLAT | DR sup | HA 307-319 db1. substitutions | | |
| 15 | AAPFTQCGYPALMPL | DR sup | HBV POL 643 | 95 | |
| 15 | AFSYMDDVVLGAKSV | DR sup | HBV POL 546 | 90 | |
| 12 | AILCWGELMTLA | DR sup | HBV core 58-69 | | |
| 16 | ALRQAILCWGELMTLA | DR sup | HBV core 54-69 | | |
| 20 | ASARFSWLSLLVPFVQWFVG | DR sup | HBs (ayw) 166-185 | | |
| 15 | DWKVCQRIVGLLGFA | DR sup | HBV POL 629 | 85 | |
| 15 | GAHLSLRGLPVCAFS | DR sup | HBV X 50 | 90 | |
| 20 | GYRWMCLRRFIIFLFILLLC | DR sup | HBs (ayw) 71-90 | | |
| 19 | HHTALRQAILCWGELMTLA | DR sup | HBV core 51-69 | | |
| | HLSLRGLPVCAFSSA | DR sup | HBV.X.52 | 90 | |
| 18 | HTALRQAILCWGELMTLA | DR sup | HBV core 52-69 | | |
| 11 | ILCWGELMTLA | DR sup | HBV core 59-69 | | |
| 15 | IVGLLGFAAPFRQCG | DR sup | HBV POL 636 | 90 | |
| | LCQVFADATPTGWGL | DR sup | HBV.POL.694 | 95 | |
| 10 | LCWGELMTLA | DR sup | HBV core 60-69 | | |
| 15 | LRQAILCWGELMTLA | DR sup | HBV core 55-69 | | |
| 20 | LSPTVWLSVIWMMWYWGPSL | DR sup | HBs (ayw) 186-205 | | |
| 16 | LSTLPETTVVRRRGRS | DR sup | Hep. B core 140-154 | | |
| 19 | MDIDPYKEFGASVELLSFL | DR sup | HBV core 1 | | |
| 25 | MDIDPYKEFGASVELLSFLPSDFFP | DR sup | HBV core 1 | | |
| 19 | MDIDPYKEFGATVELLSFL | DR sup | HBV core 1 | | |
| 20 | MDIDPYKEFGATVELLSFLP | DR sup | Hep. B core 1-20 | | |
| 25 | MDIDPYKEFGATVELLSFLPSDFFP | DR sup | HBV core 1-25 | | |
| 25 | MDIDPYKEFGATVELLSFLPSDFFP | DR sup | HBV core 1 | | |
| 25 | MDIDPYKEFGATVQLLSFLPSDFFP | DR sup | HBV core 1 | | |
| 15 | NAPILSTLPETTVVR | DR sup | HBV NUC 136 | 95 | |
| 18 | PFLLAQFTSAICSVVRRA | DR sup | HBV pol 523 | 95 | |
| 13 | PHHTALRQAILCW | DR sup | HBV core 50-62 | | |
| 14 | PHHTALRQAILCWG | DR sup | HBV core 50-63 | | |
| 16 | PHHTALRQAILCWGEL | DR sup | HBV core 50-65 | | |
| 17 | PHHTALRQAILCWGELM | DR sup | HBV core 50-66 | | |
| 18 | PHHTALRQAILCWGELMT | DR sup | HBV core 50-67 | | |
| 19 | PHHTALRQAILCWGELMTL | DR sup | HBV core 50-68 | | |
| 20 | PHHTALRQAILCWGELMTLA | DR sup | HBV core 50-69 | | |
| | PLPIHTAELLAACFA | DR sup | HBV.POL.722 | 80 | |
| 15 | PPAYRPPNAPILSTL | DR sup | HBV NUC 129 | 95 | |
| | PQAMQWNSTTFHQTL | DR sup | HBV.ENV.114 | 40 | 80 |
| 13 | QAILCWGELMTLA | DR sup | HBV core 57-69 | | |
| 15 | QCGYPALMPLYACIQ | DR sup | HBV POL 648 | 95 | |
| | RDLLDTASALYREAL | DR sup | HBV.NUC.28 | 80 | |
| 20 | RDLLDTASALYRREALESPEH | DR sup | Hep. B core 28-47 | | |
| 20 | RDLVVSYVNTNMGLKFRQLL | DR sup | Hep. B core 82-101 | | |
| 15 | RFSWLSLLVPFVQWF | DR sup | HBV ENV 332 | 100 | |
| 14 | RQAILCWGELMTLA | DR sup | HBV core 56-69 | | |
| 20 | SLDSWWTSLNFLGGTTVCLG | DR sup | HBs (ayw) 31-50 | 80 | |
| | SVRFSWLSLLVPFVQ | DR sup | HBV.ENV.330 | | |
| 17 | TALRQAILCWGELMTLA | DR sup | HBV core 53-69 | | |
| 15 | TNLLSSNLSWLSLDV | DR sup | HBVPOL416 | 90 | |
| 25 | TNMGLKFRQLLWFHI | DR sup | HBV core 91 | | |
| 25 | TNVGLKFRQLLWFHI | DR sup | HBV core 91 | | |
| 14 | TTVVRRRGRSPRRR | DR sup | HBV Core 146-159 | | |
| 15 | VCAFSSAGPCALRFT | DR sup | HBV X 60 | 90 | |
| 20 | VSFGVWIRTPPAYRPPNAPI | DR sup | HBV nuc 120 | 90 | |
| | YPALMPLYACIQSKQ | DR sup | HBV.POL.648 | 55 | 95 |
| | AEQFKQKALGLLQTA | DR sup | HCV.NS4.1730 | 86 | |
| | ANLLWRQEMGGNITR | DR sup | HCV.NS5.2238 | 86 | |
| | ARLIVFPDLGVRVCE | DR sup | HCV.NS5.2610 | 79 | |
| | ASQLSAPSLKATCTT | DR sup | HCV.NS5.2208 | 50 | 79 |
| | AVQWMNRLIAFASRG | DR sup | HCV.NS4.1917 | 100 | 100 |
| | DADLIEANLLWRQEM | DR sup | HCV.NS5.2232 | 50 | 85 |
| | EDLVNLLPAILSPGA | DR sup | HCV.NS4.1882 | 79 | 85 |
| | GALVVGVVCAAILRR | DR sup | HCV.NS4.1895 | 79 | |
| | GCSFSIFLLALLSCL | DR sup | HCV.Core.171 | 86 | |
| 20 | GPGEGAVQWMNRLIAFASRG | DR sup | HCV NS4 291-310 | | |
| | KPTLHGPTPLLYRLG | DR sup | HCV.NS4.1620 | 79 | |
| | LAGYGAGVAGALVAF | DR sup | HCV.NS4.1857 | 79 | |
| | LHGLSAFSLHSYSPG | DR sup | HCV.NS5.2919 | 79 | 79 |
| 20 | LLFNILGGWVAAQLAAPGAA | DR sup | HCV NS4 191-210 | 57 | 100 |
| | LTSMLTDPSHITAET | DR sup | HCV.NS5.2176 | | |
| 20 | NFISGIQYLAGLSTLPGNPA | DR sup | HCV NS4 1772 | | |
| | PAILSPGALVVGVVCA | DR sup | HCV.NS4.1889 | 79 | |
| | PQTFQVAHLHAPTGS | DR sup | HCV.NS3.1225 | 43 | 85 |
| | PTLWARMILMTHFFS | DR sup | HCV.NS5.2870 | 79 | 85 |
| | RAAVCTRGVAKAVDF | DR sup | HCV.NS3.1186 | 79 | |
| | TVDFSLDPTFTIETT | DR sup | HCV.NS3.1466 | 79 | |
| | VVLLFLLLADARVCS | DR sup | HCV.NS1/E2.724 | 29 | 100 |
| | WESVFTGLTHIDAHF | DR sup | HCV.NS3.1563 | 43 | 92 |
| 10 | AFVAWRNRCK | DR sup | HEL 107-116 analogs | | |
| 10 | AWAAWRNRCK | DR sup | HEL 107-116 analogs | | |
| 10 | AWEAWRNRCK | DR sup | HEL 107-116 analogs | | |
| 10 | AWLAWRNRCK | DR sup | HEL 107-116 analogs | | |
| 10 | AWVAARNRCK | DR sup | HEL 107-116 analogs | | |
| 10 | AWVAFRNRCK | DR sup | HEL 107-116 analogs | | |
| 10 | AWVAQRNRCK | DR sup | HEL 107-116 analogs | | |
| 10 | AWVAWANRCK | DR sup | HEL 107-116 analogs | | |
| 10 | AWVAWENRCK | DR sup | HEL 107-116 analogs | | |
| 10 | AWVAWKNRCK | DR sup | HEL 107-116 analogs | | |
| 10 | AWVAWRARCK | DR sup | HEL 107-116 analogs | | |
| 10 | AWVAWRNACK | DR sup | HEL 107-116 analogs | | |
| 10 | AWVAWRNECK | DR sup | HEL 107-116 analogs | | |
| 10 | AWVAWRNKCK | DR sup | HEL 107-116 analogs | | |
| 10 | AWVAWRNRCA | DR sup | HEL 107-116 analogs | | |
| 0 | AWVAWRNRCK | DR sup | HEL 107-116 | | |
| 10 | AWVAWRNRCK | DR sup | HEL 107-116 | | |
| 10 | AWVAWRNRCR | DR sup | HEL 107-116 analogs | | |
| 10 | AWVAWKNREK | DR sup | HEL 107-116 analogs | | |
| 10 | AWVAWRNRKK | DR sup | HEL 107-116 analogs | | |
| 10 | AWVAWRNRQK | DR sup | HEL 107-116 analogs | | |
| 10 | AWVAWRNRVK | DR sup | HEL 107-116 analogs | | |
| 10 | AWVAWRVRCK | DR sup | HEL 107-116 analogs | | |
| 10 | AWVEWRNRCK | DR sup | HEL 107-116 analogs | | |
| 10 | AWVSWRNRCK | DR sup | HEL 107-116 analogs | | |
| 10 | AWVVWRNRCK | DR sup | HEL 107-116 analogs | | |
| 10 | EFVAAKAAQK | DR sup | Super HEL 107-116 | | |
| 10 | EWVAWRNRCK | DR sup | HEL 107-116 analogs | | |
| 22 | LAAAMKRHGLDNYRGYSLGNWV | DR sup | HEL 8-29 | | |
| 16 | NTDGSTDYGILQINSR | DR sup | HEL 46-61 | | |
| 18 | RNRCKGTDVQAWIRGCRL | DR sup | HEL 112-129 | | |
| 16 | SVNCAKKIVSDGNGMN | DR sup | HEL91-106 | | |
| 10 | SWVAWRNRCK | DR sup | HEL 107-116 analogs | | |
| 10 | VWVAWRNRCK | DR sup | HEL 107-116 analogs | | |
| 15 | WRNAKWRNAKWRNAK | DR sup | HEL 112-116 reit. | | |
| 22 | YRGYSLGNVWCAAKFESNFNTQ | DR sup | HEL 20-41 | | |
| 13 | SPYVSRLLGICLT | DR sup | Her2/neu.777 | | |
| 15 | ASDFNLPPVVAKEIV | DR sup | HIV1 POL 765 | 80 | |
| 15 | AVQMAVFIHNFKRKG | DR sup | HIV1 POL 917 | 100 | |
| 15 | DFNLPPVVAKEIVAS | DR sup | HIV1 POL 767 | 87 | |
| 15 | DQQLLGIWGCSGKLI | DR sup | HIV1 ENV 755 | 83 | |
| 15 | DQSLKPCVKLTPLCV | DR sup | HIV1 ENV 126 | 90 | |
| 12 | DRVHPVHAGPIA | DR sup | HIV gag 245 | | |
| 14 | DRVHPVHAGPIAPG | DR sup | HIV gag 245 | | |
| 16 | DRVHPVHAGPIAPGQM | DR sup | HIV gag 245 | | |
| 18 | DRVHPVHAGPIAPGQMRE | DR sup | HIV gag 245 | | |
| 20 | DRVHPVHAGPIAPGQMREPR | DR sup | HIV gag 245 | | |
| 22 | DRVHPVHAGP1APGQMREPRGS | DR sup | HIV gag 245 | | |
| 20 | DTEVHNVWATQACVPTDPNP | DR sup | HIV env 6 | | |
| 15 | EAIIRILQQLLFIHF | DR sup | HIV1 VPR 58 | 82 | |
| 15 | EDIISLWDQSLKPCV | DR sup | HIV1 ENV 119 | 87 | |
| 15 | EKAFSPEVIPMFSAL | DR sup | HIV1 GAG 193 | 96 | |
| 15 | EKVYLAWVPAHKGIG | DRsup | HIV1.pol.711 | 74 | 93 |
| 16 | ERFAVNPGLLETSEGC | DR sup | HIV gp17 41-56 | | |
| 16 | ERYLKDQQLLGIWGCS | DRsup | HIV1 ENV 589 | | |
| 15 | ESELVSQIIEQLIKK | DR sup | HIV1 POL 696 | 80 | |
| | ETAYFLLKLAGRWPV | DR sup | HIV.POL.838 | 65 | |
| 15 | EVQLGIPHPAGLKKK | DR sup | HIV1 POL 268 | 80 | |
| 15 | FWEVQLGIPHPAGLK | DR sup | HIV1 POL 266 | 100 | |
| 16 | GARASVLSGGELDKWE | DR sup | HIV gp17 1-16 | | |
| 15 | GCTLNFPISPIETVP | DR sup | HIV1 POL 174 | 100 | |
| 15 | GEIYKRWIILGLNKI | DR sup | HIV1.gag.294 | 45 | 85 |
| 16 | GGELDKWEKIRLRPGG | DR sup | HIV gp17 9-24 | | |
| 15 | HKAIGTVLVGPTPVN | DR sup | HIV1 POL 149 | 93 | |
| 15 | IGGIGGFIKVRQYDQ | DR sup | HIV1 POL 127 | 93 | |
| | IIRILQQLLFIWRI | DR sup | HIV1.VPR.60 | 76 | 82 |
| 15 | IISLWDQSLKPCVKL | DR sup | HIV1 ENV 121 | 85 | |
| 15 | IKVVPRRKAKIIRDY | DR sup | HIV1 POL 999 | 80 | |
| 16 | ILKALGPAATLEEMMT(200.11) | DR sup | HIV | | |
| 16 | INEEAAEWERVHPVHA | DR sup | HIV gp25 73-88 | | |
| 15 | IQKLVGKLNWASQIY | DR sup | HIV1 POL 436 | 100 | |
| 15 | ISLWDQSLKPCVKLT | DR sup | HIV1 ENV 122 | 83 | |
| 16 | IVWASRELERFAVNPG | DR sup | HIV gp17 33-48 | | |
| 20 | IYKRWIILGLNKIVRMYSPV | DR sup | HIV gag 27 | | |
| 16 | IYKRWIILGLNKIVRN | DR sup | HIV gp25 129-144 | | |
| | KARVLAEAMSQVTNS | DR sup | HIV1.GAG.394 | 46 | 88 |
| 15 | KDSWTVNDIQKLVGK | DR sup | HIV1 POL 428 | 100 | |
| 16 | KQIINMWQEVGKAMYA | DR sup | HIV1 ENV 428 | | |
| 15 | KRWIILGLNKIVRMY | DR sup | HIV1.gag.298 | 77 | 88 |
| 15 | KTILKALGPAATLEE | DR sup | HIV1 GAG 366 | 92 | |
| 16 | LGKIWPSYKGRPGNFL | DR sup | HIV gp13 57-72 | | |
| 16 | LICTTAVPWNASWSNK | DR sup | HIVN1 ENV 607 | | |
| 16 | LKQIVKKLREQFGNNK | DR sup | HIV1 ENV 342 | | |
| 15 | LLGIWGCSGKLICTT | DR sup | HIV 1 ENV 758 | 81 | |
| 15 | LSIVNRVRQGYSPLS | DR sup | HIV1 ENV 877 | 81 | |
| 15 | NEQVDKLVSAGIRKV | DR sup | HIV1 POL 727 | 80 | 82 |
| | NNLLRAIEAQQHLLQ | DR sup | HIV1.ENV.719 | 61 | |
| 15 | PAGLKKKKSVTVLDV | DR sup | HIVl POL 276 | 80 | |
| 15 | PGNFLQSRPEPTAPP | DR sup | HIV1 GAG 490 | 80 | |
| 16 | PIVQNLQGQMVHQAIS | DR sup | HIV gp25 1-16 | | |
| 16 | PLGVAPTKAKRRVVQR | DR sup | HIV1 ENV 671 | | |
| 16 | QARILAVERYLKDQQL | DR sup | HIV1 ENV 582 | | |
| 15 | QGQMVHQAISPRTLN | DR sup | HIV1.gag.171 | 44 | 85 |
| 16 | QKQEPIDKELYPLTSL | DR sup | HIV gp13 97-112 | | |
| 15 | QMAVFIHNFKRKGGI | DR sup | HIV1 POL 919 | 100 | |
| 16 | RIQRGPGRAFVTIGKL | DR sup | HIV1 ENV 315 | | |
| 16 | RQILGQLQPSLQTGSE | DR sup | HIV gp17 57-72 | | |
| 16 | SLKPCVKLTPLCVTLN | DR sup | HIV1 ENV 115 | | |
| 16 | SLWDQSLKPCVKLTPL | DR sup | HIV1 ENV 825 | | |
| 15 | SPAIFQSSMTKILEP | DR sup | HIV1.pol.335 | 70 | 80 |
| 15 | SPGIWQLDCTHLEGK | DR sup | HIV1 POL 799 | 100 | |
| 15 | SQIIEQLIKKEKVYL | DR sup | HIV1 POL 701 | 80 | |
| 16 | SSGGDPEIVMHSFNCG | DR sup | HIV1 ENV 369 | | |
| 13 | TAATNAACAWLEA | DR sup | HIV nef 48 | | |
| 17 | TITLPCRIKQFINMWQE | DR sup | HIV1 ENV 413 | | |
| 15 | VDKLVSAGIRKVLFL | DR sup | HIV1 POL 730 | 80 | |
| 15 | VIPMFSALSEGATPQ | DR sup | HIV1 GAG 200 | 88 | |
| 16 | VKIEPLGVAPTICAKRR | DR sup | HIV1 ENV 667 | | |
| 15 | VLSIVNRVRQGYSPL | DR sup | HIV1 ENV 876 | 81 | |
| 15 | VNIIGRNLLTQIGCT | DR sup | HIV1 POL 162 | 87 | |
| 15 | VNIVTDSQYALGIIQ | DR sup | HIV1 POL 675 | 93 | |
| 15 | VTVYYGVPVWKEATT | DR sup | HIV1 ENV 48 | 83 | |
| 20 | VWGIKQLQARVLAVERYLKD | DR sup | HIV env 54 | | |
| 16 | WDQSLKPCVKLTPLCV | DR sup | HIV1 ENV 112 | | |
| 16 | WGCSGKLICTTAVPWN | DR sup | HIV1 ENV 601 | | |
| 15 | WRKLVDFRELNKRTQ | DR sup | HIV1 POL 250 | 80 | |
| 20 | VYVWKTTWGQYWQVLGGPVS | DR sup | gp100.150 | | |
| 17 | ALHIYMNGTMSQVQGSA | DR sup | Tyrosinase.365 | | |
| 16 | WPSVFYNRTCQCSGNF | DR sup | Tyrosinase.80 | | |
| 20 | YGQMKNGSTPMFNDINIYDL | DR sup | Tyrosinase.156 | | |
| 15 | DMYSKYGGTEIKY | DR sup | M. Leprae 67-81 | | |
| 15 | IKYNGEEYLILSARD | DR sup | M. Leprae 79-93 | | |
| 15 | IYSKYGGTEIKYNGE | DR sup | M. Leprae 70-84 | | |
| 15 | LVIPENAKEKPQEGT | DR sup | M. Leprae 28-42 | | |
| 15 | NGEEYLILSARDVLA | DR sup | M. Leprae 82-96 | | |
| 15 | PSGLVIPENAKEKPQ | DR sup | M. Leprae 25-39 | | |
| 15 | RIPVDVSEGDIVIYS | DR sup | M. Leprae 58-72 | | |
| 15 | SEGDIVIYSKYGGTE | DR sup | M. Leprae 64-78 | | |
| 15 | VAKVKIKPLEDKILV | DR sup | M. Leprae 1-15 | | |
| 15 | VKIKPLEDKILVQAG | DR sup | M. Leprae 4-18 | | |
| 15 | LQLVFGIEVVEVVPI | DR sup | MAGE2.158 | | |
| 15 | PRKLLMQDLVQENYL | DR sup | MAGE2.242 | | |
| 15 | QDFFPVIFSKASEYL | DR sup | MAGE2.144 | | |
| 15 | QLVFGIEVVEVVPIS | DR sup | MAGE2.159 | | |
| 15 | RALIETSYVKVLHHT | DR sup | MAGE2.276 | | |
| 15 | RKLLMQDLVQENYLE | DR sup | MAGE2.243 | | |
| 15 | SHLYILVTCLGLSYD | DR sup | MAGE2.173 | | |
| 15 | YEFLWGPRALIETSY | DR sup | MAGE2.269 | | |
| 15 | GEALGLVGAQAPATE | DR sup | MAGE2/3.20 | | |
| 15 | FFPVIFSKASSSLQL | DR sup | MAGE3.146 | | |
| 15 | FPVIFSKASSSLQLV | DR sup | MAGE3.147 | | |
| 15 | GIELMEVDPIGHLYI | DR sup | MAGE3.163 | | |
| 15 | QAALSRKVAELVHFL | DR sup | MAGE3.106 | | |
| 15 | QYFFPVIFSKASSSL | DR sup | MAGE3.144 | | |
| 15 | RALVETSYVKVLHHM | DR sup | MAGE3.276 | | |
| 15 | VDPIGHLYIFATCLG | DR sup | MAGE3.169 | | |
| 15 | VFGIELMEVDPIGHL | DR sup | MAGE3.161 | | |
| 15 | VGNWQYFFPVIFSKA | DR sup | MAGE3.140 | | |
| 15 | YEFLWGPRALVETSY | DR sup | MAGE3.269 | | |
| 15 | VGNWQYFFPVIFSKA | DR sup | Mage3/6.140 | | |
| 25 | MVKISGGPRISYPLLHEWALREGEE | DR sup | Mage6.290 | | |
| 24 | QVPGSDPACYEFLWGPRALIETSY | DR sup | Mage6.260 | | |
| 15 | ANPVVEWFKNTVTPR | DR sup | Human MBP 85-99(85A) | | |
| 20 | ASQKRPSQRHGSKYLATAST | DR sup | Human MBP 1-20 | | |
| 20 | AYDAQGTLSKIFKLGGRDSR | DR sup | Mouse MBP 141-160 | | |
| 11 | DENPVVHFFKN | DR sup | Human MBP 84-94 | | |
| 12 | DENPVVBFFKNI | DR sup | Human MBP 84-95 | | |
| 13 | DENPVVHFFICNIV | DR sup | Human MBP 84-96 | | |
| 14 | DENPVVHFFKNIVT | DR sup | Human MBP 84-97 | | |
| 16 | DENPVVHFFKNIVTPR | DR sup | Human MBP 84-99 | | |
| 17 | DENPVVHFFKNIVTPRT | DR sup | Human MBP 84-100 | | |
| 19 | DENPVVHFFKNTVTPRTPP | DR sup | Human MBP 84-102 | | |
| 20 | DENPVVHFFKNIVTPRTPPY | DR sup | Human MBP 84-102Y | | |
| 20 | DENPVVHFFRNIVTPRTPPY | DR sup | Human MBP 84-102Y(99R) | | |
| 15 | EAPVVHFFKNTVTPR | DR sup | Human MBP 85-99(86A) | | |
| 15 | ENAVVHFFKNIVTPR | DR sup | Human MBP 85-99(87A) | | |
| 15 | ENPA VHFFKNIVTPR | DR sup | Human MBP 85-99(88A) | | |
| 15 | ENPKVHFFKNIVTPR | DR sup | Human MBP 85-99 SAAS (88K) | | |
| 15 | ENPVAHFFKNIVTPR | DR sup | Human MBP 85-99(89A) | | |
| 15 | ENPVKHFFKNIVTPR | DR sup | Human MBP 85-99 SAAS (89K) | | |
| 15 | ENPVVAFFKNIVTPR | DR sup | Human MBP 85-99(90A) | | |
| 15 | ENPVVAFFKNIVTPR | DR sup | Human MBP 85-99 SAAS (90A) | | |
| 15 | ENPVVDFFKNTVTPR | DR sup | Human MBP 85-99 SAAS (90D) | | |
| 15 | ENPVVFFFKNIVTPR | DR sup | Human MBP 85-99 SAAS (90F) | | |
| 15 | ENPVVHAFKNIVTPR | DR sup | Human MBP 85-99(91A) | | |
| 15 | ENPVVHAFKNIVTPR | DR sup | Human MBP 85-99 SAAS (91A) | | |
| 15 | ENPVVHAFRNIVTPR | DR sup | MBP85-99 (91A,93R) | | |
| 15 | ENPVVHDFKNIVTPR | DR sup | Human MBP 85-99 SAAS (91D) | | |
| 15 | ENPVVHFAKCNIVTPR | DR sup | Human MBP 85-99(92A) | | |
| 15 | ENPVVHFARNIVTPR | DR sup | MBP85-99 (92A,93R) | | |
| 15 | ENPVVHFFANIVTPR | DR sup | Human MBP 85-99(93A) | | |
| 15 | ENPVVHFFANIVTPR | DR sup | Human MBP 85-99 SAAS (93A) | | |
| 17 | ENPVVHFFANIVTPRTP | DR sup | MBP 91K>A analog | | |
| 15 | ENPVVHFFDNIVTPR | DR sup | Human MBP 85-99 SAAS (93D) | | |
| 15 | ENPVVHFFHNIVTPR | DR sup | Human MBP 85-99 SAAS (93H) | | |
| 15 | ENPVVHFFKAIVTPR | DR sup | Human MBP 85-99(94A) | | |
| 17 | ENPVVHFFKAIVTPRTP | DR sup | MBP 92N>A analog | | |
| 15 | ENPVVHFFKKIVTPR | DR sup | Human MBP 85-99 SAAS (94K) | | |
| 15 | ENPVVHFFKNAVTPR | DR sup | Human MBP 85-99(95A) | | |
| 17 | ENPVVHFFKNAVTPRTP | DR sup | MBP 931>A analog | | |
| 15 | ENPVVHFFKNIATPR | DR sup | Human MBP 85-99(96A) | | |
| 15 | ENPWHFFKNIVAPR | DR sup | Human MBP 85-99(97A) | | |
| 17 | ENPVVHFFKNIVAPRTP | DR sup | MBP 95T>A analog | | |
| 15 | ENPWHFFKNIVTAR | DR sup | Human MBP 85-99(98A) | | |
| 15 | ENPVVHFFKNIVTPA | DR sup | Human MBP 85-99(99A) | | |
| 15 | ENPVVHFFKNTVTPA | DR sup | Human MBP 85-98A | | |
| 15 | ENPVVHFFKNIVTPR | DR sup | Human MBP 85-99 | | |
| 17 | ENPVVHFFKNIVTPRTP | DR sup | MBP 83 | | |
| 19 | ENPVVHFFKNIVTPRTPPY | DR sup | Human MBP 85-102Y | | |
| 15 | ENPVVHTKNKVTPR | DR sup | Human MBP 85-99 SAAS (95K) | | |
| 15 | ENPWHFFLNIVTPR | DR sup | Human MBP 85-99 SAAS (93L) | | |
| 15 | ENVVHFFRNIVTPR | DR sup | Human MBP 85-99 SAAS (93R) | | |
| 15 | ENPVVETKKNIVTPR | DR sup | Human MBP 85-99 SAAS (92K) | | |
| 15 | ENFVVHHFKNIVTPR | DR sup | Human MBP 85-99 SAAS (91H) | | |
| 15 | ENPVVHHFRNIVTPR | DR sup | MBP85-99 (91H,93R) | | |
| 15 | ENPVVHLFKNIVTPR | DR sup | Human MBP 85-99 SAAS (91L) | | |
| 15 | ENPVVHLFRNIVTPR | DR sup | MBP85-99 (91L,93R) | | |
| 15 | ENPVVHWFKNIVTPR | DR sup | Human MBP 85-99 SAAS (91W) | | |
| 15 | ENPVVHYFANIVTPR | DR sup | MBP85-99 (91Y,93A) | | |
| 15 | ENPVVHYFHNIVTPR | DR sup | MBP85-99 (91Y,93H) | | |
| 15 | ENPVVHYFKNIVTPR | DR sup | Human MBP 85-99 SAAS (91Y) | | |
| 15 | ENPVVHYFLNIVTPR | DR sup | MBP85-99 (91Y,93L) | | |
| 15 | ENPVVHYFRNIVTPR | DR sup | MBP85-99 (91Y,93R) | | |
| 15 | ENPVVKFFKNIVTPR | DR sup | Human MBP 85-99 SAAS (90K) | | |
| 16 | FFKNIVTPFFKNIVTP | DR sup | MBP reiterative | | |
| 20 | GFGYGGRASDYKSAHKGFKG | DR sup | Mouse MBP 121-140/Human 124-143 | | |
| 14 | HFFKNIVTPRTPPY | DR sup | Human MBP 90-102Y | | |
| 14 | HFFKNIVTPRTPPY | DR sup | Human MBP 90-102Y | | |
| 20 | IFKLGGRDSRSGSPMARR | DR sup | Mouse MBP 151-168/Human 154-171 | | |
| 12 | NPVVHFFKNIVT | DR sup | Human MBP 86-97 | | |
| 14 | NPVVHFFKNIVTPA | DR sup | Human MBP 86-98A | | |
| 14 | NPVVHFFKNIVTPR | DR sup | Human MBP 86-99 | | |
| 18 | NPVVHFFKNIVTPRTPPY | DR sup | Human MBP 86-102Y | | |
| 11 | PVVHFFKNIVT | DR sup | Human MBP 87-97 | | |
| 13 | PVVHFFKNIVTPA | DR sup | Human MBP 87-98A | | |
| 13 | PVVHFFKNIVTPR | DR sup | Human MBP 87-99 | | |
| 17 | PVVHFFKNIVTPRTPPY | DR sup | Human MBP 87-102Y | | |
| 20 | QKSHGRTQDENPVVHFFKNI | DR sup | Human MBP 74-93 | | |
| 20 | RASDYKSAHKGFKGVDAQGT | DR sup | MBP 131-152 | | |
| 20 | RASDYKSAHKGLKGHDAQGT | DR sup | MBP 131-152 F>L analog | | |
| 20 | RFFSGDRGAPKRGSGKDSHT | DR sup | Mouse MBP 41-60 | | |
| 20 | VDAQGTLSICIFKLGGRDSRS | DR sup | Hu MBP 144-163 | | |
| 20 | VDAQGTLSKLFKLGGRDSRS | DR sup | MBP 144-163 var/analog | | |
| 20 | VDAQGTLSRIFKLGGRDSRS | DR sup | MBP 144-163 var./analog | | |
| 20 | VDAQGTLSRLFKLGGRDSRS | DR sup | Rabbit MBP 144-163 | | |
| 15 | VHFFKNIVTPRTPPY | DR sup | Human MBP 89-102Y | | |
| 16 | VVHFFKNIVTPRTPPY | DR sup | Human MBP 88-102Y | | |
| 20 | YKSAHKGFKGAYDAQGTLSK | DR sup | Mouse MBP 131-150 | | |
| 15 | AKSVTCTYSPALNKM | DR sup | p53.119 | | |
| 15 | APPVAPAPAAPTPAA | DR sup | p53.70 | | |
| 15 | APSWPLSSSVPSQKT | DR sup | p53.88 | | |
| 15 | FCQLAKTCPVQLWVD | DR sup | p53.134 | | |
| 15 | FSDLWKLLPENNVLS | DR sup | p53.19 | | |
| 15 | GTRVRAMAIYKQSQH | DR sup | p53.154 | | |
| 15 | HYNYMCNSSCMGGMN | DR sup | p53.233 | | |
| 15 | LGFLHSGTAKSVTCT | DR sup | p53.111 | | |
| 15 | LSPLPSQAMDDLMLS | DR sup | p53.32 | | |
| 15 | NNVLSPLPSQAMDDL | DR sup | p53.29 | | |
| 15 | RLGFLHSGTAKSVTC | DR sup | p53.110 | | |
| 15 | RNTFRHSVVVPYEPP | DR sup | p53.209 | | |
| 15 | SWPLSSSVPSQKTYQ | DR sup | p53.90 | | |
| 15 | SYGFRLGFLHSGTAK | DR sup | p53.106 | | |
| 15 | WKLLPENNVLSPLPS | DR sup | p53.23 | | |
| 15 | YNYMCNSSCMGGMNR | DR sup | p53.234 | | |
| 14 | AKFVAAWTLKAAA | DR sup | PADRE analog | | |
| 14 | YAKFVAAWTLKAAA | DR sup | PADRE analog | | |
| 15 | AGGIAGGLALLACAG | DR sup | Pf SSP2 498 | 100 | |
| 15 | ATSVLAGLLGNVSTV | DR sup | Pf EXP1 77 | 100 | |
| 15 | AVPLAMKLIQQLNLN | DR sup | Pf SSP2 68 | 100 | |
| 15 | FLALFFIIFNKESLA | DR sup | Pf EXP1 8 | 100 | |
| 15 | GRDVQNNIVDEIKYR | DR sup | Pf SSP2 25 | 90 | |
| 15 | IFHINGKIIKNSEKD | DR sup | Pf LSA1 18 | 100 | |
| 15 | KHILYISFYFILVNL | DR sup | Pf LSA1 2 | 100 | |
| 15 | KYLVIVFLIFFDLFL | DR sup | Pf SSP2 8 | 100 | |
| 15 | LGNVSTVLLGGVGLV | DR sup | Pf EXP1 85 | 100 | |
| 15 | LIDVHDLISDMIKKE | DR sup | Pf EXP1 47 | 100 | |
| 15 | LIFFDLFLVNGRDVQ | DR sup | Pf.TRAP.15 | 100 | |
| 15 | LSVFFLALFFIIFNK | DR sup | Pf EXP1 4 | 100 | |
| 20 | NAREIIRLHSDASKNKEKAL | DR sup | Pf.SSP2 | | |
| 15 | NDRINRENANQLVVI | DR sup | Pf SSP2 145 | 100 | |
| 15 | NHAVPLAMKLIQQLN | DR sup | Pf SSP2 66 | 80 | |
| 15 | SFYFILVNLLIFHIN | DR sup | Pf LSA1 8 | 100 | |
| 15 | TVLLGGVGLVLYNTE | DR sup | Pf EXP1 90 | 100 | |
| 15 | VFFLALFFDFNKES | DR sup | Pf EXP1 6 | 100 | |
| 15 | VSTVLLGGVGLVLYN | DR sup | Pf EXP1 88 | 100 | |
| 15 | VVILTDGIPDSIQDS | DR sup | Pf SSP2 157 | 100 | |
| 20 | YADSAWENVKNVIGPFMKAV | DR sup | Pf.SSP2 | | |
| 15 | YKFVVPGAATPYAGE | DR sup | Pf SSP2 515 | 80 | |
| 15 | DQSYLQDSDPDSFQD | DR sup | Tyrosinase 448-462 (Q449) | | |
| 15 | DYSFLQDSDPDSFQD | DR sup | Tyrosinase 448-462 (F451) | | |
| 15 | DYSYFQDSDPDSFQD | DR sup | Tyrosinase 448-462 (F452) | | |
| 15 | DYSYLQDSDPDSFQD | DR sup | Tyrosinase448 | | |
| 15 | DYSYLQDSDPDSFQD | DR sup | Tyrosinase 448-462 | | |
| 15 | DYSYLQDSVPDSFQD | DR sup | Tyrosinase 448-462 (V456) | | |
| 15 | DYSYQQDSDPDSFQD | DR sup | Tyrosinase 448-462 (Q452) | | |
| 14 | NILLSNAPLGPQFP | DR sup | Tyrosinase 57-70 | | |
| 15 | QNFLLSNAPLGPQFP | DR sup | Tyrosinase 56-70 (F58) | | |
| 15 | QNIFLSNAPLGPQFP | DR sup | Tyrosinase 56-70 (F59) | | |
| 15 | QNILLSNAPLGPQFP | DR sup | Tyrosinase56 | | |
| 15 | QNILLSNAQLGPQFP | DR sup | Tyrosinase 56-70 (Q64) | | |
| 15 | QNILLSNAVLGPQFP | DR sup | Tyrosinase 56-70 (V64) | | |
| 15 | QNILLSNVPLGPQFP | DR sup | Tyrosinase 56-70 (V63) | | |
| 15 | QNILQSNAPLGPQFP | DR sup | Tyrosinase 56-70 (Q60) | | |
| 15 | QNILVSNAPLGPQFP | DR sup | Tyrosinase 56-70 (V60) | | |
| 15 | QNIQLSNAPLGPQFP | DR sup | Tyrosinase 56-70 (Q59) | | |
| 15 | QNIVLSNAPLGPQFP | DR sup | Tyrosinase 56-70 (V59) | | |
| 15 | QNVLLSNAPLGPQFP | DR sup | Tyrosinase 56-70 (V58) | | |
| 13 | SYLQDSDPDSFQD | DR sup | Tyrosinase 450-462 | | |
| 13 | SYLQDSVPDSFQD | DR sup | Tyrosinase 450-462 (V456) | | |
| 12 | YLQDSDPDSFQD | DR sup | Tyrosinase 451-462 | | |
| 14 | YSYLQDSDPDSFQD | DR sup | Tyrosinase 449-462 | | |

**Table 29b**

| Sequence | DR1 | DR2 w2§1 | DR2 w2§2 | DR4 w4 | DR4 w14 | DR4 w15 | DR5 w11 | DR5 w12 | DR6 w19 | DR7 | DR8 w2 | DR52a | DR9 | DR w53 | DR147 Cross-react. | Cross-react (4/7) | Cross-react (7/10) |
|---|---|---|---|---|---|---|---|---|---|---|---|---|---|---|---|---|---|
| FPQPQLPYSQP QPFRPQQPY | | | | | | | 0.001 | | | 0.0001 | | 0.0001 | | 4.6 | 0 | | |
| IPPYCTIAPFGIF GTN | | | | | | | 0.0001 | | | 0.17 | | 0.0001 | | 0.012 | 1 | | |
| LGQQQPFPPQQ PYPQPQPFP | | | | | | | 0.0001 | | | 0.0001 | | 0.0001 | | 0.246 | 0 | | |
| LHQQQKQQQQ PSSQVSFQQP | | | | | | | 0.0001 | | | 0.0001 | | 0.0001 | | 0.192 | 0 | | |
| LLQELCCQHL WQIPEQSQCQ | | | | | | | 0.0001 | | | 0.001 | | 0.001 | | 1.3 | 0 | | |
| LQQHNIAHGRS QVLQQSTYQ | | | | | | | | | | | | | | 0.111 | 0 | | |
| PQPFRPQQPYP QPQPQYSQP | | | | | | | 0.01 | | | 0.0001 | | 0.0001 | | 12.9 | 0 | | |
| PQPQPQYSQPQ QPISQQQQ | | | | | | | 0.0001 | | | 0.0001 | | 0.0001 | | 0.074 | 0 | | |
| PSSQVSFQQPL QQYPLGQGS | | | | | | | 0.002 | | | 0.0001 | | 0.0001 | | 8.5 | 0 | | |
| QFEEIRNLALQ TLPAMCNVY | | | | | | | 0.017 | | | 0.042 | | 0.0001 | | 1.2 | 1 | | |
| QGSVQPQQLPQ FEEIRNLAL | | | | | | | 0.015 | | | 0.16 | | 0.0001 | | 0.087 | 1 | | |
| QNPSQQQPQEQ VPLVQQQQF | | | | | | | 0.0001 | | | 0.0001 | | 0.0001 | | 0.297 | 0 | | |
| QPYPQPQPFPS QQPYLQLQP | | | | | | | 0.0001 | | | 0.0001 | | 0.0001 | | 1.9 | 0 | | |
| [?] QQLIFCMDVVL QQHNIAHGR | | | | | | | 0.001 | | | 0.002 | | 0.015 | | 1.5 | 0 | | |
| QVPLVQQQFL GQQQPFPPQ | | | | | | | | | | | | | | 5.5 | 0 | | |
| SQQPYLQLQPF PQPQLPYSQ | | | | | | | 0.102 | | | 0.006 | | 0.0001 | | 0.898 | 0 | | |
| VRVPVPQLQPQ NPSQQQPQE | | | | | | | 0.0001 | | | 0.0001 | | 0.0001 | | 7.4 | 0 | | |
| IRNLALQTLPA MCNVY | | | | | | | | | | | | | | 13 | 0 | | |
| PQPFRPQQPYP Q | | | | | | | | | | | | | | 25 | 0 | | |
| PQPFRPQQPYP QPQPQ | | | | | | | | | | | | | | 16 | 0 | | |
| QFEEIRNLALQ T | | | | | | | | | | | | | | 0.75 | 0 | | |
| QFEEIRNLALQ TLPAM | | | | | | | | | | | | | | 0.39 | 0 | | |
| QFLGQQQPFPP Q | | | | | | | | | | | | | | 0.86 | 0 | | |
| [?] QVPLVQQQFL G | | | | | | | | | | | | | | 86 | 0 | | |
| QVPLVQQQFL GQQQP | | | | | | | | | | | | | | 27 | 0 | | |
| VQQQFLGQQ QPFPPQ | | | | | | | | | | | | | | | 1 | | |
| aHAAHAAHAA HAAHAa | 0.019 | | 0.0001 | 0.0001 | 0.0001 | | 0.0001 | | | 0.0001 | | | | | 1 | | |
| CPKYVRSAKLR M . | 0.003 | | 0.007 | 0.0001 | 0.0001 | | 0.088 | | | 0.686 | | -0.006 | | | 1 | | |
| GACPKYVKQN TL | 0.0001 | | 0.0001 | 0.039 | | | -0.004 | | | 0.034 | | | | | 1 | | |
| GACPKYVKQN TLK | 0.0001 | | -0.019 | 0.012 | | | -0.004 | | | 1.2 | | | | | 1 | | |
| KQNTLKLATG MR | 0.0001 | | 0.002 | -0.003 | | | -0.004 | | | 0.031 | | | | | 1 | | |
| LAKQNTLAKQ NTLAKQNT | 0.0001 | | 1.5 | 0.3 | 0.58 | | -0.007 | | | 0.0001 | | 0.004 | | | 1 | | |
| PKAVKQNTLK LAT | 0.005 | | -0.018 | -0.003 | 0.002 | | -0.003 | | | 0.0001 | | | | | 1 | | |
| PKSVKQNTLKL AT | 0.0001 | | 0.001 | 0.78 | 0.0001 | | -0.002 | | | 0.0001 | | | | | 1 | | |
| PKYDKQGGLKI AT | 0.033 | | | -0.002 | 0.0001 | | | | | | | | | | 1 | | |
| PKYFKQFRLKI AT | 0.005 | | | 0.003 | -0.002 | | | | | | | | | | 1 | | |
| PKYGKQRFLKI AT | 0.001 | | | 0.05 | 0.005 | | | | | | | | | | 1 | | |
| PKYIKQIILKIA T | 0.004 | | | 0.09 | 0.12 | | | | | | | | | | 1 | | |
| PKYVKKNTLK LAT | 0.056 | | 0.172 | 0.015 | 0.0001 | | 0.187 | | | 0.007 | | | | | 1 | | |
| PKYVKQNKLK LAT | -0.002 | | 0.006 | -0.005 | 0.0001 | | 0.646 | | | 0.083 | | | | | 1 | | |
| PKYVKQNTKK LAT | 0.36 | | 0.373 | 0.021 | 0.0001 | | 1.2 | | | 0.018 | | | | | 1 | | |
| PKYVKQNTLK EAT | -0.002 | | 0.006 | -0.041 | 0.0001 | | 0.0001 | | | 0.029 | | | | | 1 | | |
| PKYVKQNTLK LAT | | | | 0.45 | | | | | | | | | | | 1 | | |
| PKYVKQNTLKL AT | 0.013 | | 0.0001 | 0.021 | | | 0.0001 | | | | | | | | 1 | | |
| PKYVKQNTLKI AT | 0.14 | | | 0.014 | | | | | | | | | | | 1 | | |
| PKYVKQNTIKL AT | 0.052 | | 0.005 | 0.028 | 0.0001 | | 0.003 | | | -0.004 | | 0.0001 | | | 1 | | |
| PKYVKQnTLKL AT | 0.015 | | | 0.04 | | | | | | | | | | | 1 | | |
| pKYVKQNTLKL AT | 0.013 | | | -0.004 | | | | | | | | | | | 1 | | |
| PKYVKQNTLK NAT | 0.0001 | | 0.027 | 0.031 | 0.0001 | | 0.01 | | | 0.016 | | | | | 0 | | |
| PKYVKQNTNK LAT | 0.071 | | 0.15 | 0.041 | 0.0001 | | 0.01 | | | 0.001 | | | | | 1 | | |
| RTLYQNVGTY VSVGTSTLNK | 0.26 | | -0.003 | 0.0001 | -0.002 | | -0.013 | | | 0.0001 | | -0.009 | | | 1 | | |
| VKQNTLKLAT GMR | 0.0001 | | 0.0001 | -0.006 | | | 0.026 | | | 0.0001 | | | | | 0 | | |
| YPKYVKRNTL KLAT | 0.21 | | | 0.023 | 0.004 | | | | | 0.003 | | | | | 1 | | |
| AAPFTQCGYPA LMPL | 0.034 | | 0.034 | -0.0016 | | | 0.0064 | | | 0.008 | | | | | 1 | 2 | |
| AFSYMDDVVL GAKSV | 0.0027 | | -0.0005 | 2.9 | 0.002 | | 0.0006 | | | -0.0003 | | | | -0.0005 | 1 | 1 | |
| AILCWGELMTL A | 0.0008 | 1 | | 0.0001 | | | | | | | | | | | 0 | | |
| ALRQAILCWGE LMTLA | 0.013 | 0.86 | | 0.0019 | | | | | | | | | | | 1 | | |
| ASARFSWLSLL VPFVQWFVG | 0.011 | | 0.001 | 0.029 | 0.002 | | 0.0001 | | | 0.012 | | 0.009 | | | 1 | | |
| DWKVCQRIVG LLGFA , | 0.012 | | -0.0026 | 0.003 | 0.011 | | 0.25 | | | 0.0018 | | | | 0.013 | 1 | 1 | |
| GAHLSLRGLPV CAFS | 0.78 | | 0.0042 | 0.0011 | 0.026 | | 0.0025 | | | 0.0077 | | | | 0.015 | 1 | 1 | |
| GYRWMCLRRF IIFLFILLLC | 0.002 | | 0.005 | 0.106 | 0.004 | | 0.005 | | | 0.011 | | -0.004 | | | 1 | | |
| HHTALRQAILC WGELMTLA | 0.008 | 0.31 | | 0.0002 | | | | | | | | | | | 1 | | |
| HLSLRGLPVCA FSSA | 1.3 | | | 0.0028 | | | | | | 0.013 | | | | | 1 | 1 | |
| HTALRQAILCW GELMTLA | 0.014 | 0.38 | | 0.0014 | | | | | | | | | | | 1 | | |
| ILCWGELMTLA | 0.0001 | 0.081 | | 0.0001 | | | | | | | | | | | 0 | | |
| IVGLLGFAAPF TQCG | 0.02 | | -0.0005 | -0.0007 | 0.0046 | | -0.0002 | | | 0.0009 | | | | 0.0067 | 1 | 1 | |
| LCQVFADATPT GWGL | 0.002 | | | 0.96 | | | | 0.011 | | 0.0013 | | 0.33 | | 0.078 | 1 | 1 | |
| LCWGELMTLA | 0.0001 | 0.029 | | 0.0001 | | | | | | | | | | | 0 | | |
| LRQAILCWGEL MTLA | 0.0089 | 1 | | 0.0002 | | | | | | | | | | | 1 | | |
| LSPTVWLSVIW MMWYWGPSL | 0.004 | | 0.003 | 0.076 | 0.005 | | 0.004 | | | 0.005 | | -0.004 | | | 1 | | |
| LSTLPETTVVR RRGRS | 0.0001 | | 0.001 | -0.005 | 0.002 | | 0.085 | | | -0.013 | | 0.008 | | | 0 | | |
| MDIDPYKEFGA SVELLSFL | 0.044 | | | 0.058 | 1.9 | | | | 0.0015 | 0.044 | | | | | 3 | | |
| MDIDPYKEFGA SVELLSFLPSDF FP | 0.15 | | | 0.018 | 0.46 | | | | -0.0002 | 0.15 | | | | | 2 | | |
| MDIDPYKEFGA TVELLSFL | 0.0037 | | | 0.17 | 0.46 | | | | 0.0009 | 0.003 | | | | | 1 | | |
| MDIDPYKEFGA TVELLSFLP | 0.0001 | | 0.0001 | 0.065 | 0.275 | | 0.002 | | | -0.017 | | 0.003 | | | 1 | | |
| MDIDPYKEFGA TVELLSFLPSDF FP | 0.0032 | | | 0.047 | | | | | 0.0018 | 0.004 | | | | | 1 | | |
| MDIDPYKEFGA TVELLSFLPSDF FP | 0.045 | | | 0.31 | 3 | | | | 0.0049 | 0.013 | | | | | 2 | | |
| MDIDPYKEFGA TVQLLSFLPSDF FP | 0.013 | | | 1.4 | 4.9 | | | | 0.0013 | 0.035 | | | | | 3 | | |
| NAPILSTLPETT VVR | 0.0009 | | 0.0009 | -0.0007 | 0.0054 | | -0.0002 | | | 0.0005 | | | | 0.16 | 0 | 0 | |
| PFLLAQFTSAIC SVVRRA | | | | | | | | | | | | 0.069 | | 0.15 | 0 | | |
| PHHTALRQAIL CW | 0.0003 | 0.045 | | 0.0094 | | | | | | | | | | | 0 | | |
| PHHTALRQAIL CWG | 0.0024 | 0.37 | | 0.0001 | | | | | | | | | | | 0 | | |
| PHHTALRQAIL CWGEL | 0.0021 | 0.045 | | 0.0003 | | | | | | | | | | | 0 | | |
| PHHTALRQAIL CWGELM | 0.03 | 0.067 | | 0.0082 | | | | | | | | | | | 1 | | |
| PHHTALRQAIL CWGELMT | 0.0031 | 0.075 | | 0.0055 | | | | | | | | | | | 0 | | |
| PHHTALRQAIL CWGELMTL | 0.039 | 0.54 | | 0.006 | | | | | | | | | | | 1 | | |
| PHHTALRQAIL CWGELMTLA | 0.0053 | 1.2 | | 0.001 | | | | | | | | | | | 1 | | |
| PLPIHTAELLAA CFA | 0.0046 | | | 0.049 | | | | | | -0.0003 | | | | | 1 | 1 | |
| PPAYRPPNAPIL STL | 0.0056 | | -0.0005 | 0.0038 | -0.0005 | | 0.0022 | | | 0.0024 | | | | 0.0015 | 1 | 1 | |
| PQAMQWNSTT FHQTL | 0.0012 | | | 0.03 | | | | | | 0.12 | | | | | 1 | 1 | |
| QAILCWGELM TLA | 0.0039 | 0.76 | | 0.0026 | | | | | | | | | | | 0 | | |
| QCGYPALMPL YACIQ | 0.0062 | | 0.0018 | 0.0068 | | | 0.0023 | | | 0.0006 | | | | | 1 | 1 | |
| RDLLDTASALY REAL | 0.0001 | | | 0.0092 | | | | | | 0.077 | | | | | 1 | 1 | |
| RDLLDTASALY RREALESPEH | 0.0001 | | 0.025 | -0.006 | 0.003 | | 0.0001 | | | 0.092 | | -0.003 | | | 1 | | |
| RDLVVSYVNT NMGLKFRQLL | 0.005 | | 0.003 | 0.02 | 0.017 | | 0.0001 | | | -0.017 | | 0.016 | | | 1 | | |
| RFSWLSLLVPF VQWF | 0.043 | | 0.0009 | -0.0007 | 0.0034 | | 0.0002 | | | 0.0005 | | | | 0.0031 | 1 | 1 | |
| RQAILCWGEL MTLA | 0.0016 | 1.3 | | 0.0002 | | | | | | | | | | | 0 | | |
| SLDSWWTSLNF LGGTTVCLG | 0.136 | | 0.0001 | 0.023 | 0.002 | | 0.001 | | | 0.006 | | -0.004 | | | 1 | | |
| SVRFSWLSLLV PFVQ | 0.9 | | | 0.0099 | | | | | | 0.0037 | | | | | 1 | 1 | |
| TALRQAILCWG ELMTLA | 0.0055 | 0.71 | | 0.0011 | | | | | | | | | | | 1 | | |
| TNLLSSNLSWL SLDV | 0.0016 | | -0.0005 | 0.13 | 0.023 | | 0.0006 | | | 0.0019 | | | | 0.041 | 1 | 1 | |
| TNMGLKFRQL LWFHI | -0.0005 | | | -0.0043 | 0.0081 | | | | 0.013 | 0.0022 | | | | | 0 | | |
| TNVGLKFRQLL WFHI | 0.0034 | | | 0.055 | 0.33 | | | | 0.0006 | 0.025 | | | | | 2 | | |
| TTVVRRRGRSP RRR | 0.0001 | 0.0007 | 0.041 | -0.0004 | 0.0001 | -0.0003 | 0.0075 | -0.005 | 0.0001 | 0.0001 | 0.018 | | -0.0007 | | 0 | | |
| VCAFSSAGPCA LRFT | 0.21 | | 0.26 | 0.0023 | 0.0048 | | 0.0003 | | | 0.02 | | | | 0.015 | 1 | 2 | |
| VSFGVWIRTPP AYRPPNAPI | | | | | | | | | | | | -0.012 | | 0.15 | 0 | | |
| YPALMPLYACI QSKQ | 0.24 | | | 0.0014 | | | | | | 0.0011 | | | | | 1 | 1 | |
| AEQFKQKALG LLQTA | 0.049 | | | 0.0006 | | | | | | 0.0058 | | | | | 1 | 1 | |
| ANLLWRQEMG GNITR | 0.7 | | | 0.0018 | | | | | | 0.0022 | | | | | 1 | 1 | |
| ARLIVFPDLGV RVCE | 0.0053 | | | 0.0017 | | | | | | 0.0004 | | | | | 1 | 1 | |
| ASQLSAPSLKA TCTT | 0.015 | | | 0.0056 | | | | | | 0.0006 | | | | | 1 | 1 | |
| AVQWMNRLIA FASRG | 2.2 | | | 0.0035 | | | | | | 0.0205 | | | | | 1 | 1 | |
| [?][?][?][?][?] DADLIEANLLW RQEM | 0.0088 | | | -0.001 | | | | | | 0.0025 | | | | | 1 | 1 | |
| EDLVNLLPAILS PGA | 0.37 | | | 0.011 | | | | | | 0.0015 | | | | | 1 | 1 | |
| GALVVGVVCA AILRR | 0.017 | | | 0.0067 | | | | | | 0.0043 | | | | | 1 | 1 | |
| GCSFSIFLLALL SCL | 0.006 | | | 0.0015 | | | | | | 0.003 | | | | | 1 | 1 | |
| GPGEGAVQWM NRLIAFASRG | 0.57 | | | 0.0084 | | | 0.23 | | | 0.004 | | | | | 1 | | |
| KPTLHGPTPLL YRLG . | 0.038 | | | 0.001 | | | | | | 0.0055 | | | | | 1 | 1 | |
| LAGYGAGVAG ALVAF | 0.041 | | | -0.0003 | | | | | | 0.0008 | | | | | 1 | 1 | |
| LHGLSAFSLHS YSPG | 1.6 | | | 0.0095 | | | | | | 0.007 | | | | | 1 | 1 | |
| LLFNILGGWVA AQLAAPGAA | 0.19 | | | -0.0035 | | | 0.0028 | | | 0.0004 | | | | | 1 | | |
| LTSMLTDPSHIT AET | 0.0004 | | | 0.074 | | | | | | -0.0003 | | | | | 1 | 1 | |
| NFISGIQYLAGL STLPGNPA | | | | | | | | 0.18 | | | | -0.012 | | 1.6 | 0 | | |
| PAILSPGALVV GVVCA | 0.11 | | | 0.0007 | | | | | | 0.0076 | | | | | 1 | 1 | |
| PQTFQVAHLHA PTGS | 0.24 | | | 0.0053 | | | | | | -0.0003 | | | | | 1 | 1 | |
| PTLWARMILM THFFS | 0.0064 | | | 0.02 | | | | | | 0.019 | | | | | 1 | 1 | |
| RAAVCTRGVA KAVDF | 0.01 | | | 0.0077 | | | | | | 0.0024 | | | | | 1 | 1 | |
| TVDFSLDPTFTI ETT | 0.0001 | | | 0.16 | | | | | | 0.0005 | | | | | 1 | 1 | |
| VVLLFLLLADA RVCS | 0.024 | | | 0.012 | | | | | | 0.0033 | | | | | 1 | 1 | |
| WESVFTGLTHI DAHF | 0.031 | | | 0.0068 | | | | | | 0.0005 | | | | | 1 | 1 | |
| AFVAWRNRCK | | | 1.75 | | | | | | | | | | | | 0 | | |
| AFVAWRNRCK AWAAWRNRC K | | | 0.18 | | | | | | | | | | | | 0 | | |
| AWEAWRNRCK | | | 0.15 | | | | | | | | | | | | 0 | | |
| AWLAWRNRCK | | | 0.46 | | | | | | | | | | | | 0 | | |
| AWVAARNRCK | | | 4.4 | | | | | | | | | | | | 0 | | |
| AWVAFRNRCK | | | 1.8 | | | | | | | | | | | | 0 | | |
| AWVAQRNRCK | | | 0.9 | | | | | | | | | | | | 0 | | |
| AWVAWANRC K [?] | | | 0.35 | | | | | | | | | | | | 0 | | |
| AWVAWENRC K | | | 0.16 | | | | | | | | | | | | 0 | | |
| AWVAWKNRC K . | | | 0.82 | | | | | | | | | | | | 0 | | |
| AWVAWRARC K | | | 1.2 | | | | | | | | | | | | 0 | | |
| AWVAWRNAC K | | | 0.67 | | | | | | | | | | | | 0 | | |
| AWVAWRNEC K | | | 0.17 | | | | | | | | | | | | 0 | | |
| AWVAWRNKC K | | | 0.25 | | | | | | | | | | | | 0 | | |
| AWVAWRNRC A | | | 0.021 | | | | | | | | | | | | 0 | | |
| AWVAWRNRC K | 0.0001 | | 0.45 | 0.012 | 0.0001 | | -0.004 | | | 0.0001 | | -0.009 | | | 0 | | |
| AWVAWRNRC K | 0.0001 | | 0.45 | 0.012 | 0.0001 | | -0.004 | | | 0.0001 | | -0.009 | | | 0 | | |
| AWVAWRNRC R | | | 0.105 | | | | | | | | | | | | 0 | | |
| AWVAWRNRE K | | | 0.85 | | | | | | | | | | | | 0 | | |
| AWVAWRNRK K | | | 2.8 | | | | | | | | | | | | 0 | | |
| AWVAWRNRQ K | | | 4.6 | | | | | | | | | | | | 0 | | |
| AWVAWRNRV K | | | 2.7 | | | | | | | | | | | | 0 | | |
| AWVAWRVRC K | | | 0.056 | | | | | | | | | | | | 0 | | |
| AWVEWRNRCK | | | 0.073 | | | | | | | | | | | | 0 | | |
| AWVSWRNRCK | | | 0.25 | | | | | | | | | | | | 0 | | |
| AWVVWRNRC K | | | 0.2 | | | | | | | | | | | | 0 | | |
| EFVAAKAAQK | 0.008 | | 6.7 | 0.004 | 0.0001 | | -0.016 | | | 0.0001 | | -0.002 | | | 1 | | |
| EWVAWRNRCK | | | 0.48 | | | | | | | | | | | | 0 | | |
| LAAAMKRHGL DNYRGYSLGN WV | -0.004 | | 0.051 | -0.002 | 0.0001 | | 0.24 | | | 0.005 | | -0.019 | | | 0 | | |
| NTDGSTDYGIL QINSR | 0.007 | | -0.003 | 0.01 | 0.013 | | -0.007 | | | -0.003 | | -0.014 | | | 1 | | |
| RNRCKGTDVQ AWIRGCRL | 0.01 | | 0.051 | -0.002 | 0.046 | | -0.008 | | | 0.007 | | -0.016 | | | 1 | | |
| SVNCAKKIVSD GNGMN | 0.0001 | | -0.002 | 0.04 | 0.006 | | 0.03 | | | -0.003 | | 0.044 | | | 0 | | |
| SWVAWRNRCK | | | 0.42 | | | | | | | | | | | | 0 | | |
| VWVAWRNRC K | | | 0.19 | | | | | | | | | | | | 0 | | |
| WRNAKWRNA KWRNAK | 0.005 | | 0.018 | -0.002 | 0.0001 | | 0.004 | | | 0.0001 | | -0.007 | | | 1 | | |
| YRGYSLGNWV CAAKFESNFNT Q | 0.009 | | 0.009 | -0.002 | 0.0001 | | 0.004 | | | -0.004 | | -0.019 | | | 1 | | |
| SPYVSRLLGICL T | 0.41 | | | -0.0035 | 0.32 | | | | 0.0008 | 0.022 | | 0.014 | | 0.098 | 1 | | |
| ASDFNLPPVVA KEIV | 0.0026 | | -0.0021 | -0.0028 | | | -0.0006 | | | 0.084 | | | | | 1 | 1 | |
| AVQMAVFIHNF KRKG | 0.0032 | | 2.6 | 0.032 | | | 0.3 | | | 0.0096 | | | | | 0 | 1 | |
| DFNLPPVVAKE IVAS | 0.0042 | | -0.0021 | -0.0024 | | | 0.0036 | | | 0.053 | | | | | 1 | 1 | |
| DQQLLGIWGCS GKLI | 0.029 | | 0.015 | -0.0016 | | | 0.0073 | | | 0.018 | | | | | 1 | 1 | |
| DQSLKPCVKLT PLCV | 0.0076 | | 0.0018 | -0.0016 | | | 0.005 | | | 0.011 | | | | | 1 | 1 | |
| DRVHPVHAGPI A | 0.0006 | 0.0005 | -0.0005 | -0.0027 | -0.0009 | | -0.0001 | | 0.0066 | 0.27 | -0.0014 | | | | 1 | | |
| DRVHPVHAGPI APG | 0.023 | 0.013 | 0.0019 | -0.0031 | -0.0011 | | -0.0001 | | 0.01 | 0.28 | -0.0017 | | | | 2 | | |
| DRVHPVHAGPI APGQM | 0.0066 | 0.0083 | 0.011 | -0.0036 | -0.0012 | | -0.0002 | | 0.0055 | 0.14 | -0.0019 | | | | 2 | | |
| DRVHPVHAGPI APGQMRE | 0.012 | 0.013 | 0.0051 | -0.004 | -0.0014 | | -0.0002 | | 0.0059 | 0.064 | 0.0034 | | | | 2 | | |
| DRVHPVHAGPI APGQMREPR | 0.0045 | 0.0026 | -0.0008 | -0.0045 | -0.0016 | | -0.0002 | | 0.0029 | 0.12 | -0.0024 | | | | 1 | | |
| DRVHPVHAGPI APGQMREPRGS | 0.021 | 0.0053 | 0.0014 | 0.0049 | -0.0017 | | -0.0002 | | 0.0055 | 0.24 | -0.0027 | | | | 2 | | |
| DTEVHNVWAT QACVPTDPNP | 0.0073 | | -0.0018 | 0.0027 | 0.016 | | -0.0006 | | 0.0006 | 0.0065 | 0.0007 | -0.0027 | -0.0005 | 0.011 | 1 | | |
| EAIIRILQQLLFI HF | 0.047 | | -0.0021 | 0.033 | | | 0.016 | | | 0.023 | | | | | 1 | 1 | |
| EDIISLWDQSL KPCV | 0.052 | | 0.0017 | -0.0016 | | | -0.001 | | | -0.0007 | | | | | 1 | 1 | |
| EKAFSPEVIPMF SAL | 0.0086 | | 0.0015 | 0.034 | | | -0.001 | | | 0.0023 | | | | | 1 | 1 | |
| EKVYLAWVPA HKGIG | | | | | | | | | | | | 0.0052 | | 3.5 | 0 | | |
| ERFAVNPGLLE TSEGC | 0.02 | | -0.007 | 0.0001 | 0.007 | | 0.008 | | | 0.0001 | | -0.003 | | | 1 | | |
| ERYLKDQQLL GIWGCS | 0.0018 | | -0.0004 | 0.089 | -0.0005 | | 0.004 | | | -0.0005 | | | | 0.18 | 1 | 1 | |
| ESELVSQIIEQLI KK | 0.0059 | | 0.021 | 0.0095 | | | 0.0009 | | | 0.004 | | | | | 1 | 1 | |
| ETAYFLLKLAG RWPV | 0.061 | | | 0.021 | | | | | | 0.0041 | | | | | 1 | 1 | |
| EVQLGIPHPAG LKKK | 0.002 | | 0.13 | -0.0026 | | | -0.0007 | | | -0.0005 | | | | | 0 | 1 | |
| FWEVQLGIPHP AGLK | 0.024 | | -0.0014 | 0.0033 | | | -0.0006 | | | 0.0024 | | | | | 1 | 1 | |
| GARASVLSGGE LDKWE | 0.006 | | -0.007 | 0.0001 | 0.0001 | | -0.002 | | | 0.0001 | | -0.007 | | | 1 | | |
| GCTLNFPISPIE TVP | 0.0014 | | -0.0014 | -0.0026 | | | -0.0006 | | | 0.038 | | | | | 1 | 1 | |
| GEIYKRWIILGL NKI | | | | | | | | | | | | 0.066 | | 0.16 | 0 | | |
| GGELDKWEKIR LRPGG | -0.002 | | 0.036 | 0.0001 | 0.0001 | | -0.002 | | | 0.0001 | | -0.003 | | | 0 | | |
| HKAIGTVLVGP TPVN | 0.0072 | | -0.0013 | -0.0022 | | | -0.001 | | | 0.0016 | | | | | 1 | 1 | |
| IGGIGGFIKVRQ YDQ | 0.0002 | | 0.2 | -0.0023 | | | 0.015 | | | 0.0031 | | | | | 0 | 1 | |
| IIRILQQLLFIHF RI | 0.0054 | | | 0.02 | | | | | | 0.0084 | | | | | 1 | 1 | |
| IISLWDQSLKPC VKL | 0.0057 | | 0.0061 | 0.0096 | | | 0.0059 | | | 0.0012 | | | | | 1 | 1 | |
| IKVVPRRKAKII RDY | 0.0003 | | 0.07 | -0.0024 | | | 2.5 | | | 0.003 | | | | | 0 | 1 | |
| ILKALGPAATL EEMMT(200.11) | 0.226 | | 0.0001 | 0.002 | 0.032 | | 0.0001 | | | 0.001 | | 0.0001 | | | 1 | | |
| INEEAAEWERV HPVHA | 0.008 | | -0.007 | 0.0001 | 0.005 | | 0.002 | | | 0.0001 | | 0.006 | | | 1 | | |
| IQKLVGKLNW ASQIY | 0.026 | | -0.0014 | -0.0026 | | | 0.0044 | | | 0.0043 | | | | | 1 | 1 | |
| ISLWDQSLKPC VKLT | 0.004 | | 0.029 | -0.0016 | | | 0.0024 | | | 0.0012 | | | | | 0 | 1 | |
| IVWASRELERF AVNPG | 0.002 | | -0.007 | 0.0001 | 0.013 | | 0.059 | | | 0.0001 | | -0.003 | | | 0 | | |
| IYKRWIILGLN KIVRMYSPV | 0.0044 | | -0.0009 | 0.0067 | 0.01 | | 0.0022 | | 0.0004 | 0.041 | 0.32 | 0.0073 | 0.05 | 0.185 | 1 | | |
| IYKRWIILGLN KIVRN | -0.002 | | 0.025 | 0.0001 | 0.0001 | | -0.002 | | | 0.0001 | | -0.003 | | | 0 | | |
| KARVLAEAMS QVTNS | 0.008 | | | 0.012 | | | | | | 0.0025 | | | | | 1 | 1 | |
| KDSWTVNDIQ KLVGK | 0.0027 | | -0.0014 | -0.0026 | | | 0.12 | | | -0.0005 | | | | | 0 | 0 | |
| KQIINMWQEV GKAMYA | 0.003 | | 0.0036 | 0.021 | 0.51 | | 0.0009. | | | 0.0027 | | | | 0.96 | 0 | 0 | |
| KRWIILGLNKI VRMY | | | | | | | | | | | | 0.18 | | 0.4 | 0 | | |
| KTILKALGPAA TLEE | 0.076 | | 0.01 | 0.0023 | | | -0.001 | | | 0.0006 | | | | | 1 | 1 | |
| LGKIWPSYKGR PGNFL | -0.002 | | -0.007 | 0.005 | 0.33 | | 0.072 | | | 0.0001 | | 0.005 | | | 0 | | |
| LICTTAVPWNA SWSNK | 0.0036 | | 0.0004 | 0.0012 | 0.0011 | | -0.0003 | | | 0.052 | | | | 0.0013 | 1 | 1 | |
| LKQIVKKLREQ FGNNK | 0.0003 | | 0.044 | -0.0006 | 0.0008 | | 0.0051 | | | -0.0005 | | | | 0.11 | 0 | 1 | |
| LLGIWGCSGKL ICTT | 0.028 | | 0.005 | 0.0016 | | | .0014 | | | 0.0064 | | | | | 1 | 1 | |
| LSIVNRVRQGY SPLS | 0.0045 | | 0.032 | 0.018 | | | 0.72 | | | 0.0015 | | | | | 0 | 1 | |
| NEQVDKLVSA GIRKV | 0.0024 | | 0.59 | -0.0026 | | | -0.0006 | | | 0.0028 | | | | | 0 | 1 | |
| NNLLRAIEAQQ HLLQ | 0.028 | | | 0.015 | | | | | | 0.015 | | | | | 1 | 1 | |
| PAGLKKKKSVT VLDV | 0.006 | | -0.0014 | -0.0026 | | | -0.0006 | | | 0.014 | | | | | 1 | 1 | |
| PGNFLQSRPEP TAPP | 0.097 | | 0.017 | 0.019 | | | 0.0015 | | | 0.013 | | | | | 1 | 1 | |
| PIVQNLQGQM VHQAIS | 0.008 | | -0.007 | 0.0001 | 0.0001 | | -0.002 | | | 0.0001 | | -0.003 | | | 1 | | |
| PLGVAPTKAKR RVVQR | 0.0001 | | 0.48 | -0.0006 | 0.004 | | 0.0017 | | | 0.0007 | | | | 0.025 | 0 | 1 | |
| QARILAVERYL KDQQL | 0.0056 | | 0.019 | -0.0006 | 0.0058 | | 0.033 | | | 0.001 | | | | 0.26 | 1 | 1 | |
| QGQMVHQAISP RTLN | | | | | | | | | | | | -0.0047 | | 1.2 | 0 | | |
| QKQEPIDKELY PLTSL, | 0.04 | | -0.007 | 0.0001 | 0.2 | | -0.002 , | | | 0.0001 | | -0.003 | | | 1 | | |
| QMAVFIHNFKR KGGI | 0.0005 | | 1.2 | 0.0031 | | | 1.1 | | | 0.0037 | | | | | 0 | 1 | |
| RIQRGPGRAFV TIGKL | 0.0004 | | 0.0083 | 0.0006 | -0.0005 | | 0.085 | | | -0.0005 | | | | 0.015 | 0 | 0 | |
| RQILGQLQPSL QTGSE | 0.022 | | -0.007 | 0.006 | 0.05 | | -0.002 | | | 0.0001 | | -0.003 | | | 1 | | |
| SLKPCVKLTPL CVTLN | 0.018 | | 0.012 | 0.003 | 0.013 | | 0.047 | | | 0.023 | | | | 0.12 | 1 | 1 | |
| SLWDQSLKPCV KLTPL | 0.0011 | | 0.022 | -0.0006 | 0.0005 | | 0.0047 | | | -0.0005 | | | | 0.016 | 0 | 0 | |
| SPAIFQSSMTKI LEP | | | | | | | | | | | | 1 | | 0.0025 | 0 | | |
| SPGIWQLDCTH LEGK | 0.0013 | | -0.0021 | 0.099 | | | -0.0006 | | | -0.0009 | | | | | 1 | 1 | |
| SQIIEQLIKKEK VYL | 0.0006 | | 0.031 | -0.0026 | | | | 0.052 | | 0.0005 | | | | | 0 | 1 | |
| SSGGDPEIVMH SFNCG : | 0.0001 | | -0.0004 | -0.0006 | -0.0005 | | -0.0003 | | | -0.0005 | | | | 0.12 | 0 | 0 | |
| TAATNAACAW LEA | 0.0005 | | -0.0029 | -0.015 | 0.0006 | | -0.0004 | | 0.0036 | 0.0015 | -0.0006 | 0.0011 | 0.046 | 0.0012 | 0 | | |
| TITLPCRIKQFI NMWQE | 0.0002 | | 0.0011 | 0.03 | 0.061 | | 0.0005 | | | 0.0011 | | | | 0.08 | 0 | 0 | |
| VDKLVSAGIRK VLFL | 0.0039 | | 0.15 | -0.0026 | | | 0.0045 | | | 0.012 | | | | | 0 | 1 | |
| VIPMFSALSEG ATPQ | 0.0085 | | 0.0014 | 0.0058 | | | -0.001 | | | -0.0007 | | | | | 1 | 1 | |
| VKIEPLGVAPT KAKRR | 0.0028 | | 1.5 | 0.001 | 0.074 | | -0.0003 | | | -0.0005 | | | | 0.01 | 0 | 1 | |
| VLSIVNRVRQG YSPL | 0.0031 1 | | 1.7 | 0.026 | | | 5.9 | | | 0.0092 | | | | | 0 | 1 | |
| VNIIGRNLLTQI GCT | 0.0027 | | -0.0014 | 0.062 | | | 0.0067 | | | 0.0012 | | | | | 1 | 1 | |
| VNIVTDSQYAL GIIQ | 0.062 | | -0.0014 | 0.0091 | | | -0.0007 | | | 0.018 | | | | | 1 | 1 | |
| VTVYYGVPVW KEATT | 0.0087 | | 0.027 | 0.0071 | | | 0.0021 | | | 0.016 | | | | | 1 | 2 | |
| VWGIKQLQAR VLAVERYLKD | 2.2 | | 0.54 | 0.013 | 0.047 | | 0.041 | | 0.026 | 0.13 | 0.064 | 0.018 | 0.034 | 0239 | 2 | | |
| WDQSLKPCVK LTPLCV | 0.0083 | | 0.0029 | 0.0006 | -0.0005 | | 0.0011 | | | 0.0081 | | | | 0.013 | 1 | 1 | |
| WGCSGKLICTT AVPWN . | 0.0001 | | -0.0004 | 0.0033 | -0.0005 | | -0.0003 | | | 0.031 | | | | -0.001 | 1 | 1 | |
| WRKLVDFREL NKRTQ | 0.0011 | | 0.0017 | -0.0026 | | | 0.29 | | | -0.0005 | | | | | 0 | 0 | |
| VYVWKTTWG QYWQVLGGPV S | 0.044 | | | -0.0063 | 0.0018 | | | | 0.0003 | 0.0623 | | | | -0.0016 | 2 | | |
| ALHIYMNGTM SQVQGSA | 0.014 | | | 0.026 | 0.29 | | | | 0.056 | -0.001 | | | | -0.0008 | 1 | | |
| WPSVFYNRTC QCSGNF | 0.0002 | | | -0.0048 | 0.0014 | | | | 0.009 | 0.0017 | | | | -0.0012 | 0 | | |
| YGQMKNGSTP MFNDINIYDL | 0.005 | | | 0.032 | 0.052 | | | | 0.021 | 0.021 | | | | 0.0092 | 1 | | |
| DIVIYSKYGGT EIKY | | 0.01 | 0.0031 | -0.0006 | | | -0.0002 | | | | | | | -0.0006 | 0 | | |
| IKYNGEEYLILS ARD | | 0.031 | 0.014 | 0.0016 | | | 0.0056 | | | | | | | 0.037 | 0 | | |
| IYSKYGGTEIK YNGE | | 0.0026 | 0.045 | 0.0036 | | | -0.0002 | | | | | | | -0.0008 | 0 | | |
| LVIPENAKEKP QEGT | | 0.0001 | 0.16 | -0.0006 | | | 0.0003 | | | | | | | -0.0006 | 0 | | |
| NGEEYLILSAR DVLA | | 0.0017 | 0.034 | 0.049 | | | 0.0037 | | | | | | | 0.039 | 1 | | |
| PSGLVIPENAK EKPQ | | 0.0001 | 0.17 | -0.0006 | | | -0.0002 | | | | | | | -0.0008 | 0 | | |
| RIPVDVSEGDIV IYS | | 0.64 | -0.0003 | 0.001 | | | -0.0002 | | | | | | | -0.0008 | 0 | | |
| SEGDIVIYSKY GGTE | | 0.064 | 0.0006 | -0.0006 | | | 0.0058 | | | | | | | -0.0006 | 0 | | |
| VAKVKIKPLED KILV | | 0.055 | 0.0057 | 0.0008 | | | 0.006 | | | | | | | 0.011 | 0 | | |
| VKIKPLEDKILV QAG | | 0.023 | 0.0008 | 0.0025 | | | 0.0088 | | | | | | | 0.14 | 0 | | |
| LQLVFGIEVVE VVPI : | 0.1 | | | 0.033 | | | | | | 0.27 | | | | | 2 | | |
| PRKLLMQDLV QENYL | 0.0009 | | | 0.16 | | | | | | -0.0019 | | | | | 1 | | |
| QDFFPVIFSKAS EYL | 0.011 | | | -0.0019 | | | | | | 0.27 | | | | | 2 | | |
| QLVFGIEVVEV VPIS | 0.017 | | | 0.018 | | | | | | 0.283 | | | | | 2 | | |
| RALIETSYVKV LHHT | 0.022 | | | 0.075 | | | | | | 0.13 | | | | | 3 | | |
| RKLLMQDLVQ ENYLE | -0.0003 | | | 0.14 | | | | | | -0.0019 | | | | | 1 | | |
| SHLYILVTCLG LSYD | 0.015 | | | 0.022 | | | | | | 0.0036 | | | | | 1 | | |
| YEFLWGPRALI ETSY | 1.8 | | | 0.0059 | | | | | | 0.011 | | | | | 1 | | |
| GEALGLVGAQ APATE | 0.082 | | | -0.0019 | | | | | | -0.0019 | | | | | 1 | | |
| FFPVIFSKASSS LQL | 0.048 | | | 0.12 | | | | | | 0.63 | | | | | 3 | | |
| FPVIFSKASSSL QLV | 0.026 | | | 0.049 QLV | | | | | | 0.21 | | | | | 3 | | |
| GIELMEVDPIG HLYI | 0.0094 | | | 0.028 | | | | | | 0.0049 | | | | | 1 | | |
| QAALSRKVAEL VHFL | 0.03 | | | -0.0019 | | | | | | 0.0046 | | | | | 1 | | |
| QYFFPVIFSKAS SSL | 0.099 | | | 0.18 | | | | | | 0.22 | | | | | 3 | | |
| RALVETSYVKV LHHM | 0.021 | | | 0.38 | | | | | | 0.23 | | | | | 3 | | |
| VDPIGHLYIFAT CLG | 0.0093 | | | 0.0043 | | | | | | 0.0073 | | | | | 1 | | |
| VFGIELMEVDPI GHL | 0.0039 | | | 0.057 | | | | | | -0.0019 | | | | | 1 | | |
| VGNWQYFFPVI FSKA | 0.14 | | | 2.1 | | | | | | 0.26 | | | | | 3 | | |
| YEFLWGPRAL VETSY | 3.7 | | | 0.0051 | | | | | | 0.0061 | | | | | 1 | | |
| VGNWQYFFPVI FSKA | 0.014 | | | 0.034 | 0.028 | | | | 0.0002 | 0.0076 | | | | -0.0012 | 1 | | |
| MVKISGGPRIS YPLLHEWALRE GEB | 0.018 | | | 0.0075 | 0.0034 | | | | 0.35 | 0.29 | | | | -0.0019 | 2 | | |
| QVPGSDPACYE FLWGPRALIET SY | 0.15 | | | -0.0075 | -0.0005 | | | | -0.0003 | -0.0011 | | | | 0.0021 | 1 | | |
| ANPVVHFFKNI VTPR | | 7.2 | 0.07 | | | | | | | | | | | | 0 | | |
| ASQKRPSQRHG SKYLATAST | 0.0001 | | 0.0001 | 0.019 | 0.001 | | 0.0001 | | | 0.028 | | | | | 1 | | |
| AYDAQGTLSKI FKLGGRDSR | 0.172 | | 0.201 | 0.017 | 0.212 | | 0.535 | | | 0.012 | | | | | 1 | | |
| DENPVVHFFKN | | 0.012 | 0 | | | | | | | | | | | | 0 | | |
| DENPVVHFFKN I . | | 0.55 | 0.005 | | | | | | | | | | | | 0 | | |
| DENPVVHFFKN IV | | 1.4 | 0.011 | | | | | | | | | | | | 0 | | |
| DENPVVHFFKN IVT | | 2.5 | 0.029 | | | | | | | | | | | | 0 | | |
| DENPVVHFFKN IVTPR | | 0.86 | 0.014 | | | | | | | | | | | | 0 | | |
| DENPVVHFFKN IVTPRT | | 0.69 | 0.007 | | | | | | | | | | | | 0 | | |
| [?] DENPVVHFFKN IVTP,RTPP . | | 2.8 | 0.42 | | | | | | | | | | | | 0 | | |
| DENPVVHFFKN IVTPRTPPY | | 0.76 | | | | | | | | | | | | | 0 | | |
| DENPVVHFFRN IVTPRTPPY | | 6.6 | 1.1 | | | | | | | | | | | | 0 | | |
| EAPVVHFFKNI VTPR | | 3.3 | 0.032 | | | | | | | | | | | | 0 | | |
| ENAVVHFFKNI VTPR | | 2 | 0.048 | | | | | | | | | | | | 0 | | |
| ENPAVHFFKNI VTPR | | 5.7 | 0.053 | | | | | | | | | | | | 0 | | |
| ENPKVHFFKNI VTPR | | 1.3 | | | | | | | | | | | | | 0 | | |
| ENPVAHFFKNI VTPR | | 0.065 | 0.042 | | | | | | | | | | | | 0 | | |
| ENPVKHFFKNI VTPR | | 0.023 | | | | | | | | | | | | | 0 | | |
| ENPVVAFFKNI VTPR | | 3.3 | 0.068 | | | | | | | | | | | | 0 | | |
| ENPVVAFFKNI VTPR | | 1.8 | | | | | | | | | | | | | 0 | | |
| ENPVVDFFKNI VTPR | | 0.12 | | | | | | | | | | | | | 0 | | |
| ENPVVFFFKNI VTPR | | 4.5 | | | | | | | | | | | | | 0 | | |
| ENPVVHAFKNI VTPR | | 4 | 0.022 | | | | | | | | | | | | 0 | | |
| ENPVVHAFKNI VTPR | | 4.3 | | | | | | | | | | | | | 0 | | |
| ENPVVHAFRNI VTPR | | 0.47 | | | | | | | | | | | | | 0 | | |
| ENPVVHDFKNI VTPR | | 0.022 | | | | | | | | | | | | | 0 | | |
| ENPVVHFAKNI VTPR | | 0.056 | 0.002 | | | | | | | | | | | | 0 | | |
| ENPVVHFARNI VTPR | | 0.0099 | | | | | | | | | | | | | 0 | | |
| ENPVVHFFANI VTPR | | 3.6 | 0.005 | | | | | | | | | | | | 0 | | |
| ENPVVHFFANI VTPR | | 2.6 | | | | | | | | | | | | | 0 | | |
| ENPVVHFFANI VTPRTP | | | 0.072 | | 5.8 | | | | | | | | | | 0 | | |
| ENPVVHFFDNI VTPR : | | 0.027 | | | | | | | | | | | | | 0 | | |
| ENPVVHFFHNI VTPR | | 1.5 | | | | | | | | | | | | | 0 | | |
| ENPVVHFFKAI VTPR | | 2.6 | 0.1 | | | | | | | | | | | | 0 | | |
| ENPVVHFFKAI VTPRTP | | | 0.73 | | 4.4 | | | | | | | | | | 0 | | |
| ENPVVHFFKKI VTPR | | 0.014 | | | | | | | | | | | | | 0 | | |
| ENPVVHFFKNA VTPR | | 3.8 | 0.013 | | | | | | | | | | | | 0 | | |
| ENPVVHFFKNA VTPRTP | | | 0.11 | | 4.8 | | | | | | | | | | 0 | | |
| ENPVVHFFKNI ATPR | | 2.3 | 0.022 | | | | | | | | | | | | 0 | | |
| ENPVVHFFKNI VAPR | | 13 | 0.31 | | | | | | | | | | | | 0 | | |
| ENPVVHFFKNI VAPRTP | | | 0.57 | | 3.9 | | | | | | | | | | 0 | | |
| ENPVVHFFKNI VTAR | | 9.5 | 0.046 | | | | | | | | | | | | 0 | | |
| ENPVVHFFKNI VTPA | | 4.3 | 0.007 | | | | | | | | | | | | 0 | | |
| [?] ENPVVHFFKNI VTPA | | 1.4 | 0.001 | | | | | | | | | | | | 0 | | |
| ENPVVHFFKNI VTPR | | 2.2 | 0.045 | | | | | | | | | | | | 0 | | |
| ENPVVHFFKNI VTPRTP | | | 0.21 | | 5 | | | | | | | | | | 0 | | |
| ENPVVHFFKNI VTPRTPPY | | 1.1 | 0.036 | | | | | | | | | | | | 0 | | |
| ENPVVHFFKNK VTPR | | 0.53 | | | | | | | | | | | | | 0 | | |
| ENPVVHFFLNI VTPR | | 3.2 | | | | | | | | | | | | | 0 | | |
| ENPVVHFFRNI VTPR | | 4.7 | | | | | | | | | | | | | 0 | | |
| ENPVVHFKKNI VTPR | | 0.028 | | | | | | | | | | | | | 0 | | |
| ENPVVHHFKNI VTPR | | 1.2 | | | | | | | | | | | | | 0 | | |
| ENPVVHHFRNI VTPR | | 0.59 | | | | | | | | | | | | | 0 | | |
| ENPVVHLFKNI VTPR | | 1.2 | | | | | | | | | | | | | 0 | | |
| ENPVVHLFRNI VTPR | | 1.7 | | | | | | | | | | | | | 0 | | |
| ENPVVHWFKNI VTPR | | 0.47 | | | | | | | | | | | | | 0 | | |
| ENPVVHYFANI VTPR | | 0.5 | | | | | | | | | | | | | 0 | | |
| ENPVVHYFHNI VTPR | | 0.71 | | | | | | | | | | | | | 0 | | |
| ENPVVHYFKNI VTPR | | 4.3 | | | | | | | | | | | | | 0 | | |
| ENPVVHYFLNI VTPR | | 0.52 | | | | | | | | | | | | | 0 | | |
| ENPVVHYFRNI VTPR | | 0.41 | | | | | | | | | | | | | 0 | | |
| ENPVVKFFKNI VTPR | | 1.3 | | | | | | | | | | | | | 0 | | |
| FFKNIVTPFFKN IVTP | 0.006 | | | 0.009 | 0.008 | | | | | 0.011 | | | | | 1 | | |
| GFGYGGRASD YKSAHKGFKG | 0.0001 | | 0.147 | 0.003 | 0.0001 | | 0.144 | | | 0.0001 | | | | | 0 | | |
| HFFKNIVTPRTP PY | | 0.02 | 0.056 | | | | | | | | | | | | 0 | | |
| HFFKNIVTPRTP PY | | 0.005 | 0.038 | | | | | | | | | | | | 0 | | |
| IFKLGGRDSRS GSPMARR | 0.171 | | 0.009 | 0.002 | 0.0001 | | 0.0025 | | | 0.0001 | | | | | 1 | | |
| NPVVHFFKNIV T | | 0.39 | 0.002 | | | | | | | | | | | | 0 | | |
| NPVVHFFKNIV TPA | | 1.5 | 0.0001 | | | | | | | | | | | | 0 | | |
| [?] NPVVHFFKNIV TPR | | 2.6 | 0.011 | | | | | | | | | | | | 0 | | |
| NPVVHFFKNIV TPRTPPY | | 1.3 | 0.17 | | | | | | | | | | | | 0 | | |
| PVVHFFKNIVT | | 0.028 | 0.0001 | | | | | | | | | | | | 0 | | |
| PVVHFFKNIVT PA | | 0.91 | 0.001 | | | | | | | | | | | | 0 | | |
| PVVHFFKNIVT PR | | 0.54 | 0.003 | | | | | | | | | | | | 0 | | |
| PVVHFFKNIVT PRTPPY [?] | | 3.8 | 0.48 | | | | | | | | | | | | 0 | | |
| QKSHGRTQDE NPVVHFFKNI | 0.004 | | 0.007 | 0.05 | 0.032 | | 0.0015 | | | 0.003 | | | | | 1 | | |
| RASDYKSAHK GFKGVDAQGT | | | | | | | 0.053 | | | | | | | | 0 | | |
| RASDYKSAHK GLKGHDAQGT | | | | | | | 0.099 | | | | | | | | 0 | | |
| RFFSGDRGAPK RGSGKDSHT | 0.0001 | | 0.01 | 0.158 | 0.0001 | | 0.0001 | | | 0.0001 | | | | | 1 | | |
| VDAQGTLSKIF KLGGRDSRS | | | | | | | 0.53 | | | | | | | | 0 | | |
| VDAQGTLSKLF KLGGRDSRS | | | | | | | 0.35 | | | | | | | | 0 | | |
| VDAQGTLSRIF KLGGRDSRS | | | | | | | 0.59 | | | | | | | | 0 | | |
| VDAQGTLSRLF KLGGRDSRS | | | | | | | 0.79 | | | | | | | | 0 | | |
| VHFFKNIVTPR TPPY | | 0.27 | 0.46 | | | | | | | | | | | | 0 | | |
| VVHFFKNIVTP RTPPY | | 1.4 | 0.47 | | | | | | | | | | | | 0 | | |
| YKSAHKGFKG AYDAQGTLSK | 0.002 | | 0.419 | 0.532 | 0.002 | | 0.007 | | | 0.0001 | | | | | 1 | | |
| AKSVTCTYSPA LNKM | 0.002 | | | 0.013 | | | | | | 0.17 | | | | | 1 | | |
| APPVAPAPAAP TPAA | 0.014 | | | -0.0019 | | | | | | -0.0019 | | | | | 1 | | |
| APSWPLSSSVP SQKT | 0.001 | | | 0.046 | | | | | | 0.017 | | | | | 1 | | |
| FCQLAKTCPVQ LWVD | 0.14 | | | 0.045 | | | | | | 0.42 | | | | | 3 | | |
| FSDLWKLLPEN NVLS | 1.8 | | | 0.22 | | | | | | 0.0051 | | | | | 2 | | |
| GTRVRAMAIY KQSQH | 0.15 | | | 0.49 | | | | | | 0.87 | | | | | 3 | | |
| HYNYMCNSSC MGGMN | 0.066 | | | 0.041 | | | | | | 0.0099 | | | | | 1 | | |
| LGFLHSGTAKS VTCT | 0.46 | | | 0.57 | | | | | | 0.19 | | | | | 3 | | |
| LSPLPSQAMDD LMLS | 0.011 | | | 0.0079 | | | | | | -0.0019 | | | | | 1 | | |
| NNVLSPLPSQA MDDL | 0.037 | | | 0.002 | | | | | | 0.016 | | | | | 1 | | |
| RLGFLHSGTAK SVTC | 0.028 | | | 0.057 | | | | | | 0.01 | | | | | 2 | | |
| RNTFRHSVVVP YEPP | 0.031 | | | 0.072 | | | | | | 0.27 | | | | | 3 | | |
| SWPLSSSVPSQ KTYQ | 0.0015 | | | 0.049 | | | | | | 0.013 | | | | | 1 | | |
| SYGFRLGFLHS GTAK | 0.14 | | | 0.24 | | | | | | 0.079 | | | | | 3 | | |
| WKLLPENNVLS PLPS | 0.071 | | | 0.41 | | | | | | -0.0019 | | | | | 2 | | |
| YNYMCNSSCM GGMNR | 0.041 | | | -0.0019 | | | | | | -0.0018 | | | | | 1 | | |
| AKFVAAWTLK AAA | | | | | | | | | | | | 0.27 | | 0.21 | 0 | | |
| YAKFVAAWTL KAAA | | | | | | | | | | | | 0.044 | | 0.15 | 0 | | |
| AGGIAGGLALL ACAG [?] | 0.016 | | 0.0013 | 0.0014 | 0.008 | -0.0014 | -0.0002 | 0.0007 | | -0.0003 | 0.0004 | | | -0.0005 | 1 | 1 | |
| ATSVLAGLLGN VSTV | 0.26 | | -0.0005 | 0.0021 | 0.054 | -0.0014 | 0.0008 | 0.0043 | | -0.0003 | 0.0005 | | | 0.0039 | 1 | 1 | |
| AVPLAMKLIQQ LNLN | 0.0015 | | -0.0006 | 0.0023 | 0.0097 | 0.0013 | 0.0002 | 0.13 | | 0.077 | 0.04 | | | 0.035 | 1 | 1 | |
| FLALFFIIFNKE SLA | 0.0081 | | -0.0021 | 0.043 | | 0.0082 | 0.022 | 0.0002 | 0.0021 | 0.014 | 0.0021 | | -0.001 | | 1 | 1 | |
| GRDVQNNIVDE IKYR: . | 0.0001 | 0.0001 | -0.0006 | 0.0006 | -0.0005 | 0.0014 | -0.0004 | 0.0001 | 0.081 | -0.0003 | -0.0003 | | -0.0005 | 0.085 | 0 | 1 | |
| IFHINGKIIKNSE KD | 0.032 | 0.022 | 0.066 | -0.0007 | -0.0005 | 0.0038 | 0.038 | 0.019 | 0.012 | 0.016 | 0.04 | -0.04 | 0.0093 | 0.002 | 1 | 4 | 5 |
| KHILYISFYFIL VNL | 0.0016 | | -0.0021 | 0.051 | | -0.003 | 0.0023 | 0.0009 | | 0.016 | 0.0019 | | | | 1 | 1 | |
| KYLVIVFLIFFD LFL | 0.0012 | 0.0057 | -0.002 | 0.068 | | -0.003 | -0.0009 | 0.0021 | 0.0034 | 0.013 | 0.0065 | | -0.0018 | | 1 | 1 | |
| LGNVSTVLLGG VGLV | 0.014 | 0.0001 | -0.0005 | 0.0016 | -0.0005 | -0.0014 | -0.0002 | 0.0005 | 0.0006 | -0.0003 | -0.0003 | | -0.0005 | -0.0005 | 1 | 1 | |
| LIDVHDLISDMI KKE | 0.013 | | 0.0061 | 0.031 | 0.0011 | 0.0076 | 0.0037 | 0.0001 | 0.0004 | 0.01 | 0.0096 | | 0.043 | 0.094 | 1 | 1 | |
| LIFFDLFLVNGR DVQ | 0.0042 | | | 0.0036 | | | | | | 0.047 | | | | | 1 | 1 | |
| LSVFFLALFFIIF NK | 0.0015 | 0.016 | -0.0021 | 0.0095 | | -0.0047 | 0.0015 | 0.0006 | 0.0006 | 0.009 | 0.0028 | | -0.0018 | | 0 | 1 | |
| NAREIIRLHSDA SKNKEKAL | 0.051 | 0.013 | 0.0078 | 0.024 | | | 0.01 | | -0.0001 | -0.0004 | 0.002 | | | | 1 | | |
| NDRINRENANQ LVVI | 0.077 | | 0.0015 | 0.0011 | 0.002 | 0.001 | -0.0004 | 0.0001 | | -0.0003 | -0.0003 | | | 0.0096 | 1 | 1 | |
| NHAVPLAMKLI QQLN | 0.02 | | 0.0025 | 0.0091 | | -0.003 | 0.019 | 0.24 | 0.0019 | 0.0056 | 0.17 | | 0.013 | | 1 | 1 | |
| SFYFILVNLLIF HIN | 0.0009 | 0.01 | -0.002 | 0.025 | | 0.0038 | -0.0005 | 0.0009 | 0.0004 | 0.0084 | -0.0007 | | -0.0018 | | 0 | 1 | |
| TVLLGGVGLVL YNTE | 0.49 | | -0.0005 | 0.0032 | | -0.0009 | -0.0002 | 0.0004 | | 0.0007 | -0.0002 | | | | 1 | 1 | |
| VFFLALFFIIFN KES | 0.0006 | 0.018 | -0.0021 | 0.0047 | | 0.01 | -0.0005 | 0.0002 | -0.0002 | 0.0056 | -0.0007 | | -0.0018 | | 0 | 1 | |
| VSTVLLGGVGL VLYN | 0.88 | 0.008 | 0.0005 | 0.0067 | | -0.0009 | 0.0003 | 0.0011 | 0.0002 | 0.002 | -0.0002 | | 0.012 | | 1 | 1 | |
| VVILTDGIPDSI QDS | 0.0001 | | -0.0006 | 0.12 | 0.0045 | -0.0014 | -0.0004 | 0.0001 | | -0.0003 | -0.0003 | | | 0.0114 | 1 | 1 | |
| YADSAWENVK NVIGPFMKAV | 0.055 | 0.018 | 0.036 | -0.0045 | | | 0.0009 | | -0.0001 | 0.031 | 0.01 | | | | 2 | | |
| YKFVVPGAATP YAGE | 0.11 | 0.0008 | 0.0053 | 0.0057 | | -0.0014 | 0.0036 | 0.0001 | 0.0017 | 0.016 | 0.0026 | | 0.02 | | 1 | 1 | |
| DQSYLQDSDPD SFQD | | | | 0.049 | | | | | | | | | | | 1 | | |
| DYSFLQDSDPD SFQD | | | | 0.08 | | | | | | | | | | | 1 | | |
| DYSYFQDSDPD SFQD | | | | 0.089 | | | | | | | | | | | 1 | | |
| DYSYLQDSDPD SFQD | -0.0001 | | | 0.053 | 0.0004 | | | | -0.0001 | -0.0009 | | 0.029 | | 0.0016 | 1 | | |
| DYSYLQDSDPD SFQD | | | | 0.046 | | | | | | | | | | | 1 | | |
| DYSYLQDSVPD SFQD | | | | 0.29 | | | | | | | | | | | 1 | | |
| DYSYQQDSDP DSFQD | | | | 0.046 | | | | | | | | | | | 1 | | |
| NILLSNAPLGP QFP | | | | 0.14 | | | | | | | | | | | 1 | | |
| QNFLLSNAPLG PQFP. | | | | 0.82 | | | | | | | | | | | 1 | | |
| QNIFLSNAPLGP QFP | | | | 0.14 | | | | | | | | | | | 1 | | |
| QNILLSNAPLG PQFP | 0.22 | | | 0.04 | 0.37 | | | | 0.66 | 0.085 | | -0.0024 | | 0.13 | 2 | | |
| QNILLSNAQLG PQFP | | | | 0.16 | | | | | | | | | | | 1 | | |
| QNILLSNAVLG PQFP | | | | 0.29 | | | | | | | | | | | 1 | | |
| QNILLSNVPLG PQFP | | | | 3.7 | | | | | | | | | | | 1 | | |
| | | | | 0.06 | | | | | | | | | | | 1 | | |
| | | | | 0.074 | | | | | | | | | | | 1 | | |
| QNIQLSNAPLG PQFP | | | | 0.06 | | | | | | | | | | | 1 | | |
| QNIVLSNAPLG PQFP | | | | 0.48 | | | | | | | | | | | 1 | | |
| QNVLLSNAPLG PQFP | | | | 0.047 | | | | | | | | | | | 1 | | |
| SYLQDSDPDSF QD | | | | 0.14 | | | | | | | | | | | 1 | | |
| SYLQDSVPDSF QD | | | | 0.72 | | | | | | | | | | | 1 | | |
| YLQDSDPDSFQ D | | | | 0.08 | | | | | | | | | | | 1 | | |
| YSYLQDSDPDS FQD | | | | 0.19 | | | | | | | | | | | 1 | | |

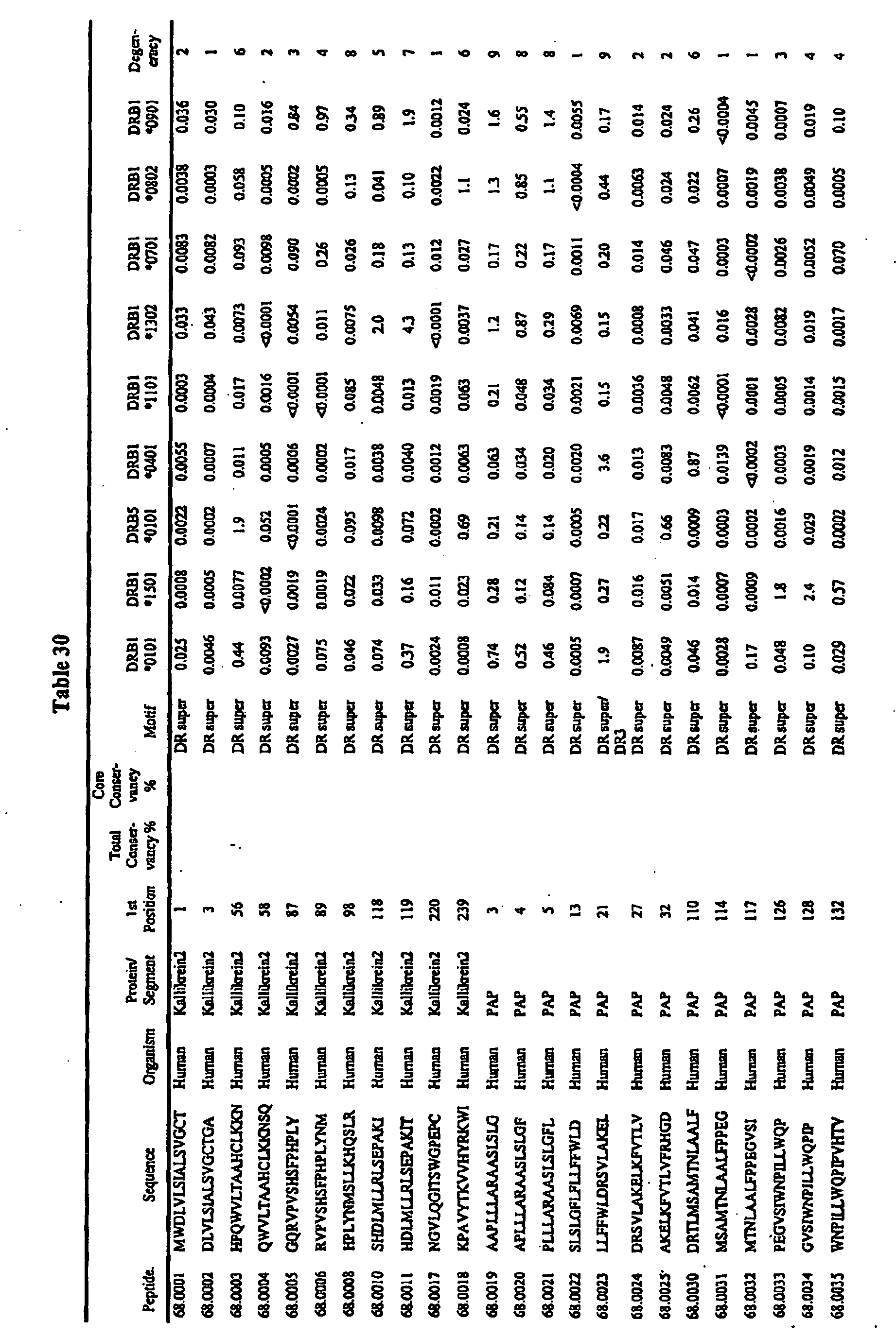

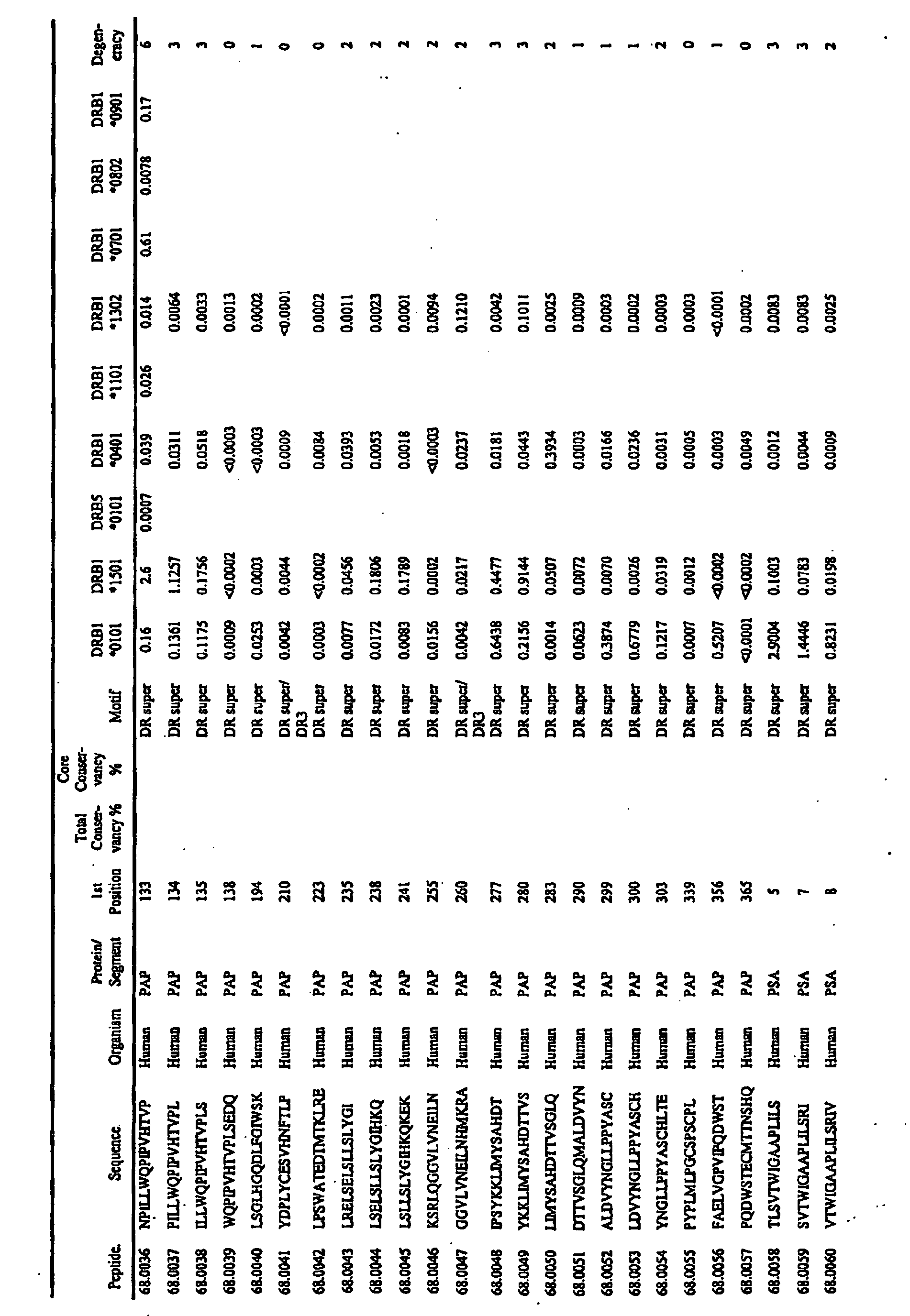

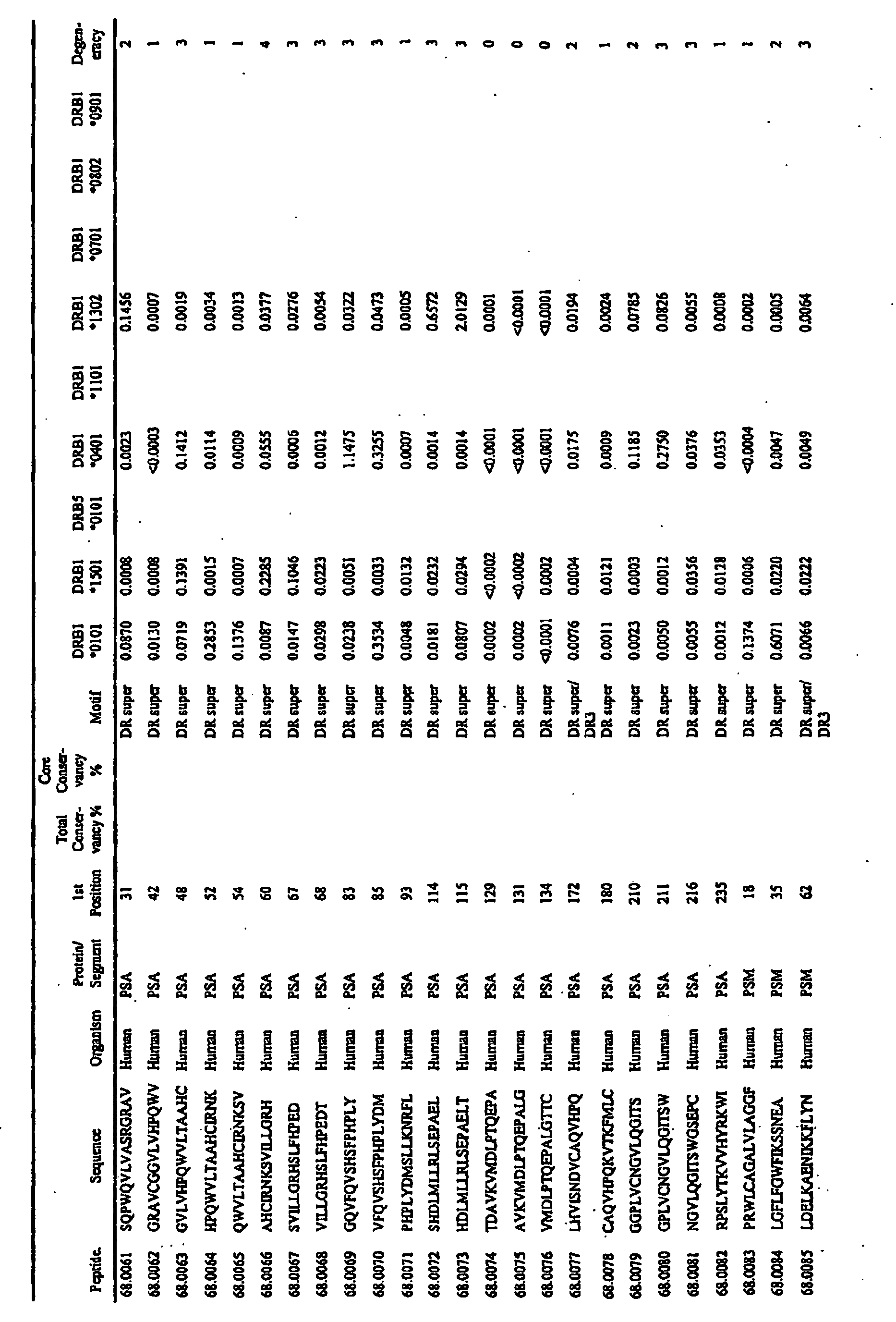

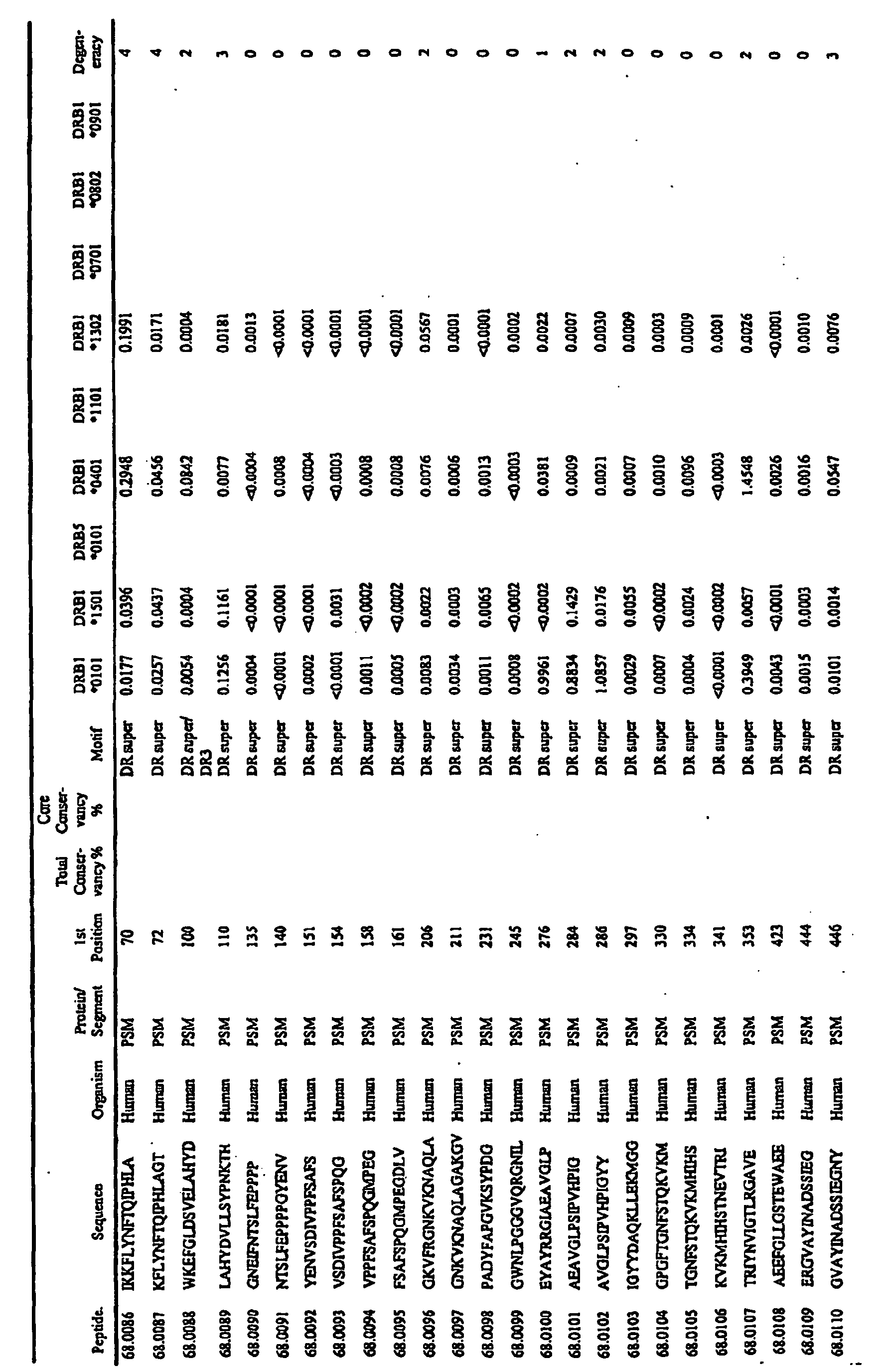

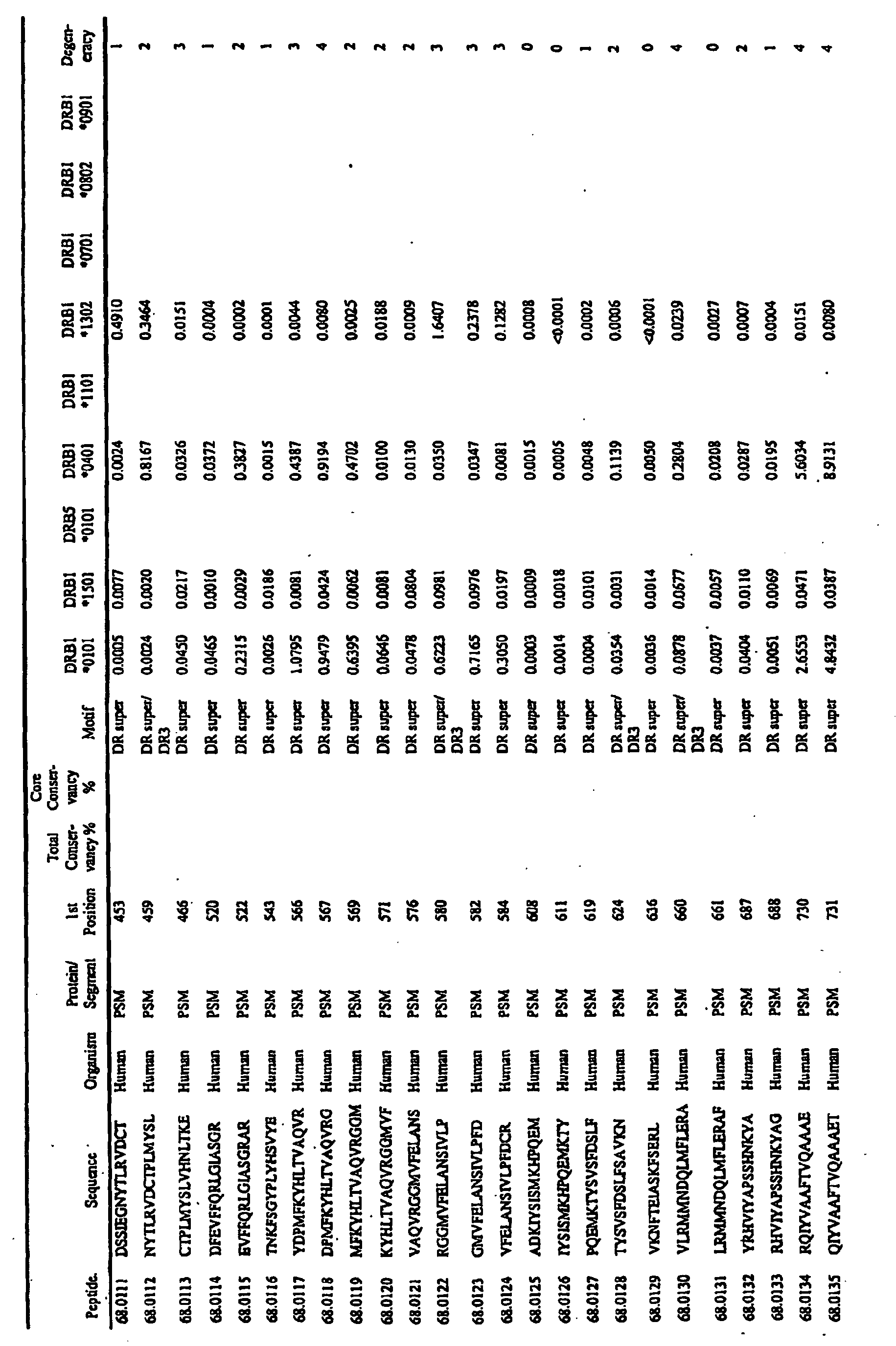

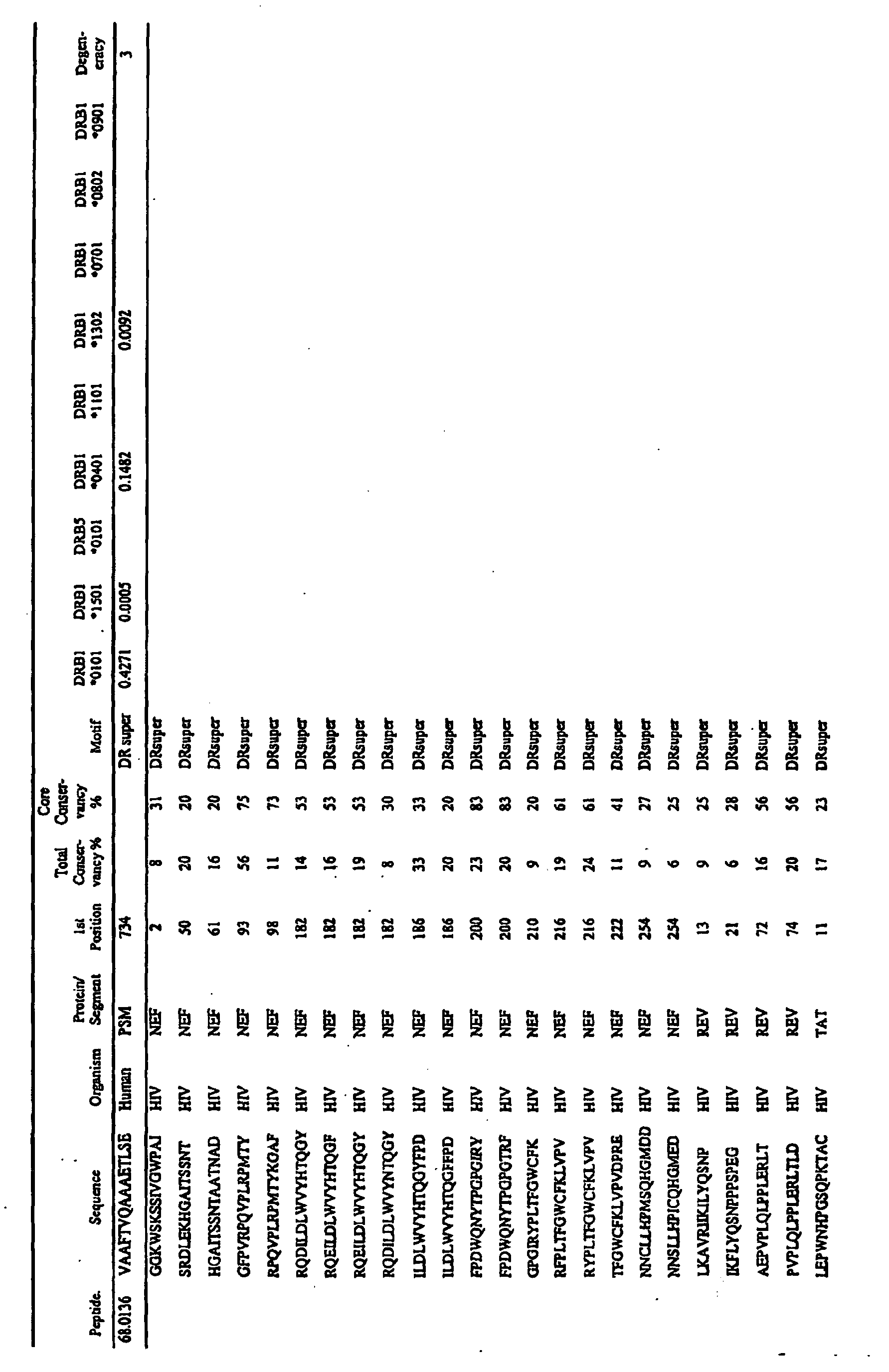

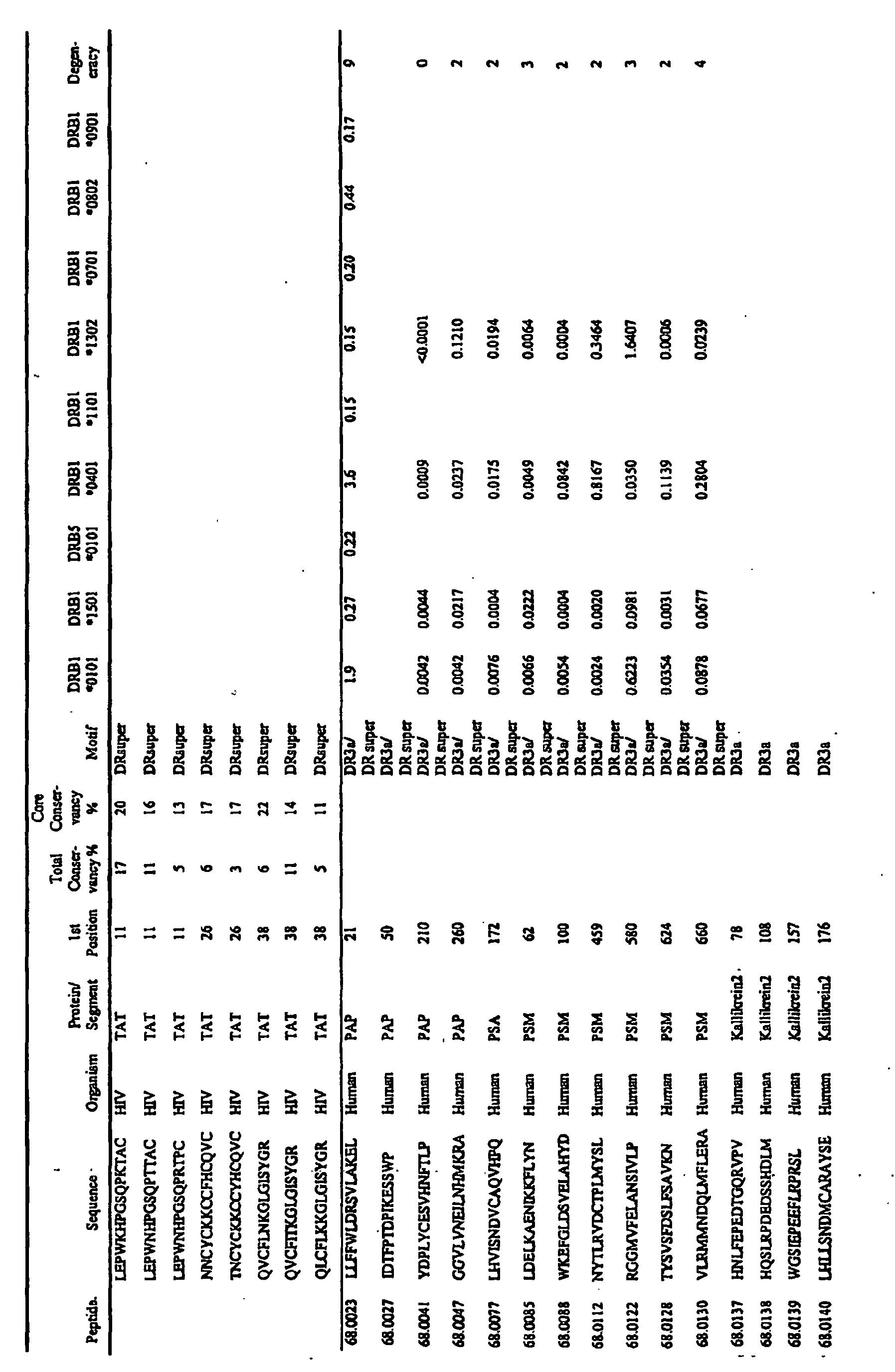

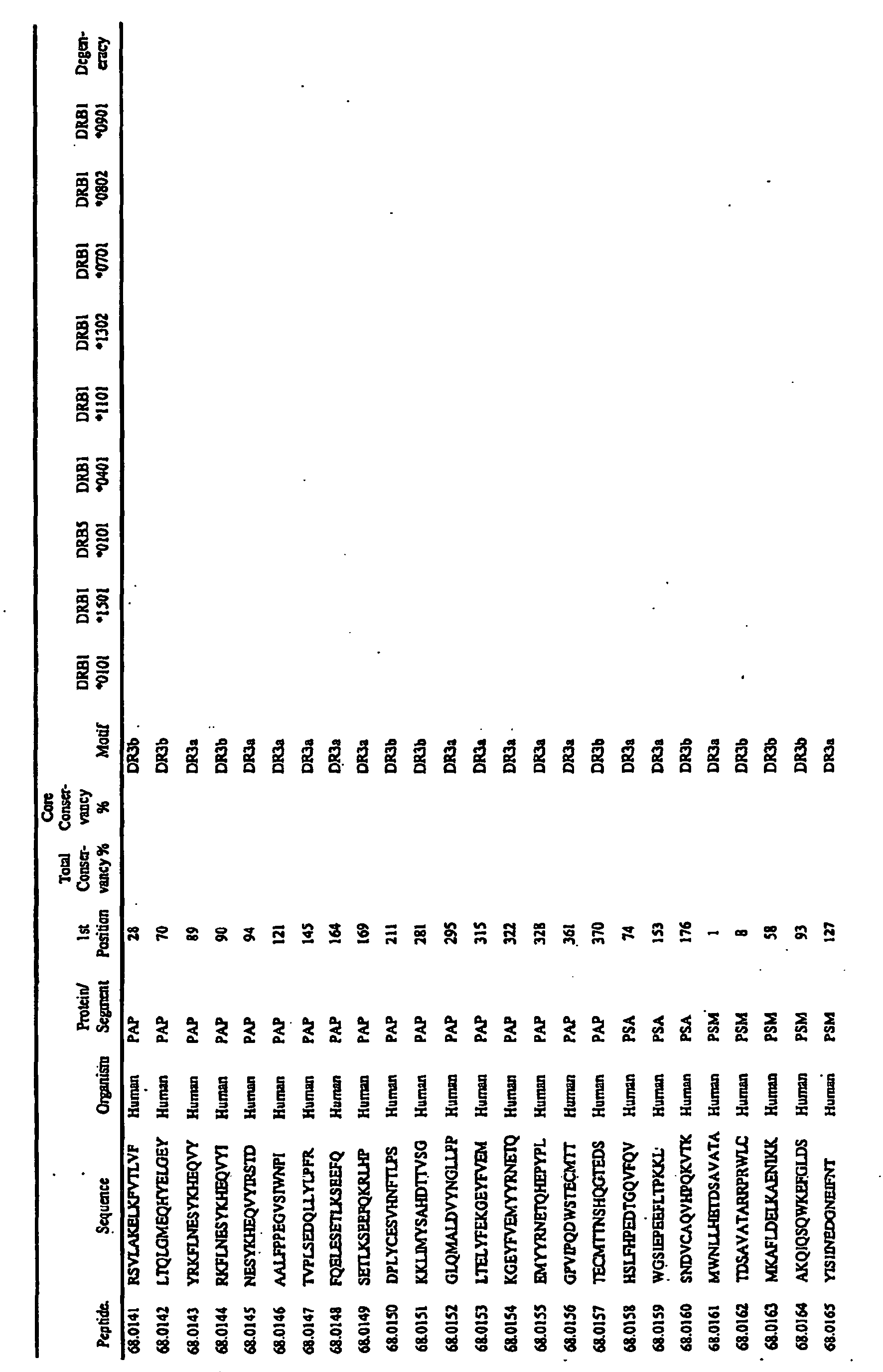

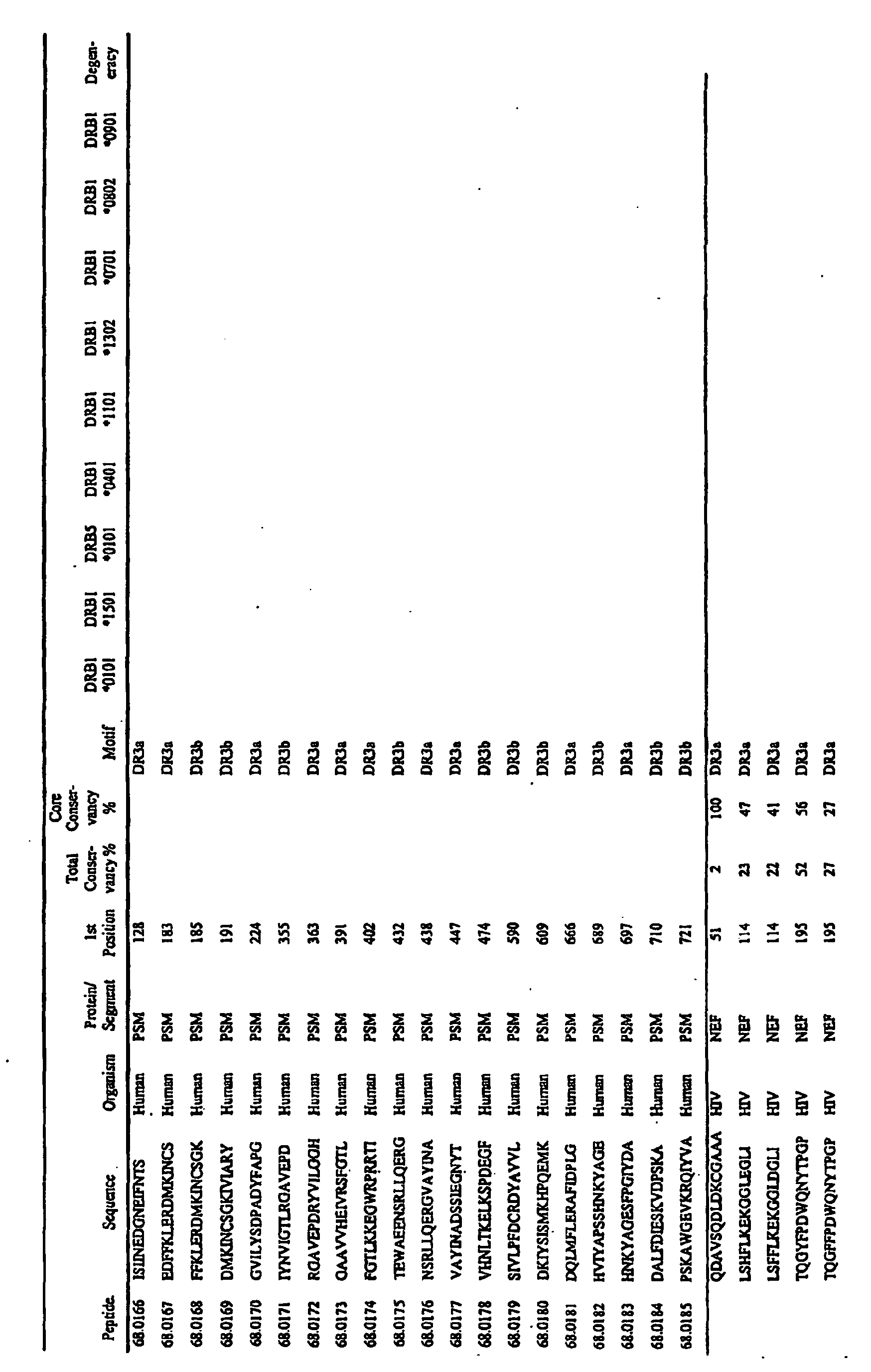

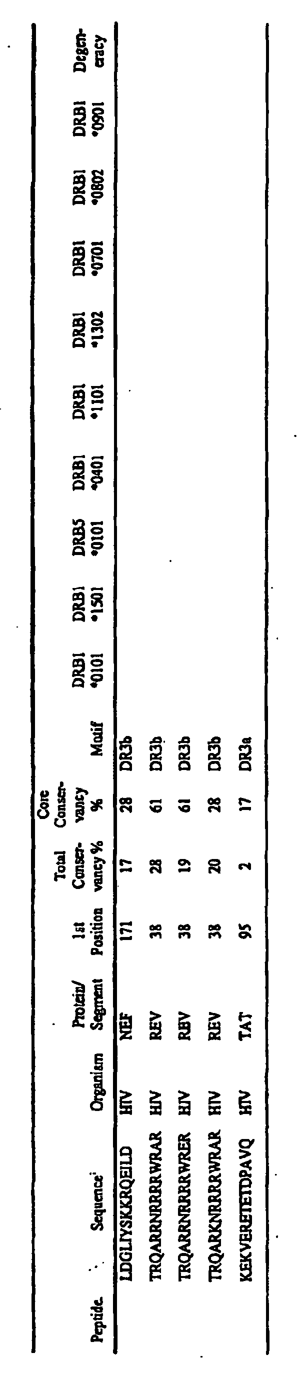

**Table 31**

| Class II motif bearing peptides for HIV regulatory proteins | | | | | | | | |
|---|---|---|---|---|---|---|---|---|
| HLA | protein | % cons. | # aa | position | sequence | position 9 | core sequence | core conservation |
| DR3a | NEF | 2 | 15 | 51 | QDAvsqdldkcgAAA | 54 | VSQDLDKCG | 100 |
| DRsuper | NEF | 23 | 15 | 200 | FPDwqnytpgpgIRY | 204 | WQNYTPGPG | 83 |
| DRsuper | NEF | 20 | 15 | 200 | FPDwqnytpgpgTRF | 204 | WQNYTPGPG | 83 |
| DRsuper | NEF | 56 | 15 | 93 | GFPvrpqvplrpMTY | 97 | VRPQVPLRP | 75 |
| DRsuper | NEF | 11 | 15 | 98 | RPQvplrpmtykGAF | 101 | VPLRPMTYK | 73 |
| DRsuper | NEF | 19 | 15 | 216 | RFPltfgwcfklVPV | 221 | LTFGWCFKL | 61 |
| DRsuper | NEF | 24 | 15 | 216 | RYPltfgwcfklVPV | 221 | LTFGWCFKL | 61 |
| DR3a | NEF | 52 | 15 | 195 | TQGyfpdwqnytPGP | 199 | YFPDWQNYT | 56 |
| DRsuper | NEF | 14 | 15 | 182 | RQDildlwvyhtQGY | 186 | ILDLWVYHT | 53 |
| DRsuper | NEF | 16 | 15 | 182 | RQEildlwvyhtQGF | 186 | ILDLWVYHT | 53 |
| DRsuper | NEF | 19 | 15 | 182 | RQEitdlwvyhtQGY | 186 | ILDLWVYHT | 53 |
| DR3a | NEF | 23 | 15 | 114 | LSHflkekggleGLI | 117 | FLKEKGGLE | 47 |
| DR3a | NEF | 22 | 15 | 114 | LSFflkekggIdGLI | 117 | FLKEKGGLD | 41 |
| DRsuper | NEF | 11 | 15 | 222 | TFGwcfklvpvdPRE | 225 | WCFKLVPVD | 41 |
| DRsuper | NEF | 33 | 15 | 186 | ILDIwvyhtqgyFPD | 190 | LWVYHTQGY | 33 |
| DRsuper | NEF | 8 | 15 | 2 | GGKwskssivgwPAI | 5 | WSKSSIVGW | 31 |
| DRsuper | NEF | 8 | 15 | 182 | RQDildlwvyntQGY | 186 | ILDLWVYNT | 30 |
| DR3b | NEF | 17 | 15 | 171 | LDGliyskkrqeILD | 174 | LIYSKKRQE | 28 |
| DR3a | NEF | 27 | 15 | 195 | TQGffpdwqnytPGP | 199 | FFPDWQNYT | 27 |
| DRsuper | NEF | 9 | 15 | 254 | NNCllhpmsqhgMDD | 257 | LLHPMSQHG | 27 |
| DRsuper | NEF | 6 | 15 | 254 | NNSIlhpicqhgMED | 257 | LLHPICQHG | 25 |
| DRsuper | NEF | 9 | 15 | 210 | GPGirypltfgwCFK | 214 | IRYPLTFGW | 20 |
| DRsuper | NEF | 16 | 15 | 61 | HGAitssntaatNAD | 64 | ITSSNTAAT | 20 |
| DRsuper | NEF | 20 | 15 | 50 | SRDlekhgaitsSNT | 58 | LEKHGAITS | 20 |
| DRsuper | NEF | 20 | 15 | 186 | ILDlwvyhtqgfFPD | 190 | LWVYHTQGF | 20 |
| | | | | | | | | |
| DR3b | REV | 28 | 15 | 38 | TRQarrnrrrrwRAR | 41 | ARRNRRRRW | 61 |
| DR3b | REV | 19 | 15 | 38 | TRQarrnrrrrwRER | 41 | ARRNRRRRW | 61 |
| DRsuper | REV | 20 | 15 | 74 | PVPlqlpplerlTLD | 77 | LQLPPLERL | 56 |
| DRsuper | REV | 16 | 15 | 72 | AEPvplqlppleRLT | 75 | VPLQLPPLB | 56 |
| DR3b | REV | 20 | 15 | 38 | TRQarknrrrrwRAR | 41 | ARKNRRRRW | 28 |
| DRsuper | REV | 6 | 15 | 21 | IKFlyqsnpppsPEG | 24 | LYQSNPPPS | 28 |
| DRsuper | REV | 9 | 15 | 13 | LKAvriikilyqSNP | 18 | VRIIKILYQ | 25 |
| | | | | | | | | |
| DRsuper | TAT | 17 | 15 | 11 | LEPwnhpgsqpkTAC | 14 | WNHPGSQPK | 23 |
| DRsuper | TAT | 6 | 15 | 38 | QVCflnkglgisYGR | 41 | FLNKGLGIS | 22 |
| DRsuper | TAT | 17 | 15 | 11 | LEPwkhpgsqpkTAC | 14 | WKHPGSQPK | 20 |
| DR3a | TAT | 2 | 15 | 95 | KEKveretetdpAVQ | 98 | VERETETDP | 17 |
| DRsuper | TAT | 6 | 15 | 26 | NNCyckkccfhcQVC | 29 | YCKKCCFHC | 17 |
| DRsuper | TAT | 3 | 15 | 26 | TNCyckkccyhcQVC | 29 | YCKKCCYHC | 17 |
| DRsuper | TAT | 11 | 15 | 11 | LEPwnhpgsqptTAC | 14 | WIVHPGSQPT | 16 |
| DRsuper | TAT | 11 | 15 | 38 | QVCfitkglgisYGR | 41 | FITKGLGIS | 14 |
| DRsuper | TAT | 5 | 15 | 11 | LEPwnhpgsqprTPC | 14 | WNHPGSQPR | 13 |
| DRsuper | TAT | 5 | is | 38 | QLCflkkglgisYGR | 41 | FLKKGLGIS | 11 |

## Claims

1. A peptide analog derived from the HPV E6 protein **characterized by** the following:
- comprising a HLA-A2 supermotif,
- having a binding affinity value of 50nM or less for at least two HLA molecules belonging to the HLA-A2 supertype selected from the group consisting of: HLA-A *0201, -A*0202, -A*0203, - A*0205, -A*0206 and -A*6802, and
- consisting of an amino acid sequeace selected from the group consisting of:
TLHDHLECV,
TLHDLCQAV,
TLHEYMLDV,
FLFADLRIV,
FLFADLTVV,
BLFKDLCIV,
FLEKDLFVV,
FLFDLCIV,
FLFDLTIV,
GICKLCLRFV,
GLCKLCLRFL,
GLYNLLIRCV,
ILYRDClAYA,
IVYRDCIAYV,
KLHELSSAV,
KLPDLCTEV,
KLPQLCTEV,
KLTNKGICDV,
KLTNTELYNL,
KLTNTGLYNV,
KLVLELTEV,
LLDVSIACV,
LLYRDDFPYA,
RLLHELCEV,
SLHEIRLQCV,
SLYGATLESI,
SLYGDTLEKL,
SLYGDTI:EQV,
SLYGETLEKI,
SLYGNTLEQT,
SVYGATLESY,
SVYGDTLEKV,
SVYGTLEKV,
SVYGNTLEQV,
YLFAFKDLGI, and
YQFAFKLCV,

2. A composition comprising the peptide according to claim 1.

3. The composition according to claim 2, further comprising a CTL and/or a HTL binding peptide.

4. The composition according to claim 3, wherein the peptides are linked directly or via an amino acid spacer.

5. The composition according to claim 4, which is a polyepitope construct.

6. The composition according to any one of claims 2 to 5, further comprising an adjuvant.

7. The composition according to any one of claims 2 to 5, further comprising a pharmaceutically acceptable execicipient.

8. The composition according to claim 7, which is a vaccine composition.

9. An isolated nucleic aicid encoding the peptide or the polyepitope construct according to claims 1 or 5.

10. A vector comprising the nucleic acid according to claim 9.

11. The peptide, composition, nucleic, acid or vector according to any one of claims 1 to 10 for use as a medicament.

12. Use of the peptide, composition, nucleic acid or vector according to any one of claims 1 to 10 for the manufacture of a medicament for inducing a T cell response against HPV.

13. Use according to claim 12 for treating HPV infection.

## Patentansprüche

1. Peptidanalogon, das von dem HPV-E6-Protein abgeleitet ist, das durch Folgendes **gekennzeichnet** ist:
- umfassend ein HLA-A2-Supermotiv,
- einen Wert der Bindungsaffinität von 50 nM oder weniger für wenigstens zwei HLA-Moleküle aufweisend, die zu dem HLA-A2-Supertyp gehören, die ausgewählt sind aus der Gruppe, bestehend aus HLA-A*0201, -A*0202, -A*0203, -A*0205,-A*0206 und -A*6802, und
- bestehend aus einer Aminosäuresequenz, ausgewählt aus der Gruppe, bestehend aus:
TLHDIILECV,
TLHDLCQAV,
TLHEYMLDV,
FLFADLRIV,
FLFADLTVV,
FLFKDLCIV,
FLFKDLFVV,
FLFRDLCIV,
FLFTDLTIV,
GICKLCLRFV,
GLCKLCLRFL,
GLYNLLIRCV,
ILYRDCIAYA,
IVYRDCIAYV,
KLHELSSAV,
KLPDLCTEV,
KLPQLCTEV,
KLTNKGICDV,
KLTNTELYNL,
KLTNTGLYNV,
KLVLELTEV,
LLDVSIACV,
LLYRDDFPYA,
RLLHELCEV,
SLHEIRLQCV,
SLYGATLESI,
SLYGDTLEKL,
SLYGDTLEQV,
SLYGETLEKI,
SLYGNTLEQT,
SVYGATLESV,
SVYGDTLEKV,
SVYGETLEKV,
SVYGNTLEQV,
YLFAFKDLCI und
YQFAFKDLCV.

2. Zusammensetzung, die das Peptid gemäß Anspruch 1 umfasst.

3. Zusammensetzung gemäß Anspruch 2, die außerdem ein CTL-und/oder ein HTL-Bindungspeptid umfasst.

4. Zusammensetzung gemäß Anspruch 3, wobei die Peptide direkt oder über einen Aminosäure-Spacer gebunden sind.

5. Zusammensetzung gemäß Anspruch 4, welche ein Polyepitop-Konstrukt ist.

6. Zusammensetzung gemäß einem der Ansprüche 2 bis 5, die außerdem ein Adjuvans umfasst.

7. Zusammensetzung gemäß einem der Ansprüche 2 bis 5, die außerdem ein pharmazeutisch verträgliches Exzipiens umfasst.

8. Zusammensetzung gemäß Anspruch 7, die eine Vakzin-Zusammensetzung ist.

9. Isolierte Nukleinsäure, die das Peptid oder das Polyepitop-Konstrukt gemäß Anspruch 1 oder 5 umfasst.

10. Vektor, der die Nukleinsäure gemäß Anspruch 9 umfasst.

11. Peptid, Zusammensetzung, Nukleinsäure oder Vektor gemäß einem der Ansprüche 1 bis 10 zur Verwendung als Medikament.

12. Verwendung des Peptids, der Zusammensetzung, der Nukleinsäure oder des Vektors gemäß einem der Ansprüche 1 bis 10 für die Herstellung eines Medikaments zum Induzieren einer T-Zellantwort gegen HPV.

13. Verwendung gemäß Anspruch 12 zum Behandeln einer HPV-Infektion.

## Revendications

1. Peptide analogue dérivé de la protéine HPV E6 **caractérisé en ce que** ledit peptide:
- comprend un supermotif HLA-A2,
- a une valeur d'affinité de liaison de 50 nM ou moins pour au moins deux molécules HLA appartenant au supertype HLA-A2 sélectionnées à partir du groupe consistant en : HLA-A*0201, -A*0202, -A*0203, -A*0205, -A*0206 et -A*6802, et
- consiste en une séquence d'acides aminés sélectionnée parmi le groupe consistant en:
TLHDIILECV,
TLHDLCQAV,
TLHEYMLDV,
FLFADLRIV,
FLFADLTVV,
FLFKDLCIV,
FLFKDLFVV,
FLFRDLCIV,
FLFTDLTIV,
GICKLCLRFV,
GLCKLCLRFL,
GLYNLLIRCV,
ILYRDCIAYA,
IVYRDCIAYV,
KLHELSSAV,
KLPDLCTEV,
KLPQLCTEV,
KLTNKGICDV,
KLTNTELYNL,
KLTNTGLYNV,
KLVLELTEV,
LLDVSIACV,
LLYRDDFPYA,
RLLHELCEV,
SLHEIRLQCV,
SLYGATLESI,
SLYGDTLEKL,
SLYGDTLEQV,
SLYGETLEKI,
SLYGNTLEQT,
SVYGATLESV,
SVYGDTLEKV,
SVYGETLEKV,
SVYGNTLEQV,
YLFAFKDLCI, et
YQFAFKDLCV.

2. Composition comprenant le peptide selon la revendication 1.

3. Composition selon la revendication 2, comprenant en outre un peptide de liaison de CTL et/ou d'HTL.

4. Composition selon la revendication 3, dans laquelle les peptides sont liés directement ou via un espaceur d'acides aminés.

5. Composition selon la revendication 4, qui est une construction polyépitope.

6. Composition selon l'une quelconque des revendications 2 à 5, comprenant en outre un adjuvant.

7. Composition selon l'une quelconque des revendications 2 à 5, comprenant en outre un excipient de qualité pharmaceutique.

8. Composition selon la revendication 7, qui est une composition de vaccin.

9. Acide nucléique isolé encodant le peptide ou la construction polyépitope selon les revendications 1 ou 5.

10. Vecteur comprenant l'acide nucléique selon la revendication 9.

11. Le peptide, la composition, l'acide nucléique ou le vecteur selon l'une quelconque des revendications 1 à 10 pour son utilisation en tant que médicament.

12. Utilisation du peptide, de la composition, de l'acide nucléique ou du vecteur selon l'une quelconque des revendications 1 à 10 pour la fabrication d'un médicament pour provoquer une réponse des cellules T contre le HPV.

13. Utilisation selon la revendication 12 pour traiter une infection HPV.
